(19) **Europäisches Patentamt**
**European Patent Office**
**Office européen des brevets**

(11) **EP 2 767 833 A2**

(12) **EUROPEAN PATENT APPLICATION**

(43) Date of publication:
**20.08.2014 Bulletin 2014/34**

(51) Int Cl.:
*G01N 33/68* (2006.01)    *C12Q 1/68* (2006.01)

(21) Application number: **14156423.7**

(22) Date of filing: **21.10.2009**

---

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **21.10.2008   US 107307 P**
**21.10.2008   US 107293 P**
**21.10.2008   US 107287 P**
**10.11.2008   US 113059 P**
**10.11.2008   US 113034 P**
**10.11.2008   US 113069 P**
**10.11.2008   US 113039 P**
**10.11.2008   US 113093 P**
**10.11.2008   US 113078 P**
**14.11.2008   US 115028 P**
**14.11.2008   US 115026 P**
**15.11.2008   US 115043 P**
**15.11.2008   US 115050 P**
**15.11.2008   US 115046 P**
**22.11.2008   US 117143 P**
**22.11.2008   US 117138 P**
**22.11.2008   US 117136 P**

(62) Document number(s) of the earlier application(s) in accordance with Art. 76 EPC:
**09822670.7 / 2 347 260**

(71) Applicant: **Astute Medical, Inc.**
**San Diego, CA 92121 (US)**

(72) Inventors:
• **Anderberg, Joseph**
**Encinitas, CA 92024 (US)**
• **Gray, Jeff**
**Solana Beach, CA 92075 (US)**
• **McPherson, Paul**
**Encinitas, CA 92024 (US)**
• **Nakamura, Kevin**
**Cardiff By the Sea, CA 92007 (US)**

(74) Representative: **Schiweck, Weinzierl & Koch**
**European Patent Attorneys**
**Landsberger Straße 98**
**80339 München (DE)**

Remarks:
This application was filed on 24-02-2014 as a divisional application to the application mentioned under INID code 62.

---

(54) **Methods and compositions for diagnosis and prognosis of renal injury and renal failure**

(57)    The present invention relates to methods and compositions for monitoring, diagnosis, prognosis, and determination of treatment regimens in subjects suffering from or suspected of having a renal injury. In particular, the invention relates to using assays that detect Thrombospondin-1 as a diagnostic and prognostic biomarker in renal injuries.

EP 2 767 833 A2

**Description**

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830, which are hereby incorporated by reference in their entirety. Renal disease and/or injury may be acute or chronic. Acute and chronic kidney disease are described as follows (from Current Medical Diagnosis & Treatment 2008, 47th Ed, McGraw Hill, New York, pages 785-815, which are hereby incorporated by reference in their entirety): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222, and which is hereby incorporated by reference in their entirety:

| Type | Risk Factors |
| --- | --- |
| **Prerenal** | |
| ECF volume depletion | Excessive diuresis, hemorrhage, GI losses, loss of intravascular fluid into the extravascular space (due to ascites, peritonitis, pancreatitis, or bums), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |

(continued)

| Intrinsic Renal | |
| --- | --- |
| Acute tubulointerstitial nephritis | Drug reaction (eg, β-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| **Postrenal** | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, JAm Soc Nephrol 16: 3365-3370, 2005, which, with the references listed therein, are hereby incorporated by reference in their entirety. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004, hereby incorporated by reference in its entirety) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, which is hereby incorporated by reference in its entirety, proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, each hereby incorporated by reference in its entirety, the RIFLE criteria provide a uniform definition of AKI which has been validated in numerous studies.

More recently, Mehta et al., Crit. Care 11:R31 (doi:10.1186.cc5713), 2007, hereby incorporated by reference in its entirety, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006; 7(4): 177-197, hereby incorporated by reference in its entirety) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the invention to provide methods and compositions for evaluating renal function in a subject. As described herein, measurement of one or more markers selected from the group consisting of soluble CD44 antigen, Angiopoietin-1, soluble Angiopoietin-1 receptor, C-X-C chemokine motif 5, soluble Endoglin, soluble Tumor-associated calcium signal transducer 1, Erythropoietin, soluble Fractalkine, Heme oxygenase 1, soluble Interleukin-1 receptor type II, soluble Interleukin-6 receptor subunit-alpha, Lymphotactin, Lymphotoxin-alpha, Stromelysin-1, C-C motif chemokine 22, C-C motif chemokine 5, and Thrombospondin-1 (collectively referred to herein as "kidney injury markers, and individually as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

These kidney injury markers may be used, individually or in panels comprising a plurality of kidney injury markers, for risk stratification (that is, to identify subjects at risk for a future injury to renal function, for future progression to reduced renal function, for future progression to ARF, for future improvement in renal function, *etc.);* for diagnosis of existing disease (that is, to identify subjects who have suffered an injury to renal function, who have progressed to reduced renal function, who have progressed to ARF, *etc.);* for monitoring for deterioration or improvement of renal function; and for predicting a future medical outcome, such as improved or worsening renal function, a decreased or increased mortality risk, a decreased or increased risk that a subject will require renal replacement therapy *(i.e.*, hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present invention relates to methods for evaluating renal status in a subject. These methods comprise performing an assay method that is configured to detect one or more kidney injury markers of the present invention in a body fluid sample obtained from the subject. The assay result(s), for example a measured concentration of one or more markers selected from the group consisting of soluble CD44 antigen, Angiopoietin-1, soluble Angiopoietin-1 receptor, C-X-C chemokine motif 5, soluble Endoglin, soluble Tumor-associated calcium signal transducer 1, Erythropoietin, soluble Fractalkine, Heme oxygenase 1, soluble Interleukin-1 receptor type II, soluble Interleukin-6 receptor subunit-alpha, Lymphotactin, Lymphotoxin-alpha, Stromelysin-1, C-C motif chemokine 22, C-C motif chemokine 5, and Thrombospondin-1 is/are then correlated to the renal status of the subject. This correlation to renal status may include correlating the assay result(s) to one or more of risk stratification, diagnosis, prognosis, staging, classifying and monitoring of the subject as described herein. Thus, the present invention utilizes one or more kidney injury markers of the present invention for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example a measured concentration of one or more markers selected from the group consisting of soluble CD44 antigen, Angiopoietin-1, soluble Angiopoietin-1 receptor, C-X-C chemokine motif 5, soluble Endoglin, soluble Tumor-associated calcium signal transducer 1, Erythropoietin, soluble Fractalkine, Heme oxygenase 1, soluble Interleukin-1 receptor type II, soluble Interleukin-6 receptor subunit-alpha, Lymphotactin, Lymphotoxin-alpha, Stromelysin-1, C-C motif chemokine 22, C-C motif chemokine 5, and Thrombospondin-1 is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example a measured concentration of one or more markers selected from the group consisting of soluble CD44 antigen, Angiopoietin-1, soluble Angiopoietin-1 receptor, C-X-C chemokine motif 5, soluble Endoglin, soluble Tumor-associated calcium signal transducer 1, Erythropoietin, soluble Fractalkine, Heme oxygenase 1, soluble Interleukin-1 receptor type II, soluble Interleukin-6 receptor subunit-alpha, Lymphotactin, Lymphotoxin-alpha, Stromelysin-1, C-C motif chemokine 22, C-C motif chemokine 5, and Thrombospondin-1 is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example a measured concentration of one or more markers selected from the group consisting of soluble CD44 antigen, Angiopoietin-1, soluble Angiopoietin-1 receptor, C-X-C chemokine motif 5, soluble Endoglin, soluble Tumor-associated calcium signal transducer 1, Erythropoietin, soluble Fractalkine, Heme oxygenase 1, soluble Interleukin-1 receptor type II, soluble Interleukin-6 receptor subunit-alpha, Lymphotactin, Lymphotoxin-alpha, Stromelysin-1, C-C motif chemokine 22, C-C motif chemokine 5, and Thrombospondin-1 is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, *etc.*), by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, *etc.* This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatinine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present invention also relates to devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, *etc.)* as well as molecules that may be indirectly detected by production of a detectable reaction product *(e.g.,* enzymes such as horseradish peroxidase, alkaline phosphatase, *etc.)* or through the use of a specific binding molecule which itself may be detectable *(e.g.,* a labeled antibody that binds to the second antibody, biotin, digoxigenin, maltose, oligohistidine, 2,4-dintrobenzene, phenylarsenate, ssDNA, dsDNA, *etc.).*

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer *(e.g.,* a diffraction grating, electrochemical sensor, etc) for generation of a signal, while in others, a signal is generated by a transducer that is spatially separate from the solid phase antibody *(e.g.,* a fluorometer that employs an excitation light source and an optical detector). This list is not meant to be limiting. Antibody-based biosensors may also be employed to determine the presence or amount of analytes that optionally eliminate the need for a labeled molecule.

BRIEF DESCRIPTION OF THE FIGURES

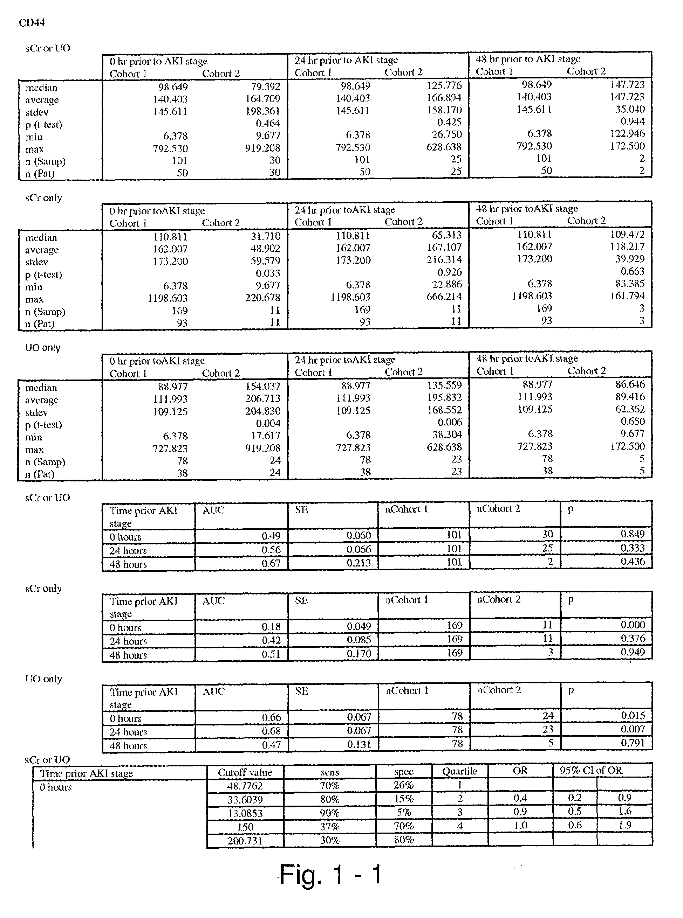

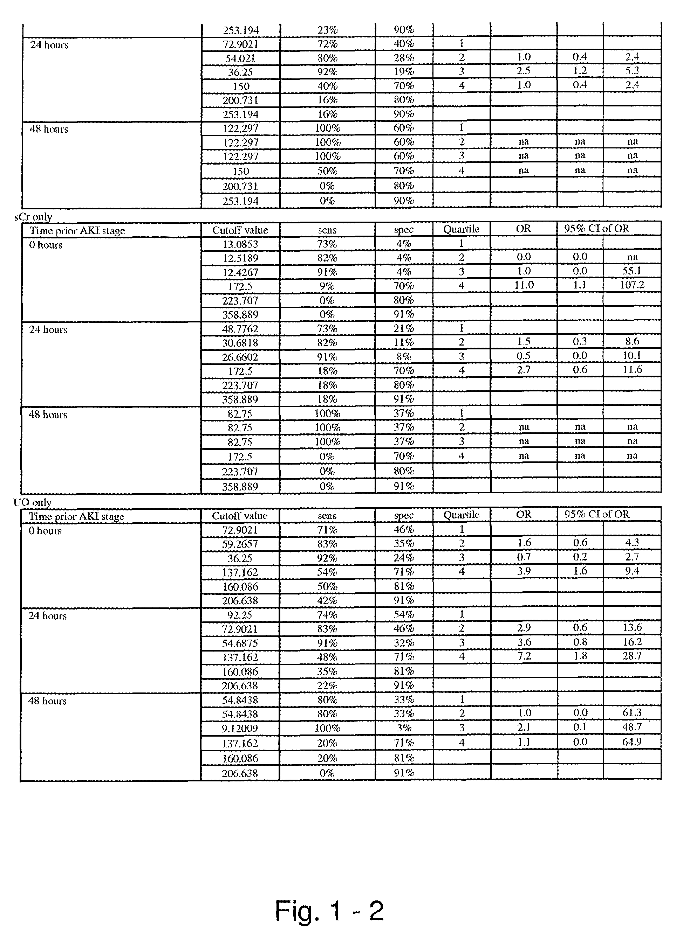

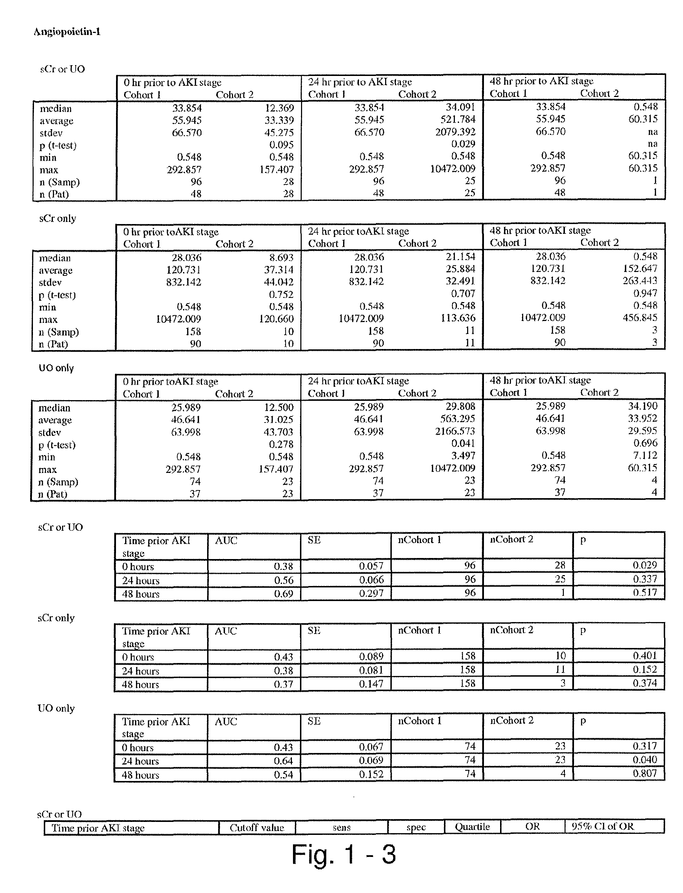

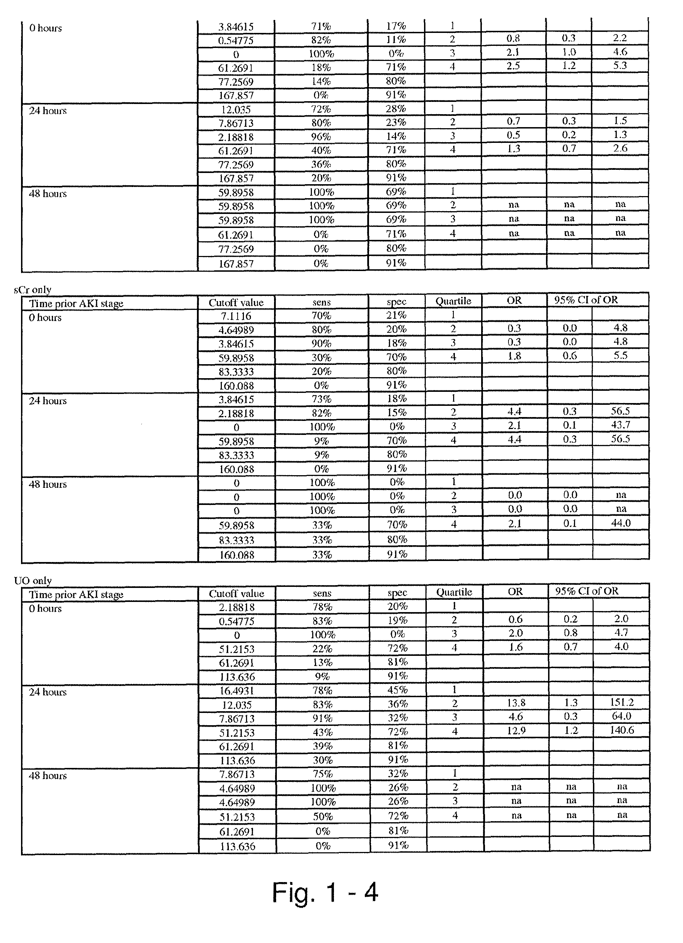

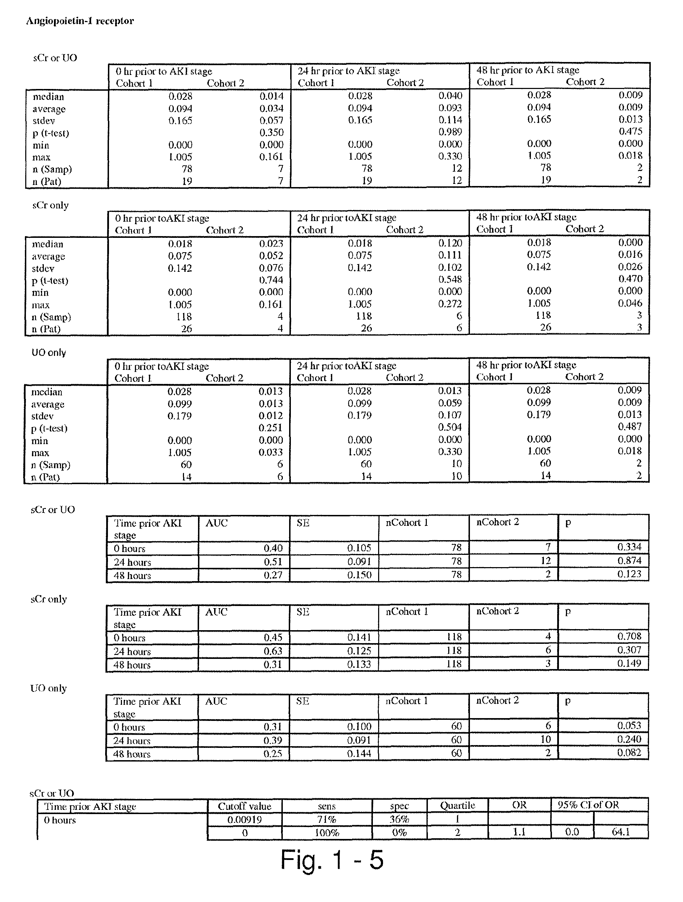

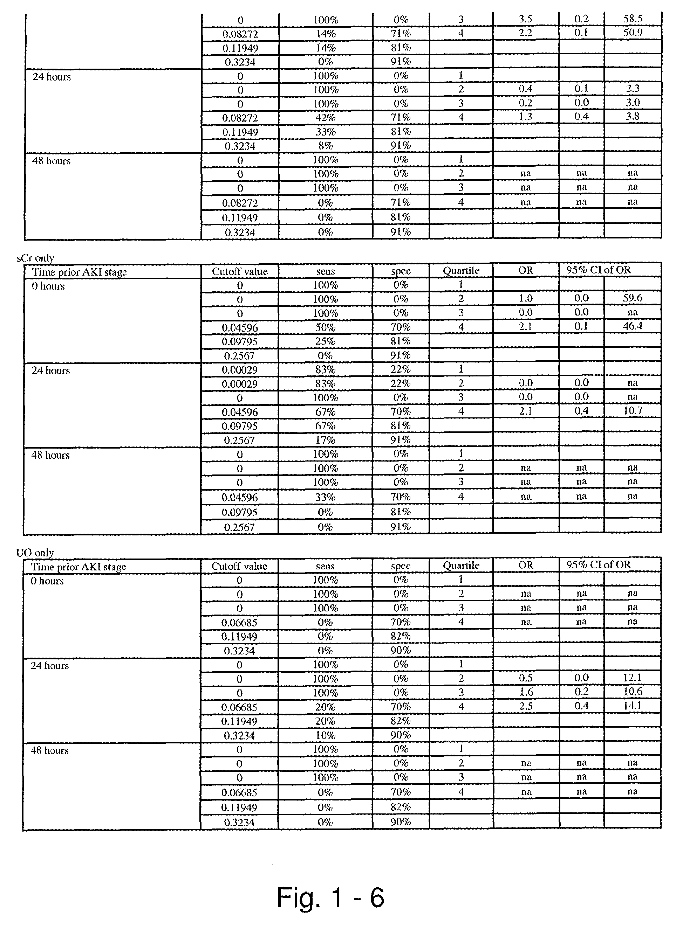

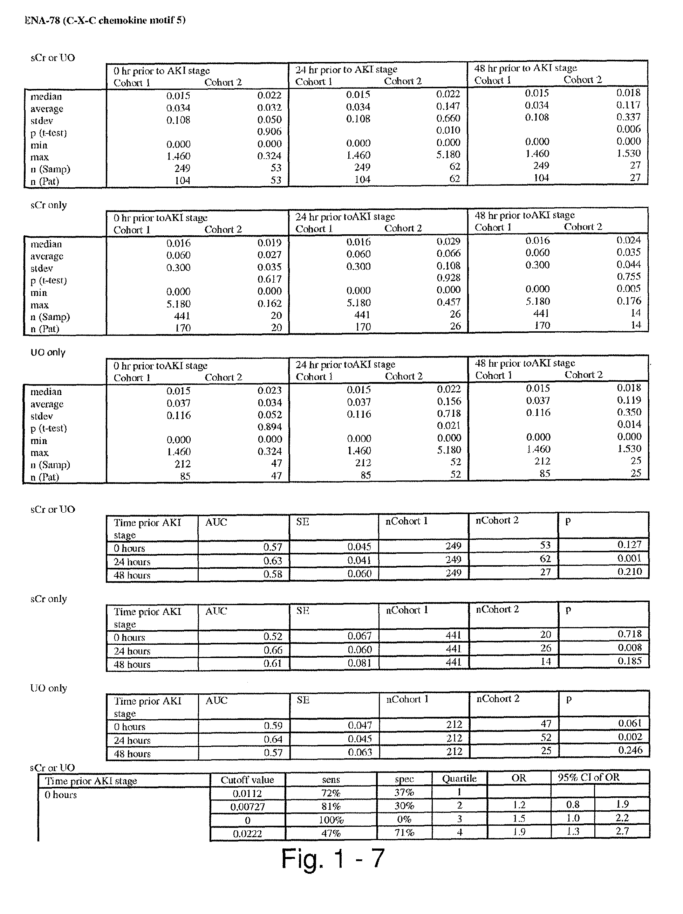

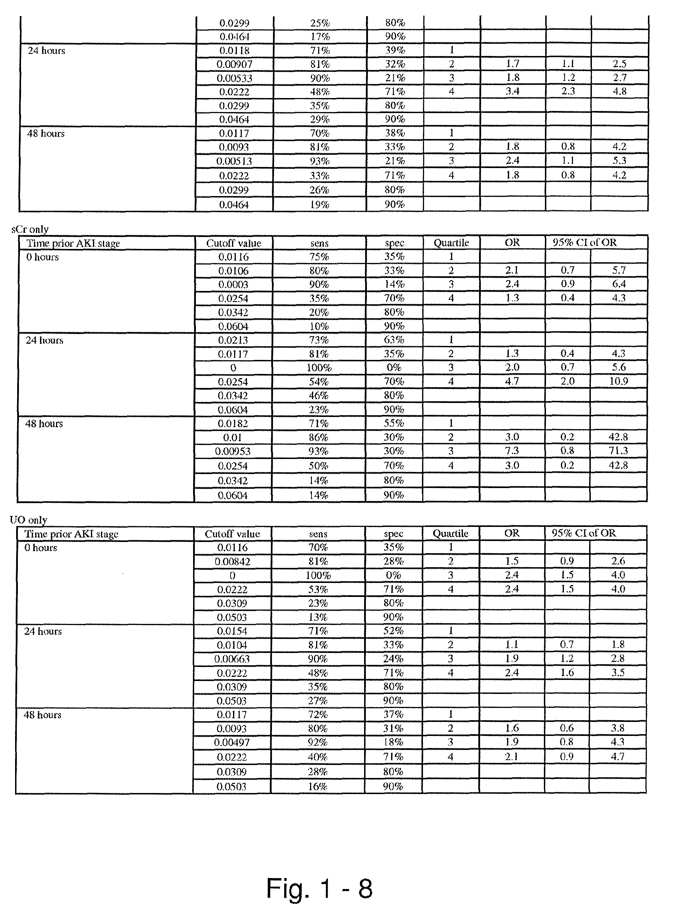

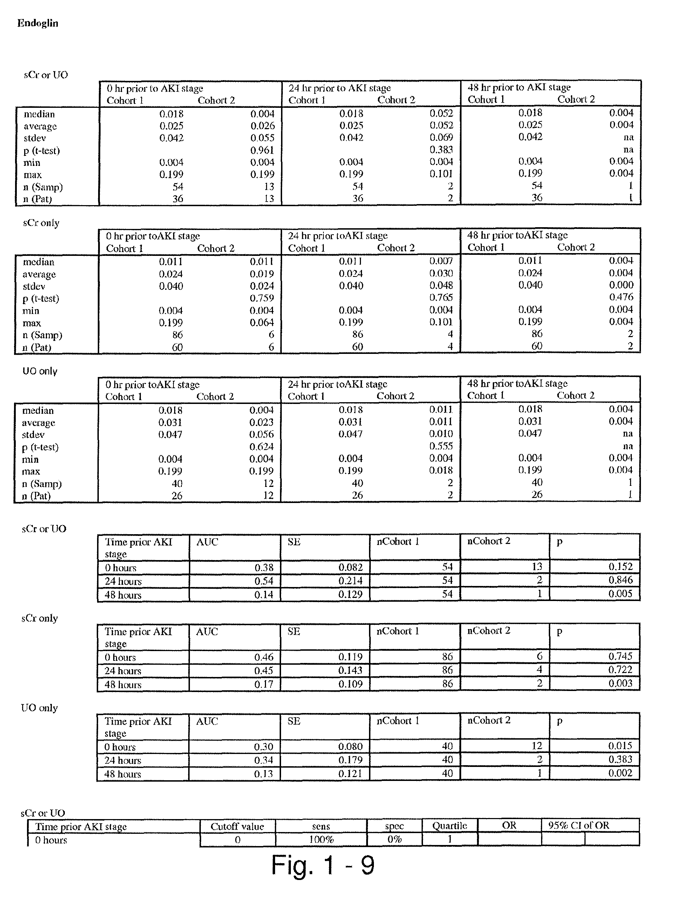

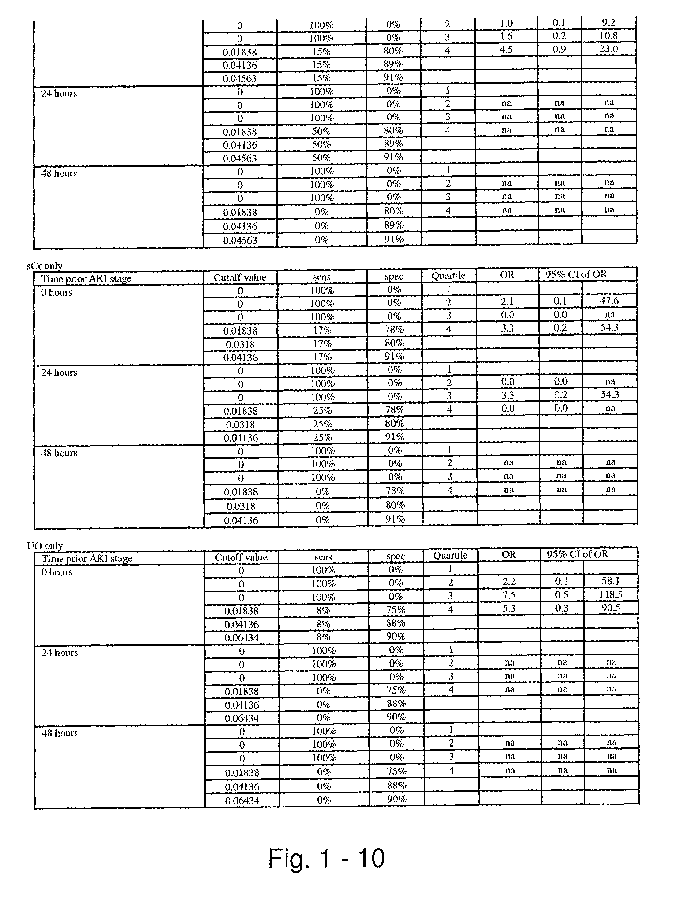

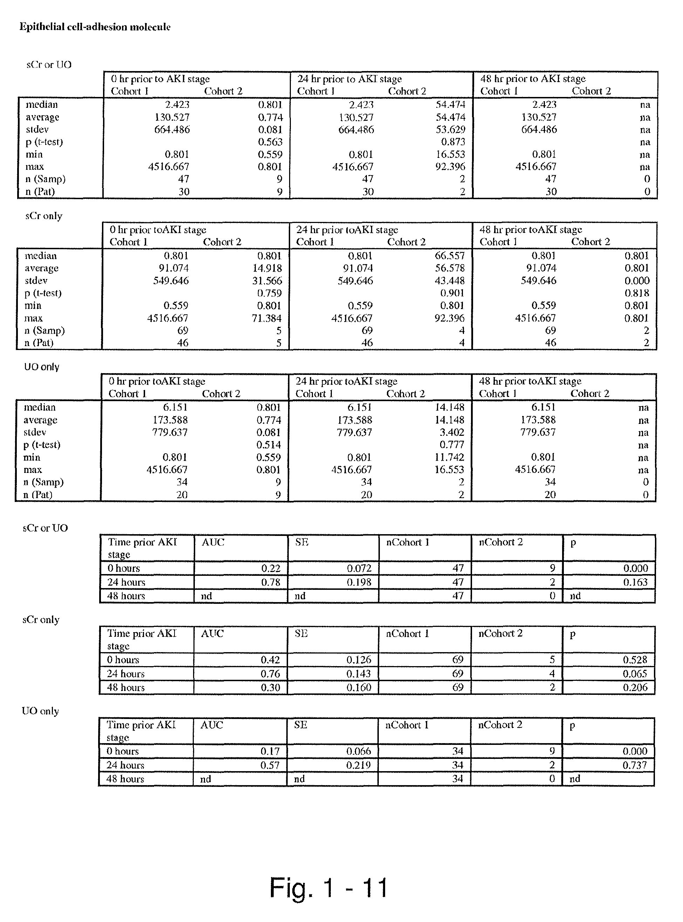

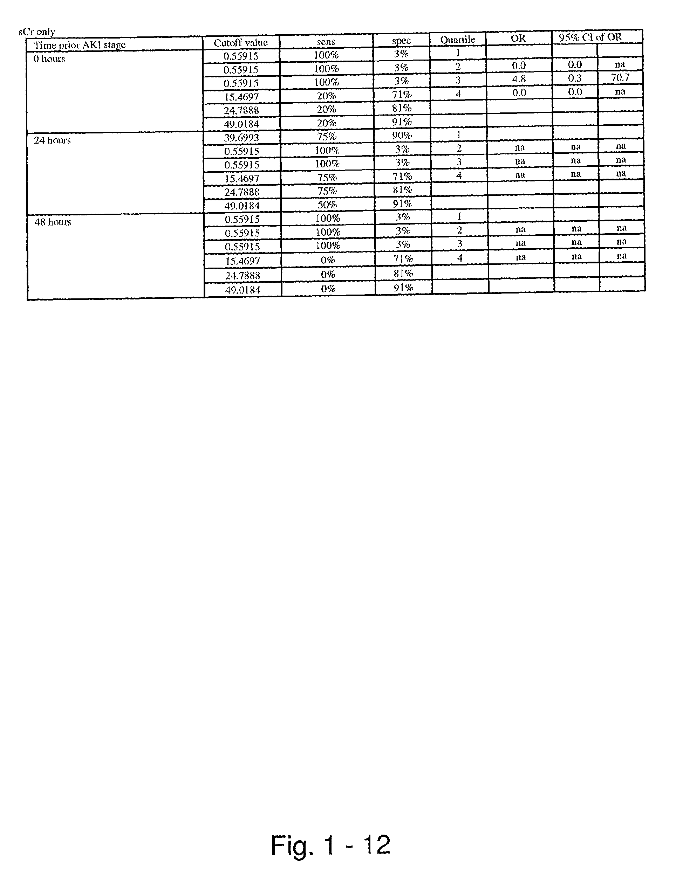

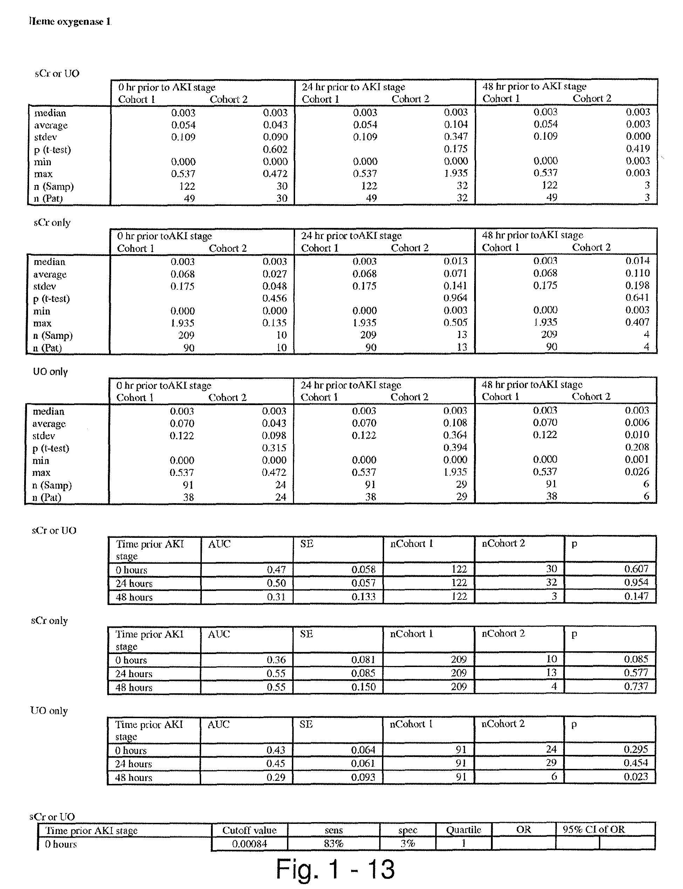

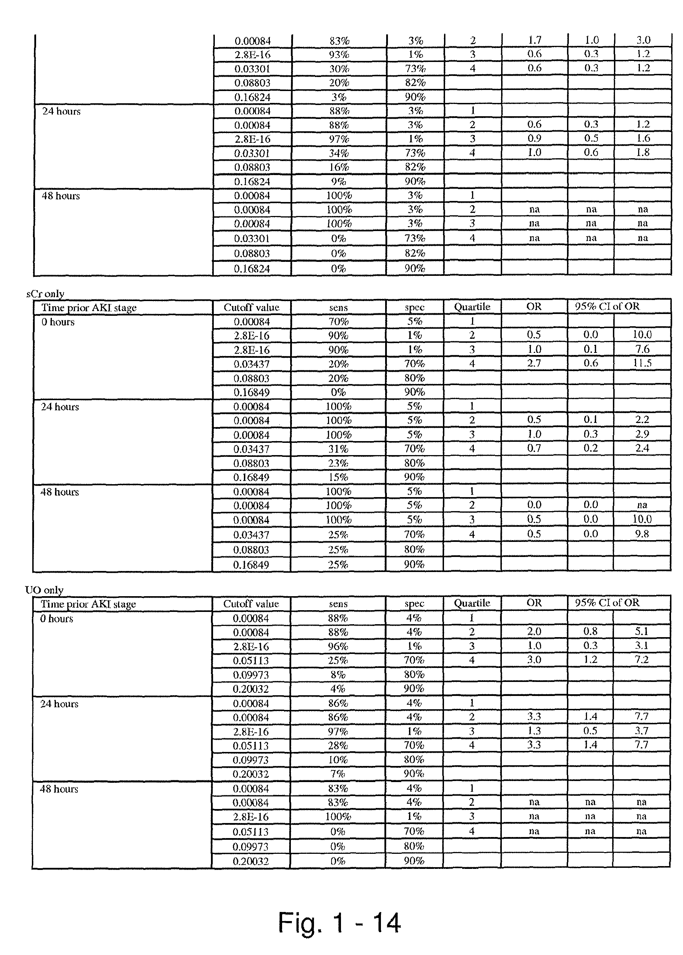

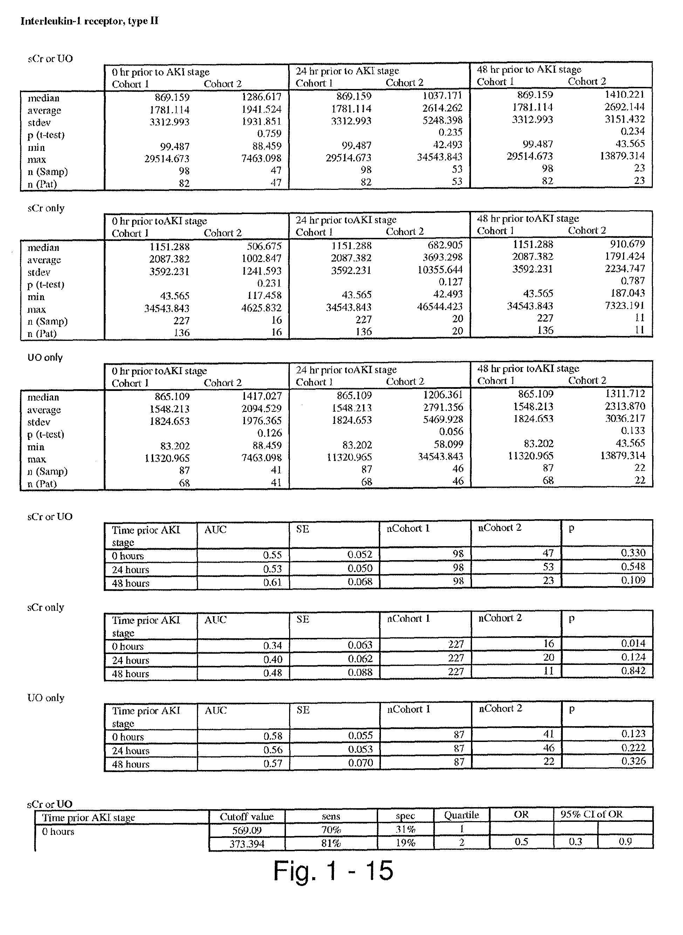

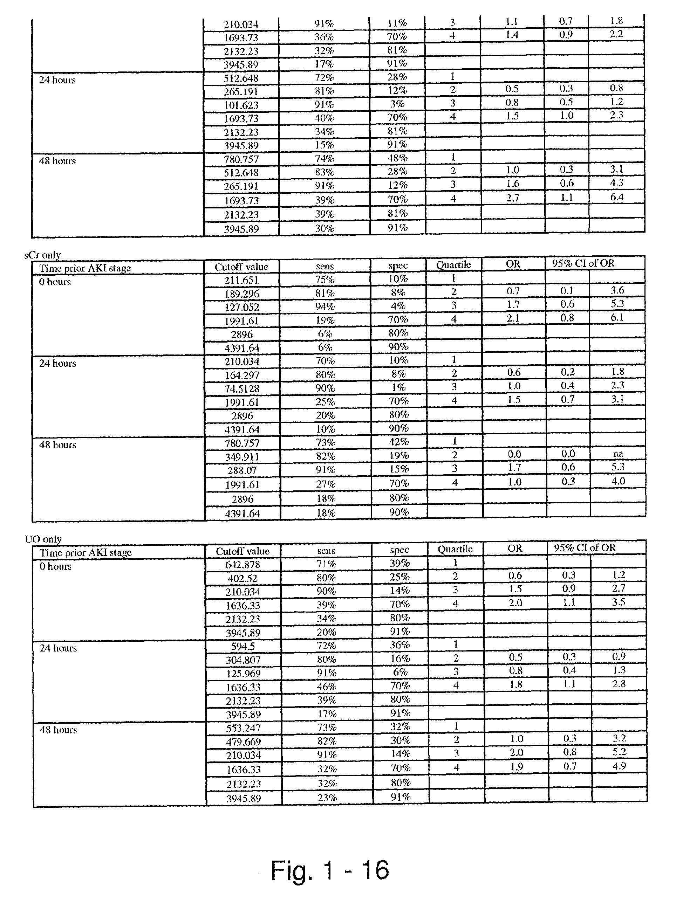

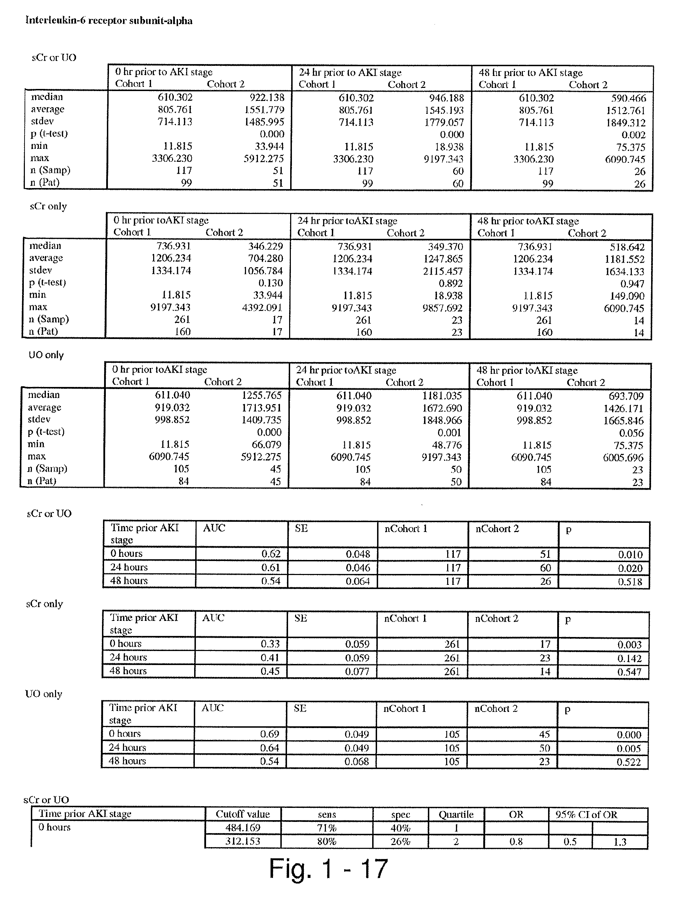

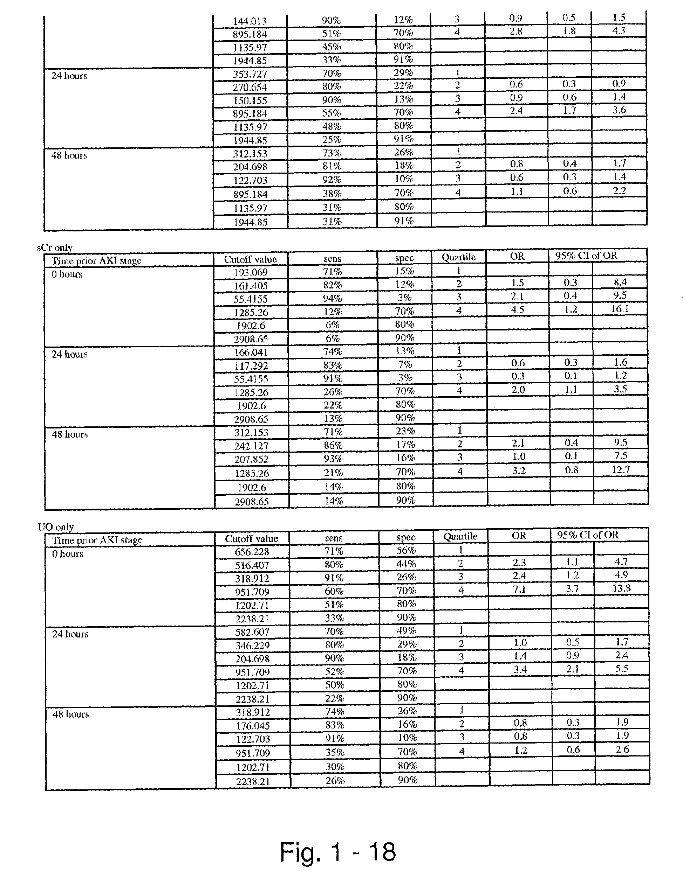

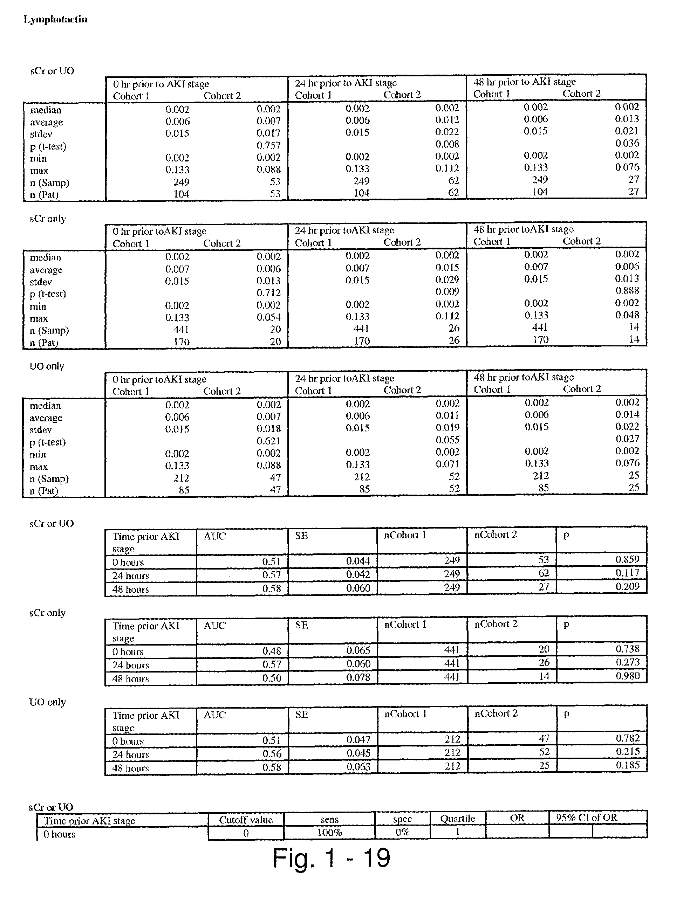

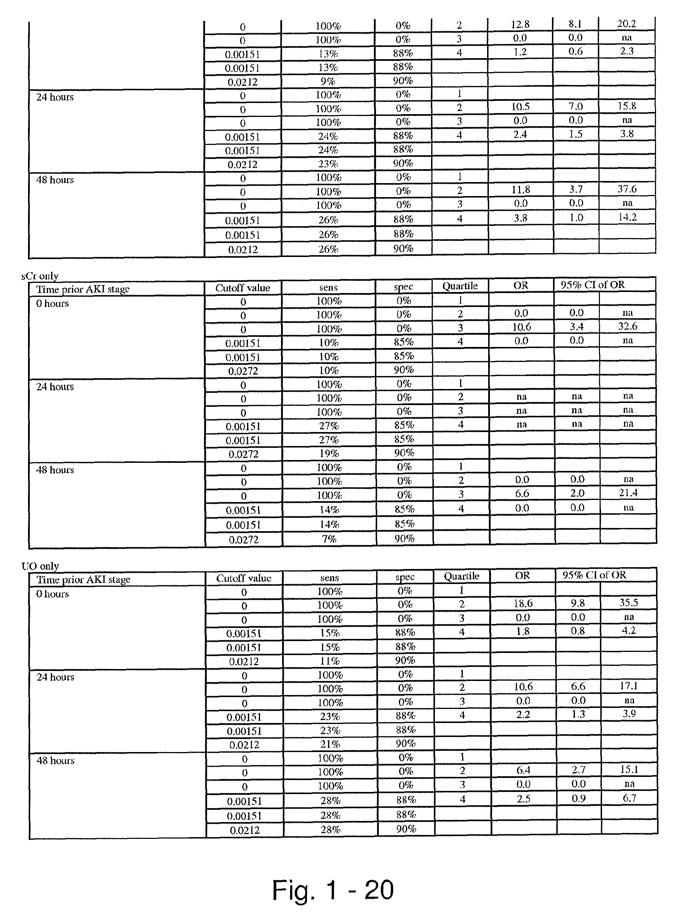

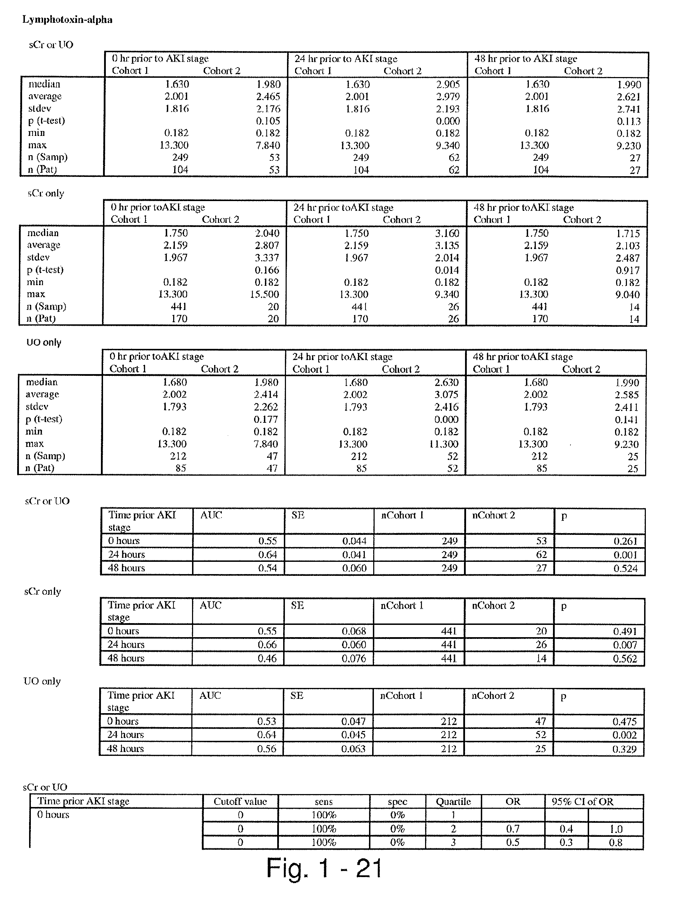

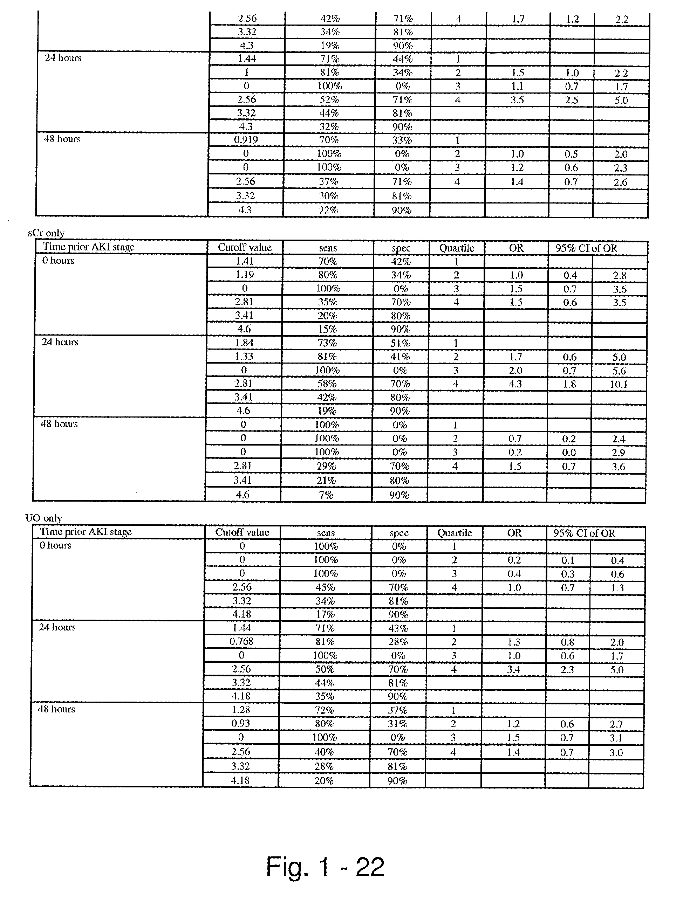

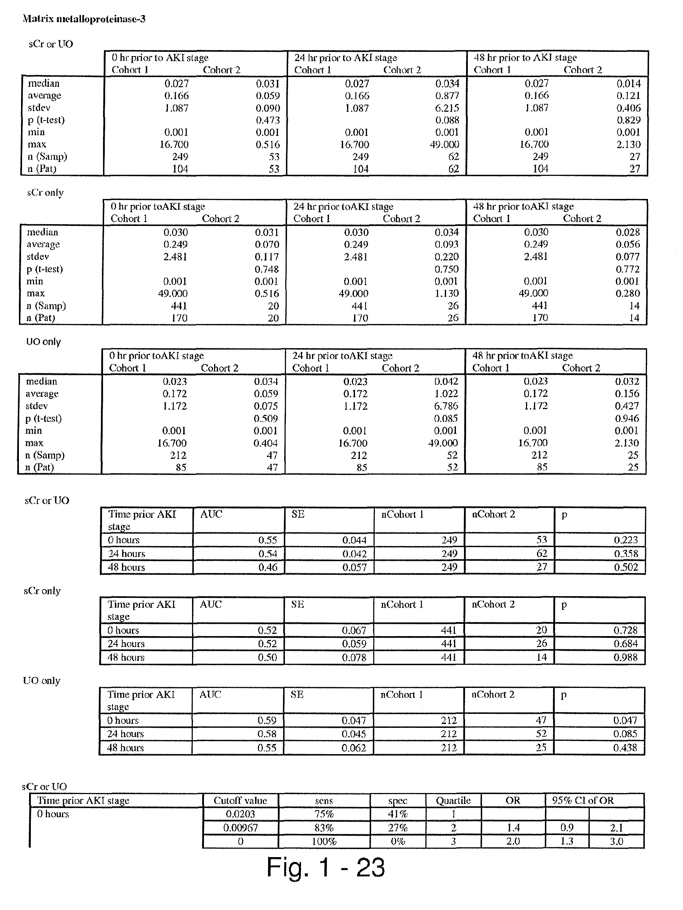

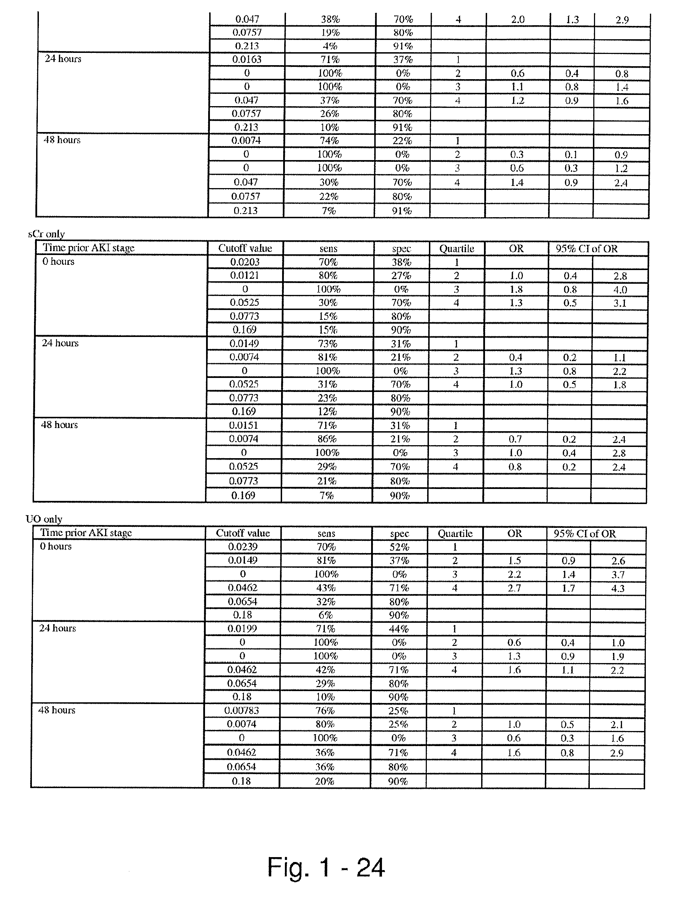

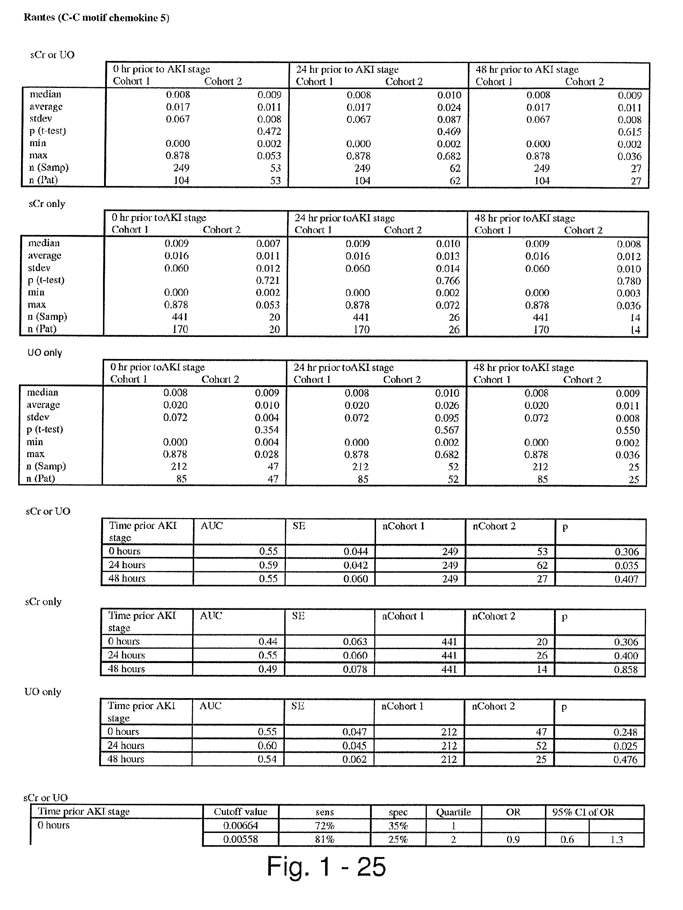

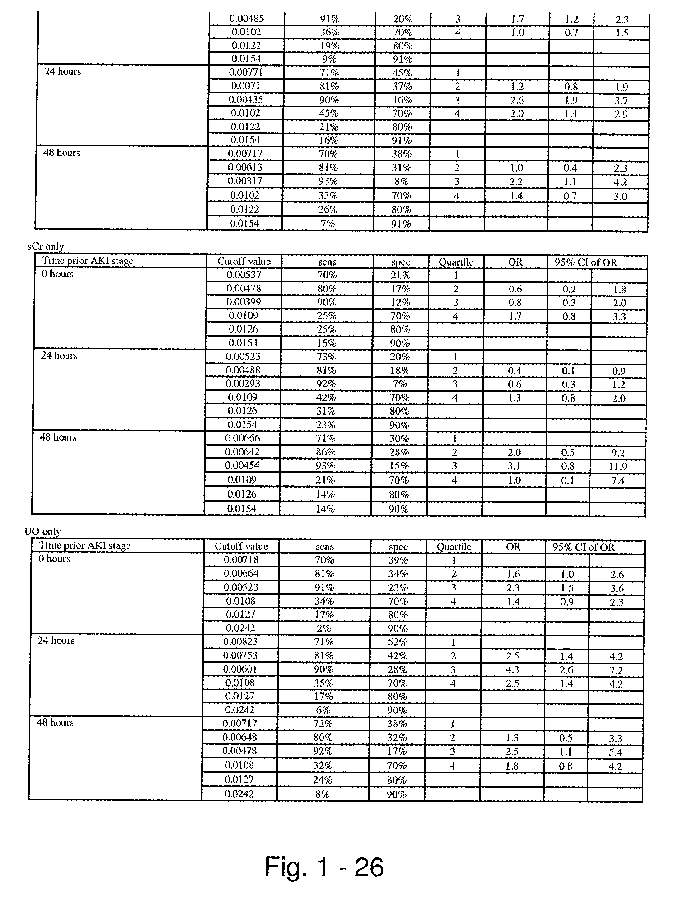

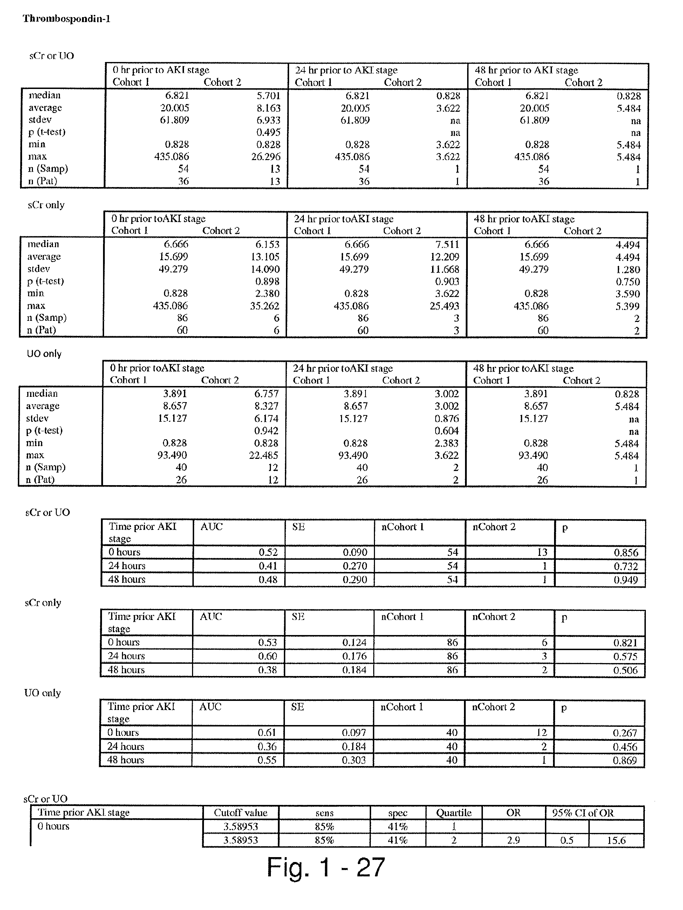

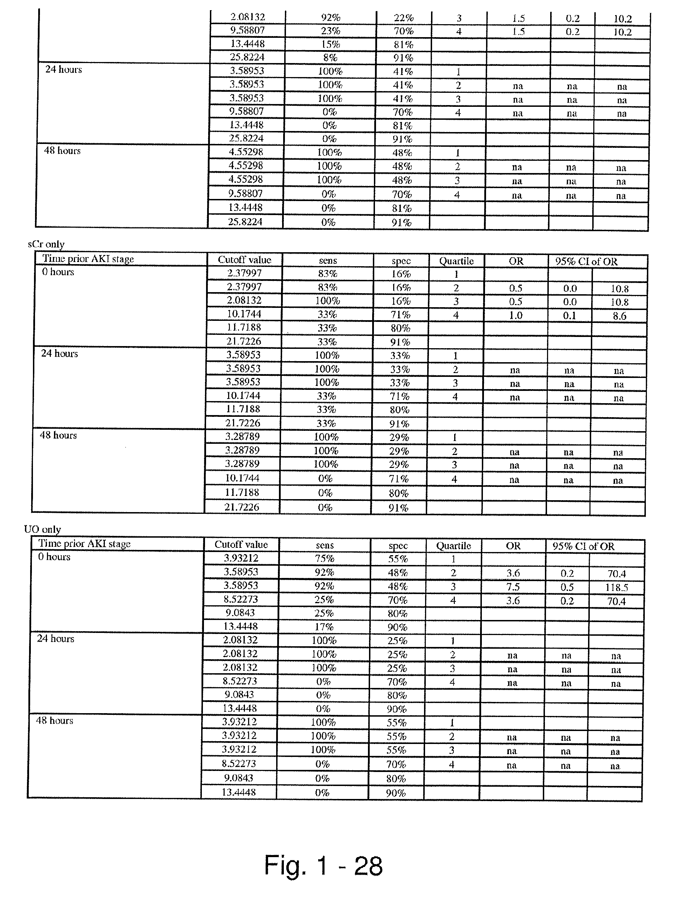

Fig. 1 provides data tables determined in accordance with Example 6 for the comparison of marker levels in urine samples collected for Cohort 1 (patients that did not progress beyond RIFLE stage 0) and in urine samples collected from subjects at 0, 24 hours, and 48 hours prior to reaching stage R, I or F in Cohort 2. Tables provide descriptive statistics, AUC analysis, and sensitivity, specificity and odds ratio calculations at various threshold (cutoff) levels for the various markers.

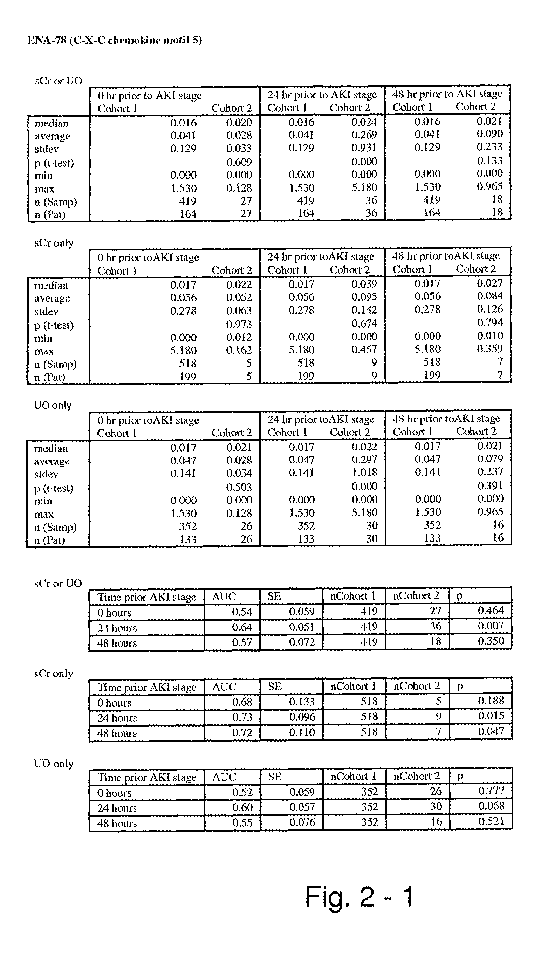

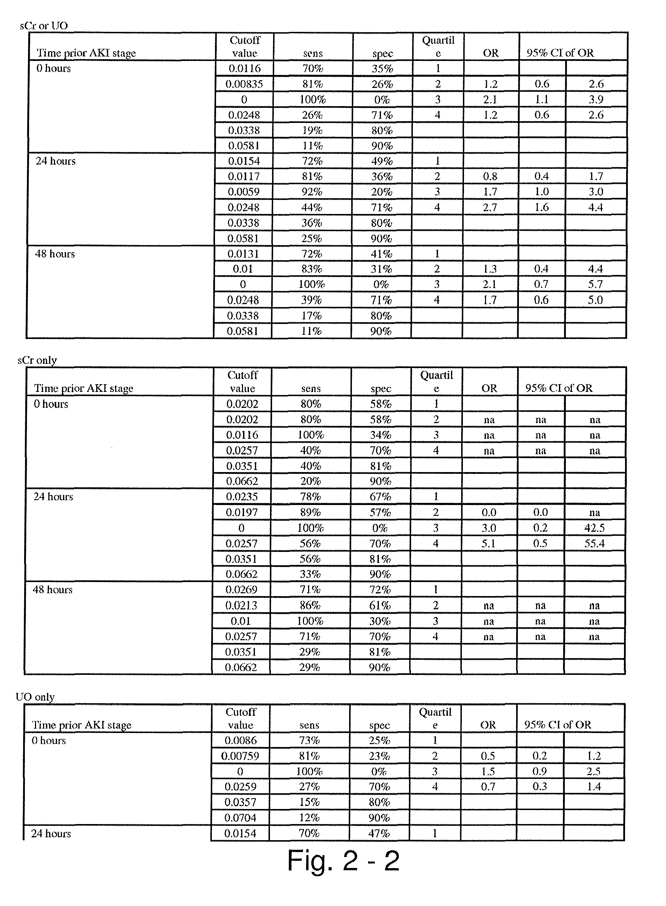

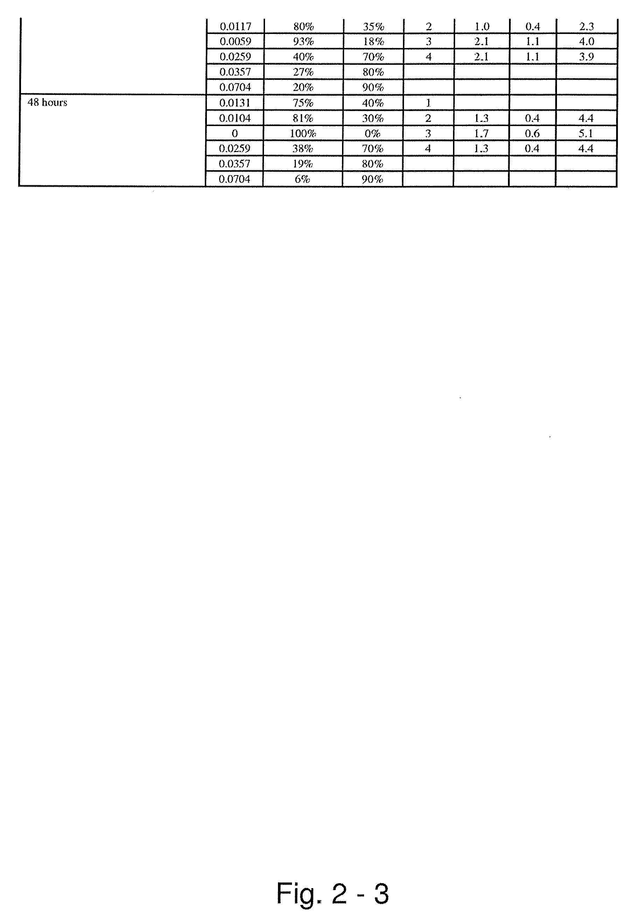

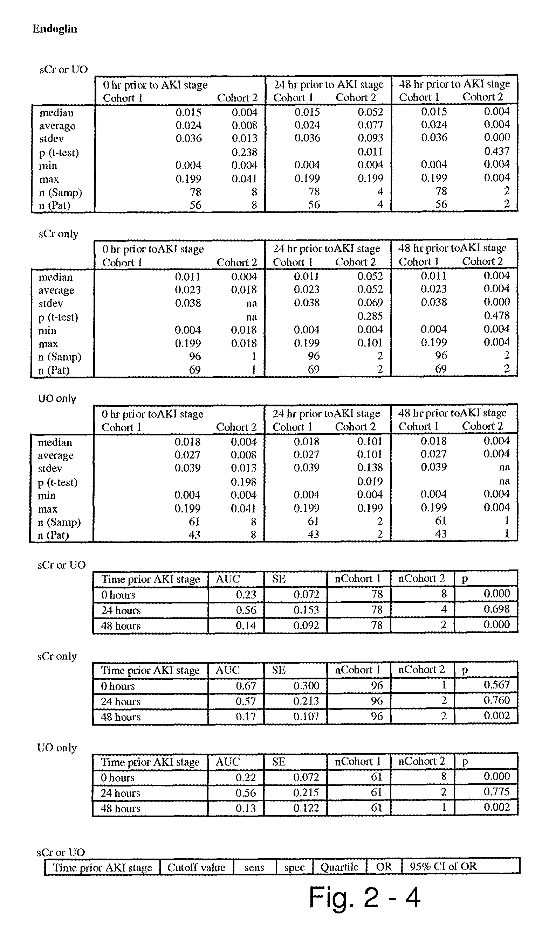

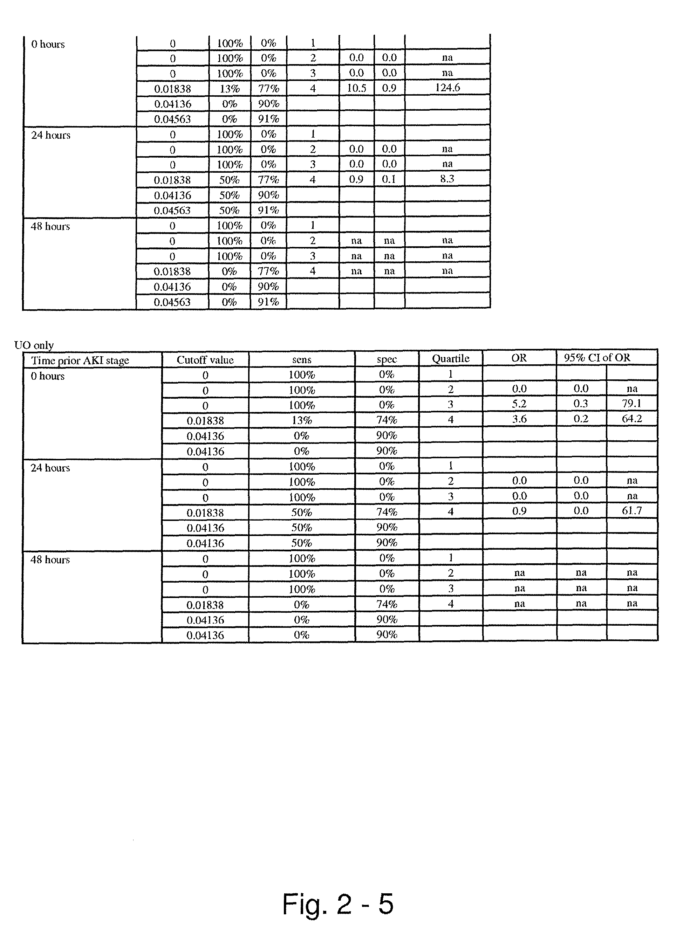

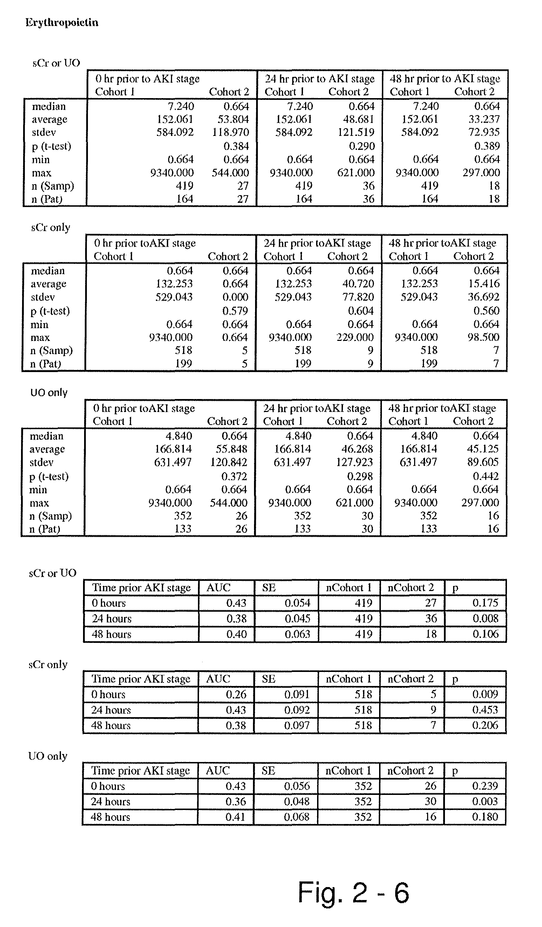

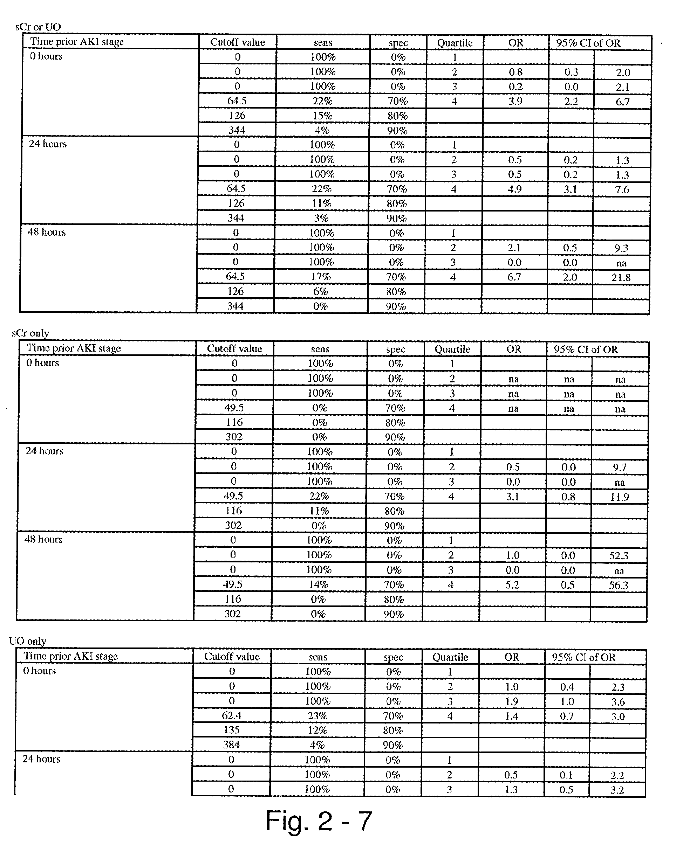

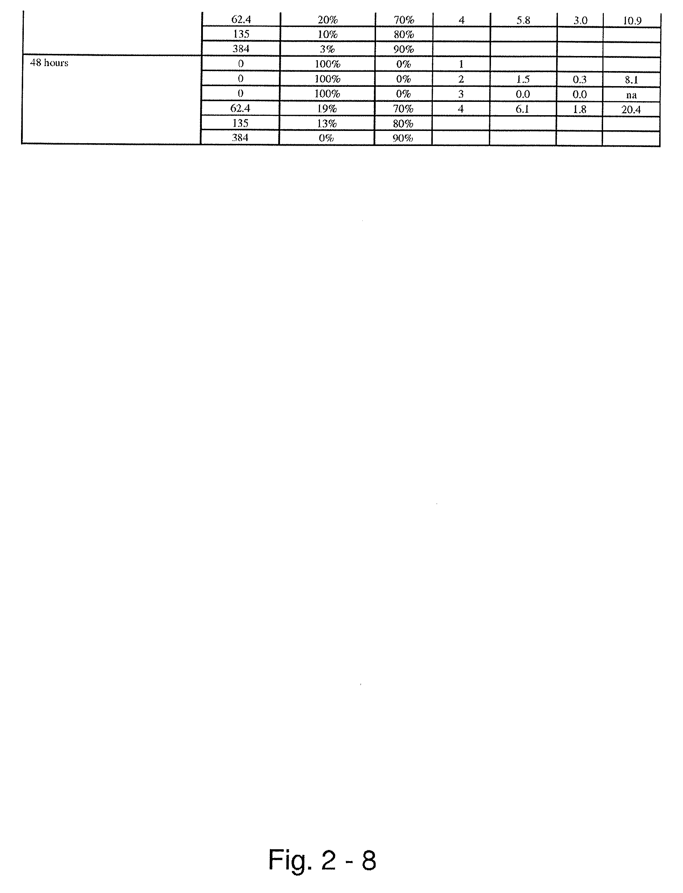

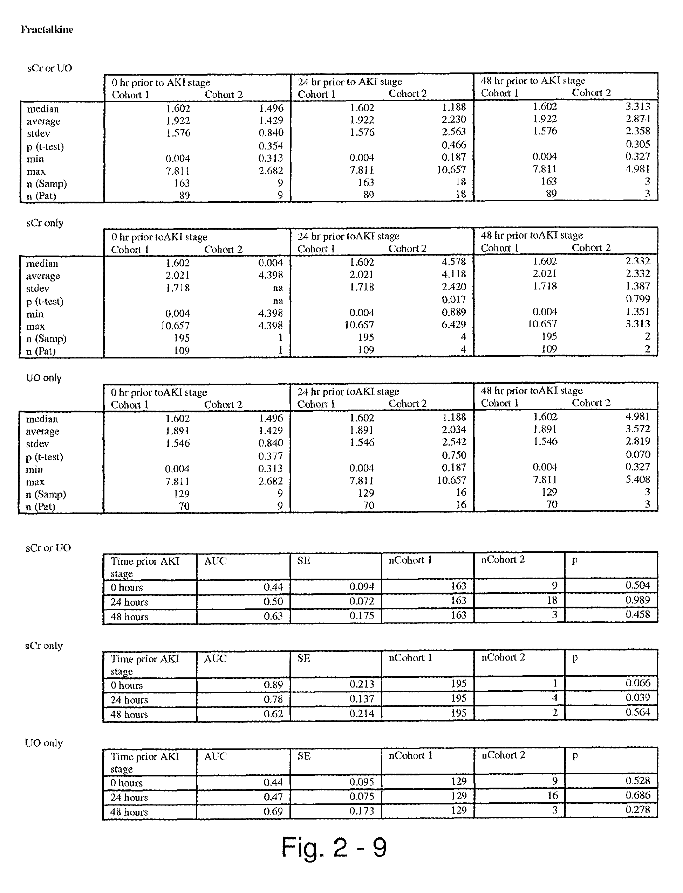

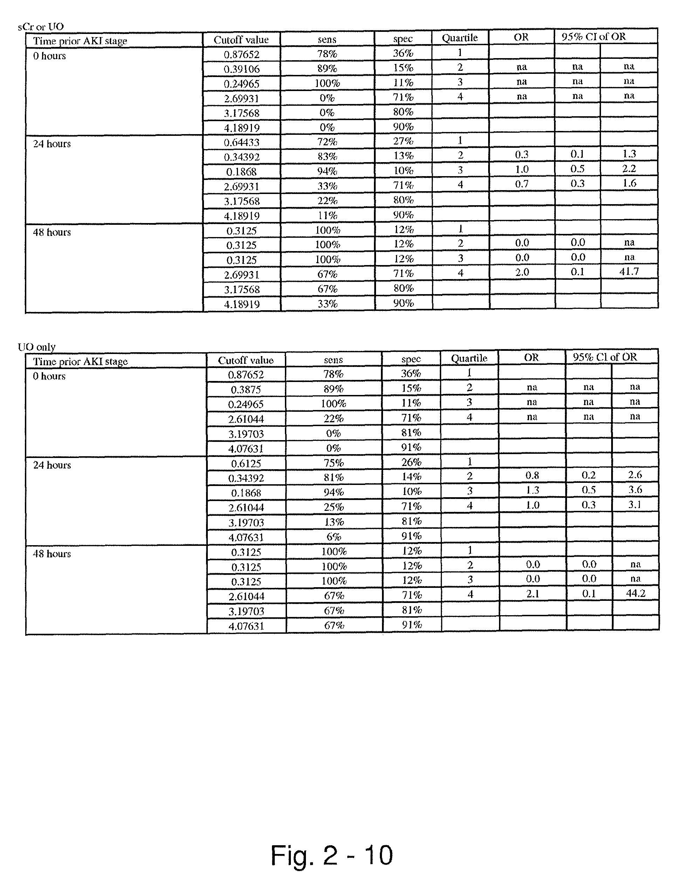

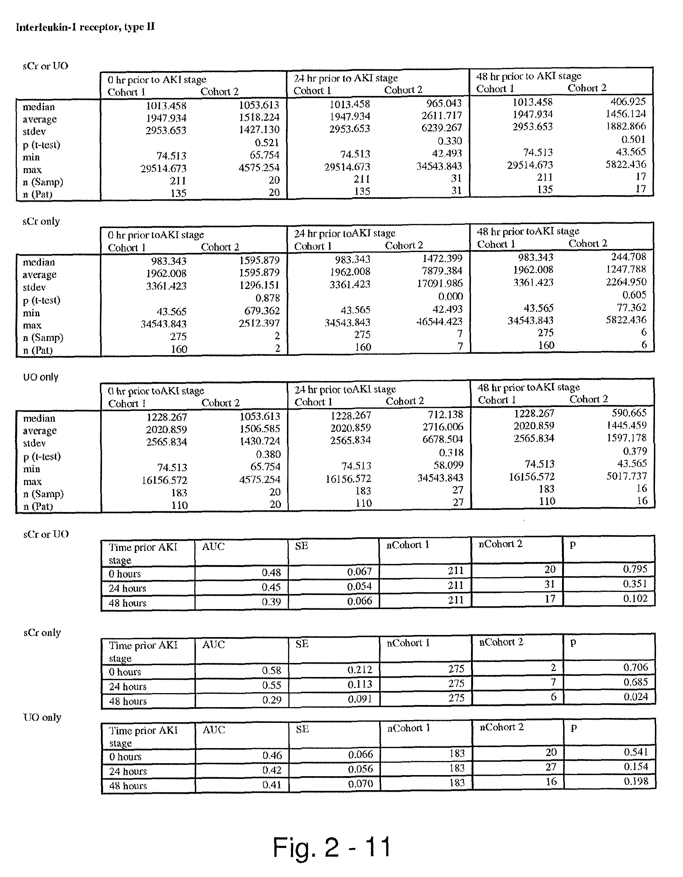

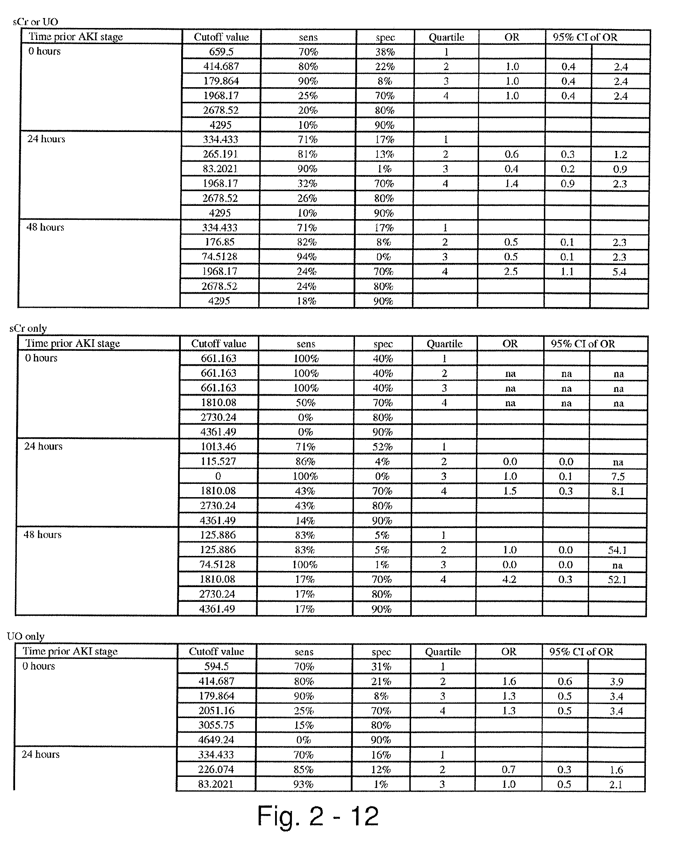

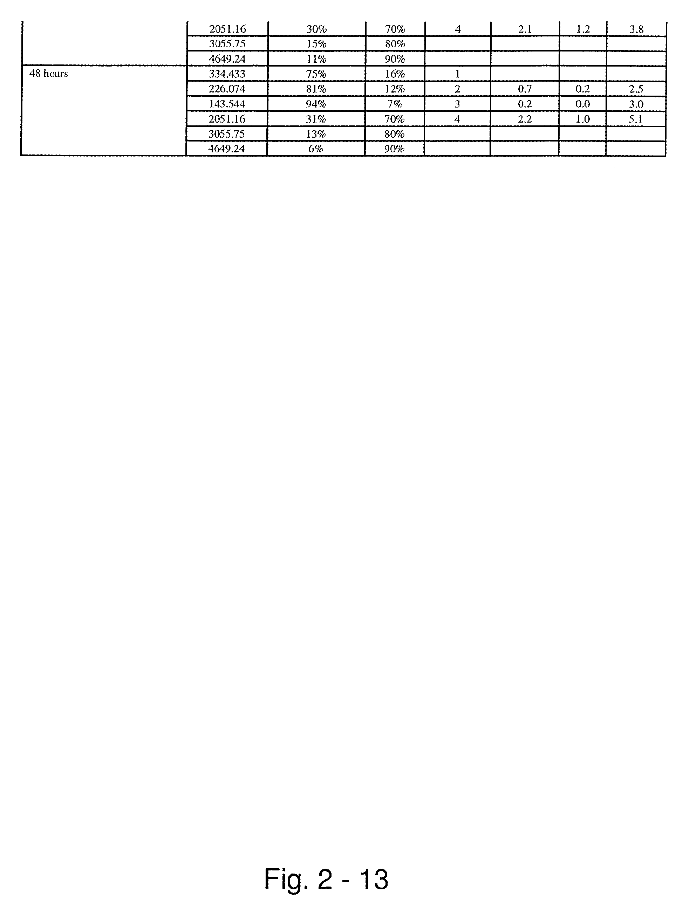

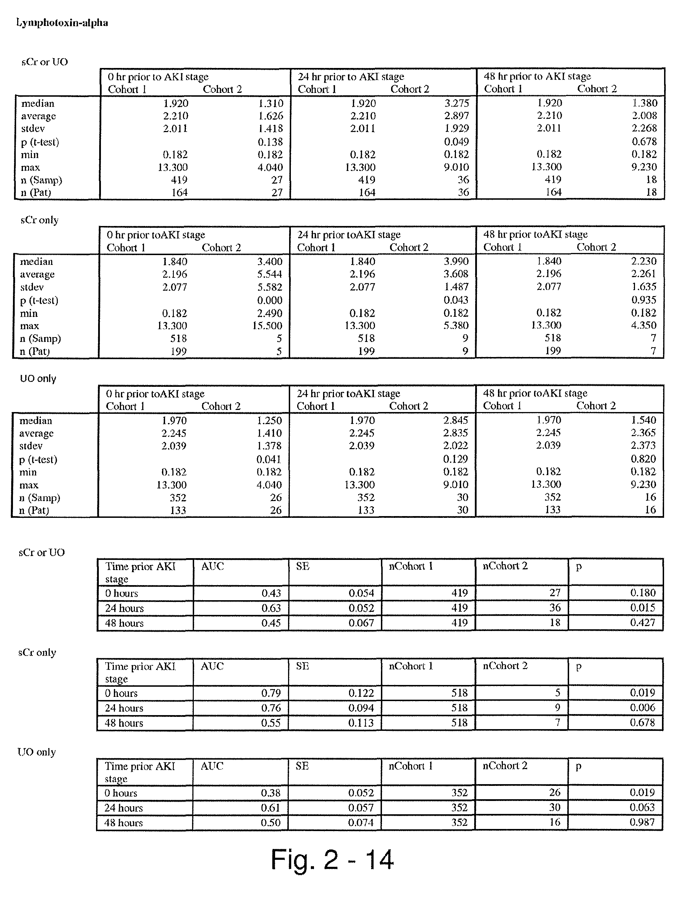

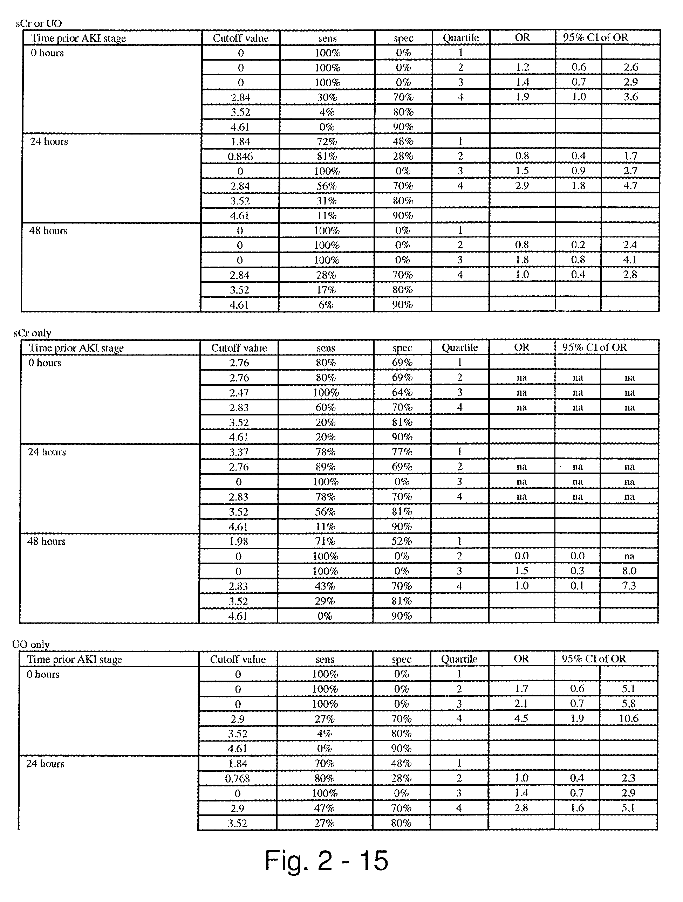

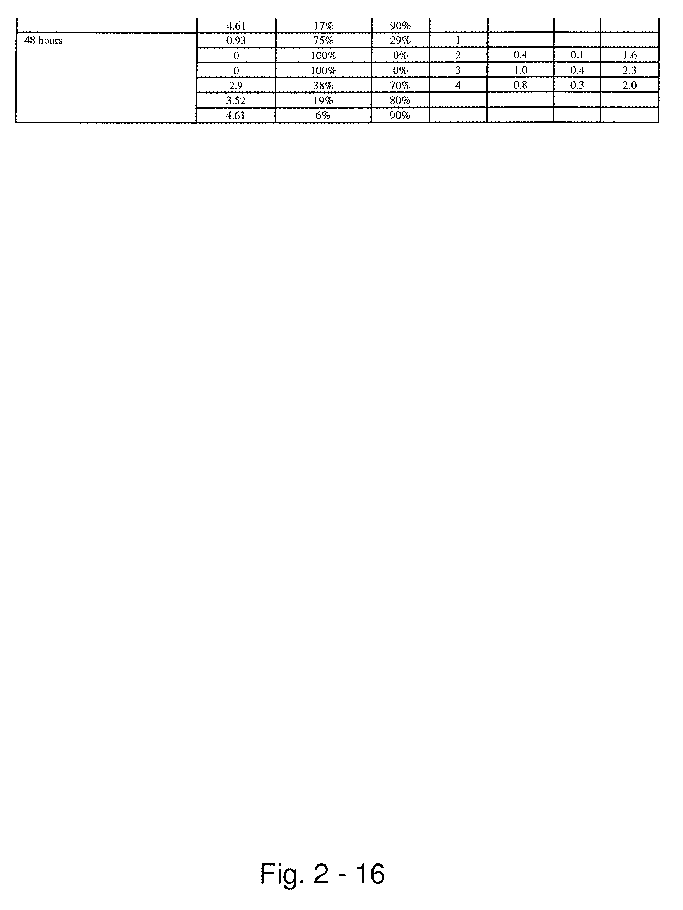

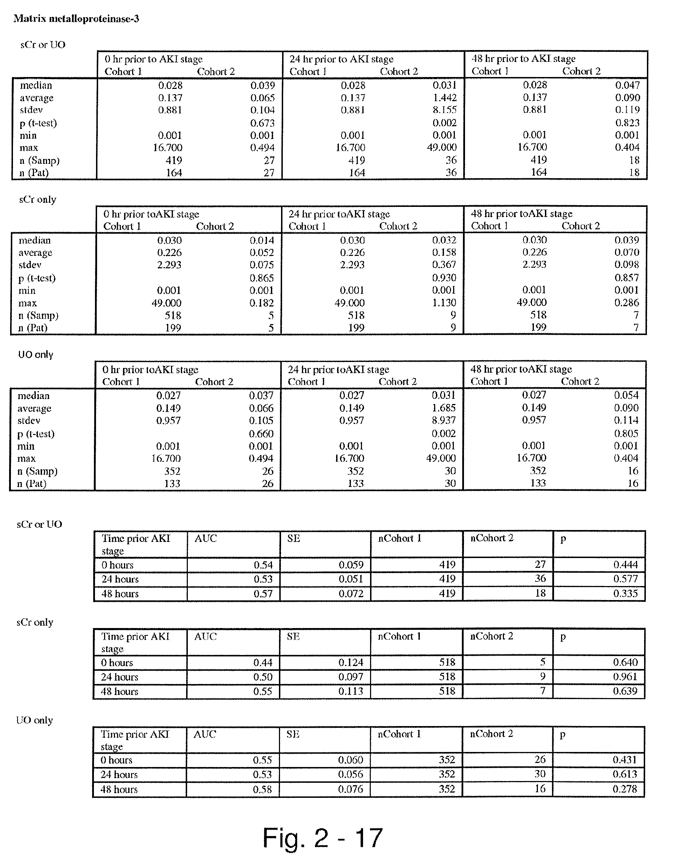

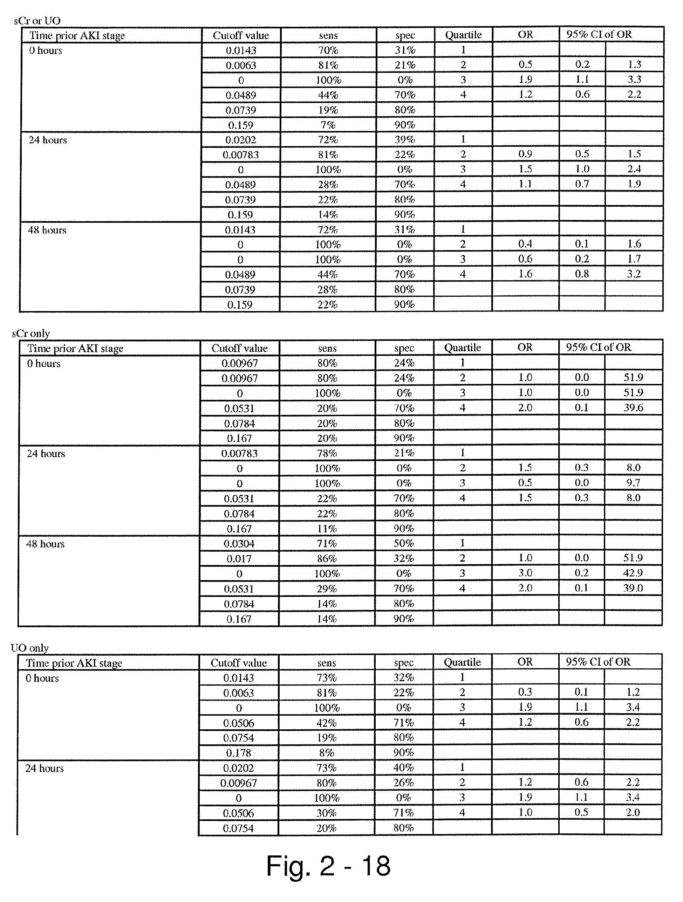

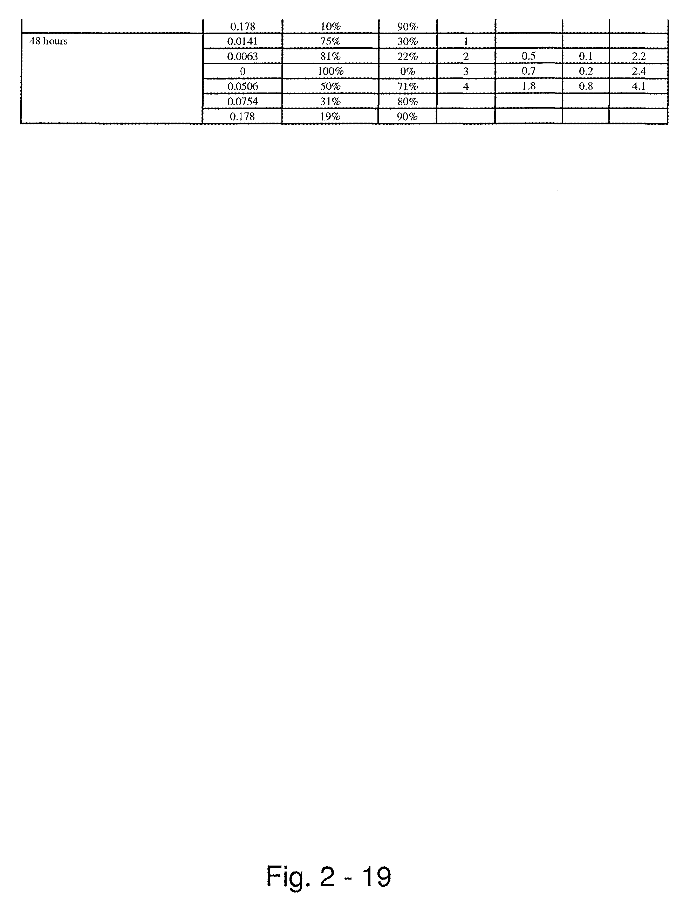

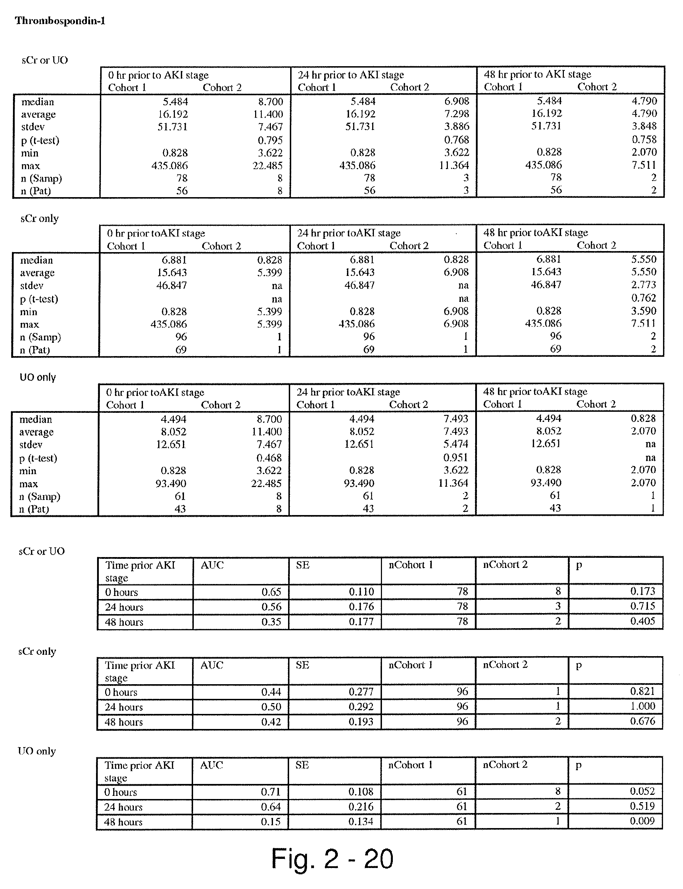

Fig. 2 provides data tables determined in accordance with Example 7 for the comparison of marker levels in urine samples collected for Cohort 1 (patients that did not progress beyond RIFLE stage 0 or R) and in urine samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

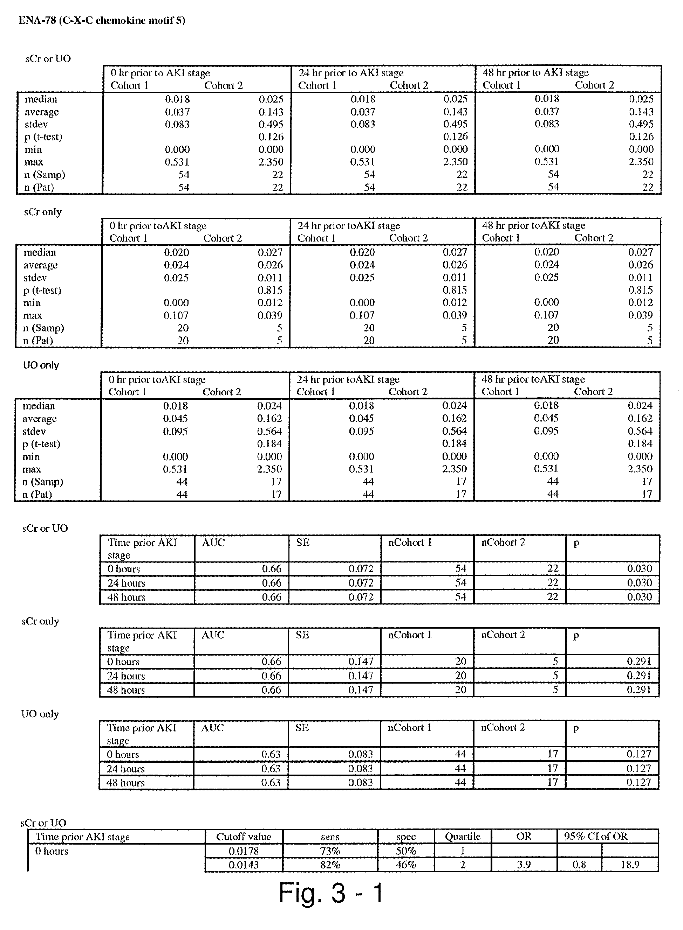

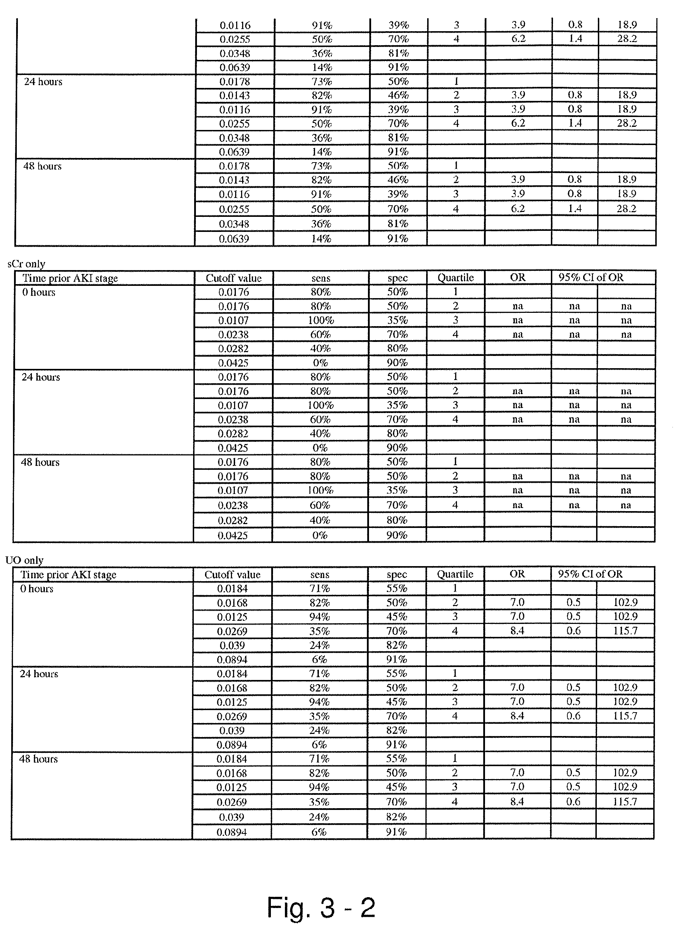

Fig. 3 provides data tables determined in accordance with Example 8 for the comparison of marker levels in urine samples collected for Cohort 1 (patients that reached, but did not progress beyond, RIFLE stage R) and in urine samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

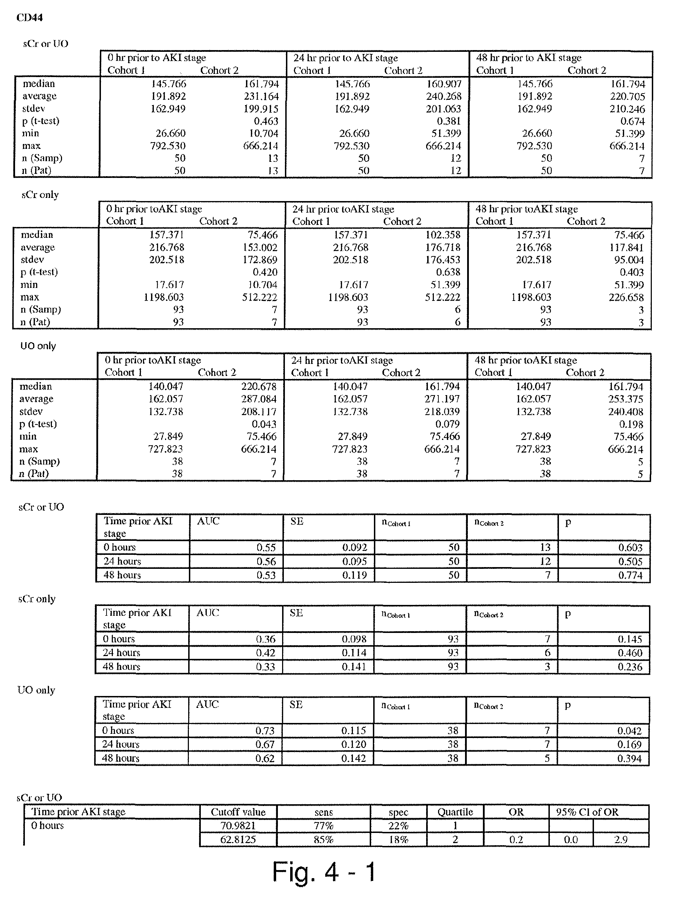

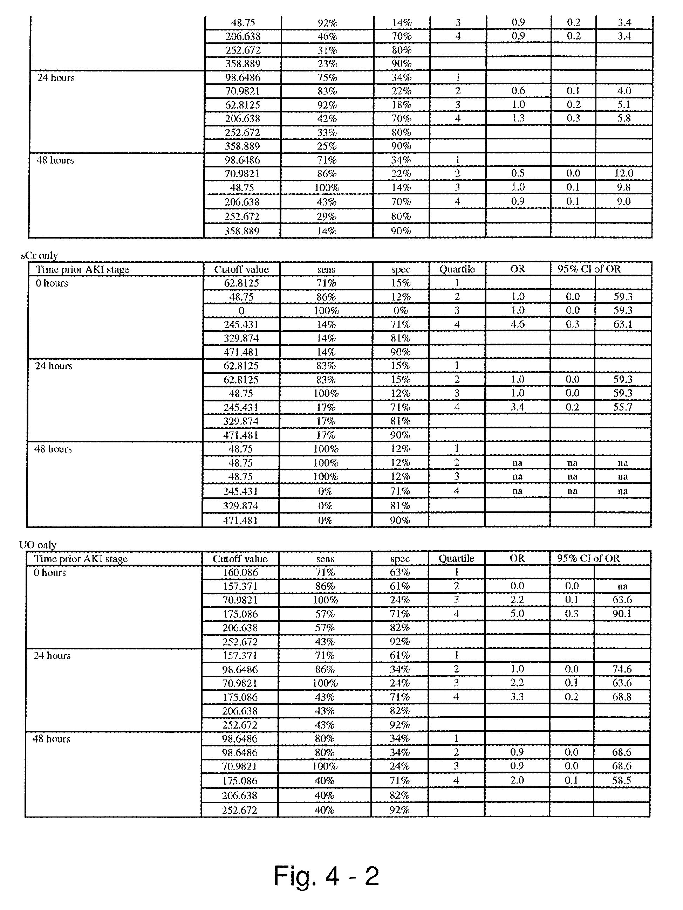

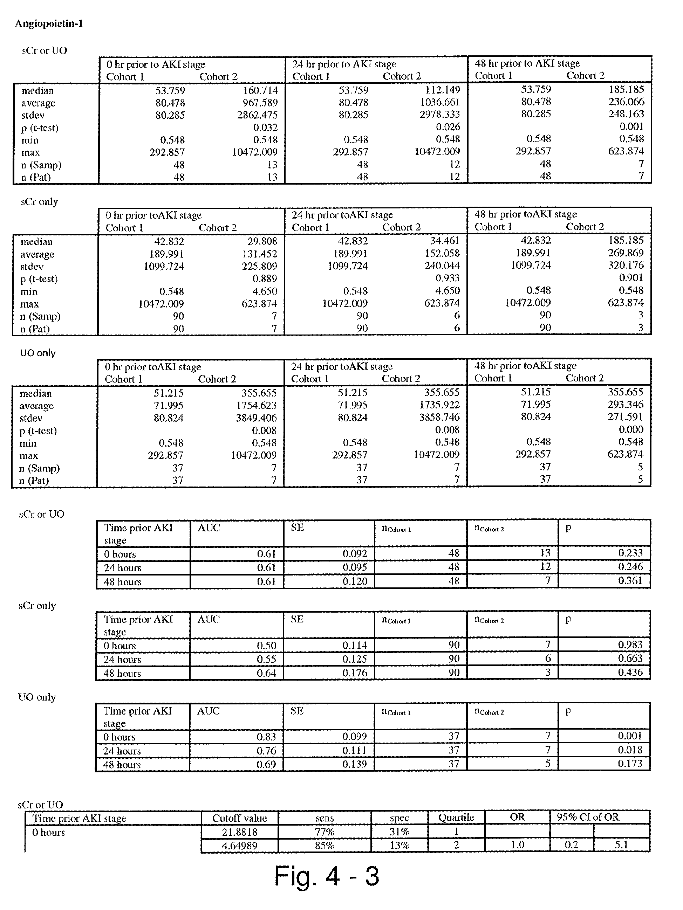

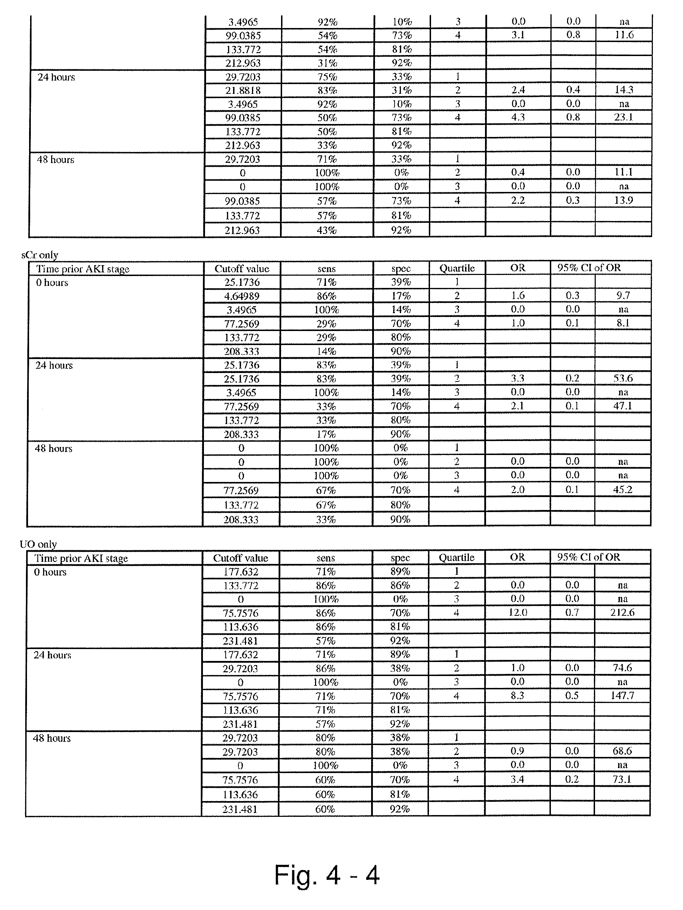

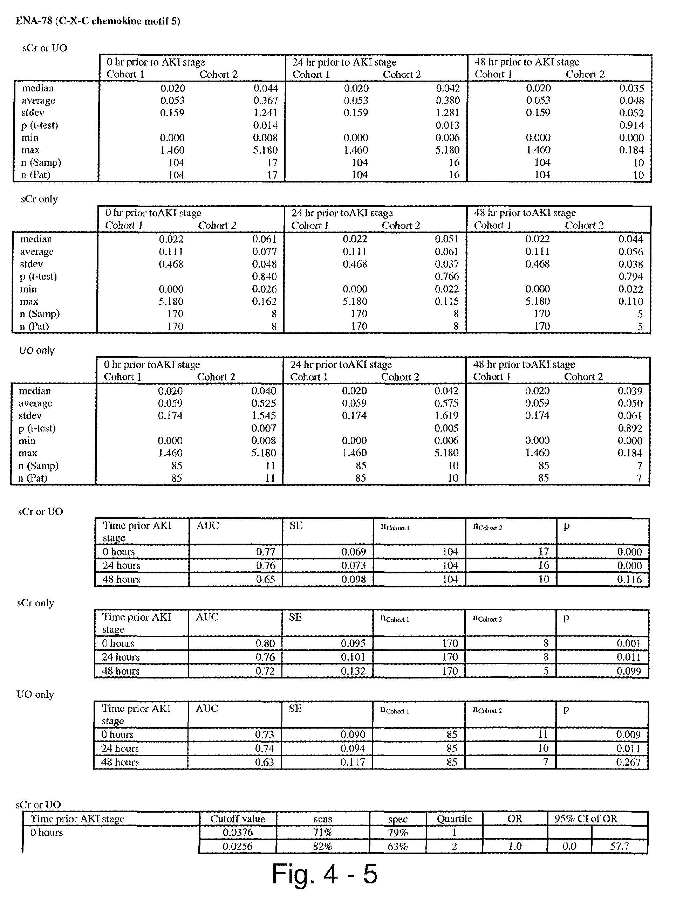

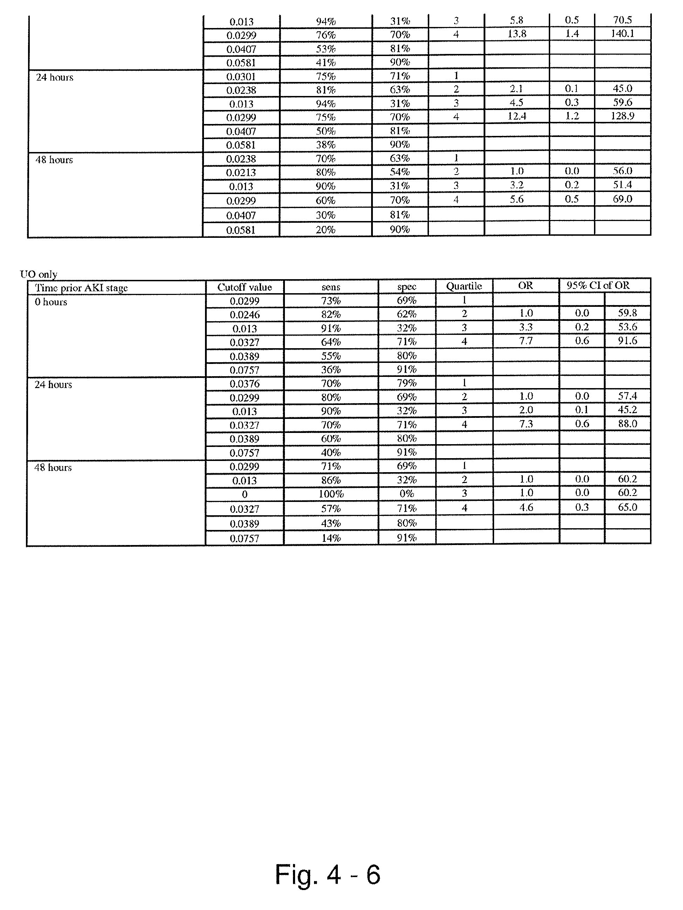

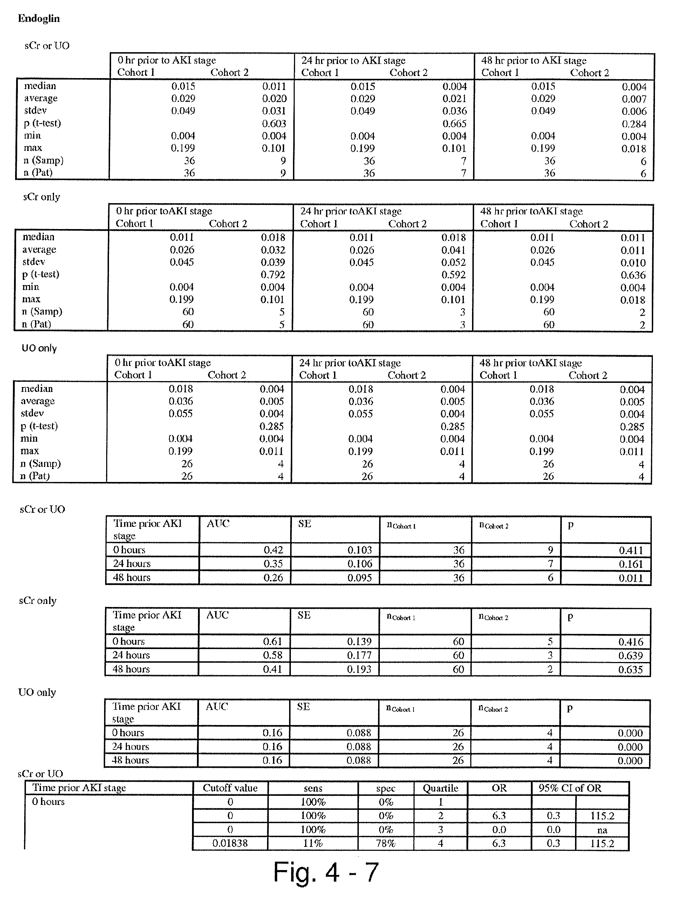

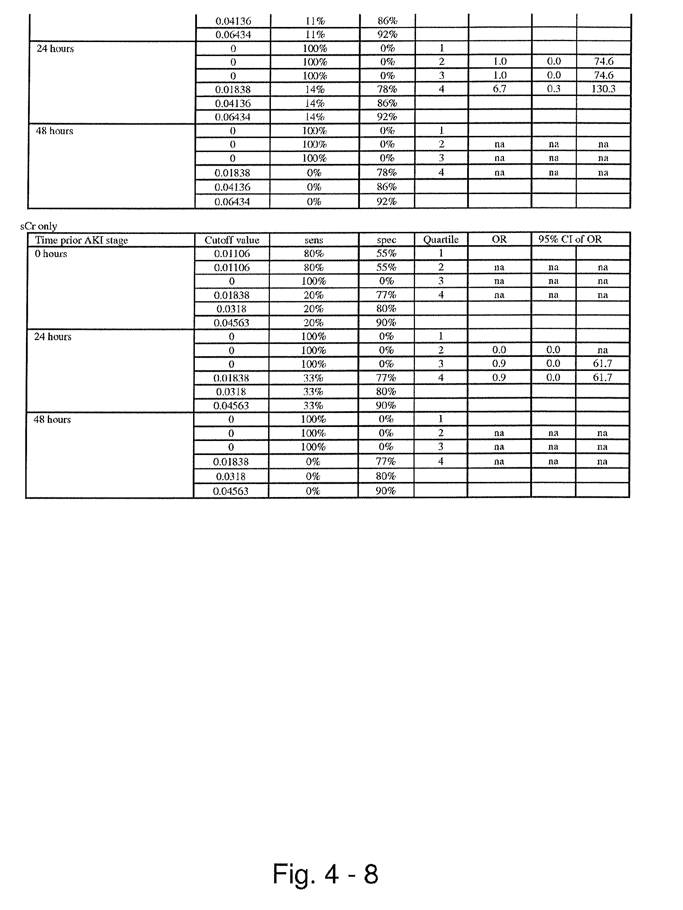

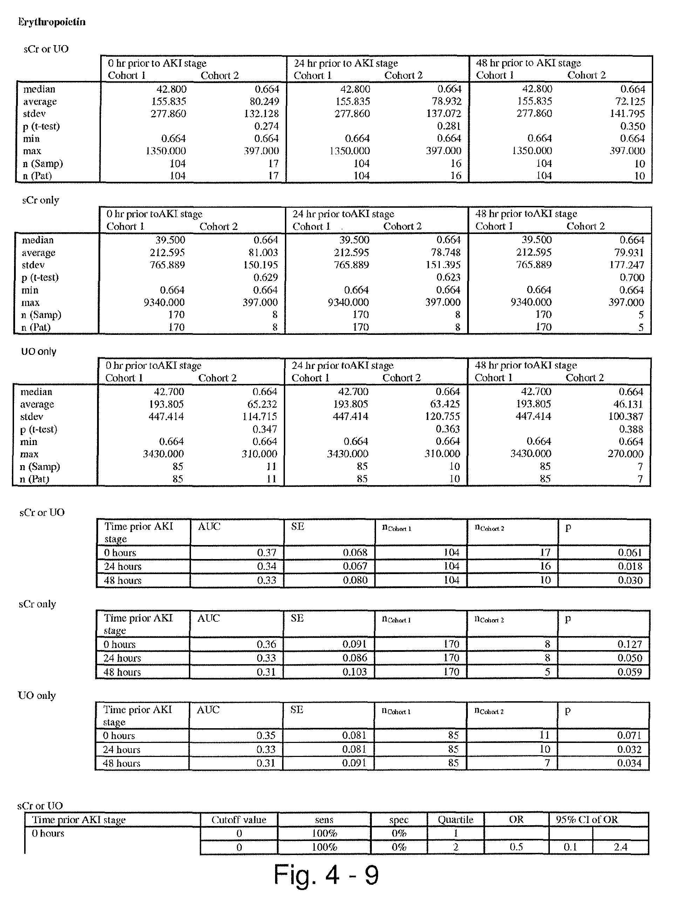

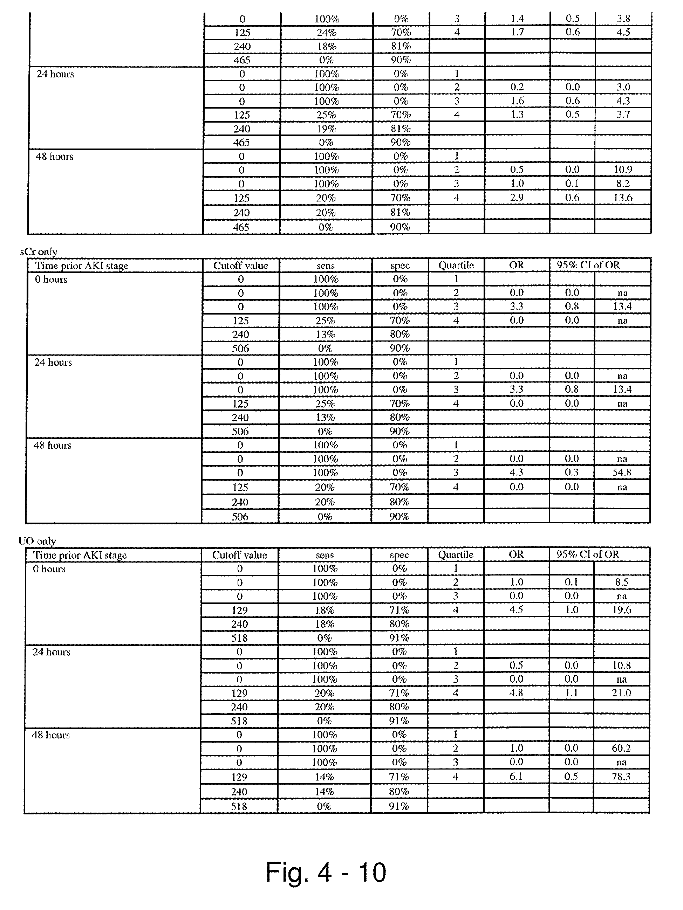

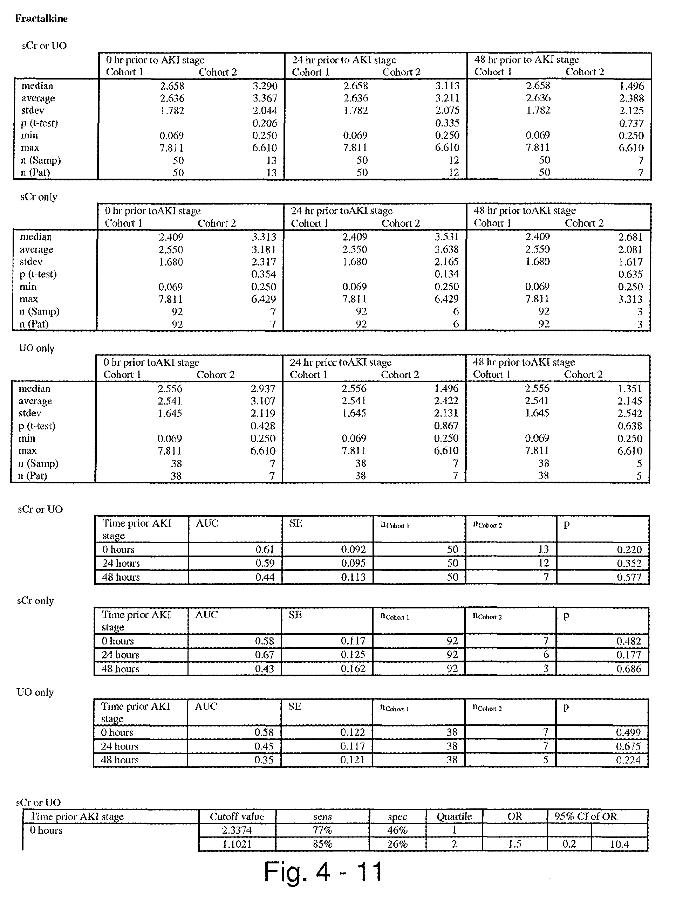

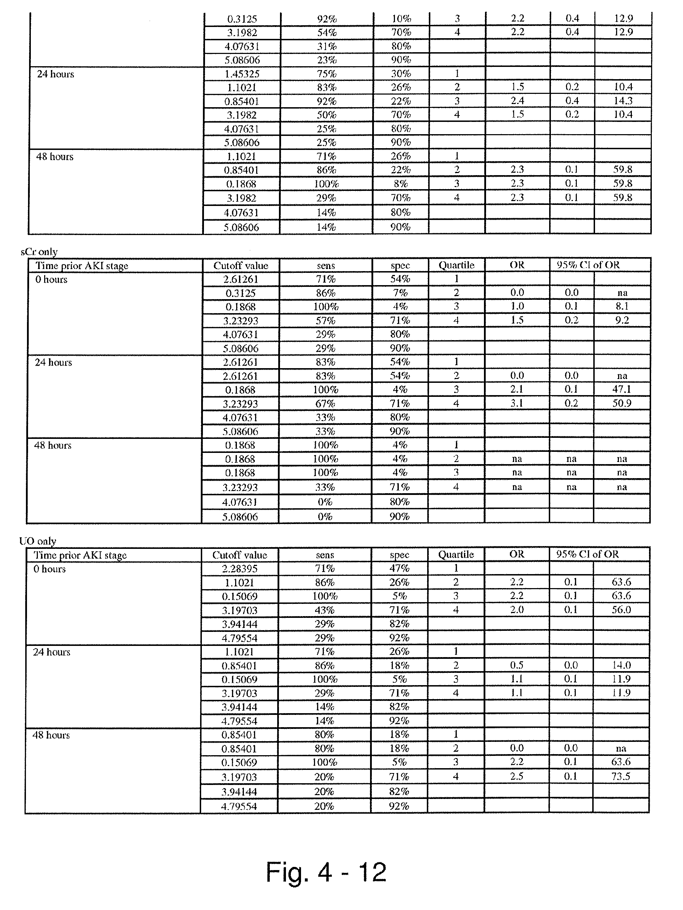

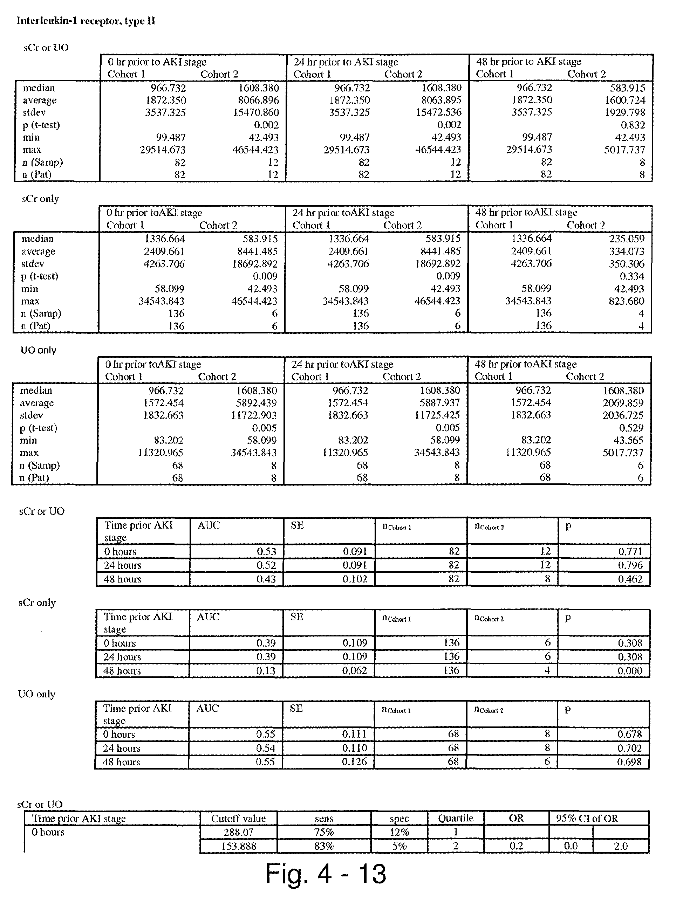

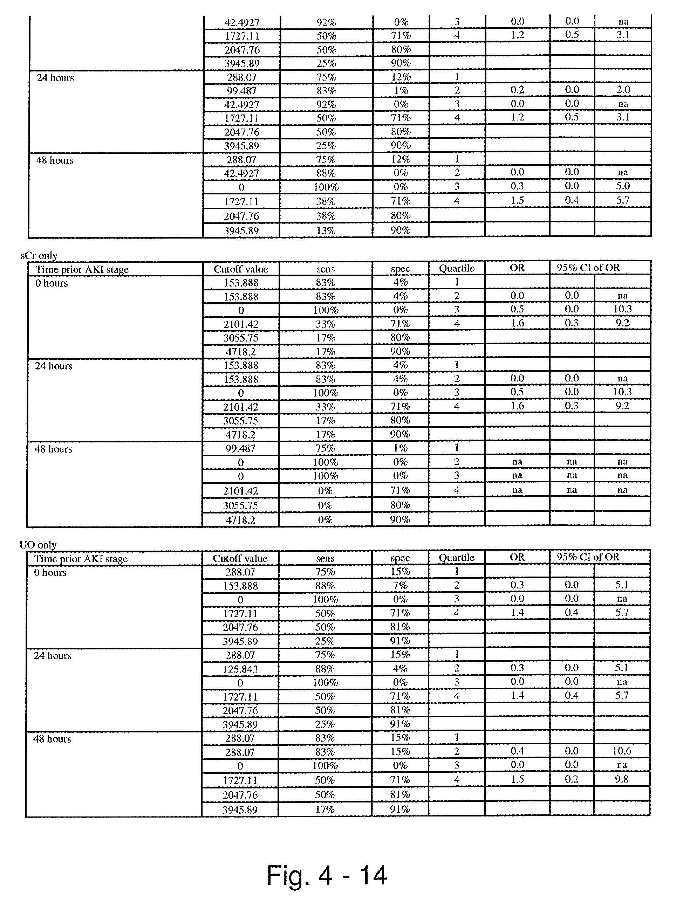

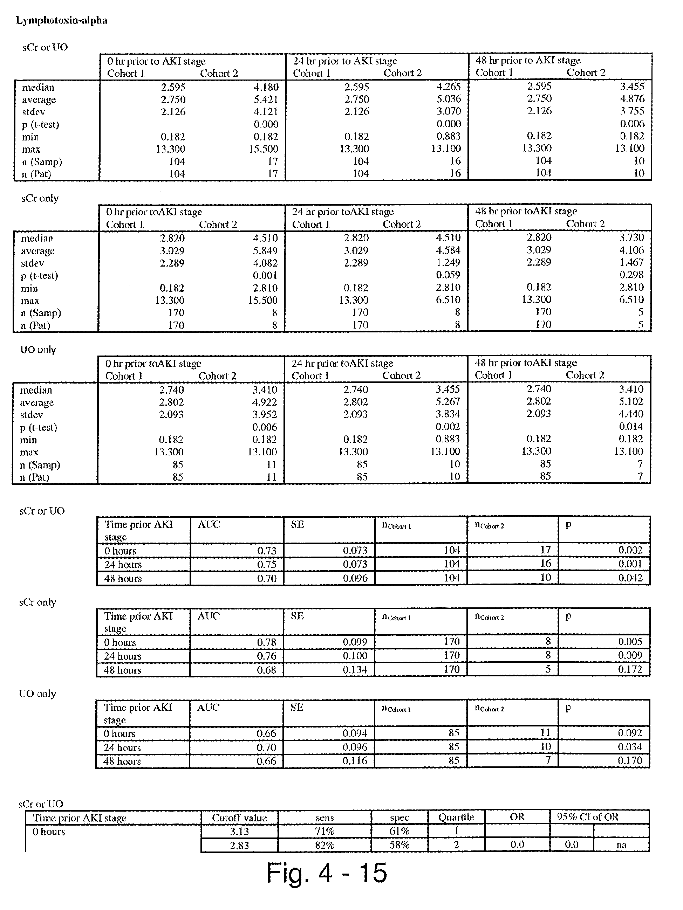

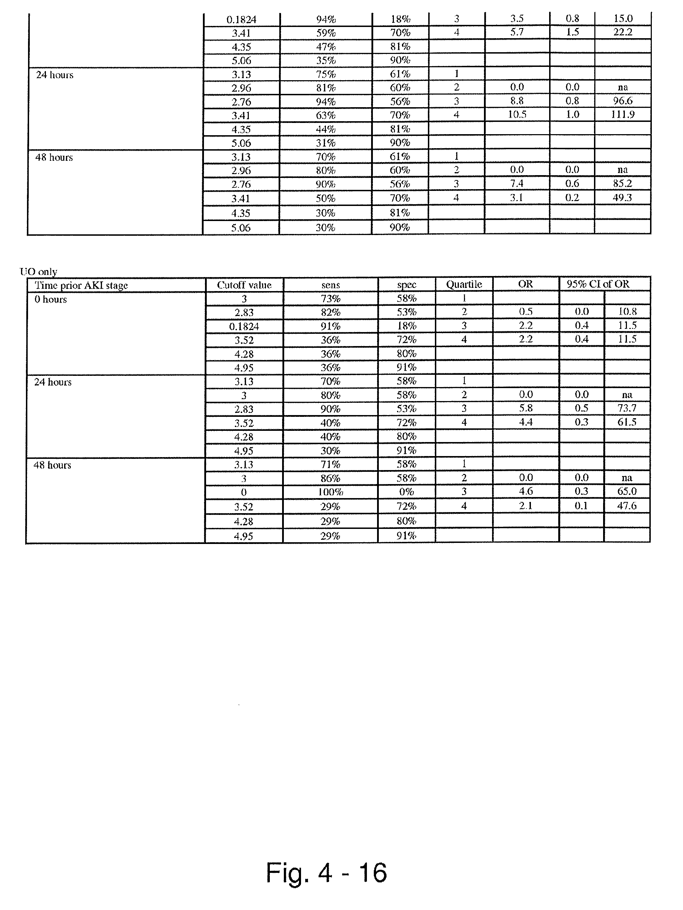

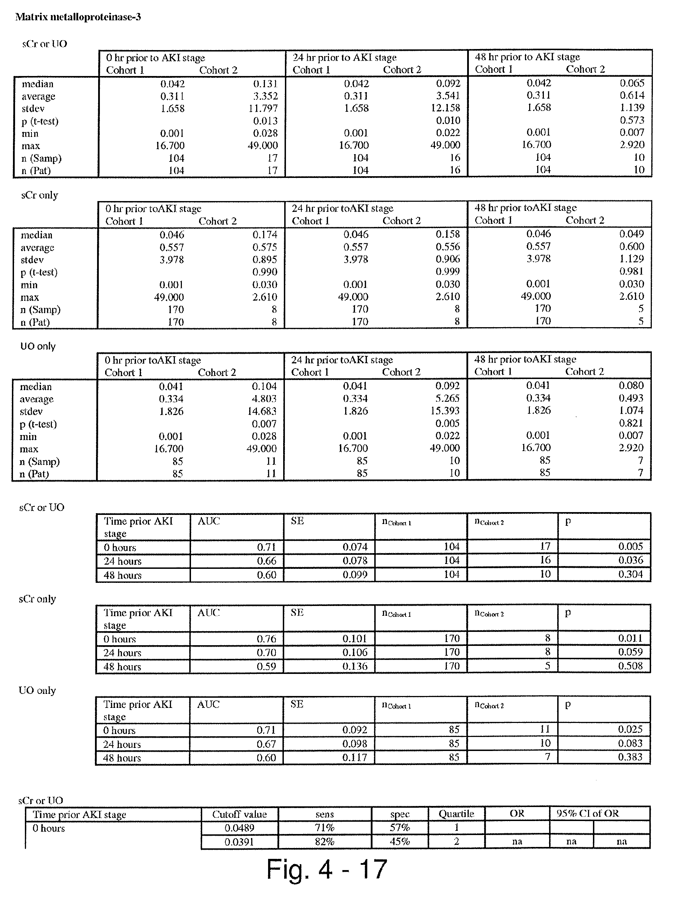

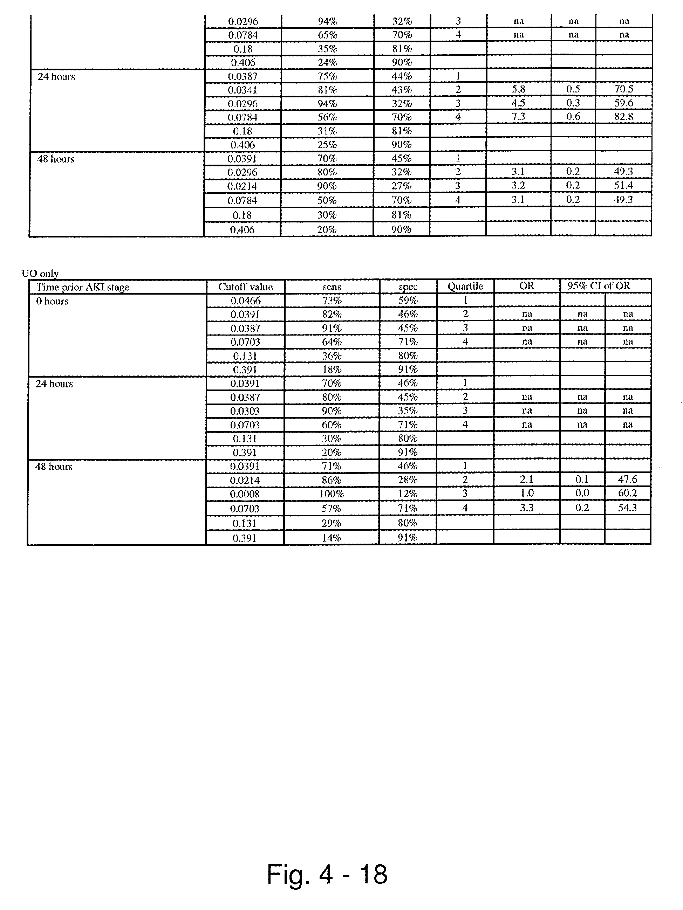

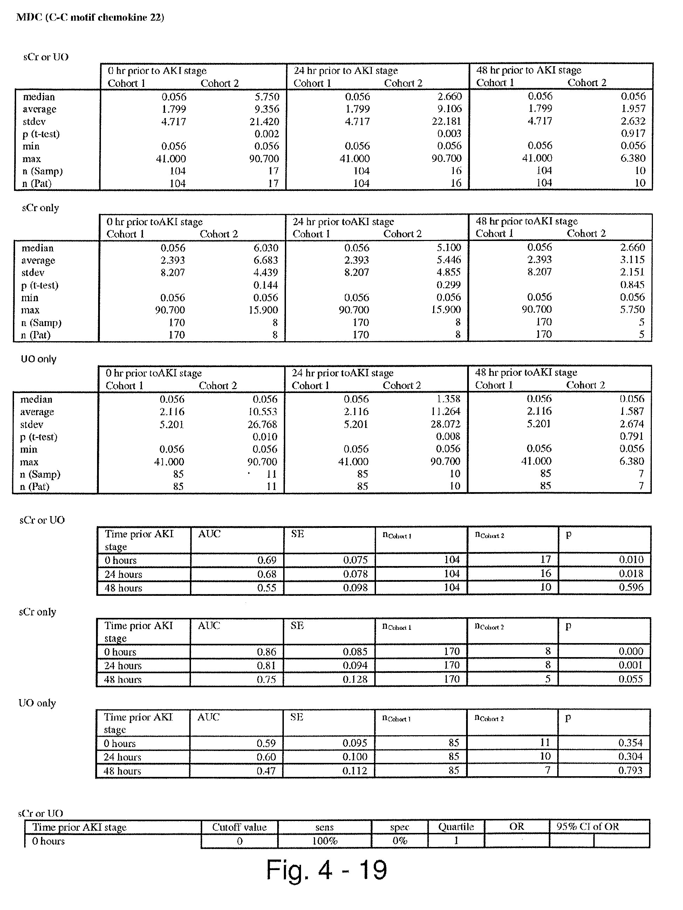

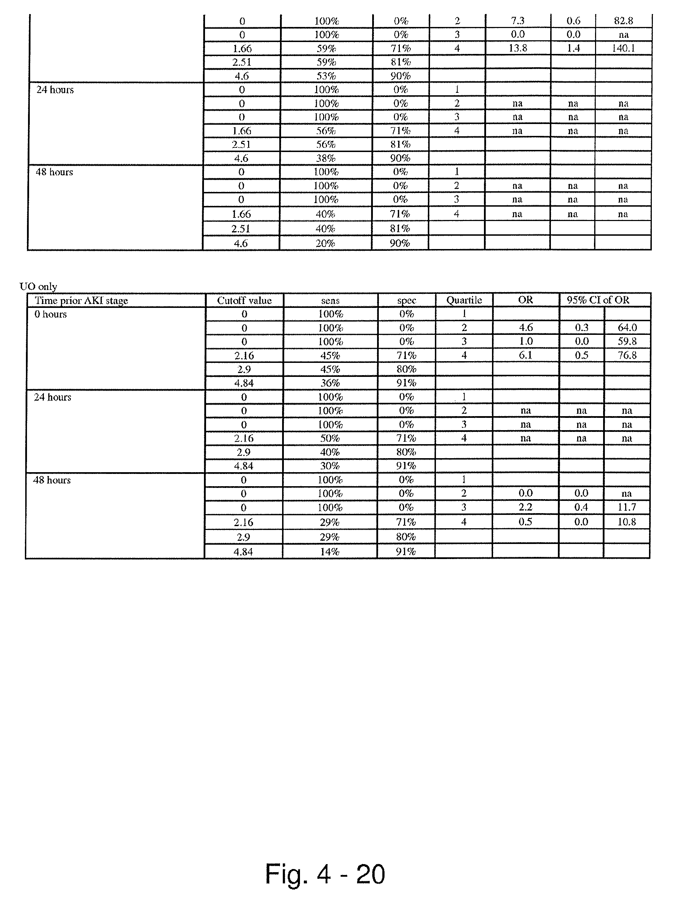

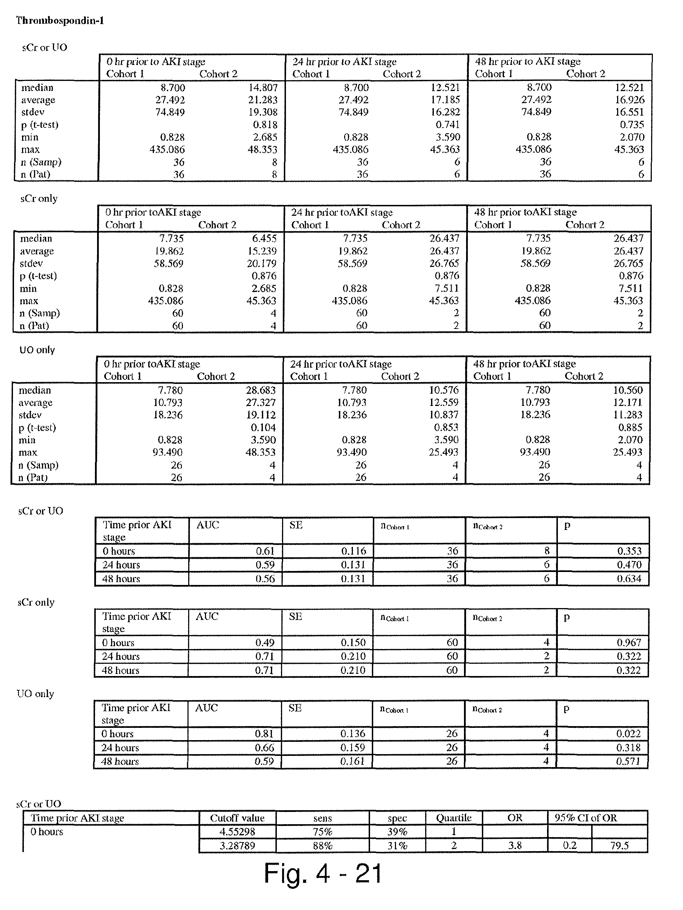

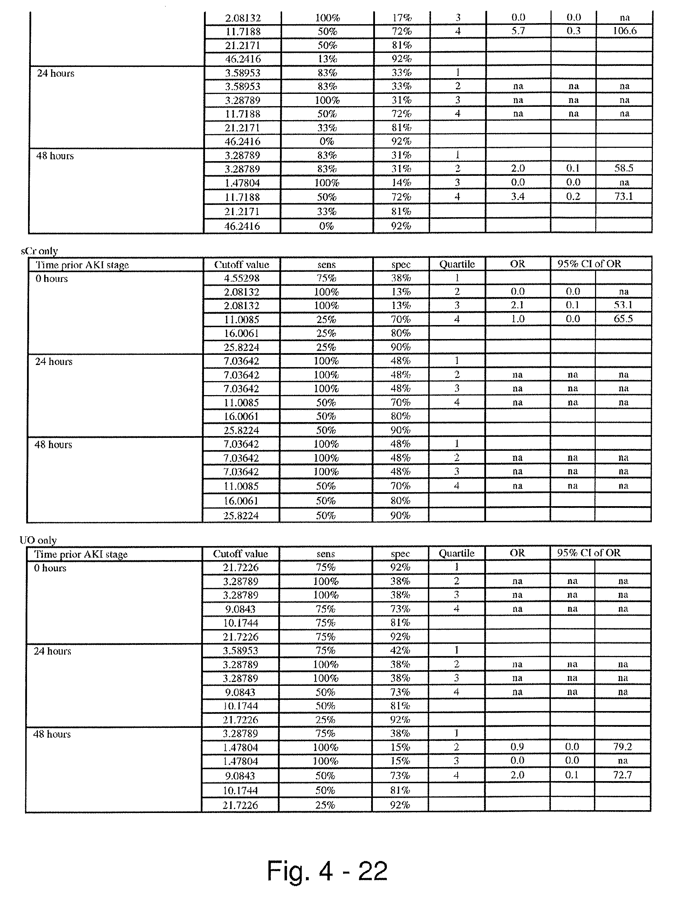

Fig. 4 provides data tables determined in accordance with Example 9 for the comparison of marker levels in urine samples collected for Cohort 1 (patients that did not progress beyond RIFLE stage 0) and in urine samples collected from subjects at 0, 24 hours, and 48 hours prior to reaching stage F in Cohort 2. Tables provide descriptive statistics, AUC analysis, and sensitivity, specificity and odds ratio calculations at various threshold (cutoff) levels for the various markers.

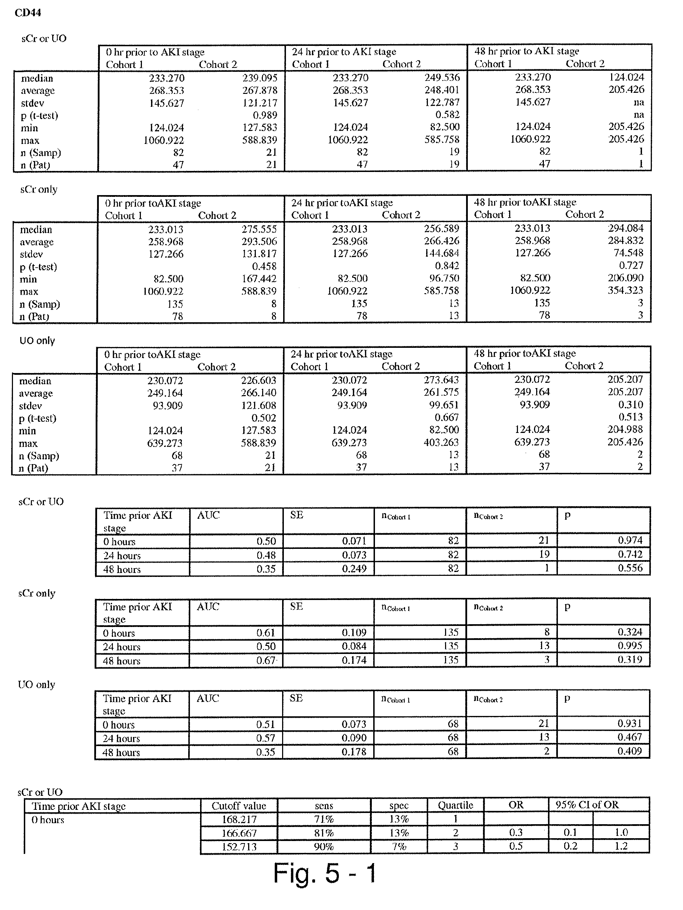

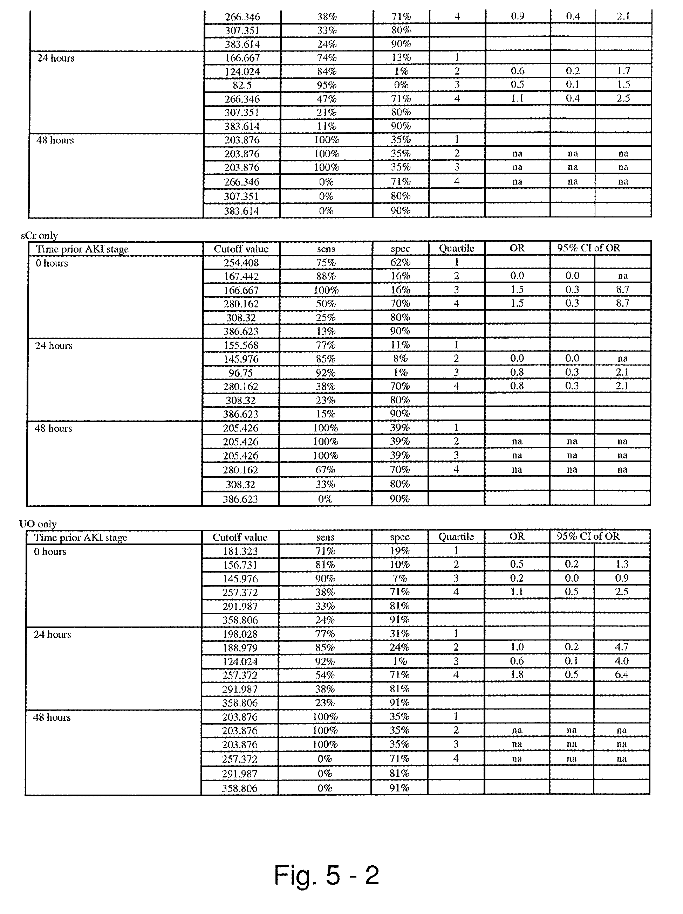

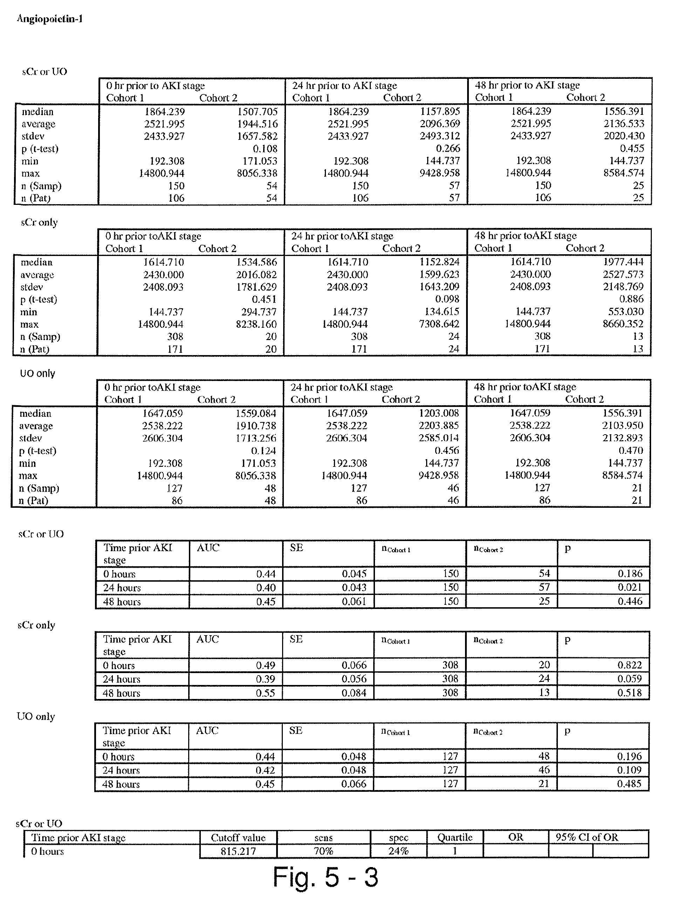

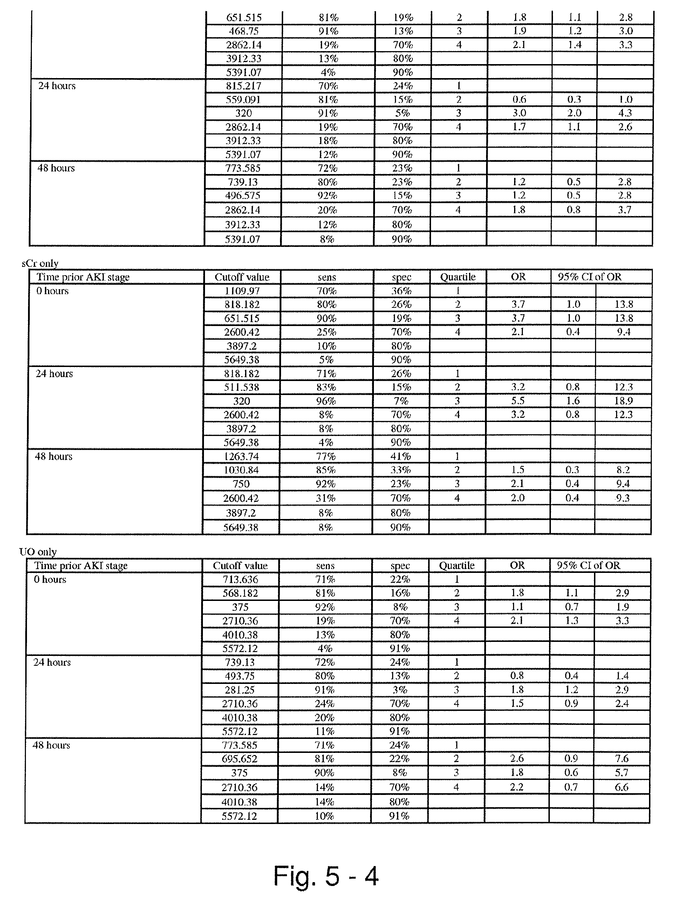

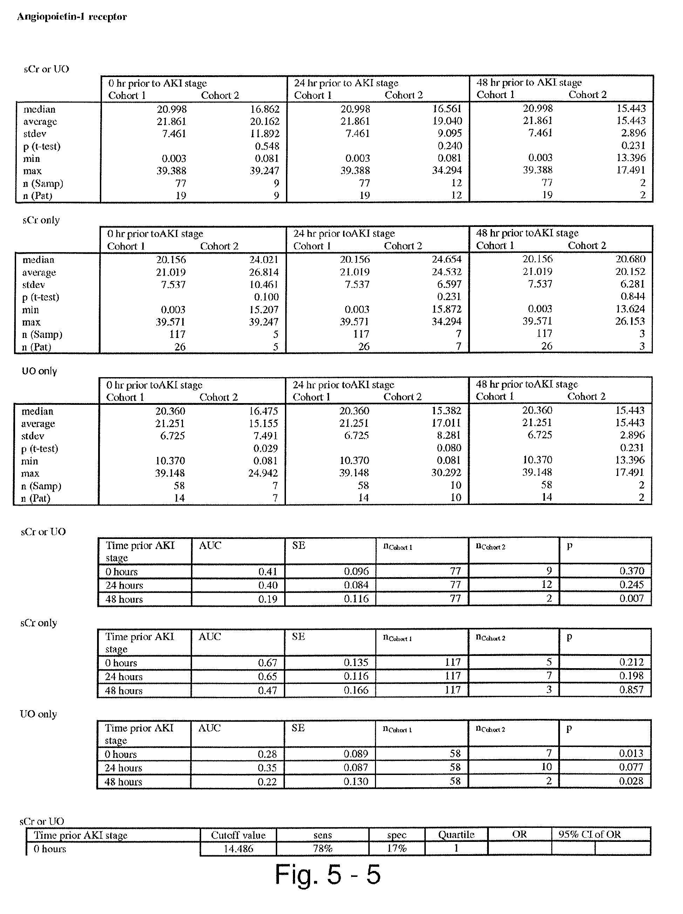

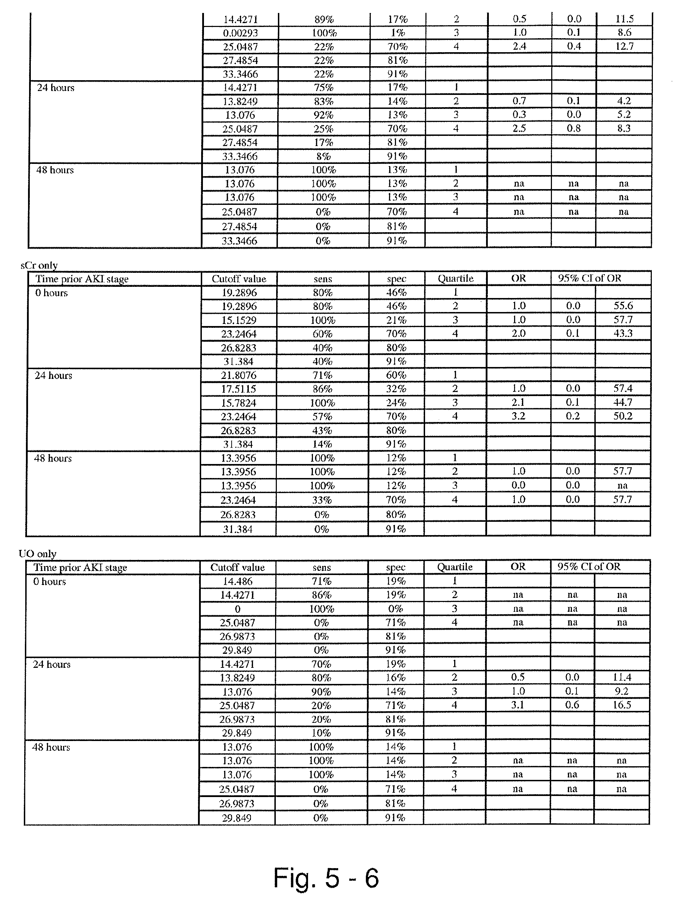

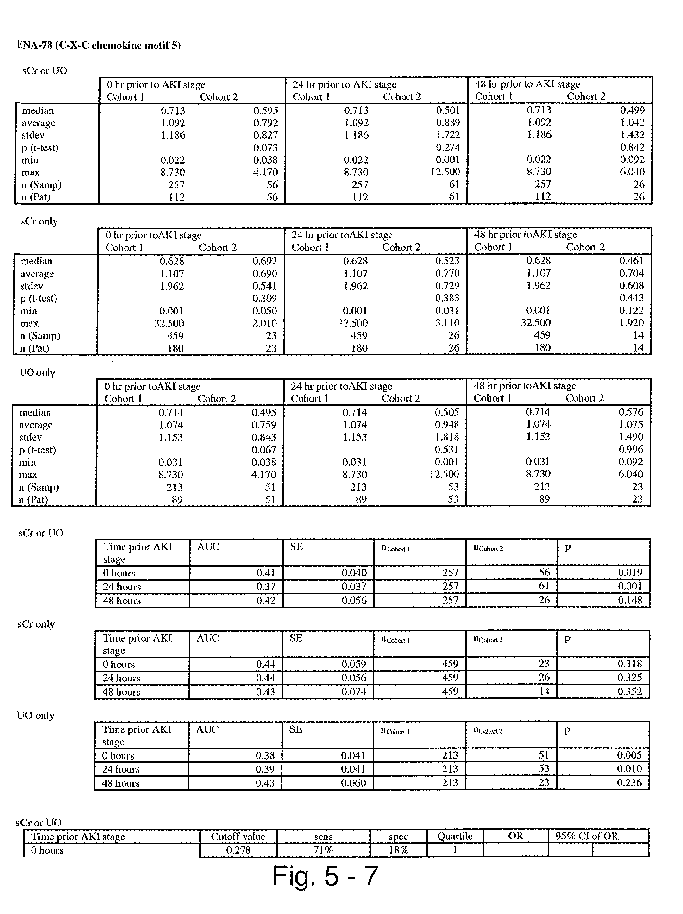

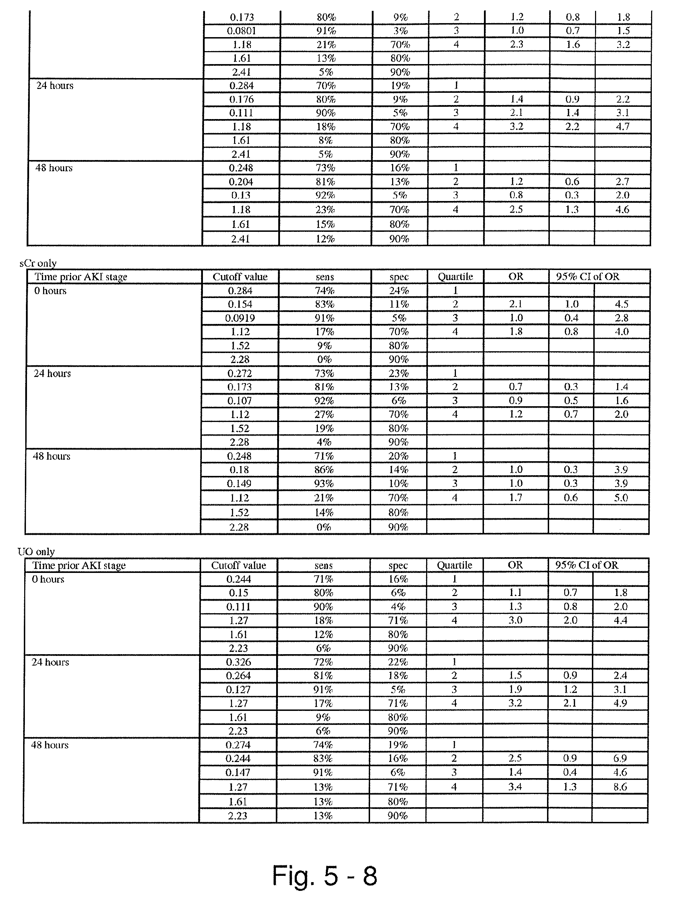

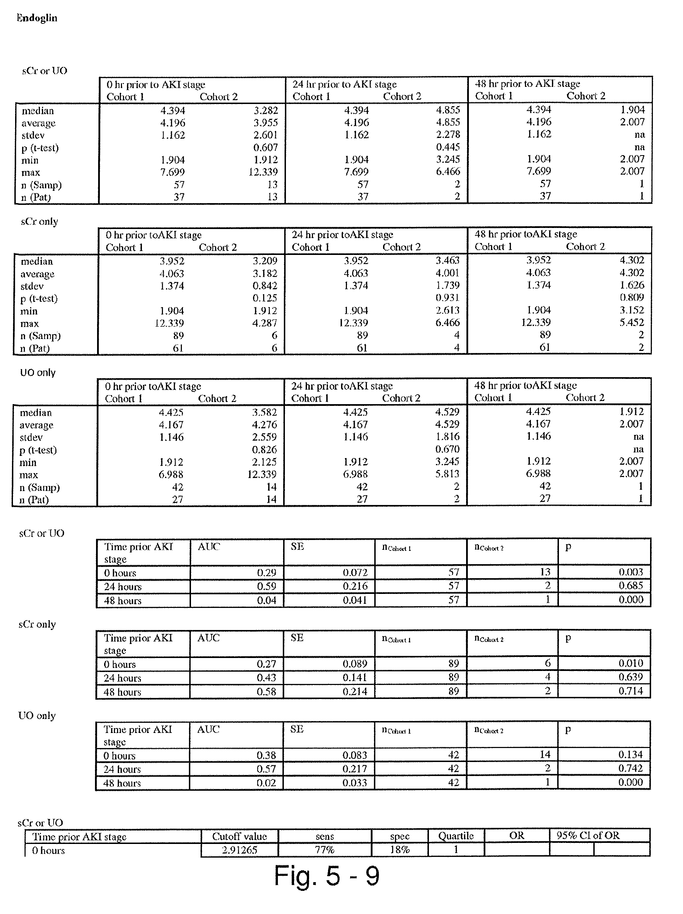

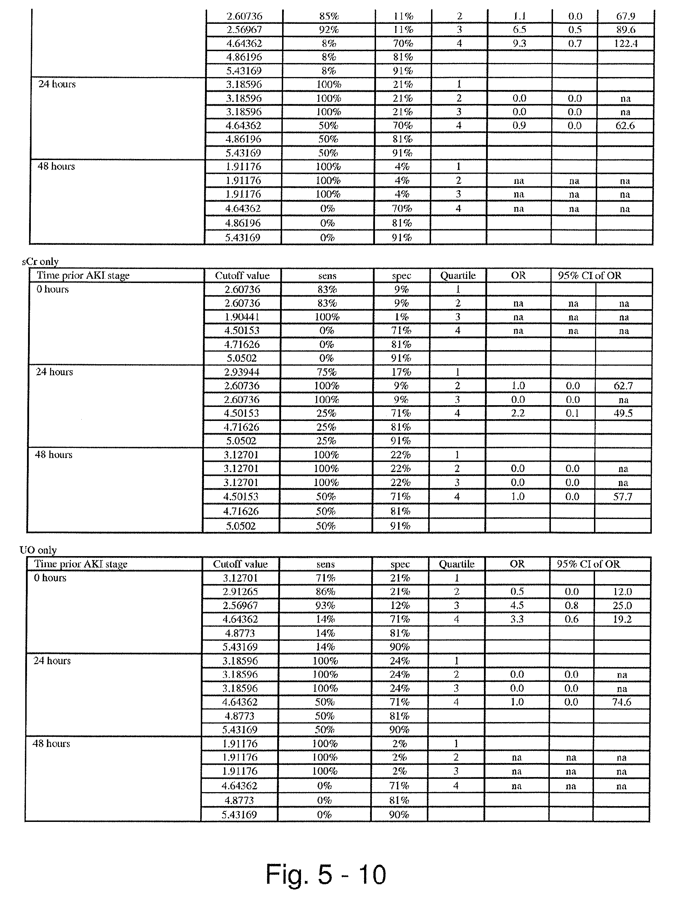

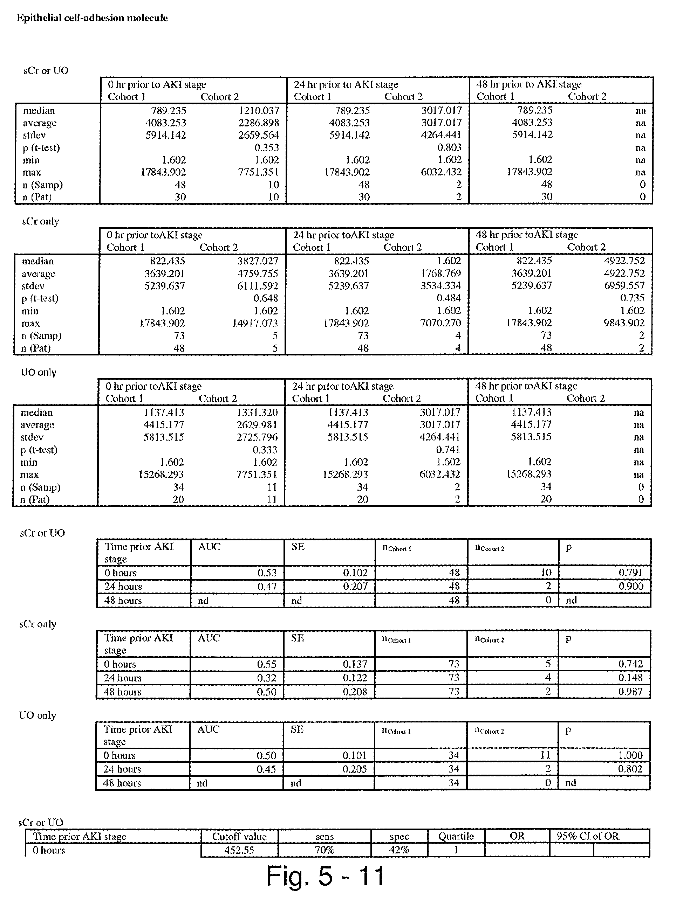

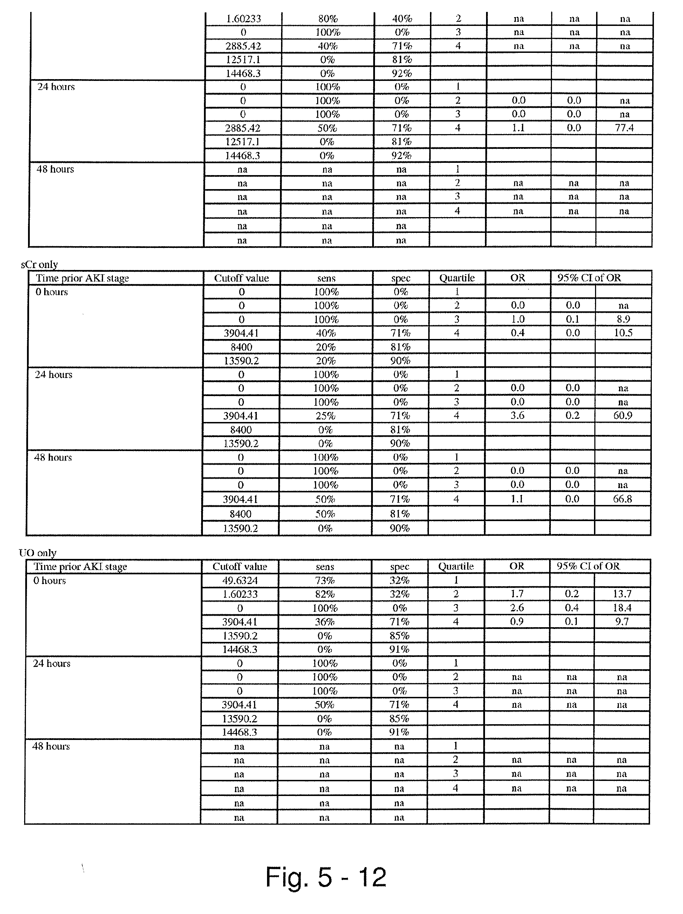

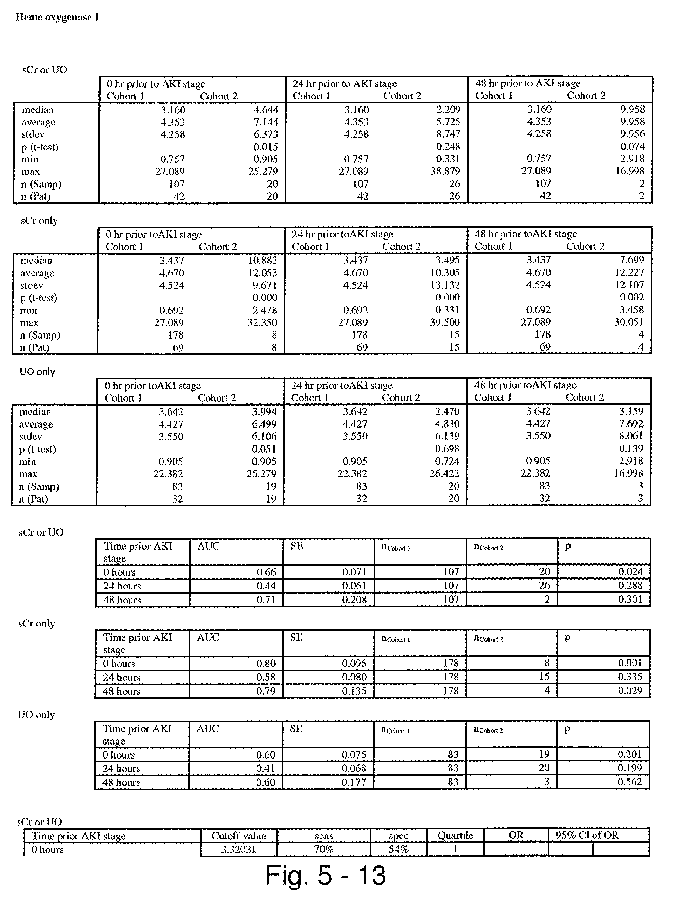

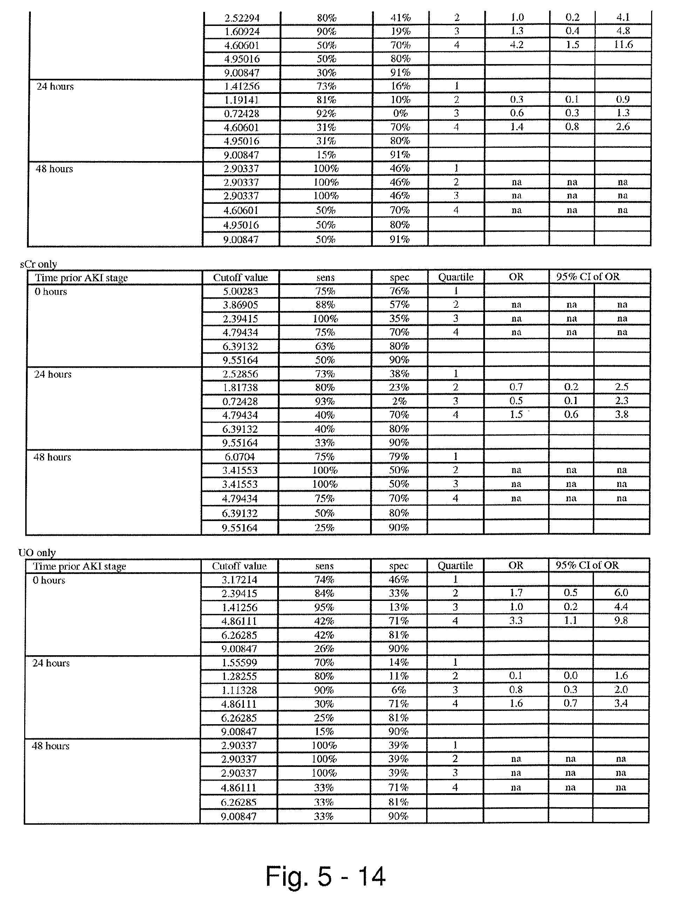

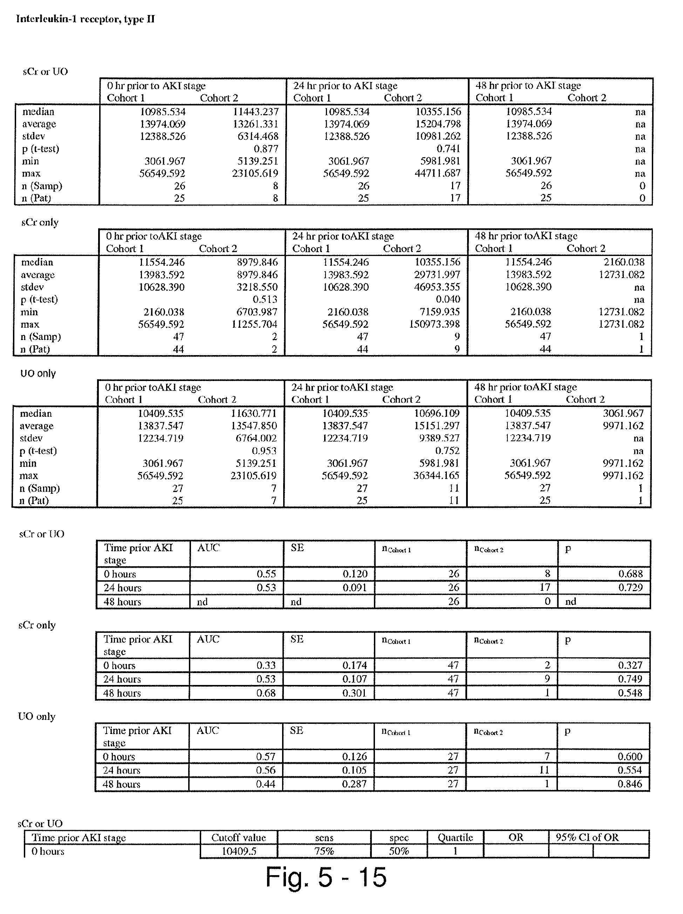

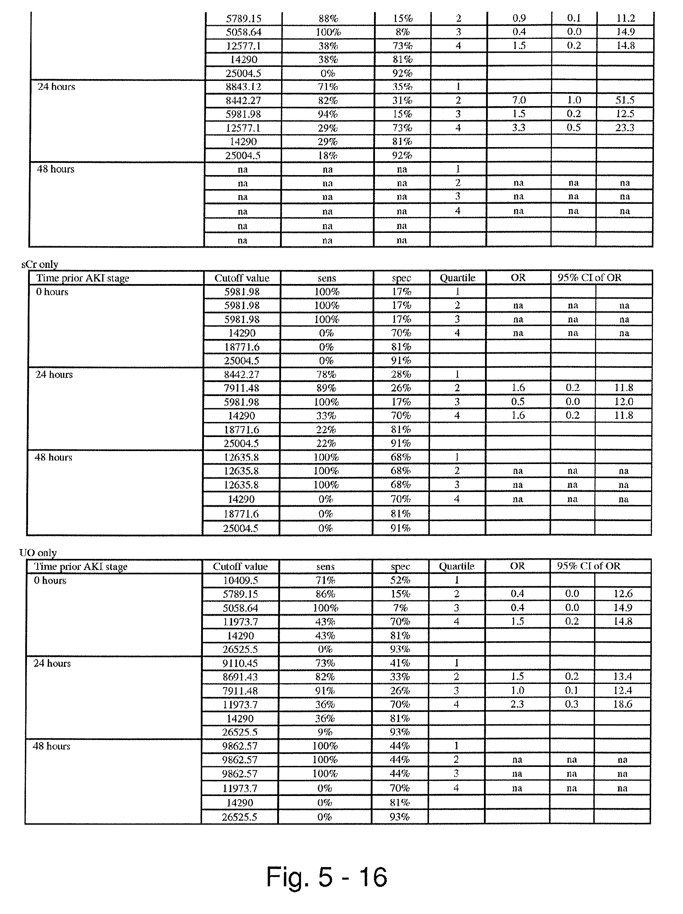

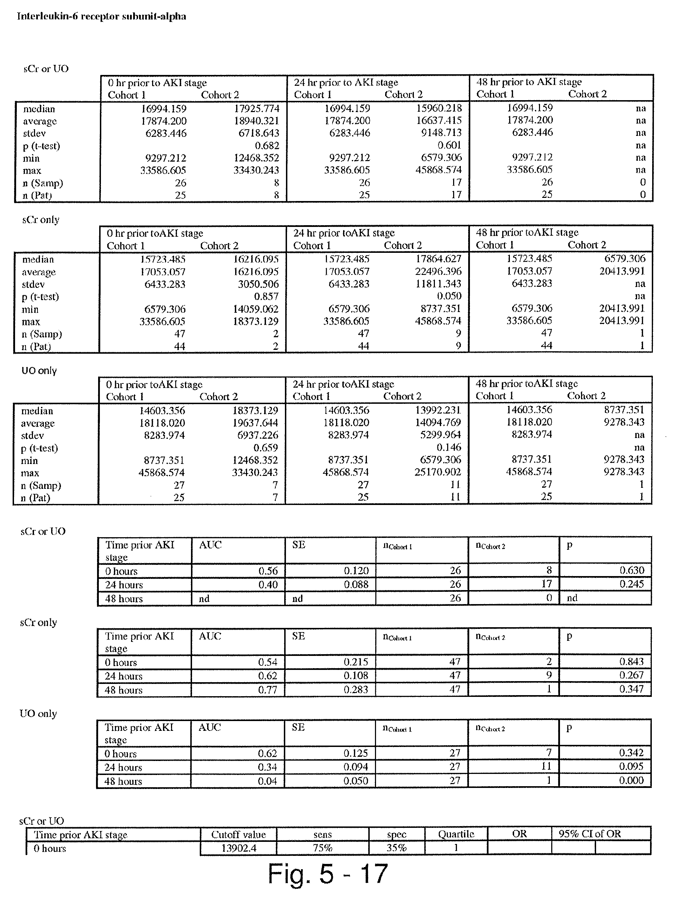

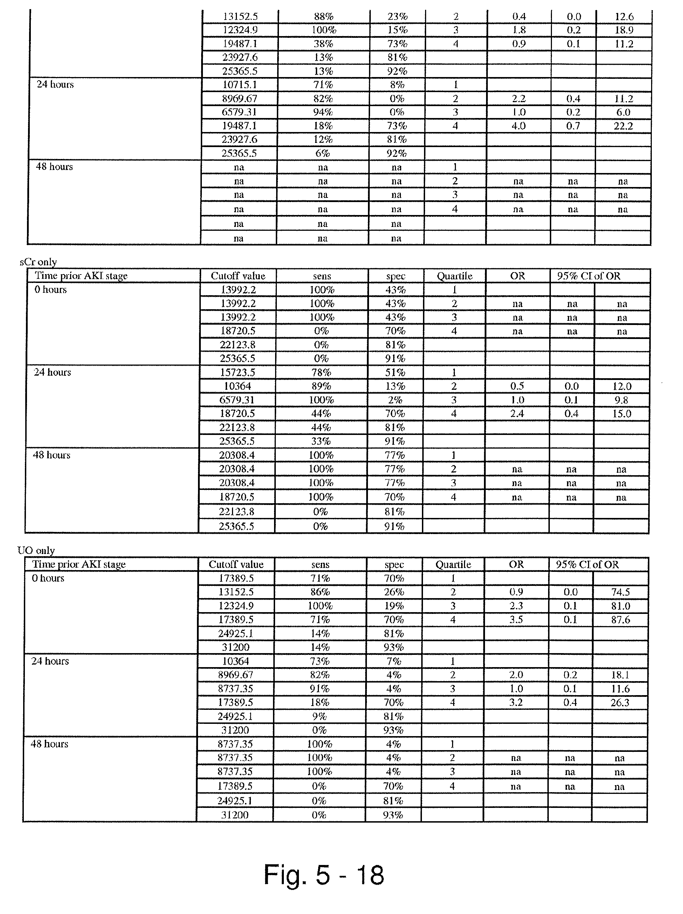

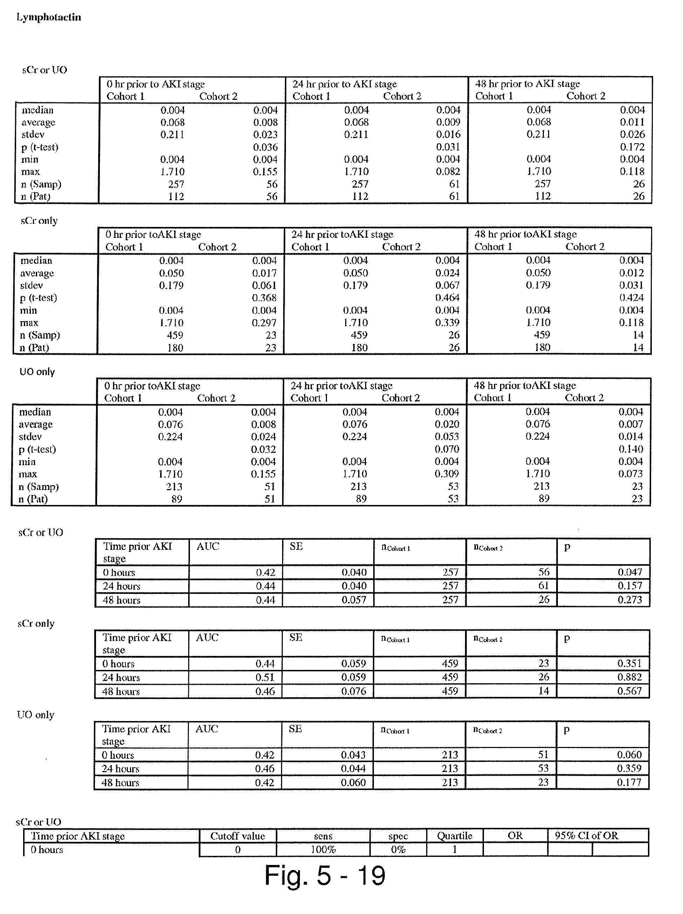

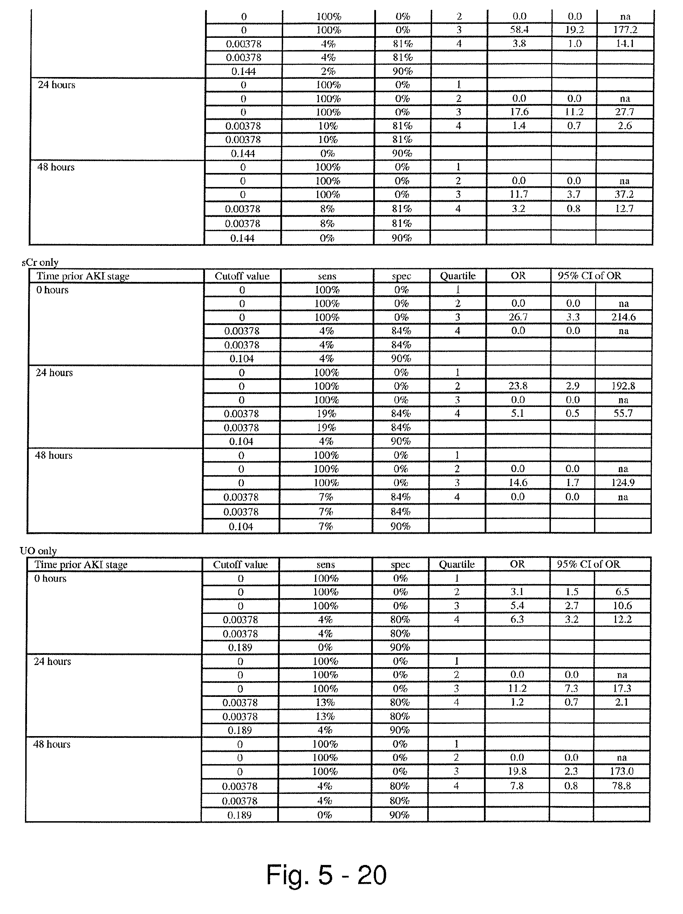

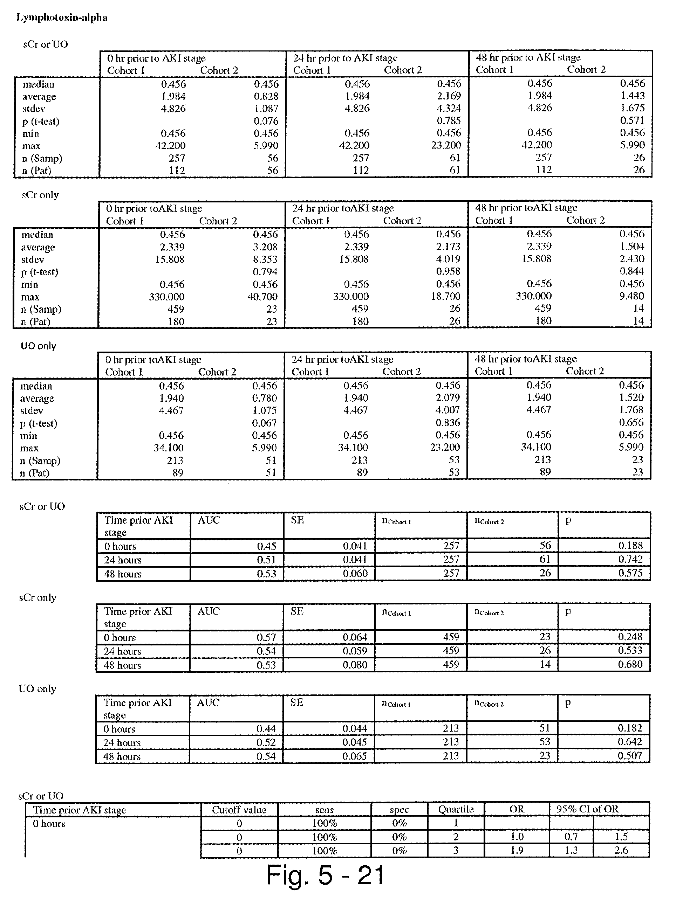

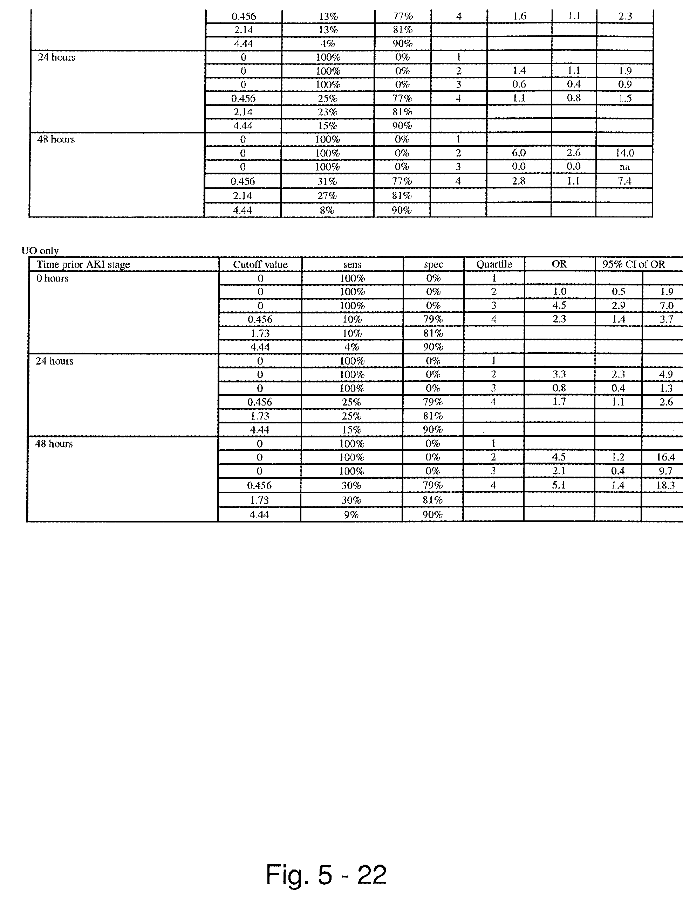

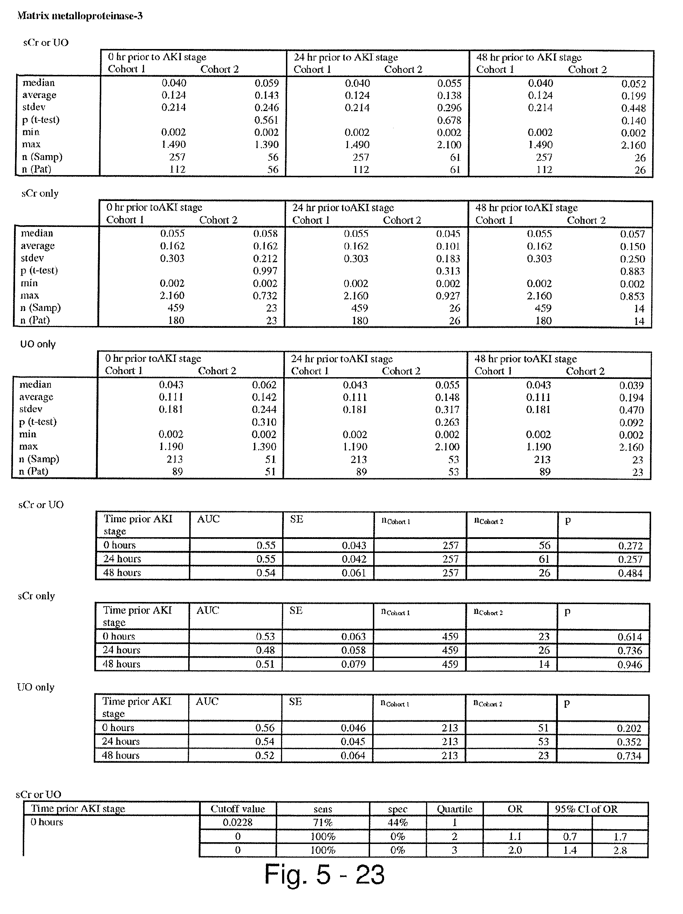

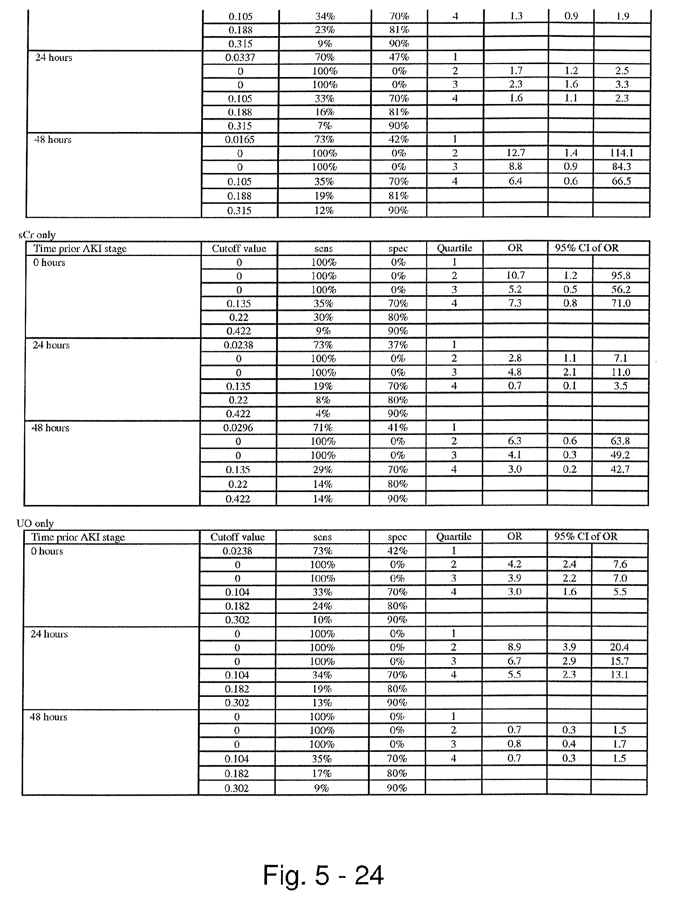

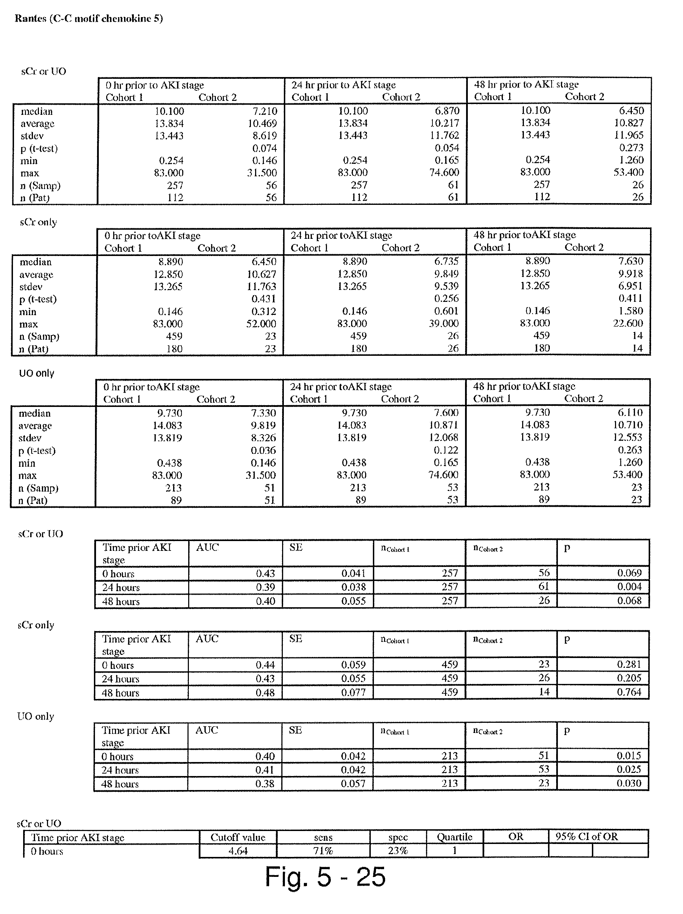

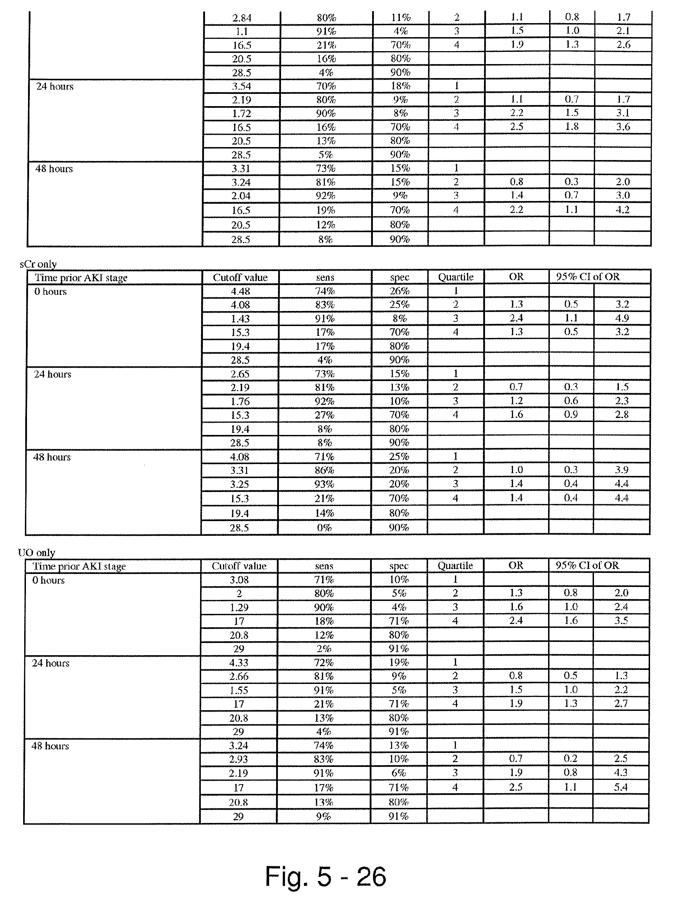

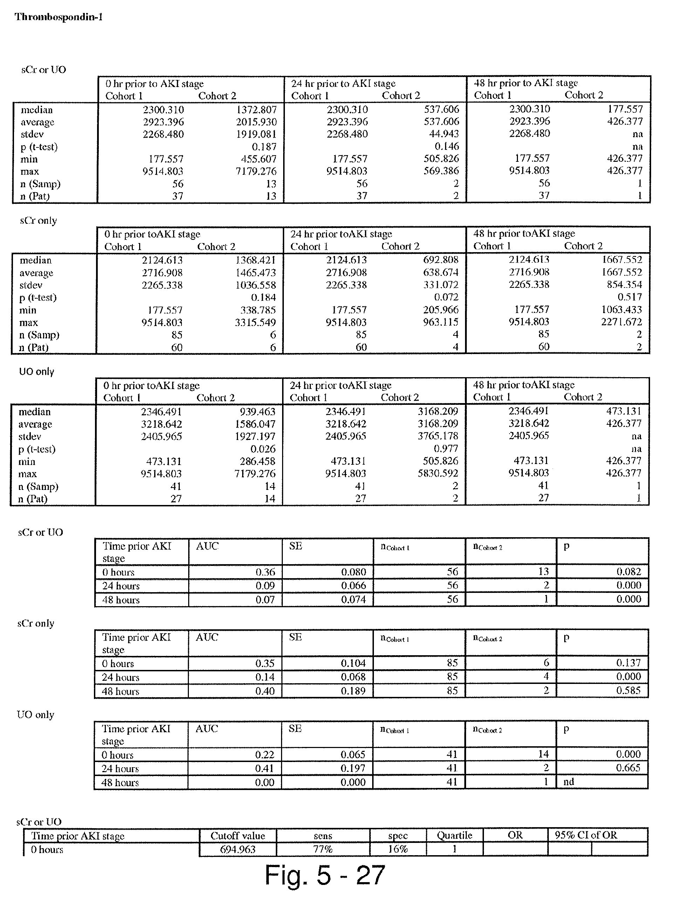

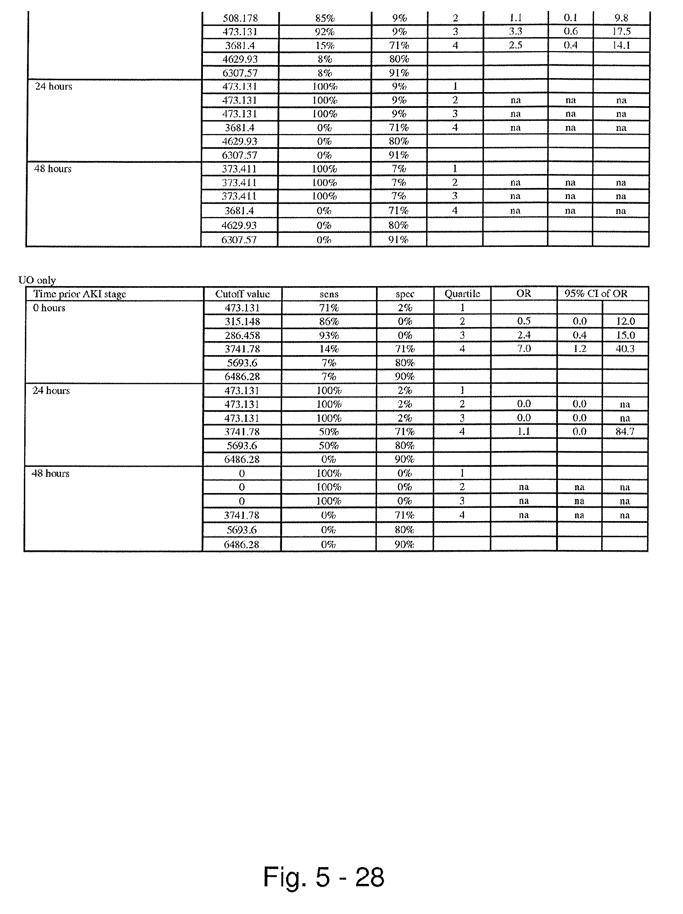

Fig. 5 provides data tables determined in accordance with Example 6 for the comparison of marker levels in plasma samples collected for Cohort 1 (patients that did not progress beyond RIFLE stage 0) and in plasma samples collected from subjects at 0, 24 hours, and 48 hours prior to reaching stage R, I or F in Cohort 2. Tables provide descriptive statistics, AUC analysis, and sensitivity, specificity and odds ratio calculations at various threshold (cutoff) levels for the various markers.

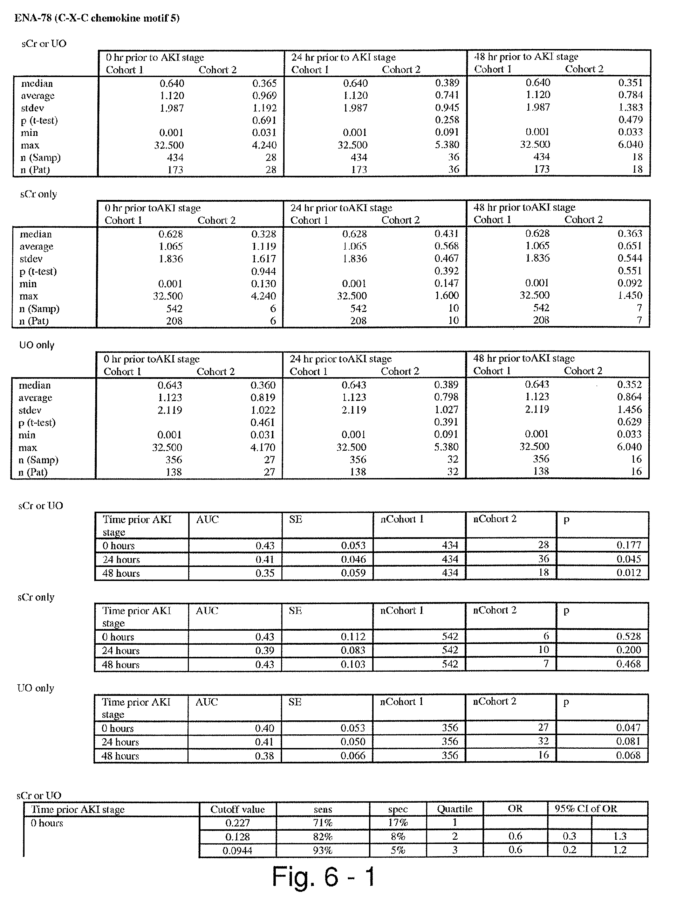

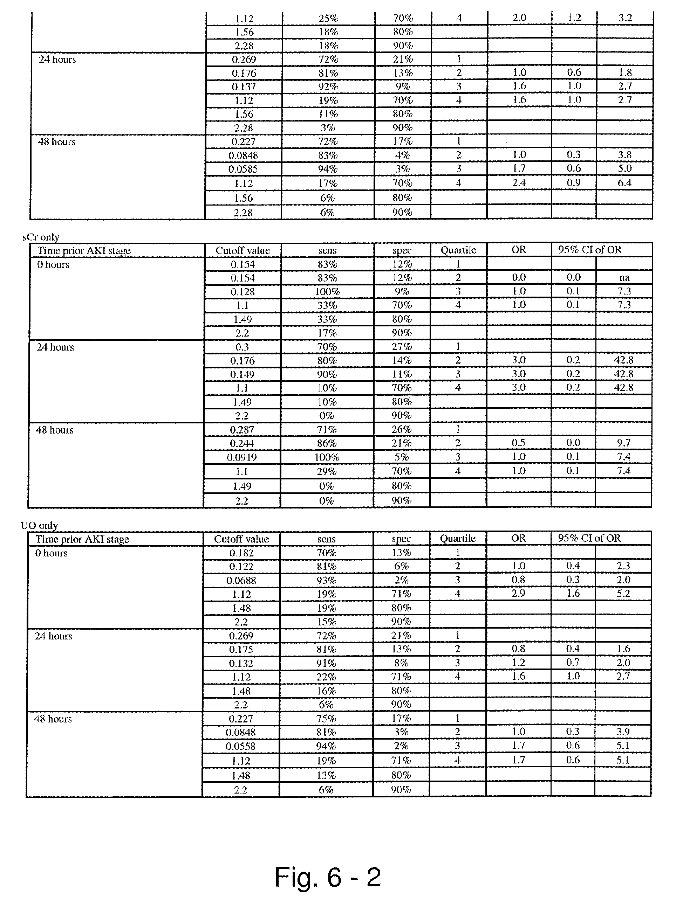

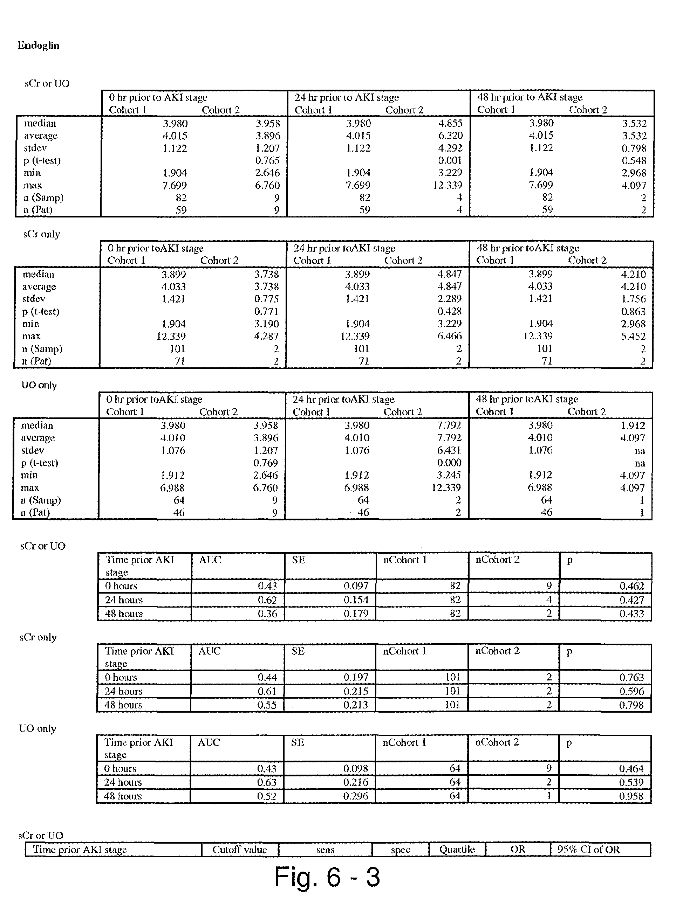

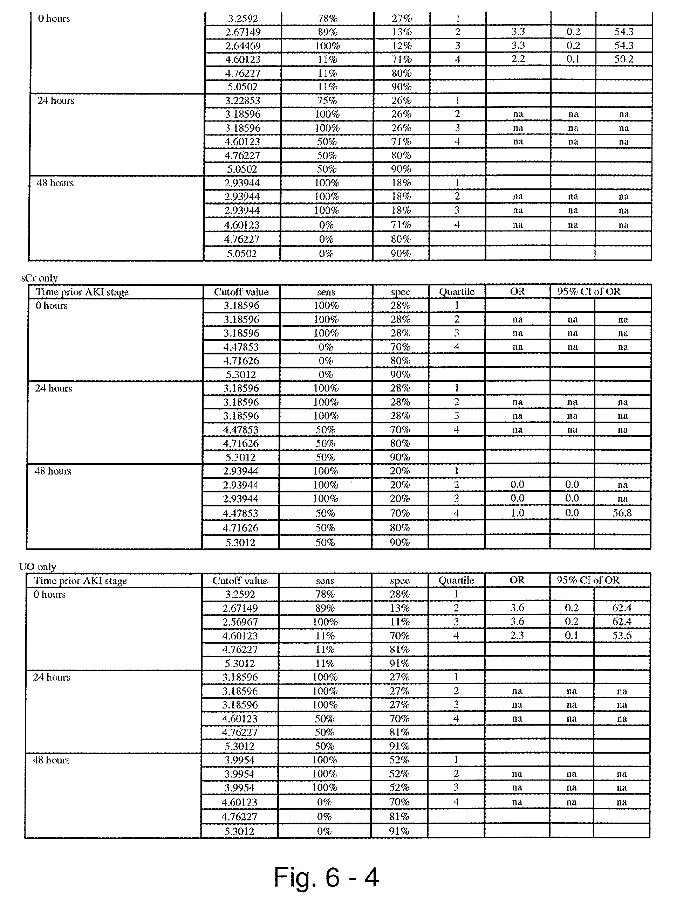

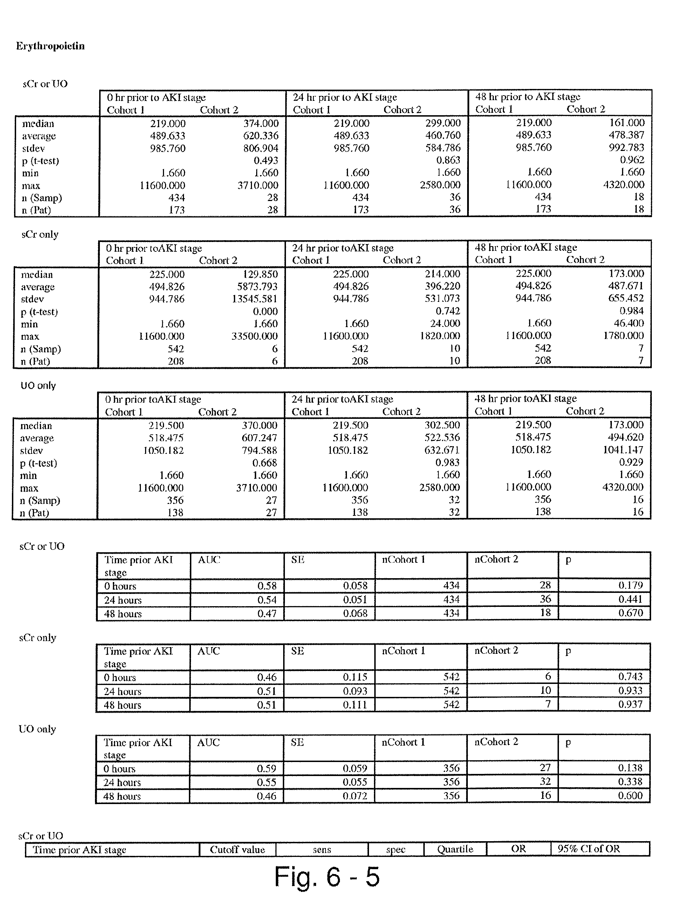

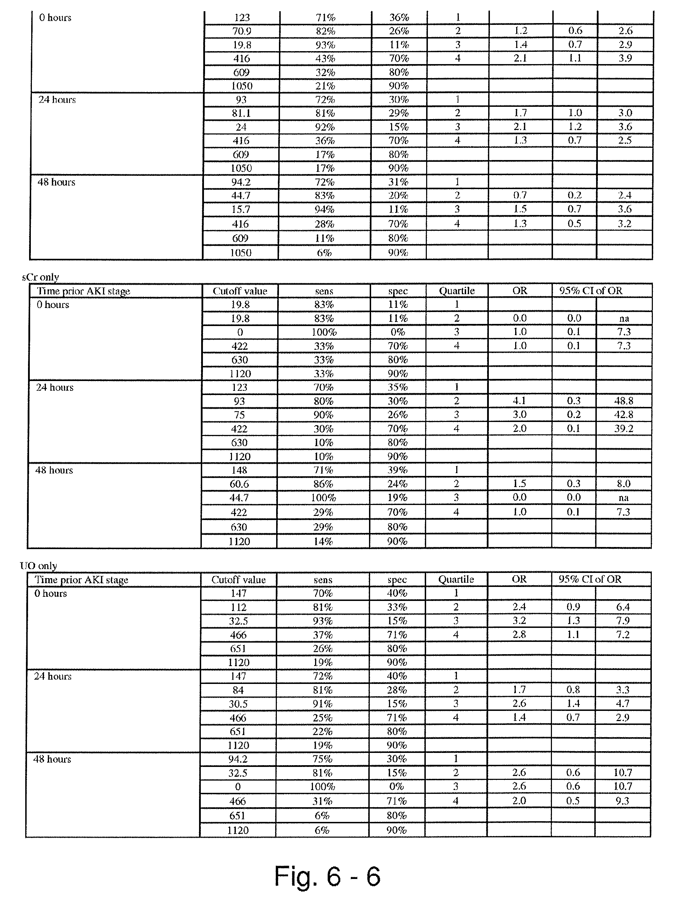

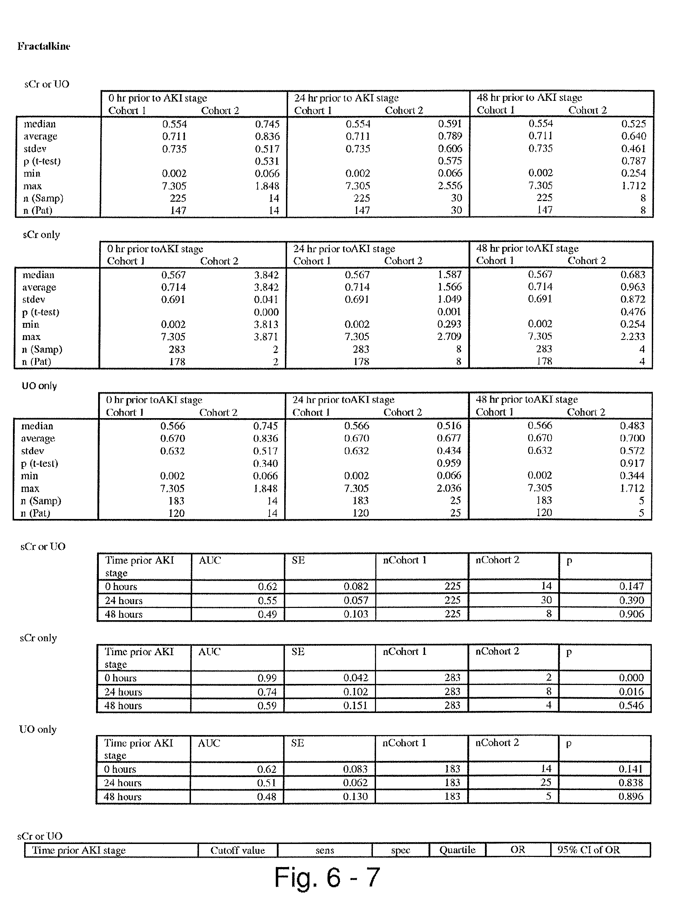

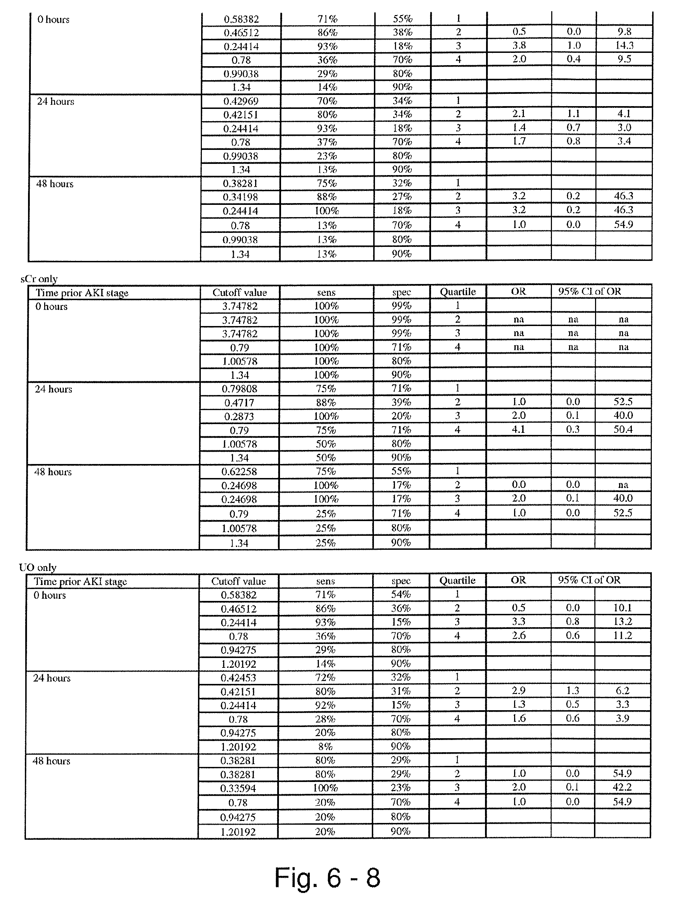

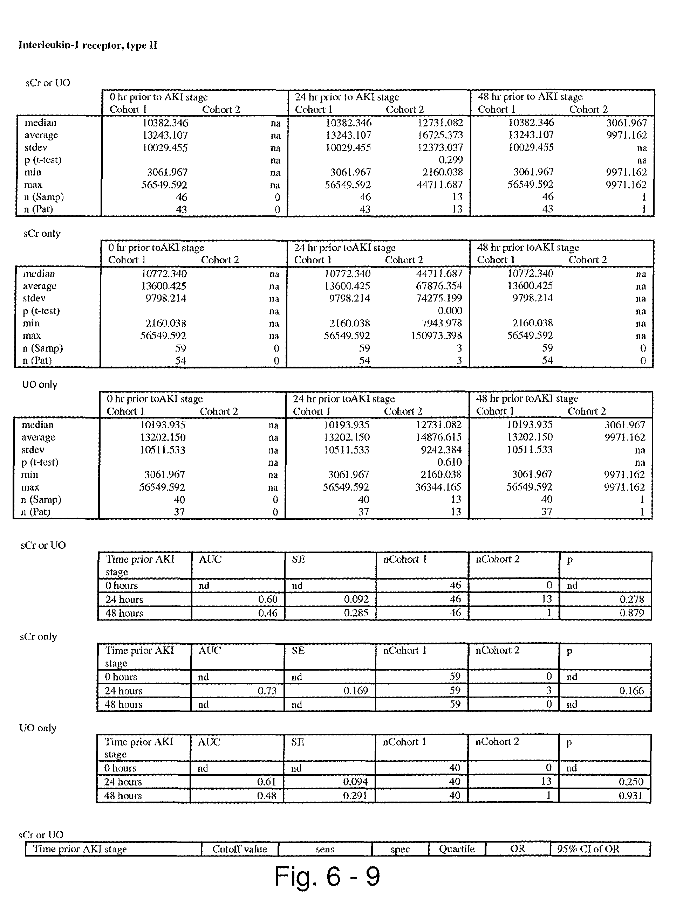

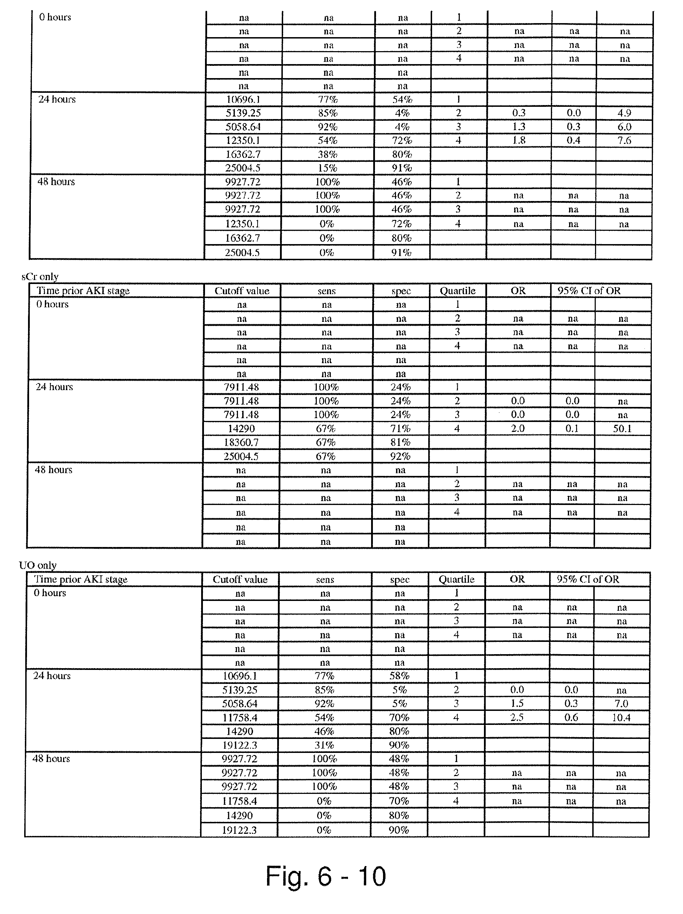

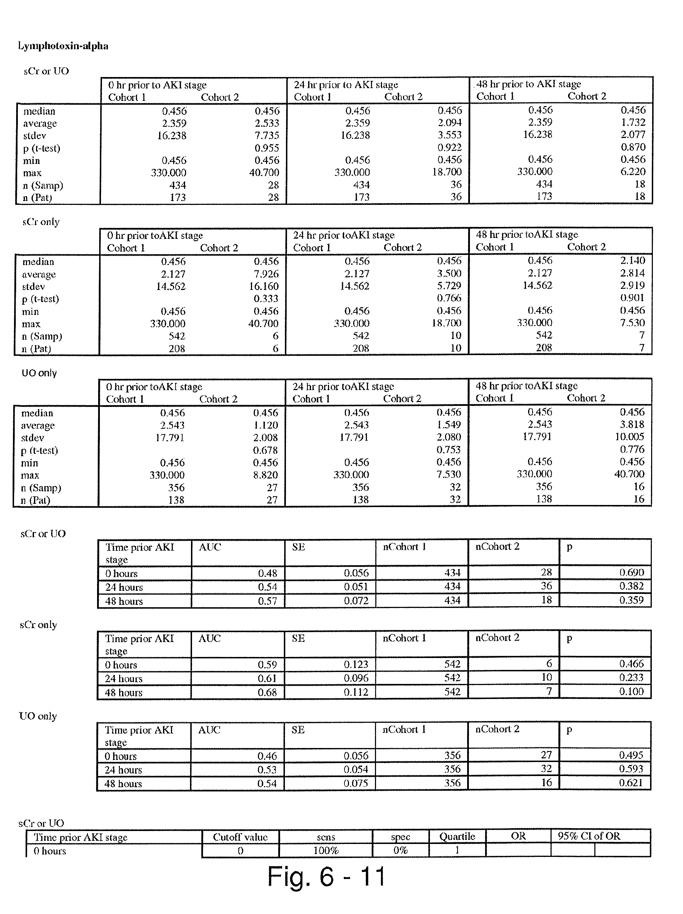

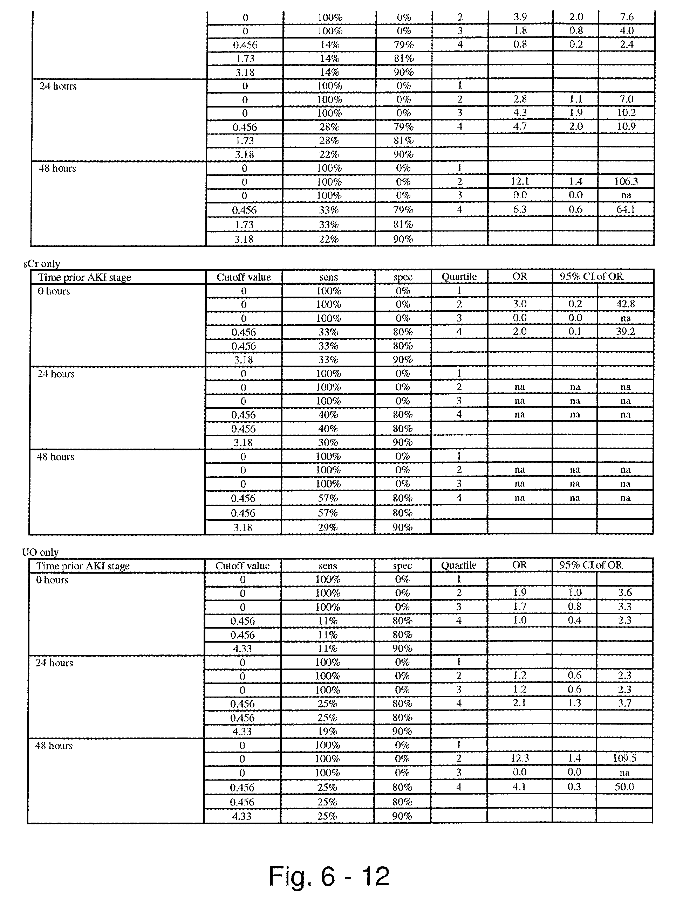

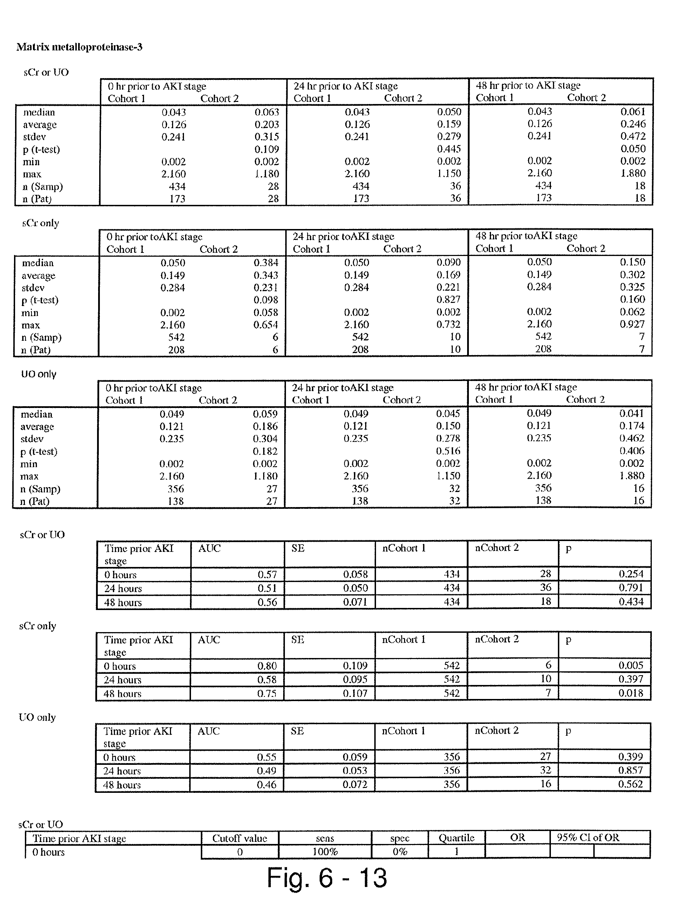

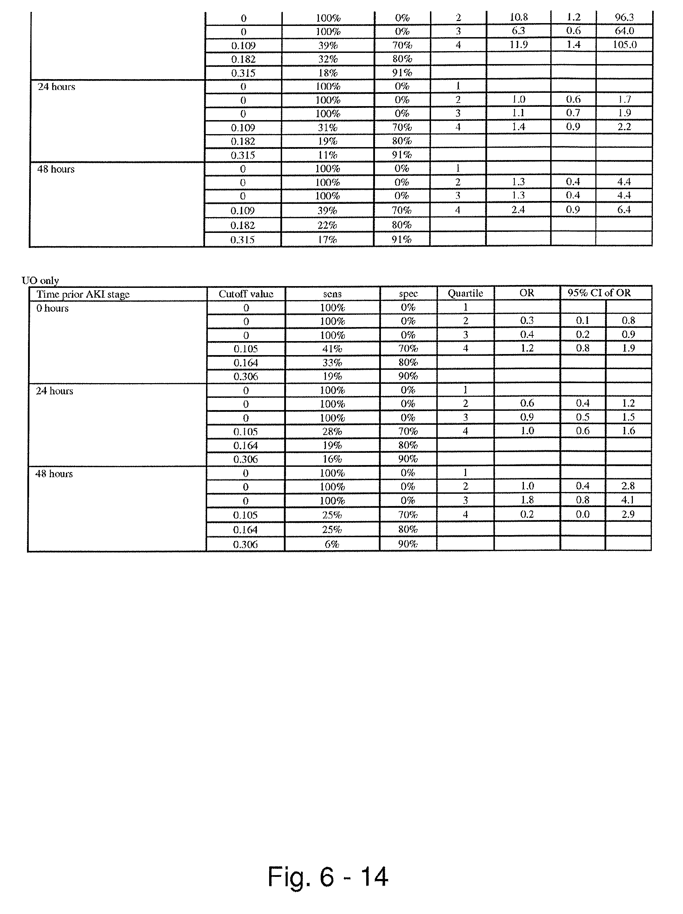

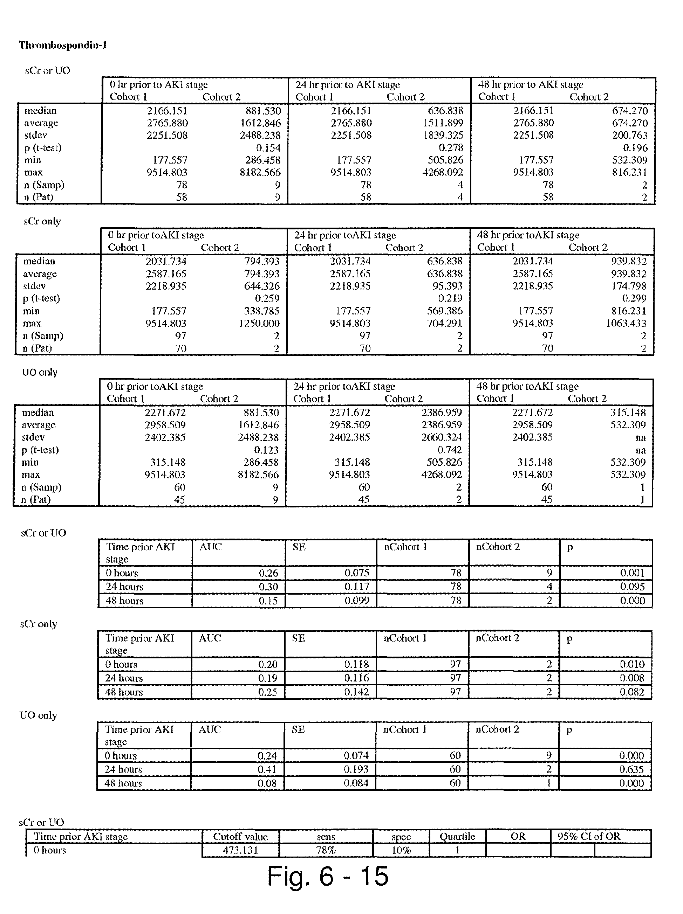

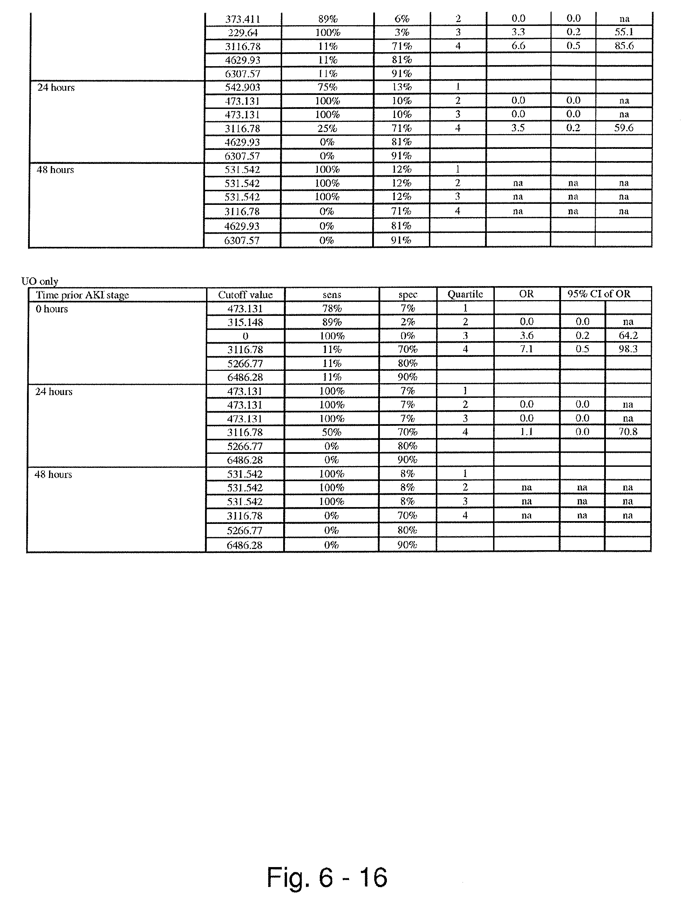

Fig. 6 provides data tables determined in accordance with Example 7 for the comparison of marker levels in plasma samples collected for Cohort 1 (patients that did not progress beyond RIFLE stage 0 or R) and in plasma samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

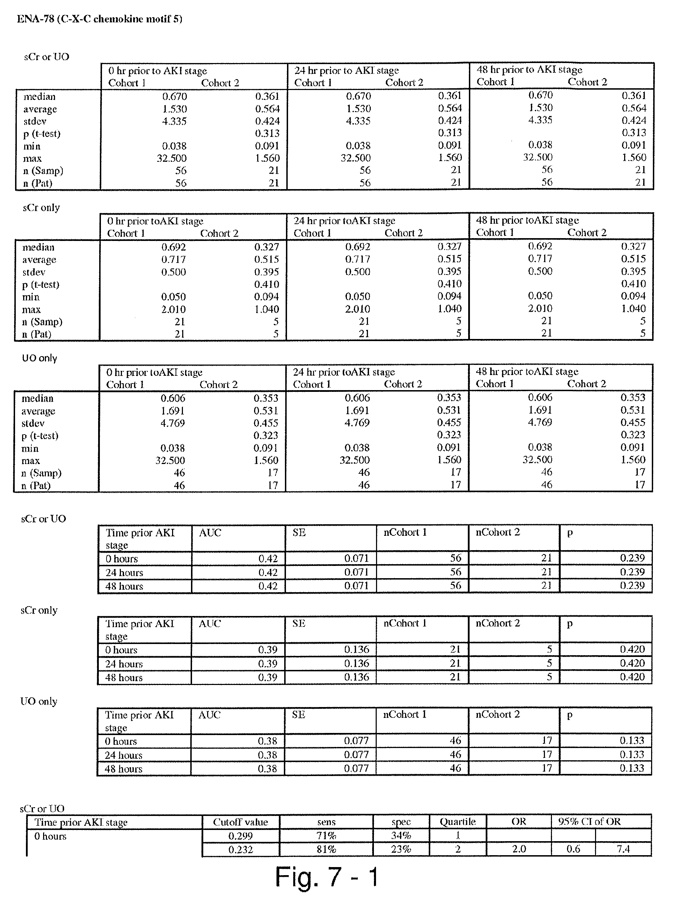

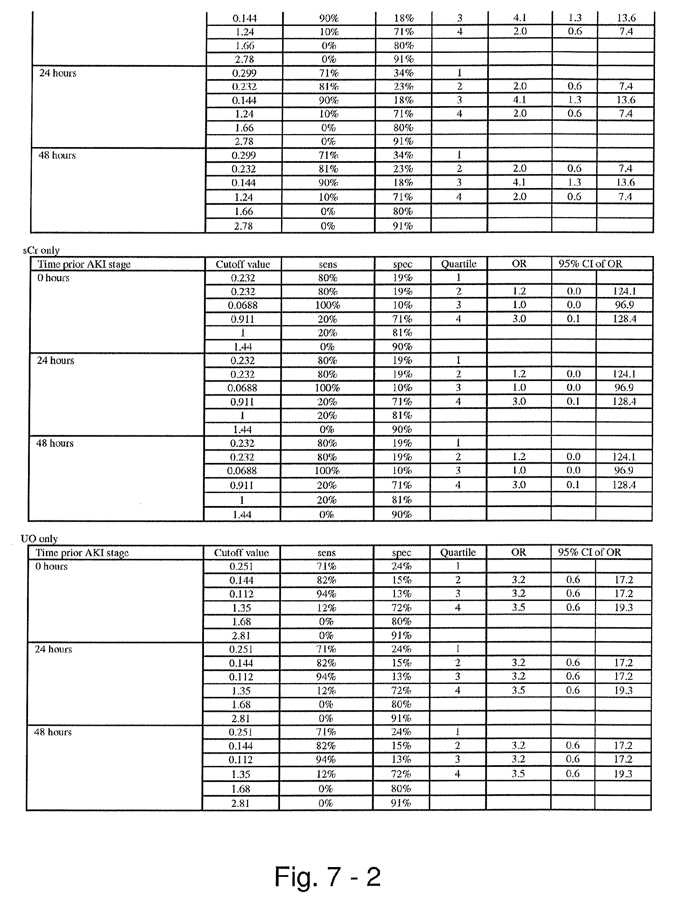

Fig. 7 provides data tables determined in accordance with Example 8 for the comparison of marker levels in plasma samples collected for Cohort 1 (patients that reached, but did not progress beyond, RIFLE stage R) and in plasma samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

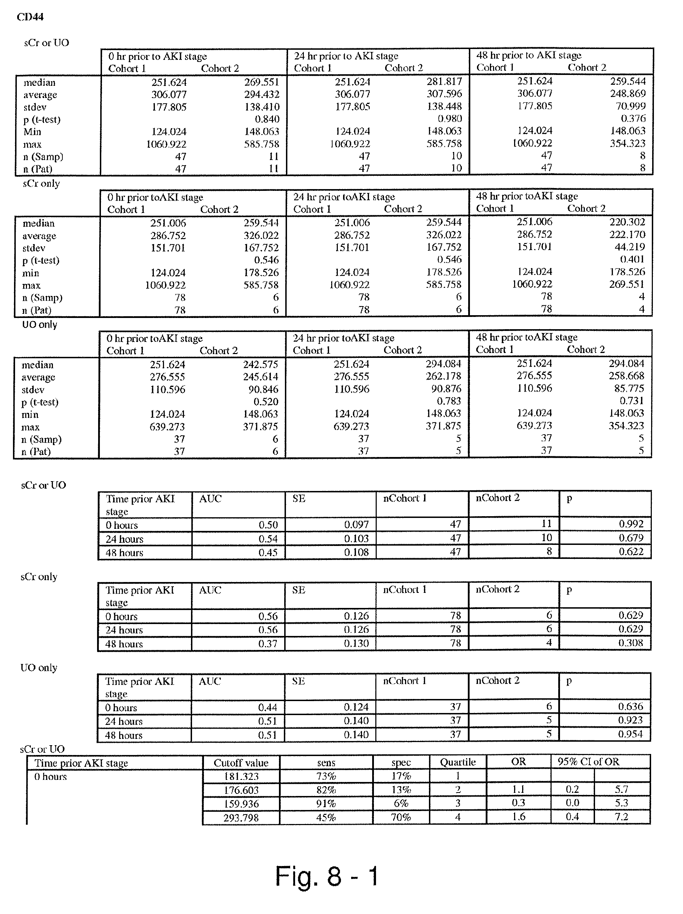

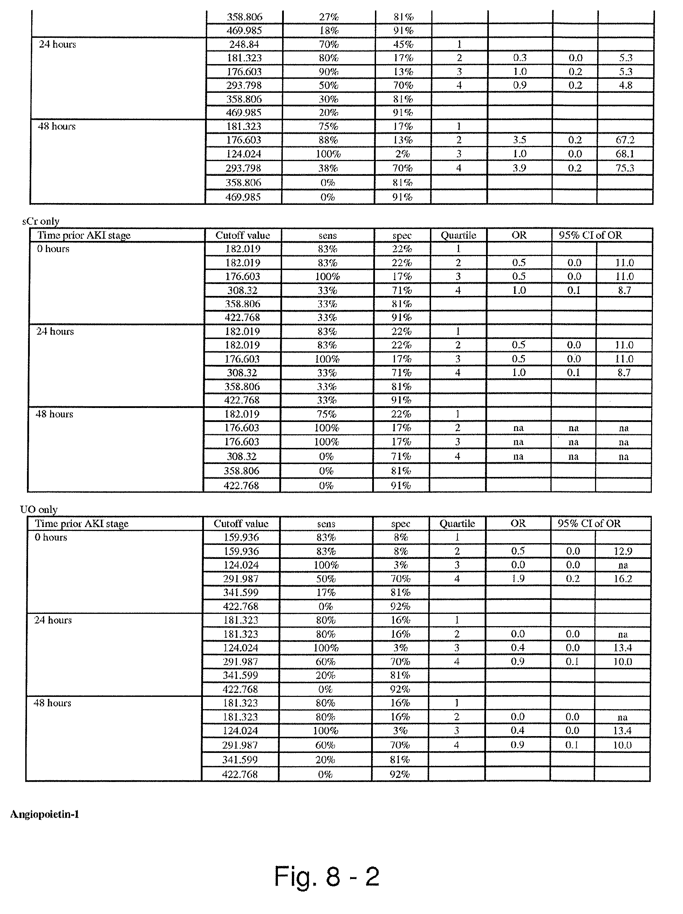

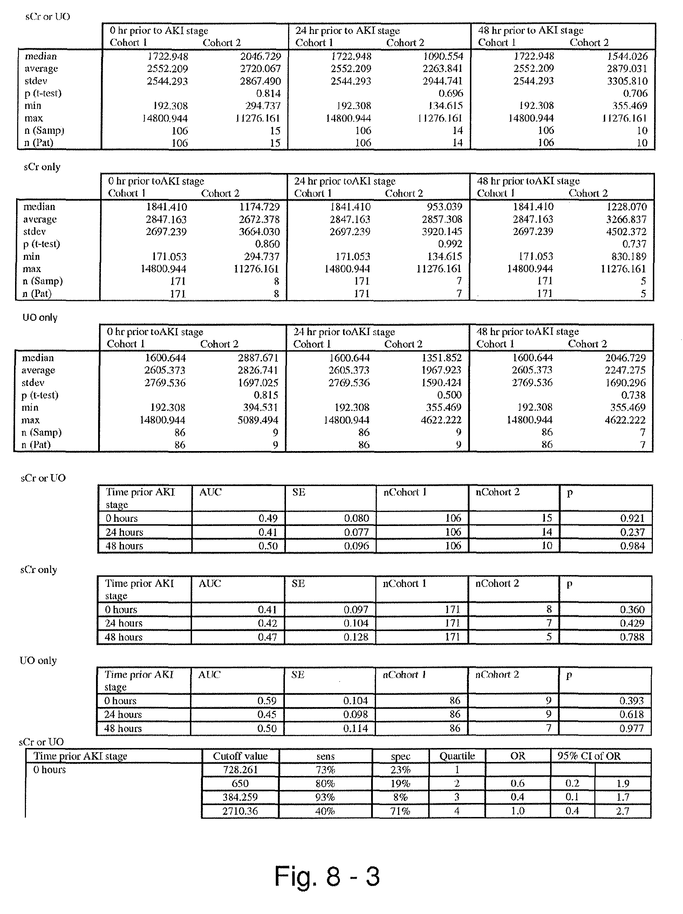

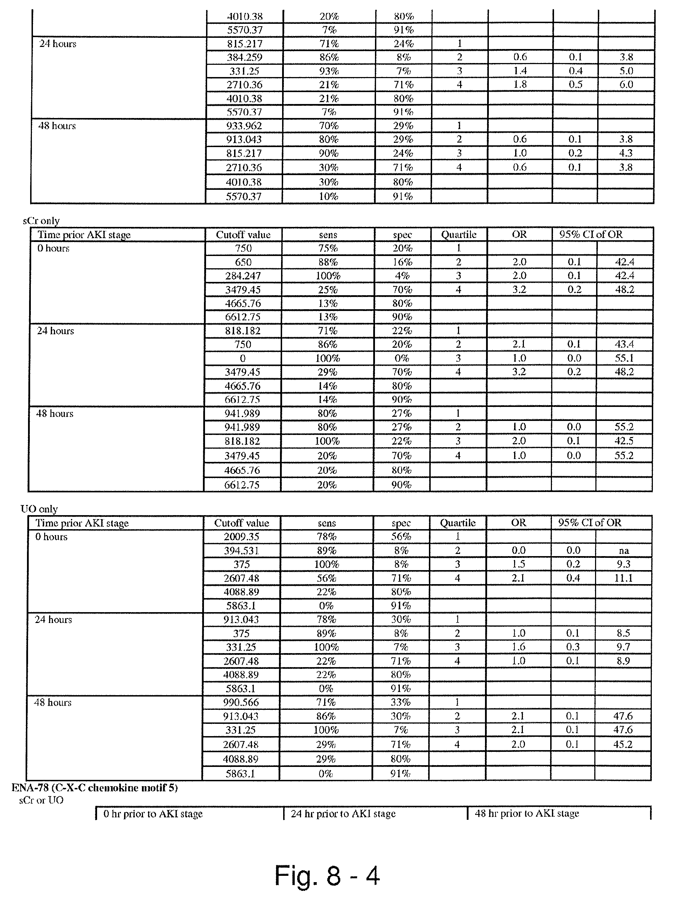

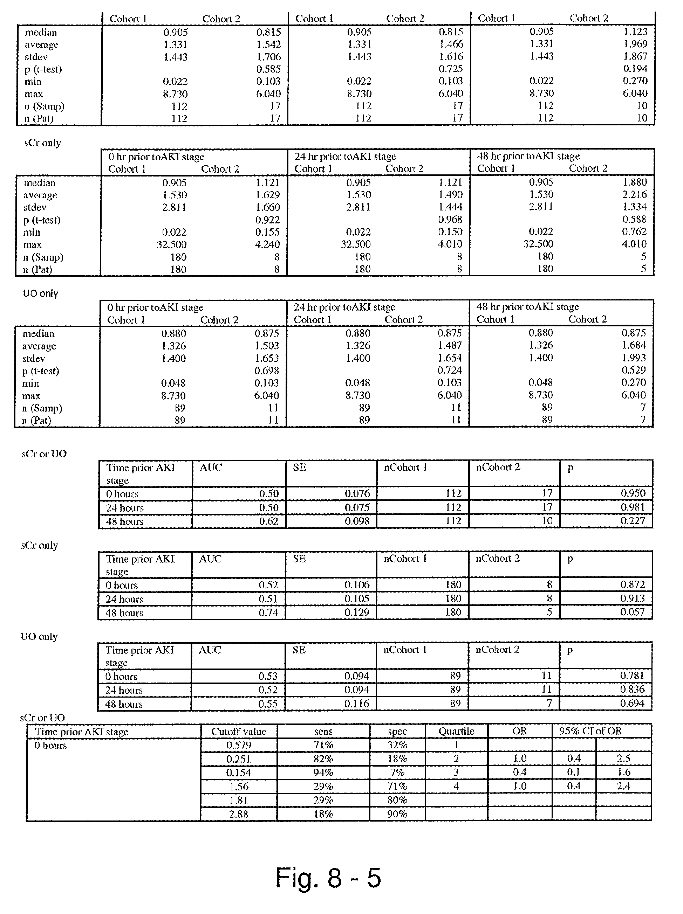

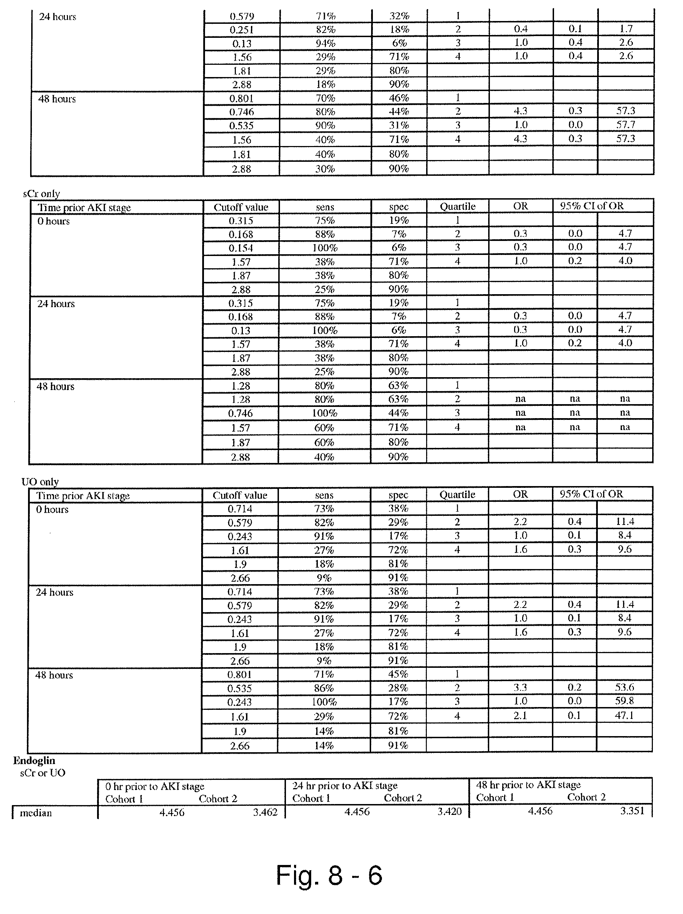

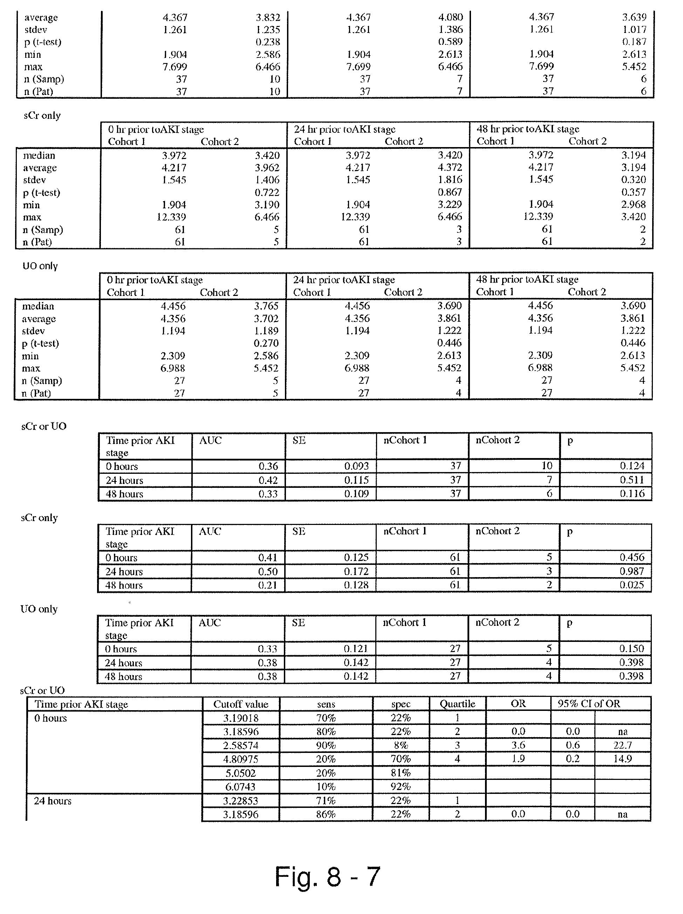

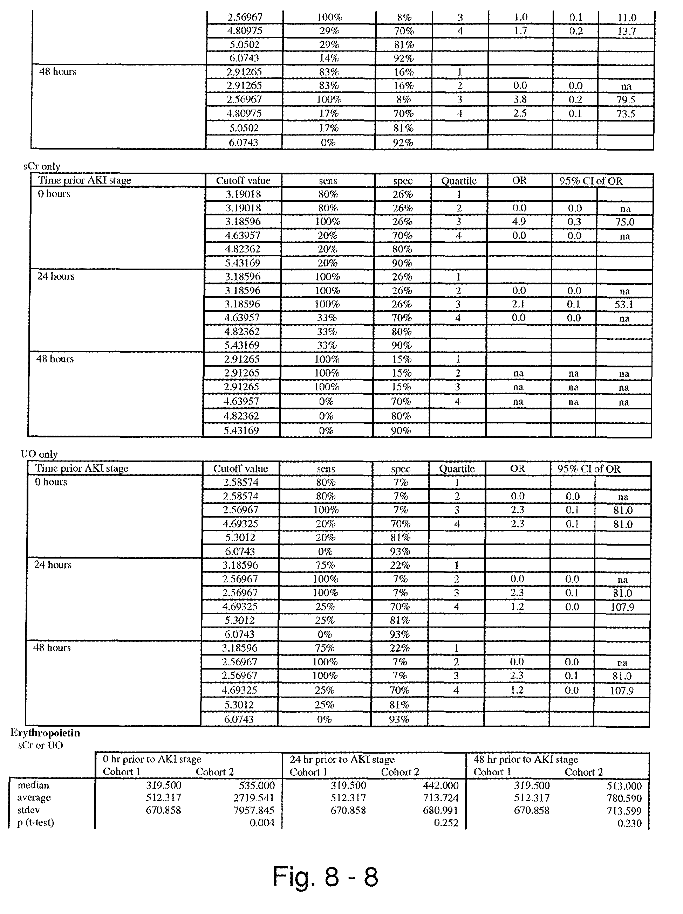

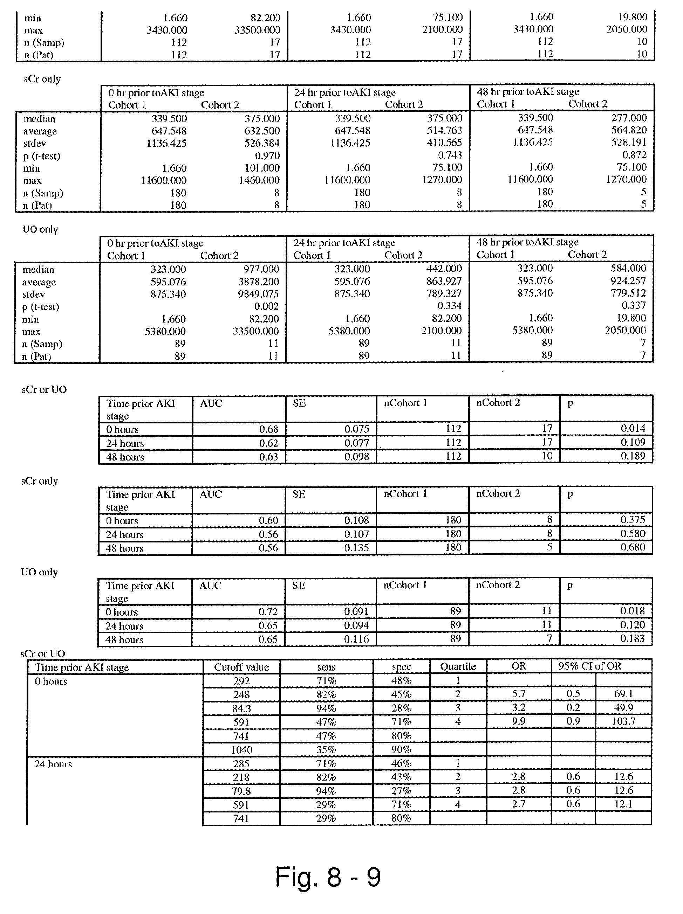

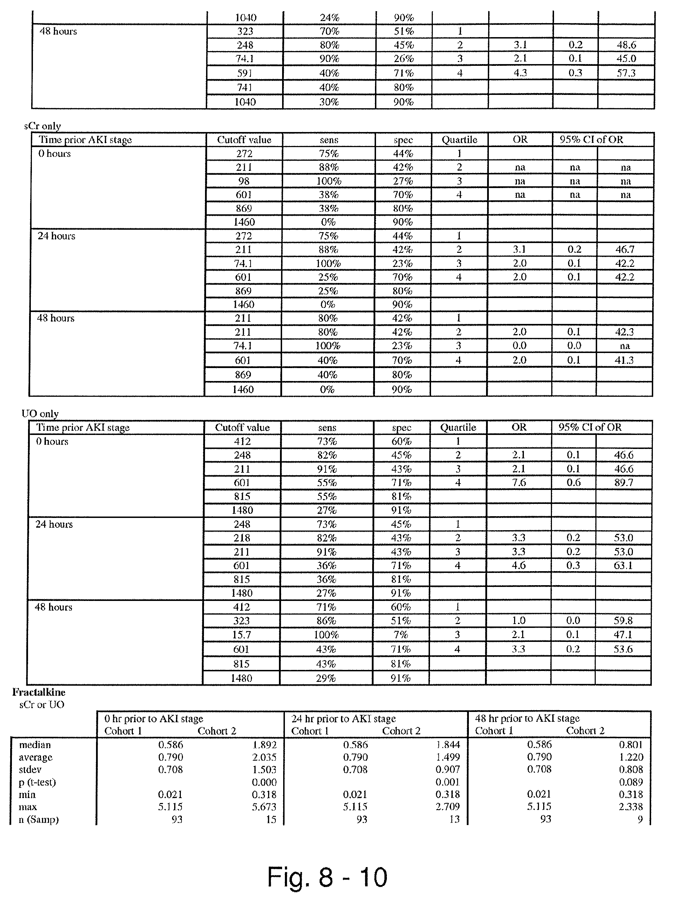

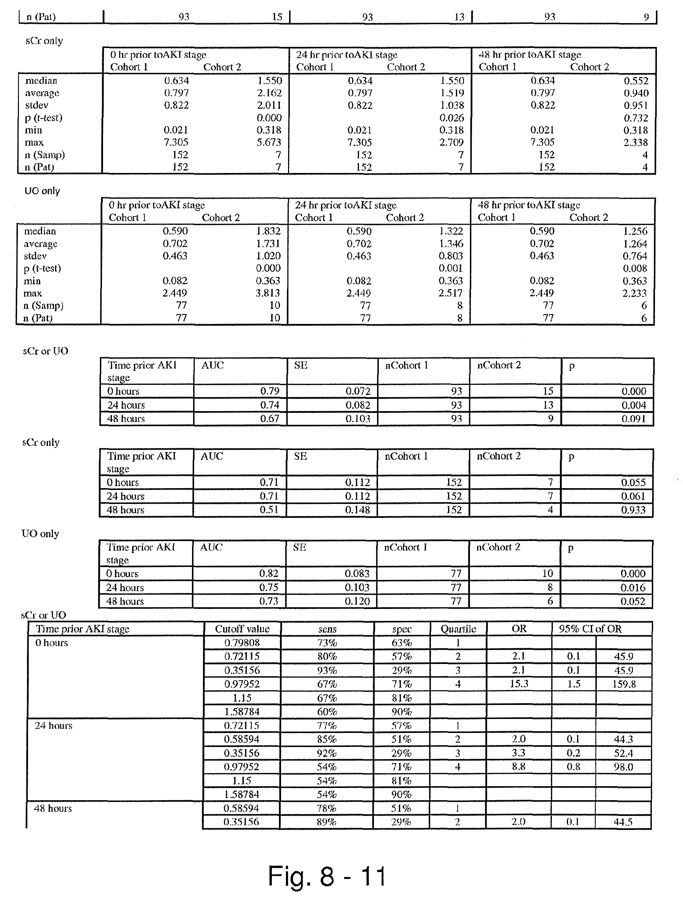

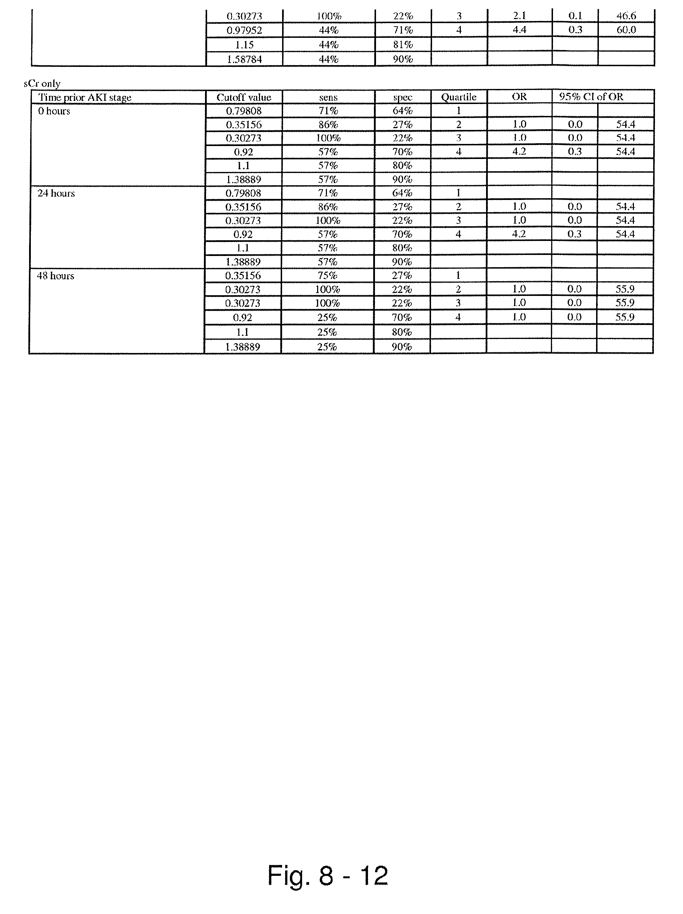

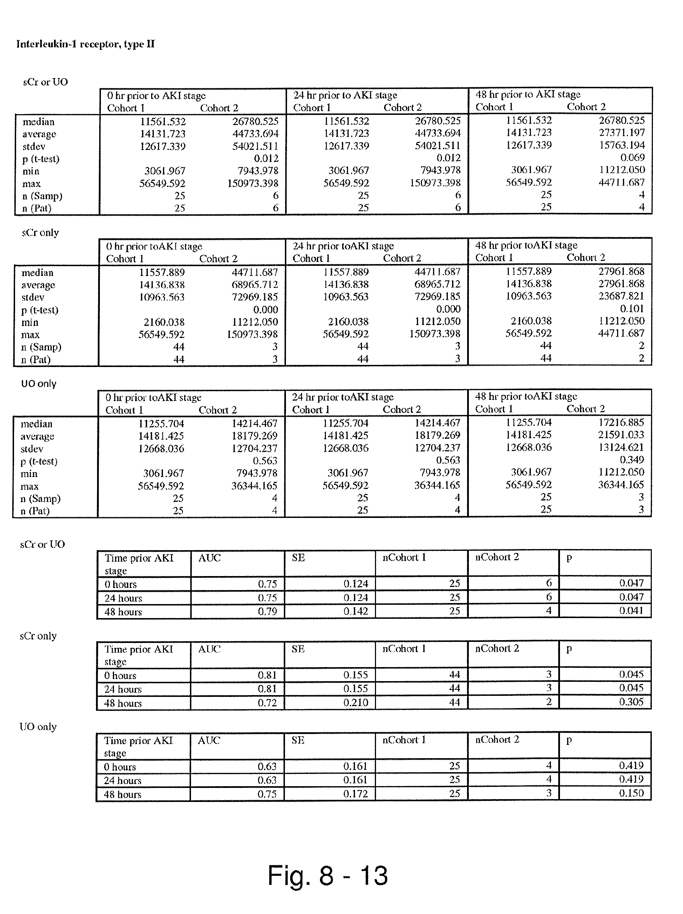

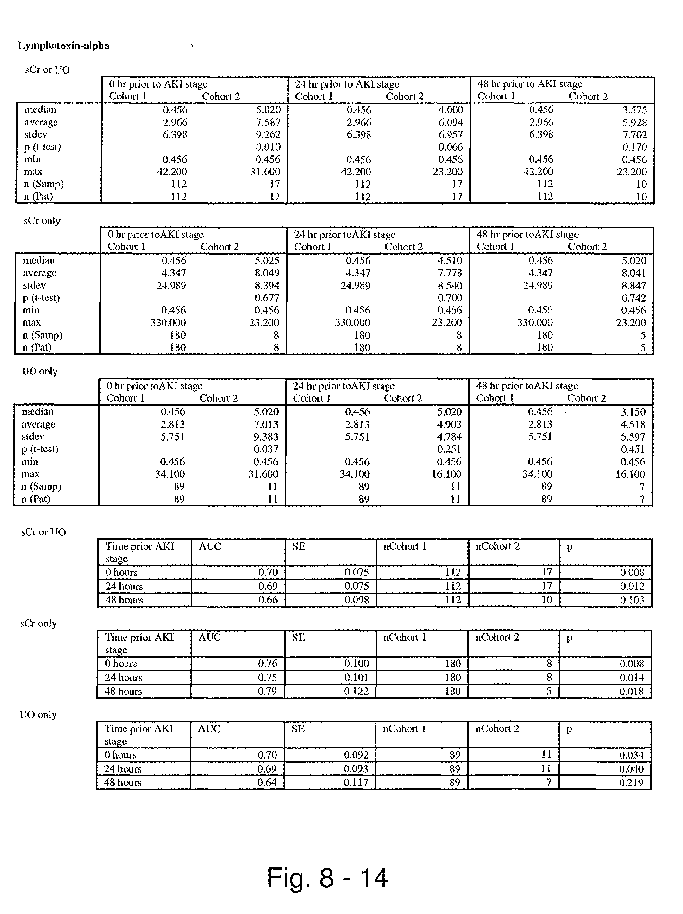

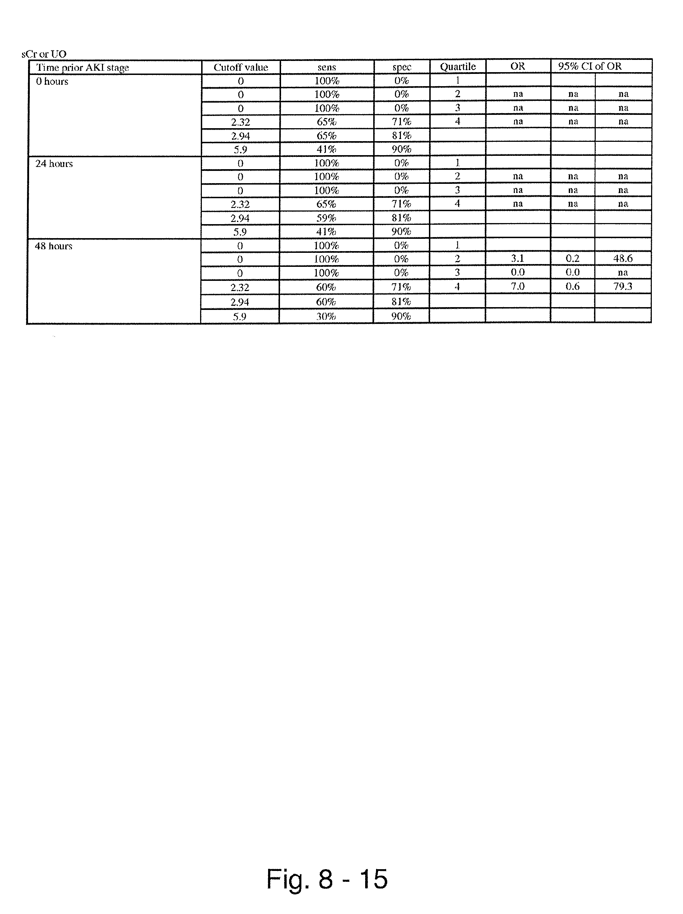

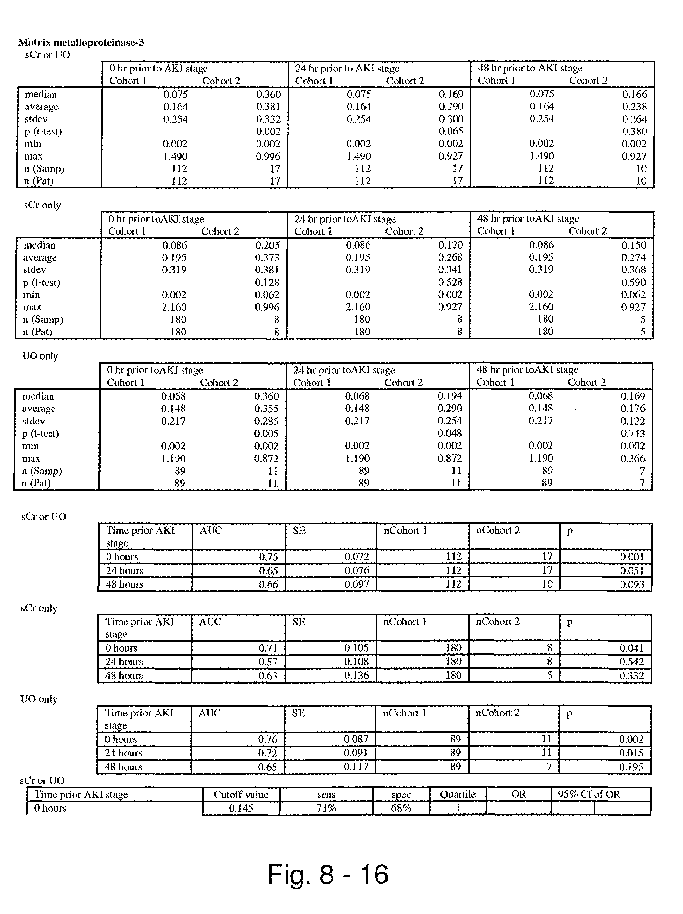

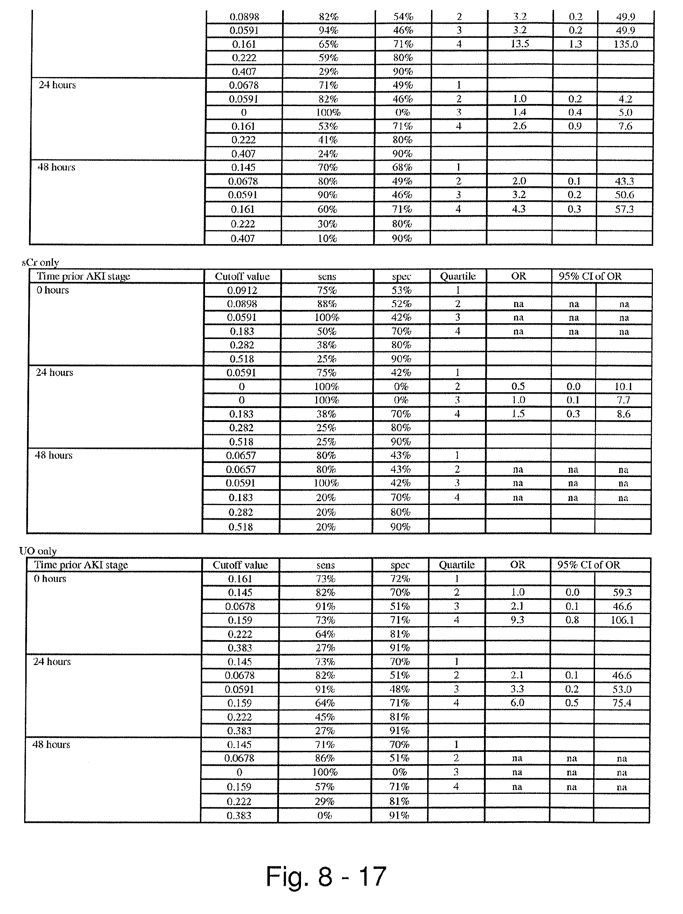

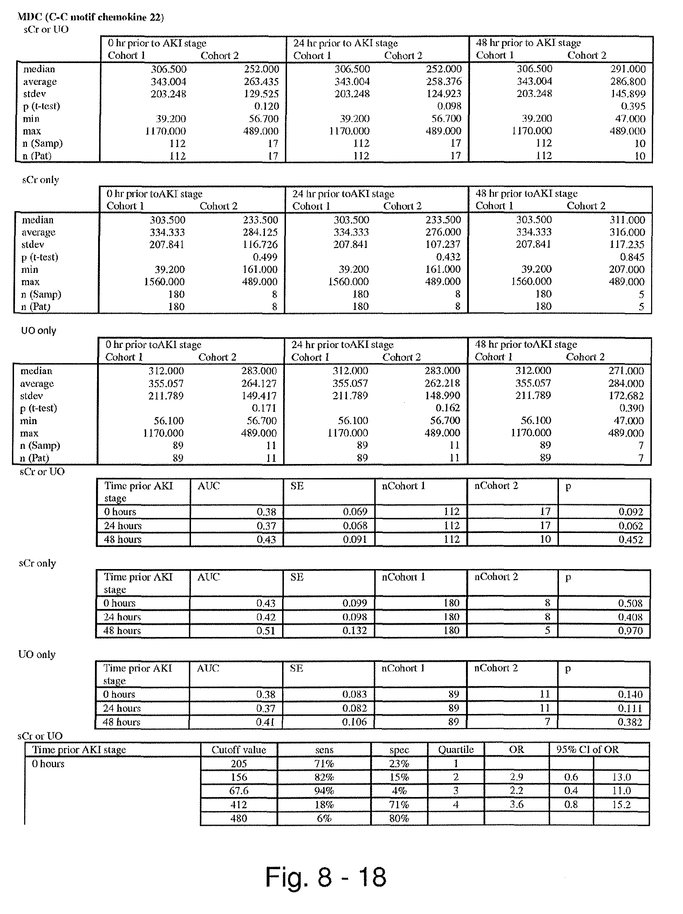

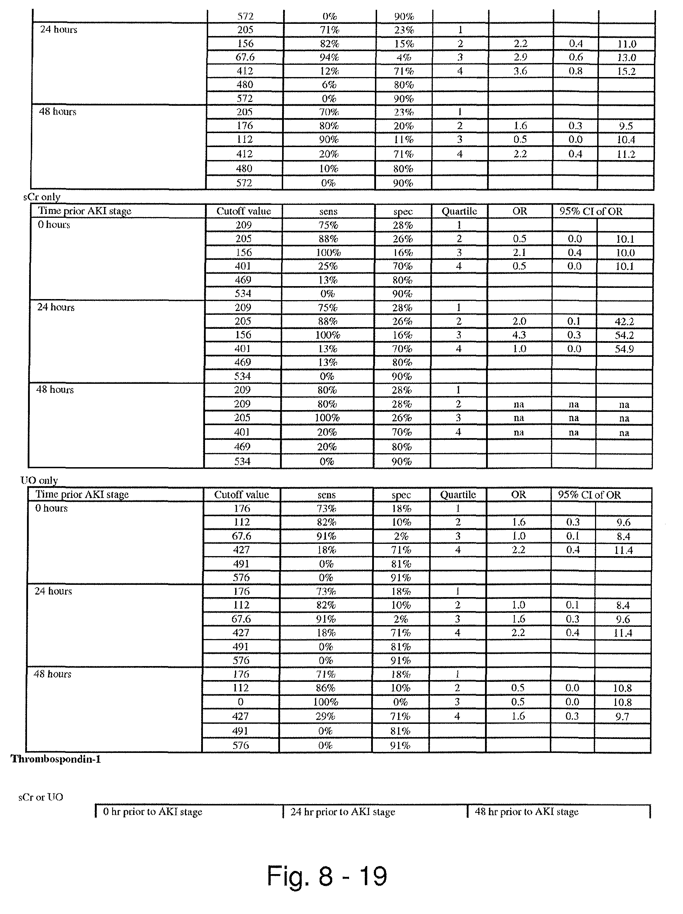

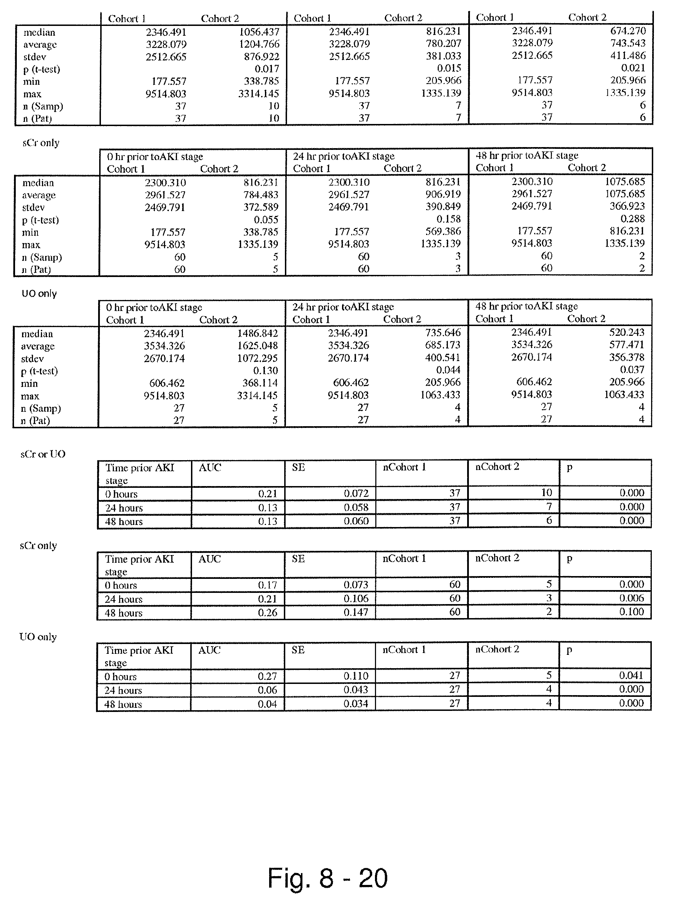

Fig. 8 provides data tables determined in accordance with Example 9 for the comparison of marker levels in plasma samples collected for Cohort 1 (patients that did not progress beyond RIFLE stage 0) and in plasma samples collected from subjects at 0, 24 hours, and 48 hours prior to reaching stage F in Cohort 2. Tables provide descriptive statistics, AUC analysis, and sensitivity, specificity and odds ratio calculations at various threshold (cutoff) levels for the various markers.

## DETAILED DESCRIPTION OF THE INVENTION

The present invention relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of one or more markers selected from the group consisting of soluble CD44 antigen, Angiopoietin-1, soluble Angiopoietin-1 receptor , C-X-C chemokine motif 5, soluble Endoglin, soluble Tumor-associated calcium signal transducer 1, Erythropoietin, soluble Fractalkine, Heme oxygenase 1, soluble Interleukin-1 receptor type II, soluble Interleukin-6 receptor subunit-alpha, Lymphotactin, Lymphotoxin-alpha, Stromelysin-1, C-C motif chemokine 22, C-C motif chemokine 5, and Thrombospondin-1, or one or more markers related thereto, are correlated to the renal status of the subject.
For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq 8.8$ $\mu$mol/L), a percentage increase in serum creatinine of greater than or equal to 20% (1.2-fold from baseline), or a reduction in urine output (documented oliguria of less than 0. 5 ml/kg per hour).
As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq 26.4$ $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."
In this regard, the skilled artisan will understand that the signals obtained from an immunoassay are a direct result of complexes formed between one or more antibodies and the target biomolecule (*i.e.*, the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.)* and nucleic acid measurements (mRNA quatitation). This list is not meant to be limiting.
As used herein, the term "CD44 antigen" refers to one or more polypeptides present in a biological sample that are derived from the CD44 antigen precursor (Swiss-Prot P01670 (SEQ ID NO: 1)).

MDKFWWHAAW GLCLVPLSLA QIDLNITCRF AGVFHVEKNG RYSISRTEAA

DLCKAFNSTL

PTMAQMEKAL SIGFETCRYG FIEGHVVIPR IHPNSICAAN NTGVYILTSN TSQYDTYCFN

ASAPPEEDCT SVTDLPNAFD GPITITIVNR DGTRYVQKGE YRTNPEDIYP SNPTDDDVSS

GSSSERSSTS GGYIFYTFST VHPIPDEDSP WITDSTDRIP ATTLMSTSAT ATETATKRQE

TWDWFSWLFL PSESKNHLHT TTQMAGTSSN TISAGWEPNE ENEDERDRHL SFSGSGIDDD

EDFISSTIST TPRAFDHTKQ NQDWTQWNPS HSNPEVLLQT TTRMTDVDRN GTTAYEGNWN

PEAHPPLIHH EHHEEEETPH STSTIQATPS STTEETATQK EQWFGNRWHE GYRQTPREDS

HSTTGTAAAS AHTSHPMQGR TTPSPEDSSW TDFFNPISHP MGRGHQAGRR MDMDSSHSTT

LQPTANPNTG LVEDLDRTGP LSMTTQQSNS QSFSTSHEGL EEDKDHPTTS TLTSSNRNDV

TGGRRDPNHS EGSTTLLEGY TSHYPHTKES RTFIPVTSAK TGSFGVTAVT VGDSNSNVNR

SLSGDQDTFH PSGGSHTTHG SESDGHSHGS QEGGANTTSG PIRTPQIPEW LIILASLLAL

ALILAVCIAV NSRRRCGQKK KLVINSGNGA VEDRKPSGLN GEASKSQEMV HLVNKESSET

PDQFMTADET RNLQNVDMKI GV

Most preferably, the CD44 antigen assay detects one or more soluble forms of CD44 antigen. CD44 antigen is a single-pass type I membrane protein having a large extracellular domain, most or all of which is present in soluble forms of CD44 antigen generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in CD44 antigen:

| Residues | Length | Domain ID |
|----------|--------|-----------|
| 1-20 | 20 | signal sequence |
| 21-742 | 722 | CD44 antigen |
| 21-649 | 629 | extracellular |
| 650-670 | 21 | transmembrane |
| 671-742 | 72 | cytoplasmic |

In addition, 16 alternative splice forms of CD44 antigen are known. Each is considered a CD44 antigen for purposes of the present invention, and soluble forms of each of these alternative splice forms is considered a soluble CD44 antigen for purposes of the present invention.

As used herein, the term "Angiopoietin-1" refers to one or more polypeptides present in a biological sample that are derived from the Angiopoietin-1 precursor (Swiss-Prot Q15389 (SEQ ID NO: 2)).

```
               10        20        30        40        50        60

MTVFLSFAFL AAILTHIGCS NQRRSPENSG RRYNRIQHGQ CAYTFILPEH DGNCRESTTD

      70        80        90       100       110       120
QYNTNALQRD APHVEPDFSS QKLQHLEHVM ENYTQWLQKL ENYIVENMKS
EMAQIQQNAV

     130       140       150       160       170       180
QNHTATMLEI GTSLLSQTAE QTRKLTDVET QVLNQTSRLE IQLLENSLST YKLEKQLLQQ

     190       200       210       220       230       240
TNEILKIHEK NSLLEHKILE MEGKHKEELD TLKEEKENLQ GLVTRQTYII QELEKQLNRA

     250       260       270       280       290       300
TTNNSVLQKQ QLELMDTVHN LVNLCTKEGV LLKGGKREEE KPFRDCADVY
QAGFNKSGIY

     310       320       330       340       350       360
TIYINNMPEP KKVFCNMDVN GGGWTVIQHR EDGSLDFQRG WKEYKMGFGN
PSGEYWLGNE

     370       380       390       400       410       420
FIFAITSQRQ YMLRIELMDW EGNRAYSQYD RFHIGNEKQN YRLYLKGHTG TAGKQSSLIL

     430       440       450       460       470       480
HGADFSTKDA DNDNCMCKCA LMLTGGWWFD ACGPSNLNGM FYTAGQNHGK
LNGIKWHYFK

     490
GPSYSLRSTT MMIRPLDF
```

The following domains have been identified in Angiopoietin-1:

| Residues | Length | Domain ID |
|----------|--------|-----------|
| 1-15 | 15 | Signal peptide |
| 16-498 | 483 | Angiopoietin-1 |

As used herein, the term "Angiopoietin-1 receptor" refers to one or more polypeptides present in a biological sample that are derived from the Angiopoietin-1 receptor precursor (Swiss-Prot Q02763 (SEQ ID NO: 3)).

```
         10        20        30        40        50        60
MDSLASLVLC GVSLLLSGTV EGAMDLILIN SLPLVSDAET SLTCIASGWR PHEPITIGRD

         70        80        90       100       110       120
FEALMNQHQD PLEVTQDVTR EWAKKVVWKR EKASKINGAY FCEGRVRGEA
IRIRTMKMRQ

        130       140       150       160       170       180
QASFLPATLT MTVDKGDNVN ISFKKVLIKE EDAVIYKNGS FIHSVPRHEV PDILEVHLPH

        190       200       210       220       230       240
```

AQPQDAGVYS ARYIGGNLFT SAFTRLIVRR CEAQKWGPEC NHLCTACMNN
GVCHEDTGEC

ICPPGFMGRT CEKACELHTF GRTCKERCSG QEGCKSYVFC LPDPYGCSCA TGWKGLQCNE

ACHPGFYGPD CKLRCSCNNG EMCDRFQGCL CSPGWQGLQC EREGIPRMTP KIVDLPDHIE

VNSGKFNPIC KASGWPLPTN EEMTLVKPDG TVLHPKDFNH TDHFSVAIFT IHRILPPDSG

VWVCSVNTVA GMVEKPFNIS VKVLPKPLNA PNVIDTGHNF AVINISSEPY FGDGPIKSKK

LLYKPVNHYE AWQHIQVTNE IVTLNYLEPR TEYELCVQLV RRGEGGEGHP GPVRRFTTAS

IGLPPPRGLN LLPKSQTTLN LTWQPIFPSS EDDFYVEVER RSVQKSDQQN IKVPGNLTSV

LLNNLHPREQ YVVRARVNTK AQGEWSEDLT AWTLSDILPP QPENIKISNI THSSAVISWT

ILDGYSISSI TIRYKVQGKN EDQHVDVKIK NATIIQYQLK GLEPETAYQV DIFAENNIGS

SNPAFSHELV TLPESQAPAD LGGGKMLLIA ILGSAGMTCL TVLLAFLIIL QLKRANVQRR

MAQAFQNVRE EPAVQFNSGT LALNRKVKNN PDPTIYPVLD WNDIKFQDVI
GEGNFGQVLK

ARIKKDGLRM DAAIKRMKEY ASKDDHRDFA GELEVLCKLG HHPNIINLLG
ACEHRGYLYL

AIEYAPHGNL LDFLRKSRVL ETDPAFAIAN STASTLSSQQ LLHFAADVAR GMDYLSQKQF

IHRDLAARNI LVGENYVAKI ADFGLSRGQE VYVKKTMGRL PVRWMAIESL
NYSVYTTNSD

VWSYGVLLWE IVSLGGTPYC GMTCAELYEK LPQGYRLEKP LNCDDEVYDL
MRQCWREKPY

ERPSFAQILV SLNRMLEERK TYVNTTLYEK FTYAGIDCSA EEAA

Most preferably, the Angiopoietin-1 receptor assay detects one or more soluble forms of Angiopoietin-1 receptor.

Angiopoietin-1 receptor is a single-pass type I membrane protein having a large extracellular domain, most or all of which is present in soluble forms of Angiopoietin-1 receptor generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Angiopoietin-1 receptor:

| Residues | Length | Domain ID |
|----------|--------|-----------|
| 1-22 | 22 | signal sequence |
| 23-1124 | 1102 | Angiopoietin-1 receptor |
| 23-745 | 723 | extracellular |
| 746-770 | 25 | transmembrane |
| 771-1124 | 354 | cytoplasmic |

As used herein, the term "C-X-C motif chemokine 5" refers to one or more polypeptides present in a biological sample that are derived from the C-X-C motif chemokine 5 precursor (Swiss-Prot P42830 (SEQ ID NO: 4)).

```
         10         20         30         40         50         60
     MSLLSSRAAR VPGPSSSLCA LLVLLLLLTQ PGPIASAGPA AAVLRELRCV CLQTTQGVHP


         70         80         90        100        110
     KMISNLQVFA IGPQCSKVEV VASLKNGKEI CLDPEAPFLK KVIQKILDGG NKEN
```

The following domains have been identified in C-X-C motif chemokine 5:

| Residues | Length | Domain ID |
|----------|--------|-----------|
| 1-36 | 36 | Signal peptide |
| 37-114 | 78 | C-X-C motif chemokine 5 |

In addition, cleaved forms representing residues 44-114 and 45-114 are produced by proteolytic cleavage after secretion from peripheral blood monocytes.

As used herein, the term "Endoglin" refers to one or more polypeptides present in a biological sample that are derived from the Endoglin precursor (Swiss-Prot P17813 (SEQ ID NO: 5)).

```
         10         20         30         40         50         60
     MDRGTLPLAV ALLLASCSLS PTSLAETVHC DLQPVGPERG EVTYTTSQVS KGCVAQAPNA


         70         80         90        100        110        120
     ILEVHVLFLE FPTGPSQLEL TLQASKQNGT WPREVLLVLS VNSSVFLHLQ ALGIPLHLAY


        130        140        150        160        170        180
     NSSLVTFQEP PGVNTTELPS FPKTQILEWA AERGPITSAA ELNDPQSILL RLGQAQGSLS


        190        200        210        220        230        240
```

FCMLEASQDM GRTLEWRPRT PALVRGCHLE GVAGHKEAHI LRVLPGHSAG
PRTVTVKVEL

```
        250       260       270       280       290       300
SCAPGDLDAV LILQGPPYVS WLIDANHNMQ IWTTGEYSFK IFPEKNIRGF KLPDTPQGLL
```

```
        310       320       330       340       350       360
GEARMLNASI VASFVELPLA SIVSLHASSC GGRLQTSPAP IQTTPPKDTC SPELLMSLIQ
```

```
        370       380       390       400       410       420
TKCADDAMTL VLKKELVAHL KCTITGLTFW DPSCEAEDRG DKFVLRSAYS
SCGMQVSASM
```

```
        430       440       450       460       470       480
ISNEAVVNIL SSSSPQRKKV HCLNMDSLSF QLGLYLSPHF LQASNTIEPG QQSFVQVRVS
```

```
        490       500       510       520       530       540
PSVSEFLLQL DSCHLDLGPE GGTVELIQGR AAKGNCVSLL SPSPEGDPRF SFLLHFYTVP
```

```
        550       560       570       580       590       600
IPKTGTLSCT VALRPKTGSQ DQEVHRTVFM RLNIISPDLS GCTSKGLVLP AVLGITFGAF
```

```
        610       620       630       640       650
LIGALLTAAL WYIYSHTRSP SKREPVVAVA APASSESSST NHSIGSTQST PCSTSSMA
```

Most preferably, the Endoglin assay detects one or more soluble forms of Endoglin. Endoglin is a single-pass type I membrane protein having a large extracellular domain, most or all of which is present in soluble forms of Endoglin generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Endoglin:

| Residues | Length | Domain ID |
|---|---|---|
| 1-25 | 25 | signal sequence |
| 26-658 | 633 | Endoglin |
| 26-586 | 561 | extracellular |
| 587-611 | 25 | transmembrane |
| 612-658 | 47 | cytoplasmic |

As used herein, the term "Tumor-associated calcium signal transducer 1" refers to one or more polypeptides present in a biological sample that are derived from the Tumor-associated calcium signal transducer 1 precursor (Swiss-Prot P16422 (SEQ ID NO: 6)).

```
            10        20        30        40        50        60
```

MAPPQVLAFG LLLAAATATF AAAQEECVCE NYKLAVNCFV NNNRQCQCTS VGAQNTVICS

          70        80        90       100       110       120
KLAAKCLVMK AEMNGSKLGR RAKPEGALQN NDGLYDPDCD ESGLFKAKQC NGTSMCWCVN

         130       140       150       160       170       180
TAGVRRTDKD TEITCSERVR TYWIIIELKH KAREKPYDSK SLRTALQKEI TTRYQLDPKF

         190       200       210       220       230       240
ITSILYENNV ITIDLVQNSS QKTQNDVDIA DVAYYFEKDV KGESLFHSKK MDLTVNGEQL

         250       260       270       280       290       300
DLDPGQTLIY YVDEKAPEFS MQGLKAGVIA VIVVVVIAVV AGIVVLVISR KKRMAKYEKA

         310
EIKEMGEMHR ELNA

Most preferably, the Tumor-associated calcium signal transducer 1 assay detects one or more soluble forms of Tumor-associated calcium signal transducer 1. Tumor-associated calcium signal transducer 1 is a single-pass type I membrane protein having a large extracellular domain, most or all of which is present in soluble forms of Tumor-associated calcium signal transducer 1 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Tumor-associated calcium signal transducer 1:

| Residues | Length | Domain ID |
|---|---|---|
| 1-23 | 23 | signal sequence |
| 24-314 | 291 | Tumor-associated calcium signal transducer 1 |
| 24-265 | 242 | extracellular |
| 266-288 | 23 | transmembrane |
| 289-314 | 26 | cytoplasmic |

As used herein, the term "Erythropoietin" refers to one or more polypeptides present in a biological sample that are derived from the Erythropoietin precursor (Swiss-Prot P01588 (SEQ ID NO: 7)).

          10        20        30        40        50        60
MGVHECPAWL WLLLSLLSLP LGLPVLGAPP RLICDSRVLE RYLLEAKEAE NITTGCAEHC

          70        80        90       100       110       120

SLNENITVPD TKVNFYAWKR MEVGQQAVEV WQGLALLSEA VLRGQALLVN SSQPWEPLQL

         130       140       150       160       170       180
HVDKAVSGLR SLTTLLRALG AQKEAISPPD AASAAPLRTI TADTFRKLFR VYSNFLRGKL

         190
KLYTGEACRT GDR

The following domains have been identified in Erythropoietin:

| Residues | Length | Domain ID |
|---|---|---|
| 1-27 | 27 | Signal peptide |
| 28-193 | 166 | Erythropoietin |
| 190-193 | 4 | Propeptide |
| 193-193 | 1 | Propeptide |

As used herein, the term "fractalkine" refers to one or more polypeptides present in a biological sample that are derived from the fractalkine precursor (Swiss-Prot P78423 (SEQ ID NO: 8)).

    10     20     30     40     50     60

MAPISLSWLL RLATFCHLTV LLAGQHHGVT KCNITCSKMT SKIPVALLIH YQQNQASCGK

    70     80     90    100   110   120

RAIILETRQH RLFCADPKEQ WVKDAMQHLD RQAAALTRNG GTFEKQIGEV

KPRTTPAAGG

  130   140   150   160   170   180

MDESVVLEPE ATGESSSLEP TPSSQEAQRA LGTSPELPTG VTGSSGTRLP PTPKAQDGGP

  190   200   210   220   230   240

VGTELFRVPP VSTAATWQSS APHQPGPSLW AEAKTSEAPS TQDPSTQAST ASSPAPEENA

  250   260   270   280   290   300

PSEGQRVWGQ GQSPRPENSL EREEMGPVPA HTDAFQDWGP GSMAHVSVVP

VSSEGTPSRE

  310   320   330   340   350   360

PVASGSWTPK AEEPIHATMD PQRLGVLITP VPDAQAATRR QAVGLLAFLG LLFCLGVAMF

  370   380   390

TYQSLQGCPR KMAGEMAEGL RYIPRSCGSN SYVLVPV

Most preferably, the fractalkine assay detects one or more soluble forms of fractalkine. Fractalkine is a single-pass type I membrane protein having a large extracellular domain, most or all of which is present in soluble forms of fractalkine generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in fractalkine:

| Residues | Length | Domain ID |
|---|---|---|
| 1-24 | 24 | signal sequence |
| 25-397 | 373 | fractalkine |
| 25-341 | 317 | extracellular |
| 342-362 | 21 | transmembrane |

(continued)

| Residues | Length | Domain ID |
|----------|--------|-------------|
| 363-397 | 35 | cytoplasmic |

As used herein, the term "Heme oxygenase 1" refers to one or more polypeptides present in a biological sample that are derived from the Heme oxygenase 1 precursor (Swiss-Prot P09601 (SEQ ID NO: 9)).

```
          10        20        30        40        50        60
MERPQPDSMP QDLSEALKEA TKEVHTQAEN AEFMRNFQKG QVTRDGFKLV
MASLYHIYVA

          70        80        90       100       110       120
LEEEIERNKE SPVFAPVYFP EELHRKAALE QDLAFWYGPR WQEVIPYTPA MQRYVKRLHE

         130       140       150       160       170       180
VGRTEPELLV AHAYTRYLGD LSGGQVLKKI AQKALDLPSS GEGLAFFTFP NIASATKFKQ

         190       200       210       220       230       240
LYRSRMNSLE MTPAVRQRVI EEAKTAFLLN IQLFEELQEL LTHDTKDQSP SRAPGLRQRA

         250       260       270       280
SNKVQDSAPV ETPRGKPPLN TRSQAPLLRW VLTLSFLVAT VAVGLYAM
```

The following domains have been identified in Heme oxygenase 1:

| Residues | Length | Domain ID |
|----------|--------|------------------|
| 1-288 | 288 | Heme oxygenase 1 |
| 25 | 1 | heme axial ligand |

As used herein, the term "interleukin-1 receptor type II" refers to one or more polypeptides present in a biological sample that are derived from the interleukin-1 receptor type II precursor (Swiss-Prot P27930 (SEQ ID NO: 10)).

MLRLYVLVMG VSAFTLQPAA HTGAARSCRF RGRHYKREFR LEGEPVALRC PQVPYWLWAS

VSPRINLTWH KNDSARTVPG EEETRMWAQD GALWLLPALQ EDSGTYVCTT RNASYCDKMS

IELRVFENTD AFLPFISYPQ ILTLSTSGVL VCPDLSEFTR DKTDVKIQWY KDSLLLDKDN

EKFLSVRGTT HLLVHDVALE DAGYYRCVLT FAHEGQQYNI TRSIELRIKK KKEETIPVII

SPLKTISASL GSRLTIPCKV FLGTGTPLTT MLWWTANDTH IESAYPGGRV TEGPRQEYSE

NNENYIEVPL IFDPVTREDL HMDFKCVVHN TLSFQTLRTT VKEASSTFSW GIVLAPLSLA

FLVLGGIWMH RRCKHRTGKA DGLTVLWPHH QDFQSYPK

Most preferably, the interleukin-1 receptor type II assay detects one or more soluble forms of interleukin-1 receptor type II. Interleukin-1 receptor type II is a single-pass type I membrane protein having a large extracellular domain, most or all of which is present in soluble forms of interleukin-1 receptor type II generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in interleukin-1 receptor type II:

| Residues | Length | Domain ID |
|---|---|---|
| 1-13 | 13 | signal sequence |
| 14-398 | 385 | interleukin-1 receptor type II |
| 14-343 | 330 | extracellular |
| 344-369 | 26 | transmembrane |
| 370-398 | 29 | cytoplasmic |

As used herein, the term "Interleukin-6 receptor subunit alpha" refers to one or more polypeptides present in a biological sample that are derived from the Interleukin-6 receptor subunit alpha precursor (Swiss-Prot P08887 (SEQ ID NO: 11)).

MLAVGCALLA ALLAAPGAAL APRRCPAQEV ARGVLTSLPG DSVTLTCPGV EPEDNATVHW

VLRKPAAGSH PSRWAGMGRR LLLRSVQLHD SGNYSCYRAG RPAGTVHLLV DVPPEEPQLS

CFRKSPLSNV VCEWGPRSTP SLTTKAVLLV RKFQNSPAED FQEPCQYSQE SQKFSCQLAV

PEGDSSFYIV SMCVASSVGS KFSKTQTFQG CGILQPDPPA NITVTAVARN PRWLSVTWQD

PHSWNSSFYR LRFELRYRAE RSKTFTTWMV KDLQHHCVIH DAWSGLRHVV QLRAQEEFGQ

GEWSEWSPEA MGTPWTESRS PPAENEVSTP MQALTTNKDD DNILFRDSAN ATSLPVQDSS

SVPLPTFLVA GGSLAFGTLL CIAIVLRFKK TWKLRALKEG KTSMHPPYSL GQLVPERPRP

TPVLVPLISP PVSPSSLGSD NTSSHNRPDA RDPRSPYDIS NTDYFFPR

Most preferably, the Interleukin-6 receptor subunit alpha assay detects one or more soluble forms of Interleukin-6 receptor subunit alpha. Interleukin-6 receptor subunit alpha is a single-pass type I membrane protein having a large extracellular domain, most or all of which is present in soluble forms of Interleukin-6 receptor subunit alpha generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Interleukin-6 receptor subunit alpha:

| Residues | Length | Domain ID |
|---|---|---|
| 1-19 | 19 | signal sequence |
| 20-468 | 449 | Interleukin-6 receptor subunit alpha |
| 20-365 | 346 | extracellular |
| 366-386 | 21 | transmembrane |
| 387-468 | 82 | cytoplasmic |

As used herein, the term "Lymphotactin" refers to one or more polypeptides present in a biological sample that are derived from the Lymphotactin precursor (Swiss-Prot P47992 (SEQ ID NO: 12)).

MRLLILALLG ICSLTAYIVE GVGSEVSDKR TCVSLTTQRL PVSRIKTYTI TEGSLRAVIF

ITKRGLKVCA DPQATWVRDV VRSMDRKSNT RNNMIQTKPT GTQQSTNTAV TLTG

The following domains have been identified in Lymphotactin:

| Residues | Length | Domain ID |
|----------|--------|-----------|
| 1-21 | 21 | Signal peptide |
| 22-114 | 93 | Lymphotactin |

As used herein, the term "Lymphotoxin-alpha" refers to one or more polypeptides present in a biological sample that are derived from the Lymphotoxin-alpha precursor (Swiss-Prot P01374 (SEQ ID NO: 13)).

```
        10        20        30        40        50        60
MTPPERLFLP RVCGTTLHLL LLGLLLVLLP GAQGLPGVGL TPSAAQTARQ HPKMHLAHST

        70        80        90       100       110       120
LKPAAHLIGD PSKQNSLLWR ANTDRAFLQD GFSLSNNSLL VPTSGIYFVY SQVVFSGKAY

       130       140       150       160       170       180
SPKATSSPLY LAHEVQLFSS QYPFHVPLLS SQKMVYPGLQ EPWLHSMYHG AAFQLTQGDQ

       190       200
LSTHTDGIPH LVLSPSTVFF GAFAL
```

The following domains have been identified in Lymphotoxin-alpha:

| Residues | Length | Domain ID |
|----------|--------|-----------|
| 1-34 | 34 | Signal peptide |
| 35-205 | 171 | Lymphotoxin-alpha |

As used herein, the term "Stromelysin-1" refers to one or more polypeptides present in a biological sample that are derived from the Stromelysin-1 precursor (Swiss-Prot P08254 (SEQ ID NO: 14)).

```
        10        20        30        40        50        60
```

MKSLPILLLL CVAVCSAYPL DGAARGEDTS MNLVQKYLEN YYDLKKDVKQ
FVRRKDSGPV

      70      80      90     100    110    120
VKKIREMQKF LGLEVTGKLD SDTLEVMRKP RCGVPDVGHF RTFPGIPKWR KTHLTYRIVN

    130   140   150   160   170   180
YTPDLPKDAV DSAVEKALKV WEEVTPLTFS RLYEGEADIM ISFAVREHGD FYPFDGPGNV

    190   200   210   220   230   240
LAHAYAPGPG INGDAHFDDD EQWTKDTTGT NLFLVAAHEI GHSLGLFHSA
NTEALMYPLY

    250   260   270   280   290   300
HSLTDLTRFR LSQDDINGIQ SLYGPPPDSP ETPLVPTEPV PPEPGTPANC DPALSFDAVS

    310   320   330   340   350   360
TLRGEILIFK DRHFWRKSLR KLEPELHLIS SFWPSLPSGV DAAYEVTSKD LVFIFKGNQF

    370   380   390   400   410   420
WAIRGNEVRA GYPRGIHTLG FPPTVRKIDA AISDKEKNKT YFFVEDKYWR FDEKRNSMEP

    430   440   450   460   470
GFPKQIAEDF PGIDSKIDAV FEEFGFFYFF TGSSQLEFDP NAKKVTHTLK SNSWLNC

The following domains have been identified in Stromelysin-1:

| Residues | Length | Domain ID |
|---|---|---|
| 1-17 | 17 | signal sequence |
| 18-99 | 82 | propeptide |
| 100-477 | 378 | Stromelysin-1 |

As used herein, the term "C-C motif chemokine 22" refers to one or more polypeptides present in a biological sample that are derived from the C-C motif chemokine 22 precursor (Swiss-Prot 000626 (SEQ ID NO: 15)).

    10    20    30    40    50    60
MARLQTALLV VLVLLAVALQ ATEAGPYGAN MEDSVCCRDY VRYRLPLRVV
KHFYWTSDSC

    70    80    90
PRPGVVLLTF RDKEICADPR VPWVKMILNK LSQ

The following domains have been identified in C-C motif chemokine 22:

| Residues | Length | Domain ID |
|---|---|---|
| 1-24 | 24 | Signal peptide |
| 25-93 | 69 | C-C motif chemokine 22 |

As used herein, the term "C-C motif chemokine 5" refers to one or more polypeptides present in a biological sample that are derived from the C-C motif chemokine 5 precursor (Swiss-Prot P13501 (SEQ ID NO: 16)).

```
        10        20        30        40        50        60
MKVSAAALAV ILIATALCAP ASASPYSSDT TPCCFAYIAR PLPRAHIKEY FYTSGKCSNP


        70        80        90
AVVFVTRKNR QVCANPEKKW VREYINSLEM S
```

The following domains have been identified in C-C motif chemokine 5:

| Residues | Length | Domain ID |
|----------|--------|-----------|
| 1-23 | 23 | Signal peptide |
| 24-91 | 68 | C-C motif chemokine 5 |

In addition, forms representing residues 26-91 and 27-91 are reportedly formed by posttranslational cleavage of C-C motif chemokine 5.

As used herein, the term "Thrombospondin-1" refers to one or more polypeptides present in a biological sample that are derived from the Thrombospondin-1 precursor (Swiss-Prot P07996 (SEQ ID NO: 17)).

```
        10        20        30        40        50        60
MGLAWGLGVL FLMHVCGTNR IPESGGDNSV FDIFELTGAA RKGSGRRLVK GPDPSSPAFR


        70        80        90       100       110       120
IEDANLIPPV PDDKFQDLVD AVRAEKGFLL LASLRQMKKT RGTLLALERK DHSGQVFSVV


       130       140       150       160       170       180
SNGKAGTLDL SLTVQGKQHV VSVEEALLAT GQWKSITLFV QEDRAQLYID
CEKMENAELD


       190       200       210       220       230       240
VPIQSVFTRD LASIARLRIA KGGVNDNFQG VLQNVRFVFG TTPEDILRNK GCSSSTSVLL


       250       260       270       280       290       300
TLDNNVVNGS SPAIRTNYIG HKTKDLQAIC GISCDELSSM VLELRGLRTI VTTLQDSIRK


       310       320       330       340       350       360
VTEENKELAN ELRRPPLCYH NGVQYRNNEE WTVDSCTECH CQNSVTICKK VSCPIMPCSN


       370       380       390       400       410       420
ATVPDGECCP RCWPSDSADD GWSPWSEWTS CSTSCGNGIQ QRGRSCDSLN
NRCEGSSVQT


       430       440       450       460       470       480
RTCHIQECDK RFKQDGGWSH WSPWSSCSVT CGDGVITRIR LCNSPSPQMN GKPCEGEARE
```

```
       490       500       510       520       530       540
TKACKKDACP INGGWGPWSP WDICSVTCGG GVQKRSRLCN NPTPQFGGKD
CVGDVTENQI

       550       560       570       580       590       600
CNKQDCPIDG CLSNPCFAGV KCTSYPDGSW KCGACPPGYS GNGIQCTDVD ECKEVPDACF

       610       620       630       640       650       660
NHNGEHRCEN TDPGYNCLPC PPRFTGSQPF GQGVEHATAN KQVCKPRNPC
TDGTHDCNKN

       670       680       690       700       710       720
AKCNYLGHYS DPMYRCECKP GYAGNGIICG EDTDLDGWPN ENLVCVANAT
YHCKKDNCPN

       730       740       750       760       770       780
LPNSGQEDYD KDGIGDACDD DDDNDKIPDD RDNCPFHYNP AQYDYDRDDV
GDRCDNCPYN

       790       800       810       820       830       840
HNPDQADTDN NGEGDACAAD IDGDGILNER DNCQYVYNVD QRDTDMDGVG
DQCDNCPLEH

       850       860       870       880       890       900
NPDQLDSDSD RIGDTCDNNQ DIDEDGHQNN LDNCPYVPNA NQADHDKDGK
GDACDHDDDN

       910       920       930       940       950       960
DGIPDDKDNC RLVPNPDQKD SDGDGRGDAC KDDFDHDSVP DIDDICPENV DISETDFRRF

       970       980       990      1000      1010      1020
QMIPLDPKGT SQNDPNWVVR HQGKELVQTV NCDPGLAVGY DEFNAVDFSG
TFFINTERDD

      1030      1040      1050      1060      1070      1080
DYAGFVFGYQ SSSRFYVVMW KQVTQSYWDT NPTRAQGYSG LSVKVVNSTT
GPGEHLRNAL

      1090      1100      1110      1120      1130      1140
WHTGNTPGQV RTLWHDPRHI GWKDFTAYRW RLSHRPKTGF IRVVMYEGKK
IMADSGPIYD

      1150      1160      1170
KTYAGGRLGL FVFSQEMVFF SDLKYECRDP
```

The following domains have been identified in Thrombospondin-1:

| Residues | Length | Domain ID |
| --- | --- | --- |
| 1-18 | 18 | Signal peptide |
| 19-1170 | 1152 | Thrombospondin-1 |
| 24-221 | 198 | TSP N-terminal |
| 316-373 | 58 | VWFC |

As used herein, the term "relating a signal to the presence or amount" of an analyte reflects this understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and *The Immunoassay Handbook,* David Wild, ed. Stockton Press, New York, 1994, each of which is hereby incorporated by reference in its entirety, including all tables, figures and claims.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377, each of which is hereby incorporated by reference in its entirety, including all tables, figures and claims. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, *etc.)* as well as molecules that may be indirectly detected by production of a detectable reaction product *(e.g.,* enzymes such as horseradish peroxidase, alkaline phosphatase, *etc.)* or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non- specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein crosslinks. A variety of heterobifunctional cross-linkers, each combining different attributes for successful conjugation, are commercially available.

In certain aspects, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ M$^{-1}$, and preferably between about $10^8$ M$^{-1}$ to about $10^9$ M$^{-1}$, about $10^9$ M$^{-1}$ to about $10^{10}$ M$^{-1}$, or about $10^{10}$ M$^{-1}$ to about $10^{12}$ M$^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: $r/c = K(n-r)$: where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098, which is hereby incorporated in its entirety, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs *(e.g.,* in sandwich assays) may interfere with one another sterically, *etc.,* assay performance of an antibody may be a more important measure than absolute affinity and specificity of an antibody.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, *etc.)* include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Cathepsin B (P07858); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, 043656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61 ); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatinine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria

males and females 18 years of age or older;

undergoing a radiographic / angiographic procedure (such as a CT scan or coronary intervention) involving the intravascular administration of contrast media;

expected to be hospitalized for at least 48 hours after contrast administration.

able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm 0.5$), 8 ($\pm 1$), 24 ($\pm 2$) 48 ($\pm 2$), and 72 ($\pm 2$) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA.

The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m$^2$ = 2 points, 20-40 mL/min/1.73 m$^2$ = 4 points, < 20 mL/min/1.73 m$^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria
males and females 18 years of age or older;
undergoing cardiovascular surgery;
Toronto/Ottawa Predictive Risk Index for Renal Replacement risk score of at least 2 (Wijeysundera et al., JAMA 297: 1801-9, 2007); and
able and willing to provide written informed consent for study participation and to comply with all study procedures.
Exclusion Criteria
known pregnancy;
previous renal transplantation;
acutely worsening renal function prior to enrollment (e.g., any category of
RIFLE criteria);
already receiving dialysis (either acute or chronic) or in imminent need of dialysis at enrollment;
currently enrolled in another clinical study or expected to be enrolled in another clinical study within 7 days of cardiac surgery that involves drug infusion or a therapeutic intervention for AKI;
known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 900 adults expected to be in the ICU for at least 48 hours will be enrolled. To be enrolled in the study, each patient must meet all of the following inclusion criteria and none of the following exclusion criteria:

Inclusion Criteria
males and females 18 years of age or older;
Study population 1: approximately 300 patients that have at least one of:

shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of:

IV antibiotics ordered in computerized physician order entry (CPOE) within 24 hours of enrollment; contrast media exposure within 24 hours of enrollment;
increased Intra-Abdominal Pressure with acute decompensated heart failure; and
severe trauma as the primary reason for ICU admission and likely to be hospitalized in the ICU for 48 hours after enrollment;

Study population 3: approximately 300 patients
expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment.

Exclusion Criteria
known pregnancy;
institutionalized individuals;
previous renal transplantation;
known acutely worsening renal function prior to enrollment (e.g., any category of RIFLE criteria);
received dialysis (either acute or chronic) within 5 days prior to enrollment or in imminent need of dialysis at the time of enrollment;
known infection with human immunodeficiency virus (HIV) or a hepatitis virus;
meets only the SBP < 90 mmHg inclusion criterion set forth above, and does not have shock in the attending physician's or principal investigator's opinion.

After providing informed consent, an EDTA anti-coagulated blood sample (10 mL) and a urine sample (25-30 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are is measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.
Concentrations are expressed in the following examples as follows: soluble CD44 antigen - ng/mL, Angiopoietin-1 - pg/mL, soluble Angiopoietin-1 receptor - ng/mL, C-X-C chemokine motif 5 - ng/mL, soluble Endoglin - pg/mL, soluble Tumor-associated calcium signal transducer 1 (also known as "epithelial cell-adhesion molecule") - pg/mL, Erythropoietin - pg/mL, soluble Fractalkine - ng/mL, Heme oxygenase 1 - ng/mL, soluble Interleukin-1 receptor - pg/mL, type II - pg/mL, soluble Interleukin-6 receptor subunit-alpha - pg/mL, Lymphotactin - ng/mL, Lymphotoxin-alpha-pg/mL, Stromelysin-1 (also known as "matrix metalloproteinase-3") - ng/mL, C-C motif chemokine 22 (also known as "MDC") - pg/mL, C-C motif chemokine 5 (also known as "Rantes") - ng/mL, and Thrombospondin-1 - ng/mL.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Kidney injury markers for evaluating renal status in patients at RIFLE Stage 0

Patients from the intensive care unit (ICU) were classified by kidney status as non-injury (0), risk of injury (R), injury (I), and failure (F) according to the maximum stage reached within 7 days of enrollment as determined by the RIFLE criteria.

Two cohorts were defined as (Cohort 1) patients that did not progress beyond stage 0, and (Cohort 2) patients that reached stage R, I, or F within 10 days. To address normal marker fluctuations that occur within patients at the ICU and thereby assess utility for monitoring AKI status, marker levels were measured in urine samples collected for Cohort 1. Marker concentrations were measured in urine samples collected from a subject at 0, 24 hours, and 48 hours prior to reaching stage R, I or F in Cohort 2. In the following tables, the time "prior max stage" represents the time at which a sample is collected, relative to the time a particular patient reaches the lowest disease stage as defined for that cohort, binned into three groups which are +/- 12 hours. For example, 24 hr prior for this example (0 vs R, I, F) would mean 24 hr (+/- 12 hours) prior to reaching stage R (or I if no sample at R, or F if no sample at R or I).

Each marker was measured by standard immunoassay methods using commercially available assay reagents. A receiver operating characteristic (ROC) curve was generated for each marker and the area under each ROC curve (AUC) was determined. Patients in Cohort 2 were also separated according to the reason for adjudication to stage R, I, or F as being based on serum creatinine measurements (sCr), being based on urine output (UO), or being based on either serum creatinine measurements or urine output. That is, for those patients adjudicated to stage R, I, or F on the basis of serum creatinine measurements alone, the stage 0 cohort may have included patients adjudicated to stage R, I, or F on the basis of urine output; for those patients adjudicated to stage R, I, or F on the basis of urine output alone, the stage 0 cohort may have included patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, for those patients adjudicated to stage R, I, or F on the basis of serum creatinine measurements or urine output, the adjudication method which yielded the most severe RIFLE stage was used.

The ability to distinguish cohort 1 (subjects remaining in RIFLE 0) from Cohort 2 (subjects progressing to RIFLE R, I or F) was determined using ROC analysis. SE is the standard error of the AUC, n is the number of sample or individual patients ("pts," as indicated). Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 1.

Example 7. Kidney injury markers for evaluating renal status in patients at RIFLE Stages 0 and R

Patients were classified and analyzed as described in Example 6. However, patients that reached stage R but did not progress to stage I or F were grouped with patients from non-injury stage 0 in Cohort 1. Cohort 2 in this example included only patients that progressed to stage I or F. Marker concentrations in urine samples were included for Cohort 1. Marker concentrations in urine samples collected within 0, 24, and 48 hours of reaching stage I or F were included for Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 2.

Example 8. Kidney injury markers for evaluating renal status in patients progressing from Stage R to Stages I and F

Patients were classified and analyzed as described in Example 6, but only those patients that reached Stage R were included in this example. Cohort 1 contained patients that reached stage R but did not progress to stage I or F within 10 days, and Cohort 2 included only patients that progressed to stage I or F. Marker concentrations in urine samples collected within 12 hours of reaching stage R were included in the analysis for both Cohort 1 and 2.

The ability to distinguish cohort 1 (subjects remaining in RIFLE R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 3.

Example 9. Kidney injury markers for evaluating renal status in patients at RIFLE Stage 0

Patients were classified and analyzed as described in Example 6. However, patients that reached stage R or I but did not progress to stage F were eliminated from the analysis. Patients from non-injury stage 0 are included in Cohort 1. Cohort 2 in this example included only patients that progressed to stage F. The maximum marker concentrations in urine samples were included for each patient in Cohort 1. The maximum marker concentrations in urine samples collected within 0, 24, and 48 hours of reaching stage F were included for each patient in Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 4.

Example 10. Kidney injury markers for evaluating renal status in patients at RIFLE Stage 0

Patients from the intensive care unit (ICU) were classified by kidney status as non-injury (0), risk of injury (R), injury (I), and failure (F) according to the maximum stage reached within 7 days of enrollment as determined by the RIFLE criteria.

Two cohorts were defined as (Cohort 1) patients that did not progress beyond stage 0, and (Cohort 2) patients that reached stage R, I, or F within 10 days. To address normal marker fluctuations that occur within patients at the ICU and thereby assess utility for monitoring AKI status, marker levels were measured in the plasma component of blood samples collected for Cohort 1. Marker concentrations were measured in the plasma component of blood samples collected from a subject at 0, 24 hours, and 48 hours prior to reaching stage R, I or F in Cohort 2. In the following tables, the time "prior max stage" represents the time at which a sample is collected, relative to the time a particular patient reaches the lowest disease stage as defined for that cohort, binned into three groups which are +/- 12 hours. For example, 24 hr prior for this example (0 vs R, I, F) would mean 24 hr (+/- 12 hours) prior to reaching stage R (or I if no sample at R, or F if no sample at R or I).

Each marker was measured by standard immunoassay methods using commercially available assay reagents. A receiver operating characteristic (ROC) curve was generated for each marker and the area under each ROC curve (AUC) was determined. Patients in Cohort 2 were also separated according to the reason for adjudication to stage R, I, or F as being based on serum creatinine measurements (sCr), being based on urine output (UO), or being based on either serum creatinine measurements or urine output. That is, for those patients adjudicated to stage R, I, or F on the basis of serum creatinine measurements alone, the stage 0 cohort may have included patients adjudicated to stage R, I, or F on the basis of urine output; for those patients adjudicated to stage R, I, or F on the basis of urine output alone, the stage 0 cohort may have included patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, for those patients adjudicated to stage R, I, or F on the basis of serum creatinine measurements or urine output, the adjudication method which yielded the most severe RIFLE stage was used.

The ability to distinguish cohort 1 (subjects remaining in RIFLE 0) from Cohort 2 (subjects progressing to RIFLE R, I or F) was determined using ROC analysis. SE is the standard error of the AUC, n is the number of sample or individual patients ("pts," as indicated). Standard errors were calculated as described in Hanley, J. A., and McNeil, B.J.,

The meaning and use of the area under a receiver operating characteristic (ROC) curve. Radiology (1982) 143: 29-36; p values were calculated with a two-tailed Z-test. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 5.

Example 11. Kidney injury markers for evaluating renal status in patients at RIFLE Stages 0 and R

Patients were classified and analyzed as described in Example 10. However, patients that reached stage R but did not progress to stage I or F were grouped with patients from non-injury stage 0 in Cohort 1. Cohort 2 in this example included only patients that progressed to stage I or F. Marker concentrations in the plasma component of blood samples were included for Cohort 1. Marker concentrations in the plasma component of blood samples collected within 0, 24, and 48 hours of reaching stage I or F were included for Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 6.

Example 12. Kidney injury markers for evaluating renal status in patients progressing from Stage R to Stages I and F

Patients were classified and analyzed as described in Example 10, but only those patients that reached Stage R were included in this example. Cohort 1 contained patients that reached stage R but did not progress to stage I or F within 10 days, and Cohort 2 included only patients that progressed to stage I or F. Marker concentrations in the plasma component of blood samples collected within 12 hours of reaching stage R were included in the analysis for both Cohort 1 and 2.

The ability to distinguish cohort 1 (subjects remaining in RIFLE R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 7.

Example 13. Kidney injury markers for evaluating renal status in patients at RIFLE Stage 0

Patients were classified and analyzed as described in Example 10. However, patients that reached stage R or I but did not progress to stage F were eliminated from the analysis. Patients from non-injury stage 0 are included in Cohort 1. Cohort 2 in this example included only patients that progressed to stage F. The maximum marker concentrations in the plasma component of blood samples were included from each patient in Cohort 1. The maximum marker concentrations in the plasma component of blood samples collected within 0, 24, and 48 hours of reaching stage F were included from each patient in Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 8.

Disclosed are furthermore the following items:

    1. A method for evaluating renal status in a subject, comprising:

        performing one or more assays configured to detect a kidney injury marker selected from the group consisting of soluble CD44 antigen, Angiopoietin-1, soluble Angiopoietin-1 receptor, C-X-C chemokine motif 5, soluble Endoglin, soluble Tumor-associated calcium signal transducer 1, Erythropoietin, soluble Fractalkine, Heme oxygenase 1, soluble Interleukin-1 receptor type II, soluble Interleukin-6 receptor subunit-alpha, Lymphotactin, Lymphotoxin-alpha, Stromelysin-1, C-C motif chemokine 22, C-C motif chemokine 5, and Thrombospondin-1 on a body fluid sample obtained from the subject to provide one or more assay results; and

correlating the assay result(s) to the renal status of the subject.

2. A method according to item 1, wherein said correlation step comprises correlating the assay result(s) to one or more of risk stratification, diagnosis, staging, prognosis, classifying and monitoring of the renal status of the subject.

3. A method according to item 1, wherein said correlating step comprises assigning a likelihood of one or more future changes in renal status to the subject based on the assay result(s).

4. A method according to item 3, wherein said one or more future changes in renal status comprise one or more of a future injury to renal function, future reduced renal function, future improvement in renal function, and future acute renal failure (ARF).

5. A method according to item 4, wherein said assay result(s) comprise one or more of:

(i) a measured concentration of soluble CD44 antigen,

(ii) a measured concentration of Angiopoietin-1,

(iii) a measured concentration of soluble Angiopoietin-1 receptor ,

(iv) a measured concentration of C-X-C chemokine motif 5,

(v) a measured concentration of soluble Endoglin,

(vi) a measured concentration of soluble Tumor-associated calcium signal transducer 1,

(vii) a measured concentration of Erythropoietin,

(viii) a measured concentration of soluble Fractalkine,

(ix) a measured concentration of Heme oxygenase 1,

(x) a measured concentration of soluble Interleukin-1 receptor type II,

(xi) a measured concentration of soluble Interleukin-6 receptor subunit-alpha,

(xii) a measured concentration of Lymphotactin,

(xiii) a measured concentration of Lymphotoxin-alpha,

(xiv) a measured concentration of Stromelysin-1,

(xv) a measured concentration of C-C motif chemokine 22,

(xvi) a measured concentration of C-C motif chemokine 5, or

(xvii) a measured concentration of Thrombospondin-1,

and said correlation step comprises, for each assay result, comparing said measured concentration to a threshold concentration, and
for a positive going marker, assigning an increased likelihood of suffering a future injury to renal function, future reduced renal function, future ARF, or a future improvement in renal function to the subject when the measured concentration is above the threshold, relative to a likelihood assigned when the measured concentration is below the threshold or assigning a decreased likelihood of suffering a future injury to renal function, future reduced renal function, future ARF, or a future improvement in renal function to the subject when the measured concentration is below the threshold, relative to a likelihood assigned when the measured concentration is above the threshold, or for a negative going marker, assigning an increased likelihood of suffering a future injury to renal function, future reduced renal function, future ARF, or a future improvement in renal function to the subject when the measured concentration is below the threshold, relative to a likelihood assigned when the measured concentration is above the threshold or assigning a decreased likelihood of suffering a future injury to renal function, future reduced renal function, future ARF, or a future improvement in renal function to the subject when the measured concentration is above the threshold, relative to a likelihood assigned when the measured concentration is below the threshold.

6. A method according to item 3, wherein said one or more future changes in renal status comprise a clinical outcome related to a renal injury suffered by the subject.

7. A method according to item 1, wherein said assay result(s) comprise one or more of:

> (i) a measured concentration of soluble CD44 antigen,
> (ii) a measured concentration of Angiopoietin-1,
> (iii) a measured concentration of soluble Angiopoietin-1 receptor ,
> (iv) a measured concentration of C-X-C chemokine motif 5,
> (v) a measured concentration of soluble Endoglin,
> (vi) a measured concentration of soluble Tumor-associated calcium signal transducer 1,
> (vii) a measured concentration of Erythropoietin,
> (viii) a measured concentration of soluble Fractalkine,
> (ix) a measured concentration of Heme oxygenase 1,
> (x) a measured concentration of soluble Interleukin-1 receptor type II,
> (xi) a measured concentration of soluble Interleukin-6 receptor subunit-alpha,
> (xii) a measured concentration of Lymphotactin,
> (xiii) a measured concentration of Lymphotoxin-alpha,
> (xiv) a measured concentration of Stromelysin-1,
> (xv) a measured concentration of C-C motif chemokine 22,
> (xvi) a measured concentration of C-C motif chemokine 5, or
> (xvii) a measured concentration of Thrombospondin-1,

and said correlation step comprises, for each assay result, comparing said measured concentration to a threshold concentration, and

for a positive going marker, assigning an increased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is above the threshold, relative to a likelihood assigned when the measured concentration is below the threshold, or assigning a decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is below the threshold, relative to a likelihood assigned when the measured concentration is above the threshold, or for a negative going marker, assigning an increased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is below the threshold, relative to a likelihood assigned when the measured concentration is above the threshold, or assigning a decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is above the threshold, relative to a likelihood assigned when the measured concentration is below the threshold.

8. A method according to item 3, wherein the likelihood of one or more future changes in renal status is that an event of interest is more or less likely to occur within 30 days of the time at which the body fluid sample is obtained from the subject.

9. A method according to item 8, wherein the likelihood of one or more future changes in renal status is that an event of interest is more or less likely to occur within a period selected from the group consisting of 21 days, 14 days, 7 days, 5 days, 96 hours, 72 hours, 48 hours, 36 hours, 24 hours, and 12 hours.

10. A method according to item 1, wherein the subject is selected for evaluation of renal status based on the pre-existence in the subject of one or more known risk factors for prerenal, intrinsic renal, or postrenal ARF.

11. A method according to item 1, wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

12. A method according to item 1, wherein said correlating step comprises assigning a diagnosis of the occurrence or nonoccurrence of one or more of an injury to renal function, reduced renal function, or ARF to the subject based on the assay result(s).

13. A method according to item 12, wherein said assay result(s) comprise one or more of:

(i) a measured concentration of soluble CD44 antigen,
(ii) a measured concentration of Angiopoietin-1,
(iii) a measured concentration of soluble Angiopoietin-1 receptor ,
(iv) a measured concentration of C-X-C chemokine motif 5,
(v) a measured concentration of soluble Endoglin,
(vi) a measured concentration of soluble Tumor-associated calcium signal transducer 1,
(vii) a measured concentration of Erythropoietin,
(viii) a measured concentration of soluble Fractalkine,
(ix) a measured concentration of Heme oxygenase 1,
(x) a measured concentration of soluble Interleukin-1 receptor type II,
(xi) a measured concentration of soluble Interleukin-6 receptor subunit-alpha,
(xii) a measured concentration of Lymphotactin,
(xiii) a measured concentration of Lymphotoxin-alpha,
(xiv) a measured concentration of Stromelysin-1,
(xv) a measured concentration of C-C motif chemokine 22,
(xvi) a measured concentration of C-C motif chemokine 5, or
(xvii) a measured concentration of Thrombospondin-1,

and said correlation step comprises, for each assay result, comparing said measured concentration to a threshold concentration, and
for a positive going marker, assigning the occurrence of an injury to renal function, reduced renal function, or ARF to the subject when the measured concentration is above the threshold, or assigning the nonoccurrence of an injury to renal function, reduced renal function, or ARF to the subject when the measured concentration is below the threshold, or
for a negative going marker, assigning the occurrence of an injury to renal function, reduced renal function, or ARF to the subject when the measured concentration is below the threshold, or assigning the nonoccurrence of an injury to renal function, reduced renal function, or ARF to the subject when the measured concentration is above the threshold.

14. A method according to item 1, wherein said correlating step comprises assessing whether or not renal function is improving or worsening in a subject who has suffered from an injury to renal function, reduced renal function, or ARF based on the assay result(s).

15. A method according to item 14, wherein said assay result(s) comprise one or more of:

(i) a measured concentration of soluble CD44 antigen,
(ii) a measured concentration of Angiopoietin-1,
(iii) a measured concentration of soluble Angiopoietin-1 receptor ,
(iv) a measured concentration of C-X-C chemokine motif 5,
(v) a measured concentration of soluble Endoglin,
(vi) a measured concentration of soluble Tumor-associated calcium signal transducer 1,

(vii) a measured concentration of Erythropoietin,
(viii) a measured concentration of soluble Fractalkine,
(ix) a measured concentration of Heme oxygenase 1,
(x) a measured concentration of soluble Interleukin-1 receptor type II,
(xi) a measured concentration of soluble Interleukin-6 receptor subunit-alpha,
(xii) a measured concentration of Lymphotactin,
(xiii) a measured concentration of Lymphotoxin-alpha,
(xiv) a measured concentration of Stromelysin-1,
(xv) a measured concentration of C-C motif chemokine 22,
(xvi) a measured concentration of C-C motif chemokine 5, or
(xvii) a measured concentration of Thrombospondin-1,

and said correlation step comprises, for each assay result, comparing said measured concentration to a threshold concentration, and

for a positive going marker, assigning a worsening of renal function to the subject when the measured concentration is above the threshold, or assigning an improvement of renal function when the measured concentration is below the threshold, or

for a negative going marker, assigning a worsening of renal function to the subject when the measured concentration is below the threshold, or assigning an improvement of renal function when the measured concentration is above the threshold.

16. A method according to item 1, wherein said method is a method of diagnosing the occurrence or nonoccurrence of an injury to renal function in said subject.

17. A method according to item 1, wherein said method is a method of diagnosing the occurrence or nonoccurrence of reduced renal function in said subject.

18. A method according to item 1, wherein said method is a method of diagnosing the occurrence or nonoccurrence of acute renal failure in said subject.

19. A method according to item 1, wherein said method is a method of diagnosing the occurrence or nonoccurrence of a need for renal replacement therapy in said subject.

20. A method according to item 1, wherein said method is a method of diagnosing the occurrence or nonoccurrence of a need for renal transplantation in said subject.

21. A method according to item 1, wherein said method is a method of assigning a risk of the future occurrence or nonoccurrence of an injury to renal function in said subject.

22. A method according to item 1, wherein said method is a method of assigning a risk of the future occurrence or nonoccurrence of reduced renal function in said subject.

23. A method according to item 1, wherein said method is a method of assigning a risk of the future occurrence or nonoccurrence of acute renal failure in said subject.

24. A method according to item 1, wherein said method is a method of assigning a risk of the future occurrence or nonoccurrence of a need for renal replacement therapy in said subject.

25. A method according to item 1, wherein said method is a method of assigning a risk of the future occurrence or nonoccurrence of a need for renal transplantation in said subject.

26. A method according to item 5, wherein said one or more future changes in renal status comprise one or more of a future injury to renal function, future reduced renal function, future improvement in renal function, and future acute renal failure (ARF) within 72 hours of the time at which the body fluid sample is obtained.

27. A method according to item 5, wherein said one or more future changes in renal status comprise one or more of a future injury to renal function, future reduced renal function, future improvement in renal function, and future acute renal failure (ARF) within 48 hours of the time at which the body fluid sample is obtained.

28. A method according to item 5, wherein said one or more future changes in renal status comprise one or more of a future injury to renal function, future reduced renal function, future improvement in renal function, and future acute renal failure (ARF) within 24 hours of the time at which the body fluid sample is obtained.

29. The use of one or more kidney injury markers selected from the group consisting of soluble CD44 antigen, Angiopoietin-1, soluble Angiopoietin-1 receptor , C-X-C chemokine motif 5, soluble Endoglin, soluble Tumor-associated calcium signal transducer 1, Erythropoietin, soluble Fractalkine, Heme oxygenase 1, soluble Interleukin-1 receptor type II, soluble Interleukin-6 receptor subunit-alpha, Lymphotactin, Lymphotoxin-alpha, Stromelysin-1, C-C motif chemokine 22, C-C motif chemokine 5, and Thrombospondin-1 for the evaluation of renal injury.

30. The use of one or more kidney injury markers selected from the group consisting of soluble CD44 antigen, Angiopoietin-1, soluble Angiopoietin-1 receptor , C-X-C chemokine motif 5, soluble Endoglin, soluble Tumor-associated calcium signal transducer 1, Erythropoietin, soluble Fractalkine, Heme oxygenase 1, soluble Interleukin-1 receptor type II, soluble Interleukin-6 receptor subunit-alpha, Lymphotactin, Lymphotoxin-alpha, Stromelysin-1, C-C motif chemokine 22, C-C motif chemokine 5, and Thrombospondin-1 for the evaluation of acute renal injury.

31. A method according to item 7, wherein the increased or decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease assigned to the subject is a likelihood that an event of interest is more or less likely to occur within 30 days of the time at which the body fluid sample is obtained from the subject.

32. A method according to item 7, wherein the increased or decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease assigned to the subject is a likelihood that an event of interest is more or less likely to occur within 72 hours of the time at which the body fluid sample is obtained from the subject.

33. A method according to item 7, wherein the increased or decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease assigned to the subject is a likelihood that an event of interest is more or less likely to occur within 24 hours of the time at which the body fluid sample is obtained from the subject.

While the invention has been described and exemplified in sufficient detail for those skilled in this art to make and use it, various alternatives, modifications, and improvements should be apparent without departing from the spirit and scope of the invention. The examples provided herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

**Claims**

1. A method for evaluating renal status in a subject, comprising:

   performing one or more assays configured to detect one or more kidney injury markers, the one or more kidney injury markers comprising Thrombospondin-1, on a body fluid sample obtained from the subject to provide one or more assay results; and
   correlating the assay result(s) to the renal status of the subject.

2. A method according to claim 1, wherein the one or more kidney injury markers further comprise soluble CD44 antigen, Angiopoietin-1, soluble Angiopoietin-1 receptor, C-X-C chemokine motif 5, soluble Endoglin, soluble Tumor-associated calcium signal transducer 1, Erythropoietin, soluble Fractalkine, Heme oxygenase 1, soluble Interleukin-1 receptor type II, soluble Interleukin-6 receptor subunit-alpha, Lymphotactin, Lymphotoxin-alpha, Stromelysin-1, C-C motif chemokine 22, and C-C motif chemokine 5.

3. A method according to claims 1 or 2, wherein said correlation step comprises:

   (i) correlating the assay result(s) to one or more of risk stratification, diagnosis, staging, prognosis, classifying and monitoring of the renal status of the subject; or
   (ii) assigning a likelihood of one or more future changes in renal status to the subject based on the assay result(s); or
   (iii) assigning a diagnosis of the occurrence or nonoccurrence of one or more of an injury to renal function, reduced renal function, or ARF to the subject based on the assay result(s).

4. A method according to claim 3, wherein a likelihood of one or more future changes in renal status to the subject based on the assay result(s) is assigned, and said one or more future changes in renal status comprise:

   (i) one or more of a future injury to renal function, future reduced renal function, future improvement in renal function, future acute renal failure (ARF) and acute kidney injury (AKI); or
   (ii) a clinical outcome related to a renal injury suffered by the subject..

5. A method according to claim 4, wherein said assay result(s) comprise a measured concentration of Thrombospondin-1, and one or more of:

   (i) a measured concentration of soluble CD44 antigen,

(ii) a measured concentration of Angiopoietin-1,
(iii) a measured concentration of soluble Angiopoietin-1 receptor,
(iv) a measured concentration of C-X-C chemokine motif 5,
(v) a measured concentration of soluble Endoglin,
(vi) a measured concentration of soluble Tumor-associated calcium signal transducer 1,
(vii) a measured concentration of Erythropoietin,
(viii) a measured concentration of soluble Fractalkine,
(ix) a measured concentration of Heme oxygenase 1,
(x) a measured concentration of soluble Interleukin-1 receptor type II,
(xi) a measured concentration of soluble Interleukin-6 receptor subunit-alpha,
(xii) a measured concentration of Lymphotactin,
(xiii) a measured concentration of Lymphotoxin-alpha,
(xiv) a measured concentration of Stromelysin-1,
(xv) a measured concentration of C-C motif chemokine 22, or
(xvi) a measured concentration of C-C motif chemokine 5,

and said correlation step comprises, for each assay result, comparing said measured concentration to a threshold concentration, and
for a positive going marker, assigning an increased likelihood of suffering a future injury to renal function, future reduced renal function, future ARF, or a future improvement in renal function to the subject when the measured concentration is above the threshold, relative to a likelihood assigned when the measured concentration is below the threshold or assigning a decreased likelihood of suffering a future injury to renal function, future reduced renal function, future ARF, or a future improvement in renal function to the subject when the measured concentration is below the threshold, relative to a likelihood assigned when the measured concentration is above the threshold, or
for a negative going marker, assigning an increased likelihood of suffering a future injury to renal function, future reduced renal function, future ARF, or a future improvement in renal function to the subject when the measured concentration is below the threshold, relative to a likelihood assigned when the measured concentration is above the threshold or assigning a decreased likelihood of suffering a future injury to renal function, future reduced renal function, future ARF, or a future improvement in renal function to the subject when the measured concentration is above the threshold, relative to a likelihood assigned when the measured concentration is below the threshold.

6. A method according to claim 1 or 2, wherein said assay result(s) comprise a measured concentration of Thrombospondin-1, and one or more of:

(i) a measured concentration of soluble CD44 antigen,
(ii) a measured concentration of Angiopoietin-1,
(iii) a measured concentration of soluble Angiopoietin-1 receptor ,
(iv) a measured concentration of C-X-C chemokine motif 5,
(v) a measured concentration of soluble Endoglin,
(vi) a measured concentration of soluble Tumor-associated calcium signal transducer 1,
(vii) a measured concentration of Erythropoietin,
(viii) a measured concentration of soluble Fractalkine,
(ix) a measured concentration of Heme oxygenase 1,
(x) a measured concentration of soluble Interleukin-1 receptor type II,
(xi) a measured concentration of soluble Interleukin-6 receptor subunit-alpha,
(xii) a measured concentration of Lymphotactin,
(xiii) a measured concentration of Lymphotoxin-alpha,
(xiv) a measured concentration of Stromelysin-1,
(xv) a measured concentration of C-C motif chemokine 22, or
(xvi) a measured concentration of C-C motif chemokine 5,

and said correlation step comprises, for each assay result, comparing said measured concentration to a threshold concentration, and
for a positive going marker, assigning an increased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is above the threshold, relative to a likelihood assigned when the measured concentration is below the threshold, or assigning a decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is below the threshold, relative to a likelihood assigned when the measured concentration is above the threshold, or for a negative going marker, assigning an increased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is below the threshold, relative to a likelihood assigned when the measured concentration is above the threshold, or assigning a decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is above the threshold, relative to a likelihood assigned when the measured concentration is below the threshold;

wherein optionally the increased or decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease assigned to the subject is a likelihood that an event of interest is more or less likely to occur within 30 days, within 72 hours, or within 24 hours of the time at which the body fluid sample is obtained from the subject.

7. A method according to claim 3, wherein the likelihood of one or more future changes in renal status is that an event of interest is more or less likely to occur within 30 days of the time at which the body fluid sample is obtained from the subject, or

within a period selected from the group consisting of 21 days, 14 days, 7 days, 5 days, 96 hours, 72 hours, 48 hours, 36 hours, 24 hours, and 12 hours.

8. A method according to claim 1 or 2, wherein the subject is selected for evaluation of renal status based on:

(i) the pre-existence in the subject of one or more known risk factors for prerenal, intrinsic renal, or postrenal ARF;
(ii) an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

9. A method according to claim 3, wherein a diagnosis of the occurrence or nonoccurrence of one or more of an injury to renal function, reduced renal function, or ARF is assigned, and wherein said assay result(s) comprise a measured concentration of Thrombospondin-1, and one or more of:

(i) a measured concentration of soluble CD44 antigen,
(ii) a measured concentration of Angiopoietin-1,
(iii) a measured concentration of soluble Angiopoietin-1 receptor ,
(iv) a measured concentration of C-X-C chemokine motif 5,
(v) a measured concentration of soluble Endoglin,
(vi) a measured concentration of soluble Tumor-associated calcium signal transducer 1,
(vii) a measured concentration of Erythropoietin,
(viii) a measured concentration of soluble Fractalkine,
(ix) a measured concentration of Heme oxygenase 1,
(x) a measured concentration of soluble Interleukin-1 receptor type II,
(xi) a measured concentration of soluble Interleukin-6 receptor subunit-alpha,
(xii) a measured concentration of Lymphotactin,
(xiii) a measured concentration of Lymphotoxin-alpha,
(xiv) a measured concentration of Stromelysin-1,
(xv) a measured concentration of C-C motif chemokine 22, or
(xvi) a measured concentration of C-C motif chemokine 5,

and said correlation step comprises, for each assay result, comparing said measured concentration to a threshold concentration, and
for a positive going marker, assigning the occurrence of an injury to renal function, reduced renal function, or ARF to the subject when the measured concentration is above the threshold, or assigning the nonoccurrence of an injury to renal function, reduced renal function, or ARF to the subject when the measured concentration is below the threshold, or

for a negative going marker, assigning the occurrence of an injury to renal function, reduced renal function, or ARF to the subject when the measured concentration is below the threshold, or assigning the nonoccurrence of an injury to renal function, reduced renal function, or ARF to the subject when the measured concentration is above the threshold.

10. A method according to claim 1 or 2, wherein said correlating step comprises assessing whether or not renal function is improving or worsening in a subject who has suffered from an injury to renal function, reduced renal function, or ARF based on the assay result(s).

11. A method according to claim 10, wherein said assay result(s) comprise a measured concentration of Thrombospondin-1, and one or more of:

    (i) a measured concentration of soluble CD44 antigen,
    (ii) a measured concentration of Angiopoietin-1,
    (iii) a measured concentration of soluble Angiopoietin-1 receptor ,
    (iv) a measured concentration of C-X-C chemokine motif 5,
    (v) a measured concentration of soluble Endoglin,
    (vi) a measured concentration of soluble Tumor-associated calcium signal transducer 1,
    (vii) a measured concentration of Erythropoietin,
    (viii) a measured concentration of soluble Fractalkine,
    (ix) a measured concentration of Heme oxygenase 1,
    (x) a measured concentration of soluble Interleukin-1 receptor type II,
    (xi) a measured concentration of soluble Interleukin-6 receptor subunit-alpha,
    (xii) a measured concentration of Lymphotactin,
    (xiii) a measured concentration of Lymphotoxin-alpha,
    (xiv) a measured concentration of Stromelysin-1,
    (xv) a measured concentration of C-C motif chemokine 22, or
    (xvi) a measured concentration of C-C motif chemokine 5,

and said correlation step comprises, for each assay result, comparing said measured concentration to a threshold concentration, and

for a positive going marker, assigning a worsening of renal function to the subject when the measured concentration is above the threshold, or assigning an improvement of renal function when the measured concentration is below the threshold, or

for a negative going marker, assigning a worsening of renal function to the subject when the measured concentration is below the threshold, or assigning an improvement of renal function when the measured concentration is above the threshold.

12. A method according to claim 1 or 2, wherein said method is a method of diagnosing

    (i) the occurrence or nonoccurrence of an injury to renal function in said subject; or
    (ii) the occurrence or nonoccurrence of reduced renal function in said subject;
    (iii) the occurrence or nonoccurrence of acute renal failure in said subject;
    (iv) the occurrence or nonoccurrence of a need for renal replacement therapy in said subject;
    (v) the occurrence or nonoccurrence of a need for renal transplantation in said subject.

13. A method according to claim 1 or 2, wherein said method is a method of assigning a risk of

    (i) the future occurrence or nonoccurrence of an injury to renal function in said subject;
    (ii) the future occurrence or nonoccurrence of reduced renal function in said subject;
    (iii) the future occurrence or nonoccurrence of acute renal failure in said subject.
    (iv) the future occurrence or nonoccurrence of a need for renal replacement therapy in said subject.
    (v) the future occurrence or nonoccurrence of a need for renal transplantation in said subject.

14. A method according to claim 5, wherein said one or more future changes in renal status comprise one or more of a future injury to renal function, future reduced renal function, future improvement in renal function, and future acute renal failure (ARF) within 72 hours, within 48 hours, or within 24 hours of the time at which the body fluid sample is obtained.

15. Use of one or more kidney injury markers for the evaluation of renal injury or acute renal injury, the one or more kidney injury markers comprising Thrombospondin-1 and one or more of soluble CD44 antigen, Angiopoietin-1, soluble Angiopoietin-1 receptor, C-X-C chemokine motif 5, soluble Endoglin, soluble Tumor-associated calcium signal transducer 1, Erythropoietin, soluble Fractalkine, Heme oxygenase 1, soluble Interleukin-1 receptor type II, soluble Interleukin-6 receptor subunit-alpha, Lymphotactin, Lymphotoxin-alpha, Stromelysin-1, C-C motif chemokine 22, and C-C motif chemokine 5.

CD44

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 98.649 | 79.392 | 98.649 | 125.776 | 98.649 | 147.723 |
| average | 140.403 | 164.709 | 140.403 | 166.894 | 140.403 | 147.723 |
| stdev | 145.611 | 198.361 | 145.611 | 158.170 | 145.611 | 35.040 |
| p (t-test) | | 0.464 | | 0.425 | | 0.944 |
| min | 6.378 | 9.677 | 6.378 | 26.750 | 6.378 | 122.946 |
| max | 792.530 | 919.208 | 792.530 | 628.638 | 792.530 | 172.500 |
| n (Samp) | 101 | 30 | 101 | 25 | 101 | 2 |
| n (Pat) | 50 | 30 | 50 | 25 | 50 | 2 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 110.811 | 31.710 | 110.811 | 65.313 | 110.811 | 109.472 |
| average | 162.007 | 48.902 | 162.007 | 167.107 | 162.007 | 118.217 |
| stdev | 173.200 | 59.579 | 173.200 | 216.314 | 173.200 | 39.929 |
| p (t-test) | | 0.033 | | 0.926 | | 0.663 |
| min | 6.378 | 9.677 | 6.378 | 22.886 | 6.378 | 83.385 |
| max | 1198.603 | 220.678 | 1198.603 | 666.214 | 1198.603 | 161.794 |
| n (Samp) | 169 | 11 | 169 | 11 | 169 | 3 |
| n (Pat) | 93 | 11 | 93 | 11 | 93 | 3 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 88.977 | 154.032 | 88.977 | 135.559 | 88.977 | 86.646 |
| average | 111.993 | 206.713 | 111.993 | 195.832 | 111.993 | 89.416 |
| stdev | 109.125 | 204.830 | 109.125 | 168.552 | 109.125 | 62.362 |
| p (t-test) | | 0.004 | | 0.006 | | 0.650 |
| min | 6.378 | 17.617 | 6.378 | 38.304 | 6.378 | 9.677 |
| max | 727.823 | 919.208 | 727.823 | 628.638 | 727.823 | 172.500 |
| n (Samp) | 78 | 24 | 78 | 23 | 78 | 5 |
| n (Pat) | 38 | 24 | 38 | 23 | 38 | 5 |

sCr or UO

| Time prior AKI stage | AUC | SE | nCohort 1 | nCohort 2 | p |
|---|---|---|---|---|---|
| 0 hours | 0.49 | 0.060 | 101 | 30 | 0.849 |
| 24 hours | 0.56 | 0.066 | 101 | 25 | 0.333 |
| 48 hours | 0.67 | 0.213 | 101 | 2 | 0.436 |

sCr only

| Time prior AKI stage | AUC | SE | nCohort 1 | nCohort 2 | p |
|---|---|---|---|---|---|
| 0 hours | 0.18 | 0.049 | 169 | 11 | 0.000 |
| 24 hours | 0.42 | 0.085 | 169 | 11 | 0.376 |
| 48 hours | 0.51 | 0.170 | 169 | 3 | 0.949 |

UO only

| Time prior AKI stage | AUC | SE | nCohort 1 | nCohort 2 | p |
|---|---|---|---|---|---|
| 0 hours | 0.66 | 0.067 | 78 | 24 | 0.015 |
| 24 hours | 0.68 | 0.067 | 78 | 23 | 0.007 |
| 48 hours | 0.47 | 0.131 | 78 | 5 | 0.791 |

sCr or UO

| Time prior AKI stage | Cutoff value | sens | spec | Quartile | OR | 95% CI of OR | |
|---|---|---|---|---|---|---|---|
| 0 hours | 48.7762 | 70% | 26% | 1 | | | |
| | 33.6039 | 80% | 15% | 2 | 0.4 | 0.2 | 0.9 |
| | 13.0853 | 90% | 5% | 3 | 0.9 | 0.5 | 1.6 |
| | 150 | 37% | 70% | 4 | 1.0 | 0.6 | 1.9 |
| | 200.731 | 30% | 80% | | | | |

# Fig. 1 - 1

| Time prior AKI stage | Cutoff value | sens | spec | Quartile | OR | 95% CI of OR | |
|---|---|---|---|---|---|---|---|
| | 253.194 | 23% | 90% | | | | |
| 24 hours | 72.9021 | 72% | 40% | 1 | | | |
| | 54.021 | 80% | 28% | 2 | 1.0 | 0.4 | 2.4 |
| | 36.25 | 92% | 19% | 3 | 2.5 | 1.2 | 5.3 |
| | 150 | 40% | 70% | 4 | 1.0 | 0.4 | 2.4 |
| | 200.731 | 16% | 80% | | | | |
| | 253.194 | 16% | 90% | | | | |
| 48 hours | 122.297 | 100% | 60% | 1 | | | |
| | 122.297 | 100% | 60% | 2 | na | na | na |
| | 122.297 | 100% | 60% | 3 | na | na | na |
| | 150 | 50% | 70% | 4 | na | na | na |
| | 200.731 | 0% | 80% | | | | |
| | 253.194 | 0% | 90% | | | | |

sCr only

| Time prior AKI stage | Cutoff value | sens | spec | Quartile | OR | 95% CI of OR | |
|---|---|---|---|---|---|---|---|
| 0 hours | 13.0853 | 73% | 4% | 1 | | | |
| | 12.5189 | 82% | 4% | 2 | 0.0 | 0.0 | na |
| | 12.4267 | 91% | 4% | 3 | 1.0 | 0.0 | 55.1 |
| | 172.5 | 9% | 70% | 4 | 11.0 | 1.1 | 107.2 |
| | 223.707 | 0% | 80% | | | | |
| | 358.889 | 0% | 91% | | | | |
| 24 hours | 48.7762 | 73% | 21% | 1 | | | |
| | 30.6818 | 82% | 11% | 2 | 1.5 | 0.3 | 8.6 |
| | 26.6602 | 91% | 8% | 3 | 0.5 | 0.0 | 10.1 |
| | 172.5 | 18% | 70% | 4 | 2.7 | 0.6 | 11.6 |
| | 223.707 | 18% | 80% | | | | |
| | 358.889 | 18% | 91% | | | | |
| 48 hours | 82.75 | 100% | 37% | 1 | | | |
| | 82.75 | 100% | 37% | 2 | na | na | na |
| | 82.75 | 100% | 37% | 3 | na | na | na |
| | 172.5 | 0% | 70% | 4 | na | na | na |
| | 223.707 | 0% | 80% | | | | |
| | 358.889 | 0% | 91% | | | | |

UO only

| Time prior AKI stage | Cutoff value | sens | spec | Quartile | OR | 95% CI of OR | |
|---|---|---|---|---|---|---|---|
| 0 hours | 72.9021 | 71% | 46% | 1 | | | |
| | 59.2657 | 83% | 35% | 2 | 1.6 | 0.6 | 4.3 |
| | 36.25 | 92% | 24% | 3 | 0.7 | 0.2 | 2.7 |
| | 137.162 | 54% | 71% | 4 | 3.9 | 1.6 | 9.4 |
| | 160.086 | 50% | 81% | | | | |
| | 206.638 | 42% | 91% | | | | |
| 24 hours | 92.25 | 74% | 54% | 1 | | | |
| | 72.9021 | 83% | 46% | 2 | 2.9 | 0.6 | 13.6 |
| | 54.6875 | 91% | 32% | 3 | 3.6 | 0.8 | 16.2 |
| | 137.162 | 48% | 71% | 4 | 7.2 | 1.8 | 28.7 |
| | 160.086 | 35% | 81% | | | | |
| | 206.638 | 22% | 91% | | | | |
| 48 hours | 54.8438 | 80% | 33% | 1 | | | |
| | 54.8438 | 80% | 33% | 2 | 1.0 | 0.0 | 61.3 |
| | 9.12009 | 100% | 3% | 3 | 2.1 | 0.1 | 48.7 |
| | 137.162 | 20% | 71% | 4 | 1.1 | 0.0 | 64.9 |
| | 160.086 | 20% | 81% | | | | |
| | 206.638 | 0% | 91% | | | | |

Fig. 1 - 2

**Angiopoietin-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 33.854 | 12.369 | 33.854 | 34.091 | 33.854 | 0.548 |
| average | 55.945 | 33.339 | 55.945 | 521.784 | 55.945 | 60.315 |
| stdev | 66.570 | 45.275 | 66.570 | 2079.392 | 66.570 | na |
| p (t-test) | | 0.095 | | 0.029 | | na |
| min | 0.548 | 0.548 | 0.548 | 0.548 | 0.548 | 60.315 |
| max | 292.857 | 157.407 | 292.857 | 10472.009 | 292.857 | 60.315 |
| n (Samp) | 96 | 28 | 96 | 25 | 96 | 1 |
| n (Pat) | 48 | 28 | 48 | 25 | 48 | 1 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 28.036 | 8.693 | 28.036 | 21.154 | 28.036 | 0.548 |
| average | 120.731 | 37.314 | 120.731 | 25.884 | 120.731 | 152.647 |
| stdev | 832.142 | 44.042 | 832.142 | 32.491 | 832.142 | 263.443 |
| p (t-test) | | 0.752 | | 0.707 | | 0.947 |
| min | 0.548 | 0.548 | 0.548 | 0.548 | 0.548 | 0.548 |
| max | 10472.009 | 120.660 | 10472.009 | 113.636 | 10472.009 | 456.845 |
| n (Samp) | 158 | 10 | 158 | 11 | 158 | 3 |
| n (Pat) | 90 | 10 | 90 | 11 | 90 | 3 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 25.989 | 12.500 | 25.989 | 29.808 | 25.989 | 34.190 |
| average | 46.641 | 31.025 | 46.641 | 563.295 | 46.641 | 33.952 |
| stdev | 63.998 | 43.703 | 63.998 | 2166.573 | 63.998 | 29.595 |
| p (t-test) | | 0.278 | | 0.041 | | 0.696 |
| min | 0.548 | 0.548 | 0.548 | 3.497 | 0.548 | 7.112 |
| max | 292.857 | 157.407 | 292.857 | 10472.009 | 292.857 | 60.315 |
| n (Samp) | 74 | 23 | 74 | 23 | 74 | 4 |
| n (Pat) | 37 | 23 | 37 | 23 | 37 | 4 |

sCr or UO

| Time prior AKI stage | AUC | SE | nCohort 1 | nCohort 2 | p |
|---|---|---|---|---|---|
| 0 hours | 0.38 | 0.057 | 96 | 28 | 0.029 |
| 24 hours | 0.56 | 0.066 | 96 | 25 | 0.337 |
| 48 hours | 0.69 | 0.297 | 96 | 1 | 0.517 |

sCr only

| Time prior AKI stage | AUC | SE | nCohort 1 | nCohort 2 | p |
|---|---|---|---|---|---|
| 0 hours | 0.43 | 0.089 | 158 | 10 | 0.401 |
| 24 hours | 0.38 | 0.081 | 158 | 11 | 0.152 |
| 48 hours | 0.37 | 0.147 | 158 | 3 | 0.374 |

UO only

| Time prior AKI stage | AUC | SE | nCohort 1 | nCohort 2 | p |
|---|---|---|---|---|---|
| 0 hours | 0.43 | 0.067 | 74 | 23 | 0.317 |
| 24 hours | 0.64 | 0.069 | 74 | 23 | 0.040 |
| 48 hours | 0.54 | 0.152 | 74 | 4 | 0.807 |

sCr or UO

| Time prior AKI stage | Cutoff value | sens | spec | Quartile | OR | 95% CI of OR |
|---|---|---|---|---|---|---|

# Fig. 1 - 3

| Time prior AKI stage | Cutoff value | sens | spec | Quartile | OR | 95% CI of OR | |
|---|---|---|---|---|---|---|---|
| 0 hours | 3.84615 | 71% | 17% | 1 | | | |
| | 0.54775 | 82% | 11% | 2 | 0.8 | 0.3 | 2.2 |
| | 0 | 100% | 0% | 3 | 2.1 | 1.0 | 4.6 |
| | 61.2691 | 18% | 71% | 4 | 2.5 | 1.2 | 5.3 |
| | 77.2569 | 14% | 80% | | | | |
| | 167.857 | 0% | 91% | | | | |
| 24 hours | 12.035 | 72% | 28% | 1 | | | |
| | 7.86713 | 80% | 23% | 2 | 0.7 | 0.3 | 1.5 |
| | 2.18818 | 96% | 14% | 3 | 0.5 | 0.2 | 1.3 |
| | 61.2691 | 40% | 71% | 4 | 1.3 | 0.7 | 2.6 |
| | 77.2569 | 36% | 80% | | | | |
| | 167.857 | 20% | 91% | | | | |
| 48 hours | 59.8958 | 100% | 69% | 1 | | | |
| | 59.8958 | 100% | 69% | 2 | na | na | na |
| | 59.8958 | 100% | 69% | 3 | na | na | na |
| | 61.2691 | 0% | 71% | 4 | na | na | na |
| | 77.2569 | 0% | 80% | | | | |
| | 167.857 | 0% | 91% | | | | |

sCr only

| Time prior AKI stage | Cutoff value | sens | spec | Quartile | OR | 95% CI of OR | |
|---|---|---|---|---|---|---|---|
| 0 hours | 7.1116 | 70% | 21% | 1 | | | |
| | 4.64989 | 80% | 20% | 2 | 0.3 | 0.0 | 4.8 |
| | 3.84615 | 90% | 18% | 3 | 0.3 | 0.0 | 4.8 |
| | 59.8958 | 30% | 70% | 4 | 1.8 | 0.6 | 5.5 |
| | 83.3333 | 20% | 80% | | | | |
| | 160.088 | 0% | 91% | | | | |
| 24 hours | 3.84615 | 73% | 18% | 1 | | | |
| | 2.18818 | 82% | 15% | 2 | 4.4 | 0.3 | 56.5 |
| | 0 | 100% | 0% | 3 | 2.1 | 0.1 | 43.7 |
| | 59.8958 | 9% | 70% | 4 | 4.4 | 0.3 | 56.5 |
| | 83.3333 | 9% | 80% | | | | |
| | 160.088 | 0% | 91% | | | | |
| 48 hours | 0 | 100% | 0% | 1 | | | |
| | 0 | 100% | 0% | 2 | 0.0 | 0.0 | na |
| | 0 | 100% | 0% | 3 | 0.0 | 0.0 | na |
| | 59.8958 | 33% | 70% | 4 | 2.1 | 0.1 | 44.0 |
| | 83.3333 | 33% | 80% | | | | |
| | 160.088 | 33% | 91% | | | | |

UO only

| Time prior AKI stage | Cutoff value | sens | spec | Quartile | OR | 95% CI of OR | |
|---|---|---|---|---|---|---|---|
| 0 hours | 2.18818 | 78% | 20% | 1 | | | |
| | 0.54775 | 83% | 19% | 2 | 0.6 | 0.2 | 2.0 |
| | 0 | 100% | 0% | 3 | 2.0 | 0.8 | 4.7 |
| | 51.2153 | 22% | 72% | 4 | 1.6 | 0.7 | 4.0 |
| | 61.2691 | 13% | 81% | | | | |
| | 113.636 | 9% | 91% | | | | |
| 24 hours | 16.4931 | 78% | 45% | 1 | | | |
| | 12.035 | 83% | 36% | 2 | 13.8 | 1.3 | 151.2 |
| | 7.86713 | 91% | 32% | 3 | 4.6 | 0.3 | 64.0 |
| | 51.2153 | 43% | 72% | 4 | 12.9 | 1.2 | 140.6 |
| | 61.2691 | 39% | 81% | | | | |
| | 113.636 | 30% | 91% | | | | |
| 48 hours | 7.86713 | 75% | 32% | 1 | | | |
| | 4.64989 | 100% | 26% | 2 | na | na | na |
| | 4.64989 | 100% | 26% | 3 | na | na | na |
| | 51.2153 | 50% | 72% | 4 | na | na | na |
| | 61.2691 | 0% | 81% | | | | |
| | 113.636 | 0% | 91% | | | | |

Fig. 1 - 4

**Angiopoietin-1 receptor**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.028 | 0.014 | 0.028 | 0.040 | 0.028 | 0.009 |
| average | 0.094 | 0.034 | 0.094 | 0.093 | 0.094 | 0.009 |
| stdev | 0.165 | 0.057 | 0.165 | 0.114 | 0.165 | 0.013 |
| p (t-test) | | 0.350 | | 0.989 | | 0.475 |
| min | 0.000 | 0.000 | 0.000 | 0.000 | 0.000 | 0.000 |
| max | 1.005 | 0.161 | 1.005 | 0.330 | 1.005 | 0.018 |
| n (Samp) | 78 | 7 | 78 | 12 | 78 | 2 |
| n (Pat) | 19 | 7 | 19 | 12 | 19 | 2 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.018 | 0.023 | 0.018 | 0.120 | 0.018 | 0.000 |
| average | 0.075 | 0.052 | 0.075 | 0.111 | 0.075 | 0.016 |
| stdev | 0.142 | 0.076 | 0.142 | 0.102 | 0.142 | 0.026 |
| p (t-test) | | 0.744 | | 0.548 | | 0.470 |
| min | 0.000 | 0.000 | 0.000 | 0.000 | 0.000 | 0.000 |
| max | 1.005 | 0.161 | 1.005 | 0.272 | 1.005 | 0.046 |
| n (Samp) | 118 | 4 | 118 | 6 | 118 | 3 |
| n (Pat) | 26 | 4 | 26 | 6 | 26 | 3 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.028 | 0.013 | 0.028 | 0.013 | 0.028 | 0.009 |
| average | 0.099 | 0.013 | 0.099 | 0.059 | 0.099 | 0.009 |
| stdev | 0.179 | 0.012 | 0.179 | 0.107 | 0.179 | 0.013 |
| p (t-test) | | 0.251 | | 0.504 | | 0.487 |
| min | 0.000 | 0.000 | 0.000 | 0.000 | 0.000 | 0.000 |
| max | 1.005 | 0.033 | 1.005 | 0.330 | 1.005 | 0.018 |
| n (Samp) | 60 | 6 | 60 | 10 | 60 | 2 |
| n (Pat) | 14 | 6 | 14 | 10 | 14 | 2 |

sCr or UO

| Time prior AKI stage | AUC | SE | nCohort 1 | nCohort 2 | p |
|---|---|---|---|---|---|
| 0 hours | 0.40 | 0.105 | 78 | 7 | 0.334 |
| 24 hours | 0.51 | 0.091 | 78 | 12 | 0.874 |
| 48 hours | 0.27 | 0.150 | 78 | 2 | 0.123 |

sCr only

| Time prior AKI stage | AUC | SE | nCohort 1 | nCohort 2 | p |
|---|---|---|---|---|---|
| 0 hours | 0.45 | 0.141 | 118 | 4 | 0.708 |
| 24 hours | 0.63 | 0.125 | 118 | 6 | 0.307 |
| 48 hours | 0.31 | 0.133 | 118 | 3 | 0.149 |

UO only

| Time prior AKI stage | AUC | SE | nCohort 1 | nCohort 2 | p |
|---|---|---|---|---|---|
| 0 hours | 0.31 | 0.100 | 60 | 6 | 0.053 |
| 24 hours | 0.39 | 0.091 | 60 | 10 | 0.240 |
| 48 hours | 0.25 | 0.144 | 60 | 2 | 0.082 |

sCr or UO

| Time prior AKI stage | Cutoff value | sens | spec | Quartile | OR | 95% CI of OR | |
|---|---|---|---|---|---|---|---|
| 0 hours | 0.00919 | 71% | 36% | 1 | | | |
| | 0 | 100% | 0% | 2 | 1.1 | 0.0 | 64.1 |

## Fig. 1 - 5

| | 0 | 100% | 0% | 3 | 3.5 | 0.2 | 58.5 |
|---|---|---|---|---|---|---|---|
| | 0.08272 | 14% | 71% | 4 | 2.2 | 0.1 | 50.9 |
| | 0.11949 | 14% | 81% | | | | |
| | 0.3234 | 0% | 91% | | | | |
| 24 hours | 0 | 100% | 0% | 1 | | | |
| | 0 | 100% | 0% | 2 | 0.4 | 0.1 | 2.3 |
| | 0 | 100% | 0% | 3 | 0.2 | 0.0 | 3.0 |
| | 0.08272 | 42% | 71% | 4 | 1.3 | 0.4 | 3.8 |
| | 0.11949 | 33% | 81% | | | | |
| | 0.3234 | 8% | 91% | | | | |
| 48 hours | 0 | 100% | 0% | 1 | | | |
| | 0 | 100% | 0% | 2 | na | na | na |
| | 0 | 100% | 0% | 3 | na | na | na |
| | 0.08272 | 0% | 71% | 4 | na | na | na |
| | 0.11949 | 0% | 81% | | | | |
| | 0.3234 | 0% | 91% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.0 | 0.0 | 59.6 |
| | 0 | 100% | 0% | 3 | 0.0 | 0.0 | na |
| | 0.04596 | 50% | 70% | 4 | 2.1 | 0.1 | 46.4 |
| | 0.09795 | 25% | 81% | | | | |
| | 0.2567 | 0% | 91% | | | | |
| 24 hours | 0.00029 | 83% | 22% | 1 | | | |
| | 0.00029 | 83% | 22% | 2 | 0.0 | 0.0 | na |
| | 0 | 100% | 0% | 3 | 0.0 | 0.0 | na |
| | 0.04596 | 67% | 70% | 4 | 2.1 | 0.4 | 10.7 |
| | 0.09795 | 67% | 81% | | | | |
| | 0.2567 | 17% | 91% | | | | |
| 48 hours | 0 | 100% | 0% | 1 | | | |
| | 0 | 100% | 0% | 2 | na | na | na |
| | 0 | 100% | 0% | 3 | na | na | na |
| | 0.04596 | 33% | 70% | 4 | na | na | na |
| | 0.09795 | 0% | 81% | | | | |
| | 0.2567 | 0% | 91% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | na | na | na |
| | 0 | 100% | 0% | 3 | na | na | na |
| | 0.06685 | 0% | 70% | 4 | na | na | na |
| | 0.11949 | 0% | 82% | | | | |
| | 0.3234 | 0% | 90% | | | | |
| 24 hours | 0 | 100% | 0% | 1 | | | |
| | 0 | 100% | 0% | 2 | 0.5 | 0.0 | 12.1 |
| | 0 | 100% | 0% | 3 | 1.6 | 0.2 | 10.6 |
| | 0.06685 | 20% | 70% | 4 | 2.5 | 0.4 | 14.1 |
| | 0.11949 | 20% | 82% | | | | |
| | 0.3234 | 10% | 90% | | | | |
| 48 hours | 0 | 100% | 0% | 1 | | | |
| | 0 | 100% | 0% | 2 | na | na | na |
| | 0 | 100% | 0% | 3 | na | na | na |
| | 0.06685 | 0% | 70% | 4 | na | na | na |
| | 0.11949 | 0% | 82% | | | | |
| | 0.3234 | 0% | 90% | | | | |

Fig. 1 - 6

**ENA-78 (C-X-C chemokine motif 5)**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.015 | 0.022 | 0.015 | 0.022 | 0.015 | 0.018 |
| average | 0.034 | 0.032 | 0.034 | 0.147 | 0.034 | 0.117 |
| stdev | 0.108 | 0.050 | 0.108 | 0.660 | 0.108 | 0.337 |
| p (t-test) | | 0.906 | | 0.010 | | 0.006 |
| min | 0.000 | 0.000 | 0.000 | 0.000 | 0.000 | 0.000 |
| max | 1.460 | 0.324 | 1.460 | 5.180 | 1.460 | 1.530 |
| n (Samp) | 249 | 53 | 249 | 62 | 249 | 27 |
| n (Pat) | 104 | 53 | 104 | 62 | 104 | 27 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.016 | 0.019 | 0.016 | 0.029 | 0.016 | 0.024 |
| average | 0.060 | 0.027 | 0.060 | 0.066 | 0.060 | 0.035 |
| stdev | 0.300 | 0.035 | 0.300 | 0.108 | 0.300 | 0.044 |
| p (t-test) | | 0.617 | | 0.928 | | 0.755 |
| min | 0.000 | 0.000 | 0.000 | 0.000 | 0.000 | 0.005 |
| max | 5.180 | 0.162 | 5.180 | 0.457 | 5.180 | 0.176 |
| n (Samp) | 441 | 20 | 441 | 26 | 441 | 14 |
| n (Pat) | 170 | 20 | 170 | 26 | 170 | 14 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.015 | 0.023 | 0.015 | 0.022 | 0.015 | 0.018 |
| average | 0.037 | 0.034 | 0.037 | 0.156 | 0.037 | 0.119 |
| stdev | 0.116 | 0.052 | 0.116 | 0.718 | 0.116 | 0.350 |
| p (t-test) | | 0.894 | | 0.021 | | 0.014 |
| min | 0.000 | 0.000 | 0.000 | 0.000 | 0.000 | 0.000 |
| max | 1.460 | 0.324 | 1.460 | 5.180 | 1.460 | 1.530 |
| n (Samp) | 212 | 47 | 212 | 52 | 212 | 25 |
| n (Pat) | 85 | 47 | 85 | 52 | 85 | 25 |

sCr or UO

| Time prior AKI stage | AUC | SE | nCohort 1 | nCohort 2 | p |
|---|---|---|---|---|---|
| 0 hours | 0.57 | 0.045 | 249 | 53 | 0.127 |
| 24 hours | 0.63 | 0.041 | 249 | 62 | 0.001 |
| 48 hours | 0.58 | 0.060 | 249 | 27 | 0.210 |

sCr only

| Time prior AKI stage | AUC | SE | nCohort 1 | nCohort 2 | p |
|---|---|---|---|---|---|
| 0 hours | 0.52 | 0.067 | 441 | 20 | 0.718 |
| 24 hours | 0.66 | 0.060 | 441 | 26 | 0.008 |
| 48 hours | 0.61 | 0.081 | 441 | 14 | 0.185 |

UO only

| Time prior AKI stage | AUC | SE | nCohort 1 | nCohort 2 | p |
|---|---|---|---|---|---|
| 0 hours | 0.59 | 0.047 | 212 | 47 | 0.061 |
| 24 hours | 0.64 | 0.045 | 212 | 52 | 0.002 |
| 48 hours | 0.57 | 0.063 | 212 | 25 | 0.246 |

sCr or UO

| Time prior AKI stage | Cutoff value | sens | spec | Quartile | OR | 95% CI of OR | |
|---|---|---|---|---|---|---|---|
| 0 hours | 0.0112 | 72% | 37% | 1 | | | |
| | 0.00727 | 81% | 30% | 2 | 1.2 | 0.8 | 1.9 |
| | 0 | 100% | 0% | 3 | 1.5 | 1.0 | 2.2 |
| | 0.0222 | 47% | 71% | 4 | 1.9 | 1.3 | 2.7 |

## Fig. 1 - 7

| | Cutoff value | sens | spec | Quartile | OR | 95% CI of OR | |
|---|---|---|---|---|---|---|---|
| | 0.0299 | 25% | 80% | | | | |
| | 0.0464 | 17% | 90% | | | | |
| 24 hours | 0.0118 | 71% | 39% | 1 | | | |
| | 0.00907 | 81% | 32% | 2 | 1.7 | 1.1 | 2.5 |
| | 0.00533 | 90% | 21% | 3 | 1.8 | 1.2 | 2.7 |
| | 0.0222 | 48% | 71% | 4 | 3.4 | 2.3 | 4.8 |
| | 0.0299 | 35% | 80% | | | | |
| | 0.0464 | 29% | 90% | | | | |
| 48 hours | 0.0117 | 70% | 38% | 1 | | | |
| | 0.0093 | 81% | 33% | 2 | 1.8 | 0.8 | 4.2 |
| | 0.00513 | 93% | 21% | 3 | 2.4 | 1.1 | 5.3 |
| | 0.0222 | 33% | 71% | 4 | 1.8 | 0.8 | 4.2 |
| | 0.0299 | 26% | 80% | | | | |
| | 0.0464 | 19% | 90% | | | | |

sCr only

| Time prior AKI stage | Cutoff value | sens | spec | Quartile | OR | 95% CI of OR | |
|---|---|---|---|---|---|---|---|
| 0 hours | 0.0116 | 75% | 35% | 1 | | | |
| | 0.0106 | 80% | 33% | 2 | 2.1 | 0.7 | 5.7 |
| | 0.0003 | 90% | 14% | 3 | 2.4 | 0.9 | 6.4 |
| | 0.0254 | 35% | 70% | 4 | 1.3 | 0.4 | 4.3 |
| | 0.0342 | 20% | 80% | | | | |
| | 0.0604 | 10% | 90% | | | | |
| 24 hours | 0.0213 | 73% | 63% | 1 | | | |
| | 0.0117 | 81% | 35% | 2 | 1.3 | 0.4 | 4.3 |
| | 0 | 100% | 0% | 3 | 2.0 | 0.7 | 5.6 |
| | 0.0254 | 54% | 70% | 4 | 4.7 | 2.0 | 10.9 |
| | 0.0342 | 46% | 80% | | | | |
| | 0.0604 | 23% | 90% | | | | |
| 48 hours | 0.0182 | 71% | 55% | 1 | | | |
| | 0.01 | 86% | 30% | 2 | 3.0 | 0.2 | 42.8 |
| | 0.00953 | 93% | 30% | 3 | 7.3 | 0.8 | 71.3 |
| | 0.0254 | 50% | 70% | 4 | 3.0 | 0.2 | 42.8 |
| | 0.0342 | 14% | 80% | | | | |
| | 0.0604 | 14% | 90% | | | | |

UO only

| Time prior AKI stage | Cutoff value | sens | spec | Quartile | OR | 95% CI of OR | |
|---|---|---|---|---|---|---|---|
| 0 hours | 0.0116 | 70% | 35% | 1 | | | |
| | 0.00842 | 81% | 28% | 2 | 1.5 | 0.9 | 2.6 |
| | 0 | 100% | 0% | 3 | 2.4 | 1.5 | 4.0 |
| | 0.0222 | 53% | 71% | 4 | 2.4 | 1.5 | 4.0 |
| | 0.0309 | 23% | 80% | | | | |
| | 0.0503 | 13% | 90% | | | | |
| 24 hours | 0.0154 | 71% | 52% | 1 | | | |
| | 0.0104 | 81% | 33% | 2 | 1.1 | 0.7 | 1.8 |
| | 0.00663 | 90% | 24% | 3 | 1.9 | 1.2 | 2.8 |
| | 0.0222 | 48% | 71% | 4 | 2.4 | 1.6 | 3.5 |
| | 0.0309 | 35% | 80% | | | | |
| | 0.0503 | 27% | 90% | | | | |
| 48 hours | 0.0117 | 72% | 37% | 1 | | | |
| | 0.0093 | 80% | 31% | 2 | 1.6 | 0.6 | 3.8 |
| | 0.00497 | 92% | 18% | 3 | 1.9 | 0.8 | 4.3 |
| | 0.0222 | 40% | 71% | 4 | 2.1 | 0.9 | 4.7 |
| | 0.0309 | 28% | 80% | | | | |
| | 0.0503 | 16% | 90% | | | | |

# Fig. 1 - 8

**Endoglin**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.018 | 0.004 | 0.018 | 0.052 | 0.018 | 0.004 |
| average | 0.025 | 0.026 | 0.025 | 0.052 | 0.025 | 0.004 |
| stdev | 0.042 | 0.055 | 0.042 | 0.069 | 0.042 | na |
| p (t-test) | | 0.961 | | 0.383 | | na |
| min | 0.004 | 0.004 | 0.004 | 0.004 | 0.004 | 0.004 |
| max | 0.199 | 0.199 | 0.199 | 0.101 | 0.199 | 0.004 |
| n (Samp) | 54 | 13 | 54 | 2 | 54 | 1 |
| n (Pat) | 36 | 13 | 36 | 2 | 36 | 1 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.011 | 0.011 | 0.011 | 0.007 | 0.011 | 0.004 |
| average | 0.024 | 0.019 | 0.024 | 0.030 | 0.024 | 0.004 |
| stdev | 0.040 | 0.024 | 0.040 | 0.048 | 0.040 | 0.000 |
| p (t-test) | | 0.759 | | 0.765 | | 0.476 |
| min | 0.004 | 0.004 | 0.004 | 0.004 | 0.004 | 0.004 |
| max | 0.199 | 0.064 | 0.199 | 0.101 | 0.199 | 0.004 |
| n (Samp) | 86 | 6 | 86 | 4 | 86 | 2 |
| n (Pat) | 60 | 6 | 60 | 4 | 60 | 2 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.018 | 0.004 | 0.018 | 0.011 | 0.018 | 0.004 |
| average | 0.031 | 0.023 | 0.031 | 0.011 | 0.031 | 0.004 |
| stdev | 0.047 | 0.056 | 0.047 | 0.010 | 0.047 | na |
| p (t-test) | | 0.624 | | 0.555 | | na |
| min | 0.004 | 0.004 | 0.004 | 0.004 | 0.004 | 0.004 |
| max | 0.199 | 0.199 | 0.199 | 0.018 | 0.199 | 0.004 |
| n (Samp) | 40 | 12 | 40 | 2 | 40 | 1 |
| n (Pat) | 26 | 12 | 26 | 2 | 26 | 1 |

sCr or UO

| Time prior AKI stage | AUC | SE | nCohort 1 | nCohort 2 | p |
|---|---|---|---|---|---|
| 0 hours | 0.38 | 0.082 | 54 | 13 | 0.152 |
| 24 hours | 0.54 | 0.214 | 54 | 2 | 0.846 |
| 48 hours | 0.14 | 0.129 | 54 | 1 | 0.005 |

sCr only

| Time prior AKI stage | AUC | SE | nCohort 1 | nCohort 2 | p |
|---|---|---|---|---|---|
| 0 hours | 0.46 | 0.119 | 86 | 6 | 0.745 |
| 24 hours | 0.45 | 0.143 | 86 | 4 | 0.722 |
| 48 hours | 0.17 | 0.109 | 86 | 2 | 0.003 |

UO only

| Time prior AKI stage | AUC | SE | nCohort 1 | nCohort 2 | p |
|---|---|---|---|---|---|
| 0 hours | 0.30 | 0.080 | 40 | 12 | 0.015 |
| 24 hours | 0.34 | 0.179 | 40 | 2 | 0.383 |
| 48 hours | 0.13 | 0.121 | 40 | 1 | 0.002 |

sCr or UO

| Time prior AKI stage | Cutoff value | sens | spec | Quartile | OR | 95% CI of OR |
|---|---|---|---|---|---|---|
| 0 hours | 0 | 100% | 0% | 1 | | |

## Fig. 1 - 9

| | Cutoff value | sens | spec | Quartile | OR | 95% CI of OR | |
|---|---|---|---|---|---|---|---|
| | 0 | 100% | 0% | 2 | 1.0 | 0.1 | 9.2 |
| | 0 | 100% | 0% | 3 | 1.6 | 0.2 | 10.8 |
| | 0.01838 | 15% | 80% | 4 | 4.5 | 0.9 | 23.0 |
| | 0.04136 | 15% | 89% | | | | |
| | 0.04563 | 15% | 91% | | | | |
| 24 hours | 0 | 100% | 0% | 1 | | | |
| | 0 | 100% | 0% | 2 | na | na | na |
| | 0 | 100% | 0% | 3 | na | na | na |
| | 0.01838 | 50% | 80% | 4 | na | na | na |
| | 0.04136 | 50% | 89% | | | | |
| | 0.04563 | 50% | 91% | | | | |
| 48 hours | 0 | 100% | 0% | 1 | | | |
| | 0 | 100% | 0% | 2 | na | na | na |
| | 0 | 100% | 0% | 3 | na | na | na |
| | 0.01838 | 0% | 80% | 4 | na | na | na |
| | 0.04136 | 0% | 89% | | | | |
| | 0.04563 | 0% | 91% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 2.1 | 0.1 | 47.6 |
| | 0 | 100% | 0% | 3 | 0.0 | 0.0 | na |
| | 0.01838 | 17% | 78% | 4 | 3.3 | 0.2 | 54.3 |
| | 0.0318 | 17% | 80% | | | | |
| | 0.04136 | 17% | 91% | | | | |
| 24 hours | 0 | 100% | 0% | 1 | | | |
| | 0 | 100% | 0% | 2 | 0.0 | 0.0 | na |
| | 0 | 100% | 0% | 3 | 3.3 | 0.2 | 54.3 |
| | 0.01838 | 25% | 78% | 4 | 0.0 | 0.0 | na |
| | 0.0318 | 25% | 80% | | | | |
| | 0.04136 | 25% | 91% | | | | |
| 48 hours | 0 | 100% | 0% | 1 | | | |
| | 0 | 100% | 0% | 2 | na | na | na |
| | 0 | 100% | 0% | 3 | na | na | na |
| | 0.01838 | 0% | 78% | 4 | na | na | na |
| | 0.0318 | 0% | 80% | | | | |
| | 0.04136 | 0% | 91% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 2.2 | 0.1 | 58.1 |
| | 0 | 100% | 0% | 3 | 7.5 | 0.5 | 118.5 |
| | 0.01838 | 8% | 75% | 4 | 5.3 | 0.3 | 90.5 |
| | 0.04136 | 8% | 88% | | | | |
| | 0.06434 | 8% | 90% | | | | |
| 24 hours | 0 | 100% | 0% | 1 | | | |
| | 0 | 100% | 0% | 2 | na | na | na |
| | 0 | 100% | 0% | 3 | na | na | na |
| | 0.01838 | 0% | 75% | 4 | na | na | na |
| | 0.04136 | 0% | 88% | | | | |
| | 0.06434 | 0% | 90% | | | | |
| 48 hours | 0 | 100% | 0% | 1 | | | |
| | 0 | 100% | 0% | 2 | na | na | na |
| | 0 | 100% | 0% | 3 | na | na | na |
| | 0.01838 | 0% | 75% | 4 | na | na | na |
| | 0.04136 | 0% | 88% | | | | |
| | 0.06434 | 0% | 90% | | | | |

Fig. 1 - 10

**Epithelial cell-adhesion molecule**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 2.423 | 0.801 | 2.423 | 54.474 | 2.423 | na |
| average | 130.527 | 0.774 | 130.527 | 54.474 | 130.527 | na |
| stdev | 664.486 | 0.081 | 664.486 | 53.629 | 664.486 | na |
| p (t-test) | | 0.563 | | 0.873 | | na |
| min | 0.801 | 0.559 | 0.801 | 16.553 | 0.801 | na |
| max | 4516.667 | 0.801 | 4516.667 | 92.396 | 4516.667 | na |
| n (Samp) | 47 | 9 | 47 | 2 | 47 | 0 |
| n (Pat) | 30 | 9 | 30 | 2 | 30 | 0 |

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 0.801 | 0.801 | 0.801 | 66.557 | 0.801 | 0.801 |
| average | 91.074 | 14.918 | 91.074 | 56.578 | 91.074 | 0.801 |
| stdev | 549.646 | 31.566 | 549.646 | 43.448 | 549.646 | 0.000 |
| p (t-test) | | 0.759 | | 0.901 | | 0.818 |
| min | 0.559 | 0.801 | 0.559 | 0.801 | 0.559 | 0.801 |
| max | 4516.667 | 71.384 | 4516.667 | 92.396 | 4516.667 | 0.801 |
| n (Samp) | 69 | 5 | 69 | 4 | 69 | 2 |
| n (Pat) | 46 | 5 | 46 | 4 | 46 | 2 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 6.151 | 0.801 | 6.151 | 14.148 | 6.151 | na |
| average | 173.588 | 0.774 | 173.588 | 14.148 | 173.588 | na |
| stdev | 779.637 | 0.081 | 779.637 | 3.402 | 779.637 | na |
| p (t-test) | | 0.514 | | 0.777 | | na |
| min | 0.801 | 0.559 | 0.801 | 11.742 | 0.801 | na |
| max | 4516.667 | 0.801 | 4516.667 | 16.553 | 4516.667 | na |
| n (Samp) | 34 | 9 | 34 | 2 | 34 | 0 |
| n (Pat) | 20 | 9 | 20 | 2 | 20 | 0 |

sCr or UO

| Time prior AKI stage | AUC | SE | nCohort 1 | nCohort 2 | p |
|---|---|---|---|---|---|
| 0 hours | 0.22 | 0.072 | 47 | 9 | 0.000 |
| 24 hours | 0.78 | 0.198 | 47 | 2 | 0.163 |
| 48 hours | nd | nd | 47 | 0 | nd |

sCr only

| Time prior AKI stage | AUC | SE | nCohort 1 | nCohort 2 | p |
|---|---|---|---|---|---|
| 0 hours | 0.42 | 0.126 | 69 | 5 | 0.528 |
| 24 hours | 0.76 | 0.143 | 69 | 4 | 0.065 |
| 48 hours | 0.30 | 0.160 | 69 | 2 | 0.206 |

UO only

| Time prior AKI stage | AUC | SE | nCohort 1 | nCohort 2 | p |
|---|---|---|---|---|---|
| 0 hours | 0.17 | 0.066 | 34 | 9 | 0.000 |
| 24 hours | 0.57 | 0.219 | 34 | 2 | 0.737 |
| 48 hours | nd | nd | 34 | 0 | nd |

Fig. 1 - 11

sCr only

| Time prior AKI stage | Cutoff value | sens | spec | Quartile | OR | 95% CI of OR | |
|---|---|---|---|---|---|---|---|
| 0 hours | 0.55915 | 100% | 3% | 1 | | | |
| | 0.55915 | 100% | 3% | 2 | 0.0 | 0.0 | na |
| | 0.55915 | 100% | 3% | 3 | 4.8 | 0.3 | 70.7 |
| | 15.4697 | 20% | 71% | 4 | 0.0 | 0.0 | na |
| | 24.7888 | 20% | 81% | | | | |
| | 49.0184 | 20% | 91% | | | | |
| 24 hours | 39.6993 | 75% | 90% | 1 | | | |
| | 0.55915 | 100% | 3% | 2 | na | na | na |
| | 0.55915 | 100% | 3% | 3 | na | na | na |
| | 15.4697 | 75% | 71% | 4 | na | na | na |
| | 24.7888 | 75% | 81% | | | | |
| | 49.0184 | 50% | 91% | | | | |
| 48 hours | 0.55915 | 100% | 3% | 1 | | | |
| | 0.55915 | 100% | 3% | 2 | na | na | na |
| | 0.55915 | 100% | 3% | 3 | na | na | na |
| | 15.4697 | 0% | 71% | 4 | na | na | na |
| | 24.7888 | 0% | 81% | | | | |
| | 49.0184 | 0% | 91% | | | | |

# Fig. 1 - 12

Heme oxygenase 1

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.003 | 0.003 | 0.003 | 0.003 | 0.003 | 0.003 |
| average | 0.054 | 0.043 | 0.054 | 0.104 | 0.054 | 0.003 |
| stdev | 0.109 | 0.090 | 0.109 | 0.347 | 0.109 | 0.000 |
| p (t-test) | | 0.602 | | 0.175 | | 0.419 |
| min | 0.000 | 0.000 | 0.000 | 0.000 | 0.000 | 0.003 |
| max | 0.537 | 0.472 | 0.537 | 1.935 | 0.537 | 0.003 |
| n (Samp) | 122 | 30 | 122 | 32 | 122 | 3 |
| n (Pat) | 49 | 30 | 49 | 32 | 49 | 3 |

sCr only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.003 | 0.003 | 0.003 | 0.013 | 0.003 | 0.014 |
| average | 0.068 | 0.027 | 0.068 | 0.071 | 0.068 | 0.110 |
| stdev | 0.175 | 0.048 | 0.175 | 0.141 | 0.175 | 0.198 |
| p (t-test) | | 0.456 | | 0.964 | | 0.641 |
| min | 0.000 | 0.000 | 0.000 | 0.003 | 0.000 | 0.003 |
| max | 1.935 | 0.135 | 1.935 | 0.505 | 1.935 | 0.407 |
| n (Samp) | 209 | 10 | 209 | 13 | 209 | 4 |
| n (Pat) | 90 | 10 | 90 | 13 | 90 | 4 |

UO only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.003 | 0.003 | 0.003 | 0.003 | 0.003 | 0.003 |
| average | 0.070 | 0.043 | 0.070 | 0.108 | 0.070 | 0.006 |
| stdev | 0.122 | 0.098 | 0.122 | 0.364 | 0.122 | 0.010 |
| p (t-test) | | 0.315 | | 0.394 | | 0.208 |
| min | 0.000 | 0.000 | 0.000 | 0.000 | 0.000 | 0.001 |
| max | 0.537 | 0.472 | 0.537 | 1.935 | 0.537 | 0.026 |
| n (Samp) | 91 | 24 | 91 | 29 | 91 | 6 |
| n (Pat) | 38 | 24 | 38 | 29 | 38 | 6 |

sCr or UO

| Time prior AKI stage | AUC | SE | nCohort 1 | nCohort 2 | p |
|---|---|---|---|---|---|
| 0 hours | 0.47 | 0.058 | 122 | 30 | 0.607 |
| 24 hours | 0.50 | 0.057 | 122 | 32 | 0.954 |
| 48 hours | 0.31 | 0.133 | 122 | 3 | 0.147 |

sCr only

| Time prior AKI stage | AUC | SE | nCohort 1 | nCohort 2 | p |
|---|---|---|---|---|---|
| 0 hours | 0.36 | 0.081 | 209 | 10 | 0.085 |
| 24 hours | 0.55 | 0.085 | 209 | 13 | 0.577 |
| 48 hours | 0.55 | 0.150 | 209 | 4 | 0.737 |

UO only

| Time prior AKI stage | AUC | SE | nCohort 1 | nCohort 2 | p |
|---|---|---|---|---|---|
| 0 hours | 0.43 | 0.064 | 91 | 24 | 0.295 |
| 24 hours | 0.45 | 0.061 | 91 | 29 | 0.454 |
| 48 hours | 0.29 | 0.093 | 91 | 6 | 0.023 |

sCr or UO

| Time prior AKI stage | Cutoff value | sens | spec | Quartile | OR | 95% CI of OR | |
|---|---|---|---|---|---|---|---|
| 0 hours | 0.00084 | 83% | 3% | 1 | | | |

Fig. 1 - 13

| | Cutoff value | sens | spec | Quartile | OR | 95% CI of OR | |
|---|---|---|---|---|---|---|---|
| | 0.00084 | 83% | 3% | 2 | 1.7 | 1.0 | 3.0 |
| | 2.8E-16 | 93% | 1% | 3 | 0.6 | 0.3 | 1.2 |
| | 0.03301 | 30% | 73% | 4 | 0.6 | 0.3 | 1.2 |
| | 0.08803 | 20% | 82% | | | | |
| | 0.16824 | 3% | 90% | | | | |
| 24 hours | 0.00084 | 88% | 3% | 1 | | | |
| | 0.00084 | 88% | 3% | 2 | 0.6 | 0.3 | 1.2 |
| | 2.8E-16 | 97% | 1% | 3 | 0.9 | 0.5 | 1.6 |
| | 0.03301 | 34% | 73% | 4 | 1.0 | 0.6 | 1.8 |
| | 0.08803 | 16% | 82% | | | | |
| | 0.16824 | 9% | 90% | | | | |
| 48 hours | 0.00084 | 100% | 3% | 1 | | | |
| | 0.00084 | 100% | 3% | 2 | na | na | na |
| | 0.00084 | 100% | 3% | 3 | na | na | na |
| | 0.03301 | 0% | 73% | 4 | na | na | na |
| | 0.08803 | 0% | 82% | | | | |
| | 0.16824 | 0% | 90% | | | | |

sCr only

| Time prior AKI stage | Cutoff value | sens | spec | Quartile | OR | 95% CI of OR | |
|---|---|---|---|---|---|---|---|
| 0 hours | 0.00084 | 70% | 5% | 1 | | | |
| | 2.8E-16 | 90% | 1% | 2 | 0.5 | 0.0 | 10.0 |
| | 2.8E-16 | 90% | 1% | 3 | 1.0 | 0.1 | 7.6 |
| | 0.03437 | 20% | 70% | 4 | 2.7 | 0.6 | 11.5 |
| | 0.08803 | 20% | 80% | | | | |
| | 0.16849 | 0% | 90% | | | | |
| 24 hours | 0.00084 | 100% | 5% | 1 | | | |
| | 0.00084 | 100% | 5% | 2 | 0.5 | 0.1 | 2.2 |
| | 0.00084 | 100% | 5% | 3 | 1.0 | 0.3 | 2.9 |
| | 0.03437 | 31% | 70% | 4 | 0.7 | 0.2 | 2.4 |
| | 0.08803 | 23% | 80% | | | | |
| | 0.16849 | 15% | 90% | | | | |
| 48 hours | 0.00084 | 100% | 5% | 1 | | | |
| | 0.00084 | 100% | 5% | 2 | 0.0 | 0.0 | na |
| | 0.00084 | 100% | 5% | 3 | 0.5 | 0.0 | 10.0 |
| | 0.03437 | 25% | 70% | 4 | 0.5 | 0.0 | 9.8 |
| | 0.08803 | 25% | 80% | | | | |
| | 0.16849 | 25% | 90% | | | | |

UO only

| Time prior AKI stage | Cutoff value | sens | spec | Quartile | OR | 95% CI of OR | |
|---|---|---|---|---|---|---|---|
| 0 hours | 0.00084 | 88% | 4% | 1 | | | |
| | 0.00084 | 88% | 4% | 2 | 2.0 | 0.8 | 5.1 |
| | 2.8E-16 | 96% | 1% | 3 | 1.0 | 0.3 | 3.1 |
| | 0.05113 | 25% | 70% | 4 | 3.0 | 1.2 | 7.2 |
| | 0.09973 | 8% | 80% | | | | |
| | 0.20032 | 4% | 90% | | | | |
| 24 hours | 0.00084 | 86% | 4% | 1 | | | |
| | 0.00084 | 86% | 4% | 2 | 3.3 | 1.4 | 7.7 |
| | 2.8E-16 | 97% | 1% | 3 | 1.3 | 0.5 | 3.7 |
| | 0.05113 | 28% | 70% | 4 | 3.3 | 1.4 | 7.7 |
| | 0.09973 | 10% | 80% | | | | |
| | 0.20032 | 7% | 90% | | | | |
| 48 hours | 0.00084 | 83% | 4% | 1 | | | |
| | 0.00084 | 83% | 4% | 2 | na | na | na |
| | 2.8E-16 | 100% | 1% | 3 | na | na | na |
| | 0.05113 | 0% | 70% | 4 | na | na | na |
| | 0.09973 | 0% | 80% | | | | |
| | 0.20032 | 0% | 90% | | | | |

Fig. 1 - 14

**Interleukin-1 receptor, type II**

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 869.159 | 1286.617 | 869.159 | 1037.171 | 869.159 | 1410.221 |
| average | 1781.114 | 1941.524 | 1781.114 | 2614.262 | 1781.114 | 2692.144 |
| stdev | 3312.993 | 1931.851 | 3312.993 | 5248.398 | 3312.993 | 3151.432 |
| p (t-test) | | 0.759 | | 0.235 | | 0.234 |
| min | 99.487 | 88.459 | 99.487 | 42.493 | 99.487 | 43.565 |
| max | 29514.673 | 7463.098 | 29514.673 | 34543.843 | 29514.673 | 13879.314 |
| n (Samp) | 98 | 47 | 98 | 53 | 98 | 23 |
| n (Pat) | 82 | 47 | 82 | 53 | 82 | 23 |

sCr only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 1151.288 | 506.675 | 1151.288 | 682.905 | 1151.288 | 910.679 |
| average | 2087.382 | 1002.847 | 2087.382 | 3693.298 | 2087.382 | 1791.424 |
| stdev | 3592.231 | 1241.593 | 3592.231 | 10355.644 | 3592.231 | 2234.747 |
| p (t-test) | | 0.231 | | 0.127 | | 0.787 |
| min | 43.565 | 117.458 | 43.565 | 42.493 | 43.565 | 187.043 |
| max | 34543.843 | 4625.832 | 34543.843 | 46544.423 | 34543.843 | 7323.191 |
| n (Samp) | 227 | 16 | 227 | 20 | 227 | 11 |
| n (Pat) | 136 | 16 | 136 | 20 | 136 | 11 |

UO only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 865.109 | 1417.027 | 865.109 | 1206.361 | 865.109 | 1311.712 |
| average | 1548.213 | 2094.529 | 1548.213 | 2791.356 | 1548.213 | 2313.870 |
| stdev | 1824.653 | 1976.365 | 1824.653 | 5469.928 | 1824.653 | 3036.217 |
| p (t-test) | | 0.126 | | 0.056 | | 0.133 |
| min | 83.202 | 88.459 | 83.202 | 58.099 | 83.202 | 43.565 |
| max | 11320.965 | 7463.098 | 11320.965 | 34543.843 | 11320.965 | 13879.314 |
| n (Samp) | 87 | 41 | 87 | 46 | 87 | 22 |
| n (Pat) | 68 | 41 | 68 | 46 | 68 | 22 |

sCr or UO

| Time prior AKI stage | AUC | SE | nCohort 1 | nCohort 2 | p |
| --- | --- | --- | --- | --- | --- |
| 0 hours | 0.55 | 0.052 | 98 | 47 | 0.330 |
| 24 hours | 0.53 | 0.050 | 98 | 53 | 0.548 |
| 48 hours | 0.61 | 0.068 | 98 | 23 | 0.109 |

sCr only

| Time prior AKI stage | AUC | SE | nCohort 1 | nCohort 2 | p |
| --- | --- | --- | --- | --- | --- |
| 0 hours | 0.34 | 0.063 | 227 | 16 | 0.014 |
| 24 hours | 0.40 | 0.062 | 227 | 20 | 0.124 |
| 48 hours | 0.48 | 0.088 | 227 | 11 | 0.842 |

UO only

| Time prior AKI stage | AUC | SE | nCohort 1 | nCohort 2 | p |
| --- | --- | --- | --- | --- | --- |
| 0 hours | 0.58 | 0.055 | 87 | 41 | 0.123 |
| 24 hours | 0.56 | 0.053 | 87 | 46 | 0.222 |
| 48 hours | 0.57 | 0.070 | 87 | 22 | 0.326 |

sCr or UO

| Time prior AKI stage | Cutoff value | sens | spec | Quartile | OR | 95% CI of OR | |
| --- | --- | --- | --- | --- | --- | --- | --- |
| 0 hours | 569.09 | 70% | 31% | 1 | | | |
| | 373.394 | 81% | 19% | 2 | 0.5 | 0.3 | 0.9 |

# Fig. 1 - 15

| Time prior AKI stage | Cutoff value | sens | spec | Quartile | OR | 95% CI of OR | |
|---|---|---|---|---|---|---|---|
| | 210.034 | 91% | 11% | 3 | 1.1 | 0.7 | 1.8 |
| | 1693.73 | 36% | 70% | 4 | 1.4 | 0.9 | 2.2 |
| | 2132.23 | 32% | 81% | | | | |
| | 3945.89 | 17% | 91% | | | | |
| 24 hours | 512.648 | 72% | 28% | 1 | | | |
| | 265.191 | 81% | 12% | 2 | 0.5 | 0.3 | 0.8 |
| | 101.623 | 91% | 3% | 3 | 0.8 | 0.5 | 1.2 |
| | 1693.73 | 40% | 70% | 4 | 1.5 | 1.0 | 2.3 |
| | 2132.23 | 34% | 81% | | | | |
| | 3945.89 | 15% | 91% | | | | |
| 48 hours | 780.757 | 74% | 48% | 1 | | | |
| | 512.648 | 83% | 28% | 2 | 1.0 | 0.3 | 3.1 |
| | 265.191 | 91% | 12% | 3 | 1.6 | 0.6 | 4.3 |
| | 1693.73 | 39% | 70% | 4 | 2.7 | 1.1 | 6.4 |
| | 2132.23 | 39% | 81% | | | | |
| | 3945.89 | 30% | 91% | | | | |

sCr only

| Time prior AKI stage | Cutoff value | sens | spec | Quartile | OR | 95% CI of OR | |
|---|---|---|---|---|---|---|---|
| 0 hours | 211.651 | 75% | 10% | 1 | | | |
| | 189.296 | 81% | 8% | 2 | 0.7 | 0.1 | 3.6 |
| | 127.052 | 94% | 4% | 3 | 1.7 | 0.6 | 5.3 |
| | 1991.61 | 19% | 70% | 4 | 2.1 | 0.8 | 6.1 |
| | 2896 | 6% | 80% | | | | |
| | 4391.64 | 6% | 90% | | | | |
| 24 hours | 210.034 | 70% | 10% | 1 | | | |
| | 164.297 | 80% | 8% | 2 | 0.6 | 0.2 | 1.8 |
| | 74.5128 | 90% | 1% | 3 | 1.0 | 0.4 | 2.3 |
| | 1991.61 | 25% | 70% | 4 | 1.5 | 0.7 | 3.1 |
| | 2896 | 20% | 80% | | | | |
| | 4391.64 | 10% | 90% | | | | |
| 48 hours | 780.757 | 73% | 42% | 1 | | | |
| | 349.911 | 82% | 19% | 2 | 0.0 | 0.0 | na |
| | 288.07 | 91% | 15% | 3 | 1.7 | 0.6 | 5.3 |
| | 1991.61 | 27% | 70% | 4 | 1.0 | 0.3 | 4.0 |
| | 2896 | 18% | 80% | | | | |
| | 4391.64 | 18% | 90% | | | | |

UO only

| Time prior AKI stage | Cutoff value | sens | spec | Quartile | OR | 95% CI of OR | |
|---|---|---|---|---|---|---|---|
| 0 hours | 642.878 | 71% | 39% | 1 | | | |
| | 402.52 | 80% | 25% | 2 | 0.6 | 0.3 | 1.2 |
| | 210.034 | 90% | 14% | 3 | 1.5 | 0.9 | 2.7 |
| | 1636.33 | 39% | 70% | 4 | 2.0 | 1.1 | 3.5 |
| | 2132.23 | 34% | 80% | | | | |
| | 3945.89 | 20% | 91% | | | | |
| 24 hours | 594.5 | 72% | 36% | 1 | | | |
| | 304.807 | 80% | 16% | 2 | 0.5 | 0.3 | 0.9 |
| | 125.969 | 91% | 6% | 3 | 0.8 | 0.4 | 1.3 |
| | 1636.33 | 46% | 70% | 4 | 1.8 | 1.1 | 2.8 |
| | 2132.23 | 39% | 80% | | | | |
| | 3945.89 | 17% | 91% | | | | |
| 48 hours | 553.247 | 73% | 32% | 1 | | | |
| | 479.669 | 82% | 30% | 2 | 1.0 | 0.3 | 3.2 |
| | 210.034 | 91% | 14% | 3 | 2.0 | 0.8 | 5.2 |
| | 1636.33 | 32% | 70% | 4 | 1.9 | 0.7 | 4.9 |
| | 2132.23 | 32% | 80% | | | | |
| | 3945.89 | 23% | 91% | | | | |

# Fig. 1 - 16

Interleukin-6 receptor subunit-alpha

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 610.302 | 922.138 | 610.302 | 946.188 | 610.302 | 590.466 |
| average | 805.761 | 1551.779 | 805.761 | 1545.193 | 805.761 | 1512.761 |
| stdev | 714.113 | 1485.995 | 714.113 | 1779.057 | 714.113 | 1849.312 |
| p (t-test) | | 0.000 | | 0.000 | | 0.002 |
| min | 11.815 | 33.944 | 11.815 | 18.938 | 11.815 | 75.375 |
| max | 3306.230 | 5912.275 | 3306.230 | 9197.343 | 3306.230 | 6090.745 |
| n (Samp) | 117 | 51 | 117 | 60 | 117 | 26 |
| n (Pat) | 99 | 51 | 99 | 60 | 99 | 26 |

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 736.931 | 346.229 | 736.931 | 349.370 | 736.931 | 518.642 |
| average | 1206.234 | 704.280 | 1206.234 | 1247.865 | 1206.234 | 1181.552 |
| stdev | 1334.174 | 1056.784 | 1334.174 | 2115.457 | 1334.174 | 1634.133 |
| p (t-test) | | 0.130 | | 0.892 | | 0.947 |
| min | 11.815 | 33.944 | 11.815 | 18.938 | 11.815 | 149.090 |
| max | 9197.343 | 4392.091 | 9197.343 | 9857.692 | 9197.343 | 6090.745 |
| n (Samp) | 261 | 17 | 261 | 23 | 261 | 14 |
| n (Pat) | 160 | 17 | 160 | 23 | 160 | 14 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 611.040 | 1255.765 | 611.040 | 1181.035 | 611.040 | 693.709 |
| average | 919.032 | 1713.951 | 919.032 | 1672.690 | 919.032 | 1426.171 |
| stdev | 998.852 | 1409.735 | 998.852 | 1848.966 | 998.852 | 1665.846 |
| p (t-test) | | 0.000 | | 0.001 | | 0.056 |
| min | 11.815 | 66.079 | 11.815 | 48.776 | 11.815 | 75.375 |
| max | 6090.745 | 5912.275 | 6090.745 | 9197.343 | 6090.745 | 6005.696 |
| n (Samp) | 105 | 45 | 105 | 50 | 105 | 23 |
| n (Pat) | 84 | 45 | 84 | 50 | 84 | 23 |

sCr or UO

| Time prior AKI stage | AUC | SE | nCohort 1 | nCohort 2 | p |
|---|---|---|---|---|---|
| 0 hours | 0.62 | 0.048 | 117 | 51 | 0.010 |
| 24 hours | 0.61 | 0.046 | 117 | 60 | 0.020 |
| 48 hours | 0.54 | 0.064 | 117 | 26 | 0.518 |

sCr only

| Time prior AKI stage | AUC | SE | nCohort 1 | nCohort 2 | p |
|---|---|---|---|---|---|
| 0 hours | 0.33 | 0.059 | 261 | 17 | 0.003 |
| 24 hours | 0.41 | 0.059 | 261 | 23 | 0.142 |
| 48 hours | 0.45 | 0.077 | 261 | 14 | 0.547 |

UO only

| Time prior AKI stage | AUC | SE | nCohort 1 | nCohort 2 | p |
|---|---|---|---|---|---|
| 0 hours | 0.69 | 0.049 | 105 | 45 | 0.000 |
| 24 hours | 0.64 | 0.049 | 105 | 50 | 0.005 |
| 48 hours | 0.54 | 0.068 | 105 | 23 | 0.522 |

sCr or UO

| Time prior AKI stage | Cutoff value | sens | spec | Quartile | OR | 95% CI of OR | |
|---|---|---|---|---|---|---|---|
| 0 hours | 484.169 | 71% | 40% | 1 | | | |
| | 312.153 | 80% | 26% | 2 | 0.8 | 0.5 | 1.3 |

# Fig. 1 - 17

| | 144.013 | 90% | 12% | 3 | 0.9 | 0.5 | 1.5 |
|---|---|---|---|---|---|---|---|
| | 895.184 | 51% | 70% | 4 | 2.8 | 1.8 | 4.3 |
| | 1135.97 | 45% | 80% | | | | |
| | 1944.85 | 33% | 91% | | | | |
| 24 hours | 353.727 | 70% | 29% | 1 | | | |
| | 270.654 | 80% | 22% | 2 | 0.6 | 0.3 | 0.9 |
| | 150.155 | 90% | 13% | 3 | 0.9 | 0.6 | 1.4 |
| | 895.184 | 55% | 70% | 4 | 2.4 | 1.7 | 3.6 |
| | 1135.97 | 48% | 80% | | | | |
| | 1944.85 | 25% | 91% | | | | |
| 48 hours | 312.153 | 73% | 26% | 1 | | | |
| | 204.698 | 81% | 18% | 2 | 0.8 | 0.4 | 1.7 |
| | 122.703 | 92% | 10% | 3 | 0.6 | 0.3 | 1.4 |
| | 895.184 | 38% | 70% | 4 | 1.1 | 0.6 | 2.2 |
| | 1135.97 | 31% | 80% | | | | |
| | 1944.85 | 31% | 91% | | | | |

sCr only

| Time prior AKI stage | Cutoff value | sens | spec | Quartile | OR | 95% CI of OR | |
|---|---|---|---|---|---|---|---|
| 0 hours | 193.069 | 71% | 15% | 1 | | | |
| | 161.405 | 82% | 12% | 2 | 1.5 | 0.3 | 8.4 |
| | 55.4155 | 94% | 3% | 3 | 2.1 | 0.4 | 9.5 |
| | 1285.26 | 12% | 70% | 4 | 4.5 | 1.2 | 16.1 |
| | 1902.6 | 6% | 80% | | | | |
| | 2908.65 | 6% | 90% | | | | |
| 24 hours | 166.041 | 74% | 13% | 1 | | | |
| | 117.292 | 83% | 7% | 2 | 0.6 | 0.3 | 1.6 |
| | 55.4155 | 91% | 3% | 3 | 0.3 | 0.1 | 1.2 |
| | 1285.26 | 26% | 70% | 4 | 2.0 | 1.1 | 3.5 |
| | 1902.6 | 22% | 80% | | | | |
| | 2908.65 | 13% | 90% | | | | |
| 48 hours | 312.153 | 71% | 23% | 1 | | | |
| | 242.127 | 86% | 17% | 2 | 2.1 | 0.4 | 9.5 |
| | 207.852 | 93% | 16% | 3 | 1.0 | 0.1 | 7.5 |
| | 1285.26 | 21% | 70% | 4 | 3.2 | 0.8 | 12.7 |
| | 1902.6 | 14% | 80% | | | | |
| | 2908.65 | 14% | 90% | | | | |

UO only

| Time prior AKI stage | Cutoff value | sens | spec | Quartile | OR | 95% CI of OR | |
|---|---|---|---|---|---|---|---|
| 0 hours | 656.228 | 71% | 56% | 1 | | | |
| | 516.407 | 80% | 44% | 2 | 2.3 | 1.1 | 4.7 |
| | 318.912 | 91% | 26% | 3 | 2.4 | 1.2 | 4.9 |
| | 951.709 | 60% | 70% | 4 | 7.1 | 3.7 | 13.8 |
| | 1202.71 | 51% | 80% | | | | |
| | 2238.21 | 33% | 90% | | | | |
| 24 hours | 582.607 | 70% | 49% | 1 | | | |
| | 346.229 | 80% | 29% | 2 | 1.0 | 0.5 | 1.7 |
| | 204.698 | 90% | 18% | 3 | 1.4 | 0.9 | 2.4 |
| | 951.709 | 52% | 70% | 4 | 3.4 | 2.1 | 5.5 |
| | 1202.71 | 50% | 80% | | | | |
| | 2238.21 | 22% | 90% | | | | |
| 48 hours | 318.912 | 74% | 26% | 1 | | | |
| | 176.045 | 83% | 16% | 2 | 0.8 | 0.3 | 1.9 |
| | 122.703 | 91% | 10% | 3 | 0.8 | 0.3 | 1.9 |
| | 951.709 | 35% | 70% | 4 | 1.2 | 0.6 | 2.6 |
| | 1202.71 | 30% | 80% | | | | |
| | 2238.21 | 26% | 90% | | | | |

Fig. 1 - 18

**Lymphotactin**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.002 | 0.002 | 0.002 | 0.002 | 0.002 | 0.002 |
| average | 0.006 | 0.007 | 0.006 | 0.012 | 0.006 | 0.013 |
| stdev | 0.015 | 0.017 | 0.015 | 0.022 | 0.015 | 0.021 |
| p (t-test) | | 0.757 | | 0.008 | | 0.036 |
| min | 0.002 | 0.002 | 0.002 | 0.002 | 0.002 | 0.002 |
| max | 0.133 | 0.088 | 0.133 | 0.112 | 0.133 | 0.076 |
| n (Samp) | 249 | 53 | 249 | 62 | 249 | 27 |
| n (Pat) | 104 | 53 | 104 | 62 | 104 | 27 |

sCr only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.002 | 0.002 | 0.002 | 0.002 | 0.002 | 0.002 |
| average | 0.007 | 0.006 | 0.007 | 0.015 | 0.007 | 0.006 |
| stdev | 0.015 | 0.013 | 0.015 | 0.029 | 0.015 | 0.013 |
| p (t-test) | | 0.712 | | 0.009 | | 0.888 |
| min | 0.002 | 0.002 | 0.002 | 0.002 | 0.002 | 0.002 |
| max | 0.133 | 0.054 | 0.133 | 0.112 | 0.133 | 0.048 |
| n (Samp) | 441 | 20 | 441 | 26 | 441 | 14 |
| n (Pat) | 170 | 20 | 170 | 26 | 170 | 14 |

UO only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.002 | 0.002 | 0.002 | 0.002 | 0.002 | 0.002 |
| average | 0.006 | 0.007 | 0.006 | 0.011 | 0.006 | 0.014 |
| stdev | 0.015 | 0.018 | 0.015 | 0.019 | 0.015 | 0.022 |
| p (t-test) | | 0.621 | | 0.055 | | 0.027 |
| min | 0.002 | 0.002 | 0.002 | 0.002 | 0.002 | 0.002 |
| max | 0.133 | 0.088 | 0.133 | 0.071 | 0.133 | 0.076 |
| n (Samp) | 212 | 47 | 212 | 52 | 212 | 25 |
| n (Pat) | 85 | 47 | 85 | 52 | 85 | 25 |

sCr or UO

| Time prior AKI stage | AUC | SE | nCohort 1 | nCohort 2 | p |
|---|---|---|---|---|---|
| 0 hours | 0.51 | 0.044 | 249 | 53 | 0.859 |
| 24 hours | 0.57 | 0.042 | 249 | 62 | 0.117 |
| 48 hours | 0.58 | 0.060 | 249 | 27 | 0.209 |

sCr only

| Time prior AKI stage | AUC | SE | nCohort 1 | nCohort 2 | p |
|---|---|---|---|---|---|
| 0 hours | 0.48 | 0.065 | 441 | 20 | 0.738 |
| 24 hours | 0.57 | 0.060 | 441 | 26 | 0.273 |
| 48 hours | 0.50 | 0.078 | 441 | 14 | 0.980 |

UO only

| Time prior AKI stage | AUC | SE | nCohort 1 | nCohort 2 | p |
|---|---|---|---|---|---|
| 0 hours | 0.51 | 0.047 | 212 | 47 | 0.782 |
| 24 hours | 0.56 | 0.045 | 212 | 52 | 0.215 |
| 48 hours | 0.58 | 0.063 | 212 | 25 | 0.185 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |

## Fig. 1 - 19

| Time prior AKI stage | Cutoff value | sens | spec | Quartile | OR | 95% CI of OR | |
|---|---|---|---|---|---|---|---|
| | 0 | 100% | 0% | 2 | 12.8 | 8.1 | 20.2 |
| | 0 | 100% | 0% | 3 | 0.0 | 0.0 | na |
| | 0.00151 | 13% | 88% | 4 | 1.2 | 0.6 | 2.3 |
| | 0.00151 | 13% | 88% | | | | |
| | 0.0212 | 9% | 90% | | | | |
| 24 hours | 0 | 100% | 0% | 1 | | | |
| | 0 | 100% | 0% | 2 | 10.5 | 7.0 | 15.8 |
| | 0 | 100% | 0% | 3 | 0.0 | 0.0 | na |
| | 0.00151 | 24% | 88% | 4 | 2.4 | 1.5 | 3.8 |
| | 0.00151 | 24% | 88% | | | | |
| | 0.0212 | 23% | 90% | | | | |
| 48 hours | 0 | 100% | 0% | 1 | | | |
| | 0 | 100% | 0% | 2 | 11.8 | 3.7 | 37.6 |
| | 0 | 100% | 0% | 3 | 0.0 | 0.0 | na |
| | 0.00151 | 26% | 88% | 4 | 3.8 | 1.0 | 14.2 |
| | 0.00151 | 26% | 88% | | | | |
| | 0.0212 | 26% | 90% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.0 | 0.0 | na |
| | 0 | 100% | 0% | 3 | 10.6 | 3.4 | 32.6 |
| | 0.00151 | 10% | 85% | 4 | 0.0 | 0.0 | na |
| | 0.00151 | 10% | 85% | | | | |
| | 0.0272 | 10% | 90% | | | | |
| 24 hours | 0 | 100% | 0% | 1 | | | |
| | 0 | 100% | 0% | 2 | na | na | na |
| | 0 | 100% | 0% | 3 | na | na | na |
| | 0.00151 | 27% | 85% | 4 | na | na | na |
| | 0.00151 | 27% | 85% | | | | |
| | 0.0272 | 19% | 90% | | | | |
| 48 hours | 0 | 100% | 0% | 1 | | | |
| | 0 | 100% | 0% | 2 | 0.0 | 0.0 | na |
| | 0 | 100% | 0% | 3 | 6.6 | 2.0 | 21.4 |
| | 0.00151 | 14% | 85% | 4 | 0.0 | 0.0 | na |
| | 0.00151 | 14% | 85% | | | | |
| | 0.0272 | 7% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 18.6 | 9.8 | 35.5 |
| | 0 | 100% | 0% | 3 | 0.0 | 0.0 | na |
| | 0.00151 | 15% | 88% | 4 | 1.8 | 0.8 | 4.2 |
| | 0.00151 | 15% | 88% | | | | |
| | 0.0212 | 11% | 90% | | | | |
| 24 hours | 0 | 100% | 0% | 1 | | | |
| | 0 | 100% | 0% | 2 | 10.6 | 6.6 | 17.1 |
| | 0 | 100% | 0% | 3 | 0.0 | 0.0 | na |
| | 0.00151 | 23% | 88% | 4 | 2.2 | 1.3 | 3.9 |
| | 0.00151 | 23% | 88% | | | | |
| | 0.0212 | 21% | 90% | | | | |
| 48 hours | 0 | 100% | 0% | 1 | | | |
| | 0 | 100% | 0% | 2 | 6.4 | 2.7 | 15.1 |
| | 0 | 100% | 0% | 3 | 0.0 | 0.0 | na |
| | 0.00151 | 28% | 88% | 4 | 2.5 | 0.9 | 6.7 |
| | 0.00151 | 28% | 88% | | | | |
| | 0.0212 | 28% | 90% | | | | |

# Fig. 1 - 20

**Lymphotoxin-alpha**

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 1.630 | 1.980 | 1.630 | 2.905 | 1.630 | 1.990 |
| average | 2.001 | 2.465 | 2.001 | 2.979 | 2.001 | 2.621 |
| stdev | 1.816 | 2.176 | 1.816 | 2.193 | 1.816 | 2.741 |
| p (t-test) | | 0.105 | | 0.000 | | 0.113 |
| min | 0.182 | 0.182 | 0.182 | 0.182 | 0.182 | 0.182 |
| max | 13.300 | 7.840 | 13.300 | 9.340 | 13.300 | 9.230 |
| n (Samp) | 249 | 53 | 249 | 62 | 249 | 27 |
| n (Pat) | 104 | 53 | 104 | 62 | 104 | 27 |

sCr only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 1.750 | 2.040 | 1.750 | 3.160 | 1.750 | 1.715 |
| average | 2.159 | 2.807 | 2.159 | 3.135 | 2.159 | 2.103 |
| stdev | 1.967 | 3.337 | 1.967 | 2.014 | 1.967 | 2.487 |
| p (t-test) | | 0.166 | | 0.014 | | 0.917 |
| min | 0.182 | 0.182 | 0.182 | 0.182 | 0.182 | 0.182 |
| max | 13.300 | 15.500 | 13.300 | 9.340 | 13.300 | 9.040 |
| n (Samp) | 441 | 20 | 441 | 26 | 441 | 14 |
| n (Pat) | 170 | 20 | 170 | 26 | 170 | 14 |

UO only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 1.680 | 1.980 | 1.680 | 2.630 | 1.680 | 1.990 |
| average | 2.002 | 2.414 | 2.002 | 3.075 | 2.002 | 2.585 |
| stdev | 1.793 | 2.262 | 1.793 | 2.416 | 1.793 | 2.411 |
| p (t-test) | | 0.177 | | 0.000 | | 0.141 |
| min | 0.182 | 0.182 | 0.182 | 0.182 | 0.182 | 0.182 |
| max | 13.300 | 7.840 | 13.300 | 11.300 | 13.300 | 9.230 |
| n (Samp) | 212 | 47 | 212 | 52 | 212 | 25 |
| n (Pat) | 85 | 47 | 85 | 52 | 85 | 25 |

sCr or UO

| Time prior AKI stage | AUC | SE | nCohort 1 | nCohort 2 | p |
| --- | --- | --- | --- | --- | --- |
| 0 hours | 0.55 | 0.044 | 249 | 53 | 0.261 |
| 24 hours | 0.64 | 0.041 | 249 | 62 | 0.001 |
| 48 hours | 0.54 | 0.060 | 249 | 27 | 0.524 |

sCr only

| Time prior AKI stage | AUC | SE | nCohort 1 | nCohort 2 | p |
| --- | --- | --- | --- | --- | --- |
| 0 hours | 0.55 | 0.068 | 441 | 20 | 0.491 |
| 24 hours | 0.66 | 0.060 | 441 | 26 | 0.007 |
| 48 hours | 0.46 | 0.076 | 441 | 14 | 0.562 |

UO only

| Time prior AKI stage | AUC | SE | nCohort 1 | nCohort 2 | p |
| --- | --- | --- | --- | --- | --- |
| 0 hours | 0.53 | 0.047 | 212 | 47 | 0.475 |
| 24 hours | 0.64 | 0.045 | 212 | 52 | 0.002 |
| 48 hours | 0.56 | 0.063 | 212 | 25 | 0.329 |

sCr or UO

| Time prior AKI stage | Cutoff value | sens | spec | Quartile | OR | 95% CI of OR | |
| --- | --- | --- | --- | --- | --- | --- | --- |
| 0 hours | 0 | 100% | 0% | 1 | | | |
| | 0 | 100% | 0% | 2 | 0.7 | 0.4 | 1.0 |
| | 0 | 100% | 0% | 3 | 0.5 | 0.3 | 0.8 |

# Fig. 1 - 21

| Time prior AKI stage | Cutoff value | sens | spec | Quartile | OR | 95% CI of OR | |
|---|---|---|---|---|---|---|---|
| | 2.56 | 42% | 71% | 4 | 1.7 | 1.2 | 2.2 |
| | 3.32 | 34% | 81% | | | | |
| | 4.3 | 19% | 90% | | | | |
| 24 hours | 1.44 | 71% | 44% | 1 | | | |
| | 1 | 81% | 34% | 2 | 1.5 | 1.0 | 2.2 |
| | 0 | 100% | 0% | 3 | 1.1 | 0.7 | 1.7 |
| | 2.56 | 52% | 71% | 4 | 3.5 | 2.5 | 5.0 |
| | 3.32 | 44% | 81% | | | | |
| | 4.3 | 32% | 90% | | | | |
| 48 hours | 0.919 | 70% | 33% | 1 | | | |
| | 0 | 100% | 0% | 2 | 1.0 | 0.5 | 2.0 |
| | 0 | 100% | 0% | 3 | 1.2 | 0.6 | 2.3 |
| | 2.56 | 37% | 71% | 4 | 1.4 | 0.7 | 2.6 |
| | 3.32 | 30% | 81% | | | | |
| | 4.3 | 22% | 90% | | | | |

sCr only

| Time prior AKI stage | Cutoff value | sens | spec | Quartile | OR | 95% CI of OR | |
|---|---|---|---|---|---|---|---|
| 0 hours | 1.41 | 70% | 42% | 1 | | | |
| | 1.19 | 80% | 34% | 2 | 1.0 | 0.4 | 2.8 |
| | 0 | 100% | 0% | 3 | 1.5 | 0.7 | 3.6 |
| | 2.81 | 35% | 70% | 4 | 1.5 | 0.6 | 3.5 |
| | 3.41 | 20% | 80% | | | | |
| | 4.6 | 15% | 90% | | | | |
| 24 hours | 1.84 | 73% | 51% | 1 | | | |
| | 1.33 | 81% | 41% | 2 | 1.7 | 0.6 | 5.0 |
| | 0 | 100% | 0% | 3 | 2.0 | 0.7 | 5.6 |
| | 2.81 | 58% | 70% | 4 | 4.3 | 1.8 | 10.1 |
| | 3.41 | 42% | 80% | | | | |
| | 4.6 | 19% | 90% | | | | |
| 48 hours | 0 | 100% | 0% | 1 | | | |
| | 0 | 100% | 0% | 2 | 0.7 | 0.2 | 2.4 |
| | 0 | 100% | 0% | 3 | 0.2 | 0.0 | 2.9 |
| | 2.81 | 29% | 70% | 4 | 1.5 | 0.7 | 3.6 |
| | 3.41 | 21% | 80% | | | | |
| | 4.6 | 7% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.2 | 0.1 | 0.4 |
| | 0 | 100% | 0% | 3 | 0.4 | 0.3 | 0.6 |
| | 2.56 | 45% | 70% | 4 | 1.0 | 0.7 | 1.3 |
| | 3.32 | 34% | 81% | | | | |
| | 4.18 | 17% | 90% | | | | |
| 24 hours | 1.44 | 71% | 43% | 1 | | | |
| | 0.768 | 81% | 28% | 2 | 1.3 | 0.8 | 2.0 |
| | 0 | 100% | 0% | 3 | 1.0 | 0.6 | 1.7 |
| | 2.56 | 50% | 70% | 4 | 3.4 | 2.3 | 5.0 |
| | 3.32 | 44% | 81% | | | | |
| | 4.18 | 35% | 90% | | | | |
| 48 hours | 1.28 | 72% | 37% | 1 | | | |
| | 0.93 | 80% | 31% | 2 | 1.2 | 0.6 | 2.7 |
| | 0 | 100% | 0% | 3 | 1.5 | 0.7 | 3.1 |
| | 2.56 | 40% | 70% | 4 | 1.4 | 0.7 | 3.0 |
| | 3.32 | 28% | 81% | | | | |
| | 4.18 | 20% | 90% | | | | |

Fig. 1 - 22

**Matrix metalloproteinase-3**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.027 | 0.031 | 0.027 | 0.034 | 0.027 | 0.014 |
| average | 0.166 | 0.059 | 0.166 | 0.877 | 0.166 | 0.121 |
| stdev | 1.087 | 0.090 | 1.087 | 6.215 | 1.087 | 0.406 |
| p (t-test) | | 0.473 | | 0.088 | | 0.829 |
| min | 0.001 | 0.001 | 0.001 | 0.001 | 0.001 | 0.001 |
| max | 16.700 | 0.516 | 16.700 | 49.000 | 16.700 | 2.130 |
| n (Samp) | 249 | 53 | 249 | 62 | 249 | 27 |
| n (Pat) | 104 | 53 | 104 | 62 | 104 | 27 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.030 | 0.031 | 0.030 | 0.034 | 0.030 | 0.028 |
| average | 0.249 | 0.070 | 0.249 | 0.093 | 0.249 | 0.056 |
| stdev | 2.481 | 0.117 | 2.481 | 0.220 | 2.481 | 0.077 |
| p (t-test) | | 0.748 | | 0.750 | | 0.772 |
| min | 0.001 | 0.001 | 0.001 | 0.001 | 0.001 | 0.001 |
| max | 49.000 | 0.516 | 49.000 | 1.130 | 49.000 | 0.280 |
| n (Samp) | 441 | 20 | 441 | 26 | 441 | 14 |
| n (Pat) | 170 | 20 | 170 | 26 | 170 | 14 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.023 | 0.034 | 0.023 | 0.042 | 0.023 | 0.032 |
| average | 0.172 | 0.059 | 0.172 | 1.022 | 0.172 | 0.156 |
| stdev | 1.172 | 0.075 | 1.172 | 6.786 | 1.172 | 0.427 |
| p (t-test) | | 0.509 | | 0.085 | | 0.946 |
| min | 0.001 | 0.001 | 0.001 | 0.001 | 0.001 | 0.001 |
| max | 16.700 | 0.404 | 16.700 | 49.000 | 16.700 | 2.130 |
| n (Samp) | 212 | 47 | 212 | 52 | 212 | 25 |
| n (Pat) | 85 | 47 | 85 | 52 | 85 | 25 |

sCr or UO

| Time prior AKI stage | AUC | SE | nCohort 1 | nCohort 2 | p |
|---|---|---|---|---|---|
| 0 hours | 0.55 | 0.044 | 249 | 53 | 0.223 |
| 24 hours | 0.54 | 0.042 | 249 | 62 | 0.358 |
| 48 hours | 0.46 | 0.057 | 249 | 27 | 0.502 |

sCr only

| Time prior AKI stage | AUC | SE | nCohort 1 | nCohort 2 | p |
|---|---|---|---|---|---|
| 0 hours | 0.52 | 0.067 | 441 | 20 | 0.728 |
| 24 hours | 0.52 | 0.059 | 441 | 26 | 0.684 |
| 48 hours | 0.50 | 0.078 | 441 | 14 | 0.988 |

UO only

| Time prior AKI stage | AUC | SE | nCohort 1 | nCohort 2 | p |
|---|---|---|---|---|---|
| 0 hours | 0.59 | 0.047 | 212 | 47 | 0.047 |
| 24 hours | 0.58 | 0.045 | 212 | 52 | 0.085 |
| 48 hours | 0.55 | 0.062 | 212 | 25 | 0.438 |

sCr or UO

| Time prior AKI stage | Cutoff value | sens | spec | Quartile | OR | 95% CI of OR | |
|---|---|---|---|---|---|---|---|
| 0 hours | 0.0203 | 75% | 41% | 1 | | | |
| | 0.00967 | 83% | 27% | 2 | 1.4 | 0.9 | 2.1 |
| | 0 | 100% | 0% | 3 | 2.0 | 1.3 | 3.0 |

# Fig. 1 - 23

| Time prior AKI stage | Cutoff value | sens | spec | Quartile | OR | 95% CI of OR | |
|---|---|---|---|---|---|---|---|
| | 0.047 | 38% | 70% | 4 | 2.0 | 1.3 | 2.9 |
| | 0.0757 | 19% | 80% | | | | |
| | 0.213 | 4% | 91% | | | | |
| 24 hours | 0.0163 | 71% | 37% | 1 | | | |
| | 0 | 100% | 0% | 2 | 0.6 | 0.4 | 0.8 |
| | 0 | 100% | 0% | 3 | 1.1 | 0.8 | 1.4 |
| | 0.047 | 37% | 70% | 4 | 1.2 | 0.9 | 1.6 |
| | 0.0757 | 26% | 80% | | | | |
| | 0.213 | 10% | 91% | | | | |
| 48 hours | 0.0074 | 74% | 22% | 1 | | | |
| | 0 | 100% | 0% | 2 | 0.3 | 0.1 | 0.9 |
| | 0 | 100% | 0% | 3 | 0.6 | 0.3 | 1.2 |
| | 0.047 | 30% | 70% | 4 | 1.4 | 0.9 | 2.4 |
| | 0.0757 | 22% | 80% | | | | |
| | 0.213 | 7% | 91% | | | | |

sCr only

| Time prior AKI stage | Cutoff value | sens | spec | Quartile | OR | 95% CI of OR | |
|---|---|---|---|---|---|---|---|
| 0 hours | 0.0203 | 70% | 38% | 1 | | | |
| | 0.0121 | 80% | 27% | 2 | 1.0 | 0.4 | 2.8 |
| | 0 | 100% | 0% | 3 | 1.8 | 0.8 | 4.0 |
| | 0.0525 | 30% | 70% | 4 | 1.3 | 0.5 | 3.1 |
| | 0.0773 | 15% | 80% | | | | |
| | 0.169 | 15% | 90% | | | | |
| 24 hours | 0.0149 | 73% | 31% | 1 | | | |
| | 0.0074 | 81% | 21% | 2 | 0.4 | 0.2 | 1.1 |
| | 0 | 100% | 0% | 3 | 1.3 | 0.8 | 2.2 |
| | 0.0525 | 31% | 70% | 4 | 1.0 | 0.5 | 1.8 |
| | 0.0773 | 23% | 80% | | | | |
| | 0.169 | 12% | 90% | | | | |
| 48 hours | 0.0151 | 71% | 31% | 1 | | | |
| | 0.0074 | 86% | 21% | 2 | 0.7 | 0.2 | 2.4 |
| | 0 | 100% | 0% | 3 | 1.0 | 0.4 | 2.8 |
| | 0.0525 | 29% | 70% | 4 | 0.8 | 0.2 | 2.4 |
| | 0.0773 | 21% | 80% | | | | |
| | 0.169 | 7% | 90% | | | | |

UO only

| Time prior AKI stage | Cutoff value | sens | spec | Quartile | OR | 95% CI of OR | |
|---|---|---|---|---|---|---|---|
| 0 hours | 0.0239 | 70% | 52% | 1 | | | |
| | 0.0149 | 81% | 37% | 2 | 1.5 | 0.9 | 2.6 |
| | 0 | 100% | 0% | 3 | 2.2 | 1.4 | 3.7 |
| | 0.0462 | 43% | 71% | 4 | 2.7 | 1.7 | 4.3 |
| | 0.0654 | 32% | 80% | | | | |
| | 0.18 | 6% | 90% | | | | |
| 24 hours | 0.0199 | 71% | 44% | 1 | | | |
| | 0 | 100% | 0% | 2 | 0.6 | 0.4 | 1.0 |
| | 0 | 100% | 0% | 3 | 1.3 | 0.9 | 1.9 |
| | 0.0462 | 42% | 71% | 4 | 1.6 | 1.1 | 2.2 |
| | 0.0654 | 29% | 80% | | | | |
| | 0.18 | 10% | 90% | | | | |
| 48 hours | 0.00783 | 76% | 25% | 1 | | | |
| | 0.0074 | 80% | 25% | 2 | 1.0 | 0.5 | 2.1 |
| | 0 | 100% | 0% | 3 | 0.6 | 0.3 | 1.6 |
| | 0.0462 | 36% | 71% | 4 | 1.6 | 0.8 | 2.9 |
| | 0.0654 | 36% | 80% | | | | |
| | 0.18 | 20% | 90% | | | | |

Fig. 1 - 24

Rantes (C-C motif chemokine 5)

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.008 | 0.009 | 0.008 | 0.010 | 0.008 | 0.009 |
| average | 0.017 | 0.011 | 0.017 | 0.024 | 0.017 | 0.011 |
| stdev | 0.067 | 0.008 | 0.067 | 0.087 | 0.067 | 0.008 |
| p (t-test) | | 0.472 | | 0.469 | | 0.615 |
| min | 0.000 | 0.002 | 0.000 | 0.002 | 0.000 | 0.002 |
| max | 0.878 | 0.053 | 0.878 | 0.682 | 0.878 | 0.036 |
| n (Samp) | 249 | 53 | 249 | 62 | 249 | 27 |
| n (Pat) | 104 | 53 | 104 | 62 | 104 | 27 |

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 0.009 | 0.007 | 0.009 | 0.010 | 0.009 | 0.008 |
| average | 0.016 | 0.011 | 0.016 | 0.013 | 0.016 | 0.012 |
| stdev | 0.060 | 0.012 | 0.060 | 0.014 | 0.060 | 0.010 |
| p (t-test) | | 0.721 | | 0.766 | | 0.780 |
| min | 0.000 | 0.002 | 0.000 | 0.002 | 0.000 | 0.003 |
| max | 0.878 | 0.053 | 0.878 | 0.072 | 0.878 | 0.036 |
| n (Samp) | 441 | 20 | 441 | 26 | 441 | 14 |
| n (Pat) | 170 | 20 | 170 | 26 | 170 | 14 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 0.008 | 0.009 | 0.008 | 0.010 | 0.008 | 0.009 |
| average | 0.020 | 0.010 | 0.020 | 0.026 | 0.020 | 0.011 |
| stdev | 0.072 | 0.004 | 0.072 | 0.095 | 0.072 | 0.008 |
| p (t-test) | | 0.354 | | 0.567 | | 0.550 |
| min | 0.000 | 0.004 | 0.000 | 0.002 | 0.000 | 0.002 |
| max | 0.878 | 0.028 | 0.878 | 0.682 | 0.878 | 0.036 |
| n (Samp) | 212 | 47 | 212 | 52 | 212 | 25 |
| n (Pat) | 85 | 47 | 85 | 52 | 85 | 25 |

sCr or UO

| Time prior AKI stage | AUC | SE | nCohort 1 | nCohort 2 | p |
|---|---|---|---|---|---|
| 0 hours | 0.55 | 0.044 | 249 | 53 | 0.306 |
| 24 hours | 0.59 | 0.042 | 249 | 62 | 0.035 |
| 48 hours | 0.55 | 0.060 | 249 | 27 | 0.407 |

sCr only

| Time prior AKI stage | AUC | SE | nCohort 1 | nCohort 2 | p |
|---|---|---|---|---|---|
| 0 hours | 0.44 | 0.063 | 441 | 20 | 0.306 |
| 24 hours | 0.55 | 0.060 | 441 | 26 | 0.400 |
| 48 hours | 0.49 | 0.078 | 441 | 14 | 0.858 |

UO only

| Time prior AKI stage | AUC | SE | nCohort 1 | nCohort 2 | p |
|---|---|---|---|---|---|
| 0 hours | 0.55 | 0.047 | 212 | 47 | 0.248 |
| 24 hours | 0.60 | 0.045 | 212 | 52 | 0.025 |
| 48 hours | 0.54 | 0.062 | 212 | 25 | 0.476 |

sCr or UO

| Time prior AKI stage | Cutoff value | sens | spec | Quartile | OR | 95% CI of OR | |
|---|---|---|---|---|---|---|---|
| 0 hours | 0.00664 | 72% | 35% | 1 | | | |
| | 0.00558 | 81% | 25% | 2 | 0.9 | 0.6 | 1.3 |

# Fig. 1 - 25

| | 0.00485 | 91% | 20% | 3 | 1.7 | 1.2 | 2.3 |
|---|---|---|---|---|---|---|---|
| | 0.0102 | 36% | 70% | 4 | 1.0 | 0.7 | 1.5 |
| | 0.0122 | 19% | 80% | | | | |
| | 0.0154 | 9% | 91% | | | | |
| 24 hours | 0.00771 | 71% | 45% | 1 | | | |
| | 0.0071 | 81% | 37% | 2 | 1.2 | 0.8 | 1.9 |
| | 0.00435 | 90% | 16% | 3 | 2.6 | 1.9 | 3.7 |
| | 0.0102 | 45% | 70% | 4 | 2.0 | 1.4 | 2.9 |
| | 0.0122 | 21% | 80% | | | | |
| | 0.0154 | 16% | 91% | | | | |
| 48 hours | 0.00717 | 70% | 38% | 1 | | | |
| | 0.00613 | 81% | 31% | 2 | 1.0 | 0.4 | 2.3 |
| | 0.00317 | 93% | 8% | 3 | 2.2 | 1.1 | 4.2 |
| | 0.0102 | 33% | 70% | 4 | 1.4 | 0.7 | 3.0 |
| | 0.0122 | 26% | 80% | | | | |
| | 0.0154 | 7% | 91% | | | | |

sCr only

| Time prior AKI stage | Cutoff value | sens | spec | Quartile | OR | 95% CI of OR | |
|---|---|---|---|---|---|---|---|
| 0 hours | 0.00537 | 70% | 21% | 1 | | | |
| | 0.00478 | 80% | 17% | 2 | 0.6 | 0.2 | 1.8 |
| | 0.00399 | 90% | 12% | 3 | 0.8 | 0.3 | 2.0 |
| | 0.0109 | 25% | 70% | 4 | 1.7 | 0.8 | 3.3 |
| | 0.0126 | 25% | 80% | | | | |
| | 0.0154 | 15% | 90% | | | | |
| 24 hours | 0.00523 | 73% | 20% | 1 | | | |
| | 0.00488 | 81% | 18% | 2 | 0.4 | 0.1 | 0.9 |
| | 0.00293 | 92% | 7% | 3 | 0.6 | 0.3 | 1.2 |
| | 0.0109 | 42% | 70% | 4 | 1.3 | 0.8 | 2.0 |
| | 0.0126 | 31% | 80% | | | | |
| | 0.0154 | 23% | 90% | | | | |
| 48 hours | 0.00666 | 71% | 30% | 1 | | | |
| | 0.00642 | 86% | 28% | 2 | 2.0 | 0.5 | 9.2 |
| | 0.00454 | 93% | 15% | 3 | 3.1 | 0.8 | 11.9 |
| | 0.0109 | 21% | 70% | 4 | 1.0 | 0.1 | 7.4 |
| | 0.0126 | 14% | 80% | | | | |
| | 0.0154 | 14% | 90% | | | | |

UO only

| Time prior AKI stage | Cutoff value | sens | spec | Quartile | OR | 95% CI of OR | |
|---|---|---|---|---|---|---|---|
| 0 hours | 0.00718 | 70% | 39% | 1 | | | |
| | 0.00664 | 81% | 34% | 2 | 1.6 | 1.0 | 2.6 |
| | 0.00523 | 91% | 23% | 3 | 2.3 | 1.5 | 3.6 |
| | 0.0108 | 34% | 70% | 4 | 1.4 | 0.9 | 2.3 |
| | 0.0127 | 17% | 80% | | | | |
| | 0.0242 | 2% | 90% | | | | |
| 24 hours | 0.00823 | 71% | 52% | 1 | | | |
| | 0.00753 | 81% | 42% | 2 | 2.5 | 1.4 | 4.2 |
| | 0.00601 | 90% | 28% | 3 | 4.3 | 2.6 | 7.2 |
| | 0.0108 | 35% | 70% | 4 | 2.5 | 1.4 | 4.2 |
| | 0.0127 | 17% | 80% | | | | |
| | 0.0242 | 6% | 90% | | | | |
| 48 hours | 0.00717 | 72% | 38% | 1 | | | |
| | 0.00648 | 80% | 32% | 2 | 1.3 | 0.5 | 3.3 |
| | 0.00478 | 92% | 17% | 3 | 2.5 | 1.1 | 5.4 |
| | 0.0108 | 32% | 70% | 4 | 1.8 | 0.8 | 4.2 |
| | 0.0127 | 24% | 80% | | | | |
| | 0.0242 | 8% | 90% | | | | |

Fig. 1 - 26

**Thrombospondin-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 6.821 | 5.701 | 6.821 | 0.828 | 6.821 | 0.828 |
| average | 20.005 | 8.163 | 20.005 | 3.622 | 20.005 | 5.484 |
| stdev | 61.809 | 6.933 | 61.809 | na | 61.809 | na |
| p (t-test) | | 0.495 | | na | | na |
| min | 0.828 | 0.828 | 0.828 | 3.622 | 0.828 | 5.484 |
| max | 435.086 | 26.296 | 435.086 | 3.622 | 435.086 | 5.484 |
| n (Samp) | 54 | 13 | 54 | 1 | 54 | 1 |
| n (Pat) | 36 | 13 | 36 | 1 | 36 | 1 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 6.666 | 6.153 | 6.666 | 7.511 | 6.666 | 4.494 |
| average | 15.699 | 13.105 | 15.699 | 12.209 | 15.699 | 4.494 |
| stdev | 49.279 | 14.090 | 49.279 | 11.668 | 49.279 | 1.280 |
| p (t-test) | | 0.898 | | 0.903 | | 0.750 |
| min | 0.828 | 2.380 | 0.828 | 3.622 | 0.828 | 3.590 |
| max | 435.086 | 35.262 | 435.086 | 25.493 | 435.086 | 5.399 |
| n (Samp) | 86 | 6 | 86 | 3 | 86 | 2 |
| n (Pat) | 60 | 6 | 60 | 3 | 60 | 2 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 3.891 | 6.757 | 3.891 | 3.002 | 3.891 | 0.828 |
| average | 8.657 | 8.327 | 8.657 | 3.002 | 8.657 | 5.484 |
| stdev | 15.127 | 6.174 | 15.127 | 0.876 | 15.127 | na |
| p (t-test) | | 0.942 | | 0.604 | | na |
| min | 0.828 | 0.828 | 0.828 | 2.383 | 0.828 | 5.484 |
| max | 93.490 | 22.485 | 93.490 | 3.622 | 93.490 | 5.484 |
| n (Samp) | 40 | 12 | 40 | 2 | 40 | 1 |
| n (Pat) | 26 | 12 | 26 | 2 | 26 | 1 |

sCr or UO

| Time prior AKI stage | AUC | SE | nCohort 1 | nCohort 2 | p |
|---|---|---|---|---|---|
| 0 hours | 0.52 | 0.090 | 54 | 13 | 0.856 |
| 24 hours | 0.41 | 0.270 | 54 | 1 | 0.732 |
| 48 hours | 0.48 | 0.290 | 54 | 1 | 0.949 |

sCr only

| Time prior AKI stage | AUC | SE | nCohort 1 | nCohort 2 | p |
|---|---|---|---|---|---|
| 0 hours | 0.53 | 0.124 | 86 | 6 | 0.821 |
| 24 hours | 0.60 | 0.176 | 86 | 3 | 0.575 |
| 48 hours | 0.38 | 0.184 | 86 | 2 | 0.506 |

UO only

| Time prior AKI stage | AUC | SE | nCohort 1 | nCohort 2 | p |
|---|---|---|---|---|---|
| 0 hours | 0.61 | 0.097 | 40 | 12 | 0.267 |
| 24 hours | 0.36 | 0.184 | 40 | 2 | 0.456 |
| 48 hours | 0.55 | 0.303 | 40 | 1 | 0.869 |

sCr or UO

| Time prior AKI stage | Cutoff value | sens | spec | Quartile | OR | 95% CI of OR | |
|---|---|---|---|---|---|---|---|
| 0 hours | 3.58953 | 85% | 41% | 1 | | | |
| | 3.58953 | 85% | 41% | 2 | 2.9 | 0.5 | 15.6 |

Fig. 1 - 27

| Time prior AKI stage | Cutoff value | sens | spec | Quartile | OR | 95% CI of OR | |
|---|---|---|---|---|---|---|---|
| | 2.08132 | 92% | 22% | 3 | 1.5 | 0.2 | 10.2 |
| | 9.58807 | 23% | 70% | 4 | 1.5 | 0.2 | 10.2 |
| | 13.4448 | 15% | 81% | | | | |
| | 25.8224 | 8% | 91% | | | | |
| 24 hours | 3.58953 | 100% | 41% | 1 | | | |
| | 3.58953 | 100% | 41% | 2 | na | na | na |
| | 3.58953 | 100% | 41% | 3 | na | na | na |
| | 9.58807 | 0% | 70% | 4 | na | na | na |
| | 13.4448 | 0% | 81% | | | | |
| | 25.8224 | 0% | 91% | | | | |
| 48 hours | 4.55298 | 100% | 48% | 1 | | | |
| | 4.55298 | 100% | 48% | 2 | na | na | na |
| | 4.55298 | 100% | 48% | 3 | na | na | na |
| | 9.58807 | 0% | 70% | 4 | na | na | na |
| | 13.4448 | 0% | 81% | | | | |
| | 25.8224 | 0% | 91% | | | | |

sCr only

| Time prior AKI stage | Cutoff value | sens | spec | Quartile | OR | 95% CI of OR | |
|---|---|---|---|---|---|---|---|
| 0 hours | 2.37997 | 83% | 16% | 1 | | | |
| | 2.37997 | 83% | 16% | 2 | 0.5 | 0.0 | 10.8 |
| | 2.08132 | 100% | 16% | 3 | 0.5 | 0.0 | 10.8 |
| | 10.1744 | 33% | 71% | 4 | 1.0 | 0.1 | 8.6 |
| | 11.7188 | 33% | 80% | | | | |
| | 21.7226 | 33% | 91% | | | | |
| 24 hours | 3.58953 | 100% | 33% | 1 | | | |
| | 3.58953 | 100% | 33% | 2 | na | na | na |
| | 3.58953 | 100% | 33% | 3 | na | na | na |
| | 10.1744 | 33% | 71% | 4 | na | na | na |
| | 11.7188 | 33% | 80% | | | | |
| | 21.7226 | 33% | 91% | | | | |
| 48 hours | 3.28789 | 100% | 29% | 1 | | | |
| | 3.28789 | 100% | 29% | 2 | na | na | na |
| | 3.28789 | 100% | 29% | 3 | na | na | na |
| | 10.1744 | 0% | 71% | 4 | na | na | na |
| | 11.7188 | 0% | 80% | | | | |
| | 21.7226 | 0% | 91% | | | | |

UO only

| Time prior AKI stage | Cutoff value | sens | spec | Quartile | OR | 95% CI of OR | |
|---|---|---|---|---|---|---|---|
| 0 hours | 3.93212 | 75% | 55% | 1 | | | |
| | 3.58953 | 92% | 48% | 2 | 3.6 | 0.2 | 70.4 |
| | 3.58953 | 92% | 48% | 3 | 7.5 | 0.5 | 118.5 |
| | 8.52273 | 25% | 70% | 4 | 3.6 | 0.2 | 70.4 |
| | 9.0843 | 25% | 80% | | | | |
| | 13.4448 | 17% | 90% | | | | |
| 24 hours | 2.08132 | 100% | 25% | 1 | | | |
| | 2.08132 | 100% | 25% | 2 | na | na | na |
| | 2.08132 | 100% | 25% | 3 | na | na | na |
| | 8.52273 | 0% | 70% | 4 | na | na | na |
| | 9.0843 | 0% | 80% | | | | |
| | 13.4448 | 0% | 90% | | | | |
| 48 hours | 3.93212 | 100% | 55% | 1 | | | |
| | 3.93212 | 100% | 55% | 2 | na | na | na |
| | 3.93212 | 100% | 55% | 3 | na | na | na |
| | 8.52273 | 0% | 70% | 4 | na | na | na |
| | 9.0843 | 0% | 80% | | | | |
| | 13.4448 | 0% | 90% | | | | |

# Fig. 1 - 28

**ENA-78 (C-X-C chemokine motif 5)**

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 0.016 | 0.020 | 0.016 | 0.024 | 0.016 | 0.021 |
| average | 0.041 | 0.028 | 0.041 | 0.269 | 0.041 | 0.090 |
| stdev | 0.129 | 0.033 | 0.129 | 0.931 | 0.129 | 0.233 |
| p (t-test) | | 0.609 | | 0.000 | | 0.133 |
| min | 0.000 | 0.000 | 0.000 | 0.000 | 0.000 | 0.000 |
| max | 1.530 | 0.128 | 1.530 | 5.180 | 1.530 | 0.965 |
| n (Samp) | 419 | 27 | 419 | 36 | 419 | 18 |
| n (Pat) | 164 | 27 | 164 | 36 | 164 | 18 |

sCr only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 0.017 | 0.022 | 0.017 | 0.039 | 0.017 | 0.027 |
| average | 0.056 | 0.052 | 0.056 | 0.095 | 0.056 | 0.084 |
| stdev | 0.278 | 0.063 | 0.278 | 0.142 | 0.278 | 0.126 |
| p (t-test) | | 0.973 | | 0.674 | | 0.794 |
| min | 0.000 | 0.012 | 0.000 | 0.000 | 0.000 | 0.010 |
| max | 5.180 | 0.162 | 5.180 | 0.457 | 5.180 | 0.359 |
| n (Samp) | 518 | 5 | 518 | 9 | 518 | 7 |
| n (Pat) | 199 | 5 | 199 | 9 | 199 | 7 |

UO only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 0.017 | 0.021 | 0.017 | 0.022 | 0.017 | 0.021 |
| average | 0.047 | 0.028 | 0.047 | 0.297 | 0.047 | 0.079 |
| stdev | 0.141 | 0.034 | 0.141 | 1.018 | 0.141 | 0.237 |
| p (t-test) | | 0.503 | | 0.000 | | 0.391 |
| min | 0.000 | 0.000 | 0.000 | 0.000 | 0.000 | 0.000 |
| max | 1.530 | 0.128 | 1.530 | 5.180 | 1.530 | 0.965 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
| --- | --- | --- | --- | --- | --- |
| 0 hours | 0.54 | 0.059 | 419 | 27 | 0.464 |
| 24 hours | 0.64 | 0.051 | 419 | 36 | 0.007 |
| 48 hours | 0.57 | 0.072 | 419 | 18 | 0.350 |

sCr only

| Time prior AKI stage | AUC | SE | nCohort 1 | nCohort 2 | p |
| --- | --- | --- | --- | --- | --- |
| 0 hours | 0.68 | 0.133 | 518 | 5 | 0.188 |
| 24 hours | 0.73 | 0.096 | 518 | 9 | 0.015 |
| 48 hours | 0.72 | 0.110 | 518 | 7 | 0.047 |

UO only

| Time prior AKI stage | AUC | SE | nCohort 1 | nCohort 2 | p |
| --- | --- | --- | --- | --- | --- |
| 0 hours | 0.52 | 0.059 | 352 | 26 | 0.777 |
| 24 hours | 0.60 | 0.057 | 352 | 30 | 0.068 |
| 48 hours | 0.55 | 0.076 | 352 | 16 | 0.521 |

# Fig. 2 - 1

sCr or UO

| Time prior AKI stage | Cutoff value | sens | spec | Quartile | OR | 95% CI of OR | |
|---|---|---|---|---|---|---|---|
| 0 hours | 0.0116 | 70% | 35% | 1 | | | |
| | 0.00835 | 81% | 26% | 2 | 1.2 | 0.6 | 2.6 |
| | 0 | 100% | 0% | 3 | 2.1 | 1.1 | 3.9 |
| | 0.0248 | 26% | 71% | 4 | 1.2 | 0.6 | 2.6 |
| | 0.0338 | 19% | 80% | | | | |
| | 0.0581 | 11% | 90% | | | | |
| 24 hours | 0.0154 | 72% | 49% | 1 | | | |
| | 0.0117 | 81% | 36% | 2 | 0.8 | 0.4 | 1.7 |
| | 0.0059 | 92% | 20% | 3 | 1.7 | 1.0 | 3.0 |
| | 0.0248 | 44% | 71% | 4 | 2.7 | 1.6 | 4.4 |
| | 0.0338 | 36% | 80% | | | | |
| | 0.0581 | 25% | 90% | | | | |
| 48 hours | 0.0131 | 72% | 41% | 1 | | | |
| | 0.01 | 83% | 31% | 2 | 1.3 | 0.4 | 4.4 |
| | 0 | 100% | 0% | 3 | 2.1 | 0.7 | 5.7 |
| | 0.0248 | 39% | 71% | 4 | 1.7 | 0.6 | 5.0 |
| | 0.0338 | 17% | 80% | | | | |
| | 0.0581 | 11% | 90% | | | | |

sCr only

| Time prior AKI stage | Cutoff value | sens | spec | Quartile | OR | 95% CI of OR | |
|---|---|---|---|---|---|---|---|
| 0 hours | 0.0202 | 80% | 58% | 1 | | | |
| | 0.0202 | 80% | 58% | 2 | na | na | na |
| | 0.0116 | 100% | 34% | 3 | na | na | na |
| | 0.0257 | 40% | 70% | 4 | na | na | na |
| | 0.0351 | 40% | 81% | | | | |
| | 0.0662 | 20% | 90% | | | | |
| 24 hours | 0.0235 | 78% | 67% | 1 | | | |
| | 0.0197 | 89% | 57% | 2 | 0.0 | 0.0 | na |
| | 0 | 100% | 0% | 3 | 3.0 | 0.2 | 42.5 |
| | 0.0257 | 56% | 70% | 4 | 5.1 | 0.5 | 55.4 |
| | 0.0351 | 56% | 81% | | | | |
| | 0.0662 | 33% | 90% | | | | |
| 48 hours | 0.0269 | 71% | 72% | 1 | | | |
| | 0.0213 | 86% | 61% | 2 | na | na | na |
| | 0.01 | 100% | 30% | 3 | na | na | na |
| | 0.0257 | 71% | 70% | 4 | na | na | na |
| | 0.0351 | 29% | 81% | | | | |
| | 0.0662 | 29% | 90% | | | | |

UO only

| Time prior AKI stage | Cutoff value | sens | spec | Quartile | OR | 95% CI of OR | |
|---|---|---|---|---|---|---|---|
| 0 hours | 0.0086 | 73% | 25% | 1 | | | |
| | 0.00759 | 81% | 23% | 2 | 0.5 | 0.2 | 1.2 |
| | 0 | 100% | 0% | 3 | 1.5 | 0.9 | 2.5 |
| | 0.0259 | 27% | 70% | 4 | 0.7 | 0.3 | 1.4 |
| | 0.0357 | 15% | 80% | | | | |
| | 0.0704 | 12% | 90% | | | | |
| 24 hours | 0.0154 | 70% | 47% | 1 | | | |

## Fig. 2 - 2

| | | | | | | | |
|---|---|---|---|---|---|---|---|
| | 0.0117 | 80% | 35% | 2 | 1.0 | 0.4 | 2.3 |
| | 0.0059 | 93% | 18% | 3 | 2.1 | 1.1 | 4.0 |
| | 0.0259 | 40% | 70% | 4 | 2.1 | 1.1 | 3.9 |
| | 0.0357 | 27% | 80% | | | | |
| | 0.0704 | 20% | 90% | | | | |
| 48 hours | 0.0131 | 75% | 40% | 1 | | | |
| | 0.0104 | 81% | 30% | 2 | 1.3 | 0.4 | 4.4 |
| | 0 | 100% | 0% | 3 | 1.7 | 0.6 | 5.1 |
| | 0.0259 | 38% | 70% | 4 | 1.3 | 0.4 | 4.4 |
| | 0.0357 | 19% | 80% | | | | |
| | 0.0704 | 6% | 90% | | | | |

# Fig. 2 - 3

**Endoglin**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.015 | 0.004 | 0.015 | 0.052 | 0.015 | 0.004 |
| average | 0.024 | 0.008 | 0.024 | 0.077 | 0.024 | 0.004 |
| stdev | 0.036 | 0.013 | 0.036 | 0.093 | 0.036 | 0.000 |
| p (t-test) | | 0.238 | | 0.011 | | 0.437 |
| min | 0.004 | 0.004 | 0.004 | 0.004 | 0.004 | 0.004 |
| max | 0.199 | 0.041 | 0.199 | 0.199 | 0.199 | 0.004 |
| n (Samp) | 78 | 8 | 78 | 4 | 78 | 2 |
| n (Pat) | 56 | 8 | 56 | 4 | 56 | 2 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.011 | 0.004 | 0.011 | 0.052 | 0.011 | 0.004 |
| average | 0.023 | 0.018 | 0.023 | 0.052 | 0.023 | 0.004 |
| stdev | 0.038 | na | 0.038 | 0.069 | 0.038 | 0.000 |
| p (t-test) | | na | | 0.285 | | 0.478 |
| min | 0.004 | 0.018 | 0.004 | 0.004 | 0.004 | 0.004 |
| max | 0.199 | 0.018 | 0.199 | 0.101 | 0.199 | 0.004 |
| n (Samp) | 96 | 1 | 96 | 2 | 96 | 2 |
| n (Pat) | 69 | 1 | 69 | 2 | 69 | 2 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.018 | 0.004 | 0.018 | 0.101 | 0.018 | 0.004 |
| average | 0.027 | 0.008 | 0.027 | 0.101 | 0.027 | 0.004 |
| stdev | 0.039 | 0.013 | 0.039 | 0.138 | 0.039 | na |
| p (t-test) | | 0.198 | | 0.019 | | na |
| min | 0.004 | 0.004 | 0.004 | 0.004 | 0.004 | 0.004 |
| max | 0.199 | 0.041 | 0.199 | 0.199 | 0.199 | 0.004 |
| n (Samp) | 61 | 8 | 61 | 2 | 61 | 1 |
| n (Pat) | 43 | 8 | 43 | 2 | 43 | 1 |

sCr or UO

| Time prior AKI stage | AUC | SE | nCohort 1 | nCohort 2 | p |
|---|---|---|---|---|---|
| 0 hours | 0.23 | 0.072 | 78 | 8 | 0.000 |
| 24 hours | 0.56 | 0.153 | 78 | 4 | 0.698 |
| 48 hours | 0.14 | 0.092 | 78 | 2 | 0.000 |

sCr only

| Time prior AKI stage | AUC | SE | nCohort 1 | nCohort 2 | p |
|---|---|---|---|---|---|
| 0 hours | 0.67 | 0.300 | 96 | 1 | 0.567 |
| 24 hours | 0.57 | 0.213 | 96 | 2 | 0.760 |
| 48 hours | 0.17 | 0.107 | 96 | 2 | 0.002 |

UO only

| Time prior AKI stage | AUC | SE | nCohort 1 | nCohort 2 | p |
|---|---|---|---|---|---|
| 0 hours | 0.22 | 0.072 | 61 | 8 | 0.000 |
| 24 hours | 0.56 | 0.215 | 61 | 2 | 0.775 |
| 48 hours | 0.13 | 0.122 | 61 | 1 | 0.002 |

sCr or UO

| Time prior AKI stage | Cutoff value | sens | spec | Quartile | OR | 95% CI of OR |
|---|---|---|---|---|---|---|

# Fig. 2 - 4

EP 2 767 833 A2

| 0 hours | 0 | 100% | 0% | 1 | | | |
| | 0 | 100% | 0% | 2 | 0.0 | 0.0 | na |
| | 0 | 100% | 0% | 3 | 0.0 | 0.0 | na |
| | 0.01838 | 13% | 77% | 4 | 10.5 | 0.9 | 124.6 |
| | 0.04136 | 0% | 90% | | | | |
| | 0.04563 | 0% | 91% | | | | |
| 24 hours | 0 | 100% | 0% | 1 | | | |
| | 0 | 100% | 0% | 2 | 0.0 | 0.0 | na |
| | 0 | 100% | 0% | 3 | 0.0 | 0.0 | na |
| | 0.01838 | 50% | 77% | 4 | 0.9 | 0.1 | 8.3 |
| | 0.04136 | 50% | 90% | | | | |
| | 0.04563 | 50% | 91% | | | | |
| 48 hours | 0 | 100% | 0% | 1 | | | |
| | 0 | 100% | 0% | 2 | na | na | na |
| | 0 | 100% | 0% | 3 | na | na | na |
| | 0.01838 | 0% | 77% | 4 | na | na | na |
| | 0.04136 | 0% | 90% | | | | |
| | 0.04563 | 0% | 91% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.0 | 0.0 | na |
| | 0 | 100% | 0% | 3 | 5.2 | 0.3 | 79.1 |
| | 0.01838 | 13% | 74% | 4 | 3.6 | 0.2 | 64.2 |
| | 0.04136 | 0% | 90% | | | | |
| | 0.04136 | 0% | 90% | | | | |
| 24 hours | 0 | 100% | 0% | 1 | | | |
| | 0 | 100% | 0% | 2 | 0.0 | 0.0 | na |
| | 0 | 100% | 0% | 3 | 0.0 | 0.0 | na |
| | 0.01838 | 50% | 74% | 4 | 0.9 | 0.0 | 61.7 |
| | 0.04136 | 50% | 90% | | | | |
| | 0.04136 | 50% | 90% | | | | |
| 48 hours | 0 | 100% | 0% | 1 | | | |
| | 0 | 100% | 0% | 2 | na | na | na |
| | 0 | 100% | 0% | 3 | na | na | na |
| | 0.01838 | 0% | 74% | 4 | na | na | na |
| | 0.04136 | 0% | 90% | | | | |
| | 0.04136 | 0% | 90% | | | | |

# Fig. 2 - 5

113

**Erythropoietin**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 7.240 | 0.664 | 7.240 | 0.664 | 7.240 | 0.664 |
| average | 152.061 | 53.804 | 152.061 | 48.681 | 152.061 | 33.237 |
| stdev | 584.092 | 118.970 | 584.092 | 121.519 | 584.092 | 72.935 |
| p (t-test) | | 0.384 | | 0.290 | | 0.389 |
| min | 0.664 | 0.664 | 0.664 | 0.664 | 0.664 | 0.664 |
| max | 9340.000 | 544.000 | 9340.000 | 621.000 | 9340.000 | 297.000 |
| n (Samp) | 419 | 27 | 419 | 36 | 419 | 18 |
| n (Pat) | 164 | 27 | 164 | 36 | 164 | 18 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.664 | 0.664 | 0.664 | 0.664 | 0.664 | 0.664 |
| average | 132.253 | 0.664 | 132.253 | 40.720 | 132.253 | 15.416 |
| stdev | 529.043 | 0.000 | 529.043 | 77.820 | 529.043 | 36.692 |
| p (t-test) | | 0.579 | | 0.604 | | 0.560 |
| min | 0.664 | 0.664 | 0.664 | 0.664 | 0.664 | 0.664 |
| max | 9340.000 | 0.664 | 9340.000 | 229.000 | 9340.000 | 98.500 |
| n (Samp) | 518 | 5 | 518 | 9 | 518 | 7 |
| n (Pat) | 199 | 5 | 199 | 9 | 199 | 7 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 4.840 | 0.664 | 4.840 | 0.664 | 4.840 | 0.664 |
| average | 166.814 | 55.848 | 166.814 | 46.268 | 166.814 | 45.125 |
| stdev | 631.497 | 120.842 | 631.497 | 127.923 | 631.497 | 89.605 |
| p (t-test) | | 0.372 | | 0.298 | | 0.442 |
| min | 0.664 | 0.664 | 0.664 | 0.664 | 0.664 | 0.664 |
| max | 9340.000 | 544.000 | 9340.000 | 621.000 | 9340.000 | 297.000 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
|---|---|---|---|---|---|
| 0 hours | 0.43 | 0.054 | 419 | 27 | 0.175 |
| 24 hours | 0.38 | 0.045 | 419 | 36 | 0.008 |
| 48 hours | 0.40 | 0.063 | 419 | 18 | 0.106 |

sCr only

| Time prior AKI stage | AUC | SE | nCohort 1 | nCohort 2 | p |
|---|---|---|---|---|---|
| 0 hours | 0.26 | 0.091 | 518 | 5 | 0.009 |
| 24 hours | 0.43 | 0.092 | 518 | 9 | 0.453 |
| 48 hours | 0.38 | 0.097 | 518 | 7 | 0.206 |

UO only

| Time prior AKI stage | AUC | SE | nCohort 1 | nCohort 2 | p |
|---|---|---|---|---|---|
| 0 hours | 0.43 | 0.056 | 352 | 26 | 0.239 |
| 24 hours | 0.36 | 0.048 | 352 | 30 | 0.003 |
| 48 hours | 0.41 | 0.068 | 352 | 16 | 0.180 |

Fig. 2 - 6

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.8 | 0.3 | 2.0 |
| | 0 | 100% | 0% | 3 | 0.2 | 0.0 | 2.1 |
| | 64.5 | 22% | 70% | 4 | 3.9 | 2.2 | 6.7 |
| | 126 | 15% | 80% | | | | |
| | 344 | 4% | 90% | | | | |
| 24 hours | 0 | 100% | 0% | 1 | | | |
| | 0 | 100% | 0% | 2 | 0.5 | 0.2 | 1.3 |
| | 0 | 100% | 0% | 3 | 0.5 | 0.2 | 1.3 |
| | 64.5 | 22% | 70% | 4 | 4.9 | 3.1 | 7.6 |
| | 126 | 11% | 80% | | | | |
| | 344 | 3% | 90% | | | | |
| 48 hours | 0 | 100% | 0% | 1 | | | |
| | 0 | 100% | 0% | 2 | 2.1 | 0.5 | 9.3 |
| | 0 | 100% | 0% | 3 | 0.0 | 0.0 | na |
| | 64.5 | 17% | 70% | 4 | 6.7 | 2.0 | 21.8 |
| | 126 | 6% | 80% | | | | |
| | 344 | 0% | 90% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | na | na | na |
| | 0 | 100% | 0% | 3 | na | na | na |
| | 49.5 | 0% | 70% | 4 | na | na | na |
| | 116 | 0% | 80% | | | | |
| | 302 | 0% | 90% | | | | |
| 24 hours | 0 | 100% | 0% | 1 | | | |
| | 0 | 100% | 0% | 2 | 0.5 | 0.0 | 9.7 |
| | 0 | 100% | 0% | 3 | 0.0 | 0.0 | na |
| | 49.5 | 22% | 70% | 4 | 3.1 | 0.8 | 11.9 |
| | 116 | 11% | 80% | | | | |
| | 302 | 0% | 90% | | | | |
| 48 hours | 0 | 100% | 0% | 1 | | | |
| | 0 | 100% | 0% | 2 | 1.0 | 0.0 | 52.3 |
| | 0 | 100% | 0% | 3 | 0.0 | 0.0 | na |
| | 49.5 | 14% | 70% | 4 | 5.2 | 0.5 | 56.3 |
| | 116 | 0% | 80% | | | | |
| | 302 | 0% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.0 | 0.4 | 2.3 |
| | 0 | 100% | 0% | 3 | 1.9 | 1.0 | 3.6 |
| | 62.4 | 23% | 70% | 4 | 1.4 | 0.7 | 3.0 |
| | 135 | 12% | 80% | | | | |
| | 384 | 4% | 90% | | | | |
| 24 hours | 0 | 100% | 0% | 1 | | | |
| | 0 | 100% | 0% | 2 | 0.5 | 0.1 | 2.2 |
| | 0 | 100% | 0% | 3 | 1.3 | 0.5 | 3.2 |

Fig. 2 - 7

|  | 62.4 | 20% | 70% | 4 | 5.8 | 3.0 | 10.9 |
|  | 135 | 10% | 80% |  |  |  |  |
|  | 384 | 3% | 90% |  |  |  |  |
| 48 hours | 0 | 100% | 0% | 1 |  |  |  |
|  | 0 | 100% | 0% | 2 | 1.5 | 0.3 | 8.1 |
|  | 0 | 100% | 0% | 3 | 0.0 | 0.0 | na |
|  | 62.4 | 19% | 70% | 4 | 6.1 | 1.8 | 20.4 |
|  | 135 | 13% | 80% |  |  |  |  |
|  | 384 | 0% | 90% |  |  |  |  |

# Fig. 2 - 8

**Fractalkine**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 1.602 | 1.496 | 1.602 | 1.188 | 1.602 | 3.313 |
| average | 1.922 | 1.429 | 1.922 | 2.230 | 1.922 | 2.874 |
| stdev | 1.576 | 0.840 | 1.576 | 2.563 | 1.576 | 2.358 |
| p (t-test) | | 0.354 | | 0.466 | | 0.305 |
| min | 0.004 | 0.313 | 0.004 | 0.187 | 0.004 | 0.327 |
| max | 7.811 | 2.682 | 7.811 | 10.657 | 7.811 | 4.981 |
| n (Samp) | 163 | 9 | 163 | 18 | 163 | 3 |
| n (Pat) | 89 | 9 | 89 | 18 | 89 | 3 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 1.602 | 0.004 | 1.602 | 4.578 | 1.602 | 2.332 |
| average | 2.021 | 4.398 | 2.021 | 4.118 | 2.021 | 2.332 |
| stdev | 1.718 | na | 1.718 | 2.420 | 1.718 | 1.387 |
| p (t-test) | | na | | 0.017 | | 0.799 |
| min | 0.004 | 4.398 | 0.004 | 0.889 | 0.004 | 1.351 |
| max | 10.657 | 4.398 | 10.657 | 6.429 | 10.657 | 3.313 |
| n (Samp) | 195 | 1 | 195 | 4 | 195 | 2 |
| n (Pat) | 109 | 1 | 109 | 4 | 109 | 2 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 1.602 | 1.496 | 1.602 | 1.188 | 1.602 | 4.981 |
| average | 1.891 | 1.429 | 1.891 | 2.034 | 1.891 | 3.572 |
| stdev | 1.546 | 0.840 | 1.546 | 2.542 | 1.546 | 2.819 |
| p (t-test) | | 0.377 | | 0.750 | | 0.070 |
| min | 0.004 | 0.313 | 0.004 | 0.187 | 0.004 | 0.327 |
| max | 7.811 | 2.682 | 7.811 | 10.657 | 7.811 | 5.408 |
| n (Samp) | 129 | 9 | 129 | 16 | 129 | 3 |
| n (Pat) | 70 | 9 | 70 | 16 | 70 | 3 |

sCr or UO

| Time prior AKI stage | AUC | SE | nCohort 1 | nCohort 2 | p |
|---|---|---|---|---|---|
| 0 hours | 0.44 | 0.094 | 163 | 9 | 0.504 |
| 24 hours | 0.50 | 0.072 | 163 | 18 | 0.989 |
| 48 hours | 0.63 | 0.175 | 163 | 3 | 0.458 |

sCr only

| Time prior AKI stage | AUC | SE | nCohort 1 | nCohort 2 | p |
|---|---|---|---|---|---|
| 0 hours | 0.89 | 0.213 | 195 | 1 | 0.066 |
| 24 hours | 0.78 | 0.137 | 195 | 4 | 0.039 |
| 48 hours | 0.62 | 0.214 | 195 | 2 | 0.564 |

UO only

| Time prior AKI stage | AUC | SE | nCohort 1 | nCohort 2 | p |
|---|---|---|---|---|---|
| 0 hours | 0.44 | 0.095 | 129 | 9 | 0.528 |
| 24 hours | 0.47 | 0.075 | 129 | 16 | 0.686 |
| 48 hours | 0.69 | 0.173 | 129 | 3 | 0.278 |

Fig. 2 - 9

sCr or UO

| Time prior AKI stage | Cutoff value | sens | spec | Quartile | OR | 95% CI of OR | | |
|---|---|---|---|---|---|---|---|---|
| 0 hours | 0.87652 | 78% | 36% | 1 | | | | |
| | 0.39106 | 89% | 15% | 2 | na | na | na | |
| | 0.24965 | 100% | 11% | 3 | na | na | na | |
| | 2.69931 | 0% | 71% | 4 | na | na | na | |
| | 3.17568 | 0% | 80% | | | | | |
| | 4.18919 | 0% | 90% | | | | | |
| 24 hours | 0.64433 | 72% | 27% | 1 | | | | |
| | 0.34392 | 83% | 13% | 2 | 0.3 | 0.1 | 1.3 | |
| | 0.1868 | 94% | 10% | 3 | 1.0 | 0.5 | 2.2 | |
| | 2.69931 | 33% | 71% | 4 | 0.7 | 0.3 | 1.6 | |
| | 3.17568 | 22% | 80% | | | | | |
| | 4.18919 | 11% | 90% | | | | | |
| 48 hours | 0.3125 | 100% | 12% | 1 | | | | |
| | 0.3125 | 100% | 12% | 2 | 0.0 | 0.0 | na | |
| | 0.3125 | 100% | 12% | 3 | 0.0 | 0.0 | na | |
| | 2.69931 | 67% | 71% | 4 | 2.0 | 0.1 | 41.7 | |
| | 3.17568 | 67% | 80% | | | | | |
| | 4.18919 | 33% | 90% | | | | | |

UO only

| Time prior AKI stage | Cutoff value | sens | spec | Quartile | OR | 95% CI of OR | | |
|---|---|---|---|---|---|---|---|---|
| 0 hours | 0.87652 | 78% | 36% | 1 | | | | |
| | 0.3875 | 89% | 15% | 2 | na | na | na | |
| | 0.24965 | 100% | 11% | 3 | na | na | na | |
| | 2.61044 | 22% | 71% | 4 | na | na | na | |
| | 3.19703 | 0% | 81% | | | | | |
| | 4.07631 | 0% | 91% | | | | | |
| 24 hours | 0.6125 | 75% | 26% | 1 | | | | |
| | 0.34392 | 81% | 14% | 2 | 0.8 | 0.2 | 2.6 | |
| | 0.1868 | 94% | 10% | 3 | 1.3 | 0.5 | 3.6 | |
| | 2.61044 | 25% | 71% | 4 | 1.0 | 0.3 | 3.1 | |
| | 3.19703 | 13% | 81% | | | | | |
| | 4.07631 | 6% | 91% | | | | | |
| 48 hours | 0.3125 | 100% | 12% | 1 | | | | |
| | 0.3125 | 100% | 12% | 2 | 0.0 | 0.0 | na | |
| | 0.3125 | 100% | 12% | 3 | 0.0 | 0.0 | na | |
| | 2.61044 | 67% | 71% | 4 | 2.1 | 0.1 | 44.2 | |
| | 3.19703 | 67% | 81% | | | | | |
| | 4.07631 | 67% | 91% | | | | | |

# Fig. 2 - 10

Interleukin-1 receptor, type II

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 1013.458 | 1053.613 | 1013.458 | 965.043 | 1013.458 | 406.925 |
| average | 1947.934 | 1518.224 | 1947.934 | 2611.717 | 1947.934 | 1456.124 |
| stdev | 2953.653 | 1427.130 | 2953.653 | 6239.267 | 2953.653 | 1882.866 |
| p (t-test) | | 0.521 | | 0.330 | | 0.501 |
| min | 74.513 | 65.754 | 74.513 | 42.493 | 74.513 | 43.565 |
| max | 29514.673 | 4575.254 | 29514.673 | 34543.843 | 29514.673 | 5822.436 |
| n (Samp) | 211 | 20 | 211 | 31 | 211 | 17 |
| n (Pat) | 135 | 20 | 135 | 31 | 135 | 17 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 983.343 | 1595.879 | 983.343 | 1472.399 | 983.343 | 244.708 |
| average | 1962.008 | 1595.879 | 1962.008 | 7879.384 | 1962.008 | 1247.788 |
| stdev | 3361.423 | 1296.151 | 3361.423 | 17091.986 | 3361.423 | 2264.950 |
| p (t-test) | | 0.878 | | 0.000 | | 0.605 |
| min | 43.565 | 679.362 | 43.565 | 42.493 | 43.565 | 77.362 |
| max | 34543.843 | 2512.397 | 34543.843 | 46544.423 | 34543.843 | 5822.436 |
| n (Samp) | 275 | 2 | 275 | 7 | 275 | 6 |
| n (Pat) | 160 | 2 | 160 | 7 | 160 | 6 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 1228.267 | 1053.613 | 1228.267 | 712.138 | 1228.267 | 590.665 |
| average | 2020.859 | 1506.585 | 2020.859 | 2716.006 | 2020.859 | 1445.459 |
| stdev | 2565.834 | 1430.724 | 2565.834 | 6678.504 | 2565.834 | 1597.178 |
| p (t-test) | | 0.380 | | 0.318 | | 0.379 |
| min | 74.513 | 65.754 | 74.513 | 58.099 | 74.513 | 43.565 |
| max | 16156.572 | 4575.254 | 16156.572 | 34543.843 | 16156.572 | 5017.737 |
| n (Samp) | 183 | 20 | 183 | 27 | 183 | 16 |
| n (Pat) | 110 | 20 | 110 | 27 | 110 | 16 |

sCr or UO

| Time prior AKI stage | AUC | SE | nCohort 1 | nCohort 2 | p |
|---|---|---|---|---|---|
| 0 hours | 0.48 | 0.067 | 211 | 20 | 0.795 |
| 24 hours | 0.45 | 0.054 | 211 | 31 | 0.351 |
| 48 hours | 0.39 | 0.066 | 211 | 17 | 0.102 |

sCr only

| Time prior AKI stage | AUC | SE | nCohort 1 | nCohort 2 | p |
|---|---|---|---|---|---|
| 0 hours | 0.58 | 0.212 | 275 | 2 | 0.706 |
| 24 hours | 0.55 | 0.113 | 275 | 7 | 0.685 |
| 48 hours | 0.29 | 0.091 | 275 | 6 | 0.024 |

UO only

| Time prior AKI stage | AUC | SE | nCohort 1 | nCohort 2 | p |
|---|---|---|---|---|---|
| 0 hours | 0.46 | 0.066 | 183 | 20 | 0.541 |
| 24 hours | 0.42 | 0.056 | 183 | 27 | 0.154 |
| 48 hours | 0.41 | 0.070 | 183 | 16 | 0.198 |

Fig. 2 - 11

sCr or UO

| Time prior AKI stage | Cutoff value | sens | spec | Quartile | OR | 95% CI of OR | |
|---|---|---|---|---|---|---|---|
| 0 hours | 659.5 | 70% | 38% | 1 | | | |
| | 414.687 | 80% | 22% | 2 | 1.0 | 0.4 | 2.4 |
| | 179.864 | 90% | 8% | 3 | 1.0 | 0.4 | 2.4 |
| | 1968.17 | 25% | 70% | 4 | 1.0 | 0.4 | 2.4 |
| | 2678.52 | 20% | 80% | | | | |
| | 4295 | 10% | 90% | | | | |
| 24 hours | 334.433 | 71% | 17% | 1 | | | |
| | 265.191 | 81% | 13% | 2 | 0.6 | 0.3 | 1.2 |
| | 83.2021 | 90% | 1% | 3 | 0.4 | 0.2 | 0.9 |
| | 1968.17 | 32% | 70% | 4 | 1.4 | 0.9 | 2.3 |
| | 2678.52 | 26% | 80% | | | | |
| | 4295 | 10% | 90% | | | | |
| 48 hours | 334.433 | 71% | 17% | 1 | | | |
| | 176.85 | 82% | 8% | 2 | 0.5 | 0.1 | 2.3 |
| | 74.5128 | 94% | 0% | 3 | 0.5 | 0.1 | 2.3 |
| | 1968.17 | 24% | 70% | 4 | 2.5 | 1.1 | 5.4 |
| | 2678.52 | 24% | 80% | | | | |
| | 4295 | 18% | 90% | | | | |

sCr only

| Time prior AKI stage | Cutoff value | sens | spec | Quartile | OR | 95% CI of OR | |
|---|---|---|---|---|---|---|---|
| 0 hours | 661.163 | 100% | 40% | 1 | | | |
| | 661.163 | 100% | 40% | 2 | na | na | na |
| | 661.163 | 100% | 40% | 3 | na | na | na |
| | 1810.08 | 50% | 70% | 4 | na | na | na |
| | 2730.24 | 0% | 80% | | | | |
| | 4361.49 | 0% | 90% | | | | |
| 24 hours | 1013.46 | 71% | 52% | 1 | | | |
| | 115.527 | 86% | 4% | 2 | 0.0 | 0.0 | na |
| | 0 | 100% | 0% | 3 | 1.0 | 0.1 | 7.5 |
| | 1810.08 | 43% | 70% | 4 | 1.5 | 0.3 | 8.1 |
| | 2730.24 | 43% | 80% | | | | |
| | 4361.49 | 14% | 90% | | | | |
| 48 hours | 125.886 | 83% | 5% | 1 | | | |
| | 125.886 | 83% | 5% | 2 | 1.0 | 0.0 | 54.1 |
| | 74.5128 | 100% | 1% | 3 | 0.0 | 0.0 | na |
| | 1810.08 | 17% | 70% | 4 | 4.2 | 0.3 | 52.1 |
| | 2730.24 | 17% | 80% | | | | |
| | 4361.49 | 17% | 90% | | | | |

UO only

| Time prior AKI stage | Cutoff value | sens | spec | Quartile | OR | 95% CI of OR | |
|---|---|---|---|---|---|---|---|
| 0 hours | 594.5 | 70% | 31% | 1 | | | |
| | 414.687 | 80% | 21% | 2 | 1.6 | 0.6 | 3.9 |
| | 179.864 | 90% | 8% | 3 | 1.3 | 0.5 | 3.4 |
| | 2051.16 | 25% | 70% | 4 | 1.3 | 0.5 | 3.4 |
| | 3055.75 | 15% | 80% | | | | |
| | 4649.24 | 0% | 90% | | | | |
| 24 hours | 334.433 | 70% | 16% | 1 | | | |
| | 226.074 | 85% | 12% | 2 | 0.7 | 0.3 | 1.6 |
| | 83.2021 | 93% | 1% | 3 | 1.0 | 0.5 | 2.1 |

Fig. 2 - 12

| | 2051.16 | 30% | 70% | 4 | 2.1 | 1.2 | 3.8 |
| | 3055.75 | 15% | 80% | | | | |
| | 4649.24 | 11% | 90% | | | | |
| 48 hours | 334.433 | 75% | 16% | 1 | | | |
| | 226.074 | 81% | 12% | 2 | 0.7 | 0.2 | 2.5 |
| | 143.544 | 94% | 7% | 3 | 0.2 | 0.0 | 3.0 |
| | 2051.16 | 31% | 70% | 4 | 2.2 | 1.0 | 5.1 |
| | 3055.75 | 13% | 80% | | | | |
| | 4649.24 | 6% | 90% | | | | |

# Fig. 2 - 13

**Lymphotoxin-alpha**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 1.920 | 1.310 | 1.920 | 3.275 | 1.920 | 1.380 |
| average | 2.210 | 1.626 | 2.210 | 2.897 | 2.210 | 2.008 |
| stdev | 2.011 | 1.418 | 2.011 | 1.929 | 2.011 | 2.268 |
| p (t-test) | | 0.138 | | 0.049 | | 0.678 |
| min | 0.182 | 0.182 | 0.182 | 0.182 | 0.182 | 0.182 |
| max | 13.300 | 4.040 | 13.300 | 9.010 | 13.300 | 9.230 |
| n (Samp) | 419 | 27 | 419 | 36 | 419 | 18 |
| n (Pat) | 164 | 27 | 164 | 36 | 164 | 18 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 1.840 | 3.400 | 1.840 | 3.990 | 1.840 | 2.230 |
| average | 2.196 | 5.544 | 2.196 | 3.608 | 2.196 | 2.261 |
| stdev | 2.077 | 5.582 | 2.077 | 1.487 | 2.077 | 1.635 |
| p (t-test) | | 0.000 | | 0.043 | | 0.935 |
| min | 0.182 | 2.490 | 0.182 | 0.182 | 0.182 | 0.182 |
| max | 13.300 | 15.500 | 13.300 | 5.380 | 13.300 | 4.350 |
| n (Samp) | 518 | 5 | 518 | 9 | 518 | 7 |
| n (Pat) | 199 | 5 | 199 | 9 | 199 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 1.970 | 1.250 | 1.970 | 2.845 | 1.970 | 1.540 |
| average | 2.245 | 1.410 | 2.245 | 2.835 | 2.245 | 2.365 |
| stdev | 2.039 | 1.378 | 2.039 | 2.022 | 2.039 | 2.373 |
| p (t-test) | | 0.041 | | 0.129 | | 0.820 |
| min | 0.182 | 0.182 | 0.182 | 0.182 | 0.182 | 0.182 |
| max | 13.300 | 4.040 | 13.300 | 9.010 | 13.300 | 9.230 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
|---|---|---|---|---|---|
| 0 hours | 0.43 | 0.054 | 419 | 27 | 0.180 |
| 24 hours | 0.63 | 0.052 | 419 | 36 | 0.015 |
| 48 hours | 0.45 | 0.067 | 419 | 18 | 0.427 |

sCr only

| Time prior AKI stage | AUC | SE | nCohort 1 | nCohort 2 | p |
|---|---|---|---|---|---|
| 0 hours | 0.79 | 0.122 | 518 | 5 | 0.019 |
| 24 hours | 0.76 | 0.094 | 518 | 9 | 0.006 |
| 48 hours | 0.55 | 0.113 | 518 | 7 | 0.678 |

UO only

| Time prior AKI stage | AUC | SE | nCohort 1 | nCohort 2 | p |
|---|---|---|---|---|---|
| 0 hours | 0.38 | 0.052 | 352 | 26 | 0.019 |
| 24 hours | 0.61 | 0.057 | 352 | 30 | 0.063 |
| 48 hours | 0.50 | 0.074 | 352 | 16 | 0.987 |

# Fig. 2 - 14

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.2 | 0.6 | 2.6 |
| | 0 | 100% | 0% | 3 | 1.4 | 0.7 | 2.9 |
| | 2.84 | 30% | 70% | 4 | 1.9 | 1.0 | 3.6 |
| | 3.52 | 4% | 80% | | | | |
| | 4.61 | 0% | 90% | | | | |
| 24 hours | 1.84 | 72% | 48% | 1 | | | |
| | 0.846 | 81% | 28% | 2 | 0.8 | 0.4 | 1.7 |
| | 0 | 100% | 0% | 3 | 1.5 | 0.9 | 2.7 |
| | 2.84 | 56% | 70% | 4 | 2.9 | 1.8 | 4.7 |
| | 3.52 | 31% | 80% | | | | |
| | 4.61 | 11% | 90% | | | | |
| 48 hours | 0 | 100% | 0% | 1 | | | |
| | 0 | 100% | 0% | 2 | 0.8 | 0.2 | 2.4 |
| | 0 | 100% | 0% | 3 | 1.8 | 0.8 | 4.1 |
| | 2.84 | 28% | 70% | 4 | 1.0 | 0.4 | 2.8 |
| | 3.52 | 17% | 80% | | | | |
| | 4.61 | 6% | 90% | | | | |

sCr only

| Time prior AKI stage | Cutoff value | sens | spec | Quartile | OR | 95% CI of OR | |
|---|---|---|---|---|---|---|---|
| 0 hours | 2.76 | 80% | 69% | 1 | | | |
| | 2.76 | 80% | 69% | 2 | na | na | na |
| | 2.47 | 100% | 64% | 3 | na | na | na |
| | 2.83 | 60% | 70% | 4 | na | na | na |
| | 3.52 | 20% | 81% | | | | |
| | 4.61 | 20% | 90% | | | | |
| 24 hours | 3.37 | 78% | 77% | 1 | | | |
| | 2.76 | 89% | 69% | 2 | na | na | na |
| | 0 | 100% | 0% | 3 | na | na | na |
| | 2.83 | 78% | 70% | 4 | na | na | na |
| | 3.52 | 56% | 81% | | | | |
| | 4.61 | 11% | 90% | | | | |
| 48 hours | 1.98 | 71% | 52% | 1 | | | |
| | 0 | 100% | 0% | 2 | 0.0 | 0.0 | na |
| | 0 | 100% | 0% | 3 | 1.5 | 0.3 | 8.0 |
| | 2.83 | 43% | 70% | 4 | 1.0 | 0.1 | 7.3 |
| | 3.52 | 29% | 81% | | | | |
| | 4.61 | 0% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.7 | 0.6 | 5.1 |
| | 0 | 100% | 0% | 3 | 2.1 | 0.7 | 5.8 |
| | 2.9 | 27% | 70% | 4 | 4.5 | 1.9 | 10.6 |
| | 3.52 | 4% | 80% | | | | |
| | 4.61 | 0% | 90% | | | | |
| 24 hours | 1.84 | 70% | 48% | 1 | | | |
| | 0.768 | 80% | 28% | 2 | 1.0 | 0.4 | 2.3 |
| | 0 | 100% | 0% | 3 | 1.4 | 0.7 | 2.9 |
| | 2.9 | 47% | 70% | 4 | 2.8 | 1.6 | 5.1 |
| | 3.52 | 27% | 80% | | | | |

Fig. 2 - 15

| | 4.61 | 17% | 90% | | | | |
|---|---|---|---|---|---|---|---|
| 48 hours | 0.93 | 75% | 29% | 1 | | | |
| | 0 | 100% | 0% | 2 | 0.4 | 0.1 | 1.6 |
| | 0 | 100% | 0% | 3 | 1.0 | 0.4 | 2.3 |
| | 2.9 | 38% | 70% | 4 | 0.8 | 0.3 | 2.0 |
| | 3.52 | 19% | 80% | | | | |
| | 4.61 | 6% | 90% | | | | |

Fig. 2 - 16

**Matrix metalloproteinase-3**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.028 | 0.039 | 0.028 | 0.031 | 0.028 | 0.047 |
| average | 0.137 | 0.065 | 0.137 | 1.442 | 0.137 | 0.090 |
| stdev | 0.881 | 0.104 | 0.881 | 8.155 | 0.881 | 0.119 |
| p (t-test) | | 0.673 | | 0.002 | | 0.823 |
| min | 0.001 | 0.001 | 0.001 | 0.001 | 0.001 | 0.001 |
| max | 16.700 | 0.494 | 16.700 | 49.000 | 16.700 | 0.404 |
| n (Samp) | 419 | 27 | 419 | 36 | 419 | 18 |
| n (Pat) | 164 | 27 | 164 | 36 | 164 | 18 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.030 | 0.014 | 0.030 | 0.032 | 0.030 | 0.039 |
| average | 0.226 | 0.052 | 0.226 | 0.158 | 0.226 | 0.070 |
| stdev | 2.293 | 0.075 | 2.293 | 0.367 | 2.293 | 0.098 |
| p (t-test) | | 0.865 | | 0.930 | | 0.857 |
| min | 0.001 | 0.001 | 0.001 | 0.001 | 0.001 | 0.001 |
| max | 49.000 | 0.182 | 49.000 | 1.130 | 49.000 | 0.286 |
| n (Samp) | 518 | 5 | 518 | 9 | 518 | 7 |
| n (Pat) | 199 | 5 | 199 | 9 | 199 | 7 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.027 | 0.037 | 0.027 | 0.031 | 0.027 | 0.054 |
| average | 0.149 | 0.066 | 0.149 | 1.685 | 0.149 | 0.090 |
| stdev | 0.957 | 0.105 | 0.957 | 8.937 | 0.957 | 0.114 |
| p (t-test) | | 0.660 | | 0.002 | | 0.805 |
| min | 0.001 | 0.001 | 0.001 | 0.001 | 0.001 | 0.001 |
| max | 16.700 | 0.494 | 16.700 | 49.000 | 16.700 | 0.404 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
|---|---|---|---|---|---|
| 0 hours | 0.54 | 0.059 | 419 | 27 | 0.444 |
| 24 hours | 0.53 | 0.051 | 419 | 36 | 0.577 |
| 48 hours | 0.57 | 0.072 | 419 | 18 | 0.335 |

sCr only

| Time prior AKI stage | AUC | SE | nCohort 1 | nCohort 2 | p |
|---|---|---|---|---|---|
| 0 hours | 0.44 | 0.124 | 518 | 5 | 0.640 |
| 24 hours | 0.50 | 0.097 | 518 | 9 | 0.961 |
| 48 hours | 0.55 | 0.113 | 518 | 7 | 0.639 |

UO only

| Time prior AKI stage | AUC | SE | nCohort 1 | nCohort 2 | p |
|---|---|---|---|---|---|
| 0 hours | 0.55 | 0.060 | 352 | 26 | 0.431 |
| 24 hours | 0.53 | 0.056 | 352 | 30 | 0.613 |
| 48 hours | 0.58 | 0.076 | 352 | 16 | 0.278 |

# Fig. 2 - 17

sCr or UO

| Time prior AKI stage | Cutoff value | sens | spec | Quartile | OR | 95% CI of OR | |
|---|---|---|---|---|---|---|---|
| 0 hours | 0.0143 | 70% | 31% | 1 | | | |
| | 0.0063 | 81% | 21% | 2 | 0.5 | 0.2 | 1.3 |
| | 0 | 100% | 0% | 3 | 1.9 | 1.1 | 3.3 |
| | 0.0489 | 44% | 70% | 4 | 1.2 | 0.6 | 2.2 |
| | 0.0739 | 19% | 80% | | | | |
| | 0.159 | 7% | 90% | | | | |
| 24 hours | 0.0202 | 72% | 39% | 1 | | | |
| | 0.00783 | 81% | 22% | 2 | 0.9 | 0.5 | 1.5 |
| | 0 | 100% | 0% | 3 | 1.5 | 1.0 | 2.4 |
| | 0.0489 | 28% | 70% | 4 | 1.1 | 0.7 | 1.9 |
| | 0.0739 | 22% | 80% | | | | |
| | 0.159 | 14% | 90% | | | | |
| 48 hours | 0.0143 | 72% | 31% | 1 | | | |
| | 0 | 100% | 0% | 2 | 0.4 | 0.1 | 1.6 |
| | 0 | 100% | 0% | 3 | 0.6 | 0.2 | 1.7 |
| | 0.0489 | 44% | 70% | 4 | 1.6 | 0.8 | 3.2 |
| | 0.0739 | 28% | 80% | | | | |
| | 0.159 | 22% | 90% | | | | |

sCr only

| Time prior AKI stage | Cutoff value | sens | spec | Quartile | OR | 95% CI of OR | |
|---|---|---|---|---|---|---|---|
| 0 hours | 0.00967 | 80% | 24% | 1 | | | |
| | 0.00967 | 80% | 24% | 2 | 1.0 | 0.0 | 51.9 |
| | 0 | 100% | 0% | 3 | 1.0 | 0.0 | 51.9 |
| | 0.0531 | 20% | 70% | 4 | 2.0 | 0.1 | 39.6 |
| | 0.0784 | 20% | 80% | | | | |
| | 0.167 | 20% | 90% | | | | |
| 24 hours | 0.00783 | 78% | 21% | 1 | | | |
| | 0 | 100% | 0% | 2 | 1.5 | 0.3 | 8.0 |
| | 0 | 100% | 0% | 3 | 0.5 | 0.0 | 9.7 |
| | 0.0531 | 22% | 70% | 4 | 1.5 | 0.3 | 8.0 |
| | 0.0784 | 22% | 80% | | | | |
| | 0.167 | 11% | 90% | | | | |
| 48 hours | 0.0304 | 71% | 50% | 1 | | | |
| | 0.017 | 86% | 32% | 2 | 1.0 | 0.0 | 51.9 |
| | 0 | 100% | 0% | 3 | 3.0 | 0.2 | 42.9 |
| | 0.0531 | 29% | 70% | 4 | 2.0 | 0.1 | 39.0 |
| | 0.0784 | 14% | 80% | | | | |
| | 0.167 | 14% | 90% | | | | |

UO only

| Time prior AKI stage | Cutoff value | sens | spec | Quartile | OR | 95% CI of OR | |
|---|---|---|---|---|---|---|---|
| 0 hours | 0.0143 | 73% | 32% | 1 | | | |
| | 0.0063 | 81% | 22% | 2 | 0.3 | 0.1 | 1.2 |
| | 0 | 100% | 0% | 3 | 1.9 | 1.1 | 3.4 |
| | 0.0506 | 42% | 71% | 4 | 1.2 | 0.6 | 2.2 |
| | 0.0754 | 19% | 80% | | | | |
| | 0.178 | 8% | 90% | | | | |
| 24 hours | 0.0202 | 73% | 40% | 1 | | | |
| | 0.00967 | 80% | 26% | 2 | 1.2 | 0.6 | 2.2 |
| | 0 | 100% | 0% | 3 | 1.9 | 1.1 | 3.4 |
| | 0.0506 | 30% | 71% | 4 | 1.0 | 0.5 | 2.0 |
| | 0.0754 | 20% | 80% | | | | |

# Fig. 2 - 18

| | 0.178 | 10% | 90% | | | | |
|---|---|---|---|---|---|---|---|
| 48 hours | 0.0141 | 75% | 30% | 1 | | | |
| | 0.0063 | 81% | 22% | 2 | 0.5 | 0.1 | 2.2 |
| | 0 | 100% | 0% | 3 | 0.7 | 0.2 | 2.4 |
| | 0.0506 | 50% | 71% | 4 | 1.8 | 0.8 | 4.1 |
| | 0.0754 | 31% | 80% | | | | |
| | 0.178 | 19% | 90% | | | | |

# Fig. 2 - 19

**Thrombospondin-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 5.484 | 8.700 | 5.484 | 6.908 | 5.484 | 4.790 |
| average | 16.192 | 11.400 | 16.192 | 7.298 | 16.192 | 4.790 |
| stdev | 51.731 | 7.467 | 51.731 | 3.886 | 51.731 | 3.848 |
| p (t-test) | | 0.795 | | 0.768 | | 0.758 |
| min | 0.828 | 3.622 | 0.828 | 3.622 | 0.828 | 2.070 |
| max | 435.086 | 22.485 | 435.086 | 11.364 | 435.086 | 7.511 |
| n (Samp) | 78 | 8 | 78 | 3 | 78 | 2 |
| n (Pat) | 56 | 8 | 56 | 3 | 56 | 2 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 6.881 | 0.828 | 6.881 | 0.828 | 6.881 | 5.550 |
| average | 15.643 | 5.399 | 15.643 | 6.908 | 15.643 | 5.550 |
| stdev | 46.847 | na | 46.847 | na | 46.847 | 2.773 |
| p (t-test) | | na | | na | | 0.762 |
| min | 0.828 | 5.399 | 0.828 | 6.908 | 0.828 | 3.590 |
| max | 435.086 | 5.399 | 435.086 | 6.908 | 435.086 | 7.511 |
| n (Samp) | 96 | 1 | 96 | 1 | 96 | 2 |
| n (Pat) | 69 | 1 | 69 | 1 | 69 | 2 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 4.494 | 8.700 | 4.494 | 7.493 | 4.494 | 0.828 |
| average | 8.052 | 11.400 | 8.052 | 7.493 | 8.052 | 2.070 |
| stdev | 12.651 | 7.467 | 12.651 | 5.474 | 12.651 | na |
| p (t-test) | | 0.468 | | 0.951 | | na |
| min | 0.828 | 3.622 | 0.828 | 3.622 | 0.828 | 2.070 |
| max | 93.490 | 22.485 | 93.490 | 11.364 | 93.490 | 2.070 |
| n (Samp) | 61 | 8 | 61 | 2 | 61 | 1 |
| n (Pat) | 43 | 8 | 43 | 2 | 43 | 1 |

sCr or UO

| Time prior AKI stage | AUC | SE | nCohort 1 | nCohort 2 | p |
|---|---|---|---|---|---|
| 0 hours | 0.65 | 0.110 | 78 | 8 | 0.173 |
| 24 hours | 0.56 | 0.176 | 78 | 3 | 0.715 |
| 48 hours | 0.35 | 0.177 | 78 | 2 | 0.405 |

sCr only

| Time prior AKI stage | AUC | SE | nCohort 1 | nCohort 2 | p |
|---|---|---|---|---|---|
| 0 hours | 0.44 | 0.277 | 96 | 1 | 0.821 |
| 24 hours | 0.50 | 0.292 | 96 | 1 | 1.000 |
| 48 hours | 0.42 | 0.193 | 96 | 2 | 0.676 |

UO only

| Time prior AKI stage | AUC | SE | nCohort 1 | nCohort 2 | p |
|---|---|---|---|---|---|
| 0 hours | 0.71 | 0.108 | 61 | 8 | 0.052 |
| 24 hours | 0.64 | 0.216 | 61 | 2 | 0.519 |
| 48 hours | 0.15 | 0.134 | 61 | 1 | 0.009 |

## Fig. 2 - 20

**ENA-78 (C-X-C chemokine motif 5)**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.018 | 0.025 | 0.018 | 0.025 | 0.018 | 0.025 |
| average | 0.037 | 0.143 | 0.037 | 0.143 | 0.037 | 0.143 |
| stdev | 0.083 | 0.495 | 0.083 | 0.495 | 0.083 | 0.495 |
| p (t-test) | | 0.126 | | 0.126 | | 0.126 |
| min | 0.000 | 0.000 | 0.000 | 0.000 | 0.000 | 0.000 |
| max | 0.531 | 2.350 | 0.531 | 2.350 | 0.531 | 2.350 |
| n (Samp) | 54 | 22 | 54 | 22 | 54 | 22 |
| n (Pat) | 54 | 22 | 54 | 22 | 54 | 22 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.020 | 0.027 | 0.020 | 0.027 | 0.020 | 0.027 |
| average | 0.024 | 0.026 | 0.024 | 0.026 | 0.024 | 0.026 |
| stdev | 0.025 | 0.011 | 0.025 | 0.011 | 0.025 | 0.011 |
| p (t-test) | | 0.815 | | 0.815 | | 0.815 |
| min | 0.000 | 0.012 | 0.000 | 0.012 | 0.000 | 0.012 |
| max | 0.107 | 0.039 | 0.107 | 0.039 | 0.107 | 0.039 |
| n (Samp) | 20 | 5 | 20 | 5 | 20 | 5 |
| n (Pat) | 20 | 5 | 20 | 5 | 20 | 5 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.018 | 0.024 | 0.018 | 0.024 | 0.018 | 0.024 |
| average | 0.045 | 0.162 | 0.045 | 0.162 | 0.045 | 0.162 |
| stdev | 0.095 | 0.564 | 0.095 | 0.564 | 0.095 | 0.564 |
| p (t-test) | | 0.184 | | 0.184 | | 0.184 |
| min | 0.000 | 0.000 | 0.000 | 0.000 | 0.000 | 0.000 |
| max | 0.531 | 2.350 | 0.531 | 2.350 | 0.531 | 2.350 |
| n (Samp) | 44 | 17 | 44 | 17 | 44 | 17 |
| n (Pat) | 44 | 17 | 44 | 17 | 44 | 17 |

sCr or UO

| Time prior AKI stage | AUC | SE | nCohort 1 | nCohort 2 | p |
|---|---|---|---|---|---|
| 0 hours | 0.66 | 0.072 | 54 | 22 | 0.030 |
| 24 hours | 0.66 | 0.072 | 54 | 22 | 0.030 |
| 48 hours | 0.66 | 0.072 | 54 | 22 | 0.030 |

sCr only

| Time prior AKI stage | AUC | SE | nCohort 1 | nCohort 2 | p |
|---|---|---|---|---|---|
| 0 hours | 0.66 | 0.147 | 20 | 5 | 0.291 |
| 24 hours | 0.66 | 0.147 | 20 | 5 | 0.291 |
| 48 hours | 0.66 | 0.147 | 20 | 5 | 0.291 |

UO only

| Time prior AKI stage | AUC | SE | nCohort 1 | nCohort 2 | p |
|---|---|---|---|---|---|
| 0 hours | 0.63 | 0.083 | 44 | 17 | 0.127 |
| 24 hours | 0.63 | 0.083 | 44 | 17 | 0.127 |
| 48 hours | 0.63 | 0.083 | 44 | 17 | 0.127 |

sCr or UO

| Time prior AKI stage | Cutoff value | sens | spec | Quartile | OR | 95% CI of OR | |
|---|---|---|---|---|---|---|---|
| 0 hours | 0.0178 | 73% | 50% | 1 | | | |
| | 0.0143 | 82% | 46% | 2 | 3.9 | 0.8 | 18.9 |

# Fig. 3 - 1

| | 0.0116 | 91% | 39% | 3 | 3.9 | 0.8 | 18.9 |
|---|---|---|---|---|---|---|---|
| | 0.0255 | 50% | 70% | 4 | 6.2 | 1.4 | 28.2 |
| | 0.0348 | 36% | 81% | | | | |
| | 0.0639 | 14% | 91% | | | | |
| 24 hours | 0.0178 | 73% | 50% | 1 | | | |
| | 0.0143 | 82% | 46% | 2 | 3.9 | 0.8 | 18.9 |
| | 0.0116 | 91% | 39% | 3 | 3.9 | 0.8 | 18.9 |
| | 0.0255 | 50% | 70% | 4 | 6.2 | 1.4 | 28.2 |
| | 0.0348 | 36% | 81% | | | | |
| | 0.0639 | 14% | 91% | | | | |
| 48 hours | 0.0178 | 73% | 50% | 1 | | | |
| | 0.0143 | 82% | 46% | 2 | 3.9 | 0.8 | 18.9 |
| | 0.0116 | 91% | 39% | 3 | 3.9 | 0.8 | 18.9 |
| | 0.0255 | 50% | 70% | 4 | 6.2 | 1.4 | 28.2 |
| | 0.0348 | 36% | 81% | | | | |
| | 0.0639 | 14% | 91% | | | | |

sCr only

| Time prior AKI stage | Cutoff value | sens | spec | Quartile | OR | 95% CI of OR | |
|---|---|---|---|---|---|---|---|
| 0 hours | 0.0176 | 80% | 50% | 1 | | | |
| | 0.0176 | 80% | 50% | 2 | na | na | na |
| | 0.0107 | 100% | 35% | 3 | na | na | na |
| | 0.0238 | 60% | 70% | 4 | na | na | na |
| | 0.0282 | 40% | 80% | | | | |
| | 0.0425 | 0% | 90% | | | | |
| 24 hours | 0.0176 | 80% | 50% | 1 | | | |
| | 0.0176 | 80% | 50% | 2 | na | na | na |
| | 0.0107 | 100% | 35% | 3 | na | na | na |
| | 0.0238 | 60% | 70% | 4 | na | na | na |
| | 0.0282 | 40% | 80% | | | | |
| | 0.0425 | 0% | 90% | | | | |
| 48 hours | 0.0176 | 80% | 50% | 1 | | | |
| | 0.0176 | 80% | 50% | 2 | na | na | na |
| | 0.0107 | 100% | 35% | 3 | na | na | na |
| | 0.0238 | 60% | 70% | 4 | na | na | na |
| | 0.0282 | 40% | 80% | | | | |
| | 0.0425 | 0% | 90% | | | | |

UO only

| Time prior AKI stage | Cutoff value | sens | spec | Quartile | OR | 95% CI of OR | |
|---|---|---|---|---|---|---|---|
| 0 hours | 0.0184 | 71% | 55% | 1 | | | |
| | 0.0168 | 82% | 50% | 2 | 7.0 | 0.5 | 102.9 |
| | 0.0125 | 94% | 45% | 3 | 7.0 | 0.5 | 102.9 |
| | 0.0269 | 35% | 70% | 4 | 8.4 | 0.6 | 115.7 |
| | 0.039 | 24% | 82% | | | | |
| | 0.0894 | 6% | 91% | | | | |
| 24 hours | 0.0184 | 71% | 55% | 1 | | | |
| | 0.0168 | 82% | 50% | 2 | 7.0 | 0.5 | 102.9 |
| | 0.0125 | 94% | 45% | 3 | 7.0 | 0.5 | 102.9 |
| | 0.0269 | 35% | 70% | 4 | 8.4 | 0.6 | 115.7 |
| | 0.039 | 24% | 82% | | | | |
| | 0.0894 | 6% | 91% | | | | |
| 48 hours | 0.0184 | 71% | 55% | 1 | | | |
| | 0.0168 | 82% | 50% | 2 | 7.0 | 0.5 | 102.9 |
| | 0.0125 | 94% | 45% | 3 | 7.0 | 0.5 | 102.9 |
| | 0.0269 | 35% | 70% | 4 | 8.4 | 0.6 | 115.7 |
| | 0.039 | 24% | 82% | | | | |
| | 0.0894 | 6% | 91% | | | | |

Fig. 3 - 2

**CD44**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 145.766 | 161.794 | 145.766 | 160.907 | 145.766 | 161.794 |
| average | 191.892 | 231.164 | 191.892 | 240.268 | 191.892 | 220.705 |
| stdev | 162.949 | 199.915 | 162.949 | 201.063 | 162.949 | 210.246 |
| p (t-test) |  | 0.463 |  | 0.381 |  | 0.674 |
| min | 26.660 | 10.704 | 26.660 | 51.399 | 26.660 | 51.399 |
| max | 792.530 | 666.214 | 792.530 | 666.214 | 792.530 | 666.214 |
| n (Samp) | 50 | 13 | 50 | 12 | 50 | 7 |
| n (Pat) | 50 | 13 | 50 | 12 | 50 | 7 |

sCr only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 157.371 | 75.466 | 157.371 | 102.358 | 157.371 | 75.466 |
| average | 216.768 | 153.002 | 216.768 | 176.718 | 216.768 | 117.841 |
| stdev | 202.518 | 172.869 | 202.518 | 176.453 | 202.518 | 95.004 |
| p (t-test) |  | 0.420 |  | 0.638 |  | 0.403 |
| min | 17.617 | 10.704 | 17.617 | 51.399 | 17.617 | 51.399 |
| max | 1198.603 | 512.222 | 1198.603 | 512.222 | 1198.603 | 226.658 |
| n (Samp) | 93 | 7 | 93 | 6 | 93 | 3 |
| n (Pat) | 93 | 7 | 93 | 6 | 93 | 3 |

UO only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 140.047 | 220.678 | 140.047 | 161.794 | 140.047 | 161.794 |
| average | 162.057 | 287.084 | 162.057 | 271.197 | 162.057 | 253.375 |
| stdev | 132.738 | 208.117 | 132.738 | 218.039 | 132.738 | 240.408 |
| p (t-test) |  | 0.043 |  | 0.079 |  | 0.198 |
| min | 27.849 | 75.466 | 27.849 | 75.466 | 27.849 | 75.466 |
| max | 727.823 | 666.214 | 727.823 | 666.214 | 727.823 | 666.214 |
| n (Samp) | 38 | 7 | 38 | 7 | 38 | 5 |
| n (Pat) | 38 | 7 | 38 | 7 | 38 | 5 |

sCr or UO

| Time prior AKI stage | AUC | SE | $n_{Cohort\ 1}$ | $n_{Cohort\ 2}$ | p |
|---|---|---|---|---|---|
| 0 hours | 0.55 | 0.092 | 50 | 13 | 0.603 |
| 24 hours | 0.56 | 0.095 | 50 | 12 | 0.505 |
| 48 hours | 0.53 | 0.119 | 50 | 7 | 0.774 |

sCr only

| Time prior AKI stage | AUC | SE | $n_{Cohort\ 1}$ | $n_{Cohort\ 2}$ | p |
|---|---|---|---|---|---|
| 0 hours | 0.36 | 0.098 | 93 | 7 | 0.145 |
| 24 hours | 0.42 | 0.114 | 93 | 6 | 0.460 |
| 48 hours | 0.33 | 0.141 | 93 | 3 | 0.236 |

UO only

| Time prior AKI stage | AUC | SE | $n_{Cohort\ 1}$ | $n_{Cohort\ 2}$ | p |
|---|---|---|---|---|---|
| 0 hours | 0.73 | 0.115 | 38 | 7 | 0.042 |
| 24 hours | 0.67 | 0.120 | 38 | 7 | 0.169 |
| 48 hours | 0.62 | 0.142 | 38 | 5 | 0.394 |

sCr or UO

| Time prior AKI stage | Cutoff value | sens | spec | Quartile | OR | 95% CI of OR | |
|---|---|---|---|---|---|---|---|
| 0 hours | 70.9821 | 77% | 22% | 1 |  |  |  |
|  | 62.8125 | 85% | 18% | 2 | 0.2 | 0.0 | 2.9 |

## Fig. 4 - 1

| | 48.75 | 92% | 14% | 3 | 0.9 | 0.2 | 3.4 |
|---|---|---|---|---|---|---|---|
| | 206.638 | 46% | 70% | 4 | 0.9 | 0.2 | 3.4 |
| | 252.672 | 31% | 80% | | | | |
| | 358.889 | 23% | 90% | | | | |
| 24 hours | 98.6486 | 75% | 34% | 1 | | | |
| | 70.9821 | 83% | 22% | 2 | 0.6 | 0.1 | 4.0 |
| | 62.8125 | 92% | 18% | 3 | 1.0 | 0.2 | 5.1 |
| | 206.638 | 42% | 70% | 4 | 1.3 | 0.3 | 5.8 |
| | 252.672 | 33% | 80% | | | | |
| | 358.889 | 25% | 90% | | | | |
| 48 hours | 98.6486 | 71% | 34% | 1 | | | |
| | 70.9821 | 86% | 22% | 2 | 0.5 | 0.0 | 12.0 |
| | 48.75 | 100% | 14% | 3 | 1.0 | 0.1 | 9.8 |
| | 206.638 | 43% | 70% | 4 | 0.9 | 0.1 | 9.0 |
| | 252.672 | 29% | 80% | | | | |
| | 358.889 | 14% | 90% | | | | |

sCr only

| Time prior AKI stage | Cutoff value | sens | spec | Quartile | OR | 95% CI of OR | |
|---|---|---|---|---|---|---|---|
| 0 hours | 62.8125 | 71% | 15% | 1 | | | |
| | 48.75 | 86% | 12% | 2 | 1.0 | 0.0 | 59.3 |
| | 0 | 100% | 0% | 3 | 1.0 | 0.0 | 59.3 |
| | 245.431 | 14% | 71% | 4 | 4.6 | 0.3 | 63.1 |
| | 329.874 | 14% | 81% | | | | |
| | 471.481 | 14% | 90% | | | | |
| 24 hours | 62.8125 | 83% | 15% | 1 | | | |
| | 62.8125 | 83% | 15% | 2 | 1.0 | 0.0 | 59.3 |
| | 48.75 | 100% | 12% | 3 | 1.0 | 0.0 | 59.3 |
| | 245.431 | 17% | 71% | 4 | 3.4 | 0.2 | 55.7 |
| | 329.874 | 17% | 81% | | | | |
| | 471.481 | 17% | 90% | | | | |
| 48 hours | 48.75 | 100% | 12% | 1 | | | |
| | 48.75 | 100% | 12% | 2 | na | na | na |
| | 48.75 | 100% | 12% | 3 | na | na | na |
| | 245.431 | 0% | 71% | 4 | na | na | na |
| | 329.874 | 0% | 81% | | | | |
| | 471.481 | 0% | 90% | | | | |

UO only

| Time prior AKI stage | Cutoff value | sens | spec | Quartile | OR | 95% CI of OR | |
|---|---|---|---|---|---|---|---|
| 0 hours | 160.086 | 71% | 63% | 1 | | | |
| | 157.371 | 86% | 61% | 2 | 0.0 | 0.0 | na |
| | 70.9821 | 100% | 24% | 3 | 2.2 | 0.1 | 63.6 |
| | 175.086 | 57% | 71% | 4 | 5.0 | 0.3 | 90.1 |
| | 206.638 | 57% | 82% | | | | |
| | 252.672 | 43% | 92% | | | | |
| 24 hours | 157.371 | 71% | 61% | 1 | | | |
| | 98.6486 | 86% | 34% | 2 | 1.0 | 0.0 | 74.6 |
| | 70.9821 | 100% | 24% | 3 | 2.2 | 0.1 | 63.6 |
| | 175.086 | 43% | 71% | 4 | 3.3 | 0.2 | 68.8 |
| | 206.638 | 43% | 82% | | | | |
| | 252.672 | 43% | 92% | | | | |
| 48 hours | 98.6486 | 80% | 34% | 1 | | | |
| | 98.6486 | 80% | 34% | 2 | 0.9 | 0.0 | 68.6 |
| | 70.9821 | 100% | 24% | 3 | 0.9 | 0.0 | 68.6 |
| | 175.086 | 40% | 71% | 4 | 2.0 | 0.1 | 58.5 |
| | 206.638 | 40% | 82% | | | | |
| | 252.672 | 40% | 92% | | | | |

Fig. 4 - 2

**Angiopoietin-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 53.759 | 160.714 | 53.759 | 112.149 | 53.759 | 185.185 |
| average | 80.478 | 967.589 | 80.478 | 1036.661 | 80.478 | 236.066 |
| stdev | 80.285 | 2862.475 | 80.285 | 2978.333 | 80.285 | 248.163 |
| p (t-test) | | 0.032 | | 0.026 | | 0.001 |
| min | 0.548 | 0.548 | 0.548 | 0.548 | 0.548 | 0.548 |
| max | 292.857 | 10472.009 | 292.857 | 10472.009 | 292.857 | 623.874 |
| n (Samp) | 48 | 13 | 48 | 12 | 48 | 7 |
| n (Pat) | 48 | 13 | 48 | 12 | 48 | 7 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 42.832 | 29.808 | 42.832 | 34.461 | 42.832 | 185.185 |
| average | 189.991 | 131.452 | 189.991 | 152.058 | 189.991 | 269.869 |
| stdev | 1099.724 | 225.809 | 1099.724 | 240.044 | 1099.724 | 320.176 |
| p (t-test) | | 0.889 | | 0.933 | | 0.901 |
| min | 0.548 | 4.650 | 0.548 | 4.650 | 0.548 | 0.548 |
| max | 10472.009 | 623.874 | 10472.009 | 623.874 | 10472.009 | 623.874 |
| n (Samp) | 90 | 7 | 90 | 6 | 90 | 3 |
| n (Pat) | 90 | 7 | 90 | 6 | 90 | 3 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 51.215 | 355.655 | 51.215 | 355.655 | 51.215 | 355.655 |
| average | 71.995 | 1754.623 | 71.995 | 1735.922 | 71.995 | 293.346 |
| stdev | 80.824 | 3849.406 | 80.824 | 3858.746 | 80.824 | 271.591 |
| p (t-test) | | 0.008 | | 0.008 | | 0.000 |
| min | 0.548 | 0.548 | 0.548 | 0.548 | 0.548 | 0.548 |
| max | 292.857 | 10472.009 | 292.857 | 10472.009 | 292.857 | 623.874 |
| n (Samp) | 37 | 7 | 37 | 7 | 37 | 5 |
| n (Pat) | 37 | 7 | 37 | 7 | 37 | 5 |

sCr or UO

| Time prior AKI stage | AUC | SE | $n_{Cohort\ 1}$ | $n_{Cohort\ 2}$ | p |
|---|---|---|---|---|---|
| 0 hours | 0.61 | 0.092 | 48 | 13 | 0.233 |
| 24 hours | 0.61 | 0.095 | 48 | 12 | 0.246 |
| 48 hours | 0.61 | 0.120 | 48 | 7 | 0.361 |

sCr only

| Time prior AKI stage | AUC | SE | $n_{Cohort\ 1}$ | $n_{Cohort\ 2}$ | p |
|---|---|---|---|---|---|
| 0 hours | 0.50 | 0.114 | 90 | 7 | 0.983 |
| 24 hours | 0.55 | 0.125 | 90 | 6 | 0.663 |
| 48 hours | 0.64 | 0.176 | 90 | 3 | 0.436 |

UO only

| Time prior AKI stage | AUC | SE | $n_{Cohort\ 1}$ | $n_{Cohort\ 2}$ | p |
|---|---|---|---|---|---|
| 0 hours | 0.83 | 0.099 | 37 | 7 | 0.001 |
| 24 hours | 0.76 | 0.111 | 37 | 7 | 0.018 |
| 48 hours | 0.69 | 0.139 | 37 | 5 | 0.173 |

sCr or UO

| Time prior AKI stage | Cutoff value | sens | spec | Quartile | OR | 95% CI of OR | |
|---|---|---|---|---|---|---|---|
| 0 hours | 21.8818 | 77% | 31% | 1 | | | |
| | 4.64989 | 85% | 13% | 2 | 1.0 | 0.2 | 5.1 |

## Fig. 4 - 3

| | Cutoff value | sens | spec | Quartile | OR | 95% CI of OR | |
|---|---|---|---|---|---|---|---|
| | 3.4965 | 92% | 10% | 3 | 0.0 | 0.0 | na |
| | 99.0385 | 54% | 73% | 4 | 3.1 | 0.8 | 11.6 |
| | 133.772 | 54% | 81% | | | | |
| | 212.963 | 31% | 92% | | | | |
| 24 hours | 29.7203 | 75% | 33% | 1 | | | |
| | 21.8818 | 83% | 31% | 2 | 2.4 | 0.4 | 14.3 |
| | 3.4965 | 92% | 10% | 3 | 0.0 | 0.0 | na |
| | 99.0385 | 50% | 73% | 4 | 4.3 | 0.8 | 23.1 |
| | 133.772 | 50% | 81% | | | | |
| | 212.963 | 33% | 92% | | | | |
| 48 hours | 29.7203 | 71% | 33% | 1 | | | |
| | 0 | 100% | 0% | 2 | 0.4 | 0.0 | 11.1 |
| | 0 | 100% | 0% | 3 | 0.0 | 0.0 | na |
| | 99.0385 | 57% | 73% | 4 | 2.2 | 0.3 | 13.9 |
| | 133.772 | 57% | 81% | | | | |
| | 212.963 | 43% | 92% | | | | |

sCr only

| Time prior AKI stage | Cutoff value | sens | spec | Quartile | OR | 95% CI of OR | |
|---|---|---|---|---|---|---|---|
| 0 hours | 25.1736 | 71% | 39% | 1 | | | |
| | 4.64989 | 86% | 17% | 2 | 1.6 | 0.3 | 9.7 |
| | 3.4965 | 100% | 14% | 3 | 0.0 | 0.0 | na |
| | 77.2569 | 29% | 70% | 4 | 1.0 | 0.1 | 8.1 |
| | 133.772 | 29% | 80% | | | | |
| | 208.333 | 14% | 90% | | | | |
| 24 hours | 25.1736 | 83% | 39% | 1 | | | |
| | 25.1736 | 83% | 39% | 2 | 3.3 | 0.2 | 53.6 |
| | 3.4965 | 100% | 14% | 3 | 0.0 | 0.0 | na |
| | 77.2569 | 33% | 70% | 4 | 2.1 | 0.1 | 47.1 |
| | 133.772 | 33% | 80% | | | | |
| | 208.333 | 17% | 90% | | | | |
| 48 hours | 0 | 100% | 0% | 1 | | | |
| | 0 | 100% | 0% | 2 | 0.0 | 0.0 | na |
| | 0 | 100% | 0% | 3 | 0.0 | 0.0 | na |
| | 77.2569 | 67% | 70% | 4 | 2.0 | 0.1 | 45.2 |
| | 133.772 | 67% | 80% | | | | |
| | 208.333 | 33% | 90% | | | | |

UO only

| Time prior AKI stage | Cutoff value | sens | spec | Quartile | OR | 95% CI of OR | |
|---|---|---|---|---|---|---|---|
| 0 hours | 177.632 | 71% | 89% | 1 | | | |
| | 133.772 | 86% | 86% | 2 | 0.0 | 0.0 | na |
| | 0 | 100% | 0% | 3 | 0.0 | 0.0 | na |
| | 75.7576 | 86% | 70% | 4 | 12.0 | 0.7 | 212.6 |
| | 113.636 | 86% | 81% | | | | |
| | 231.481 | 57% | 92% | | | | |
| 24 hours | 177.632 | 71% | 89% | 1 | | | |
| | 29.7203 | 86% | 38% | 2 | 1.0 | 0.0 | 74.6 |
| | 0 | 100% | 0% | 3 | 0.0 | 0.0 | na |
| | 75.7576 | 71% | 70% | 4 | 8.3 | 0.5 | 147.7 |
| | 113.636 | 71% | 81% | | | | |
| | 231.481 | 57% | 92% | | | | |
| 48 hours | 29.7203 | 80% | 38% | 1 | | | |
| | 29.7203 | 80% | 38% | 2 | 0.9 | 0.0 | 68.6 |
| | 0 | 100% | 0% | 3 | 0.0 | 0.0 | na |
| | 75.7576 | 60% | 70% | 4 | 3.4 | 0.2 | 73.1 |
| | 113.636 | 60% | 81% | | | | |
| | 231.481 | 60% | 92% | | | | |

# Fig. 4 - 4

ENA-78 (C-X-C chemokine motif 5)

sCr or UO

| | 0 hr prior to AKI stage Cohort 1 | Cohort 2 | 24 hr prior to AKI stage Cohort 1 | Cohort 2 | 48 hr prior to AKI stage Cohort 1 | Cohort 2 |
|---|---|---|---|---|---|---|
| median | 0.020 | 0.044 | 0.020 | 0.042 | 0.020 | 0.035 |
| average | 0.053 | 0.367 | 0.053 | 0.380 | 0.053 | 0.048 |
| stdev | 0.159 | 1.241 | 0.159 | 1.281 | 0.159 | 0.052 |
| p (t-test) | | 0.014 | | 0.013 | | 0.914 |
| min | 0.000 | 0.008 | 0.000 | 0.006 | 0.000 | 0.000 |
| max | 1.460 | 5.180 | 1.460 | 5.180 | 1.460 | 0.184 |
| n (Samp) | 104 | 17 | 104 | 16 | 104 | 10 |
| n (Pat) | 104 | 17 | 104 | 16 | 104 | 10 |

sCr only

| | 0 hr prior to AKI stage Cohort 1 | Cohort 2 | 24 hr prior to AKI stage Cohort 1 | Cohort 2 | 48 hr prior to AKI stage Cohort 1 | Cohort 2 |
|---|---|---|---|---|---|---|
| median | 0.022 | 0.061 | 0.022 | 0.051 | 0.022 | 0.044 |
| average | 0.111 | 0.077 | 0.111 | 0.061 | 0.111 | 0.056 |
| stdev | 0.468 | 0.048 | 0.468 | 0.037 | 0.468 | 0.038 |
| p (t-test) | | 0.840 | | 0.766 | | 0.794 |
| min | 0.000 | 0.026 | 0.000 | 0.022 | 0.000 | 0.022 |
| max | 5.180 | 0.162 | 5.180 | 0.115 | 5.180 | 0.110 |
| n (Samp) | 170 | 8 | 170 | 8 | 170 | 5 |
| n (Pat) | 170 | 8 | 170 | 8 | 170 | 5 |

UO only

| | 0 hr prior to AKI stage Cohort 1 | Cohort 2 | 24 hr prior to AKI stage Cohort 1 | Cohort 2 | 48 hr prior to AKI stage Cohort 1 | Cohort 2 |
|---|---|---|---|---|---|---|
| median | 0.020 | 0.040 | 0.020 | 0.042 | 0.020 | 0.039 |
| average | 0.059 | 0.525 | 0.059 | 0.575 | 0.059 | 0.050 |
| stdev | 0.174 | 1.545 | 0.174 | 1.619 | 0.174 | 0.061 |
| p (t-test) | | 0.007 | | 0.005 | | 0.892 |
| min | 0.000 | 0.008 | 0.000 | 0.006 | 0.000 | 0.000 |
| max | 1.460 | 5.180 | 1.460 | 5.180 | 1.460 | 0.184 |
| n (Samp) | 85 | 11 | 85 | 10 | 85 | 7 |
| n (Pat) | 85 | 11 | 85 | 10 | 85 | 7 |

sCr or UO

| Time prior AKI stage | AUC | SE | $n_{Cohort 1}$ | $n_{Cohort 2}$ | p |
|---|---|---|---|---|---|
| 0 hours | 0.77 | 0.069 | 104 | 17 | 0.000 |
| 24 hours | 0.76 | 0.073 | 104 | 16 | 0.000 |
| 48 hours | 0.65 | 0.098 | 104 | 10 | 0.116 |

sCr only

| Time prior AKI stage | AUC | SE | $n_{Cohort 1}$ | $n_{Cohort 2}$ | p |
|---|---|---|---|---|---|
| 0 hours | 0.80 | 0.095 | 170 | 8 | 0.001 |
| 24 hours | 0.76 | 0.101 | 170 | 8 | 0.011 |
| 48 hours | 0.72 | 0.132 | 170 | 5 | 0.099 |

UO only

| Time prior AKI stage | AUC | SE | $n_{Cohort 1}$ | $n_{Cohort 2}$ | p |
|---|---|---|---|---|---|
| 0 hours | 0.73 | 0.090 | 85 | 11 | 0.009 |
| 24 hours | 0.74 | 0.094 | 85 | 10 | 0.011 |
| 48 hours | 0.63 | 0.117 | 85 | 7 | 0.267 |

sCr or UO

| Time prior AKI stage | Cutoff value | sens | spec | Quartile | OR | 95% CI of OR | |
|---|---|---|---|---|---|---|---|
| 0 hours | 0.0376 | 71% | 79% | 1 | | | |
| | 0.0256 | 82% | 63% | 2 | 1.0 | 0.0 | 57.7 |

Fig. 4 - 5

| | Cutoff value | sens | spec | Quartile | OR | 95% CI of OR | |
|---|---|---|---|---|---|---|---|
| | 0.013 | 94% | 31% | 3 | 5.8 | 0.5 | 70.5 |
| | 0.0299 | 76% | 70% | 4 | 13.8 | 1.4 | 140.1 |
| | 0.0407 | 53% | 81% | | | | |
| | 0.0581 | 41% | 90% | | | | |
| 24 hours | 0.0301 | 75% | 71% | 1 | | | |
| | 0.0238 | 81% | 63% | 2 | 2.1 | 0.1 | 45.0 |
| | 0.013 | 94% | 31% | 3 | 4.5 | 0.3 | 59.6 |
| | 0.0299 | 75% | 70% | 4 | 12.4 | 1.2 | 128.9 |
| | 0.0407 | 50% | 81% | | | | |
| | 0.0581 | 38% | 90% | | | | |
| 48 hours | 0.0238 | 70% | 63% | 1 | | | |
| | 0.0213 | 80% | 54% | 2 | 1.0 | 0.0 | 56.0 |
| | 0.013 | 90% | 31% | 3 | 3.2 | 0.2 | 51.4 |
| | 0.0299 | 60% | 70% | 4 | 5.6 | 0.5 | 69.0 |
| | 0.0407 | 30% | 81% | | | | |
| | 0.0581 | 20% | 90% | | | | |

UO only

| Time prior AKI stage | Cutoff value | sens | spec | Quartile | OR | 95% CI of OR | |
|---|---|---|---|---|---|---|---|
| 0 hours | 0.0299 | 73% | 69% | 1 | | | |
| | 0.0246 | 82% | 62% | 2 | 1.0 | 0.0 | 59.8 |
| | 0.013 | 91% | 32% | 3 | 3.3 | 0.2 | 53.6 |
| | 0.0327 | 64% | 71% | 4 | 7.7 | 0.6 | 91.6 |
| | 0.0389 | 55% | 80% | | | | |
| | 0.0757 | 36% | 91% | | | | |
| 24 hours | 0.0376 | 70% | 79% | 1 | | | |
| | 0.0299 | 80% | 69% | 2 | 1.0 | 0.0 | 57.4 |
| | 0.013 | 90% | 32% | 3 | 2.0 | 0.1 | 45.2 |
| | 0.0327 | 70% | 71% | 4 | 7.3 | 0.6 | 88.0 |
| | 0.0389 | 60% | 80% | | | | |
| | 0.0757 | 40% | 91% | | | | |
| 48 hours | 0.0299 | 71% | 69% | 1 | | | |
| | 0.013 | 86% | 32% | 2 | 1.0 | 0.0 | 60.2 |
| | 0 | 100% | 0% | 3 | 1.0 | 0.0 | 60.2 |
| | 0.0327 | 57% | 71% | 4 | 4.6 | 0.3 | 65.0 |
| | 0.0389 | 43% | 80% | | | | |
| | 0.0757 | 14% | 91% | | | | |

# Fig. 4 - 6

**Endoglin**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.015 | 0.011 | 0.015 | 0.004 | 0.015 | 0.004 |
| average | 0.029 | 0.020 | 0.029 | 0.021 | 0.029 | 0.007 |
| stdev | 0.049 | 0.031 | 0.049 | 0.036 | 0.049 | 0.006 |
| p (t-test) | | 0.603 | | 0.665 | | 0.284 |
| min | 0.004 | 0.004 | 0.004 | 0.004 | 0.004 | 0.004 |
| max | 0.199 | 0.101 | 0.199 | 0.101 | 0.199 | 0.018 |
| n (Samp) | 36 | 9 | 36 | 7 | 36 | 6 |
| n (Pat) | 36 | 9 | 36 | 7 | 36 | 6 |

sCr only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.011 | 0.018 | 0.011 | 0.018 | 0.011 | 0.011 |
| average | 0.026 | 0.032 | 0.026 | 0.041 | 0.026 | 0.011 |
| stdev | 0.045 | 0.039 | 0.045 | 0.052 | 0.045 | 0.010 |
| p (t-test) | | 0.792 | | 0.592 | | 0.636 |
| min | 0.004 | 0.004 | 0.004 | 0.004 | 0.004 | 0.004 |
| max | 0.199 | 0.101 | 0.199 | 0.101 | 0.199 | 0.018 |
| n (Samp) | 60 | 5 | 60 | 3 | 60 | 2 |
| n (Pat) | 60 | 5 | 60 | 3 | 60 | 2 |

UO only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.018 | 0.004 | 0.018 | 0.004 | 0.018 | 0.004 |
| average | 0.036 | 0.005 | 0.036 | 0.005 | 0.036 | 0.005 |
| stdev | 0.055 | 0.004 | 0.055 | 0.004 | 0.055 | 0.004 |
| p (t-test) | | 0.285 | | 0.285 | | 0.285 |
| min | 0.004 | 0.004 | 0.004 | 0.004 | 0.004 | 0.004 |
| max | 0.199 | 0.011 | 0.199 | 0.011 | 0.199 | 0.011 |
| n (Samp) | 26 | 4 | 26 | 4 | 26 | 4 |
| n (Pat) | 26 | 4 | 26 | 4 | 26 | 4 |

sCr or UO

| Time prior AKI stage | AUC | SE | $n_{Cohort 1}$ | $n_{Cohort 2}$ | p |
|---|---|---|---|---|---|
| 0 hours | 0.42 | 0.103 | 36 | 9 | 0.411 |
| 24 hours | 0.35 | 0.106 | 36 | 7 | 0.161 |
| 48 hours | 0.26 | 0.095 | 36 | 6 | 0.011 |

sCr only

| Time prior AKI stage | AUC | SE | $n_{Cohort 1}$ | $n_{Cohort 2}$ | p |
|---|---|---|---|---|---|
| 0 hours | 0.61 | 0.139 | 60 | 5 | 0.416 |
| 24 hours | 0.58 | 0.177 | 60 | 3 | 0.639 |
| 48 hours | 0.41 | 0.193 | 60 | 2 | 0.635 |

UO only

| Time prior AKI stage | AUC | SE | $n_{Cohort 1}$ | $n_{Cohort 2}$ | p |
|---|---|---|---|---|---|
| 0 hours | 0.16 | 0.088 | 26 | 4 | 0.000 |
| 24 hours | 0.16 | 0.088 | 26 | 4 | 0.000 |
| 48 hours | 0.16 | 0.088 | 26 | 4 | 0.000 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 6.3 | 0.3 | 115.2 |
| | 0 | 100% | 0% | 3 | 0.0 | 0.0 | na |
| | 0.01838 | 11% | 78% | 4 | 6.3 | 0.3 | 115.2 |

# Fig. 4 - 7

| | 0.04136 | 11% | 86% | | | | |
|---|---|---|---|---|---|---|---|
| | 0.06434 | 11% | 92% | | | | |
| 24 hours | 0 | 100% | 0% | 1 | | | |
| | 0 | 100% | 0% | 2 | 1.0 | 0.0 | 74.6 |
| | 0 | 100% | 0% | 3 | 1.0 | 0.0 | 74.6 |
| | 0.01838 | 14% | 78% | 4 | 6.7 | 0.3 | 130.3 |
| | 0.04136 | 14% | 86% | | | | |
| | 0.06434 | 14% | 92% | | | | |
| 48 hours | 0 | 100% | 0% | 1 | | | |
| | 0 | 100% | 0% | 2 | na | na | na |
| | 0 | 100% | 0% | 3 | na | na | na |
| | 0.01838 | 0% | 78% | 4 | na | na | na |
| | 0.04136 | 0% | 86% | | | | |
| | 0.06434 | 0% | 92% | | | | |

sCr only

| Time prior AKI stage | Cutoff value | sens | spec | Quartile | OR | 95% CI of OR | |
|---|---|---|---|---|---|---|---|
| 0 hours | 0.01106 | 80% | 55% | 1 | | | |
| | 0.01106 | 80% | 55% | 2 | na | na | na |
| | 0 | 100% | 0% | 3 | na | na | na |
| | 0.01838 | 20% | 77% | 4 | na | na | na |
| | 0.0318 | 20% | 80% | | | | |
| | 0.04563 | 20% | 90% | | | | |
| 24 hours | 0 | 100% | 0% | 1 | | | |
| | 0 | 100% | 0% | 2 | 0.0 | 0.0 | na |
| | 0 | 100% | 0% | 3 | 0.9 | 0.0 | 61.7 |
| | 0.01838 | 33% | 77% | 4 | 0.9 | 0.0 | 61.7 |
| | 0.0318 | 33% | 80% | | | | |
| | 0.04563 | 33% | 90% | | | | |
| 48 hours | 0 | 100% | 0% | 1 | | | |
| | 0 | 100% | 0% | 2 | na | na | na |
| | 0 | 100% | 0% | 3 | na | na | na |
| | 0.01838 | 0% | 77% | 4 | na | na | na |
| | 0.0318 | 0% | 80% | | | | |
| | 0.04563 | 0% | 90% | | | | |

Fig. 4 - 8

**Erythropoietin**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 42.800 | 0.664 | 42.800 | 0.664 | 42.800 | 0.664 |
| average | 155.835 | 80.249 | 155.835 | 78.932 | 155.835 | 72.125 |
| stdev | 277.860 | 132.128 | 277.860 | 137.072 | 277.860 | 141.795 |
| p (t-test) | | 0.274 | | 0.281 | | 0.350 |
| min | 0.664 | 0.664 | 0.664 | 0.664 | 0.664 | 0.664 |
| max | 1350.000 | 397.000 | 1350.000 | 397.000 | 1350.000 | 397.000 |
| n (Samp) | 104 | 17 | 104 | 16 | 104 | 10 |
| n (Pat) | 104 | 17 | 104 | 16 | 104 | 10 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 39.500 | 0.664 | 39.500 | 0.664 | 39.500 | 0.664 |
| average | 212.595 | 81.003 | 212.595 | 78.748 | 212.595 | 79.931 |
| stdev | 765.889 | 150.195 | 765.889 | 151.395 | 765.889 | 177.247 |
| p (t-test) | | 0.629 | | 0.623 | | 0.700 |
| min | 0.664 | 0.664 | 0.664 | 0.664 | 0.664 | 0.664 |
| max | 9340.000 | 397.000 | 9340.000 | 397.000 | 9340.000 | 397.000 |
| n (Samp) | 170 | 8 | 170 | 8 | 170 | 5 |
| n (Pat) | 170 | 8 | 170 | 8 | 170 | 5 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 42.700 | 0.664 | 42.700 | 0.664 | 42.700 | 0.664 |
| average | 193.805 | 65.232 | 193.805 | 63.425 | 193.805 | 46.131 |
| stdev | 447.414 | 114.715 | 447.414 | 120.755 | 447.414 | 100.387 |
| p (t-test) | | 0.347 | | 0.363 | | 0.388 |
| min | 0.664 | 0.664 | 0.664 | 0.664 | 0.664 | 0.664 |
| max | 3430.000 | 310.000 | 3430.000 | 310.000 | 3430.000 | 270.000 |
| n (Samp) | 85 | 11 | 85 | 10 | 85 | 7 |
| n (Pat) | 85 | 11 | 85 | 10 | 85 | 7 |

sCr or UO

| Time prior AKI stage | AUC | SE | $n_{Cohort\ 1}$ | $n_{Cohort\ 2}$ | p |
|---|---|---|---|---|---|
| 0 hours | 0.37 | 0.068 | 104 | 17 | 0.061 |
| 24 hours | 0.34 | 0.067 | 104 | 16 | 0.018 |
| 48 hours | 0.33 | 0.080 | 104 | 10 | 0.030 |

sCr only

| Time prior AKI stage | AUC | SE | $n_{Cohort\ 1}$ | $n_{Cohort\ 2}$ | p |
|---|---|---|---|---|---|
| 0 hours | 0.36 | 0.091 | 170 | 8 | 0.127 |
| 24 hours | 0.33 | 0.086 | 170 | 8 | 0.050 |
| 48 hours | 0.31 | 0.103 | 170 | 5 | 0.059 |

UO only

| Time prior AKI stage | AUC | SE | $n_{Cohort\ 1}$ | $n_{Cohort\ 2}$ | p |
|---|---|---|---|---|---|
| 0 hours | 0.35 | 0.081 | 85 | 11 | 0.071 |
| 24 hours | 0.33 | 0.081 | 85 | 10 | 0.032 |
| 48 hours | 0.31 | 0.091 | 85 | 7 | 0.034 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.5 | 0.1 | 2.4 |

# Fig. 4 - 9

| | Cutoff value | sens | spec | Quartile | OR | 95% CI of OR | |
|---|---|---|---|---|---|---|---|
| | 0 | 100% | 0% | 3 | 1.4 | 0.5 | 3.8 |
| | 125 | 24% | 70% | 4 | 1.7 | 0.6 | 4.5 |
| | 240 | 18% | 81% | | | | |
| | 465 | 0% | 90% | | | | |
| 24 hours | 0 | 100% | 0% | 1 | | | |
| | 0 | 100% | 0% | 2 | 0.2 | 0.0 | 3.0 |
| | 0 | 100% | 0% | 3 | 1.6 | 0.6 | 4.3 |
| | 125 | 25% | 70% | 4 | 1.3 | 0.5 | 3.7 |
| | 240 | 19% | 81% | | | | |
| | 465 | 0% | 90% | | | | |
| 48 hours | 0 | 100% | 0% | 1 | | | |
| | 0 | 100% | 0% | 2 | 0.5 | 0.0 | 10.9 |
| | 0 | 100% | 0% | 3 | 1.0 | 0.1 | 8.2 |
| | 125 | 20% | 70% | 4 | 2.9 | 0.6 | 13.6 |
| | 240 | 20% | 81% | | | | |
| | 465 | 0% | 90% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.0 | 0.0 | na |
| | 0 | 100% | 0% | 3 | 3.3 | 0.8 | 13.4 |
| | 125 | 25% | 70% | 4 | 0.0 | 0.0 | na |
| | 240 | 13% | 80% | | | | |
| | 506 | 0% | 90% | | | | |
| 24 hours | 0 | 100% | 0% | 1 | | | |
| | 0 | 100% | 0% | 2 | 0.0 | 0.0 | na |
| | 0 | 100% | 0% | 3 | 3.3 | 0.8 | 13.4 |
| | 125 | 25% | 70% | 4 | 0.0 | 0.0 | na |
| | 240 | 13% | 80% | | | | |
| | 506 | 0% | 90% | | | | |
| 48 hours | 0 | 100% | 0% | 1 | | | |
| | 0 | 100% | 0% | 2 | 0.0 | 0.0 | na |
| | 0 | 100% | 0% | 3 | 4.3 | 0.3 | 54.8 |
| | 125 | 20% | 70% | 4 | 0.0 | 0.0 | na |
| | 240 | 20% | 80% | | | | |
| | 506 | 0% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.0 | 0.1 | 8.5 |
| | 0 | 100% | 0% | 3 | 0.0 | 0.0 | na |
| | 129 | 18% | 71% | 4 | 4.5 | 1.0 | 19.6 |
| | 240 | 18% | 80% | | | | |
| | 518 | 0% | 91% | | | | |
| 24 hours | 0 | 100% | 0% | 1 | | | |
| | 0 | 100% | 0% | 2 | 0.5 | 0.0 | 10.8 |
| | 0 | 100% | 0% | 3 | 0.0 | 0.0 | na |
| | 129 | 20% | 71% | 4 | 4.8 | 1.1 | 21.0 |
| | 240 | 20% | 80% | | | | |
| | 518 | 0% | 91% | | | | |
| 48 hours | 0 | 100% | 0% | 1 | | | |
| | 0 | 100% | 0% | 2 | 1.0 | 0.0 | 60.2 |
| | 0 | 100% | 0% | 3 | 0.0 | 0.0 | na |
| | 129 | 14% | 71% | 4 | 6.1 | 0.5 | 78.3 |
| | 240 | 14% | 80% | | | | |
| | 518 | 0% | 91% | | | | |

Fig. 4 - 10

**Fractalkine**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 2.658 | 3.290 | 2.658 | 3.113 | 2.658 | 1.496 |
| average | 2.636 | 3.367 | 2.636 | 3.211 | 2.636 | 2.388 |
| stdev | 1.782 | 2.044 | 1.782 | 2.075 | 1.782 | 2.125 |
| p (t-test) | | 0.206 | | 0.335 | | 0.737 |
| min | 0.069 | 0.250 | 0.069 | 0.250 | 0.069 | 0.250 |
| max | 7.811 | 6.610 | 7.811 | 6.610 | 7.811 | 6.610 |
| n (Samp) | 50 | 13 | 50 | 12 | 50 | 7 |
| n (Pat) | 50 | 13 | 50 | 12 | 50 | 7 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 2.409 | 3.313 | 2.409 | 3.531 | 2.409 | 2.681 |
| average | 2.550 | 3.181 | 2.550 | 3.638 | 2.550 | 2.081 |
| stdev | 1.680 | 2.317 | 1.680 | 2.165 | 1.680 | 1.617 |
| p (t-test) | | 0.354 | | 0.134 | | 0.635 |
| min | 0.069 | 0.250 | 0.069 | 0.250 | 0.069 | 0.250 |
| max | 7.811 | 6.429 | 7.811 | 6.429 | 7.811 | 3.313 |
| n (Samp) | 92 | 7 | 92 | 6 | 92 | 3 |
| n (Pat) | 92 | 7 | 92 | 6 | 92 | 3 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 2.556 | 2.937 | 2.556 | 1.496 | 2.556 | 1.351 |
| average | 2.541 | 3.107 | 2.541 | 2.422 | 2.541 | 2.145 |
| stdev | 1.645 | 2.119 | 1.645 | 2.131 | 1.645 | 2.542 |
| p (t-test) | | 0.428 | | 0.867 | | 0.638 |
| min | 0.069 | 0.250 | 0.069 | 0.250 | 0.069 | 0.250 |
| max | 7.811 | 6.610 | 7.811 | 6.610 | 7.811 | 6.610 |
| n (Samp) | 38 | 7 | 38 | 7 | 38 | 5 |
| n (Pat) | 38 | 7 | 38 | 7 | 38 | 5 |

sCr or UO

| Time prior AKI stage | AUC | SE | $n_{Cohort\ 1}$ | $n_{Cohort\ 2}$ | p |
|---|---|---|---|---|---|
| 0 hours | 0.61 | 0.092 | 50 | 13 | 0.220 |
| 24 hours | 0.59 | 0.095 | 50 | 12 | 0.352 |
| 48 hours | 0.44 | 0.113 | 50 | 7 | 0.577 |

sCr only

| Time prior AKI stage | AUC | SE | $n_{Cohort\ 1}$ | $n_{Cohort\ 2}$ | p |
|---|---|---|---|---|---|
| 0 hours | 0.58 | 0.117 | 92 | 7 | 0.482 |
| 24 hours | 0.67 | 0.125 | 92 | 6 | 0.177 |
| 48 hours | 0.43 | 0.162 | 92 | 3 | 0.686 |

UO only

| Time prior AKI stage | AUC | SE | $n_{Cohort\ 1}$ | $n_{Cohort\ 2}$ | p |
|---|---|---|---|---|---|
| 0 hours | 0.58 | 0.122 | 38 | 7 | 0.499 |
| 24 hours | 0.45 | 0.117 | 38 | 7 | 0.675 |
| 48 hours | 0.35 | 0.121 | 38 | 5 | 0.224 |

sCr or UO

| Time prior AKI stage | Cutoff value | sens | spec | Quartile | OR | 95% CI of OR | |
|---|---|---|---|---|---|---|---|
| 0 hours | 2.3374 | 77% | 46% | 1 | | | |
| | 1.1021 | 85% | 26% | 2 | 1.5 | 0.2 | 10.4 |

# Fig. 4 - 11

|  | 0.3125 | 92% | 10% | 3 | 2.2 | 0.4 | 12.9 |
|  | 3.1982 | 54% | 70% | 4 | 2.2 | 0.4 | 12.9 |
|  | 4.07631 | 31% | 80% |  |  |  |  |
|  | 5.08606 | 23% | 90% |  |  |  |  |
| 24 hours | 1.45325 | 75% | 30% | 1 |  |  |  |
|  | 1.1021 | 83% | 26% | 2 | 1.5 | 0.2 | 10.4 |
|  | 0.85401 | 92% | 22% | 3 | 2.4 | 0.4 | 14.3 |
|  | 3.1982 | 50% | 70% | 4 | 1.5 | 0.2 | 10.4 |
|  | 4.07631 | 25% | 80% |  |  |  |  |
|  | 5.08606 | 25% | 90% |  |  |  |  |
| 48 hours | 1.1021 | 71% | 26% | 1 |  |  |  |
|  | 0.85401 | 86% | 22% | 2 | 2.3 | 0.1 | 59.8 |
|  | 0.1868 | 100% | 8% | 3 | 2.3 | 0.1 | 59.8 |
|  | 3.1982 | 29% | 70% | 4 | 2.3 | 0.1 | 59.8 |
|  | 4.07631 | 14% | 80% |  |  |  |  |
|  | 5.08606 | 14% | 90% |  |  |  |  |

sCr only

| Time prior AKI stage | Cutoff value | sens | spec | Quartile | OR | 95% CI of OR | |
|---|---|---|---|---|---|---|---|
| 0 hours | 2.61261 | 71% | 54% | 1 |  |  |  |
|  | 0.3125 | 86% | 7% | 2 | 0.0 | 0.0 | na |
|  | 0.1868 | 100% | 4% | 3 | 1.0 | 0.1 | 8.1 |
|  | 3.23293 | 57% | 71% | 4 | 1.5 | 0.2 | 9.2 |
|  | 4.07631 | 29% | 80% |  |  |  |  |
|  | 5.08606 | 29% | 90% |  |  |  |  |
| 24 hours | 2.61261 | 83% | 54% | 1 |  |  |  |
|  | 2.61261 | 83% | 54% | 2 | 0.0 | 0.0 | na |
|  | 0.1868 | 100% | 4% | 3 | 2.1 | 0.1 | 47.1 |
|  | 3.23293 | 67% | 71% | 4 | 3.1 | 0.2 | 50.9 |
|  | 4.07631 | 33% | 80% |  |  |  |  |
|  | 5.08606 | 33% | 90% |  |  |  |  |
| 48 hours | 0.1868 | 100% | 4% | 1 |  |  |  |
|  | 0.1868 | 100% | 4% | 2 | na | na | na |
|  | 0.1868 | 100% | 4% | 3 | na | na | na |
|  | 3.23293 | 33% | 71% | 4 | na | na | na |
|  | 4.07631 | 0% | 80% |  |  |  |  |
|  | 5.08606 | 0% | 90% |  |  |  |  |

UO only

| Time prior AKI stage | Cutoff value | sens | spec | Quartile | OR | 95% CI of OR | |
|---|---|---|---|---|---|---|---|
| 0 hours | 2.28395 | 71% | 47% | 1 |  |  |  |
|  | 1.1021 | 86% | 26% | 2 | 2.2 | 0.1 | 63.6 |
|  | 0.15069 | 100% | 5% | 3 | 2.2 | 0.1 | 63.6 |
|  | 3.19703 | 43% | 71% | 4 | 2.0 | 0.1 | 56.0 |
|  | 3.94144 | 29% | 82% |  |  |  |  |
|  | 4.79554 | 29% | 92% |  |  |  |  |
| 24 hours | 1.1021 | 71% | 26% | 1 |  |  |  |
|  | 0.85401 | 86% | 18% | 2 | 0.5 | 0.0 | 14.0 |
|  | 0.15069 | 100% | 5% | 3 | 1.1 | 0.1 | 11.9 |
|  | 3.19703 | 29% | 71% | 4 | 1.1 | 0.1 | 11.9 |
|  | 3.94144 | 14% | 82% |  |  |  |  |
|  | 4.79554 | 14% | 92% |  |  |  |  |
| 48 hours | 0.85401 | 80% | 18% | 1 |  |  |  |
|  | 0.85401 | 80% | 18% | 2 | 0.0 | 0.0 | na |
|  | 0.15069 | 100% | 5% | 3 | 2.2 | 0.1 | 63.6 |
|  | 3.19703 | 20% | 71% | 4 | 2.5 | 0.1 | 73.5 |
|  | 3.94144 | 20% | 82% |  |  |  |  |
|  | 4.79554 | 20% | 92% |  |  |  |  |

## Fig. 4 - 12

**Interleukin-1 receptor, type II**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 966.732 | 1608.380 | 966.732 | 1608.380 | 966.732 | 583.915 |
| average | 1872.350 | 8066.896 | 1872.350 | 8063.895 | 1872.350 | 1600.724 |
| stdev | 3537.325 | 15470.860 | 3537.325 | 15472.536 | 3537.325 | 1929.798 |
| p (t-test) | | 0.002 | | 0.002 | | 0.832 |
| min | 99.487 | 42.493 | 99.487 | 42.493 | 99.487 | 42.493 |
| max | 29514.673 | 46544.423 | 29514.673 | 46544.423 | 29514.673 | 5017.737 |
| n (Samp) | 82 | 12 | 82 | 12 | 82 | 8 |
| n (Pat) | 82 | 12 | 82 | 12 | 82 | 8 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 1336.664 | 583.915 | 1336.664 | 583.915 | 1336.664 | 235.059 |
| average | 2409.661 | 8441.485 | 2409.661 | 8441.485 | 2409.661 | 334.073 |
| stdev | 4263.706 | 18692.892 | 4263.706 | 18692.892 | 4263.706 | 350.306 |
| p (t-test) | | 0.009 | | 0.009 | | 0.334 |
| min | 58.099 | 42.493 | 58.099 | 42.493 | 58.099 | 42.493 |
| max | 34543.843 | 46544.423 | 34543.843 | 46544.423 | 34543.843 | 823.680 |
| n (Samp) | 136 | 6 | 136 | 6 | 136 | 4 |
| n (Pat) | 136 | 6 | 136 | 6 | 136 | 4 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 966.732 | 1608.380 | 966.732 | 1608.380 | 966.732 | 1608.380 |
| average | 1572.454 | 5892.439 | 1572.454 | 5887.937 | 1572.454 | 2069.859 |
| stdev | 1832.663 | 11722.903 | 1832.663 | 11725.425 | 1832.663 | 2036.725 |
| p (t-test) | | 0.005 | | 0.005 | | 0.529 |
| min | 83.202 | 58.099 | 83.202 | 58.099 | 83.202 | 43.565 |
| max | 11320.965 | 34543.843 | 11320.965 | 34543.843 | 11320.965 | 5017.737 |
| n (Samp) | 68 | 8 | 68 | 8 | 68 | 6 |
| n (Pat) | 68 | 8 | 68 | 8 | 68 | 6 |

sCr or UO

| Time prior AKI stage | AUC | SE | $n_{Cohort\,1}$ | $n_{Cohort\,2}$ | p |
|---|---|---|---|---|---|
| 0 hours | 0.53 | 0.091 | 82 | 12 | 0.771 |
| 24 hours | 0.52 | 0.091 | 82 | 12 | 0.796 |
| 48 hours | 0.43 | 0.102 | 82 | 8 | 0.462 |

sCr only

| Time prior AKI stage | AUC | SE | $n_{Cohort\,1}$ | $n_{Cohort\,2}$ | p |
|---|---|---|---|---|---|
| 0 hours | 0.39 | 0.109 | 136 | 6 | 0.308 |
| 24 hours | 0.39 | 0.109 | 136 | 6 | 0.308 |
| 48 hours | 0.13 | 0.062 | 136 | 4 | 0.000 |

UO only

| Time prior AKI stage | AUC | SE | $n_{Cohort\,1}$ | $n_{Cohort\,2}$ | p |
|---|---|---|---|---|---|
| 0 hours | 0.55 | 0.111 | 68 | 8 | 0.678 |
| 24 hours | 0.54 | 0.110 | 68 | 8 | 0.702 |
| 48 hours | 0.55 | 0.126 | 68 | 6 | 0.698 |

sCr or UO

| Time prior AKI stage | Cutoff value | sens | spec | Quartile | OR | 95% CI of OR | |
|---|---|---|---|---|---|---|---|
| 0 hours | 288.07 | 75% | 12% | 1 | | | |
| | 153.888 | 83% | 5% | 2 | 0.2 | 0.0 | 2.0 |

Fig. 4 - 13

| Time prior AKI stage | Cutoff value | sens | spec | Quartile | OR | 95% CI of OR | |
|---|---|---|---|---|---|---|---|
| | 42.4927 | 92% | 0% | 3 | 0.0 | 0.0 | na |
| | 1727.11 | 50% | 71% | 4 | 1.2 | 0.5 | 3.1 |
| | 2047.76 | 50% | 80% | | | | |
| | 3945.89 | 25% | 90% | | | | |
| 24 hours | 288.07 | 75% | 12% | 1 | | | |
| | 99.487 | 83% | 1% | 2 | 0.2 | 0.0 | 2.0 |
| | 42.4927 | 92% | 0% | 3 | 0.0 | 0.0 | na |
| | 1727.11 | 50% | 71% | 4 | 1.2 | 0.5 | 3.1 |
| | 2047.76 | 50% | 80% | | | | |
| | 3945.89 | 25% | 90% | | | | |
| 48 hours | 288.07 | 75% | 12% | 1 | | | |
| | 42.4927 | 88% | 0% | 2 | 0.0 | 0.0 | na |
| | 0 | 100% | 0% | 3 | 0.3 | 0.0 | 5.0 |
| | 1727.11 | 38% | 71% | 4 | 1.5 | 0.4 | 5.7 |
| | 2047.76 | 38% | 80% | | | | |
| | 3945.89 | 13% | 90% | | | | |

sCr only

| Time prior AKI stage | Cutoff value | sens | spec | Quartile | OR | 95% CI of OR | |
|---|---|---|---|---|---|---|---|
| 0 hours | 153.888 | 83% | 4% | 1 | | | |
| | 153.888 | 83% | 4% | 2 | 0.0 | 0.0 | na |
| | 0 | 100% | 0% | 3 | 0.5 | 0.0 | 10.3 |
| | 2101.42 | 33% | 71% | 4 | 1.6 | 0.3 | 9.2 |
| | 3055.75 | 17% | 80% | | | | |
| | 4718.2 | 17% | 90% | | | | |
| 24 hours | 153.888 | 83% | 4% | 1 | | | |
| | 153.888 | 83% | 4% | 2 | 0.0 | 0.0 | na |
| | 0 | 100% | 0% | 3 | 0.5 | 0.0 | 10.3 |
| | 2101.42 | 33% | 71% | 4 | 1.6 | 0.3 | 9.2 |
| | 3055.75 | 17% | 80% | | | | |
| | 4718.2 | 17% | 90% | | | | |
| 48 hours | 99.487 | 75% | 1% | 1 | | | |
| | 0 | 100% | 0% | 2 | na | na | na |
| | 0 | 100% | 0% | 3 | na | na | na |
| | 2101.42 | 0% | 71% | 4 | na | na | na |
| | 3055.75 | 0% | 80% | | | | |
| | 4718.2 | 0% | 90% | | | | |

UO only

| Time prior AKI stage | Cutoff value | sens | spec | Quartile | OR | 95% CI of OR | |
|---|---|---|---|---|---|---|---|
| 0 hours | 288.07 | 75% | 15% | 1 | | | |
| | 153.888 | 88% | 7% | 2 | 0.3 | 0.0 | 5.1 |
| | 0 | 100% | 0% | 3 | 0.0 | 0.0 | na |
| | 1727.11 | 50% | 71% | 4 | 1.4 | 0.4 | 5.7 |
| | 2047.76 | 50% | 81% | | | | |
| | 3945.89 | 25% | 91% | | | | |
| 24 hours | 288.07 | 75% | 15% | 1 | | | |
| | 125.843 | 88% | 4% | 2 | 0.3 | 0.0 | 5.1 |
| | 0 | 100% | 0% | 3 | 0.0 | 0.0 | na |
| | 1727.11 | 50% | 71% | 4 | 1.4 | 0.4 | 5.7 |
| | 2047.76 | 50% | 81% | | | | |
| | 3945.89 | 25% | 91% | | | | |
| 48 hours | 288.07 | 83% | 15% | 1 | | | |
| | 288.07 | 83% | 15% | 2 | 0.4 | 0.0 | 10.6 |
| | 0 | 100% | 0% | 3 | 0.0 | 0.0 | na |
| | 1727.11 | 50% | 71% | 4 | 1.5 | 0.2 | 9.8 |
| | 2047.76 | 50% | 81% | | | | |
| | 3945.89 | 17% | 91% | | | | |

Fig. 4 - 14

**Lymphotoxin-alpha**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 2.595 | 4.180 | 2.595 | 4.265 | 2.595 | 3.455 |
| average | 2.750 | 5.421 | 2.750 | 5.036 | 2.750 | 4.876 |
| stdev | 2.126 | 4.121 | 2.126 | 3.070 | 2.126 | 3.755 |
| p (t-test) | | 0.000 | | 0.000 | | 0.006 |
| min | 0.182 | 0.182 | 0.182 | 0.883 | 0.182 | 0.182 |
| max | 13.300 | 15.500 | 13.300 | 13.100 | 13.300 | 13.100 |
| n (Samp) | 104 | 17 | 104 | 16 | 104 | 10 |
| n (Pat) | 104 | 17 | 104 | 16 | 104 | 10 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 2.820 | 4.510 | 2.820 | 4.510 | 2.820 | 3.730 |
| average | 3.029 | 5.849 | 3.029 | 4.584 | 3.029 | 4.106 |
| stdev | 2.289 | 4.082 | 2.289 | 1.249 | 2.289 | 1.467 |
| p (t-test) | | 0.001 | | 0.059 | | 0.298 |
| min | 0.182 | 2.810 | 0.182 | 2.810 | 0.182 | 2.810 |
| max | 13.300 | 15.500 | 13.300 | 6.510 | 13.300 | 6.510 |
| n (Samp) | 170 | 8 | 170 | 8 | 170 | 5 |
| n (Pat) | 170 | 8 | 170 | 8 | 170 | 5 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 2.740 | 3.410 | 2.740 | 3.455 | 2.740 | 3.410 |
| average | 2.802 | 4.922 | 2.802 | 5.267 | 2.802 | 5.102 |
| stdev | 2.093 | 3.952 | 2.093 | 3.834 | 2.093 | 4.440 |
| p (t-test) | | 0.006 | | 0.002 | | 0.014 |
| min | 0.182 | 0.182 | 0.182 | 0.883 | 0.182 | 0.182 |
| max | 13.300 | 13.100 | 13.300 | 13.100 | 13.300 | 13.100 |
| n (Samp) | 85 | 11 | 85 | 10 | 85 | 7 |
| n (Pat) | 85 | 11 | 85 | 10 | 85 | 7 |

sCr or UO

| Time prior AKI stage | AUC | SE | $n_{Cohort 1}$ | $n_{Cohort 2}$ | p |
|---|---|---|---|---|---|
| 0 hours | 0.73 | 0.073 | 104 | 17 | 0.002 |
| 24 hours | 0.75 | 0.073 | 104 | 16 | 0.001 |
| 48 hours | 0.70 | 0.096 | 104 | 10 | 0.042 |

sCr only

| Time prior AKI stage | AUC | SE | $n_{Cohort 1}$ | $n_{Cohort 2}$ | p |
|---|---|---|---|---|---|
| 0 hours | 0.78 | 0.099 | 170 | 8 | 0.005 |
| 24 hours | 0.76 | 0.100 | 170 | 8 | 0.009 |
| 48 hours | 0.68 | 0.134 | 170 | 5 | 0.172 |

UO only

| Time prior AKI stage | AUC | SE | $n_{Cohort 1}$ | $n_{Cohort 2}$ | p |
|---|---|---|---|---|---|
| 0 hours | 0.66 | 0.094 | 85 | 11 | 0.092 |
| 24 hours | 0.70 | 0.096 | 85 | 10 | 0.034 |
| 48 hours | 0.66 | 0.116 | 85 | 7 | 0.170 |

sCr or UO

| Time prior AKI stage | Cutoff value | sens | spec | Quartile | OR | 95% CI of OR | |
|---|---|---|---|---|---|---|---|
| 0 hours | 3.13 | 71% | 61% | 1 | | | |
| | 2.83 | 82% | 58% | 2 | 0.0 | 0.0 | na |

# Fig. 4 - 15

| Time prior AKI stage | Cutoff value | sens | spec | Quartile | OR | 95% CI of OR | |
|---|---|---|---|---|---|---|---|
| | 0.1824 | 94% | 18% | 3 | 3.5 | 0.8 | 15.0 |
| | 3.41 | 59% | 70% | 4 | 5.7 | 1.5 | 22.2 |
| | 4.35 | 47% | 81% | | | | |
| | 5.06 | 35% | 90% | | | | |
| 24 hours | 3.13 | 75% | 61% | 1 | | | |
| | 2.96 | 81% | 60% | 2 | 0.0 | 0.0 | na |
| | 2.76 | 94% | 56% | 3 | 8.8 | 0.8 | 96.6 |
| | 3.41 | 63% | 70% | 4 | 10.5 | 1.0 | 111.9 |
| | 4.35 | 44% | 81% | | | | |
| | 5.06 | 31% | 90% | | | | |
| 48 hours | 3.13 | 70% | 61% | 1 | | | |
| | 2.96 | 80% | 60% | 2 | 0.0 | 0.0 | na |
| | 2.76 | 90% | 56% | 3 | 7.4 | 0.6 | 85.2 |
| | 3.41 | 50% | 70% | 4 | 3.1 | 0.2 | 49.3 |
| | 4.35 | 30% | 81% | | | | |
| | 5.06 | 30% | 90% | | | | |

UO only

| Time prior AKI stage | Cutoff value | sens | spec | Quartile | OR | 95% CI of OR | |
|---|---|---|---|---|---|---|---|
| 0 hours | 3 | 73% | 58% | 1 | | | |
| | 2.83 | 82% | 53% | 2 | 0.5 | 0.0 | 10.8 |
| | 0.1824 | 91% | 18% | 3 | 2.2 | 0.4 | 11.5 |
| | 3.52 | 36% | 72% | 4 | 2.2 | 0.4 | 11.5 |
| | 4.28 | 36% | 80% | | | | |
| | 4.95 | 36% | 91% | | | | |
| 24 hours | 3.13 | 70% | 58% | 1 | | | |
| | 3 | 80% | 58% | 2 | 0.0 | 0.0 | na |
| | 2.83 | 90% | 53% | 3 | 5.8 | 0.5 | 73.7 |
| | 3.52 | 40% | 72% | 4 | 4.4 | 0.3 | 61.5 |
| | 4.28 | 40% | 80% | | | | |
| | 4.95 | 30% | 91% | | | | |
| 48 hours | 3.13 | 71% | 58% | 1 | | | |
| | 3 | 86% | 58% | 2 | 0.0 | 0.0 | na |
| | 0 | 100% | 0% | 3 | 4.6 | 0.3 | 65.0 |
| | 3.52 | 29% | 72% | 4 | 2.1 | 0.1 | 47.6 |
| | 4.28 | 29% | 80% | | | | |
| | 4.95 | 29% | 91% | | | | |

# Fig. 4 - 16

**Matrix metalloproteinase-3**

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 0.042 | 0.131 | 0.042 | 0.092 | 0.042 | 0.065 |
| average | 0.311 | 3.352 | 0.311 | 3.541 | 0.311 | 0.614 |
| stdev | 1.658 | 11.797 | 1.658 | 12.158 | 1.658 | 1.139 |
| p (t-test) | | 0.013 | | 0.010 | | 0.573 |
| min | 0.001 | 0.028 | 0.001 | 0.022 | 0.001 | 0.007 |
| max | 16.700 | 49.000 | 16.700 | 49.000 | 16.700 | 2.920 |
| n (Samp) | 104 | 17 | 104 | 16 | 104 | 10 |
| n (Pat) | 104 | 17 | 104 | 16 | 104 | 10 |

sCr only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 0.046 | 0.174 | 0.046 | 0.158 | 0.046 | 0.049 |
| average | 0.557 | 0.575 | 0.557 | 0.556 | 0.557 | 0.600 |
| stdev | 3.978 | 0.895 | 3.978 | 0.906 | 3.978 | 1.129 |
| p (t-test) | | 0.990 | | 0.999 | | 0.981 |
| min | 0.001 | 0.030 | 0.001 | 0.030 | 0.001 | 0.030 |
| max | 49.000 | 2.610 | 49.000 | 2.610 | 49.000 | 2.610 |
| n (Samp) | 170 | 8 | 170 | 8 | 170 | 5 |
| n (Pat) | 170 | 8 | 170 | 8 | 170 | 5 |

UO only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 0.041 | 0.104 | 0.041 | 0.092 | 0.041 | 0.080 |
| average | 0.334 | 4.803 | 0.334 | 5.265 | 0.334 | 0.493 |
| stdev | 1.826 | 14.683 | 1.826 | 15.393 | 1.826 | 1.074 |
| p (t-test) | | 0.007 | | 0.005 | | 0.821 |
| min | 0.001 | 0.028 | 0.001 | 0.022 | 0.001 | 0.007 |
| max | 16.700 | 49.000 | 16.700 | 49.000 | 16.700 | 2.920 |
| n (Samp) | 85 | 11 | 85 | 10 | 85 | 7 |
| n (Pat) | 85 | 11 | 85 | 10 | 85 | 7 |

sCr or UO

| Time prior AKI stage | AUC | SE | $n_{Cohort 1}$ | $n_{Cohort 2}$ | p |
| --- | --- | --- | --- | --- | --- |
| 0 hours | 0.71 | 0.074 | 104 | 17 | 0.005 |
| 24 hours | 0.66 | 0.078 | 104 | 16 | 0.036 |
| 48 hours | 0.60 | 0.099 | 104 | 10 | 0.304 |

sCr only

| Time prior AKI stage | AUC | SE | $n_{Cohort 1}$ | $n_{Cohort 2}$ | p |
| --- | --- | --- | --- | --- | --- |
| 0 hours | 0.76 | 0.101 | 170 | 8 | 0.011 |
| 24 hours | 0.70 | 0.106 | 170 | 8 | 0.059 |
| 48 hours | 0.59 | 0.136 | 170 | 5 | 0.508 |

UO only

| Time prior AKI stage | AUC | SE | $n_{Cohort 1}$ | $n_{Cohort 2}$ | p |
| --- | --- | --- | --- | --- | --- |
| 0 hours | 0.71 | 0.092 | 85 | 11 | 0.025 |
| 24 hours | 0.67 | 0.098 | 85 | 10 | 0.083 |
| 48 hours | 0.60 | 0.117 | 85 | 7 | 0.383 |

sCr or UO

| Time prior AKI stage | Cutoff value | sens | spec | Quartile | OR | 95% CI of OR | |
| --- | --- | --- | --- | --- | --- | --- | --- |
| 0 hours | 0.0489 | 71% | 57% | 1 | | | |
| | 0.0391 | 82% | 45% | 2 | na | na | na |

Fig. 4 - 17

| | Cutoff value | sens | spec | Quartile | OR | 95% CI of OR | |
|---|---|---|---|---|---|---|---|
| | 0.0296 | 94% | 32% | 3 | na | na | na |
| | 0.0784 | 65% | 70% | 4 | na | na | na |
| | 0.18 | 35% | 81% | | | | |
| | 0.406 | 24% | 90% | | | | |
| 24 hours | 0.0387 | 75% | 44% | 1 | | | |
| | 0.0341 | 81% | 43% | 2 | 5.8 | 0.5 | 70.5 |
| | 0.0296 | 94% | 32% | 3 | 4.5 | 0.3 | 59.6 |
| | 0.0784 | 56% | 70% | 4 | 7.3 | 0.6 | 82.8 |
| | 0.18 | 31% | 81% | | | | |
| | 0.406 | 25% | 90% | | | | |
| 48 hours | 0.0391 | 70% | 45% | 1 | | | |
| | 0.0296 | 80% | 32% | 2 | 3.1 | 0.2 | 49.3 |
| | 0.0214 | 90% | 27% | 3 | 3.2 | 0.2 | 51.4 |
| | 0.0784 | 50% | 70% | 4 | 3.1 | 0.2 | 49.3 |
| | 0.18 | 30% | 81% | | | | |
| | 0.406 | 20% | 90% | | | | |

UO only

| Time prior AKI stage | Cutoff value | sens | spec | Quartile | OR | 95% CI of OR | |
|---|---|---|---|---|---|---|---|
| 0 hours | 0.0466 | 73% | 59% | 1 | | | |
| | 0.0391 | 82% | 46% | 2 | na | na | na |
| | 0.0387 | 91% | 45% | 3 | na | na | na |
| | 0.0703 | 64% | 71% | 4 | na | na | na |
| | 0.131 | 36% | 80% | | | | |
| | 0.391 | 18% | 91% | | | | |
| 24 hours | 0.0391 | 70% | 46% | 1 | | | |
| | 0.0387 | 80% | 45% | 2 | na | na | na |
| | 0.0303 | 90% | 35% | 3 | na | na | na |
| | 0.0703 | 60% | 71% | 4 | na | na | na |
| | 0.131 | 30% | 80% | | | | |
| | 0.391 | 20% | 91% | | | | |
| 48 hours | 0.0391 | 71% | 46% | 1 | | | |
| | 0.0214 | 86% | 28% | 2 | 2.1 | 0.1 | 47.6 |
| | 0.0008 | 100% | 12% | 3 | 1.0 | 0.0 | 60.2 |
| | 0.0703 | 57% | 71% | 4 | 3.3 | 0.2 | 54.3 |
| | 0.131 | 29% | 80% | | | | |
| | 0.391 | 14% | 91% | | | | |

# Fig. 4 - 18

**MDC (C-C motif chemokine 22)**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.056 | 5.750 | 0.056 | 2.660 | 0.056 | 0.056 |
| average | 1.799 | 9.356 | 1.799 | 9.106 | 1.799 | 1.957 |
| stdev | 4.717 | 21.420 | 4.717 | 22.181 | 4.717 | 2.632 |
| p (t-test) | | 0.002 | | 0.003 | | 0.917 |
| min | 0.056 | 0.056 | 0.056 | 0.056 | 0.056 | 0.056 |
| max | 41.000 | 90.700 | 41.000 | 90.700 | 41.000 | 6.380 |
| n (Samp) | 104 | 17 | 104 | 16 | 104 | 10 |
| n (Pat) | 104 | 17 | 104 | 16 | 104 | 10 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.056 | 6.030 | 0.056 | 5.100 | 0.056 | 2.660 |
| average | 2.393 | 6.683 | 2.393 | 5.446 | 2.393 | 3.115 |
| stdev | 8.207 | 4.439 | 8.207 | 4.855 | 8.207 | 2.151 |
| p (t-test) | | 0.144 | | 0.299 | | 0.845 |
| min | 0.056 | 0.056 | 0.056 | 0.056 | 0.056 | 0.056 |
| max | 90.700 | 15.900 | 90.700 | 15.900 | 90.700 | 5.750 |
| n (Samp) | 170 | 8 | 170 | 8 | 170 | 5 |
| n (Pat) | 170 | 8 | 170 | 8 | 170 | 5 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.056 | 0.056 | 0.056 | 1.358 | 0.056 | 0.056 |
| average | 2.116 | 10.553 | 2.116 | 11.264 | 2.116 | 1.587 |
| stdev | 5.201 | 26.768 | 5.201 | 28.072 | 5.201 | 2.674 |
| p (t-test) | | 0.010 | | 0.008 | | 0.791 |
| min | 0.056 | 0.056 | 0.056 | 0.056 | 0.056 | 0.056 |
| max | 41.000 | 90.700 | 41.000 | 90.700 | 41.000 | 6.380 |
| n (Samp) | 85 | · 11 | 85 | 10 | 85 | 7 |
| n (Pat) | 85 | 11 | 85 | 10 | 85 | 7 |

sCr or UO

| Time prior AKI stage | AUC | SE | n$_{Cohort 1}$ | n$_{Cohort 2}$ | p |
|---|---|---|---|---|---|
| 0 hours | 0.69 | 0.075 | 104 | 17 | 0.010 |
| 24 hours | 0.68 | 0.078 | 104 | 16 | 0.018 |
| 48 hours | 0.55 | 0.098 | 104 | 10 | 0.596 |

sCr only

| Time prior AKI stage | AUC | SE | n$_{Cohort 1}$ | n$_{Cohort 2}$ | p |
|---|---|---|---|---|---|
| 0 hours | 0.86 | 0.085 | 170 | 8 | 0.000 |
| 24 hours | 0.81 | 0.094 | 170 | 8 | 0.001 |
| 48 hours | 0.75 | 0.128 | 170 | 5 | 0.055 |

UO only

| Time prior AKI stage | AUC | SE | n$_{Cohort 1}$ | n$_{Cohort 2}$ | p |
|---|---|---|---|---|---|
| 0 hours | 0.59 | 0.095 | 85 | 11 | 0.354 |
| 24 hours | 0.60 | 0.100 | 85 | 10 | 0.304 |
| 48 hours | 0.47 | 0.112 | 85 | 7 | 0.793 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |

## Fig. 4 - 19

| Time prior AKI stage | Cutoff value | sens | spec | Quartile | OR | 95% CI of OR | |
|---|---|---|---|---|---|---|---|
| | 0 | 100% | 0% | 2 | 7.3 | 0.6 | 82.8 |
| | 0 | 100% | 0% | 3 | 0.0 | 0.0 | na |
| | 1.66 | 59% | 71% | 4 | 13.8 | 1.4 | 140.1 |
| | 2.51 | 59% | 81% | | | | |
| | 4.6 | 53% | 90% | | | | |
| 24 hours | 0 | 100% | 0% | 1 | | | |
| | 0 | 100% | 0% | 2 | na | na | na |
| | 0 | 100% | 0% | 3 | na | na | na |
| | 1.66 | 56% | 71% | 4 | na | na | na |
| | 2.51 | 56% | 81% | | | | |
| | 4.6 | 38% | 90% | | | | |
| 48 hours | 0 | 100% | 0% | 1 | | | |
| | 0 | 100% | 0% | 2 | na | na | na |
| | 0 | 100% | 0% | 3 | na | na | na |
| | 1.66 | 40% | 71% | 4 | na | na | na |
| | 2.51 | 40% | 81% | | | | |
| | 4.6 | 20% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 4.6 | 0.3 | 64.0 |
| | 0 | 100% | 0% | 3 | 1.0 | 0.0 | 59.8 |
| | 2.16 | 45% | 71% | 4 | 6.1 | 0.5 | 76.8 |
| | 2.9 | 45% | 80% | | | | |
| | 4.84 | 36% | 91% | | | | |
| 24 hours | 0 | 100% | 0% | 1 | | | |
| | 0 | 100% | 0% | 2 | na | na | na |
| | 0 | 100% | 0% | 3 | na | na | na |
| | 2.16 | 50% | 71% | 4 | na | na | na |
| | 2.9 | 40% | 80% | | | | |
| | 4.84 | 30% | 91% | | | | |
| 48 hours | 0 | 100% | 0% | 1 | | | |
| | 0 | 100% | 0% | 2 | 0.0 | 0.0 | na |
| | 0 | 100% | 0% | 3 | 2.2 | 0.4 | 11.7 |
| | 2.16 | 29% | 71% | 4 | 0.5 | 0.0 | 10.8 |
| | 2.9 | 29% | 80% | | | | |
| | 4.84 | 14% | 91% | | | | |

# Fig. 4 - 20

**Thrombospondin-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 8.700 | 14.807 | 8.700 | 12.521 | 8.700 | 12.521 |
| average | 27.492 | 21.283 | 27.492 | 17.185 | 27.492 | 16.926 |
| stdev | 74.849 | 19.308 | 74.849 | 16.282 | 74.849 | 16.551 |
| p (t-test) | | 0.818 | | 0.741 | | 0.735 |
| min | 0.828 | 2.685 | 0.828 | 3.590 | 0.828 | 2.070 |
| max | 435.086 | 48.353 | 435.086 | 45.363 | 435.086 | 45.363 |
| n (Samp) | 36 | 8 | 36 | 6 | 36 | 6 |
| n (Pat) | 36 | 8 | 36 | 6 | 36 | 6 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 7.735 | 6.455 | 7.735 | 26.437 | 7.735 | 26.437 |
| average | 19.862 | 15.239 | 19.862 | 26.437 | 19.862 | 26.437 |
| stdev | 58.569 | 20.179 | 58.569 | 26.765 | 58.569 | 26.765 |
| p (t-test) | | 0.876 | | 0.876 | | 0.876 |
| min | 0.828 | 2.685 | 0.828 | 7.511 | 0.828 | 7.511 |
| max | 435.086 | 45.363 | 435.086 | 45.363 | 435.086 | 45.363 |
| n (Samp) | 60 | 4 | 60 | 2 | 60 | 2 |
| n (Pat) | 60 | 4 | 60 | 2 | 60 | 2 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 7.780 | 28.683 | 7.780 | 10.576 | 7.780 | 10.560 |
| average | 10.793 | 27.327 | 10.793 | 12.559 | 10.793 | 12.171 |
| stdev | 18.236 | 19.112 | 18.236 | 10.837 | 18.236 | 11.283 |
| p (t-test) | | 0.104 | | 0.853 | | 0.885 |
| min | 0.828 | 3.590 | 0.828 | 3.590 | 0.828 | 2.070 |
| max | 93.490 | 48.353 | 93.490 | 25.493 | 93.490 | 25.493 |
| n (Samp) | 26 | 4 | 26 | 4 | 26 | 4 |
| n (Pat) | 26 | 4 | 26 | 4 | 26 | 4 |

sCr or UO

| Time prior AKI stage | AUC | SE | n$_{Cohort 1}$ | n$_{Cohort 2}$ | p |
|---|---|---|---|---|---|
| 0 hours | 0.61 | 0.116 | 36 | 8 | 0.353 |
| 24 hours | 0.59 | 0.131 | 36 | 6 | 0.470 |
| 48 hours | 0.56 | 0.131 | 36 | 6 | 0.634 |

sCr only

| Time prior AKI stage | AUC | SE | n$_{Cohort 1}$ | n$_{Cohort 2}$ | p |
|---|---|---|---|---|---|
| 0 hours | 0.49 | 0.150 | 60 | 4 | 0.967 |
| 24 hours | 0.71 | 0.210 | 60 | 2 | 0.322 |
| 48 hours | 0.71 | 0.210 | 60 | 2 | 0.322 |

UO only

| Time prior AKI stage | AUC | SE | n$_{Cohort 1}$ | n$_{Cohort 2}$ | p |
|---|---|---|---|---|---|
| 0 hours | 0.81 | 0.136 | 26 | 4 | 0.022 |
| 24 hours | 0.66 | 0.159 | 26 | 4 | 0.318 |
| 48 hours | 0.59 | 0.161 | 26 | 4 | 0.571 |

sCr or UO

| Time prior AKI stage | Cutoff value | sens | spec | Quartile | OR | 95% CI of OR | |
|---|---|---|---|---|---|---|---|
| 0 hours | 4.55298 | 75% | 39% | 1 | | | |
| | 3.28789 | 88% | 31% | 2 | 3.8 | 0.2 | 79.5 |

Fig. 4 - 21

| | 2.08132 | 100% | 17% | 3 | 0.0 | 0.0 | na |
|---|---|---|---|---|---|---|---|
| | 11.7188 | 50% | 72% | 4 | 5.7 | 0.3 | 106.6 |
| | 21.2171 | 50% | 81% | | | | |
| | 46.2416 | 13% | 92% | | | | |
| 24 hours | 3.58953 | 83% | 33% | 1 | | | |
| | 3.58953 | 83% | 33% | 2 | na | na | na |
| | 3.28789 | 100% | 31% | 3 | na | na | na |
| | 11.7188 | 50% | 72% | 4 | na | na | na |
| | 21.2171 | 33% | 81% | | | | |
| | 46.2416 | 0% | 92% | | | | |
| 48 hours | 3.28789 | 83% | 31% | 1 | | | |
| | 3.28789 | 83% | 31% | 2 | 2.0 | 0.1 | 58.5 |
| | 1.47804 | 100% | 14% | 3 | 0.0 | 0.0 | na |
| | 11.7188 | 50% | 72% | 4 | 3.4 | 0.2 | 73.1 |
| | 21.2171 | 33% | 81% | | | | |
| | 46.2416 | 0% | 92% | | | | |

sCr only

| Time prior AKI stage | Cutoff value | sens | spec | Quartile | OR | 95% CI of OR | |
|---|---|---|---|---|---|---|---|
| 0 hours | 4.55298 | 75% | 38% | 1 | | | |
| | 2.08132 | 100% | 13% | 2 | 0.0 | 0.0 | na |
| | 2.08132 | 100% | 13% | 3 | 2.1 | 0.1 | 53.1 |
| | 11.0085 | 25% | 70% | 4 | 1.0 | 0.0 | 65.5 |
| | 16.0061 | 25% | 80% | | | | |
| | 25.8224 | 25% | 90% | | | | |
| 24 hours | 7.03642 | 100% | 48% | 1 | | | |
| | 7.03642 | 100% | 48% | 2 | na | na | na |
| | 7.03642 | 100% | 48% | 3 | na | na | na |
| | 11.0085 | 50% | 70% | 4 | na | na | na |
| | 16.0061 | 50% | 80% | | | | |
| | 25.8224 | 50% | 90% | | | | |
| 48 hours | 7.03642 | 100% | 48% | 1 | | | |
| | 7.03642 | 100% | 48% | 2 | na | na | na |
| | 7.03642 | 100% | 48% | 3 | na | na | na |
| | 11.0085 | 50% | 70% | 4 | na | na | na |
| | 16.0061 | 50% | 80% | | | | |
| | 25.8224 | 50% | 90% | | | | |

UO only

| Time prior AKI stage | Cutoff value | sens | spec | Quartile | OR | 95% CI of OR | |
|---|---|---|---|---|---|---|---|
| 0 hours | 21.7226 | 75% | 92% | 1 | | | |
| | 3.28789 | 100% | 38% | 2 | na | na | na |
| | 3.28789 | 100% | 38% | 3 | na | na | na |
| | 9.0843 | 75% | 73% | 4 | na | na | na |
| | 10.1744 | 75% | 81% | | | | |
| | 21.7226 | 75% | 92% | | | | |
| 24 hours | 3.58953 | 75% | 42% | 1 | | | |
| | 3.28789 | 100% | 38% | 2 | na | na | na |
| | 3.28789 | 100% | 38% | 3 | na | na | na |
| | 9.0843 | 50% | 73% | 4 | na | na | na |
| | 10.1744 | 50% | 81% | | | | |
| | 21.7226 | 25% | 92% | | | | |
| 48 hours | 3.28789 | 75% | 38% | 1 | | | |
| | 1.47804 | 100% | 15% | 2 | 0.9 | 0.0 | 79.2 |
| | 1.47804 | 100% | 15% | 3 | 0.0 | 0.0 | na |
| | 9.0843 | 50% | 73% | 4 | 2.0 | 0.1 | 72.7 |
| | 10.1744 | 50% | 81% | | | | |
| | 21.7226 | 25% | 92% | | | | |

# Fig. 4 - 22

CD44

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 233.270 | 239.095 | 233.270 | 249.536 | 233.270 | 124.024 |
| average | 268.353 | 267.878 | 268.353 | 248.401 | 268.353 | 205.426 |
| stdev | 145.627 | 121.217 | 145.627 | 122.787 | 145.627 | na |
| p (t-test) | | 0.989 | | 0.582 | | na |
| min | 124.024 | 127.583 | 124.024 | 82.500 | 124.024 | 205.426 |
| max | 1060.922 | 588.839 | 1060.922 | 585.758 | 1060.922 | 205.426 |
| n (Samp) | 82 | 21 | 82 | 19 | 82 | 1 |
| n (Pat) | 47 | 21 | 47 | 19 | 47 | 1 |

sCr only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 233.013 | 275.555 | 233.013 | 256.589 | 233.013 | 294.084 |
| average | 258.968 | 293.506 | 258.968 | 266.426 | 258.968 | 284.832 |
| stdev | 127.266 | 131.817 | 127.266 | 144.684 | 127.266 | 74.548 |
| p (t-test) | | 0.458 | | 0.842 | | 0.727 |
| min | 82.500 | 167.442 | 82.500 | 96.750 | 82.500 | 206.090 |
| max | 1060.922 | 588.839 | 1060.922 | 585.758 | 1060.922 | 354.323 |
| n (Samp) | 135 | 8 | 135 | 13 | 135 | 3 |
| n (Pat) | 78 | 8 | 78 | 13 | 78 | 3 |

UO only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 230.072 | 226.603 | 230.072 | 273.643 | 230.072 | 205.207 |
| average | 249.164 | 266.140 | 249.164 | 261.575 | 249.164 | 205.207 |
| stdev | 93.909 | 121.608 | 93.909 | 99.651 | 93.909 | 0.310 |
| p (t-test) | | 0.502 | | 0.667 | | 0.513 |
| min | 124.024 | 127.583 | 124.024 | 82.500 | 124.024 | 204.988 |
| max | 639.273 | 588.839 | 639.273 | 403.263 | 639.273 | 205.426 |
| n (Samp) | 68 | 21 | 68 | 13 | 68 | 2 |
| n (Pat) | 37 | 21 | 37 | 13 | 37 | 2 |

sCr or UO

| Time prior AKI stage | AUC | SE | $n_{Cohort 1}$ | $n_{Cohort 2}$ | p |
| --- | --- | --- | --- | --- | --- |
| 0 hours | 0.50 | 0.071 | 82 | 21 | 0.974 |
| 24 hours | 0.48 | 0.073 | 82 | 19 | 0.742 |
| 48 hours | 0.35 | 0.249 | 82 | 1 | 0.556 |

sCr only

| Time prior AKI stage | AUC | SE | $n_{Cohort 1}$ | $n_{Cohort 2}$ | p |
| --- | --- | --- | --- | --- | --- |
| 0 hours | 0.61 | 0.109 | 135 | 8 | 0.324 |
| 24 hours | 0.50 | 0.084 | 135 | 13 | 0.995 |
| 48 hours | 0.67 | 0.174 | 135 | 3 | 0.319 |

UO only

| Time prior AKI stage | AUC | SE | $n_{Cohort 1}$ | $n_{Cohort 2}$ | p |
| --- | --- | --- | --- | --- | --- |
| 0 hours | 0.51 | 0.073 | 68 | 21 | 0.931 |
| 24 hours | 0.57 | 0.090 | 68 | 13 | 0.467 |
| 48 hours | 0.35 | 0.178 | 68 | 2 | 0.409 |

sCr or UO

| Time prior AKI stage | Cutoff value | sens | spec | Quartile | OR | 95% CI of OR | |
| --- | --- | --- | --- | --- | --- | --- | --- |
| 0 hours | 168.217 | 71% | 13% | 1 | | | |
| | 166.667 | 81% | 13% | 2 | 0.3 | 0.1 | 1.0 |
| | 152.713 | 90% | 7% | 3 | 0.5 | 0.2 | 1.2 |

# Fig. 5 - 1

|  | 266.346 | 38% | 71% | 4 | 0.9 | 0.4 | 2.1 |
|---|---|---|---|---|---|---|---|
|  | 307.351 | 33% | 80% |  |  |  |  |
|  | 383.614 | 24% | 90% |  |  |  |  |
| 24 hours | 166.667 | 74% | 13% | 1 |  |  |  |
|  | 124.024 | 84% | 1% | 2 | 0.6 | 0.2 | 1.7 |
|  | 82.5 | 95% | 0% | 3 | 0.5 | 0.1 | 1.5 |
|  | 266.346 | 47% | 71% | 4 | 1.1 | 0.4 | 2.5 |
|  | 307.351 | 21% | 80% |  |  |  |  |
|  | 383.614 | 11% | 90% |  |  |  |  |
| 48 hours | 203.876 | 100% | 35% | 1 |  |  |  |
|  | 203.876 | 100% | 35% | 2 | na | na | na |
|  | 203.876 | 100% | 35% | 3 | na | na | na |
|  | 266.346 | 0% | 71% | 4 | na | na | na |
|  | 307.351 | 0% | 80% |  |  |  |  |
|  | 383.614 | 0% | 90% |  |  |  |  |

sCr only

| Time prior AKI stage | Cutoff value | sens | spec | Quartile | OR | 95% CI of OR | |
|---|---|---|---|---|---|---|---|
| 0 hours | 254.408 | 75% | 62% | 1 |  |  |  |
|  | 167.442 | 88% | 16% | 2 | 0.0 | 0.0 | na |
|  | 166.667 | 100% | 16% | 3 | 1.5 | 0.3 | 8.7 |
|  | 280.162 | 50% | 70% | 4 | 1.5 | 0.3 | 8.7 |
|  | 308.32 | 25% | 80% |  |  |  |  |
|  | 386.623 | 13% | 90% |  |  |  |  |
| 24 hours | 155.568 | 77% | 11% | 1 |  |  |  |
|  | 145.976 | 85% | 8% | 2 | 0.0 | 0.0 | na |
|  | 96.75 | 92% | 1% | 3 | 0.8 | 0.3 | 2.1 |
|  | 280.162 | 38% | 70% | 4 | 0.8 | 0.3 | 2.1 |
|  | 308.32 | 23% | 80% |  |  |  |  |
|  | 386.623 | 15% | 90% |  |  |  |  |
| 48 hours | 205.426 | 100% | 39% | 1 |  |  |  |
|  | 205.426 | 100% | 39% | 2 | na | na | na |
|  | 205.426 | 100% | 39% | 3 | na | na | na |
|  | 280.162 | 67% | 70% | 4 | na | na | na |
|  | 308.32 | 33% | 80% |  |  |  |  |
|  | 386.623 | 0% | 90% |  |  |  |  |

UO only

| Time prior AKI stage | Cutoff value | sens | spec | Quartile | OR | 95% CI of OR | |
|---|---|---|---|---|---|---|---|
| 0 hours | 181.323 | 71% | 19% | 1 |  |  |  |
|  | 156.731 | 81% | 10% | 2 | 0.5 | 0.2 | 1.3 |
|  | 145.976 | 90% | 7% | 3 | 0.2 | 0.0 | 0.9 |
|  | 257.372 | 38% | 71% | 4 | 1.1 | 0.5 | 2.5 |
|  | 291.987 | 33% | 81% |  |  |  |  |
|  | 358.806 | 24% | 91% |  |  |  |  |
| 24 hours | 198.028 | 77% | 31% | 1 |  |  |  |
|  | 188.979 | 85% | 24% | 2 | 1.0 | 0.2 | 4.7 |
|  | 124.024 | 92% | 1% | 3 | 0.6 | 0.1 | 4.0 |
|  | 257.372 | 54% | 71% | 4 | 1.8 | 0.5 | 6.4 |
|  | 291.987 | 38% | 81% |  |  |  |  |
|  | 358.806 | 23% | 91% |  |  |  |  |
| 48 hours | 203.876 | 100% | 35% | 1 |  |  |  |
|  | 203.876 | 100% | 35% | 2 | na | na | na |
|  | 203.876 | 100% | 35% | 3 | na | na | na |
|  | 257.372 | 0% | 71% | 4 | na | na | na |
|  | 291.987 | 0% | 81% |  |  |  |  |
|  | 358.806 | 0% | 91% |  |  |  |  |

Fig. 5 - 2

Angiopoietin-1

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 1864.239 | 1507.705 | 1864.239 | 1157.895 | 1864.239 | 1556.391 |
| average | 2521.995 | 1944.516 | 2521.995 | 2096.369 | 2521.995 | 2136.533 |
| stdev | 2433.927 | 1657.582 | 2433.927 | 2493.312 | 2433.927 | 2020.430 |
| p (t-test) | | 0.108 | | 0.266 | | 0.455 |
| min | 192.308 | 171.053 | 192.308 | 144.737 | 192.308 | 144.737 |
| max | 14800.944 | 8056.338 | 14800.944 | 9428.958 | 14800.944 | 8584.574 |
| n (Samp) | 150 | 54 | 150 | 57 | 150 | 25 |
| n (Pat) | 106 | 54 | 106 | 57 | 106 | 25 |

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 1614.710 | 1534.586 | 1614.710 | 1152.824 | 1614.710 | 1977.444 |
| average | 2430.000 | 2016.082 | 2430.000 | 1599.623 | 2430.000 | 2527.573 |
| stdev | 2408.093 | 1781.629 | 2408.093 | 1643.209 | 2408.093 | 2148.769 |
| p (t-test) | | 0.451 | | 0.098 | | 0.886 |
| min | 144.737 | 294.737 | 144.737 | 134.615 | 144.737 | 553.030 |
| max | 14800.944 | 8238.160 | 14800.944 | 7308.642 | 14800.944 | 8660.352 |
| n (Samp) | 308 | 20 | 308 | 24 | 308 | 13 |
| n (Pat) | 171 | 20 | 171 | 24 | 171 | 13 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 1647.059 | 1559.084 | 1647.059 | 1203.008 | 1647.059 | 1556.391 |
| average | 2538.222 | 1910.738 | 2538.222 | 2203.885 | 2538.222 | 2103.950 |
| stdev | 2606.304 | 1713.256 | 2606.304 | 2585.014 | 2606.304 | 2132.893 |
| p (t-test) | | 0.124 | | 0.456 | | 0.470 |
| min | 192.308 | 171.053 | 192.308 | 144.737 | 192.308 | 144.737 |
| max | 14800.944 | 8056.338 | 14800.944 | 9428.958 | 14800.944 | 8584.574 |
| n (Samp) | 127 | 48 | 127 | 46 | 127 | 21 |
| n (Pat) | 86 | 48 | 86 | 46 | 86 | 21 |

sCr or UO

| Time prior AKI stage | AUC | SE | $n_{Cohort\ 1}$ | $n_{Cohort\ 2}$ | p |
|---|---|---|---|---|---|
| 0 hours | 0.44 | 0.045 | 150 | 54 | 0.186 |
| 24 hours | 0.40 | 0.043 | 150 | 57 | 0.021 |
| 48 hours | 0.45 | 0.061 | 150 | 25 | 0.446 |

sCr only

| Time prior AKI stage | AUC | SE | $n_{Cohort\ 1}$ | $n_{Cohort\ 2}$ | p |
|---|---|---|---|---|---|
| 0 hours | 0.49 | 0.066 | 308 | 20 | 0.822 |
| 24 hours | 0.39 | 0.056 | 308 | 24 | 0.059 |
| 48 hours | 0.55 | 0.084 | 308 | 13 | 0.518 |

UO only

| Time prior AKI stage | AUC | SE | $n_{Cohort\ 1}$ | $n_{Cohort\ 2}$ | p |
|---|---|---|---|---|---|
| 0 hours | 0.44 | 0.048 | 127 | 48 | 0.196 |
| 24 hours | 0.42 | 0.048 | 127 | 46 | 0.109 |
| 48 hours | 0.45 | 0.066 | 127 | 21 | 0.485 |

sCr or UO

| Time prior AKI stage | Cutoff value | sens | spec | Quartile | OR | 95% CI of OR | |
|---|---|---|---|---|---|---|---|
| 0 hours | 815.217 | 70% | 24% | 1 | | | |

# Fig. 5 - 3

| Time prior AKI stage | Cutoff value | sens | spec | Quartile | OR | 95% CI of OR | |
|---|---|---|---|---|---|---|---|
| | 651.515 | 81% | 19% | 2 | 1.8 | 1.1 | 2.8 |
| | 468.75 | 91% | 13% | 3 | 1.9 | 1.2 | 3.0 |
| | 2862.14 | 19% | 70% | 4 | 2.1 | 1.4 | 3.3 |
| | 3912.33 | 13% | 80% | | | | |
| | 5391.07 | 4% | 90% | | | | |
| 24 hours | 815.217 | 70% | 24% | 1 | | | |
| | 559.091 | 81% | 15% | 2 | 0.6 | 0.3 | 1.0 |
| | 320 | 91% | 5% | 3 | 3.0 | 2.0 | 4.3 |
| | 2862.14 | 19% | 70% | 4 | 1.7 | 1.1 | 2.6 |
| | 3912.33 | 18% | 80% | | | | |
| | 5391.07 | 12% | 90% | | | | |
| 48 hours | 773.585 | 72% | 23% | 1 | | | |
| | 739.13 | 80% | 23% | 2 | 1.2 | 0.5 | 2.8 |
| | 496.575 | 92% | 15% | 3 | 1.2 | 0.5 | 2.8 |
| | 2862.14 | 20% | 70% | 4 | 1.8 | 0.8 | 3.7 |
| | 3912.33 | 12% | 80% | | | | |
| | 5391.07 | 8% | 90% | | | | |

sCr only

| Time prior AKI stage | Cutoff value | sens | spec | Quartile | OR | 95% CI of OR | |
|---|---|---|---|---|---|---|---|
| 0 hours | 1109.97 | 70% | 36% | 1 | | | |
| | 818.182 | 80% | 26% | 2 | 3.7 | 1.0 | 13.8 |
| | 651.515 | 90% | 19% | 3 | 3.7 | 1.0 | 13.8 |
| | 2600.42 | 25% | 70% | 4 | 2.1 | 0.4 | 9.4 |
| | 3897.2 | 10% | 80% | | | | |
| | 5649.38 | 5% | 90% | | | | |
| 24 hours | 818.182 | 71% | 26% | 1 | | | |
| | 511.538 | 83% | 15% | 2 | 3.2 | 0.8 | 12.3 |
| | 320 | 96% | 7% | 3 | 5.5 | 1.6 | 18.9 |
| | 2600.42 | 8% | 70% | 4 | 3.2 | 0.8 | 12.3 |
| | 3897.2 | 8% | 80% | | | | |
| | 5649.38 | 4% | 90% | | | | |
| 48 hours | 1263.74 | 77% | 41% | 1 | | | |
| | 1030.84 | 85% | 33% | 2 | 1.5 | 0.3 | 8.2 |
| | 750 | 92% | 23% | 3 | 2.1 | 0.4 | 9.4 |
| | 2600.42 | 31% | 70% | 4 | 2.0 | 0.4 | 9.3 |
| | 3897.2 | 8% | 80% | | | | |
| | 5649.38 | 8% | 90% | | | | |

UO only

| Time prior AKI stage | Cutoff value | sens | spec | Quartile | OR | 95% CI of OR | |
|---|---|---|---|---|---|---|---|
| 0 hours | 713.636 | 71% | 22% | 1 | | | |
| | 568.182 | 81% | 16% | 2 | 1.8 | 1.1 | 2.9 |
| | 375 | 92% | 8% | 3 | 1.1 | 0.7 | 1.9 |
| | 2710.36 | 19% | 70% | 4 | 2.1 | 1.3 | 3.3 |
| | 4010.38 | 13% | 80% | | | | |
| | 5572.12 | 4% | 91% | | | | |
| 24 hours | 739.13 | 72% | 24% | 1 | | | |
| | 493.75 | 80% | 13% | 2 | 0.8 | 0.4 | 1.4 |
| | 281.25 | 91% | 3% | 3 | 1.8 | 1.2 | 2.9 |
| | 2710.36 | 24% | 70% | 4 | 1.5 | 0.9 | 2.4 |
| | 4010.38 | 20% | 80% | | | | |
| | 5572.12 | 11% | 91% | | | | |
| 48 hours | 773.585 | 71% | 24% | 1 | | | |
| | 695.652 | 81% | 22% | 2 | 2.6 | 0.9 | 7.6 |
| | 375 | 90% | 8% | 3 | 1.8 | 0.6 | 5.7 |
| | 2710.36 | 14% | 70% | 4 | 2.2 | 0.7 | 6.6 |
| | 4010.38 | 14% | 80% | | | | |
| | 5572.12 | 10% | 91% | | | | |

Fig. 5 - 4

**Angiopoietin-1 receptor**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 20.998 | 16.862 | 20.998 | 16.561 | 20.998 | 15.443 |
| average | 21.861 | 20.162 | 21.861 | 19.040 | 21.861 | 15.443 |
| stdev | 7.461 | 11.892 | 7.461 | 9.095 | 7.461 | 2.896 |
| p (t-test) | | 0.548 | | 0.240 | | 0.231 |
| min | 0.003 | 0.081 | 0.003 | 0.081 | 0.003 | 13.396 |
| max | 39.388 | 39.247 | 39.388 | 34.294 | 39.388 | 17.491 |
| n (Samp) | 77 | 9 | 77 | 12 | 77 | 2 |
| n (Pat) | 19 | 9 | 19 | 12 | 19 | 2 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 20.156 | 24.021 | 20.156 | 24.654 | 20.156 | 20.680 |
| average | 21.019 | 26.814 | 21.019 | 24.532 | 21.019 | 20.152 |
| stdev | 7.537 | 10.461 | 7.537 | 6.597 | 7.537 | 6.281 |
| p (t-test) | | 0.100 | | 0.231 | | 0.844 |
| min | 0.003 | 15.207 | 0.003 | 15.872 | 0.003 | 13.624 |
| max | 39.571 | 39.247 | 39.571 | 34.294 | 39.571 | 26.153 |
| n (Samp) | 117 | 5 | 117 | 7 | 117 | 3 |
| n (Pat) | 26 | 5 | 26 | 7 | 26 | 3 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 20.360 | 16.475 | 20.360 | 15.382 | 20.360 | 15.443 |
| average | 21.251 | 15.155 | 21.251 | 17.011 | 21.251 | 15.443 |
| stdev | 6.725 | 7.491 | 6.725 | 8.281 | 6.725 | 2.896 |
| p (t-test) | | 0.029 | | 0.080 | | 0.231 |
| min | 10.370 | 0.081 | 10.370 | 0.081 | 10.370 | 13.396 |
| max | 39.148 | 24.942 | 39.148 | 30.292 | 39.148 | 17.491 |
| n (Samp) | 58 | 7 | 58 | 10 | 58 | 2 |
| n (Pat) | 14 | 7 | 14 | 10 | 14 | 2 |

sCr or UO

| Time prior AKI stage | AUC | SE | $n_{Cohort 1}$ | $n_{Cohort 2}$ | p |
|---|---|---|---|---|---|
| 0 hours | 0.41 | 0.096 | 77 | 9 | 0.370 |
| 24 hours | 0.40 | 0.084 | 77 | 12 | 0.245 |
| 48 hours | 0.19 | 0.116 | 77 | 2 | 0.007 |

sCr only

| Time prior AKI stage | AUC | SE | $n_{Cohort 1}$ | $n_{Cohort 2}$ | p |
|---|---|---|---|---|---|
| 0 hours | 0.67 | 0.135 | 117 | 5 | 0.212 |
| 24 hours | 0.65 | 0.116 | 117 | 7 | 0.198 |
| 48 hours | 0.47 | 0.166 | 117 | 3 | 0.857 |

UO only

| Time prior AKI stage | AUC | SE | $n_{Cohort 1}$ | $n_{Cohort 2}$ | p |
|---|---|---|---|---|---|
| 0 hours | 0.28 | 0.089 | 58 | 7 | 0.013 |
| 24 hours | 0.35 | 0.087 | 58 | 10 | 0.077 |
| 48 hours | 0.22 | 0.130 | 58 | 2 | 0.028 |

sCr or UO

| Time prior AKI stage | Cutoff value | sens | spec | Quartile | OR | 95% CI of OR | |
|---|---|---|---|---|---|---|---|
| 0 hours | 14.486 | 78% | 17% | 1 | | | |

# Fig. 5 - 5

| Time prior AKI stage | Cutoff value | sens | spec | Quartile | OR | 95% CI of OR | |
|---|---|---|---|---|---|---|---|
| | 14.4271 | 89% | 17% | 2 | 0.5 | 0.0 | 11.5 |
| | 0.00293 | 100% | 1% | 3 | 1.0 | 0.1 | 8.6 |
| | 25.0487 | 22% | 70% | 4 | 2.4 | 0.4 | 12.7 |
| | 27.4854 | 22% | 81% | | | | |
| | 33.3466 | 22% | 91% | | | | |
| 24 hours | 14.4271 | 75% | 17% | 1 | | | |
| | 13.8249 | 83% | 14% | 2 | 0.7 | 0.1 | 4.2 |
| | 13.076 | 92% | 13% | 3 | 0.3 | 0.0 | 5.2 |
| | 25.0487 | 25% | 70% | 4 | 2.5 | 0.8 | 8.3 |
| | 27.4854 | 17% | 81% | | | | |
| | 33.3466 | 8% | 91% | | | | |
| 48 hours | 13.076 | 100% | 13% | 1 | | | |
| | 13.076 | 100% | 13% | 2 | na | na | na |
| | 13.076 | 100% | 13% | 3 | na | na | na |
| | 25.0487 | 0% | 70% | 4 | na | na | na |
| | 27.4854 | 0% | 81% | | | | |
| | 33.3466 | 0% | 91% | | | | |

sCr only

| Time prior AKI stage | Cutoff value | sens | spec | Quartile | OR | 95% CI of OR | |
|---|---|---|---|---|---|---|---|
| 0 hours | 19.2896 | 80% | 46% | 1 | | | |
| | 19.2896 | 80% | 46% | 2 | 1.0 | 0.0 | 55.6 |
| | 15.1529 | 100% | 21% | 3 | 1.0 | 0.0 | 57.7 |
| | 23.2464 | 60% | 70% | 4 | 2.0 | 0.1 | 43.3 |
| | 26.8283 | 40% | 80% | | | | |
| | 31.384 | 40% | 91% | | | | |
| 24 hours | 21.8076 | 71% | 60% | 1 | | | |
| | 17.5115 | 86% | 32% | 2 | 1.0 | 0.0 | 57.4 |
| | 15.7824 | 100% | 24% | 3 | 2.1 | 0.1 | 44.7 |
| | 23.2464 | 57% | 70% | 4 | 3.2 | 0.2 | 50.2 |
| | 26.8283 | 43% | 80% | | | | |
| | 31.384 | 14% | 91% | | | | |
| 48 hours | 13.3956 | 100% | 12% | 1 | | | |
| | 13.3956 | 100% | 12% | 2 | 1.0 | 0.0 | 57.7 |
| | 13.3956 | 100% | 12% | 3 | 0.0 | 0.0 | na |
| | 23.2464 | 33% | 70% | 4 | 1.0 | 0.0 | 57.7 |
| | 26.8283 | 0% | 80% | | | | |
| | 31.384 | 0% | 91% | | | | |

UO only

| Time prior AKI stage | Cutoff value | sens | spec | Quartile | OR | 95% CI of OR | |
|---|---|---|---|---|---|---|---|
| 0 hours | 14.486 | 71% | 19% | 1 | | | |
| | 14.4271 | 86% | 19% | 2 | na | na | na |
| | 0 | 100% | 0% | 3 | na | na | na |
| | 25.0487 | 0% | 71% | 4 | na | na | na |
| | 26.9873 | 0% | 81% | | | | |
| | 29.849 | 0% | 91% | | | | |
| 24 hours | 14.4271 | 70% | 19% | 1 | | | |
| | 13.8249 | 80% | 16% | 2 | 0.5 | 0.0 | 11.4 |
| | 13.076 | 90% | 14% | 3 | 1.0 | 0.1 | 9.2 |
| | 25.0487 | 20% | 71% | 4 | 3.1 | 0.6 | 16.5 |
| | 26.9873 | 20% | 81% | | | | |
| | 29.849 | 10% | 91% | | | | |
| 48 hours | 13.076 | 100% | 14% | 1 | | | |
| | 13.076 | 100% | 14% | 2 | na | na | na |
| | 13.076 | 100% | 14% | 3 | na | na | na |
| | 25.0487 | 0% | 71% | 4 | na | na | na |
| | 26.9873 | 0% | 81% | | | | |
| | 29.849 | 0% | 91% | | | | |

## Fig. 5 - 6

ENA-78 (C-X-C chemokine motif 5)

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.713 | 0.595 | 0.713 | 0.501 | 0.713 | 0.499 |
| average | 1.092 | 0.792 | 1.092 | 0.889 | 1.092 | 1.042 |
| stdev | 1.186 | 0.827 | 1.186 | 1.722 | 1.186 | 1.432 |
| p (t-test) | | 0.073 | | 0.274 | | 0.842 |
| min | 0.022 | 0.038 | 0.022 | 0.001 | 0.022 | 0.092 |
| max | 8.730 | 4.170 | 8.730 | 12.500 | 8.730 | 6.040 |
| n (Samp) | 257 | 56 | 257 | 61 | 257 | 26 |
| n (Pat) | 112 | 56 | 112 | 61 | 112 | 26 |

sCr only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.628 | 0.692 | 0.628 | 0.523 | 0.628 | 0.461 |
| average | 1.107 | 0.690 | 1.107 | 0.770 | 1.107 | 0.704 |
| stdev | 1.962 | 0.541 | 1.962 | 0.729 | 1.962 | 0.608 |
| p (t-test) | | 0.309 | | 0.383 | | 0.443 |
| min | 0.001 | 0.050 | 0.001 | 0.031 | 0.001 | 0.122 |
| max | 32.500 | 2.010 | 32.500 | 3.110 | 32.500 | 1.920 |
| n (Samp) | 459 | 23 | 459 | 26 | 459 | 14 |
| n (Pat) | 180 | 23 | 180 | 26 | 180 | 14 |

UO only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.714 | 0.495 | 0.714 | 0.505 | 0.714 | 0.576 |
| average | 1.074 | 0.759 | 1.074 | 0.948 | 1.074 | 1.075 |
| stdev | 1.153 | 0.843 | 1.153 | 1.818 | 1.153 | 1.490 |
| p (t-test) | | 0.067 | | 0.531 | | 0.996 |
| min | 0.031 | 0.038 | 0.031 | 0.001 | 0.031 | 0.092 |
| max | 8.730 | 4.170 | 8.730 | 12.500 | 8.730 | 6.040 |
| n (Samp) | 213 | 51 | 213 | 53 | 213 | 23 |
| n (Pat) | 89 | 51 | 89 | 53 | 89 | 23 |

sCr or UO

| Time prior AKI stage | AUC | SE | $n_{Cohort 1}$ | $n_{Cohort 2}$ | p |
|---|---|---|---|---|---|
| 0 hours | 0.41 | 0.040 | 257 | 56 | 0.019 |
| 24 hours | 0.37 | 0.037 | 257 | 61 | 0.001 |
| 48 hours | 0.42 | 0.056 | 257 | 26 | 0.148 |

sCr only

| Time prior AKI stage | AUC | SE | $n_{Cohort 1}$ | $n_{Cohort 2}$ | p |
|---|---|---|---|---|---|
| 0 hours | 0.44 | 0.059 | 459 | 23 | 0.318 |
| 24 hours | 0.44 | 0.056 | 459 | 26 | 0.325 |
| 48 hours | 0.43 | 0.074 | 459 | 14 | 0.352 |

UO only

| Time prior AKI stage | AUC | SE | $n_{Cohort 1}$ | $n_{Cohort 2}$ | p |
|---|---|---|---|---|---|
| 0 hours | 0.38 | 0.041 | 213 | 51 | 0.005 |
| 24 hours | 0.39 | 0.041 | 213 | 53 | 0.010 |
| 48 hours | 0.43 | 0.060 | 213 | 23 | 0.236 |

sCr or UO

| Time prior AKI stage | Cutoff value | sens | spec | Quartile | OR | 95% CI of OR | |
|---|---|---|---|---|---|---|---|
| 0 hours | 0.278 | 71% | 18% | 1 | | | |

# Fig. 5 - 7

| Time prior AKI stage | Cutoff value | sens | spec | Quartile | OR | 95% CI of OR | |
|---|---|---|---|---|---|---|---|
| | 0.173 | 80% | 9% | 2 | 1.2 | 0.8 | 1.8 |
| | 0.0801 | 91% | 3% | 3 | 1.0 | 0.7 | 1.5 |
| | 1.18 | 21% | 70% | 4 | 2.3 | 1.6 | 3.2 |
| | 1.61 | 13% | 80% | | | | |
| | 2.41 | 5% | 90% | | | | |
| 24 hours | 0.284 | 70% | 19% | 1 | | | |
| | 0.176 | 80% | 9% | 2 | 1.4 | 0.9 | 2.2 |
| | 0.111 | 90% | 5% | 3 | 2.1 | 1.4 | 3.1 |
| | 1.18 | 18% | 70% | 4 | 3.2 | 2.2 | 4.7 |
| | 1.61 | 8% | 80% | | | | |
| | 2.41 | 5% | 90% | | | | |
| 48 hours | 0.248 | 73% | 16% | 1 | | | |
| | 0.204 | 81% | 13% | 2 | 1.2 | 0.6 | 2.7 |
| | 0.13 | 92% | 5% | 3 | 0.8 | 0.3 | 2.0 |
| | 1.18 | 23% | 70% | 4 | 2.5 | 1.3 | 4.6 |
| | 1.61 | 15% | 80% | | | | |
| | 2.41 | 12% | 90% | | | | |

sCr only

| Time prior AKI stage | Cutoff value | sens | spec | Quartile | OR | 95% CI of OR | |
|---|---|---|---|---|---|---|---|
| 0 hours | 0.284 | 74% | 24% | 1 | | | |
| | 0.154 | 83% | 11% | 2 | 2.1 | 1.0 | 4.5 |
| | 0.0919 | 91% | 5% | 3 | 1.0 | 0.4 | 2.8 |
| | 1.12 | 17% | 70% | 4 | 1.8 | 0.8 | 4.0 |
| | 1.52 | 9% | 80% | | | | |
| | 2.28 | 0% | 90% | | | | |
| 24 hours | 0.272 | 73% | 23% | 1 | | | |
| | 0.173 | 81% | 13% | 2 | 0.7 | 0.3 | 1.4 |
| | 0.107 | 92% | 6% | 3 | 0.9 | 0.5 | 1.6 |
| | 1.12 | 27% | 70% | 4 | 1.2 | 0.7 | 2.0 |
| | 1.52 | 19% | 80% | | | | |
| | 2.28 | 4% | 90% | | | | |
| 48 hours | 0.248 | 71% | 20% | 1 | | | |
| | 0.18 | 86% | 14% | 2 | 1.0 | 0.3 | 3.9 |
| | 0.149 | 93% | 10% | 3 | 1.0 | 0.3 | 3.9 |
| | 1.12 | 21% | 70% | 4 | 1.7 | 0.6 | 5.0 |
| | 1.52 | 14% | 80% | | | | |
| | 2.28 | 0% | 90% | | | | |

UO only

| Time prior AKI stage | Cutoff value | sens | spec | Quartile | OR | 95% CI of OR | |
|---|---|---|---|---|---|---|---|
| 0 hours | 0.244 | 71% | 16% | 1 | | | |
| | 0.15 | 80% | 6% | 2 | 1.1 | 0.7 | 1.8 |
| | 0.111 | 90% | 4% | 3 | 1.3 | 0.8 | 2.0 |
| | 1.27 | 18% | 71% | 4 | 3.0 | 2.0 | 4.4 |
| | 1.61 | 12% | 80% | | | | |
| | 2.23 | 6% | 90% | | | | |
| 24 hours | 0.326 | 72% | 22% | 1 | | | |
| | 0.264 | 81% | 18% | 2 | 1.5 | 0.9 | 2.4 |
| | 0.127 | 91% | 5% | 3 | 1.9 | 1.2 | 3.1 |
| | 1.27 | 17% | 71% | 4 | 3.2 | 2.1 | 4.9 |
| | 1.61 | 9% | 80% | | | | |
| | 2.23 | 6% | 90% | | | | |
| 48 hours | 0.274 | 74% | 19% | 1 | | | |
| | 0.244 | 83% | 16% | 2 | 2.5 | 0.9 | 6.9 |
| | 0.147 | 91% | 6% | 3 | 1.4 | 0.4 | 4.6 |
| | 1.27 | 13% | 71% | 4 | 3.4 | 1.3 | 8.6 |
| | 1.61 | 13% | 80% | | | | |
| | 2.23 | 13% | 90% | | | | |

Fig. 5 - 8

Endoglin

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 4.394 | 3.282 | 4.394 | 4.855 | 4.394 | 1.904 |
| average | 4.196 | 3.955 | 4.196 | 4.855 | 4.196 | 2.007 |
| stdev | 1.162 | 2.601 | 1.162 | 2.278 | 1.162 | na |
| p (t-test) | | 0.607 | | 0.445 | | na |
| min | 1.904 | 1.912 | 1.904 | 3.245 | 1.904 | 2.007 |
| max | 7.699 | 12.339 | 7.699 | 6.466 | 7.699 | 2.007 |
| n (Samp) | 57 | 13 | 57 | 2 | 57 | 1 |
| n (Pat) | 37 | 13 | 37 | 2 | 37 | 1 |

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 3.952 | 3.209 | 3.952 | 3.463 | 3.952 | 4.302 |
| average | 4.063 | 3.182 | 4.063 | 4.001 | 4.063 | 4.302 |
| stdev | 1.374 | 0.842 | 1.374 | 1.739 | 1.374 | 1.626 |
| p (t-test) | | 0.125 | | 0.931 | | 0.809 |
| min | 1.904 | 1.912 | 1.904 | 2.613 | 1.904 | 3.152 |
| max | 12.339 | 4.287 | 12.339 | 6.466 | 12.339 | 5.452 |
| n (Samp) | 89 | 6 | 89 | 4 | 89 | 2 |
| n (Pat) | 61 | 6 | 61 | 4 | 61 | 2 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 4.425 | 3.582 | 4.425 | 4.529 | 4.425 | 1.912 |
| average | 4.167 | 4.276 | 4.167 | 4.529 | 4.167 | 2.007 |
| stdev | 1.146 | 2.559 | 1.146 | 1.816 | 1.146 | na |
| p (t-test) | | 0.826 | | 0.670 | | na |
| min | 1.912 | 2.125 | 1.912 | 3.245 | 1.912 | 2.007 |
| max | 6.988 | 12.339 | 6.988 | 5.813 | 6.988 | 2.007 |
| n (Samp) | 42 | 14 | 42 | 2 | 42 | 1 |
| n (Pat) | 27 | 14 | 27 | 2 | 27 | 1 |

sCr or UO

| Time prior AKI stage | AUC | SE | $n_{Cohort 1}$ | $n_{Cohort 2}$ | p |
|---|---|---|---|---|---|
| 0 hours | 0.29 | 0.072 | 57 | 13 | 0.003 |
| 24 hours | 0.59 | 0.216 | 57 | 2 | 0.685 |
| 48 hours | 0.04 | 0.041 | 57 | 1 | 0.000 |

sCr only

| Time prior AKI stage | AUC | SE | $n_{Cohort 1}$ | $n_{Cohort 2}$ | p |
|---|---|---|---|---|---|
| 0 hours | 0.27 | 0.089 | 89 | 6 | 0.010 |
| 24 hours | 0.43 | 0.141 | 89 | 4 | 0.639 |
| 48 hours | 0.58 | 0.214 | 89 | 2 | 0.714 |

UO only

| Time prior AKI stage | AUC | SE | $n_{Cohort 1}$ | $n_{Cohort 2}$ | p |
|---|---|---|---|---|---|
| 0 hours | 0.38 | 0.083 | 42 | 14 | 0.134 |
| 24 hours | 0.57 | 0.217 | 42 | 2 | 0.742 |
| 48 hours | 0.02 | 0.033 | 42 | 1 | 0.000 |

sCr or UO

| Time prior AKI stage | Cutoff value | sens | spec | Quartile | OR | 95% CI of OR | |
|---|---|---|---|---|---|---|---|
| 0 hours | 2.91265 | 77% | 18% | 1 | | | |

# Fig. 5 - 9

| Time prior AKI stage | Cutoff value | sens | spec | Quartile | OR | 95% CI of OR | |
|---|---|---|---|---|---|---|---|
| | 2.60736 | 85% | 11% | 2 | 1.1 | 0.0 | 67.9 |
| | 2.56967 | 92% | 11% | 3 | 6.5 | 0.5 | 89.6 |
| | 4.64362 | 8% | 70% | 4 | 9.3 | 0.7 | 122.4 |
| | 4.86196 | 8% | 81% | | | | |
| | 5.43169 | 8% | 91% | | | | |
| 24 hours | 3.18596 | 100% | 21% | 1 | | | |
| | 3.18596 | 100% | 21% | 2 | 0.0 | 0.0 | na |
| | 3.18596 | 100% | 21% | 3 | 0.0 | 0.0 | na |
| | 4.64362 | 50% | 70% | 4 | 0.9 | 0.0 | 62.6 |
| | 4.86196 | 50% | 81% | | | | |
| | 5.43169 | 50% | 91% | | | | |
| 48 hours | 1.91176 | 100% | 4% | 1 | | | |
| | 1.91176 | 100% | 4% | 2 | na | na | na |
| | 1.91176 | 100% | 4% | 3 | na | na | na |
| | 4.64362 | 0% | 70% | 4 | na | na | na |
| | 4.86196 | 0% | 81% | | | | |
| | 5.43169 | 0% | 91% | | | | |

sCr only

| Time prior AKI stage | Cutoff value | sens | spec | Quartile | OR | 95% CI of OR | |
|---|---|---|---|---|---|---|---|
| 0 hours | 2.60736 | 83% | 9% | 1 | | | |
| | 2.60736 | 83% | 9% | 2 | na | na | na |
| | 1.90441 | 100% | 1% | 3 | na | na | na |
| | 4.50153 | 0% | 71% | 4 | na | na | na |
| | 4.71626 | 0% | 81% | | | | |
| | 5.0502 | 0% | 91% | | | | |
| 24 hours | 2.93944 | 75% | 17% | 1 | | | |
| | 2.60736 | 100% | 9% | 2 | 1.0 | 0.0 | 62.7 |
| | 2.60736 | 100% | 9% | 3 | 0.0 | 0.0 | na |
| | 4.50153 | 25% | 71% | 4 | 2.2 | 0.1 | 49.5 |
| | 4.71626 | 25% | 81% | | | | |
| | 5.0502 | 25% | 91% | | | | |
| 48 hours | 3.12701 | 100% | 22% | 1 | | | |
| | 3.12701 | 100% | 22% | 2 | 0.0 | 0.0 | na |
| | 3.12701 | 100% | 22% | 3 | 0.0 | 0.0 | na |
| | 4.50153 | 50% | 71% | 4 | 1.0 | 0.0 | 57.7 |
| | 4.71626 | 50% | 81% | | | | |
| | 5.0502 | 50% | 91% | | | | |

UO only

| Time prior AKI stage | Cutoff value | sens | spec | Quartile | OR | 95% CI of OR | |
|---|---|---|---|---|---|---|---|
| 0 hours | 3.12701 | 71% | 21% | 1 | | | |
| | 2.91265 | 86% | 21% | 2 | 0.5 | 0.0 | 12.0 |
| | 2.56967 | 93% | 12% | 3 | 4.5 | 0.8 | 25.0 |
| | 4.64362 | 14% | 71% | 4 | 3.3 | 0.6 | 19.2 |
| | 4.8773 | 14% | 81% | | | | |
| | 5.43169 | 14% | 90% | | | | |
| 24 hours | 3.18596 | 100% | 24% | 1 | | | |
| | 3.18596 | 100% | 24% | 2 | 0.0 | 0.0 | na |
| | 3.18596 | 100% | 24% | 3 | 0.0 | 0.0 | na |
| | 4.64362 | 50% | 71% | 4 | 1.0 | 0.0 | 74.6 |
| | 4.8773 | 50% | 81% | | | | |
| | 5.43169 | 50% | 90% | | | | |
| 48 hours | 1.91176 | 100% | 2% | 1 | | | |
| | 1.91176 | 100% | 2% | 2 | na | na | na |
| | 1.91176 | 100% | 2% | 3 | na | na | na |
| | 4.64362 | 0% | 71% | 4 | na | na | na |
| | 4.8773 | 0% | 81% | | | | |
| | 5.43169 | 0% | 90% | | | | |

# Fig. 5 - 10

**Epithelial cell-adhesion molecule**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 789.235 | 1210.037 | 789.235 | 3017.017 | 789.235 | na |
| average | 4083.253 | 2286.898 | 4083.253 | 3017.017 | 4083.253 | na |
| stdev | 5914.142 | 2659.564 | 5914.142 | 4264.441 | 5914.142 | na |
| p (t-test) | | 0.353 | | 0.803 | | na |
| min | 1.602 | 1.602 | 1.602 | 1.602 | 1.602 | na |
| max | 17843.902 | 7751.351 | 17843.902 | 6032.432 | 17843.902 | na |
| n (Samp) | 48 | 10 | 48 | 2 | 48 | 0 |
| n (Pat) | 30 | 10 | 30 | 2 | 30 | 0 |

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 822.435 | 3827.027 | 822.435 | 1.602 | 822.435 | 4922.752 |
| average | 3639.201 | 4759.755 | 3639.201 | 1768.769 | 3639.201 | 4922.752 |
| stdev | 5239.637 | 6111.592 | 5239.637 | 3534.334 | 5239.637 | 6959.557 |
| p (t-test) | | 0.648 | | 0.484 | | 0.735 |
| min | 1.602 | 1.602 | 1.602 | 1.602 | 1.602 | 1.602 |
| max | 17843.902 | 14917.073 | 17843.902 | 7070.270 | 17843.902 | 9843.902 |
| n (Samp) | 73 | 5 | 73 | 4 | 73 | 2 |
| n (Pat) | 48 | 5 | 48 | 4 | 48 | 2 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 1137.413 | 1331.320 | 1137.413 | 3017.017 | 1137.413 | na |
| average | 4415.177 | 2629.981 | 4415.177 | 3017.017 | 4415.177 | na |
| stdev | 5813.515 | 2725.796 | 5813.515 | 4264.441 | 5813.515 | na |
| p (t-test) | | 0.333 | | 0.741 | | na |
| min | 1.602 | 1.602 | 1.602 | 1.602 | 1.602 | na |
| max | 15268.293 | 7751.351 | 15268.293 | 6032.432 | 15268.293 | na |
| n (Samp) | 34 | 11 | 34 | 2 | 34 | 0 |
| n (Pat) | 20 | 11 | 20 | 2 | 20 | 0 |

sCr or UO

| Time prior AKI stage | AUC | SE | $n_{Cohort\ 1}$ | $n_{Cohort\ 2}$ | p |
|---|---|---|---|---|---|
| 0 hours | 0.53 | 0.102 | 48 | 10 | 0.791 |
| 24 hours | 0.47 | 0.207 | 48 | 2 | 0.900 |
| 48 hours | nd | nd | 48 | 0 | nd |

sCr only

| Time prior AKI stage | AUC | SE | $n_{Cohort\ 1}$ | $n_{Cohort\ 2}$ | p |
|---|---|---|---|---|---|
| 0 hours | 0.55 | 0.137 | 73 | 5 | 0.742 |
| 24 hours | 0.32 | 0.122 | 73 | 4 | 0.148 |
| 48 hours | 0.50 | 0.208 | 73 | 2 | 0.987 |

UO only

| Time prior AKI stage | AUC | SE | $n_{Cohort\ 1}$ | $n_{Cohort\ 2}$ | p |
|---|---|---|---|---|---|
| 0 hours | 0.50 | 0.101 | 34 | 11 | 1.000 |
| 24 hours | 0.45 | 0.205 | 34 | 2 | 0.802 |
| 48 hours | nd | nd | 34 | 0 | nd |

sCr or UO

| Time prior AKI stage | Cutoff value | sens | spec | Quartile | OR | 95% CI of OR | |
|---|---|---|---|---|---|---|---|
| 0 hours | 452.55 | 70% | 42% | 1 | | | |

## Fig. 5 - 11

| | 1.60233 | 80% | 40% | 2 | na | na | na |
|---|---|---|---|---|---|---|---|
| | 0 | 100% | 0% | 3 | na | na | na |
| | 2885.42 | 40% | 71% | 4 | na | na | na |
| | 12517.1 | 0% | 81% | | | | |
| | 14468.3 | 0% | 92% | | | | |
| 24 hours | 0 | 100% | 0% | 1 | | | |
| | 0 | 100% | 0% | 2 | 0.0 | 0.0 | na |
| | 0 | 100% | 0% | 3 | 0.0 | 0.0 | na |
| | 2885.42 | 50% | 71% | 4 | 1.1 | 0.0 | 77.4 |
| | 12517.1 | 0% | 81% | | | | |
| | 14468.3 | 0% | 92% | | | | |
| 48 hours | na | na | na | 1 | | | |
| | na | na | na | 2 | na | na | na |
| | na | na | na | 3 | na | na | na |
| | na | na | na | 4 | na | na | na |
| | na | na | na | | | | |
| | na | na | na | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.0 | 0.0 | na |
| | 0 | 100% | 0% | 3 | 1.0 | 0.1 | 8.9 |
| | 3904.41 | 40% | 71% | 4 | 0.4 | 0.0 | 10.5 |
| | 8400 | 20% | 81% | | | | |
| | 13590.2 | 20% | 90% | | | | |
| 24 hours | 0 | 100% | 0% | 1 | | | |
| | 0 | 100% | 0% | 2 | 0.0 | 0.0 | na |
| | 0 | 100% | 0% | 3 | 0.0 | 0.0 | na |
| | 3904.41 | 25% | 71% | 4 | 3.6 | 0.2 | 60.9 |
| | 8400 | 0% | 81% | | | | |
| | 13590.2 | 0% | 90% | | | | |
| 48 hours | 0 | 100% | 0% | 1 | | | |
| | 0 | 100% | 0% | 2 | 0.0 | 0.0 | na |
| | 0 | 100% | 0% | 3 | 0.0 | 0.0 | na |
| | 3904.41 | 50% | 71% | 4 | 1.1 | 0.0 | 66.8 |
| | 8400 | 50% | 81% | | | | |
| | 13590.2 | 0% | 90% | | | | |

UO only

| Time prior AKI stage | Cutoff value | sens | spec | Quartile | OR | 95% CI of OR | |
|---|---|---|---|---|---|---|---|
| 0 hours | 49.6324 | 73% | 32% | 1 | | | |
| | 1.60233 | 82% | 32% | 2 | 1.7 | 0.2 | 13.7 |
| | 0 | 100% | 0% | 3 | 2.6 | 0.4 | 18.4 |
| | 3904.41 | 36% | 71% | 4 | 0.9 | 0.1 | 9.7 |
| | 13590.2 | 0% | 85% | | | | |
| | 14468.3 | 0% | 91% | | | | |
| 24 hours | 0 | 100% | 0% | 1 | | | |
| | 0 | 100% | 0% | 2 | na | na | na |
| | 0 | 100% | 0% | 3 | na | na | na |
| | 3904.41 | 50% | 71% | 4 | na | na | na |
| | 13590.2 | 0% | 85% | | | | |
| | 14468.3 | 0% | 91% | | | | |
| 48 hours | na | na | na | 1 | | | |
| | na | na | na | 2 | na | na | na |
| | na | na | na | 3 | na | na | na |
| | na | na | na | 4 | na | na | na |
| | na | na | na | | | | |
| | na | na | na | | | | |

# Fig. 5 - 12

**Heme oxygenase 1**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 3.160 | 4.644 | 3.160 | 2.209 | 3.160 | 9.958 |
| average | 4.353 | 7.144 | 4.353 | 5.725 | 4.353 | 9.958 |
| stdev | 4.258 | 6.373 | 4.258 | 8.747 | 4.258 | 9.956 |
| p (t-test) | | 0.015 | | 0.248 | | 0.074 |
| min | 0.757 | 0.905 | 0.757 | 0.331 | 0.757 | 2.918 |
| max | 27.089 | 25.279 | 27.089 | 38.879 | 27.089 | 16.998 |
| n (Samp) | 107 | 20 | 107 | 26 | 107 | 2 |
| n (Pat) | 42 | 20 | 42 | 26 | 42 | 2 |

sCr only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 3.437 | 10.883 | 3.437 | 3.495 | 3.437 | 7.699 |
| average | 4.670 | 12.053 | 4.670 | 10.305 | 4.670 | 12.227 |
| stdev | 4.524 | 9.671 | 4.524 | 13.132 | 4.524 | 12.107 |
| p (t-test) | | 0.000 | | 0.000 | | 0.002 |
| min | 0.692 | 2.478 | 0.692 | 0.331 | 0.692 | 3.458 |
| max | 27.089 | 32.350 | 27.089 | 39.500 | 27.089 | 30.051 |
| n (Samp) | 178 | 8 | 178 | 15 | 178 | 4 |
| n (Pat) | 69 | 8 | 69 | 15 | 69 | 4 |

UO only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 3.642 | 3.994 | 3.642 | 2.470 | 3.642 | 3.159 |
| average | 4.427 | 6.499 | 4.427 | 4.830 | 4.427 | 7.692 |
| stdev | 3.550 | 6.106 | 3.550 | 6.139 | 3.550 | 8.061 |
| p (t-test) | | 0.051 | | 0.698 | | 0.139 |
| min | 0.905 | 0.905 | 0.905 | 0.724 | 0.905 | 2.918 |
| max | 22.382 | 25.279 | 22.382 | 26.422 | 22.382 | 16.998 |
| n (Samp) | 83 | 19 | 83 | 20 | 83 | 3 |
| n (Pat) | 32 | 19 | 32 | 20 | 32 | 3 |

sCr or UO

| Time prior AKI stage | AUC | SE | $n_{Cohort 1}$ | $n_{Cohort 2}$ | p |
|---|---|---|---|---|---|
| 0 hours | 0.66 | 0.071 | 107 | 20 | 0.024 |
| 24 hours | 0.44 | 0.061 | 107 | 26 | 0.288 |
| 48 hours | 0.71 | 0.208 | 107 | 2 | 0.301 |

sCr only

| Time prior AKI stage | AUC | SE | $n_{Cohort 1}$ | $n_{Cohort 2}$ | p |
|---|---|---|---|---|---|
| 0 hours | 0.80 | 0.095 | 178 | 8 | 0.001 |
| 24 hours | 0.58 | 0.080 | 178 | 15 | 0.335 |
| 48 hours | 0.79 | 0.135 | 178 | 4 | 0.029 |

UO only

| Time prior AKI stage | AUC | SE | $n_{Cohort 1}$ | $n_{Cohort 2}$ | p |
|---|---|---|---|---|---|
| 0 hours | 0.60 | 0.075 | 83 | 19 | 0.201 |
| 24 hours | 0.41 | 0.068 | 83 | 20 | 0.199 |
| 48 hours | 0.60 | 0.177 | 83 | 3 | 0.562 |

sCr or UO

| Time prior AKI stage | Cutoff value | sens | spec | Quartile | OR | 95% CI of OR | |
|---|---|---|---|---|---|---|---|
| 0 hours | 3.32031 | 70% | 54% | 1 | | | |

# Fig. 5 - 13

| | 2.52294 | 80% | 41% | 2 | 1.0 | 0.2 | 4.1 |
| | 1.60924 | 90% | 19% | 3 | 1.3 | 0.4 | 4.8 |
| | 4.60601 | 50% | 70% | 4 | 4.2 | 1.5 | 11.6 |
| | 4.95016 | 50% | 80% | | | | |
| | 9.00847 | 30% | 91% | | | | |
| 24 hours | 1.41256 | 73% | 16% | 1 | | | |
| | 1.19141 | 81% | 10% | 2 | 0.3 | 0.1 | 0.9 |
| | 0.72428 | 92% | 0% | 3 | 0.6 | 0.3 | 1.3 |
| | 4.60601 | 31% | 70% | 4 | 1.4 | 0.8 | 2.6 |
| | 4.95016 | 31% | 80% | | | | |
| | 9.00847 | 15% | 91% | | | | |
| 48 hours | 2.90337 | 100% | 46% | 1 | | | |
| | 2.90337 | 100% | 46% | 2 | na | na | na |
| | 2.90337 | 100% | 46% | 3 | na | na | na |
| | 4.60601 | 50% | 70% | 4 | na | na | na |
| | 4.95016 | 50% | 80% | | | | |
| | 9.00847 | 50% | 91% | | | | |

sCr only

| Time prior AKI stage | Cutoff value | sens | spec | Quartile | OR | 95% CI of OR | |
| --- | --- | --- | --- | --- | --- | --- | --- |
| 0 hours | 5.00283 | 75% | 76% | 1 | | | |
| | 3.86905 | 88% | 57% | 2 | na | na | na |
| | 2.39415 | 100% | 35% | 3 | na | na | na |
| | 4.79434 | 75% | 70% | 4 | na | na | na |
| | 6.39132 | 63% | 80% | | | | |
| | 9.55164 | 50% | 90% | | | | |
| 24 hours | 2.52856 | 73% | 38% | 1 | | | |
| | 1.81738 | 80% | 23% | 2 | 0.7 | 0.2 | 2.5 |
| | 0.72428 | 93% | 2% | 3 | 0.5 | 0.1 | 2.3 |
| | 4.79434 | 40% | 70% | 4 | 1.5 | 0.6 | 3.8 |
| | 6.39132 | 40% | 80% | | | | |
| | 9.55164 | 33% | 90% | | | | |
| 48 hours | 6.0704 | 75% | 79% | 1 | | | |
| | 3.41553 | 100% | 50% | 2 | na | na | na |
| | 3.41553 | 100% | 50% | 3 | na | na | na |
| | 4.79434 | 75% | 70% | 4 | na | na | na |
| | 6.39132 | 50% | 80% | | | | |
| | 9.55164 | 25% | 90% | | | | |

UO only

| Time prior AKI stage | Cutoff value | sens | spec | Quartile | OR | 95% CI of OR | |
| --- | --- | --- | --- | --- | --- | --- | --- |
| 0 hours | 3.17214 | 74% | 46% | 1 | | | |
| | 2.39415 | 84% | 33% | 2 | 1.7 | 0.5 | 6.0 |
| | 1.41256 | 95% | 13% | 3 | 1.0 | 0.2 | 4.4 |
| | 4.86111 | 42% | 71% | 4 | 3.3 | 1.1 | 9.8 |
| | 6.26285 | 42% | 81% | | | | |
| | 9.00847 | 26% | 90% | | | | |
| 24 hours | 1.55599 | 70% | 14% | 1 | | | |
| | 1.28255 | 80% | 11% | 2 | 0.1 | 0.0 | 1.6 |
| | 1.11328 | 90% | 6% | 3 | 0.8 | 0.3 | 2.0 |
| | 4.86111 | 30% | 71% | 4 | 1.6 | 0.7 | 3.4 |
| | 6.26285 | 25% | 81% | | | | |
| | 9.00847 | 15% | 90% | | | | |
| 48 hours | 2.90337 | 100% | 39% | 1 | | | |
| | 2.90337 | 100% | 39% | 2 | na | na | na |
| | 2.90337 | 100% | 39% | 3 | na | na | na |
| | 4.86111 | 33% | 71% | 4 | na | na | na |
| | 6.26285 | 33% | 81% | | | | |
| | 9.00847 | 33% | 90% | | | | |

Fig. 5 - 14

**Interleukin-1 receptor, type II**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 10985.534 | 11443.237 | 10985.534 | 10355.156 | 10985.534 | na |
| average | 13974.069 | 13261.331 | 13974.069 | 15204.798 | 13974.069 | na |
| stdev | 12388.526 | 6314.468 | 12388.526 | 10981.262 | 12388.526 | na |
| p (t-test) | | 0.877 | | 0.741 | | na |
| min | 3061.967 | 5139.251 | 3061.967 | 5981.981 | 3061.967 | na |
| max | 56549.592 | 23105.619 | 56549.592 | 44711.687 | 56549.592 | na |
| n (Samp) | 26 | 8 | 26 | 17 | 26 | 0 |
| n (Pat) | 25 | 8 | 25 | 17 | 25 | 0 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 11554.246 | 8979.846 | 11554.246 | 10355.156 | 11554.246 | 2160.038 |
| average | 13983.592 | 8979.846 | 13983.592 | 29731.997 | 13983.592 | 12731.082 |
| stdev | 10628.390 | 3218.550 | 10628.390 | 46953.355 | 10628.390 | na |
| p (t-test) | | 0.513 | | 0.040 | | na |
| min | 2160.038 | 6703.987 | 2160.038 | 7159.935 | 2160.038 | 12731.082 |
| max | 56549.592 | 11255.704 | 56549.592 | 150973.398 | 56549.592 | 12731.082 |
| n (Samp) | 47 | 2 | 47 | 9 | 47 | 1 |
| n (Pat) | 44 | 2 | 44 | 9 | 44 | 1 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 10409.535 | 11630.771 | 10409.535 | 10696.109 | 10409.535 | 3061.967 |
| average | 13837.547 | 13547.850 | 13837.547 | 15151.297 | 13837.547 | 9971.162 |
| stdev | 12234.719 | 6764.002 | 12234.719 | 9389.527 | 12234.719 | na |
| p (t-test) | | 0.953 | | 0.752 | | na |
| min | 3061.967 | 5139.251 | 3061.967 | 5981.981 | 3061.967 | 9971.162 |
| max | 56549.592 | 23105.619 | 56549.592 | 36344.165 | 56549.592 | 9971.162 |
| n (Samp) | 27 | 7 | 27 | 11 | 27 | 1 |
| n (Pat) | 25 | 7 | 25 | 11 | 25 | 1 |

sCr or UO

| Time prior AKI stage | AUC | SE | $n_{Cohort 1}$ | $n_{Cohort 2}$ | p |
|---|---|---|---|---|---|
| 0 hours | 0.55 | 0.120 | 26 | 8 | 0.688 |
| 24 hours | 0.53 | 0.091 | 26 | 17 | 0.729 |
| 48 hours | nd | nd | 26 | 0 | nd |

sCr only

| Time prior AKI stage | AUC | SE | $n_{Cohort 1}$ | $n_{Cohort 2}$ | p |
|---|---|---|---|---|---|
| 0 hours | 0.33 | 0.174 | 47 | 2 | 0.327 |
| 24 hours | 0.53 | 0.107 | 47 | 9 | 0.749 |
| 48 hours | 0.68 | 0.301 | 47 | 1 | 0.548 |

UO only

| Time prior AKI stage | AUC | SE | $n_{Cohort 1}$ | $n_{Cohort 2}$ | p |
|---|---|---|---|---|---|
| 0 hours | 0.57 | 0.126 | 27 | 7 | 0.600 |
| 24 hours | 0.56 | 0.105 | 27 | 11 | 0.554 |
| 48 hours | 0.44 | 0.287 | 27 | 1 | 0.846 |

sCr or UO

| Time prior AKI stage | Cutoff value | sens | spec | Quartile | OR | 95% CI of OR | |
|---|---|---|---|---|---|---|---|
| 0 hours | 10409.5 | 75% | 50% | 1 | | | |

## Fig. 5 - 15

| | 5789.15 | 88% | 15% | 2 | 0.9 | 0.1 | 11.2 |
|---|---|---|---|---|---|---|---|
| | 5058.64 | 100% | 8% | 3 | 0.4 | 0.0 | 14.9 |
| | 12577.1 | 38% | 73% | 4 | 1.5 | 0.2 | 14.8 |
| | 14290 | 38% | 81% | | | | |
| | 25004.5 | 0% | 92% | | | | |
| 24 hours | 8843.12 | 71% | 35% | 1 | | | |
| | 8442.27 | 82% | 31% | 2 | 7.0 | 1.0 | 51.5 |
| | 5981.98 | 94% | 15% | 3 | 1.5 | 0.2 | 12.5 |
| | 12577.1 | 29% | 73% | 4 | 3.3 | 0.5 | 23.3 |
| | 14290 | 29% | 81% | | | | |
| | 25004.5 | 18% | 92% | | | | |
| 48 hours | na | na | na | 1 | | | |
| | na | na | na | 2 | na | na | na |
| | na | na | na | 3 | na | na | na |
| | na | na | na | 4 | na | na | na |
| | na | na | na | | | | |
| | na | na | na | | | | |

sCr only

| Time prior AKI stage | Cutoff value | sens | spec | Quartile | OR | 95% CI of OR | |
|---|---|---|---|---|---|---|---|
| 0 hours | 5981.98 | 100% | 17% | 1 | | | |
| | 5981.98 | 100% | 17% | 2 | na | na | na |
| | 5981.98 | 100% | 17% | 3 | na | na | na |
| | 14290 | 0% | 70% | 4 | na | na | na |
| | 18771.6 | 0% | 81% | | | | |
| | 25004.5 | 0% | 91% | | | | |
| 24 hours | 8442.27 | 78% | 28% | 1 | | | |
| | 7911.48 | 89% | 26% | 2 | 1.6 | 0.2 | 11.8 |
| | 5981.98 | 100% | 17% | 3 | 0.5 | 0.0 | 12.0 |
| | 14290 | 33% | 70% | 4 | 1.6 | 0.2 | 11.8 |
| | 18771.6 | 22% | 81% | | | | |
| | 25004.5 | 22% | 91% | | | | |
| 48 hours | 12635.8 | 100% | 68% | 1 | | | |
| | 12635.8 | 100% | 68% | 2 | na | na | na |
| | 12635.8 | 100% | 68% | 3 | na | na | na |
| | 14290 | 0% | 70% | 4 | na | na | na |
| | 18771.6 | 0% | 81% | | | | |
| | 25004.5 | 0% | 91% | | | | |

UO only

| Time prior AKI stage | Cutoff value | sens | spec | Quartile | OR | 95% CI of OR | |
|---|---|---|---|---|---|---|---|
| 0 hours | 10409.5 | 71% | 52% | 1 | | | |
| | 5789.15 | 86% | 15% | 2 | 0.4 | 0.0 | 12.6 |
| | 5058.64 | 100% | 7% | 3 | 0.4 | 0.0 | 14.9 |
| | 11973.7 | 43% | 70% | 4 | 1.5 | 0.2 | 14.8 |
| | 14290 | 43% | 81% | | | | |
| | 26525.5 | 0% | 93% | | | | |
| 24 hours | 9110.45 | 73% | 41% | 1 | | | |
| | 8691.43 | 82% | 33% | 2 | 1.5 | 0.2 | 13.4 |
| | 7911.48 | 91% | 26% | 3 | 1.0 | 0.1 | 12.4 |
| | 11973.7 | 36% | 70% | 4 | 2.3 | 0.3 | 18.6 |
| | 14290 | 36% | 81% | | | | |
| | 26525.5 | 9% | 93% | | | | |
| 48 hours | 9862.57 | 100% | 44% | 1 | | | |
| | 9862.57 | 100% | 44% | 2 | na | na | na |
| | 9862.57 | 100% | 44% | 3 | na | na | na |
| | 11973.7 | 0% | 70% | 4 | na | na | na |
| | 14290 | 0% | 81% | | | | |
| | 26525.5 | 0% | 93% | | | | |

Fig. 5 - 16

## EP 2 767 833 A2

**Interleukin-6 receptor subunit-alpha**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 16994.159 | 17925.774 | 16994.159 | 15960.218 | 16994.159 | na |
| average | 17874.200 | 18940.321 | 17874.200 | 16637.415 | 17874.200 | na |
| stdev | 6283.446 | 6718.643 | 6283.446 | 9148.713 | 6283.446 | na |
| p (t-test) | | 0.682 | | 0.601 | | na |
| min | 9297.212 | 12468.352 | 9297.212 | 6579.306 | 9297.212 | na |
| max | 33586.605 | 33430.243 | 33586.605 | 45868.574 | 33586.605 | na |
| n (Samp) | 26 | 8 | 26 | 17 | 26 | 0 |
| n (Pat) | 25 | 8 | 25 | 17 | 25 | 0 |

sCr only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 15723.485 | 16216.095 | 15723.485 | 17864.627 | 15723.485 | 6579.306 |
| average | 17053.057 | 16216.095 | 17053.057 | 22496.396 | 17053.057 | 20413.991 |
| stdev | 6433.283 | 3050.506 | 6433.283 | 11811.343 | 6433.283 | na |
| p (t-test) | | 0.857 | | 0.050 | | na |
| min | 6579.306 | 14059.062 | 6579.306 | 8737.351 | 6579.306 | 20413.991 |
| max | 33586.605 | 18373.129 | 33586.605 | 45868.574 | 33586.605 | 20413.991 |
| n (Samp) | 47 | 2 | 47 | 9 | 47 | 1 |
| n (Pat) | 44 | 2 | 44 | 9 | 44 | 1 |

UO only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 14603.356 | 18373.129 | 14603.356 | 13992.231 | 14603.356 | 8737.351 |
| average | 18118.020 | 19637.644 | 18118.020 | 14094.769 | 18118.020 | 9278.343 |
| stdev | 8283.974 | 6937.226 | 8283.974 | 5299.964 | 8283.974 | na |
| p (t-test) | | 0.659 | | 0.146 | | na |
| min | 8737.351 | 12468.352 | 8737.351 | 6579.306 | 8737.351 | 9278.343 |
| max | 45868.574 | 33430.243 | 45868.574 | 25170.902 | 45868.574 | 9278.343 |
| n (Samp) | 27 | 7 | 27 | 11 | 27 | 1 |
| n (Pat) | 25 | 7 | 25 | 11 | 25 | 1 |

sCr or UO

| Time prior AKI stage | AUC | SE | nCohort 1 | nCohort 2 | p |
|---|---|---|---|---|---|
| 0 hours | 0.56 | 0.120 | 26 | 8 | 0.630 |
| 24 hours | 0.40 | 0.088 | 26 | 17 | 0.245 |
| 48 hours | nd | nd | 26 | 0 | nd |

sCr only

| Time prior AKI stage | AUC | SE | nCohort 1 | nCohort 2 | p |
|---|---|---|---|---|---|
| 0 hours | 0.54 | 0.215 | 47 | 2 | 0.843 |
| 24 hours | 0.62 | 0.108 | 47 | 9 | 0.267 |
| 48 hours | 0.77 | 0.283 | 47 | 1 | 0.347 |

UO only

| Time prior AKI stage | AUC | SE | nCohort 1 | nCohort 2 | p |
|---|---|---|---|---|---|
| 0 hours | 0.62 | 0.125 | 27 | 7 | 0.342 |
| 24 hours | 0.34 | 0.094 | 27 | 11 | 0.095 |
| 48 hours | 0.04 | 0.050 | 27 | 1 | 0.000 |

sCr or UO

| Time prior AKI stage | Cutoff value | sens | spec | Quartile | OR | 95% CI of OR | |
|---|---|---|---|---|---|---|---|
| 0 hours | 13902.4 | 75% | 35% | 1 | | | |

## Fig. 5 - 17

169

| | 13152.5 | 88% | 23% | 2 | 0.4 | 0.0 | 12.6 |
|---|---|---|---|---|---|---|---|
| | 12324.9 | 100% | 15% | 3 | 1.8 | 0.2 | 18.9 |
| | 19487.1 | 38% | 73% | 4 | 0.9 | 0.1 | 11.2 |
| | 23927.6 | 13% | 81% | | | | |
| | 25365.5 | 13% | 92% | | | | |
| 24 hours | 10715.1 | 71% | 8% | 1 | | | |
| | 8969.67 | 82% | 0% | 2 | 2.2 | 0.4 | 11.2 |
| | 6579.31 | 94% | 0% | 3 | 1.0 | 0.2 | 6.0 |
| | 19487.1 | 18% | 73% | 4 | 4.0 | 0.7 | 22.2 |
| | 23927.6 | 12% | 81% | | | | |
| | 25365.5 | 6% | 92% | | | | |
| 48 hours | na | na | na | 1 | | | |
| | na | na | na | 2 | na | na | na |
| | na | na | na | 3 | na | na | na |
| | na | na | na | 4 | na | na | na |
| | na | na | na | | | | |
| | na | na | na | | | | |

sCr only

| Time prior AKI stage | Cutoff value | sens | spec | Quartile | OR | 95% CI of OR | |
|---|---|---|---|---|---|---|---|
| 0 hours | 13992.2 | 100% | 43% | 1 | | | |
| | 13992.2 | 100% | 43% | 2 | na | na | na |
| | 13992.2 | 100% | 43% | 3 | na | na | na |
| | 18720.5 | 0% | 70% | 4 | na | na | na |
| | 22123.8 | 0% | 81% | | | | |
| | 25365.5 | 0% | 91% | | | | |
| 24 hours | 15723.5 | 78% | 51% | 1 | | | |
| | 10364 | 89% | 13% | 2 | 0.5 | 0.0 | 12.0 |
| | 6579.31 | 100% | 2% | 3 | 1.0 | 0.1 | 9.8 |
| | 18720.5 | 44% | 70% | 4 | 2.4 | 0.4 | 15.0 |
| | 22123.8 | 44% | 81% | | | | |
| | 25365.5 | 33% | 91% | | | | |
| 48 hours | 20308.4 | 100% | 77% | 1 | | | |
| | 20308.4 | 100% | 77% | 2 | na | na | na |
| | 20308.4 | 100% | 77% | 3 | na | na | na |
| | 18720.5 | 100% | 70% | 4 | na | na | na |
| | 22123.8 | 0% | 81% | | | | |
| | 25365.5 | 0% | 91% | | | | |

UO only

| Time prior AKI stage | Cutoff value | sens | spec | Quartile | OR | 95% CI of OR | |
|---|---|---|---|---|---|---|---|
| 0 hours | 17389.5 | 71% | 70% | 1 | | | |
| | 13152.5 | 86% | 26% | 2 | 0.9 | 0.0 | 74.5 |
| | 12324.9 | 100% | 19% | 3 | 2.3 | 0.1 | 81.0 |
| | 17389.5 | 71% | 70% | 4 | 3.5 | 0.1 | 87.6 |
| | 24925.1 | 14% | 81% | | | | |
| | 31200 | 14% | 93% | | | | |
| 24 hours | 10364 | 73% | 7% | 1 | | | |
| | 8969.67 | 82% | 4% | 2 | 2.0 | 0.2 | 18.1 |
| | 8737.35 | 91% | 4% | 3 | 1.0 | 0.1 | 11.6 |
| | 17389.5 | 18% | 70% | 4 | 3.2 | 0.4 | 26.3 |
| | 24925.1 | 9% | 81% | | | | |
| | 31200 | 0% | 93% | | | | |
| 48 hours | 8737.35 | 100% | 4% | 1 | | | |
| | 8737.35 | 100% | 4% | 2 | na | na | na |
| | 8737.35 | 100% | 4% | 3 | na | na | na |
| | 17389.5 | 0% | 70% | 4 | na | na | na |
| | 24925.1 | 0% | 81% | | | | |
| | 31200 | 0% | 93% | | | | |

Fig. 5 - 18

**Lymphotactin**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.004 | 0.004 | 0.004 | 0.004 | 0.004 | 0.004 |
| average | 0.068 | 0.008 | 0.068 | 0.009 | 0.068 | 0.011 |
| stdev | 0.211 | 0.023 | 0.211 | 0.016 | 0.211 | 0.026 |
| p (t-test) | | 0.036 | | 0.031 | | 0.172 |
| min | 0.004 | 0.004 | 0.004 | 0.004 | 0.004 | 0.004 |
| max | 1.710 | 0.155 | 1.710 | 0.082 | 1.710 | 0.118 |
| n (Samp) | 257 | 56 | 257 | 61 | 257 | 26 |
| n (Pat) | 112 | 56 | 112 | 61 | 112 | 26 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.004 | 0.004 | 0.004 | 0.004 | 0.004 | 0.004 |
| average | 0.050 | 0.017 | 0.050 | 0.024 | 0.050 | 0.012 |
| stdev | 0.179 | 0.061 | 0.179 | 0.067 | 0.179 | 0.031 |
| p (t-test) | | 0.368 | | 0.464 | | 0.424 |
| min | 0.004 | 0.004 | 0.004 | 0.004 | 0.004 | 0.004 |
| max | 1.710 | 0.297 | 1.710 | 0.339 | 1.710 | 0.118 |
| n (Samp) | 459 | 23 | 459 | 26 | 459 | 14 |
| n (Pat) | 180 | 23 | 180 | 26 | 180 | 14 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.004 | 0.004 | 0.004 | 0.004 | 0.004 | 0.004 |
| average | 0.076 | 0.008 | 0.076 | 0.020 | 0.076 | 0.007 |
| stdev | 0.224 | 0.024 | 0.224 | 0.053 | 0.224 | 0.014 |
| p (t-test) | | 0.032 | | 0.070 | | 0.140 |
| min | 0.004 | 0.004 | 0.004 | 0.004 | 0.004 | 0.004 |
| max | 1.710 | 0.155 | 1.710 | 0.309 | 1.710 | 0.073 |
| n (Samp) | 213 | 51 | 213 | 53 | 213 | 23 |
| n (Pat) | 89 | 51 | 89 | 53 | 89 | 23 |

sCr or UO

| Time prior AKI stage | AUC | SE | $n_{Cohort 1}$ | $n_{Cohort 2}$ | p |
|---|---|---|---|---|---|
| 0 hours | 0.42 | 0.040 | 257 | 56 | 0.047 |
| 24 hours | 0.44 | 0.040 | 257 | 61 | 0.157 |
| 48 hours | 0.44 | 0.057 | 257 | 26 | 0.273 |

sCr only

| Time prior AKI stage | AUC | SE | $n_{Cohort 1}$ | $n_{Cohort 2}$ | p |
|---|---|---|---|---|---|
| 0 hours | 0.44 | 0.059 | 459 | 23 | 0.351 |
| 24 hours | 0.51 | 0.059 | 459 | 26 | 0.882 |
| 48 hours | 0.46 | 0.076 | 459 | 14 | 0.567 |

UO only

| Time prior AKI stage | AUC | SE | $n_{Cohort 1}$ | $n_{Cohort 2}$ | p |
|---|---|---|---|---|---|
| 0 hours | 0.42 | 0.043 | 213 | 51 | 0.060 |
| 24 hours | 0.46 | 0.044 | 213 | 53 | 0.359 |
| 48 hours | 0.42 | 0.060 | 213 | 23 | 0.177 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |

# Fig. 5 - 19

| | 0 | 100% | 0% | 2 | 0.0 | 0.0 | na |
| | 0 | 100% | 0% | 3 | 58.4 | 19.2 | 177.2 |
| | 0.00378 | 4% | 81% | 4 | 3.8 | 1.0 | 14.1 |
| | 0.00378 | 4% | 81% | | | | |
| | 0.144 | 2% | 90% | | | | |
| 24 hours | 0 | 100% | 0% | 1 | | | |
| | 0 | 100% | 0% | 2 | 0.0 | 0.0 | na |
| | 0 | 100% | 0% | 3 | 17.6 | 11.2 | 27.7 |
| | 0.00378 | 10% | 81% | 4 | 1.4 | 0.7 | 2.6 |
| | 0.00378 | 10% | 81% | | | | |
| | 0.144 | 0% | 90% | | | | |
| 48 hours | 0 | 100% | 0% | 1 | | | |
| | 0 | 100% | 0% | 2 | 0.0 | 0.0 | na |
| | 0 | 100% | 0% | 3 | 11.7 | 3.7 | 37.2 |
| | 0.00378 | 8% | 81% | 4 | 3.2 | 0.8 | 12.7 |
| | 0.00378 | 8% | 81% | | | | |
| | 0.144 | 0% | 90% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.0 | 0.0 | na |
| | 0 | 100% | 0% | 3 | 26.7 | 3.3 | 214.6 |
| | 0.00378 | 4% | 84% | 4 | 0.0 | 0.0 | na |
| | 0.00378 | 4% | 84% | | | | |
| | 0.104 | 4% | 90% | | | | |
| 24 hours | 0 | 100% | 0% | 1 | | | |
| | 0 | 100% | 0% | 2 | 23.8 | 2.9 | 192.8 |
| | 0 | 100% | 0% | 3 | 0.0 | 0.0 | na |
| | 0.00378 | 19% | 84% | 4 | 5.1 | 0.5 | 55.7 |
| | 0.00378 | 19% | 84% | | | | |
| | 0.104 | 4% | 90% | | | | |
| 48 hours | 0 | 100% | 0% | 1 | | | |
| | 0 | 100% | 0% | 2 | 0.0 | 0.0 | na |
| | 0 | 100% | 0% | 3 | 14.6 | 1.7 | 124.9 |
| | 0.00378 | 7% | 84% | 4 | 0.0 | 0.0 | na |
| | 0.00378 | 7% | 84% | | | | |
| | 0.104 | 7% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 3.1 | 1.5 | 6.5 |
| | 0 | 100% | 0% | 3 | 5.4 | 2.7 | 10.6 |
| | 0.00378 | 4% | 80% | 4 | 6.3 | 3.2 | 12.2 |
| | 0.00378 | 4% | 80% | | | | |
| | 0.189 | 0% | 90% | | | | |
| 24 hours | 0 | 100% | 0% | 1 | | | |
| | 0 | 100% | 0% | 2 | 0.0 | 0.0 | na |
| | 0 | 100% | 0% | 3 | 11.2 | 7.3 | 17.3 |
| | 0.00378 | 13% | 80% | 4 | 1.2 | 0.7 | 2.1 |
| | 0.00378 | 13% | 80% | | | | |
| | 0.189 | 4% | 90% | | | | |
| 48 hours | 0 | 100% | 0% | 1 | | | |
| | 0 | 100% | 0% | 2 | 0.0 | 0.0 | na |
| | 0 | 100% | 0% | 3 | 19.8 | 2.3 | 173.0 |
| | 0.00378 | 4% | 80% | 4 | 7.8 | 0.8 | 78.8 |
| | 0.00378 | 4% | 80% | | | | |
| | 0.189 | 0% | 90% | | | | |

# Fig. 5 - 20

**EP 2 767 833 A2**

**Lymphotoxin-alpha**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.456 | 0.456 | 0.456 | 0.456 | 0.456 | 0.456 |
| average | 1.984 | 0.828 | 1.984 | 2.169 | 1.984 | 1.443 |
| stdev | 4.826 | 1.087 | 4.826 | 4.324 | 4.826 | 1.675 |
| p (t-test) | | 0.076 | | 0.785 | | 0.571 |
| min | 0.456 | 0.456 | 0.456 | 0.456 | 0.456 | 0.456 |
| max | 42.200 | 5.990 | 42.200 | 23.200 | 42.200 | 5.990 |
| n (Samp) | 257 | 56 | 257 | 61 | 257 | 26 |
| n (Pat) | 112 | 56 | 112 | 61 | 112 | 26 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.456 | 0.456 | 0.456 | 0.456 | 0.456 | 0.456 |
| average | 2.339 | 3.208 | 2.339 | 2.173 | 2.339 | 1.504 |
| stdev | 15.808 | 8.353 | 15.808 | 4.019 | 15.808 | 2.430 |
| p (t-test) | | 0.794 | | 0.958 | | 0.844 |
| min | 0.456 | 0.456 | 0.456 | 0.456 | 0.456 | 0.456 |
| max | 330.000 | 40.700 | 330.000 | 18.700 | 330.000 | 9.480 |
| n (Samp) | 459 | 23 | 459 | 26 | 459 | 14 |
| n (Pat) | 180 | 23 | 180 | 26 | 180 | 14 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.456 | 0.456 | 0.456 | 0.456 | 0.456 | 0.456 |
| average | 1.940 | 0.780 | 1.940 | 2.079 | 1.940 | 1.520 |
| stdev | 4.467 | 1.075 | 4.467 | 4.007 | 4.467 | 1.768 |
| p (t-test) | | 0.067 | | 0.836 | | 0.656 |
| min | 0.456 | 0.456 | 0.456 | 0.456 | 0.456 | 0.456 |
| max | 34.100 | 5.990 | 34.100 | 23.200 | 34.100 | 5.990 |
| n (Samp) | 213 | 51 | 213 | 53 | 213 | 23 |
| n (Pat) | 89 | 51 | 89 | 53 | 89 | 23 |

sCr or UO

| Time prior AKI stage | AUC | SE | n Cohort 1 | n Cohort 2 | p |
|---|---|---|---|---|---|
| 0 hours | 0.45 | 0.041 | 257 | 56 | 0.188 |
| 24 hours | 0.51 | 0.041 | 257 | 61 | 0.742 |
| 48 hours | 0.53 | 0.060 | 257 | 26 | 0.575 |

sCr only

| Time prior AKI stage | AUC | SE | n Cohort 1 | n Cohort 2 | p |
|---|---|---|---|---|---|
| 0 hours | 0.57 | 0.064 | 459 | 23 | 0.248 |
| 24 hours | 0.54 | 0.059 | 459 | 26 | 0.533 |
| 48 hours | 0.53 | 0.080 | 459 | 14 | 0.680 |

UO only

| Time prior AKI stage | AUC | SE | n Cohort 1 | n Cohort 2 | p |
|---|---|---|---|---|---|
| 0 hours | 0.44 | 0.044 | 213 | 51 | 0.182 |
| 24 hours | 0.52 | 0.045 | 213 | 53 | 0.642 |
| 48 hours | 0.54 | 0.065 | 213 | 23 | 0.507 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.0 | 0.7 | 1.5 |
| | 0 | 100% | 0% | 3 | 1.9 | 1.3 | 2.6 |

## Fig. 5 - 21

| | 0.456 | 13% | 77% | 4 | 1.6 | 1.1 | 2.3 |
|---|---|---|---|---|---|---|---|
| | 2.14 | 13% | 81% | | | | |
| | 4.44 | 4% | 90% | | | | |
| 24 hours | 0 | 100% | 0% | 1 | | | |
| | 0 | 100% | 0% | 2 | 1.4 | 1.1 | 1.9 |
| | 0 | 100% | 0% | 3 | 0.6 | 0.4 | 0.9 |
| | 0.456 | 25% | 77% | 4 | 1.1 | 0.8 | 1.5 |
| | 2.14 | 23% | 81% | | | | |
| | 4.44 | 15% | 90% | | | | |
| 48 hours | 0 | 100% | 0% | 1 | | | |
| | 0 | 100% | 0% | 2 | 6.0 | 2.6 | 14.0 |
| | 0 | 100% | 0% | 3 | 0.0 | 0.0 | na |
| | 0.456 | 31% | 77% | 4 | 2.8 | 1.1 | 7.4 |
| | 2.14 | 27% | 81% | | | | |
| | 4.44 | 8% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.0 | 0.5 | 1.9 |
| | 0 | 100% | 0% | 3 | 4.5 | 2.9 | 7.0 |
| | 0.456 | 10% | 79% | 4 | 2.3 | 1.4 | 3.7 |
| | 1.73 | 10% | 81% | | | | |
| | 4.44 | 4% | 90% | | | | |
| 24 hours | 0 | 100% | 0% | 1 | | | |
| | 0 | 100% | 0% | 2 | 3.3 | 2.3 | 4.9 |
| | 0 | 100% | 0% | 3 | 0.8 | 0.4 | 1.3 |
| | 0.456 | 25% | 79% | 4 | 1.7 | 1.1 | 2.6 |
| | 1.73 | 25% | 81% | | | | |
| | 4.44 | 15% | 90% | | | | |
| 48 hours | 0 | 100% | 0% | 1 | | | |
| | 0 | 100% | 0% | 2 | 4.5 | 1.2 | 16.4 |
| | 0 | 100% | 0% | 3 | 2.1 | 0.4 | 9.7 |
| | 0.456 | 30% | 79% | 4 | 5.1 | 1.4 | 18.3 |
| | 1.73 | 30% | 81% | | | | |
| | 4.44 | 9% | 90% | | | | |

Fig. 5 - 22

Matrix metalloproteinase-3

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.040 | 0.059 | 0.040 | 0.055 | 0.040 | 0.052 |
| average | 0.124 | 0.143 | 0.124 | 0.138 | 0.124 | 0.199 |
| stdev | 0.214 | 0.246 | 0.214 | 0.296 | 0.214 | 0.448 |
| p (t-test) | | 0.561 | | 0.678 | | 0.140 |
| min | 0.002 | 0.002 | 0.002 | 0.002 | 0.002 | 0.002 |
| max | 1.490 | 1.390 | 1.490 | 2.100 | 1.490 | 2.160 |
| n (Samp) | 257 | 56 | 257 | 61 | 257 | 26 |
| n (Pat) | 112 | 56 | 112 | 61 | 112 | 26 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.055 | 0.058 | 0.055 | 0.045 | 0.055 | 0.057 |
| average | 0.162 | 0.162 | 0.162 | 0.101 | 0.162 | 0.150 |
| stdev | 0.303 | 0.212 | 0.303 | 0.183 | 0.303 | 0.250 |
| p (t-test) | | 0.997 | | 0.313 | | 0.883 |
| min | 0.002 | 0.002 | 0.002 | 0.002 | 0.002 | 0.002 |
| max | 2.160 | 0.732 | 2.160 | 0.927 | 2.160 | 0.853 |
| n (Samp) | 459 | 23 | 459 | 26 | 459 | 14 |
| n (Pat) | 180 | 23 | 180 | 26 | 180 | 14 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.043 | 0.062 | 0.043 | 0.055 | 0.043 | 0.039 |
| average | 0.111 | 0.142 | 0.111 | 0.148 | 0.111 | 0.194 |
| stdev | 0.181 | 0.244 | 0.181 | 0.317 | 0.181 | 0.470 |
| p (t-test) | | 0.310 | | 0.263 | | 0.092 |
| min | 0.002 | 0.002 | 0.002 | 0.002 | 0.002 | 0.002 |
| max | 1.190 | 1.390 | 1.190 | 2.100 | 1.190 | 2.160 |
| n (Samp) | 213 | 51 | 213 | 53 | 213 | 23 |
| n (Pat) | 89 | 51 | 89 | 53 | 89 | 23 |

sCr or UO

| Time prior AKI stage | AUC | SE | $n_{Cohort\ 1}$ | $n_{Cohort\ 2}$ | p |
|---|---|---|---|---|---|
| 0 hours | 0.55 | 0.043 | 257 | 56 | 0.272 |
| 24 hours | 0.55 | 0.042 | 257 | 61 | 0.257 |
| 48 hours | 0.54 | 0.061 | 257 | 26 | 0.484 |

sCr only

| Time prior AKI stage | AUC | SE | $n_{Cohort\ 1}$ | $n_{Cohort\ 2}$ | p |
|---|---|---|---|---|---|
| 0 hours | 0.53 | 0.063 | 459 | 23 | 0.614 |
| 24 hours | 0.48 | 0.058 | 459 | 26 | 0.736 |
| 48 hours | 0.51 | 0.079 | 459 | 14 | 0.946 |

UO only

| Time prior AKI stage | AUC | SE | $n_{Cohort\ 1}$ | $n_{Cohort\ 2}$ | p |
|---|---|---|---|---|---|
| 0 hours | 0.56 | 0.046 | 213 | 51 | 0.202 |
| 24 hours | 0.54 | 0.045 | 213 | 53 | 0.352 |
| 48 hours | 0.52 | 0.064 | 213 | 23 | 0.734 |

sCr or UO

| Time prior AKI stage | Cutoff value | sens | spec | Quartile | OR | 95% CI of OR | |
|---|---|---|---|---|---|---|---|
| 0 hours | 0.0228 | 71% | 44% | 1 | | | |
| | 0 | 100% | 0% | 2 | 1.1 | 0.7 | 1.7 |
| | 0 | 100% | 0% | 3 | 2.0 | 1.4 | 2.8 |

# Fig. 5 - 23

| | 0.105 | 34% | 70% | 4 | 1.3 | 0.9 | 1.9 |
|---|---|---|---|---|---|---|---|
| | 0.188 | 23% | 81% | | | | |
| | 0.315 | 9% | 90% | | | | |
| 24 hours | 0.0337 | 70% | 47% | 1 | | | |
| | 0 | 100% | 0% | 2 | 1.7 | 1.2 | 2.5 |
| | 0 | 100% | 0% | 3 | 2.3 | 1.6 | 3.3 |
| | 0.105 | 33% | 70% | 4 | 1.6 | 1.1 | 2.3 |
| | 0.188 | 16% | 81% | | | | |
| | 0.315 | 7% | 90% | | | | |
| 48 hours | 0.0165 | 73% | 42% | 1 | | | |
| | 0 | 100% | 0% | 2 | 12.7 | 1.4 | 114.1 |
| | 0 | 100% | 0% | 3 | 8.8 | 0.9 | 84.3 |
| | 0.105 | 35% | 70% | 4 | 6.4 | 0.6 | 66.5 |
| | 0.188 | 19% | 81% | | | | |
| | 0.315 | 12% | 90% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 10.7 | 1.2 | 95.8 |
| | 0 | 100% | 0% | 3 | 5.2 | 0.5 | 56.2 |
| | 0.135 | 35% | 70% | 4 | 7.3 | 0.8 | 71.0 |
| | 0.22 | 30% | 80% | | | | |
| | 0.422 | 9% | 90% | | | | |
| 24 hours | 0.0238 | 73% | 37% | 1 | | | |
| | 0 | 100% | 0% | 2 | 2.8 | 1.1 | 7.1 |
| | 0 | 100% | 0% | 3 | 4.8 | 2.1 | 11.0 |
| | 0.135 | 19% | 70% | 4 | 0.7 | 0.1 | 3.5 |
| | 0.22 | 8% | 80% | | | | |
| | 0.422 | 4% | 90% | | | | |
| 48 hours | 0.0296 | 71% | 41% | 1 | | | |
| | 0 | 100% | 0% | 2 | 6.3 | 0.6 | 63.8 |
| | 0 | 100% | 0% | 3 | 4.1 | 0.3 | 49.2 |
| | 0.135 | 29% | 70% | 4 | 3.0 | 0.2 | 42.7 |
| | 0.22 | 14% | 80% | | | | |
| | 0.422 | 14% | 90% | | | | |

UO only

| Time prior AKI stage | Cutoff value | sens | spec | Quartile | OR | 95% CI of OR | |
|---|---|---|---|---|---|---|---|
| 0 hours | 0.0238 | 73% | 42% | 1 | | | |
| | 0 | 100% | 0% | 2 | 4.2 | 2.4 | 7.6 |
| | 0 | 100% | 0% | 3 | 3.9 | 2.2 | 7.0 |
| | 0.104 | 33% | 70% | 4 | 3.0 | 1.6 | 5.5 |
| | 0.182 | 24% | 80% | | | | |
| | 0.302 | 10% | 90% | | | | |
| 24 hours | 0 | 100% | 0% | 1 | | | |
| | 0 | 100% | 0% | 2 | 8.9 | 3.9 | 20.4 |
| | 0 | 100% | 0% | 3 | 6.7 | 2.9 | 15.7 |
| | 0.104 | 34% | 70% | 4 | 5.5 | 2.3 | 13.1 |
| | 0.182 | 19% | 80% | | | | |
| | 0.302 | 13% | 90% | | | | |
| 48 hours | 0 | 100% | 0% | 1 | | | |
| | 0 | 100% | 0% | 2 | 0.7 | 0.3 | 1.5 |
| | 0 | 100% | 0% | 3 | 0.8 | 0.4 | 1.7 |
| | 0.104 | 35% | 70% | 4 | 0.7 | 0.3 | 1.5 |
| | 0.182 | 17% | 80% | | | | |
| | 0.302 | 9% | 90% | | | | |

Fig. 5 - 24

**Rantes (C-C motif chemokine 5)**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 10.100 | 7.210 | 10.100 | 6.870 | 10.100 | 6.450 |
| average | 13.834 | 10.469 | 13.834 | 10.217 | 13.834 | 10.827 |
| stdev | 13.443 | 8.619 | 13.443 | 11.762 | 13.443 | 11.965 |
| p (t-test) | | 0.074 | | 0.054 | | 0.273 |
| min | 0.254 | 0.146 | 0.254 | 0.165 | 0.254 | 1.260 |
| max | 83.000 | 31.500 | 83.000 | 74.600 | 83.000 | 53.400 |
| n (Samp) | 257 | 56 | 257 | 61 | 257 | 26 |
| n (Pat) | 112 | 56 | 112 | 61 | 112 | 26 |

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 8.890 | 6.450 | 8.890 | 6.735 | 8.890 | 7.630 |
| average | 12.850 | 10.627 | 12.850 | 9.849 | 12.850 | 9.918 |
| stdev | 13.265 | 11.763 | 13.265 | 9.539 | 13.265 | 6.951 |
| p (t-test) | | 0.431 | | 0.256 | | 0.411 |
| min | 0.146 | 0.312 | 0.146 | 0.601 | 0.146 | 1.580 |
| max | 83.000 | 52.000 | 83.000 | 39.000 | 83.000 | 22.600 |
| n (Samp) | 459 | 23 | 459 | 26 | 459 | 14 |
| n (Pat) | 180 | 23 | 180 | 26 | 180 | 14 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 9.730 | 7.330 | 9.730 | 7.600 | 9.730 | 6.110 |
| average | 14.083 | 9.819 | 14.083 | 10.871 | 14.083 | 10.710 |
| stdev | 13.819 | 8.326 | 13.819 | 12.068 | 13.819 | 12.553 |
| p (t-test) | | 0.036 | | 0.122 | | 0.263 |
| min | 0.438 | 0.146 | 0.438 | 0.165 | 0.438 | 1.260 |
| max | 83.000 | 31.500 | 83.000 | 74.600 | 83.000 | 53.400 |
| n (Samp) | 213 | 51 | 213 | 53 | 213 | 23 |
| n (Pat) | 89 | 51 | 89 | 53 | 89 | 23 |

sCr or UO

| Time prior AKI stage | AUC | SE | $n_{Cohort\ 1}$ | $n_{Cohort\ 2}$ | P |
|---|---|---|---|---|---|
| 0 hours | 0.43 | 0.041 | 257 | 56 | 0.069 |
| 24 hours | 0.39 | 0.038 | 257 | 61 | 0.004 |
| 48 hours | 0.40 | 0.055 | 257 | 26 | 0.068 |

sCr only

| Time prior AKI stage | AUC | SE | $n_{Cohort\ 1}$ | $n_{Cohort\ 2}$ | P |
|---|---|---|---|---|---|
| 0 hours | 0.44 | 0.059 | 459 | 23 | 0.281 |
| 24 hours | 0.43 | 0.055 | 459 | 26 | 0.205 |
| 48 hours | 0.48 | 0.077 | 459 | 14 | 0.764 |

UO only

| Time prior AKI stage | AUC | SE | $n_{Cohort\ 1}$ | $n_{Cohort\ 2}$ | P |
|---|---|---|---|---|---|
| 0 hours | 0.40 | 0.042 | 213 | 51 | 0.015 |
| 24 hours | 0.41 | 0.042 | 213 | 53 | 0.025 |
| 48 hours | 0.38 | 0.057 | 213 | 23 | 0.030 |

sCr or UO

| Time prior AKI stage | Cutoff value | sens | spec | Quartile | OR | 95% CI of OR | |
|---|---|---|---|---|---|---|---|
| 0 hours | 4.64 | 71% | 23% | 1 | | | |

## Fig. 5 - 25

| | 2.84 | 80% | 11% | 2 | 1.1 | 0.8 | 1.7 |
|---|---|---|---|---|---|---|---|
| | 1.1 | 91% | 4% | 3 | 1.5 | 1.0 | 2.1 |
| | 16.5 | 21% | 70% | 4 | 1.9 | 1.3 | 2.6 |
| | 20.5 | 16% | 80% | | | | |
| | 28.5 | 4% | 90% | | | | |
| 24 hours | 3.54 | 70% | 18% | 1 | | | |
| | 2.19 | 80% | 9% | 2 | 1.1 | 0.7 | 1.7 |
| | 1.72 | 90% | 8% | 3 | 2.2 | 1.5 | 3.1 |
| | 16.5 | 16% | 70% | 4 | 2.5 | 1.8 | 3.6 |
| | 20.5 | 13% | 80% | | | | |
| | 28.5 | 5% | 90% | | | | |
| 48 hours | 3.31 | 73% | 15% | 1 | | | |
| | 3.24 | 81% | 15% | 2 | 0.8 | 0.3 | 2.0 |
| | 2.04 | 92% | 9% | 3 | 1.4 | 0.7 | 3.0 |
| | 16.5 | 19% | 70% | 4 | 2.2 | 1.1 | 4.2 |
| | 20.5 | 12% | 80% | | | | |
| | 28.5 | 8% | 90% | | | | |

sCr only

| Time prior AKI stage | Cutoff value | sens | spec | Quartile | OR | 95% CI of OR | |
|---|---|---|---|---|---|---|---|
| 0 hours | 4.48 | 74% | 26% | 1 | | | |
| | 4.08 | 83% | 25% | 2 | 1.3 | 0.5 | 3.2 |
| | 1.43 | 91% | 8% | 3 | 2.4 | 1.1 | 4.9 |
| | 15.3 | 17% | 70% | 4 | 1.3 | 0.5 | 3.2 |
| | 19.4 | 17% | 80% | | | | |
| | 28.5 | 4% | 90% | | | | |
| 24 hours | 2.65 | 73% | 15% | 1 | | | |
| | 2.19 | 81% | 13% | 2 | 0.7 | 0.3 | 1.5 |
| | 1.76 | 92% | 10% | 3 | 1.2 | 0.6 | 2.3 |
| | 15.3 | 27% | 70% | 4 | 1.6 | 0.9 | 2.8 |
| | 19.4 | 8% | 80% | | | | |
| | 28.5 | 8% | 90% | | | | |
| 48 hours | 4.08 | 71% | 25% | 1 | | | |
| | 3.31 | 86% | 20% | 2 | 1.0 | 0.3 | 3.9 |
| | 3.25 | 93% | 20% | 3 | 1.4 | 0.4 | 4.4 |
| | 15.3 | 21% | 70% | 4 | 1.4 | 0.4 | 4.4 |
| | 19.4 | 14% | 80% | | | | |
| | 28.5 | 0% | 90% | | | | |

UO only

| Time prior AKI stage | Cutoff value | sens | spec | Quartile | OR | 95% CI of OR | |
|---|---|---|---|---|---|---|---|
| 0 hours | 3.08 | 71% | 10% | 1 | | | |
| | 2 | 80% | 5% | 2 | 1.3 | 0.8 | 2.0 |
| | 1.29 | 90% | 4% | 3 | 1.6 | 1.0 | 2.4 |
| | 17 | 18% | 71% | 4 | 2.4 | 1.6 | 3.5 |
| | 20.8 | 12% | 80% | | | | |
| | 29 | 2% | 91% | | | | |
| 24 hours | 4.33 | 72% | 19% | 1 | | | |
| | 2.66 | 81% | 9% | 2 | 0.8 | 0.5 | 1.3 |
| | 1.55 | 91% | 5% | 3 | 1.5 | 1.0 | 2.2 |
| | 17 | 21% | 71% | 4 | 1.9 | 1.3 | 2.7 |
| | 20.8 | 13% | 80% | | | | |
| | 29 | 4% | 91% | | | | |
| 48 hours | 3.24 | 74% | 13% | 1 | | | |
| | 2.93 | 83% | 10% | 2 | 0.7 | 0.2 | 2.5 |
| | 2.19 | 91% | 6% | 3 | 1.9 | 0.8 | 4.3 |
| | 17 | 17% | 71% | 4 | 2.5 | 1.1 | 5.4 |
| | 20.8 | 13% | 80% | | | | |
| | 29 | 9% | 91% | | | | |

Fig. 5 - 26

**Thrombospondin-1**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 2300.310 | 1372.807 | 2300.310 | 537.606 | 2300.310 | 177.557 |
| average | 2923.396 | 2015.930 | 2923.396 | 537.606 | 2923.396 | 426.377 |
| stdev | 2268.480 | 1919.081 | 2268.480 | 44.943 | 2268.480 | na |
| p (t-test) | | 0.187 | | 0.146 | | na |
| min | 177.557 | 455.607 | 177.557 | 505.826 | 177.557 | 426.377 |
| max | 9514.803 | 7179.276 | 9514.803 | 569.386 | 9514.803 | 426.377 |
| n (Samp) | 56 | 13 | 56 | 2 | 56 | 1 |
| n (Pat) | 37 | 13 | 37 | 2 | 37 | 1 |

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 2124.613 | 1368.421 | 2124.613 | 692.808 | 2124.613 | 1667.552 |
| average | 2716.908 | 1465.473 | 2716.908 | 638.674 | 2716.908 | 1667.552 |
| stdev | 2265.338 | 1036.558 | 2265.338 | 331.072 | 2265.338 | 854.354 |
| p (t-test) | | 0.184 | | 0.072 | | 0.517 |
| min | 177.557 | 338.785 | 177.557 | 205.966 | 177.557 | 1063.433 |
| max | 9514.803 | 3315.549 | 9514.803 | 963.115 | 9514.803 | 2271.672 |
| n (Samp) | 85 | 6 | 85 | 4 | 85 | 2 |
| n (Pat) | 60 | 6 | 60 | 4 | 60 | 2 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 2346.491 | 939.463 | 2346.491 | 3168.209 | 2346.491 | 473.131 |
| average | 3218.642 | 1586.047 | 3218.642 | 3168.209 | 3218.642 | 426.377 |
| stdev | 2405.965 | 1927.197 | 2405.965 | 3765.178 | 2405.965 | na |
| p (t-test) | | 0.026 | | 0.977 | | na |
| min | 473.131 | 286.458 | 473.131 | 505.826 | 473.131 | 426.377 |
| max | 9514.803 | 7179.276 | 9514.803 | 5830.592 | 9514.803 | 426.377 |
| n (Samp) | 41 | 14 | 41 | 2 | 41 | 1 |
| n (Pat) | 27 | 14 | 27 | 2 | 27 | 1 |

sCr or UO

| Time prior AKI stage | AUC | SE | $n_{Cohort 1}$ | $n_{Cohort 2}$ | p |
|---|---|---|---|---|---|
| 0 hours | 0.36 | 0.080 | 56 | 13 | 0.082 |
| 24 hours | 0.09 | 0.066 | 56 | 2 | 0.000 |
| 48 hours | 0.07 | 0.074 | 56 | 1 | 0.000 |

sCr only

| Time prior AKI stage | AUC | SE | $n_{Cohort 1}$ | $n_{Cohort 2}$ | p |
|---|---|---|---|---|---|
| 0 hours | 0.35 | 0.104 | 85 | 6 | 0.137 |
| 24 hours | 0.14 | 0.068 | 85 | 4 | 0.000 |
| 48 hours | 0.40 | 0.189 | 85 | 2 | 0.585 |

UO only

| Time prior AKI stage | AUC | SE | $n_{Cohort 1}$ | $n_{Cohort 2}$ | p |
|---|---|---|---|---|---|
| 0 hours | 0.22 | 0.065 | 41 | 14 | 0.000 |
| 24 hours | 0.41 | 0.197 | 41 | 2 | 0.665 |
| 48 hours | 0.00 | 0.000 | 41 | 1 | nd |

sCr or UO

| Time prior AKI stage | Cutoff value | sens | spec | Quartile | OR | 95% CI of OR |
|---|---|---|---|---|---|---|
| 0 hours | 694.963 | 77% | 16% | 1 | | |

## Fig. 5 - 27

|  | 508.178 | 85% | 9% | 2 | 1.1 | 0.1 | 9.8 |
|---|---|---|---|---|---|---|---|
|  | 473.131 | 92% | 9% | 3 | 3.3 | 0.6 | 17.5 |
|  | 3681.4 | 15% | 71% | 4 | 2.5 | 0.4 | 14.1 |
|  | 4629.93 | 8% | 80% |  |  |  |  |
|  | 6307.57 | 8% | 91% |  |  |  |  |
| 24 hours | 473.131 | 100% | 9% | 1 |  |  |  |
|  | 473.131 | 100% | 9% | 2 | na | na | na |
|  | 473.131 | 100% | 9% | 3 | na | na | na |
|  | 3681.4 | 0% | 71% | 4 | na | na | na |
|  | 4629.93 | 0% | 80% |  |  |  |  |
|  | 6307.57 | 0% | 91% |  |  |  |  |
| 48 hours | 373.411 | 100% | 7% | 1 |  |  |  |
|  | 373.411 | 100% | 7% | 2 | na | na | na |
|  | 373.411 | 100% | 7% | 3 | na | na | na |
|  | 3681.4 | 0% | 71% | 4 | na | na | na |
|  | 4629.93 | 0% | 80% |  |  |  |  |
|  | 6307.57 | 0% | 91% |  |  |  |  |

UO only

| Time prior AKI stage | Cutoff value | sens | spec | Quartile | OR | 95% CI of OR | |
|---|---|---|---|---|---|---|---|
| 0 hours | 473.131 | 71% | 2% | 1 |  |  |  |
|  | 315.148 | 86% | 0% | 2 | 0.5 | 0.0 | 12.0 |
|  | 286.458 | 93% | 0% | 3 | 2.4 | 0.4 | 15.0 |
|  | 3741.78 | 14% | 71% | 4 | 7.0 | 1.2 | 40.3 |
|  | 5693.6 | 7% | 80% |  |  |  |  |
|  | 6486.28 | 7% | 90% |  |  |  |  |
| 24 hours | 473.131 | 100% | 2% | 1 |  |  |  |
|  | 473.131 | 100% | 2% | 2 | 0.0 | 0.0 | na |
|  | 473.131 | 100% | 2% | 3 | 0.0 | 0.0 | na |
|  | 3741.78 | 50% | 71% | 4 | 1.1 | 0.0 | 84.7 |
|  | 5693.6 | 50% | 80% |  |  |  |  |
|  | 6486.28 | 0% | 90% |  |  |  |  |
| 48 hours | 0 | 100% | 0% | 1 |  |  |  |
|  | 0 | 100% | 0% | 2 | na | na | na |
|  | 0 | 100% | 0% | 3 | na | na | na |
|  | 3741.78 | 0% | 71% | 4 | na | na | na |
|  | 5693.6 | 0% | 80% |  |  |  |  |
|  | 6486.28 | 0% | 90% |  |  |  |  |

# Fig. 5 - 28

**ENA-78 (C-X-C chemokine motif 5)**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.640 | 0.365 | 0.640 | 0.389 | 0.640 | 0.351 |
| average | 1.120 | 0.969 | 1.120 | 0.741 | 1.120 | 0.784 |
| stdev | 1.987 | 1.192 | 1.987 | 0.945 | 1.987 | 1.383 |
| p (t-test) | | 0.691 | | 0.258 | | 0.479 |
| min | 0.001 | 0.031 | 0.001 | 0.091 | 0.001 | 0.033 |
| max | 32.500 | 4.240 | 32.500 | 5.380 | 32.500 | 6.040 |
| n (Samp) | 434 | 28 | 434 | 36 | 434 | 18 |
| n (Pat) | 173 | 28 | 173 | 36 | 173 | 18 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.628 | 0.328 | 0.628 | 0.431 | 0.628 | 0.363 |
| average | 1.065 | 1.119 | 1.065 | 0.568 | 1.065 | 0.651 |
| stdev | 1.836 | 1.617 | 1.836 | 0.467 | 1.836 | 0.544 |
| p (t-test) | | 0.944 | | 0.392 | | 0.551 |
| min | 0.001 | 0.130 | 0.001 | 0.147 | 0.001 | 0.092 |
| max | 32.500 | 4.240 | 32.500 | 1.600 | 32.500 | 1.450 |
| n (Samp) | 542 | 6 | 542 | 10 | 542 | 7 |
| n (Pat) | 208 | 6 | 208 | 10 | 208 | 7 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.643 | 0.360 | 0.643 | 0.389 | 0.643 | 0.352 |
| average | 1.123 | 0.819 | 1.123 | 0.798 | 1.123 | 0.864 |
| stdev | 2.119 | 1.022 | 2.119 | 1.027 | 2.119 | 1.456 |
| p (t-test) | | 0.461 | | 0.391 | | 0.629 |
| min | 0.001 | 0.031 | 0.001 | 0.091 | 0.001 | 0.033 |
| max | 32.500 | 4.170 | 32.500 | 5.380 | 32.500 | 6.040 |
| n (Samp) | 356 | 27 | 356 | 32 | 356 | 16 |
| n (Pat) | 138 | 27 | 138 | 32 | 138 | 16 |

sCr or UO

| Time prior AKI stage | AUC | SE | nCohort 1 | nCohort 2 | p |
|---|---|---|---|---|---|
| 0 hours | 0.43 | 0.053 | 434 | 28 | 0.177 |
| 24 hours | 0.41 | 0.046 | 434 | 36 | 0.045 |
| 48 hours | 0.35 | 0.059 | 434 | 18 | 0.012 |

sCr only

| Time prior AKI stage | AUC | SE | nCohort 1 | nCohort 2 | p |
|---|---|---|---|---|---|
| 0 hours | 0.43 | 0.112 | 542 | 6 | 0.528 |
| 24 hours | 0.39 | 0.083 | 542 | 10 | 0.200 |
| 48 hours | 0.43 | 0.103 | 542 | 7 | 0.468 |

UO only

| Time prior AKI stage | AUC | SE | nCohort 1 | nCohort 2 | p |
|---|---|---|---|---|---|
| 0 hours | 0.40 | 0.053 | 356 | 27 | 0.047 |
| 24 hours | 0.41 | 0.050 | 356 | 32 | 0.081 |
| 48 hours | 0.38 | 0.066 | 356 | 16 | 0.068 |

sCr or UO

| Time prior AKI stage | Cutoff value | sens | spec | Quartile | OR | 95% CI of OR | |
|---|---|---|---|---|---|---|---|
| 0 hours | 0.227 | 71% | 17% | 1 | | | |
| | 0.128 | 82% | 8% | 2 | 0.6 | 0.3 | 1.3 |
| | 0.0944 | 93% | 5% | 3 | 0.6 | 0.2 | 1.2 |

# Fig. 6 - 1

| Time prior AKI stage | Cutoff value | sens | spec | Quartile | OR | 95% CI of OR | |
|---|---|---|---|---|---|---|---|
| | 1.12 | 25% | 70% | 4 | 2.0 | 1.2 | 3.2 |
| | 1.56 | 18% | 80% | | | | |
| | 2.28 | 18% | 90% | | | | |
| 24 hours | 0.269 | 72% | 21% | 1 | | | |
| | 0.176 | 81% | 13% | 2 | 1.0 | 0.6 | 1.8 |
| | 0.137 | 92% | 9% | 3 | 1.6 | 1.0 | 2.7 |
| | 1.12 | 19% | 70% | 4 | 1.6 | 1.0 | 2.7 |
| | 1.56 | 11% | 80% | | | | |
| | 2.28 | 3% | 90% | | | | |
| 48 hours | 0.227 | 72% | 17% | 1 | | | |
| | 0.0848 | 83% | 4% | 2 | 1.0 | 0.3 | 3.8 |
| | 0.0585 | 94% | 3% | 3 | 1.7 | 0.6 | 5.0 |
| | 1.12 | 17% | 70% | 4 | 2.4 | 0.9 | 6.4 |
| | 1.56 | 6% | 80% | | | | |
| | 2.28 | 6% | 90% | | | | |

sCr only

| Time prior AKI stage | Cutoff value | sens | spec | Quartile | OR | 95% CI of OR | |
|---|---|---|---|---|---|---|---|
| 0 hours | 0.154 | 83% | 12% | 1 | | | |
| | 0.154 | 83% | 12% | 2 | 0.0 | 0.0 | na |
| | 0.128 | 100% | 9% | 3 | 1.0 | 0.1 | 7.3 |
| | 1.1 | 33% | 70% | 4 | 1.0 | 0.1 | 7.3 |
| | 1.49 | 33% | 80% | | | | |
| | 2.2 | 17% | 90% | | | | |
| 24 hours | 0.3 | 70% | 27% | 1 | | | |
| | 0.176 | 80% | 14% | 2 | 3.0 | 0.2 | 42.8 |
| | 0.149 | 90% | 11% | 3 | 3.0 | 0.2 | 42.8 |
| | 1.1 | 10% | 70% | 4 | 3.0 | 0.2 | 42.8 |
| | 1.49 | 10% | 80% | | | | |
| | 2.2 | 0% | 90% | | | | |
| 48 hours | 0.287 | 71% | 26% | 1 | | | |
| | 0.244 | 86% | 21% | 2 | 0.5 | 0.0 | 9.7 |
| | 0.0919 | 100% | 5% | 3 | 1.0 | 0.1 | 7.4 |
| | 1.1 | 29% | 70% | 4 | 1.0 | 0.1 | 7.4 |
| | 1.49 | 0% | 80% | | | | |
| | 2.2 | 0% | 90% | | | | |

UO only

| Time prior AKI stage | Cutoff value | sens | spec | Quartile | OR | 95% CI of OR | |
|---|---|---|---|---|---|---|---|
| 0 hours | 0.182 | 70% | 13% | 1 | | | |
| | 0.122 | 81% | 6% | 2 | 1.0 | 0.4 | 2.3 |
| | 0.0688 | 93% | 2% | 3 | 0.8 | 0.3 | 2.0 |
| | 1.12 | 19% | 71% | 4 | 2.9 | 1.6 | 5.2 |
| | 1.48 | 19% | 80% | | | | |
| | 2.2 | 15% | 90% | | | | |
| 24 hours | 0.269 | 72% | 21% | 1 | | | |
| | 0.175 | 81% | 13% | 2 | 0.8 | 0.4 | 1.6 |
| | 0.132 | 91% | 8% | 3 | 1.2 | 0.7 | 2.0 |
| | 1.12 | 22% | 71% | 4 | 1.6 | 1.0 | 2.7 |
| | 1.48 | 16% | 80% | | | | |
| | 2.2 | 6% | 90% | | | | |
| 48 hours | 0.227 | 75% | 17% | 1 | | | |
| | 0.0848 | 81% | 3% | 2 | 1.0 | 0.3 | 3.9 |
| | 0.0558 | 94% | 2% | 3 | 1.7 | 0.6 | 5.1 |
| | 1.12 | 19% | 71% | 4 | 1.7 | 0.6 | 5.1 |
| | 1.48 | 13% | 80% | | | | |
| | 2.2 | 6% | 90% | | | | |

Fig. 6 - 2

**Endoglin**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 3.980 | 3.958 | 3.980 | 4.855 | 3.980 | 3.532 |
| average | 4.015 | 3.896 | 4.015 | 6.320 | 4.015 | 3.532 |
| stdev | 1.122 | 1.207 | 1.122 | 4.292 | 1.122 | 0.798 |
| p (t-test) | | 0.765 | | 0.001 | | 0.548 |
| min | 1.904 | 2.646 | 1.904 | 3.229 | 1.904 | 2.968 |
| max | 7.699 | 6.760 | 7.699 | 12.339 | 7.699 | 4.097 |
| n (Samp) | 82 | 9 | 82 | 4 | 82 | 2 |
| n (Pat) | 59 | 9 | 59 | 4 | 59 | 2 |

sCr only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 3.899 | 3.738 | 3.899 | 4.847 | 3.899 | 4.210 |
| average | 4.033 | 3.738 | 4.033 | 4.847 | 4.033 | 4.210 |
| stdev | 1.421 | 0.775 | 1.421 | 2.289 | 1.421 | 1.756 |
| p (t-test) | | 0.771 | | 0.428 | | 0.863 |
| min | 1.904 | 3.190 | 1.904 | 3.229 | 1.904 | 2.968 |
| max | 12.339 | 4.287 | 12.339 | 6.466 | 12.339 | 5.452 |
| n (Samp) | 101 | 2 | 101 | 2 | 101 | 2 |
| n (Pat) | 71 | 2 | 71 | 2 | 71 | 2 |

UO only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 3.980 | 3.958 | 3.980 | 7.792 | 3.980 | 1.912 |
| average | 4.010 | 3.896 | 4.010 | 7.792 | 4.010 | 4.097 |
| stdev | 1.076 | 1.207 | 1.076 | 6.431 | 1.076 | na |
| p (t-test) | | 0.769 | | 0.000 | | na |
| min | 1.912 | 2.646 | 1.912 | 3.245 | 1.912 | 4.097 |
| max | 6.988 | 6.760 | 6.988 | 12.339 | 6.988 | 4.097 |
| n (Samp) | 64 | 9 | 64 | 2 | 64 | 1 |
| n (Pat) | 46 | 9 | 46 | 2 | 46 | 1 |

sCr or UO

| Time prior AKI stage | AUC | SE | nCohort 1 | nCohort 2 | p |
|---|---|---|---|---|---|
| 0 hours | 0.43 | 0.097 | 82 | 9 | 0.462 |
| 24 hours | 0.62 | 0.154 | 82 | 4 | 0.427 |
| 48 hours | 0.36 | 0.179 | 82 | 2 | 0.433 |

sCr only

| Time prior AKI stage | AUC | SE | nCohort 1 | nCohort 2 | p |
|---|---|---|---|---|---|
| 0 hours | 0.44 | 0.197 | 101 | 2 | 0.763 |
| 24 hours | 0.61 | 0.215 | 101 | 2 | 0.596 |
| 48 hours | 0.55 | 0.213 | 101 | 2 | 0.798 |

UO only

| Time prior AKI stage | AUC | SE | nCohort 1 | nCohort 2 | p |
|---|---|---|---|---|---|
| 0 hours | 0.43 | 0.098 | 64 | 9 | 0.464 |
| 24 hours | 0.63 | 0.216 | 64 | 2 | 0.539 |
| 48 hours | 0.52 | 0.296 | 64 | 1 | 0.958 |

sCr or UO

| Time prior AKI stage | Cutoff value | sens | spec | Quartile | OR | 95% CI of OR |
|---|---|---|---|---|---|---|

**Fig. 6 - 3**

| Time prior AKI stage | Cutoff value | sens | spec | Quartile | OR | 95% CI of OR | |
|---|---|---|---|---|---|---|---|
| 0 hours | 3.2592 | 78% | 27% | 1 | | | |
| | 2.67149 | 89% | 13% | 2 | 3.3 | 0.2 | 54.3 |
| | 2.64469 | 100% | 12% | 3 | 3.3 | 0.2 | 54.3 |
| | 4.60123 | 11% | 71% | 4 | 2.2 | 0.1 | 50.2 |
| | 4.76227 | 11% | 80% | | | | |
| | 5.0502 | 11% | 90% | | | | |
| 24 hours | 3.22853 | 75% | 26% | 1 | | | |
| | 3.18596 | 100% | 26% | 2 | na | na | na |
| | 3.18596 | 100% | 26% | 3 | na | na | na |
| | 4.60123 | 50% | 71% | 4 | na | na | na |
| | 4.76227 | 50% | 80% | | | | |
| | 5.0502 | 50% | 90% | | | | |
| 48 hours | 2.93944 | 100% | 18% | 1 | | | |
| | 2.93944 | 100% | 18% | 2 | na | na | na |
| | 2.93944 | 100% | 18% | 3 | na | na | na |
| | 4.60123 | 0% | 71% | 4 | na | na | na |
| | 4.76227 | 0% | 80% | | | | |
| | 5.0502 | 0% | 90% | | | | |

sCr only

| Time prior AKI stage | Cutoff value | sens | spec | Quartile | OR | 95% CI of OR | |
|---|---|---|---|---|---|---|---|
| 0 hours | 3.18596 | 100% | 28% | 1 | | | |
| | 3.18596 | 100% | 28% | 2 | na | na | na |
| | 3.18596 | 100% | 28% | 3 | na | na | na |
| | 4.47853 | 0% | 70% | 4 | na | na | na |
| | 4.71626 | 0% | 80% | | | | |
| | 5.3012 | 0% | 90% | | | | |
| 24 hours | 3.18596 | 100% | 28% | 1 | | | |
| | 3.18596 | 100% | 28% | 2 | na | na | na |
| | 3.18596 | 100% | 28% | 3 | na | na | na |
| | 4.47853 | 50% | 70% | 4 | na | na | na |
| | 4.71626 | 50% | 80% | | | | |
| | 5.3012 | 50% | 90% | | | | |
| 48 hours | 2.93944 | 100% | 20% | 1 | | | |
| | 2.93944 | 100% | 20% | 2 | 0.0 | 0.0 | na |
| | 2.93944 | 100% | 20% | 3 | 0.0 | 0.0 | na |
| | 4.47853 | 50% | 70% | 4 | 1.0 | 0.0 | 56.8 |
| | 4.71626 | 50% | 80% | | | | |
| | 5.3012 | 50% | 90% | | | | |

UO only

| Time prior AKI stage | Cutoff value | sens | spec | Quartile | OR | 95% CI of OR | |
|---|---|---|---|---|---|---|---|
| 0 hours | 3.2592 | 78% | 28% | 1 | | | |
| | 2.67149 | 89% | 13% | 2 | 3.6 | 0.2 | 62.4 |
| | 2.56967 | 100% | 11% | 3 | 3.6 | 0.2 | 62.4 |
| | 4.60123 | 11% | 70% | 4 | 2.3 | 0.1 | 53.6 |
| | 4.76227 | 11% | 81% | | | | |
| | 5.3012 | 11% | 91% | | | | |
| 24 hours | 3.18596 | 100% | 27% | 1 | | | |
| | 3.18596 | 100% | 27% | 2 | na | na | na |
| | 3.18596 | 100% | 27% | 3 | na | na | na |
| | 4.60123 | 50% | 70% | 4 | na | na | na |
| | 4.76227 | 50% | 81% | | | | |
| | 5.3012 | 50% | 91% | | | | |
| 48 hours | 3.9954 | 100% | 52% | 1 | | | |
| | 3.9954 | 100% | 52% | 2 | na | na | na |
| | 3.9954 | 100% | 52% | 3 | na | na | na |
| | 4.60123 | 0% | 70% | 4 | na | na | na |
| | 4.76227 | 0% | 81% | | | | |
| | 5.3012 | 0% | 91% | | | | |

Fig. 6 - 4

**Erythropoietin**

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 219.000 | 374.000 | 219.000 | 299.000 | 219.000 | 161.000 |
| average | 489.633 | 620.336 | 489.633 | 460.760 | 489.633 | 478.387 |
| stdev | 985.760 | 806.904 | 985.760 | 584.786 | 985.760 | 992.783 |
| p (t-test) | | 0.493 | | 0.863 | | 0.962 |
| min | 1.660 | 1.660 | 1.660 | 1.660 | 1.660 | 1.660 |
| max | 11600.000 | 3710.000 | 11600.000 | 2580.000 | 11600.000 | 4320.000 |
| n (Samp) | 434 | 28 | 434 | 36 | 434 | 18 |
| n (Pat) | 173 | 28 | 173 | 36 | 173 | 18 |

sCr only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 225.000 | 129.850 | 225.000 | 214.000 | 225.000 | 173.000 |
| average | 494.826 | 5873.793 | 494.826 | 396.220 | 494.826 | 487.671 |
| stdev | 944.786 | 13545.581 | 944.786 | 531.073 | 944.786 | 655.452 |
| p (t-test) | | 0.000 | | 0.742 | | 0.984 |
| min | 1.660 | 1.660 | 1.660 | 24.000 | 1.660 | 46.400 |
| max | 11600.000 | 33500.000 | 11600.000 | 1820.000 | 11600.000 | 1780.000 |
| n (Samp) | 542 | 6 | 542 | 10 | 542 | 7 |
| n (Pat) | 208 | 6 | 208 | 10 | 208 | 7 |

UO only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 219.500 | 370.000 | 219.500 | 302.500 | 219.500 | 173.000 |
| average | 518.475 | 607.247 | 518.475 | 522.536 | 518.475 | 494.620 |
| stdev | 1050.182 | 794.588 | 1050.182 | 632.671 | 1050.182 | 1041.147 |
| p (t-test) | | 0.668 | | 0.983 | | 0.929 |
| min | 1.660 | 1.660 | 1.660 | 1.660 | 1.660 | 1.660 |
| max | 11600.000 | 3710.000 | 11600.000 | 2580.000 | 11600.000 | 4320.000 |
| n (Samp) | 356 | 27 | 356 | 32 | 356 | 16 |
| n (Pat) | 138 | 27 | 138 | 32 | 138 | 16 |

sCr or UO

| Time prior AKI stage | AUC | SE | nCohort 1 | nCohort 2 | p |
| --- | --- | --- | --- | --- | --- |
| 0 hours | 0.58 | 0.058 | 434 | 28 | 0.179 |
| 24 hours | 0.54 | 0.051 | 434 | 36 | 0.441 |
| 48 hours | 0.47 | 0.068 | 434 | 18 | 0.670 |

sCr only

| Time prior AKI stage | AUC | SE | nCohort 1 | nCohort 2 | p |
| --- | --- | --- | --- | --- | --- |
| 0 hours | 0.46 | 0.115 | 542 | 6 | 0.743 |
| 24 hours | 0.51 | 0.093 | 542 | 10 | 0.933 |
| 48 hours | 0.51 | 0.111 | 542 | 7 | 0.937 |

UO only

| Time prior AKI stage | AUC | SE | nCohort 1 | nCohort 2 | p |
| --- | --- | --- | --- | --- | --- |
| 0 hours | 0.59 | 0.059 | 356 | 27 | 0.138 |
| 24 hours | 0.55 | 0.055 | 356 | 32 | 0.338 |
| 48 hours | 0.46 | 0.072 | 356 | 16 | 0.600 |

sCr or UO

| Time prior AKI stage | Cutoff value | sens | spec | Quartile | OR | 95% CI of OR |
| --- | --- | --- | --- | --- | --- | --- |

Fig. 6 - 5

| | | | | | | 95% CI of OR | |
|---|---|---|---|---|---|---|---|
| 0 hours | 123 | 71% | 36% | 1 | | | |
| | 70.9 | 82% | 26% | 2 | 1.2 | 0.6 | 2.6 |
| | 19.8 | 93% | 11% | 3 | 1.4 | 0.7 | 2.9 |
| | 416 | 43% | 70% | 4 | 2.1 | 1.1 | 3.9 |
| | 609 | 32% | 80% | | | | |
| | 1050 | 21% | 90% | | | | |
| 24 hours | 93 | 72% | 30% | 1 | | | |
| | 81.1 | 81% | 29% | 2 | 1.7 | 1.0 | 3.0 |
| | 24 | 92% | 15% | 3 | 2.1 | 1.2 | 3.6 |
| | 416 | 36% | 70% | 4 | 1.3 | 0.7 | 2.5 |
| | 609 | 17% | 80% | | | | |
| | 1050 | 17% | 90% | | | | |
| 48 hours | 94.2 | 72% | 31% | 1 | | | |
| | 44.7 | 83% | 20% | 2 | 0.7 | 0.2 | 2.4 |
| | 15.7 | 94% | 11% | 3 | 1.5 | 0.7 | 3.6 |
| | 416 | 28% | 70% | 4 | 1.3 | 0.5 | 3.2 |
| | 609 | 11% | 80% | | | | |
| | 1050 | 6% | 90% | | | | |

sCr only

| Time prior AKI stage | Cutoff value | sens | spec | Quartile | OR | 95% CI of OR | |
|---|---|---|---|---|---|---|---|
| 0 hours | 19.8 | 83% | 11% | 1 | | | |
| | 19.8 | 83% | 11% | 2 | 0.0 | 0.0 | na |
| | 0 | 100% | 0% | 3 | 1.0 | 0.1 | 7.3 |
| | 422 | 33% | 70% | 4 | 1.0 | 0.1 | 7.3 |
| | 630 | 33% | 80% | | | | |
| | 1120 | 33% | 90% | | | | |
| 24 hours | 123 | 70% | 35% | 1 | | | |
| | 93 | 80% | 30% | 2 | 4.1 | 0.3 | 48.8 |
| | 75 | 90% | 26% | 3 | 3.0 | 0.2 | 42.8 |
| | 422 | 30% | 70% | 4 | 2.0 | 0.1 | 39.2 |
| | 630 | 10% | 80% | | | | |
| | 1120 | 10% | 90% | | | | |
| 48 hours | 148 | 71% | 39% | 1 | | | |
| | 60.6 | 86% | 24% | 2 | 1.5 | 0.3 | 8.0 |
| | 44.7 | 100% | 19% | 3 | 0.0 | 0.0 | na |
| | 422 | 29% | 70% | 4 | 1.0 | 0.1 | 7.3 |
| | 630 | 29% | 80% | | | | |
| | 1120 | 14% | 90% | | | | |

UO only

| Time prior AKI stage | Cutoff value | sens | spec | Quartile | OR | 95% CI of OR | |
|---|---|---|---|---|---|---|---|
| 0 hours | 147 | 70% | 40% | 1 | | | |
| | 112 | 81% | 33% | 2 | 2.4 | 0.9 | 6.4 |
| | 32.5 | 93% | 15% | 3 | 3.2 | 1.3 | 7.9 |
| | 466 | 37% | 71% | 4 | 2.8 | 1.1 | 7.2 |
| | 651 | 26% | 80% | | | | |
| | 1120 | 19% | 90% | | | | |
| 24 hours | 147 | 72% | 40% | 1 | | | |
| | 84 | 81% | 28% | 2 | 1.7 | 0.8 | 3.3 |
| | 30.5 | 91% | 15% | 3 | 2.6 | 1.4 | 4.7 |
| | 466 | 25% | 71% | 4 | 1.4 | 0.7 | 2.9 |
| | 651 | 22% | 80% | | | | |
| | 1120 | 19% | 90% | | | | |
| 48 hours | 94.2 | 75% | 30% | 1 | | | |
| | 32.5 | 81% | 15% | 2 | 2.6 | 0.6 | 10.7 |
| | 0 | 100% | 0% | 3 | 2.6 | 0.6 | 10.7 |
| | 466 | 31% | 71% | 4 | 2.0 | 0.5 | 9.3 |
| | 651 | 6% | 80% | | | | |
| | 1120 | 6% | 90% | | | | |

# Fig. 6 - 6

**Fractalkine**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.554 | 0.745 | 0.554 | 0.591 | 0.554 | 0.525 |
| average | 0.711 | 0.836 | 0.711 | 0.789 | 0.711 | 0.640 |
| stdev | 0.735 | 0.517 | 0.735 | 0.606 | 0.735 | 0.461 |
| p (t-test) | | 0.531 | | 0.575 | | 0.787 |
| min | 0.002 | 0.066 | 0.002 | 0.066 | 0.002 | 0.254 |
| max | 7.305 | 1.848 | 7.305 | 2.556 | 7.305 | 1.712 |
| n (Samp) | 225 | 14 | 225 | 30 | 225 | 8 |
| n (Pat) | 147 | 14 | 147 | 30 | 147 | 8 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.567 | 3.842 | 0.567 | 1.587 | 0.567 | 0.683 |
| average | 0.714 | 3.842 | 0.714 | 1.566 | 0.714 | 0.963 |
| stdev | 0.691 | 0.041 | 0.691 | 1.049 | 0.691 | 0.872 |
| p (t-test) | | 0.000 | | 0.001 | | 0.476 |
| min | 0.002 | 3.813 | 0.002 | 0.293 | 0.002 | 0.254 |
| max | 7.305 | 3.871 | 7.305 | 2.709 | 7.305 | 2.233 |
| n (Samp) | 283 | 2 | 283 | 8 | 283 | 4 |
| n (Pat) | 178 | 2 | 178 | 8 | 178 | 4 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.566 | 0.745 | 0.566 | 0.516 | 0.566 | 0.483 |
| average | 0.670 | 0.836 | 0.670 | 0.677 | 0.670 | 0.700 |
| stdev | 0.632 | 0.517 | 0.632 | 0.434 | 0.632 | 0.572 |
| p (t-test) | | 0.340 | | 0.959 | | 0.917 |
| min | 0.002 | 0.066 | 0.002 | 0.066 | 0.002 | 0.344 |
| max | 7.305 | 1.848 | 7.305 | 2.036 | 7.305 | 1.712 |
| n (Samp) | 183 | 14 | 183 | 25 | 183 | 5 |
| n (Pat) | 120 | 14 | 120 | 25 | 120 | 5 |

sCr or UO

| Time prior AKI stage | AUC | SE | nCohort 1 | nCohort 2 | p |
|---|---|---|---|---|---|
| 0 hours | 0.62 | 0.082 | 225 | 14 | 0.147 |
| 24 hours | 0.55 | 0.057 | 225 | 30 | 0.390 |
| 48 hours | 0.49 | 0.103 | 225 | 8 | 0.906 |

sCr only

| Time prior AKI stage | AUC | SE | nCohort 1 | nCohort 2 | p |
|---|---|---|---|---|---|
| 0 hours | 0.99 | 0.042 | 283 | 2 | 0.000 |
| 24 hours | 0.74 | 0.102 | 283 | 8 | 0.016 |
| 48 hours | 0.59 | 0.151 | 283 | 4 | 0.546 |

UO only

| Time prior AKI stage | AUC | SE | nCohort 1 | nCohort 2 | p |
|---|---|---|---|---|---|
| 0 hours | 0.62 | 0.083 | 183 | 14 | 0.141 |
| 24 hours | 0.51 | 0.062 | 183 | 25 | 0.838 |
| 48 hours | 0.48 | 0.130 | 183 | 5 | 0.896 |

sCr or UO

| Time prior AKI stage | Cutoff value | sens | spec | Quartile | OR | 95% CI of OR |
|---|---|---|---|---|---|---|

## Fig. 6 - 7

| | 0.58382 | 71% | 55% | 1 | | | |
| 0 hours | 0.46512 | 86% | 38% | 2 | 0.5 | 0.0 | 9.8 |
| | 0.24414 | 93% | 18% | 3 | 3.8 | 1.0 | 14.3 |
| | 0.78 | 36% | 70% | 4 | 2.0 | 0.4 | 9.5 |
| | 0.99038 | 29% | 80% | | | | |
| | 1.34 | 14% | 90% | | | | |
| 24 hours | 0.42969 | 70% | 34% | 1 | | | |
| | 0.42151 | 80% | 34% | 2 | 2.1 | 1.1 | 4.1 |
| | 0.24414 | 93% | 18% | 3 | 1.4 | 0.7 | 3.0 |
| | 0.78 | 37% | 70% | 4 | 1.7 | 0.8 | 3.4 |
| | 0.99038 | 23% | 80% | | | | |
| | 1.34 | 13% | 90% | | | | |
| 48 hours | 0.38281 | 75% | 32% | 1 | | | |
| | 0.34198 | 88% | 27% | 2 | 3.2 | 0.2 | 46.3 |
| | 0.24414 | 100% | 18% | 3 | 3.2 | 0.2 | 46.3 |
| | 0.78 | 13% | 70% | 4 | 1.0 | 0.0 | 54.9 |
| | 0.99038 | 13% | 80% | | | | |
| | 1.34 | 13% | 90% | | | | |

sCr only

| Time prior AKI stage | Cutoff value | sens | spec | Quartile | OR | 95% CI of OR | |
|---|---|---|---|---|---|---|---|
| 0 hours | 3.74782 | 100% | 99% | 1 | | | |
| | 3.74782 | 100% | 99% | 2 | na | na | na |
| | 3.74782 | 100% | 99% | 3 | na | na | na |
| | 0.79 | 100% | 71% | 4 | na | na | na |
| | 1.00578 | 100% | 80% | | | | |
| | 1.34 | 100% | 90% | | | | |
| 24 hours | 0.79808 | 75% | 71% | 1 | | | |
| | 0.4717 | 88% | 39% | 2 | 1.0 | 0.0 | 52.5 |
| | 0.2873 | 100% | 20% | 3 | 2.0 | 0.1 | 40.0 |
| | 0.79 | 75% | 71% | 4 | 4.1 | 0.3 | 50.4 |
| | 1.00578 | 50% | 80% | | | | |
| | 1.34 | 50% | 90% | | | | |
| 48 hours | 0.62258 | 75% | 55% | 1 | | | |
| | 0.24698 | 100% | 17% | 2 | 0.0 | 0.0 | na |
| | 0.24698 | 100% | 17% | 3 | 2.0 | 0.1 | 40.0 |
| | 0.79 | 25% | 71% | 4 | 1.0 | 0.0 | 52.5 |
| | 1.00578 | 25% | 80% | | | | |
| | 1.34 | 25% | 90% | | | | |

UO only

| Time prior AKI stage | Cutoff value | sens | spec | Quartile | OR | 95% CI of OR | |
|---|---|---|---|---|---|---|---|
| 0 hours | 0.58382 | 71% | 54% | 1 | | | |
| | 0.46512 | 86% | 36% | 2 | 0.5 | 0.0 | 10.1 |
| | 0.24414 | 93% | 15% | 3 | 3.3 | 0.8 | 13.2 |
| | 0.78 | 36% | 70% | 4 | 2.6 | 0.6 | 11.2 |
| | 0.94275 | 29% | 80% | | | | |
| | 1.20192 | 14% | 90% | | | | |
| 24 hours | 0.42453 | 72% | 32% | 1 | | | |
| | 0.42151 | 80% | 31% | 2 | 2.9 | 1.3 | 6.2 |
| | 0.24414 | 92% | 15% | 3 | 1.3 | 0.5 | 3.3 |
| | 0.78 | 28% | 70% | 4 | 1.6 | 0.6 | 3.9 |
| | 0.94275 | 20% | 80% | | | | |
| | 1.20192 | 8% | 90% | | | | |
| 48 hours | 0.38281 | 80% | 29% | 1 | | | |
| | 0.38281 | 80% | 29% | 2 | 1.0 | 0.0 | 54.9 |
| | 0.33594 | 100% | 23% | 3 | 2.0 | 0.1 | 42.2 |
| | 0.78 | 20% | 70% | 4 | 1.0 | 0.0 | 54.9 |
| | 0.94275 | 20% | 80% | | | | |
| | 1.20192 | 20% | 90% | | | | |

Fig. 6 - 8

**Interleukin-1 receptor, type II**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 10382.346 | na | 10382.346 | 12731.082 | 10382.346 | 3061.967 |
| average | 13243.107 | na | 13243.107 | 16725.373 | 13243.107 | 9971.162 |
| stdev | 10029.455 | na | 10029.455 | 12373.037 | 10029.455 | na |
| p (t-test) | | na | | 0.299 | | na |
| min | 3061.967 | na | 3061.967 | 2160.038 | 3061.967 | 9971.162 |
| max | 56549.592 | na | 56549.592 | 44711.687 | 56549.592 | 9971.162 |
| n (Samp) | 46 | 0 | 46 | 13 | 46 | 1 |
| n (Pat) | 43 | 0 | 43 | 13 | 43 | 1 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 10772.340 | na | 10772.340 | 44711.687 | 10772.340 | na |
| average | 13600.425 | na | 13600.425 | 67876.354 | 13600.425 | na |
| stdev | 9798.214 | na | 9798.214 | 74275.199 | 9798.214 | na |
| p (t-test) | | na | | 0.000 | | na |
| min | 2160.038 | na | 2160.038 | 7943.978 | 2160.038 | na |
| max | 56549.592 | na | 56549.592 | 150973.398 | 56549.592 | na |
| n (Samp) | 59 | 0 | 59 | 3 | 59 | 0 |
| n (Pat) | 54 | 0 | 54 | 3 | 54 | 0 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 10193.935 | na | 10193.935 | 12731.082 | 10193.935 | 3061.967 |
| average | 13202.150 | na | 13202.150 | 14876.615 | 13202.150 | 9971.162 |
| stdev | 10511.533 | na | 10511.533 | 9242.384 | 10511.533 | na |
| p (t-test) | | na | | 0.610 | | na |
| min | 3061.967 | na | 3061.967 | 2160.038 | 3061.967 | 9971.162 |
| max | 56549.592 | na | 56549.592 | 36344.165 | 56549.592 | 9971.162 |
| n (Samp) | 40 | 0 | 40 | 13 | 40 | 1 |
| n (Pat) | 37 | 0 | 37 | 13 | 37 | 1 |

sCr or UO

| Time prior AKI stage | AUC | SE | nCohort 1 | nCohort 2 | p |
|---|---|---|---|---|---|
| 0 hours | nd | nd | 46 | 0 | nd |
| 24 hours | 0.60 | 0.092 | 46 | 13 | 0.278 |
| 48 hours | 0.46 | 0.285 | 46 | 1 | 0.879 |

sCr only

| Time prior AKI stage | AUC | SE | nCohort 1 | nCohort 2 | p |
|---|---|---|---|---|---|
| 0 hours | nd | nd | 59 | 0 | nd |
| 24 hours | 0.73 | 0.169 | 59 | 3 | 0.166 |
| 48 hours | nd | nd | 59 | 0 | nd |

UO only

| Time prior AKI stage | AUC | SE | nCohort 1 | nCohort 2 | p |
|---|---|---|---|---|---|
| 0 hours | nd | nd | 40 | 0 | nd |
| 24 hours | 0.61 | 0.094 | 40 | 13 | 0.250 |
| 48 hours | 0.48 | 0.291 | 40 | 1 | 0.931 |

sCr or UO

| Time prior AKI stage | Cutoff value | sens | spec | Quartile | OR | 95% CI of OR |
|---|---|---|---|---|---|---|

## Fig. 6 - 9

| 0 hours | na | na | na | 1 | | | |
|---|---|---|---|---|---|---|---|
| | na | na | na | 2 | na | na | na |
| | na | na | na | 3 | na | na | na |
| | na | na | na | 4 | na | na | na |
| | na | na | na | | | | |
| | na | na | na | | | | |
| 24 hours | 10696.1 | 77% | 54% | 1 | | | |
| | 5139.25 | 85% | 4% | 2 | 0.3 | 0.0 | 4.9 |
| | 5058.64 | 92% | 4% | 3 | 1.3 | 0.3 | 6.0 |
| | 12350.1 | 54% | 72% | 4 | 1.8 | 0.4 | 7.6 |
| | 16362.7 | 38% | 80% | | | | |
| | 25004.5 | 15% | 91% | | | | |
| 48 hours | 9927.72 | 100% | 46% | 1 | | | |
| | 9927.72 | 100% | 46% | 2 | na | na | na |
| | 9927.72 | 100% | 46% | 3 | na | na | na |
| | 12350.1 | 0% | 72% | 4 | na | na | na |
| | 16362.7 | 0% | 80% | | | | |
| | 25004.5 | 0% | 91% | | | | |

sCr only

| Time prior AKI stage | Cutoff value | sens | spec | Quartile | OR | 95% CI of OR | |
|---|---|---|---|---|---|---|---|
| 0 hours | na | na | na | 1 | | | |
| | na | na | na | 2 | na | na | na |
| | na | na | na | 3 | na | na | na |
| | na | na | na | 4 | na | na | na |
| | na | na | na | | | | |
| | na | na | na | | | | |
| 24 hours | 7911.48 | 100% | 24% | 1 | | | |
| | 7911.48 | 100% | 24% | 2 | 0.0 | 0.0 | na |
| | 7911.48 | 100% | 24% | 3 | 0.0 | 0.0 | na |
| | 14290 | 67% | 71% | 4 | 2.0 | 0.1 | 50.1 |
| | 18360.7 | 67% | 81% | | | | |
| | 25004.5 | 67% | 92% | | | | |
| 48 hours | na | na | na | 1 | | | |
| | na | na | na | 2 | na | na | na |
| | na | na | na | 3 | na | na | na |
| | na | na | na | 4 | na | na | na |
| | na | na | na | | | | |
| | na | na | na | | | | |

UO only

| Time prior AKI stage | Cutoff value | sens | spec | Quartile | OR | 95% CI of OR | |
|---|---|---|---|---|---|---|---|
| 0 hours | na | na | na | 1 | | | |
| | na | na | na | 2 | na | na | na |
| | na | na | na | 3 | na | na | na |
| | na | na | na | 4 | na | na | na |
| | na | na | na | | | | |
| | na | na | na | | | | |
| 24 hours | 10696.1 | 77% | 58% | 1 | | | |
| | 5139.25 | 85% | 5% | 2 | 0.0 | 0.0 | na |
| | 5058.64 | 92% | 5% | 3 | 1.5 | 0.3 | 7.0 |
| | 11758.4 | 54% | 70% | 4 | 2.5 | 0.6 | 10.4 |
| | 14290 | 46% | 80% | | | | |
| | 19122.3 | 31% | 90% | | | | |
| 48 hours | 9927.72 | 100% | 48% | 1 | | | |
| | 9927.72 | 100% | 48% | 2 | na | na | na |
| | 9927.72 | 100% | 48% | 3 | na | na | na |
| | 11758.4 | 0% | 70% | 4 | na | na | na |
| | 14290 | 0% | 80% | | | | |
| | 19122.3 | 0% | 90% | | | | |

# Fig. 6 - 10

Lymphotoxin-alpha

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.456 | 0.456 | 0.456 | 0.456 | 0.456 | 0.456 |
| average | 2.359 | 2.533 | 2.359 | 2.094 | 2.359 | 1.732 |
| stdev | 16.238 | 7.735 | 16.238 | 3.553 | 16.238 | 2.077 |
| p (t-test) | | 0.955 | | 0.922 | | 0.870 |
| min | 0.456 | 0.456 | 0.456 | 0.456 | 0.456 | 0.456 |
| max | 330.000 | 40.700 | 330.000 | 18.700 | 330.000 | 6.220 |
| n (Samp) | 434 | 28 | 434 | 36 | 434 | 18 |
| n (Pat) | 173 | 28 | 173 | 36 | 173 | 18 |

sCr only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 0.456 | 0.456 | 0.456 | 0.456 | 0.456 | 2.140 |
| average | 2.127 | 7.926 | 2.127 | 3.500 | 2.127 | 2.814 |
| stdev | 14.562 | 16.160 | 14.562 | 5.729 | 14.562 | 2.919 |
| p (t-test) | | 0.333 | | 0.766 | | 0.901 |
| min | 0.456 | 0.456 | 0.456 | 0.456 | 0.456 | 0.456 |
| max | 330.000 | 40.700 | 330.000 | 18.700 | 330.000 | 7.530 |
| n (Samp) | 542 | 6 | 542 | 10 | 542 | 7 |
| n (Pat) | 208 | 6 | 208 | 10 | 208 | 7 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 0.456 | 0.456 | 0.456 | 0.456 | 0.456 | 0.456 |
| average | 2.543 | 1.120 | 2.543 | 1.549 | 2.543 | 3.818 |
| stdev | 17.791 | 2.008 | 17.791 | 2.080 | 17.791 | 10.005 |
| p (t-test) | | 0.678 | | 0.753 | | 0.776 |
| min | 0.456 | 0.456 | 0.456 | 0.456 | 0.456 | 0.456 |
| max | 330.000 | 8.820 | 330.000 | 7.530 | 330.000 | 40.700 |
| n (Samp) | 356 | 27 | 356 | 32 | 356 | 16 |
| n (Pat) | 138 | 27 | 138 | 32 | 138 | 16 |

sCr or UO

| Time prior AKI stage | AUC | SE | nCohort 1 | nCohort 2 | p |
|---|---|---|---|---|---|
| 0 hours | 0.48 | 0.056 | 434 | 28 | 0.690 |
| 24 hours | 0.54 | 0.051 | 434 | 36 | 0.382 |
| 48 hours | 0.57 | 0.072 | 434 | 18 | 0.359 |

sCr only

| Time prior AKI stage | AUC | SE | nCohort 1 | nCohort 2 | p |
|---|---|---|---|---|---|
| 0 hours | 0.59 | 0.123 | 542 | 6 | 0.466 |
| 24 hours | 0.61 | 0.096 | 542 | 10 | 0.233 |
| 48 hours | 0.68 | 0.112 | 542 | 7 | 0.100 |

UO only

| Time prior AKI stage | AUC | SE | nCohort 1 | nCohort 2 | p |
|---|---|---|---|---|---|
| 0 hours | 0.46 | 0.056 | 356 | 27 | 0.495 |
| 24 hours | 0.53 | 0.054 | 356 | 32 | 0.593 |
| 48 hours | 0.54 | 0.075 | 356 | 16 | 0.621 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |

## Fig. 6 - 11

| Time prior AKI stage | Cutoff value | sens | spec | Quartile | OR | 95% CI of OR | |
|---|---|---|---|---|---|---|---|
| | 0 | 100% | 0% | 2 | 3.9 | 2.0 | 7.6 |
| | 0 | 100% | 0% | 3 | 1.8 | 0.8 | 4.0 |
| | 0.456 | 14% | 79% | 4 | 0.8 | 0.2 | 2.4 |
| | 1.73 | 14% | 81% | | | | |
| | 3.18 | 14% | 90% | | | | |
| 24 hours | 0 | 100% | 0% | 1 | | | |
| | 0 | 100% | 0% | 2 | 2.8 | 1.1 | 7.0 |
| | 0 | 100% | 0% | 3 | 4.3 | 1.9 | 10.2 |
| | 0.456 | 28% | 79% | 4 | 4.7 | 2.0 | 10.9 |
| | 1.73 | 28% | 81% | | | | |
| | 3.18 | 22% | 90% | | | | |
| 48 hours | 0 | 100% | 0% | 1 | | | |
| | 0 | 100% | 0% | 2 | 12.1 | 1.4 | 106.3 |
| | 0 | 100% | 0% | 3 | 0.0 | 0.0 | na |
| | 0.456 | 33% | 79% | 4 | 6.3 | 0.6 | 64.1 |
| | 1.73 | 33% | 81% | | | | |
| | 3.18 | 22% | 90% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 3.0 | 0.2 | 42.8 |
| | 0 | 100% | 0% | 3 | 0.0 | 0.0 | na |
| | 0.456 | 33% | 80% | 4 | 2.0 | 0.1 | 39.2 |
| | 0.456 | 33% | 80% | | | | |
| | 3.18 | 33% | 90% | | | | |
| 24 hours | 0 | 100% | 0% | 1 | | | |
| | 0 | 100% | 0% | 2 | na | na | na |
| | 0 | 100% | 0% | 3 | na | na | na |
| | 0.456 | 40% | 80% | 4 | na | na | na |
| | 0.456 | 40% | 80% | | | | |
| | 3.18 | 30% | 90% | | | | |
| 48 hours | 0 | 100% | 0% | 1 | | | |
| | 0 | 100% | 0% | 2 | na | na | na |
| | 0 | 100% | 0% | 3 | na | na | na |
| | 0.456 | 57% | 80% | 4 | na | na | na |
| | 0.456 | 57% | 80% | | | | |
| | 3.18 | 29% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.9 | 1.0 | 3.6 |
| | 0 | 100% | 0% | 3 | 1.7 | 0.8 | 3.3 |
| | 0.456 | 11% | 80% | 4 | 1.0 | 0.4 | 2.3 |
| | 0.456 | 11% | 80% | | | | |
| | 4.33 | 11% | 90% | | | | |
| 24 hours | 0 | 100% | 0% | 1 | | | |
| | 0 | 100% | 0% | 2 | 1.2 | 0.6 | 2.3 |
| | 0 | 100% | 0% | 3 | 1.2 | 0.6 | 2.3 |
| | 0.456 | 25% | 80% | 4 | 2.1 | 1.3 | 3.7 |
| | 0.456 | 25% | 80% | | | | |
| | 4.33 | 19% | 90% | | | | |
| 48 hours | 0 | 100% | 0% | 1 | | | |
| | 0 | 100% | 0% | 2 | 12.3 | 1.4 | 109.5 |
| | 0 | 100% | 0% | 3 | 0.0 | 0.0 | na |
| | 0.456 | 25% | 80% | 4 | 4.1 | 0.3 | 50.0 |
| | 0.456 | 25% | 80% | | | | |
| | 4.33 | 25% | 90% | | | | |

# Fig. 6 - 12

**Matrix metalloproteinase-3**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.043 | 0.063 | 0.043 | 0.050 | 0.043 | 0.061 |
| average | 0.126 | 0.203 | 0.126 | 0.159 | 0.126 | 0.246 |
| stdev | 0.241 | 0.315 | 0.241 | 0.279 | 0.241 | 0.472 |
| p (t-test) | | 0.109 | | 0.445 | | 0.050 |
| min | 0.002 | 0.002 | 0.002 | 0.002 | 0.002 | 0.002 |
| max | 2.160 | 1.180 | 2.160 | 1.150 | 2.160 | 1.880 |
| n (Samp) | 434 | 28 | 434 | 36 | 434 | 18 |
| n (Pat) | 173 | 28 | 173 | 36 | 173 | 18 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.050 | 0.384 | 0.050 | 0.090 | 0.050 | 0.150 |
| average | 0.149 | 0.343 | 0.149 | 0.169 | 0.149 | 0.302 |
| stdev | 0.284 | 0.231 | 0.284 | 0.221 | 0.284 | 0.325 |
| p (t-test) | | 0.098 | | 0.827 | | 0.160 |
| min | 0.002 | 0.058 | 0.002 | 0.002 | 0.002 | 0.062 |
| max | 2.160 | 0.654 | 2.160 | 0.732 | 2.160 | 0.927 |
| n (Samp) | 542 | 6 | 542 | 10 | 542 | 7 |
| n (Pat) | 208 | 6 | 208 | 10 | 208 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.049 | 0.059 | 0.049 | 0.045 | 0.049 | 0.041 |
| average | 0.121 | 0.186 | 0.121 | 0.150 | 0.121 | 0.174 |
| stdev | 0.235 | 0.304 | 0.235 | 0.278 | 0.235 | 0.462 |
| p (t-test) | | 0.182 | | 0.516 | | 0.406 |
| min | 0.002 | 0.002 | 0.002 | 0.002 | 0.002 | 0.002 |
| max | 2.160 | 1.180 | 2.160 | 1.150 | 2.160 | 1.880 |
| n (Samp) | 356 | 27 | 356 | 32 | 356 | 16 |
| n (Pat) | 138 | 27 | 138 | 32 | 138 | 16 |

sCr or UO

| Time prior AKI stage | AUC | SE | nCohort 1 | nCohort 2 | p |
|---|---|---|---|---|---|
| 0 hours | 0.57 | 0.058 | 434 | 28 | 0.254 |
| 24 hours | 0.51 | 0.050 | 434 | 36 | 0.791 |
| 48 hours | 0.56 | 0.071 | 434 | 18 | 0.434 |

sCr only

| Time prior AKI stage | AUC | SE | nCohort 1 | nCohort 2 | p |
|---|---|---|---|---|---|
| 0 hours | 0.80 | 0.109 | 542 | 6 | 0.005 |
| 24 hours | 0.58 | 0.095 | 542 | 10 | 0.397 |
| 48 hours | 0.75 | 0.107 | 542 | 7 | 0.018 |

UO only

| Time prior AKI stage | AUC | SE | nCohort 1 | nCohort 2 | p |
|---|---|---|---|---|---|
| 0 hours | 0.55 | 0.059 | 356 | 27 | 0.399 |
| 24 hours | 0.49 | 0.053 | 356 | 32 | 0.857 |
| 48 hours | 0.46 | 0.072 | 356 | 16 | 0.562 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |

## Fig. 6 - 13

| | Cutoff value | sens | spec | Quartile | OR | 95% CI of OR | |
|---|---|---|---|---|---|---|---|
| | 0 | 100% | 0% | 2 | 10.8 | 1.2 | 96.3 |
| | 0 | 100% | 0% | 3 | 6.3 | 0.6 | 64.0 |
| | 0.109 | 39% | 70% | 4 | 11.9 | 1.4 | 105.0 |
| | 0.182 | 32% | 80% | | | | |
| | 0.315 | 18% | 91% | | | | |
| 24 hours | 0 | 100% | 0% | 1 | | | |
| | 0 | 100% | 0% | 2 | 1.0 | 0.6 | 1.7 |
| | 0 | 100% | 0% | 3 | 1.1 | 0.7 | 1.9 |
| | 0.109 | 31% | 70% | 4 | 1.4 | 0.9 | 2.2 |
| | 0.182 | 19% | 80% | | | | |
| | 0.315 | 11% | 91% | | | | |
| 48 hours | 0 | 100% | 0% | 1 | | | |
| | 0 | 100% | 0% | 2 | 1.3 | 0.4 | 4.4 |
| | 0 | 100% | 0% | 3 | 1.3 | 0.4 | 4.4 |
| | 0.109 | 39% | 70% | 4 | 2.4 | 0.9 | 6.4 |
| | 0.182 | 22% | 80% | | | | |
| | 0.315 | 17% | 91% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.3 | 0.1 | 0.8 |
| | 0 | 100% | 0% | 3 | 0.4 | 0.2 | 0.9 |
| | 0.105 | 41% | 70% | 4 | 1.2 | 0.8 | 1.9 |
| | 0.164 | 33% | 80% | | | | |
| | 0.306 | 19% | 90% | | | | |
| 24 hours | 0 | 100% | 0% | 1 | | | |
| | 0 | 100% | 0% | 2 | 0.6 | 0.4 | 1.2 |
| | 0 | 100% | 0% | 3 | 0.9 | 0.5 | 1.5 |
| | 0.105 | 28% | 70% | 4 | 1.0 | 0.6 | 1.6 |
| | 0.164 | 19% | 80% | | | | |
| | 0.306 | 16% | 90% | | | | |
| 48 hours | 0 | 100% | 0% | 1 | | | |
| | 0 | 100% | 0% | 2 | 1.0 | 0.4 | 2.8 |
| | 0 | 100% | 0% | 3 | 1.8 | 0.8 | 4.1 |
| | 0.105 | 25% | 70% | 4 | 0.2 | 0.0 | 2.9 |
| | 0.164 | 25% | 80% | | | | |
| | 0.306 | 6% | 90% | | | | |

# Fig. 6 - 14

**Thrombospondin-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 2166.151 | 881.530 | 2166.151 | 636.838 | 2166.151 | 674.270 |
| average | 2765.880 | 1612.846 | 2765.880 | 1511.899 | 2765.880 | 674.270 |
| stdev | 2251.508 | 2488.238 | 2251.508 | 1839.325 | 2251.508 | 200.763 |
| p (t-test) | | 0.154 | | 0.278 | | 0.196 |
| min | 177.557 | 286.458 | 177.557 | 505.826 | 177.557 | 532.309 |
| max | 9514.803 | 8182.566 | 9514.803 | 4268.092 | 9514.803 | 816.231 |
| n (Samp) | 78 | 9 | 78 | 4 | 78 | 2 |
| n (Pat) | 58 | 9 | 58 | 4 | 58 | 2 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 2031.734 | 794.393 | 2031.734 | 636.838 | 2031.734 | 939.832 |
| average | 2587.165 | 794.393 | 2587.165 | 636.838 | 2587.165 | 939.832 |
| stdev | 2218.935 | 644.326 | 2218.935 | 95.393 | 2218.935 | 174.798 |
| p (t-test) | | 0.259 | | 0.219 | | 0.299 |
| min | 177.557 | 338.785 | 177.557 | 569.386 | 177.557 | 816.231 |
| max | 9514.803 | 1250.000 | 9514.803 | 704.291 | 9514.803 | 1063.433 |
| n (Samp) | 97 | 2 | 97 | 2 | 97 | 2 |
| n (Pat) | 70 | 2 | 70 | 2 | 70 | 2 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 2271.672 | 881.530 | 2271.672 | 2386.959 | 2271.672 | 315.148 |
| average | 2958.509 | 1612.846 | 2958.509 | 2386.959 | 2958.509 | 532.309 |
| stdev | 2402.385 | 2488.238 | 2402.385 | 2660.324 | 2402.385 | na |
| p (t-test) | | 0.123 | | 0.742 | | na |
| min | 315.148 | 286.458 | 315.148 | 505.826 | 315.148 | 532.309 |
| max | 9514.803 | 8182.566 | 9514.803 | 4268.092 | 9514.803 | 532.309 |
| n (Samp) | 60 | 9 | 60 | 2 | 60 | 1 |
| n (Pat) | 45 | 9 | 45 | 2 | 45 | 1 |

sCr or UO

| Time prior AKI stage | AUC | SE | nCohort 1 | nCohort 2 | p |
|---|---|---|---|---|---|
| 0 hours | 0.26 | 0.075 | 78 | 9 | 0.001 |
| 24 hours | 0.30 | 0.117 | 78 | 4 | 0.095 |
| 48 hours | 0.15 | 0.099 | 78 | 2 | 0.000 |

sCr only

| Time prior AKI stage | AUC | SE | nCohort 1 | nCohort 2 | p |
|---|---|---|---|---|---|
| 0 hours | 0.20 | 0.118 | 97 | 2 | 0.010 |
| 24 hours | 0.19 | 0.116 | 97 | 2 | 0.008 |
| 48 hours | 0.25 | 0.142 | 97 | 2 | 0.082 |

UO only

| Time prior AKI stage | AUC | SE | nCohort 1 | nCohort 2 | p |
|---|---|---|---|---|---|
| 0 hours | 0.24 | 0.074 | 60 | 9 | 0.000 |
| 24 hours | 0.41 | 0.193 | 60 | 2 | 0.635 |
| 48 hours | 0.08 | 0.084 | 60 | 1 | 0.000 |

sCr or UO

| Time prior AKI stage | Cutoff value | sens | spec | Quartile | OR | 95% CI of OR | |
|---|---|---|---|---|---|---|---|
| 0 hours | 473.131 | 78% | 10% | 1 | | | |

# Fig. 6 - 15

|  | 373.411 | 89% | 6% | 2 | 0.0 | 0.0 | na |
|  | 229.64 | 100% | 3% | 3 | 3.3 | 0.2 | 55.1 |
|  | 3116.78 | 11% | 71% | 4 | 6.6 | 0.5 | 85.6 |
|  | 4629.93 | 11% | 81% |  |  |  |  |
|  | 6307.57 | 11% | 91% |  |  |  |  |
| 24 hours | 542.903 | 75% | 13% | 1 |  |  |  |
|  | 473.131 | 100% | 10% | 2 | 0.0 | 0.0 | na |
|  | 473.131 | 100% | 10% | 3 | 0.0 | 0.0 | na |
|  | 3116.78 | 25% | 71% | 4 | 3.5 | 0.2 | 59.6 |
|  | 4629.93 | 0% | 81% |  |  |  |  |
|  | 6307.57 | 0% | 91% |  |  |  |  |
| 48 hours | 531.542 | 100% | 12% | 1 |  |  |  |
|  | 531.542 | 100% | 12% | 2 | na | na | na |
|  | 531.542 | 100% | 12% | 3 | na | na | na |
|  | 3116.78 | 0% | 71% | 4 | na | na | na |
|  | 4629.93 | 0% | 81% |  |  |  |  |
|  | 6307.57 | 0% | 91% |  |  |  |  |

UO only

| Time prior AKI stage | Cutoff value | sens | spec | Quartile | OR | 95% CI of OR | |
|---|---|---|---|---|---|---|---|
| 0 hours | 473.131 | 78% | 7% | 1 |  |  |  |
|  | 315.148 | 89% | 2% | 2 | 0.0 | 0.0 | na |
|  | 0 | 100% | 0% | 3 | 3.6 | 0.2 | 64.2 |
|  | 3116.78 | 11% | 70% | 4 | 7.1 | 0.5 | 98.3 |
|  | 5266.77 | 11% | 80% |  |  |  |  |
|  | 6486.28 | 11% | 90% |  |  |  |  |
| 24 hours | 473.131 | 100% | 7% | 1 |  |  |  |
|  | 473.131 | 100% | 7% | 2 | 0.0 | 0.0 | na |
|  | 473.131 | 100% | 7% | 3 | 0.0 | 0.0 | na |
|  | 3116.78 | 50% | 70% | 4 | 1.1 | 0.0 | 70.8 |
|  | 5266.77 | 0% | 80% |  |  |  |  |
|  | 6486.28 | 0% | 90% |  |  |  |  |
| 48 hours | 531.542 | 100% | 8% | 1 |  |  |  |
|  | 531.542 | 100% | 8% | 2 | na | na | na |
|  | 531.542 | 100% | 8% | 3 | na | na | na |
|  | 3116.78 | 0% | 70% | 4 | na | na | na |
|  | 5266.77 | 0% | 80% |  |  |  |  |
|  | 6486.28 | 0% | 90% |  |  |  |  |

Fig. 6 - 16

**ENA-78 (C-X-C chemokine motif 5)**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.670 | 0.361 | 0.670 | 0.361 | 0.670 | 0.361 |
| average | 1.530 | 0.564 | 1.530 | 0.564 | 1.530 | 0.564 |
| stdev | 4.335 | 0.424 | 4.335 | 0.424 | 4.335 | 0.424 |
| p (t-test) | | 0.313 | | 0.313 | | 0.313 |
| min | 0.038 | 0.091 | 0.038 | 0.091 | 0.038 | 0.091 |
| max | 32.500 | 1.560 | 32.500 | 1.560 | 32.500 | 1.560 |
| n (Samp) | 56 | 21 | 56 | 21 | 56 | 21 |
| n (Pat) | 56 | 21 | 56 | 21 | 56 | 21 |

sCr only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.692 | 0.327 | 0.692 | 0.327 | 0.692 | 0.327 |
| average | 0.717 | 0.515 | 0.717 | 0.515 | 0.717 | 0.515 |
| stdev | 0.500 | 0.395 | 0.500 | 0.395 | 0.500 | 0.395 |
| p (t-test) | | 0.410 | | 0.410 | | 0.410 |
| min | 0.050 | 0.094 | 0.050 | 0.094 | 0.050 | 0.094 |
| max | 2.010 | 1.040 | 2.010 | 1.040 | 2.010 | 1.040 |
| n (Samp) | 21 | 5 | 21 | 5 | 21 | 5 |
| n (Pat) | 21 | 5 | 21 | 5 | 21 | 5 |

UO only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.606 | 0.353 | 0.606 | 0.353 | 0.606 | 0.353 |
| average | 1.691 | 0.531 | 1.691 | 0.531 | 1.691 | 0.531 |
| stdev | 4.769 | 0.455 | 4.769 | 0.455 | 4.769 | 0.455 |
| p (t-test) | | 0.323 | | 0.323 | | 0.323 |
| min | 0.038 | 0.091 | 0.038 | 0.091 | 0.038 | 0.091 |
| max | 32.500 | 1.560 | 32.500 | 1.560 | 32.500 | 1.560 |
| n (Samp) | 46 | 17 | 46 | 17 | 46 | 17 |
| n (Pat) | 46 | 17 | 46 | 17 | 46 | 17 |

sCr or UO

| Time prior AKI stage | AUC | SE | nCohort 1 | nCohort 2 | p |
|---|---|---|---|---|---|
| 0 hours | 0.42 | 0.071 | 56 | 21 | 0.239 |
| 24 hours | 0.42 | 0.071 | 56 | 21 | 0.239 |
| 48 hours | 0.42 | 0.071 | 56 | 21 | 0.239 |

sCr only

| Time prior AKI stage | AUC | SE | nCohort 1 | nCohort 2 | p |
|---|---|---|---|---|---|
| 0 hours | 0.39 | 0.136 | 21 | 5 | 0.420 |
| 24 hours | 0.39 | 0.136 | 21 | 5 | 0.420 |
| 48 hours | 0.39 | 0.136 | 21 | 5 | 0.420 |

UO only

| Time prior AKI stage | AUC | SE | nCohort 1 | nCohort 2 | p |
|---|---|---|---|---|---|
| 0 hours | 0.38 | 0.077 | 46 | 17 | 0.133 |
| 24 hours | 0.38 | 0.077 | 46 | 17 | 0.133 |
| 48 hours | 0.38 | 0.077 | 46 | 17 | 0.133 |

sCr or UO

| Time prior AKI stage | Cutoff value | sens | spec | Quartile | OR | 95% CI of OR | |
|---|---|---|---|---|---|---|---|
| 0 hours | 0.299 | 71% | 34% | 1 | | | |
| | 0.232 | 81% | 23% | 2 | 2.0 | 0.6 | 7.4 |

**Fig. 7 - 1**

| | 0.144 | 90% | 18% | 3 | 4.1 | 1.3 | 13.6 |
|---|---|---|---|---|---|---|---|
| | 1.24 | 10% | 71% | 4 | 2.0 | 0.6 | 7.4 |
| | 1.66 | 0% | 80% | | | | |
| | 2.78 | 0% | 91% | | | | |
| 24 hours | 0.299 | 71% | 34% | 1 | | | |
| | 0.232 | 81% | 23% | 2 | 2.0 | 0.6 | 7.4 |
| | 0.144 | 90% | 18% | 3 | 4.1 | 1.3 | 13.6 |
| | 1.24 | 10% | 71% | 4 | 2.0 | 0.6 | 7.4 |
| | 1.66 | 0% | 80% | | | | |
| | 2.78 | 0% | 91% | | | | |
| 48 hours | 0.299 | 71% | 34% | 1 | | | |
| | 0.232 | 81% | 23% | 2 | 2.0 | 0.6 | 7.4 |
| | 0.144 | 90% | 18% | 3 | 4.1 | 1.3 | 13.6 |
| | 1.24 | 10% | 71% | 4 | 2.0 | 0.6 | 7.4 |
| | 1.66 | 0% | 80% | | | | |
| | 2.78 | 0% | 91% | | | | |

sCr only

| Time prior AKI stage | Cutoff value | sens | spec | Quartile | OR | 95% CI of OR | |
|---|---|---|---|---|---|---|---|
| 0 hours | 0.232 | 80% | 19% | 1 | | | |
| | 0.232 | 80% | 19% | 2 | 1.2 | 0.0 | 124.1 |
| | 0.0688 | 100% | 10% | 3 | 1.0 | 0.0 | 96.9 |
| | 0.911 | 20% | 71% | 4 | 3.0 | 0.1 | 128.4 |
| | 1 | 20% | 81% | | | | |
| | 1.44 | 0% | 90% | | | | |
| 24 hours | 0.232 | 80% | 19% | 1 | | | |
| | 0.232 | 80% | 19% | 2 | 1.2 | 0.0 | 124.1 |
| | 0.0688 | 100% | 10% | 3 | 1.0 | 0.0 | 96.9 |
| | 0.911 | 20% | 71% | 4 | 3.0 | 0.1 | 128.4 |
| | 1 | 20% | 81% | | | | |
| | 1.44 | 0% | 90% | | | | |
| 48 hours | 0.232 | 80% | 19% | 1 | | | |
| | 0.232 | 80% | 19% | 2 | 1.2 | 0.0 | 124.1 |
| | 0.0688 | 100% | 10% | 3 | 1.0 | 0.0 | 96.9 |
| | 0.911 | 20% | 71% | 4 | 3.0 | 0.1 | 128.4 |
| | 1 | 20% | 81% | | | | |
| | 1.44 | 0% | 90% | | | | |

UO only

| Time prior AKI stage | Cutoff value | sens | spec | Quartile | OR | 95% CI of OR | |
|---|---|---|---|---|---|---|---|
| 0 hours | 0.251 | 71% | 24% | 1 | | | |
| | 0.144 | 82% | 15% | 2 | 3.2 | 0.6 | 17.2 |
| | 0.112 | 94% | 13% | 3 | 3.2 | 0.6 | 17.2 |
| | 1.35 | 12% | 72% | 4 | 3.5 | 0.6 | 19.3 |
| | 1.68 | 0% | 80% | | | | |
| | 2.81 | 0% | 91% | | | | |
| 24 hours | 0.251 | 71% | 24% | 1 | | | |
| | 0.144 | 82% | 15% | 2 | 3.2 | 0.6 | 17.2 |
| | 0.112 | 94% | 13% | 3 | 3.2 | 0.6 | 17.2 |
| | 1.35 | 12% | 72% | 4 | 3.5 | 0.6 | 19.3 |
| | 1.68 | 0% | 80% | | | | |
| | 2.81 | 0% | 91% | | | | |
| 48 hours | 0.251 | 71% | 24% | 1 | | | |
| | 0.144 | 82% | 15% | 2 | 3.2 | 0.6 | 17.2 |
| | 0.112 | 94% | 13% | 3 | 3.2 | 0.6 | 17.2 |
| | 1.35 | 12% | 72% | 4 | 3.5 | 0.6 | 19.3 |
| | 1.68 | 0% | 80% | | | | |
| | 2.81 | 0% | 91% | | | | |

# Fig. 7 - 2

CD44

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 251.624 | 269.551 | 251.624 | 281.817 | 251.624 | 259.544 |
| average | 306.077 | 294.432 | 306.077 | 307.596 | 306.077 | 248.869 |
| stdev | 177.805 | 138.410 | 177.805 | 138.448 | 177.805 | 70.999 |
| p (t-test) | | 0.840 | | 0.980 | | 0.376 |
| Min | 124.024 | 148.063 | 124.024 | 148.063 | 124.024 | 148.063 |
| max | 1060.922 | 585.758 | 1060.922 | 585.758 | 1060.922 | 354.323 |
| n (Samp) | 47 | 11 | 47 | 10 | 47 | 8 |
| n (Pat) | 47 | 11 | 47 | 10 | 47 | 8 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 251.006 | 259.544 | 251.006 | 259.544 | 251.006 | 220.302 |
| average | 286.752 | 326.022 | 286.752 | 326.022 | 286.752 | 222.170 |
| stdev | 151.701 | 167.752 | 151.701 | 167.752 | 151.701 | 44.219 |
| p (t-test) | | 0.546 | | 0.546 | | 0.401 |
| min | 124.024 | 178.526 | 124.024 | 178.526 | 124.024 | 178.526 |
| max | 1060.922 | 585.758 | 1060.922 | 585.758 | 1060.922 | 269.551 |
| n (Samp) | 78 | 6 | 78 | 6 | 78 | 4 |
| n (Pat) | 78 | 6 | 78 | 6 | 78 | 4 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 251.624 | 242.575 | 251.624 | 294.084 | 251.624 | 294.084 |
| average | 276.555 | 245.614 | 276.555 | 262.178 | 276.555 | 258.668 |
| stdev | 110.596 | 90.846 | 110.596 | 90.876 | 110.596 | 85.775 |
| p (t-test) | | 0.520 | | 0.783 | | 0.731 |
| min | 124.024 | 148.063 | 124.024 | 148.063 | 124.024 | 148.063 |
| max | 639.273 | 371.875 | 639.273 | 371.875 | 639.273 | 354.323 |
| n (Samp) | 37 | 6 | 37 | 5 | 37 | 5 |
| n (Pat) | 37 | 6 | 37 | 5 | 37 | 5 |

sCr or UO

| Time prior AKI stage | AUC | SE | nCohort 1 | nCohort 2 | p |
|---|---|---|---|---|---|
| 0 hours | 0.50 | 0.097 | 47 | 11 | 0.992 |
| 24 hours | 0.54 | 0.103 | 47 | 10 | 0.679 |
| 48 hours | 0.45 | 0.108 | 47 | 8 | 0.622 |

sCr only

| Time prior AKI stage | AUC | SE | nCohort 1 | nCohort 2 | p |
|---|---|---|---|---|---|
| 0 hours | 0.56 | 0.126 | 78 | 6 | 0.629 |
| 24 hours | 0.56 | 0.126 | 78 | 6 | 0.629 |
| 48 hours | 0.37 | 0.130 | 78 | 4 | 0.308 |

UO only

| Time prior AKI stage | AUC | SE | nCohort 1 | nCohort 2 | p |
|---|---|---|---|---|---|
| 0 hours | 0.44 | 0.124 | 37 | 6 | 0.636 |
| 24 hours | 0.51 | 0.140 | 37 | 5 | 0.923 |
| 48 hours | 0.51 | 0.140 | 37 | 5 | 0.954 |

sCr or UO

| Time prior AKI stage | Cutoff value | sens | spec | Quartile | OR | 95% CI of OR | |
|---|---|---|---|---|---|---|---|
| 0 hours | 181.323 | 73% | 17% | 1 | | | |
| | 176.603 | 82% | 13% | 2 | 1.1 | 0.2 | 5.7 |
| | 159.936 | 91% | 6% | 3 | 0.3 | 0.0 | 5.3 |
| | 293.798 | 45% | 70% | 4 | 1.6 | 0.4 | 7.2 |

Fig. 8 - 1

| | Cutoff value | sens | spec | Quartile | OR | 95% CI of OR | |
|---|---|---|---|---|---|---|---|
| | 358.806 | 27% | 81% | | | | |
| | 469.985 | 18% | 91% | | | | |
| 24 hours | 248.84 | 70% | 45% | 1 | | | |
| | 181.323 | 80% | 17% | 2 | 0.3 | 0.0 | 5.3 |
| | 176.603 | 90% | 13% | 3 | 1.0 | 0.2 | 5.3 |
| | 293.798 | 50% | 70% | 4 | 0.9 | 0.2 | 4.8 |
| | 358.806 | 30% | 81% | | | | |
| | 469.985 | 20% | 91% | | | | |
| 48 hours | 181.323 | 75% | 17% | 1 | | | |
| | 176.603 | 88% | 13% | 2 | 3.5 | 0.2 | 67.2 |
| | 124.024 | 100% | 2% | 3 | 1.0 | 0.0 | 68.1 |
| | 293.798 | 38% | 70% | 4 | 3.9 | 0.2 | 75.3 |
| | 358.806 | 0% | 81% | | | | |
| | 469.985 | 0% | 91% | | | | |

sCr only

| Time prior AKI stage | Cutoff value | sens | spec | Quartile | OR | 95% CI of OR | |
|---|---|---|---|---|---|---|---|
| 0 hours | 182.019 | 83% | 22% | 1 | | | |
| | 182.019 | 83% | 22% | 2 | 0.5 | 0.0 | 11.0 |
| | 176.603 | 100% | 17% | 3 | 0.5 | 0.0 | 11.0 |
| | 308.32 | 33% | 71% | 4 | 1.0 | 0.1 | 8.7 |
| | 358.806 | 33% | 81% | | | | |
| | 422.768 | 33% | 91% | | | | |
| 24 hours | 182.019 | 83% | 22% | 1 | | | |
| | 182.019 | 83% | 22% | 2 | 0.5 | 0.0 | 11.0 |
| | 176.603 | 100% | 17% | 3 | 0.5 | 0.0 | 11.0 |
| | 308.32 | 33% | 71% | 4 | 1.0 | 0.1 | 8.7 |
| | 358.806 | 33% | 81% | | | | |
| | 422.768 | 33% | 91% | | | | |
| 48 hours | 182.019 | 75% | 22% | 1 | | | |
| | 176.603 | 100% | 17% | 2 | na | na | na |
| | 176.603 | 100% | 17% | 3 | na | na | na |
| | 308.32 | 0% | 71% | 4 | na | na | na |
| | 358.806 | 0% | 81% | | | | |
| | 422.768 | 0% | 91% | | | | |

UO only

| Time prior AKI stage | Cutoff value | sens | spec | Quartile | OR | 95% CI of OR | |
|---|---|---|---|---|---|---|---|
| 0 hours | 159.936 | 83% | 8% | 1 | | | |
| | 159.936 | 83% | 8% | 2 | 0.5 | 0.0 | 12.9 |
| | 124.024 | 100% | 3% | 3 | 0.0 | 0.0 | na |
| | 291.987 | 50% | 70% | 4 | 1.9 | 0.2 | 16.2 |
| | 341.599 | 17% | 81% | | | | |
| | 422.768 | 0% | 92% | | | | |
| 24 hours | 181.323 | 80% | 16% | 1 | | | |
| | 181.323 | 80% | 16% | 2 | 0.0 | 0.0 | na |
| | 124.024 | 100% | 3% | 3 | 0.4 | 0.0 | 13.4 |
| | 291.987 | 60% | 70% | 4 | 0.9 | 0.1 | 10.0 |
| | 341.599 | 20% | 81% | | | | |
| | 422.768 | 0% | 92% | | | | |
| 48 hours | 181.323 | 80% | 16% | 1 | | | |
| | 181.323 | 80% | 16% | 2 | 0.0 | 0.0 | na |
| | 124.024 | 100% | 3% | 3 | 0.4 | 0.0 | 13.4 |
| | 291.987 | 60% | 70% | 4 | 0.9 | 0.1 | 10.0 |
| | 341.599 | 20% | 81% | | | | |
| | 422.768 | 0% | 92% | | | | |

**Angiopoietin-1**

# Fig. 8 - 2

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 1722.948 | 2046.729 | 1722.948 | 1090.554 | 1722.948 | 1544.026 |
| average | 2552.209 | 2720.067 | 2552.209 | 2263.841 | 2552.209 | 2879.031 |
| stdev | 2544.293 | 2867.490 | 2544.293 | 2944.741 | 2544.293 | 3305.810 |
| p (t-test) | | 0.814 | | 0.696 | | 0.706 |
| min | 192.308 | 294.737 | 192.308 | 134.615 | 192.308 | 355.469 |
| max | 14800.944 | 11276.161 | 14800.944 | 11276.161 | 14800.944 | 11276.161 |
| n (Samp) | 106 | 15 | 106 | 14 | 106 | 10 |
| n (Pat) | 106 | 15 | 106 | 14 | 106 | 10 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 1841.410 | 1174.729 | 1841.410 | 953.039 | 1841.410 | 1228.070 |
| average | 2847.163 | 2672.378 | 2847.163 | 2857.308 | 2847.163 | 3266.837 |
| stdev | 2697.239 | 3664.030 | 2697.239 | 3920.145 | 2697.239 | 4502.372 |
| p (t-test) | | 0.860 | | 0.992 | | 0.737 |
| min | 171.053 | 294.737 | 171.053 | 134.615 | 171.053 | 830.189 |
| max | 14800.944 | 11276.161 | 14800.944 | 11276.161 | 14800.944 | 11276.161 |
| n (Samp) | 171 | 8 | 171 | 7 | 171 | 5 |
| n (Pat) | 171 | 8 | 171 | 7 | 171 | 5 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 1600.644 | 2887.671 | 1600.644 | 1351.852 | 1600.644 | 2046.729 |
| average | 2605.373 | 2826.741 | 2605.373 | 1967.923 | 2605.373 | 2247.275 |
| stdev | 2769.536 | 1697.025 | 2769.536 | 1590.424 | 2769.536 | 1690.296 |
| p (t-test) | | 0.815 | | 0.500 | | 0.738 |
| min | 192.308 | 394.531 | 192.308 | 355.469 | 192.308 | 355.469 |
| max | 14800.944 | 5089.494 | 14800.944 | 4622.222 | 14800.944 | 4622.222 |
| n (Samp) | 86 | 9 | 86 | 9 | 86 | 7 |
| n (Pat) | 86 | 9 | 86 | 9 | 86 | 7 |

sCr or UO

| Time prior AKI stage | AUC | SE | nCohort 1 | nCohort 2 | p |
|---|---|---|---|---|---|
| 0 hours | 0.49 | 0.080 | 106 | 15 | 0.921 |
| 24 hours | 0.41 | 0.077 | 106 | 14 | 0.237 |
| 48 hours | 0.50 | 0.096 | 106 | 10 | 0.984 |

sCr only

| Time prior AKI stage | AUC | SE | nCohort 1 | nCohort 2 | p |
|---|---|---|---|---|---|
| 0 hours | 0.41 | 0.097 | 171 | 8 | 0.360 |
| 24 hours | 0.42 | 0.104 | 171 | 7 | 0.429 |
| 48 hours | 0.47 | 0.128 | 171 | 5 | 0.788 |

UO only

| Time prior AKI stage | AUC | SE | nCohort 1 | nCohort 2 | p |
|---|---|---|---|---|---|
| 0 hours | 0.59 | 0.104 | 86 | 9 | 0.393 |
| 24 hours | 0.45 | 0.098 | 86 | 9 | 0.618 |
| 48 hours | 0.50 | 0.114 | 86 | 7 | 0.977 |

sCr or UO

| Time prior AKI stage | Cutoff value | sens | spec | Quartile | OR | 95% CI of OR | |
|---|---|---|---|---|---|---|---|
| 0 hours | 728.261 | 73% | 23% | 1 | | | |
| | 650 | 80% | 19% | 2 | 0.6 | 0.2 | 1.9 |
| | 384.259 | 93% | 8% | 3 | 0.4 | 0.1 | 1.7 |
| | 2710.36 | 40% | 71% | 4 | 1.0 | 0.4 | 2.7 |

# Fig. 8 - 3

| Time prior AKI stage | Cutoff value | sens | spec | Quartile | OR | 95% CI of OR | |
|---|---|---|---|---|---|---|---|
| | 4010.38 | 20% | 80% | | | | |
| | 5570.37 | 7% | 91% | | | | |
| 24 hours | 815.217 | 71% | 24% | 1 | | | |
| | 384.259 | 86% | 8% | 2 | 0.6 | 0.1 | 3.8 |
| | 331.25 | 93% | 7% | 3 | 1.4 | 0.4 | 5.0 |
| | 2710.36 | 21% | 71% | 4 | 1.8 | 0.5 | 6.0 |
| | 4010.38 | 21% | 80% | | | | |
| | 5570.37 | 7% | 91% | | | | |
| 48 hours | 933.962 | 70% | 29% | 1 | | | |
| | 913.043 | 80% | 29% | 2 | 0.6 | 0.1 | 3.8 |
| | 815.217 | 90% | 24% | 3 | 1.0 | 0.2 | 4.3 |
| | 2710.36 | 30% | 71% | 4 | 0.6 | 0.1 | 3.8 |
| | 4010.38 | 30% | 80% | | | | |
| | 5570.37 | 10% | 91% | | | | |

sCr only

| Time prior AKI stage | Cutoff value | sens | spec | Quartile | OR | 95% CI of OR | |
|---|---|---|---|---|---|---|---|
| 0 hours | 750 | 75% | 20% | 1 | | | |
| | 650 | 88% | 16% | 2 | 2.0 | 0.1 | 42.4 |
| | 284.247 | 100% | 4% | 3 | 2.0 | 0.1 | 42.4 |
| | 3479.45 | 25% | 70% | 4 | 3.2 | 0.2 | 48.2 |
| | 4665.76 | 13% | 80% | | | | |
| | 6612.75 | 13% | 90% | | | | |
| 24 hours | 818.182 | 71% | 22% | 1 | | | |
| | 750 | 86% | 20% | 2 | 2.1 | 0.1 | 43.4 |
| | 0 | 100% | 0% | 3 | 1.0 | 0.0 | 55.1 |
| | 3479.45 | 29% | 70% | 4 | 3.2 | 0.2 | 48.2 |
| | 4665.76 | 14% | 80% | | | | |
| | 6612.75 | 14% | 90% | | | | |
| 48 hours | 941.989 | 80% | 27% | 1 | | | |
| | 941.989 | 80% | 27% | 2 | 1.0 | 0.0 | 55.2 |
| | 818.182 | 100% | 22% | 3 | 2.0 | 0.1 | 42.5 |
| | 3479.45 | 20% | 70% | 4 | 1.0 | 0.0 | 55.2 |
| | 4665.76 | 20% | 80% | | | | |
| | 6612.75 | 20% | 90% | | | | |

UO only

| Time prior AKI stage | Cutoff value | sens | spec | Quartile | OR | 95% CI of OR | |
|---|---|---|---|---|---|---|---|
| 0 hours | 2009.35 | 78% | 56% | 1 | | | |
| | 394.531 | 89% | 8% | 2 | 0.0 | 0.0 | na |
| | 375 | 100% | 8% | 3 | 1.5 | 0.2 | 9.3 |
| | 2607.48 | 56% | 71% | 4 | 2.1 | 0.4 | 11.1 |
| | 4088.89 | 22% | 80% | | | | |
| | 5863.1 | 0% | 91% | | | | |
| 24 hours | 913.043 | 78% | 30% | 1 | | | |
| | 375 | 89% | 8% | 2 | 1.0 | 0.1 | 8.5 |
| | 331.25 | 100% | 7% | 3 | 1.6 | 0.3 | 9.7 |
| | 2607.48 | 22% | 71% | 4 | 1.0 | 0.1 | 8.9 |
| | 4088.89 | 22% | 80% | | | | |
| | 5863.1 | 0% | 91% | | | | |
| 48 hours | 990.566 | 71% | 33% | 1 | | | |
| | 913.043 | 86% | 30% | 2 | 2.1 | 0.1 | 47.6 |
| | 331.25 | 100% | 7% | 3 | 2.1 | 0.1 | 47.6 |
| | 2607.48 | 29% | 71% | 4 | 2.0 | 0.1 | 45.2 |
| | 4088.89 | 29% | 80% | | | | |
| | 5863.1 | 0% | 91% | | | | |

ENA-78 (C-X-C chemokine motif 5)

sCr or UO

| 0 hr prior to AKI stage | 24 hr prior to AKI stage | 48 hr prior to AKI stage | |
|---|---|---|---|

# Fig. 8 - 4

|  | Cohort 1 | Cohort 2 | Cohort 1 | Cohort 2 | Cohort 1 | Cohort 2 |
|---|---|---|---|---|---|---|
| median | 0.905 | 0.815 | 0.905 | 0.815 | 0.905 | 1.123 |
| average | 1.331 | 1.542 | 1.331 | 1.466 | 1.331 | 1.969 |
| stdev | 1.443 | 1.706 | 1.443 | 1.616 | 1.443 | 1.867 |
| p (t-test) |  | 0.585 |  | 0.725 |  | 0.194 |
| min | 0.022 | 0.103 | 0.022 | 0.103 | 0.022 | 0.270 |
| max | 8.730 | 6.040 | 8.730 | 6.040 | 8.730 | 6.040 |
| n (Samp) | 112 | 17 | 112 | 17 | 112 | 10 |
| n (Pat) | 112 | 17 | 112 | 17 | 112 | 10 |

sCr only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.905 | 1.121 | 0.905 | 1.121 | 0.905 | 1.880 |
| average | 1.530 | 1.629 | 1.530 | 1.490 | 1.530 | 2.216 |
| stdev | 2.811 | 1.660 | 2.811 | 1.444 | 2.811 | 1.334 |
| p (t-test) |  | 0.922 |  | 0.968 |  | 0.588 |
| min | 0.022 | 0.155 | 0.022 | 0.150 | 0.022 | 0.762 |
| max | 32.500 | 4.240 | 32.500 | 4.010 | 32.500 | 4.010 |
| n (Samp) | 180 | 8 | 180 | 8 | 180 | 5 |
| n (Pat) | 180 | 8 | 180 | 8 | 180 | 5 |

UO only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.880 | 0.875 | 0.880 | 0.875 | 0.880 | 0.875 |
| average | 1.326 | 1.503 | 1.326 | 1.487 | 1.326 | 1.684 |
| stdev | 1.400 | 1.653 | 1.400 | 1.654 | 1.400 | 1.993 |
| p (t-test) |  | 0.698 |  | 0.724 |  | 0.529 |
| min | 0.048 | 0.103 | 0.048 | 0.103 | 0.048 | 0.270 |
| max | 8.730 | 6.040 | 8.730 | 6.040 | 8.730 | 6.040 |
| n (Samp) | 89 | 11 | 89 | 11 | 89 | 7 |
| n (Pat) | 89 | 11 | 89 | 11 | 89 | 7 |

sCr or UO

| Time prior AKI stage | AUC | SE | nCohort 1 | nCohort 2 | p |
|---|---|---|---|---|---|
| 0 hours | 0.50 | 0.076 | 112 | 17 | 0.950 |
| 24 hours | 0.50 | 0.075 | 112 | 17 | 0.981 |
| 48 hours | 0.62 | 0.098 | 112 | 10 | 0.227 |

sCr only

| Time prior AKI stage | AUC | SE | nCohort 1 | nCohort 2 | p |
|---|---|---|---|---|---|
| 0 hours | 0.52 | 0.106 | 180 | 8 | 0.872 |
| 24 hours | 0.51 | 0.105 | 180 | 8 | 0.913 |
| 48 hours | 0.74 | 0.129 | 180 | 5 | 0.057 |

UO only

| Time prior AKI stage | AUC | SE | nCohort 1 | nCohort 2 | p |
|---|---|---|---|---|---|
| 0 hours | 0.53 | 0.094 | 89 | 11 | 0.781 |
| 24 hours | 0.52 | 0.094 | 89 | 11 | 0.836 |
| 48 hours | 0.55 | 0.116 | 89 | 7 | 0.694 |

sCr or UO

| Time prior AKI stage | Cutoff value | sens | spec | Quartile | OR | 95% CI of OR | |
|---|---|---|---|---|---|---|---|
| 0 hours | 0.579 | 71% | 32% | 1 |  |  |  |
|  | 0.251 | 82% | 18% | 2 | 1.0 | 0.4 | 2.5 |
|  | 0.154 | 94% | 7% | 3 | 0.4 | 0.1 | 1.6 |
|  | 1.56 | 29% | 71% | 4 | 1.0 | 0.4 | 2.4 |
|  | 1.81 | 29% | 80% |  |  |  |  |
|  | 2.88 | 18% | 90% |  |  |  |  |

# Fig. 8 - 5

| | | | | | | | |
|---|---|---|---|---|---|---|---|
| 24 hours | 0.579 | 71% | 32% | 1 | | | |
| | 0.251 | 82% | 18% | 2 | 0.4 | 0.1 | 1.7 |
| | 0.13 | 94% | 6% | 3 | 1.0 | 0.4 | 2.6 |
| | 1.56 | 29% | 71% | 4 | 1.0 | 0.4 | 2.6 |
| | 1.81 | 29% | 80% | | | | |
| | 2.88 | 18% | 90% | | | | |
| 48 hours | 0.801 | 70% | 46% | 1 | | | |
| | 0.746 | 80% | 44% | 2 | 4.3 | 0.3 | 57.3 |
| | 0.535 | 90% | 31% | 3 | 1.0 | 0.0 | 57.7 |
| | 1.56 | 40% | 71% | 4 | 4.3 | 0.3 | 57.3 |
| | 1.81 | 40% | 80% | | | | |
| | 2.88 | 30% | 90% | | | | |

sCr only

| Time prior AKI stage | Cutoff value | sens | spec | Quartile | OR | 95% CI of OR | |
|---|---|---|---|---|---|---|---|
| 0 hours | 0.315 | 75% | 19% | 1 | | | |
| | 0.168 | 88% | 7% | 2 | 0.3 | 0.0 | 4.7 |
| | 0.154 | 100% | 6% | 3 | 0.3 | 0.0 | 4.7 |
| | 1.57 | 38% | 71% | 4 | 1.0 | 0.2 | 4.0 |
| | 1.87 | 38% | 80% | | | | |
| | 2.88 | 25% | 90% | | | | |
| 24 hours | 0.315 | 75% | 19% | 1 | | | |
| | 0.168 | 88% | 7% | 2 | 0.3 | 0.0 | 4.7 |
| | 0.13 | 100% | 6% | 3 | 0.3 | 0.0 | 4.7 |
| | 1.57 | 38% | 71% | 4 | 1.0 | 0.2 | 4.0 |
| | 1.87 | 38% | 80% | | | | |
| | 2.88 | 25% | 90% | | | | |
| 48 hours | 1.28 | 80% | 63% | 1 | | | |
| | 1.28 | 80% | 63% | 2 | na | na | na |
| | 0.746 | 100% | 44% | 3 | na | na | na |
| | 1.57 | 60% | 71% | 4 | na | na | na |
| | 1.87 | 60% | 80% | | | | |
| | 2.88 | 40% | 90% | | | | |

UO only

| Time prior AKI stage | Cutoff value | sens | spec | Quartile | OR | 95% CI of OR | |
|---|---|---|---|---|---|---|---|
| 0 hours | 0.714 | 73% | 38% | 1 | | | |
| | 0.579 | 82% | 29% | 2 | 2.2 | 0.4 | 11.4 |
| | 0.243 | 91% | 17% | 3 | 1.0 | 0.1 | 8.4 |
| | 1.61 | 27% | 72% | 4 | 1.6 | 0.3 | 9.6 |
| | 1.9 | 18% | 81% | | | | |
| | 2.66 | 9% | 91% | | | | |
| 24 hours | 0.714 | 73% | 38% | 1 | | | |
| | 0.579 | 82% | 29% | 2 | 2.2 | 0.4 | 11.4 |
| | 0.243 | 91% | 17% | 3 | 1.0 | 0.1 | 8.4 |
| | 1.61 | 27% | 72% | 4 | 1.6 | 0.3 | 9.6 |
| | 1.9 | 18% | 81% | | | | |
| | 2.66 | 9% | 91% | | | | |
| 48 hours | 0.801 | 71% | 45% | 1 | | | |
| | 0.535 | 86% | 28% | 2 | 3.3 | 0.2 | 53.6 |
| | 0.243 | 100% | 17% | 3 | 1.0 | 0.0 | 59.8 |
| | 1.61 | 29% | 72% | 4 | 2.1 | 0.1 | 47.1 |
| | 1.9 | 14% | 81% | | | | |
| | 2.66 | 14% | 91% | | | | |

Endoglin
sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 4.456 | 3.462 | 4.456 | 3.420 | 4.456 | 3.351 |

Fig. 8 - 6

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| average | 4.367 | 3.832 | 4.367 | 4.080 | 4.367 | 3.639 |
| stdev | 1.261 | 1.235 | 1.261 | 1.386 | 1.261 | 1.017 |
| p (t-test) | | 0.238 | | 0.589 | | 0.187 |
| min | 1.904 | 2.586 | 1.904 | 2.613 | 1.904 | 2.613 |
| max | 7.699 | 6.466 | 7.699 | 6.466 | 7.699 | 5.452 |
| n (Samp) | 37 | 10 | 37 | 7 | 37 | 6 |
| n (Pat) | 37 | 10 | 37 | 7 | 37 | 6 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 3.972 | 3.420 | 3.972 | 3.420 | 3.972 | 3.194 |
| average | 4.217 | 3.962 | 4.217 | 4.372 | 4.217 | 3.194 |
| stdev | 1.545 | 1.406 | 1.545 | 1.816 | 1.545 | 0.320 |
| p (t-test) | | 0.722 | | 0.867 | | 0.357 |
| min | 1.904 | 3.190 | 1.904 | 3.229 | 1.904 | 2.968 |
| max | 12.339 | 6.466 | 12.339 | 6.466 | 12.339 | 3.420 |
| n (Samp) | 61 | 5 | 61 | 3 | 61 | 2 |
| n (Pat) | 61 | 5 | 61 | 3 | 61 | 2 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 4.456 | 3.765 | 4.456 | 3.690 | 4.456 | 3.690 |
| average | 4.356 | 3.702 | 4.356 | 3.861 | 4.356 | 3.861 |
| stdev | 1.194 | 1.189 | 1.194 | 1.222 | 1.194 | 1.222 |
| p (t-test) | | 0.270 | | 0.446 | | 0.446 |
| min | 2.309 | 2.586 | 2.309 | 2.613 | 2.309 | 2.613 |
| max | 6.988 | 5.452 | 6.988 | 5.452 | 6.988 | 5.452 |
| n (Samp) | 27 | 5 | 27 | 4 | 27 | 4 |
| n (Pat) | 27 | 5 | 27 | 4 | 27 | 4 |

sCr or UO

| Time prior AKI stage | AUC | SE | nCohort 1 | nCohort 2 | p |
|---|---|---|---|---|---|
| 0 hours | 0.36 | 0.093 | 37 | 10 | 0.124 |
| 24 hours | 0.42 | 0.115 | 37 | 7 | 0.511 |
| 48 hours | 0.33 | 0.109 | 37 | 6 | 0.116 |

sCr only

| Time prior AKI stage | AUC | SE | nCohort 1 | nCohort 2 | p |
|---|---|---|---|---|---|
| 0 hours | 0.41 | 0.125 | 61 | 5 | 0.456 |
| 24 hours | 0.50 | 0.172 | 61 | 3 | 0.987 |
| 48 hours | 0.21 | 0.128 | 61 | 2 | 0.025 |

UO only

| Time prior AKI stage | AUC | SE | nCohort 1 | nCohort 2 | p |
|---|---|---|---|---|---|
| 0 hours | 0.33 | 0.121 | 27 | 5 | 0.150 |
| 24 hours | 0.38 | 0.142 | 27 | 4 | 0.398 |
| 48 hours | 0.38 | 0.142 | 27 | 4 | 0.398 |

sCr or UO

| Time prior AKI stage | Cutoff value | sens | spec | Quartile | OR | 95% CI of OR | |
|---|---|---|---|---|---|---|---|
| 0 hours | 3.19018 | 70% | 22% | 1 | | | |
| | 3.18596 | 80% | 22% | 2 | 0.0 | 0.0 | na |
| | 2.58574 | 90% | 8% | 3 | 3.6 | 0.6 | 22.7 |
| | 4.80975 | 20% | 70% | 4 | 1.9 | 0.2 | 14.9 |
| | 5.0502 | 20% | 81% | | | | |
| | 6.0743 | 10% | 92% | | | | |
| 24 hours | 3.22853 | 71% | 22% | 1 | | | |
| | 3.18596 | 86% | 22% | 2 | 0.0 | 0.0 | na |

# Fig. 8 - 7

| Time prior AKI stage | Cutoff value | sens | spec | Quartile | OR | 95% CI of OR | |
|---|---|---|---|---|---|---|---|
| | 2.56967 | 100% | 8% | 3 | 1.0 | 0.1 | 11.0 |
| | 4.80975 | 29% | 70% | 4 | 1.7 | 0.2 | 13.7 |
| | 5.0502 | 29% | 81% | | | | |
| | 6.0743 | 14% | 92% | | | | |
| 48 hours | 2.91265 | 83% | 16% | 1 | | | |
| | 2.91265 | 83% | 16% | 2 | 0.0 | 0.0 | na |
| | 2.56967 | 100% | 8% | 3 | 3.8 | 0.2 | 79.5 |
| | 4.80975 | 17% | 70% | 4 | 2.5 | 0.1 | 73.5 |
| | 5.0502 | 17% | 81% | | | | |
| | 6.0743 | 0% | 92% | | | | |

sCr only

| Time prior AKI stage | Cutoff value | sens | spec | Quartile | OR | 95% CI of OR | |
|---|---|---|---|---|---|---|---|
| 0 hours | 3.19018 | 80% | 26% | 1 | | | |
| | 3.19018 | 80% | 26% | 2 | 0.0 | 0.0 | na |
| | 3.18596 | 100% | 26% | 3 | 4.9 | 0.3 | 75.0 |
| | 4.63957 | 20% | 70% | 4 | 0.0 | 0.0 | na |
| | 4.82362 | 20% | 80% | | | | |
| | 5.43169 | 20% | 90% | | | | |
| 24 hours | 3.18596 | 100% | 26% | 1 | | | |
| | 3.18596 | 100% | 26% | 2 | 0.0 | 0.0 | na |
| | 3.18596 | 100% | 26% | 3 | 2.1 | 0.1 | 53.1 |
| | 4.63957 | 33% | 70% | 4 | 0.0 | 0.0 | na |
| | 4.82362 | 33% | 80% | | | | |
| | 5.43169 | 33% | 90% | | | | |
| 48 hours | 2.91265 | 100% | 15% | 1 | | | |
| | 2.91265 | 100% | 15% | 2 | na | na | na |
| | 2.91265 | 100% | 15% | 3 | na | na | na |
| | 4.63957 | 0% | 70% | 4 | na | na | na |
| | 4.82362 | 0% | 80% | | | | |
| | 5.43169 | 0% | 90% | | | | |

UO only

| Time prior AKI stage | Cutoff value | sens | spec | Quartile | OR | 95% CI of OR | |
|---|---|---|---|---|---|---|---|
| 0 hours | 2.58574 | 80% | 7% | 1 | | | |
| | 2.58574 | 80% | 7% | 2 | 0.0 | 0.0 | na |
| | 2.56967 | 100% | 7% | 3 | 2.3 | 0.1 | 81.0 |
| | 4.69325 | 20% | 70% | 4 | 2.3 | 0.1 | 81.0 |
| | 5.3012 | 20% | 81% | | | | |
| | 6.0743 | 0% | 93% | | | | |
| 24 hours | 3.18596 | 75% | 22% | 1 | | | |
| | 2.56967 | 100% | 7% | 2 | 0.0 | 0.0 | na |
| | 2.56967 | 100% | 7% | 3 | 2.3 | 0.1 | 81.0 |
| | 4.69325 | 25% | 70% | 4 | 1.2 | 0.0 | 107.9 |
| | 5.3012 | 25% | 81% | | | | |
| | 6.0743 | 0% | 93% | | | | |
| 48 hours | 3.18596 | 75% | 22% | 1 | | | |
| | 2.56967 | 100% | 7% | 2 | 0.0 | 0.0 | na |
| | 2.56967 | 100% | 7% | 3 | 2.3 | 0.1 | 81.0 |
| | 4.69325 | 25% | 70% | 4 | 1.2 | 0.0 | 107.9 |
| | 5.3012 | 25% | 81% | | | | |
| | 6.0743 | 0% | 93% | | | | |

Erythropoietin
sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 319.500 | 535.000 | 319.500 | 442.000 | 319.500 | 513.000 |
| average | 512.317 | 2719.541 | 512.317 | 713.724 | 512.317 | 780.590 |
| stdev | 670.858 | 7957.845 | 670.858 | 680.991 | 670.858 | 713.599 |
| p (t-test) | | 0.004 | | 0.252 | | 0.230 |

# Fig. 8 - 8

| | | | | | | |
|---|---|---|---|---|---|---|
| min | 1.660 | 82.200 | 1.660 | 75.100 | 1.660 | 19.800 |
| max | 3430.000 | 33500.000 | 3430.000 | 2100.000 | 3430.000 | 2050.000 |
| n (Samp) | 112 | 17 | 112 | 17 | 112 | 10 |
| n (Pat) | 112 | 17 | 112 | 17 | 112 | 10 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 339.500 | 375.000 | 339.500 | 375.000 | 339.500 | 277.000 |
| average | 647.548 | 632.500 | 647.548 | 514.763 | 647.548 | 564.820 |
| stdev | 1136.425 | 526.384 | 1136.425 | 410.565 | 1136.425 | 528.191 |
| p (t-test) | | 0.970 | | 0.743 | | 0.872 |
| min | 1.660 | 101.000 | 1.660 | 75.100 | 1.660 | 75.100 |
| max | 11600.000 | 1460.000 | 11600.000 | 1270.000 | 11600.000 | 1270.000 |
| n (Samp) | 180 | 8 | 180 | 8 | 180 | 5 |
| n (Pat) | 180 | 8 | 180 | 8 | 180 | 5 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 323.000 | 977.000 | 323.000 | 442.000 | 323.000 | 584.000 |
| average | 595.076 | 3878.200 | 595.076 | 863.927 | 595.076 | 924.257 |
| stdev | 875.340 | 9849.075 | 875.340 | 789.327 | 875.340 | 779.512 |
| p (t-test) | | 0.002 | | 0.334 | | 0.337 |
| min | 1.660 | 82.200 | 1.660 | 82.200 | 1.660 | 19.800 |
| max | 5380.000 | 33500.000 | 5380.000 | 2100.000 | 5380.000 | 2050.000 |
| n (Samp) | 89 | 11 | 89 | 11 | 89 | 7 |
| n (Pat) | 89 | 11 | 89 | 11 | 89 | 7 |

sCr or UO

| Time prior AKI stage | AUC | SE | nCohort 1 | nCohort 2 | p |
|---|---|---|---|---|---|
| 0 hours | 0.68 | 0.075 | 112 | 17 | 0.014 |
| 24 hours | 0.62 | 0.077 | 112 | 17 | 0.109 |
| 48 hours | 0.63 | 0.098 | 112 | 10 | 0.189 |

sCr only

| Time prior AKI stage | AUC | SE | nCohort 1 | nCohort 2 | p |
|---|---|---|---|---|---|
| 0 hours | 0.60 | 0.108 | 180 | 8 | 0.375 |
| 24 hours | 0.56 | 0.107 | 180 | 8 | 0.580 |
| 48 hours | 0.56 | 0.135 | 180 | 5 | 0.680 |

UO only

| Time prior AKI stage | AUC | SE | nCohort 1 | nCohort 2 | p |
|---|---|---|---|---|---|
| 0 hours | 0.72 | 0.091 | 89 | 11 | 0.018 |
| 24 hours | 0.65 | 0.094 | 89 | 11 | 0.120 |
| 48 hours | 0.65 | 0.116 | 89 | 7 | 0.183 |

sCr or UO

| Time prior AKI stage | Cutoff value | sens | spec | Quartile | OR | 95% CI of OR | |
|---|---|---|---|---|---|---|---|
| 0 hours | 292 | 71% | 48% | 1 | | | |
| | 248 | 82% | 45% | 2 | 5.7 | 0.5 | 69.1 |
| | 84.3 | 94% | 28% | 3 | 3.2 | 0.2 | 49.9 |
| | 591 | 47% | 71% | 4 | 9.9 | 0.9 | 103.7 |
| | 741 | 47% | 80% | | | | |
| | 1040 | 35% | 90% | | | | |
| 24 hours | 285 | 71% | 46% | 1 | | | |
| | 218 | 82% | 43% | 2 | 2.8 | 0.6 | 12.6 |
| | 79.8 | 94% | 27% | 3 | 2.8 | 0.6 | 12.6 |
| | 591 | 29% | 71% | 4 | 2.7 | 0.6 | 12.1 |
| | 741 | 29% | 80% | | | | |

# Fig. 8 - 9

|  | 1040 | 24% | 90% |  |  |  |  |
|---|---|---|---|---|---|---|---|
| 48 hours | 323 | 70% | 51% | 1 |  |  |  |
|  | 248 | 80% | 45% | 2 | 3.1 | 0.2 | 48.6 |
|  | 74.1 | 90% | 26% | 3 | 2.1 | 0.1 | 45.0 |
|  | 591 | 40% | 71% | 4 | 4.3 | 0.3 | 57.3 |
|  | 741 | 40% | 80% |  |  |  |  |
|  | 1040 | 30% | 90% |  |  |  |  |

sCr only

| Time prior AKI stage | Cutoff value | sens | spec | Quartile | OR | 95% CI of OR | |
|---|---|---|---|---|---|---|---|
| 0 hours | 272 | 75% | 44% | 1 |  |  |  |
|  | 211 | 88% | 42% | 2 | na | na | na |
|  | 98 | 100% | 27% | 3 | na | na | na |
|  | 601 | 38% | 70% | 4 | na | na | na |
|  | 869 | 38% | 80% |  |  |  |  |
|  | 1460 | 0% | 90% |  |  |  |  |
| 24 hours | 272 | 75% | 44% | 1 |  |  |  |
|  | 211 | 88% | 42% | 2 | 3.1 | 0.2 | 46.7 |
|  | 74.1 | 100% | 23% | 3 | 2.0 | 0.1 | 42.2 |
|  | 601 | 25% | 70% | 4 | 2.0 | 0.1 | 42.2 |
|  | 869 | 25% | 80% |  |  |  |  |
|  | 1460 | 0% | 90% |  |  |  |  |
| 48 hours | 211 | 80% | 42% | 1 |  |  |  |
|  | 211 | 80% | 42% | 2 | 2.0 | 0.1 | 42.3 |
|  | 74.1 | 100% | 23% | 3 | 0.0 | 0.0 | na |
|  | 601 | 40% | 70% | 4 | 2.0 | 0.1 | 41.3 |
|  | 869 | 40% | 80% |  |  |  |  |
|  | 1460 | 0% | 90% |  |  |  |  |

UO only

| Time prior AKI stage | Cutoff value | sens | spec | Quartile | OR | 95% CI of OR | |
|---|---|---|---|---|---|---|---|
| 0 hours | 412 | 73% | 60% | 1 |  |  |  |
|  | 248 | 82% | 45% | 2 | 2.1 | 0.1 | 46.6 |
|  | 211 | 91% | 43% | 3 | 2.1 | 0.1 | 46.6 |
|  | 601 | 55% | 71% | 4 | 7.6 | 0.6 | 89.7 |
|  | 815 | 55% | 81% |  |  |  |  |
|  | 1480 | 27% | 91% |  |  |  |  |
| 24 hours | 248 | 73% | 45% | 1 |  |  |  |
|  | 218 | 82% | 43% | 2 | 3.3 | 0.2 | 53.0 |
|  | 211 | 91% | 43% | 3 | 3.3 | 0.2 | 53.0 |
|  | 601 | 36% | 71% | 4 | 4.6 | 0.3 | 63.1 |
|  | 815 | 36% | 81% |  |  |  |  |
|  | 1480 | 27% | 91% |  |  |  |  |
| 48 hours | 412 | 71% | 60% | 1 |  |  |  |
|  | 323 | 86% | 51% | 2 | 1.0 | 0.0 | 59.8 |
|  | 15.7 | 100% | 7% | 3 | 2.1 | 0.1 | 47.1 |
|  | 601 | 43% | 71% | 4 | 3.3 | 0.2 | 53.6 |
|  | 815 | 43% | 81% |  |  |  |  |
|  | 1480 | 29% | 91% |  |  |  |  |

Fractalkine
sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.586 | 1.892 | 0.586 | 1.844 | 0.586 | 0.801 |
| average | 0.790 | 2.035 | 0.790 | 1.499 | 0.790 | 1.220 |
| stdev | 0.708 | 1.503 | 0.708 | 0.907 | 0.708 | 0.808 |
| p (t-test) |  | 0.000 |  | 0.001 |  | 0.089 |
| min | 0.021 | 0.318 | 0.021 | 0.318 | 0.021 | 0.318 |
| max | 5.115 | 5.673 | 5.115 | 2.709 | 5.115 | 2.338 |
| n (Samp) | 93 | 15 | 93 | 13 | 93 | 9 |

# Fig. 8 - 10

| n (Pat) | 93 | 15 | 93 | 13 | 93 | 9 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.634 | 1.550 | 0.634 | 1.550 | 0.634 | 0.552 |
| average | 0.797 | 2.162 | 0.797 | 1.519 | 0.797 | 0.940 |
| stdev | 0.822 | 2.011 | 0.822 | 1.038 | 0.822 | 0.951 |
| p (t-test) | | 0.000 | | 0.026 | | 0.732 |
| min | 0.021 | 0.318 | 0.021 | 0.318 | 0.021 | 0.318 |
| max | 7.305 | 5.673 | 7.305 | 2.709 | 7.305 | 2.338 |
| n (Samp) | 152 | 7 | 152 | 7 | 152 | 4 |
| n (Pat) | 152 | 7 | 152 | 7 | 152 | 4 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.590 | 1.832 | 0.590 | 1.322 | 0.590 | 1.256 |
| average | 0.702 | 1.731 | 0.702 | 1.346 | 0.702 | 1.264 |
| stdev | 0.463 | 1.020 | 0.463 | 0.803 | 0.463 | 0.764 |
| p (t-test) | | 0.000 | | 0.001 | | 0.008 |
| min | 0.082 | 0.363 | 0.082 | 0.363 | 0.082 | 0.363 |
| max | 2.449 | 3.813 | 2.449 | 2.517 | 2.449 | 2.233 |
| n (Samp) | 77 | 10 | 77 | 8 | 77 | 6 |
| n (Pat) | 77 | 10 | 77 | 8 | 77 | 6 |

sCr or UO

| Time prior AKI stage | AUC | SE | nCohort 1 | nCohort 2 | p |
|---|---|---|---|---|---|
| 0 hours | 0.79 | 0.072 | 93 | 15 | 0.000 |
| 24 hours | 0.74 | 0.082 | 93 | 13 | 0.004 |
| 48 hours | 0.67 | 0.103 | 93 | 9 | 0.091 |

sCr only

| Time prior AKI stage | AUC | SE | nCohort 1 | nCohort 2 | p |
|---|---|---|---|---|---|
| 0 hours | 0.71 | 0.112 | 152 | 7 | 0.055 |
| 24 hours | 0.71 | 0.112 | 152 | 7 | 0.061 |
| 48 hours | 0.51 | 0.148 | 152 | 4 | 0.933 |

UO only

| Time prior AKI stage | AUC | SE | nCohort 1 | nCohort 2 | p |
|---|---|---|---|---|---|
| 0 hours | 0.82 | 0.083 | 77 | 10 | 0.000 |
| 24 hours | 0.75 | 0.103 | 77 | 8 | 0.016 |
| 48 hours | 0.73 | 0.120 | 77 | 6 | 0.052 |

sCr or UO

| Time prior AKI stage | Cutoff value | sens | spec | Quartile | OR | 95% CI of OR | |
|---|---|---|---|---|---|---|---|
| 0 hours | 0.79808 | 73% | 63% | 1 | | | |
| | 0.72115 | 80% | 57% | 2 | 2.1 | 0.1 | 45.9 |
| | 0.35156 | 93% | 29% | 3 | 2.1 | 0.1 | 45.9 |
| | 0.97952 | 67% | 71% | 4 | 15.3 | 1.5 | 159.8 |
| | 1.15 | 67% | 81% | | | | |
| | 1.58784 | 60% | 90% | | | | |
| 24 hours | 0.72115 | 77% | 57% | 1 | | | |
| | 0.58594 | 85% | 51% | 2 | 2.0 | 0.1 | 44.3 |
| | 0.35156 | 92% | 29% | 3 | 3.3 | 0.2 | 52.4 |
| | 0.97952 | 54% | 71% | 4 | 8.8 | 0.8 | 98.0 |
| | 1.15 | 54% | 81% | | | | |
| | 1.58784 | 54% | 90% | | | | |
| 48 hours | 0.58594 | 78% | 51% | 1 | | | |
| | 0.35156 | 89% | 29% | 2 | 2.0 | 0.1 | 44.5 |

# Fig. 8 - 11

| | 0.30273 | 100% | 22% | 3 | 2.1 | 0.1 | 46.6 |
| | 0.97952 | 44% | 71% | 4 | 4.4 | 0.3 | 60.0 |
| | 1.15 | 44% | 81% | | | | |
| | 1.58784 | 44% | 90% | | | | |

sCr only

| Time prior AKI stage | Cutoff value | sens | spec | Quartile | OR | 95% CI of OR | |
|---|---|---|---|---|---|---|---|
| 0 hours | 0.79808 | 71% | 64% | 1 | | | |
| | 0.35156 | 86% | 27% | 2 | 1.0 | 0.0 | 54.4 |
| | 0.30273 | 100% | 22% | 3 | 1.0 | 0.0 | 54.4 |
| | 0.92 | 57% | 70% | 4 | 4.2 | 0.3 | 54.4 |
| | 1.1 | 57% | 80% | | | | |
| | 1.38889 | 57% | 90% | | | | |
| 24 hours | 0.79808 | 71% | 64% | 1 | | | |
| | 0.35156 | 86% | 27% | 2 | 1.0 | 0.0 | 54.4 |
| | 0.30273 | 100% | 22% | 3 | 1.0 | 0.0 | 54.4 |
| | 0.92 | 57% | 70% | 4 | 4.2 | 0.3 | 54.4 |
| | 1.1 | 57% | 80% | | | | |
| | 1.38889 | 57% | 90% | | | | |
| 48 hours | 0.35156 | 75% | 27% | 1 | | | |
| | 0.30273 | 100% | 22% | 2 | 1.0 | 0.0 | 55.9 |
| | 0.30273 | 100% | 22% | 3 | 1.0 | 0.0 | 55.9 |
| | 0.92 | 25% | 70% | 4 | 1.0 | 0.0 | 55.9 |
| | 1.1 | 25% | 80% | | | | |
| | 1.38889 | 25% | 90% | | | | |

Fig. 8 - 12

**Interleukin-1 receptor, type II**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 11561.532 | 26780.525 | 11561.532 | 26780.525 | 11561.532 | 26780.525 |
| average | 14131.723 | 44733.694 | 14131.723 | 44733.694 | 14131.723 | 27371.197 |
| stdev | 12617.339 | 54021.511 | 12617.339 | 54021.511 | 12617.339 | 15763.194 |
| p (t-test) | | 0.012 | | 0.012 | | 0.069 |
| min | 3061.967 | 7943.978 | 3061.967 | 7943.978 | 3061.967 | 11212.050 |
| max | 56549.592 | 150973.398 | 56549.592 | 150973.398 | 56549.592 | 44711.687 |
| n (Samp) | 25 | 6 | 25 | 6 | 25 | 4 |
| n (Pat) | 25 | 6 | 25 | 6 | 25 | 4 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 11557.889 | 44711.687 | 11557.889 | 44711.687 | 11557.889 | 27961.868 |
| average | 14136.838 | 68965.712 | 14136.838 | 68965.712 | 14136.838 | 27961.868 |
| stdev | 10963.563 | 72969.185 | 10963.563 | 72969.185 | 10963.563 | 23687.821 |
| p (t-test) | | 0.000 | | 0.000 | | 0.101 |
| min | 2160.038 | 11212.050 | 2160.038 | 11212.050 | 2160.038 | 11212.050 |
| max | 56549.592 | 150973.398 | 56549.592 | 150973.398 | 56549.592 | 44711.687 |
| n (Samp) | 44 | 3 | 44 | 3 | 44 | 2 |
| n (Pat) | 44 | 3 | 44 | 3 | 44 | 2 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 11255.704 | 14214.467 | 11255.704 | 14214.467 | 11255.704 | 17216.885 |
| average | 14181.425 | 18179.269 | 14181.425 | 18179.269 | 14181.425 | 21591.033 |
| stdev | 12668.036 | 12704.237 | 12668.036 | 12704.237 | 12668.036 | 13124.621 |
| p (t-test) | | 0.563 | | 0.563 | | 0.349 |
| min | 3061.967 | 7943.978 | 3061.967 | 7943.978 | 3061.967 | 11212.050 |
| max | 56549.592 | 36344.165 | 56549.592 | 36344.165 | 56549.592 | 36344.165 |
| n (Samp) | 25 | 4 | 25 | 4 | 25 | 3 |
| n (Pat) | 25 | 4 | 25 | 4 | 25 | 3 |

sCr or UO

| Time prior AKI stage | AUC | SE | nCohort 1 | nCohort 2 | p |
|---|---|---|---|---|---|
| 0 hours | 0.75 | 0.124 | 25 | 6 | 0.047 |
| 24 hours | 0.75 | 0.124 | 25 | 6 | 0.047 |
| 48 hours | 0.79 | 0.142 | 25 | 4 | 0.041 |

sCr only

| Time prior AKI stage | AUC | SE | nCohort 1 | nCohort 2 | p |
|---|---|---|---|---|---|
| 0 hours | 0.81 | 0.155 | 44 | 3 | 0.045 |
| 24 hours | 0.81 | 0.155 | 44 | 3 | 0.045 |
| 48 hours | 0.72 | 0.210 | 44 | 2 | 0.305 |

UO only

| Time prior AKI stage | AUC | SE | nCohort 1 | nCohort 2 | p |
|---|---|---|---|---|---|
| 0 hours | 0.63 | 0.161 | 25 | 4 | 0.419 |
| 24 hours | 0.63 | 0.161 | 25 | 4 | 0.419 |
| 48 hours | 0.75 | 0.172 | 25 | 3 | 0.150 |

# Fig. 8 - 13

Lymphotoxin-alpha

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.456 | 5.020 | 0.456 | 4.000 | 0.456 | 3.575 |
| average | 2.966 | 7.587 | 2.966 | 6.094 | 2.966 | 5.928 |
| stdev | 6.398 | 9.262 | 6.398 | 6.957 | 6.398 | 7.702 |
| p (t-test) | | 0.010 | | 0.066 | | 0.170 |
| min | 0.456 | 0.456 | 0.456 | 0.456 | 0.456 | 0.456 |
| max | 42.200 | 31.600 | 42.200 | 23.200 | 42.200 | 23.200 |
| n (Samp) | 112 | 17 | 112 | 17 | 112 | 10 |
| n (Pat) | 112 | 17 | 112 | 17 | 112 | 10 |

sCr only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.456 | 5.025 | 0.456 | 4.510 | 0.456 | 5.020 |
| average | 4.347 | 8.049 | 4.347 | 7.778 | 4.347 | 8.041 |
| stdev | 24.989 | 8.394 | 24.989 | 8.540 | 24.989 | 8.847 |
| p (t-test) | | 0.677 | | 0.700 | | 0.742 |
| min | 0.456 | 0.456 | 0.456 | 0.456 | 0.456 | 0.456 |
| max | 330.000 | 23.200 | 330.000 | 23.200 | 330.000 | 23.200 |
| n (Samp) | 180 | 8 | 180 | 8 | 180 | 5 |
| n (Pat) | 180 | 8 | 180 | 8 | 180 | 5 |

UO only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 0.456 | 5.020 | 0.456 | 5.020 | 0.456 | 3.150 |
| average | 2.813 | 7.013 | 2.813 | 4.903 | 2.813 | 4.518 |
| stdev | 5.751 | 9.383 | 5.751 | 4.784 | 5.751 | 5.597 |
| p (t-test) | | 0.037 | | 0.251 | | 0.451 |
| min | 0.456 | 0.456 | 0.456 | 0.456 | 0.456 | 0.456 |
| max | 34.100 | 31.600 | 34.100 | 16.100 | 34.100 | 16.100 |
| n (Samp) | 89 | 11 | 89 | 11 | 89 | 7 |
| n (Pat) | 89 | 11 | 89 | 11 | 89 | 7 |

sCr or UO

| Time prior AKI stage | AUC | SE | nCohort 1 | nCohort 2 | p |
|---|---|---|---|---|---|
| 0 hours | 0.70 | 0.075 | 112 | 17 | 0.008 |
| 24 hours | 0.69 | 0.075 | 112 | 17 | 0.012 |
| 48 hours | 0.66 | 0.098 | 112 | 10 | 0.103 |

sCr only

| Time prior AKI stage | AUC | SE | nCohort 1 | nCohort 2 | p |
|---|---|---|---|---|---|
| 0 hours | 0.76 | 0.100 | 180 | 8 | 0.008 |
| 24 hours | 0.75 | 0.101 | 180 | 8 | 0.014 |
| 48 hours | 0.79 | 0.122 | 180 | 5 | 0.018 |

UO only

| Time prior AKI stage | AUC | SE | nCohort 1 | nCohort 2 | p |
|---|---|---|---|---|---|
| 0 hours | 0.70 | 0.092 | 89 | 11 | 0.034 |
| 24 hours | 0.69 | 0.093 | 89 | 11 | 0.040 |
| 48 hours | 0.64 | 0.117 | 89 | 7 | 0.219 |

# Fig. 8 - 14

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | na | na | na |
| | 0 | 100% | 0% | 3 | na | na | na |
| | 2.32 | 65% | 71% | 4 | na | na | na |
| | 2.94 | 65% | 81% | | | | |
| | 5.9 | 41% | 90% | | | | |
| 24 hours | 0 | 100% | 0% | 1 | | | |
| | 0 | 100% | 0% | 2 | na | na | na |
| | 0 | 100% | 0% | 3 | na | na | na |
| | 2.32 | 65% | 71% | 4 | na | na | na |
| | 2.94 | 59% | 81% | | | | |
| | 5.9 | 41% | 90% | | | | |
| 48 hours | 0 | 100% | 0% | 1 | | | |
| | 0 | 100% | 0% | 2 | 3.1 | 0.2 | 48.6 |
| | 0 | 100% | 0% | 3 | 0.0 | 0.0 | na |
| | 2.32 | 60% | 71% | 4 | 7.0 | 0.6 | 79.3 |
| | 2.94 | 60% | 81% | | | | |
| | 5.9 | 30% | 90% | | | | |

Fig. 8 - 15

**Matrix metalloproteinase-3**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.075 | 0.360 | 0.075 | 0.169 | 0.075 | 0.166 |
| average | 0.164 | 0.381 | 0.164 | 0.290 | 0.164 | 0.238 |
| stdev | 0.254 | 0.332 | 0.254 | 0.300 | 0.254 | 0.264 |
| p (t-test) | | 0.002 | | 0.065 | | 0.380 |
| min | 0.002 | 0.002 | 0.002 | 0.002 | 0.002 | 0.002 |
| max | 1.490 | 0.996 | 1.490 | 0.927 | 1.490 | 0.927 |
| n (Samp) | 112 | 17 | 112 | 17 | 112 | 10 |
| n (Pat) | 112 | 17 | 112 | 17 | 112 | 10 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.086 | 0.205 | 0.086 | 0.120 | 0.086 | 0.150 |
| average | 0.195 | 0.373 | 0.195 | 0.268 | 0.195 | 0.274 |
| stdev | 0.319 | 0.381 | 0.319 | 0.341 | 0.319 | 0.368 |
| p (t-test) | | 0.128 | | 0.528 | | 0.590 |
| min | 0.002 | 0.062 | 0.002 | 0.002 | 0.002 | 0.062 |
| max | 2.160 | 0.996 | 2.160 | 0.927 | 2.160 | 0.927 |
| n (Samp) | 180 | 8 | 180 | 8 | 180 | 5 |
| n (Pat) | 180 | 8 | 180 | 8 | 180 | 5 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.068 | 0.360 | 0.068 | 0.194 | 0.068 | 0.169 |
| average | 0.148 | 0.355 | 0.148 | 0.290 | 0.148 | 0.176 |
| stdev | 0.217 | 0.285 | 0.217 | 0.254 | 0.217 | 0.122 |
| p (t-test) | | 0.005 | | 0.048 | | 0.743 |
| min | 0.002 | 0.002 | 0.002 | 0.002 | 0.002 | 0.002 |
| max | 1.190 | 0.872 | 1.190 | 0.872 | 1.190 | 0.366 |
| n (Samp) | 89 | 11 | 89 | 11 | 89 | 7 |
| n (Pat) | 89 | 11 | 89 | 11 | 89 | 7 |

sCr or UO

| Time prior AKI stage | AUC | SE | nCohort 1 | nCohort 2 | p |
|---|---|---|---|---|---|
| 0 hours | 0.75 | 0.072 | 112 | 17 | 0.001 |
| 24 hours | 0.65 | 0.076 | 112 | 17 | 0.051 |
| 48 hours | 0.66 | 0.097 | 112 | 10 | 0.093 |

sCr only

| Time prior AKI stage | AUC | SE | nCohort 1 | nCohort 2 | p |
|---|---|---|---|---|---|
| 0 hours | 0.71 | 0.105 | 180 | 8 | 0.041 |
| 24 hours | 0.57 | 0.108 | 180 | 8 | 0.542 |
| 48 hours | 0.63 | 0.136 | 180 | 5 | 0.332 |

UO only

| Time prior AKI stage | AUC | SE | nCohort 1 | nCohort 2 | p |
|---|---|---|---|---|---|
| 0 hours | 0.76 | 0.087 | 89 | 11 | 0.002 |
| 24 hours | 0.72 | 0.091 | 89 | 11 | 0.015 |
| 48 hours | 0.65 | 0.117 | 89 | 7 | 0.195 |

sCr or UO

| Time prior AKI stage | Cutoff value | sens | spec | Quartile | OR | 95% CI of OR | |
|---|---|---|---|---|---|---|---|
| 0 hours | 0.145 | 71% | 68% | 1 | | | |

# Fig. 8 - 16

| | 0.0898 | 82% | 54% | 2 | 3.2 | 0.2 | 49.9 |
|---|---|---|---|---|---|---|---|
| | 0.0591 | 94% | 46% | 3 | 3.2 | 0.2 | 49.9 |
| | 0.161 | 65% | 71% | 4 | 13.5 | 1.3 | 135.0 |
| | 0.222 | 59% | 80% | | | | |
| | 0.407 | 29% | 90% | | | | |
| 24 hours | 0.0678 | 71% | 49% | 1 | | | |
| | 0.0591 | 82% | 46% | 2 | 1.0 | 0.2 | 4.2 |
| | 0 | 100% | 0% | 3 | 1.4 | 0.4 | 5.0 |
| | 0.161 | 53% | 71% | 4 | 2.6 | 0.9 | 7.6 |
| | 0.222 | 41% | 80% | | | | |
| | 0.407 | 24% | 90% | | | | |
| 48 hours | 0.145 | 70% | 68% | 1 | | | |
| | 0.0678 | 80% | 49% | 2 | 2.0 | 0.1 | 43.3 |
| | 0.0591 | 90% | 46% | 3 | 3.2 | 0.2 | 50.6 |
| | 0.161 | 60% | 71% | 4 | 4.3 | 0.3 | 57.3 |
| | 0.222 | 30% | 80% | | | | |
| | 0.407 | 10% | 90% | | | | |

sCr only

| Time prior AKI stage | Cutoff value | sens | spec | Quartile | OR | 95% CI of OR | |
|---|---|---|---|---|---|---|---|
| 0 hours | 0.0912 | 75% | 53% | 1 | | | |
| | 0.0898 | 88% | 52% | 2 | na | na | na |
| | 0.0591 | 100% | 42% | 3 | na | na | na |
| | 0.183 | 50% | 70% | 4 | na | na | na |
| | 0.282 | 38% | 80% | | | | |
| | 0.518 | 25% | 90% | | | | |
| 24 hours | 0.0591 | 75% | 42% | 1 | | | |
| | 0 | 100% | 0% | 2 | 0.5 | 0.0 | 10.1 |
| | 0 | 100% | 0% | 3 | 1.0 | 0.1 | 7.7 |
| | 0.183 | 38% | 70% | 4 | 1.5 | 0.3 | 8.6 |
| | 0.282 | 25% | 80% | | | | |
| | 0.518 | 25% | 90% | | | | |
| 48 hours | 0.0657 | 80% | 43% | 1 | | | |
| | 0.0657 | 80% | 43% | 2 | na | na | na |
| | 0.0591 | 100% | 42% | 3 | na | na | na |
| | 0.183 | 20% | 70% | 4 | na | na | na |
| | 0.282 | 20% | 80% | | | | |
| | 0.518 | 20% | 90% | | | | |

UO only

| Time prior AKI stage | Cutoff value | sens | spec | Quartile | OR | 95% CI of OR | |
|---|---|---|---|---|---|---|---|
| 0 hours | 0.161 | 73% | 72% | 1 | | | |
| | 0.145 | 82% | 70% | 2 | 1.0 | 0.0 | 59.3 |
| | 0.0678 | 91% | 51% | 3 | 2.1 | 0.1 | 46.6 |
| | 0.159 | 73% | 71% | 4 | 9.3 | 0.8 | 106.1 |
| | 0.222 | 64% | 81% | | | | |
| | 0.383 | 27% | 91% | | | | |
| 24 hours | 0.145 | 73% | 70% | 1 | | | |
| | 0.0678 | 82% | 51% | 2 | 2.1 | 0.1 | 46.6 |
| | 0.0591 | 91% | 48% | 3 | 3.3 | 0.2 | 53.0 |
| | 0.159 | 64% | 71% | 4 | 6.0 | 0.5 | 75.4 |
| | 0.222 | 45% | 81% | | | | |
| | 0.383 | 27% | 91% | | | | |
| 48 hours | 0.145 | 71% | 70% | 1 | | | |
| | 0.0678 | 86% | 51% | 2 | na | na | na |
| | 0 | 100% | 0% | 3 | na | na | na |
| | 0.159 | 57% | 71% | 4 | na | na | na |
| | 0.222 | 29% | 81% | | | | |
| | 0.383 | 0% | 91% | | | | |

# Fig. 8 - 17

**MDC (C-C motif chemokine 22)**

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 306.500 | 252.000 | 306.500 | 252.000 | 306.500 | 291.000 |
| average | 343.004 | 263.435 | 343.004 | 258.376 | 343.004 | 286.800 |
| stdev | 203.248 | 129.525 | 203.248 | 124.923 | 203.248 | 145.899 |
| p (t-test) | | 0.120 | | 0.098 | | 0.395 |
| min | 39.200 | 56.700 | 39.200 | 56.700 | 39.200 | 47.000 |
| max | 1170.000 | 489.000 | 1170.000 | 489.000 | 1170.000 | 489.000 |
| n (Samp) | 112 | 17 | 112 | 17 | 112 | 10 |
| n (Pat) | 112 | 17 | 112 | 17 | 112 | 10 |

sCr only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 303.500 | 233.500 | 303.500 | 233.500 | 303.500 | 311.000 |
| average | 334.333 | 284.125 | 334.333 | 276.000 | 334.333 | 316.000 |
| stdev | 207.841 | 116.726 | 207.841 | 107.237 | 207.841 | 117.235 |
| p (t-test) | | 0.499 | | 0.432 | | 0.845 |
| min | 39.200 | 161.000 | 39.200 | 161.000 | 39.200 | 207.000 |
| max | 1560.000 | 489.000 | 1560.000 | 489.000 | 1560.000 | 489.000 |
| n (Samp) | 180 | 8 | 180 | 8 | 180 | 5 |
| n (Pat) | 180 | 8 | 180 | 8 | 180 | 5 |

UO only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 312.000 | 283.000 | 312.000 | 283.000 | 312.000 | 271.000 |
| average | 355.057 | 264.127 | 355.057 | 262.218 | 355.057 | 284.000 |
| stdev | 211.789 | 149.025 | 211.789 | 148.990 | 211.789 | 172.682 |
| p (t-test) | | 0.171 | | 0.162 | | 0.390 |
| min | 56.100 | 56.700 | 56.100 | 56.700 | 56.100 | 47.000 |
| max | 1170.000 | 489.000 | 1170.000 | 489.000 | 1170.000 | 489.000 |
| n (Samp) | 89 | 11 | 89 | 11 | 89 | 7 |
| n (Pat) | 89 | 11 | 89 | 11 | 89 | 7 |

sCr or UO

| Time prior AKI stage | AUC | SE | nCohort 1 | nCohort 2 | p |
| --- | --- | --- | --- | --- | --- |
| 0 hours | 0.38 | 0.069 | 112 | 17 | 0.092 |
| 24 hours | 0.37 | 0.068 | 112 | 17 | 0.062 |
| 48 hours | 0.43 | 0.091 | 112 | 10 | 0.452 |

sCr only

| Time prior AKI stage | AUC | SE | nCohort 1 | nCohort 2 | p |
| --- | --- | --- | --- | --- | --- |
| 0 hours | 0.43 | 0.099 | 180 | 8 | 0.508 |
| 24 hours | 0.42 | 0.098 | 180 | 8 | 0.408 |
| 48 hours | 0.51 | 0.132 | 180 | 5 | 0.970 |

UO only

| Time prior AKI stage | AUC | SE | nCohort 1 | nCohort 2 | p |
| --- | --- | --- | --- | --- | --- |
| 0 hours | 0.38 | 0.083 | 89 | 11 | 0.140 |
| 24 hours | 0.37 | 0.082 | 89 | 11 | 0.111 |
| 48 hours | 0.41 | 0.106 | 89 | 7 | 0.382 |

sCr or UO

| Time prior AKI stage | Cutoff value | sens | spec | Quartile | OR | 95% CI of OR | |
| --- | --- | --- | --- | --- | --- | --- | --- |
| 0 hours | 205 | 71% | 23% | 1 | | | |
| | 156 | 82% | 15% | 2 | 2.9 | 0.6 | 13.0 |
| | 67.6 | 94% | 4% | 3 | 2.2 | 0.4 | 11.0 |
| | 412 | 18% | 71% | 4 | 3.6 | 0.8 | 15.2 |
| | 480 | 6% | 80% | | | | |

# Fig. 8 - 18

| | 572 | 0% | 90% | | | | |
|---|---|---|---|---|---|---|---|
| 24 hours | 205 | 71% | 23% | 1 | | | |
| | 156 | 82% | 15% | 2 | 2.2 | 0.4 | 11.0 |
| | 67.6 | 94% | 4% | 3 | 2.9 | 0.6 | 13.0 |
| | 412 | 12% | 71% | 4 | 3.6 | 0.8 | 15.2 |
| | 480 | 6% | 80% | | | | |
| | 572 | 0% | 90% | | | | |
| 48 hours | 205 | 70% | 23% | 1 | | | |
| | 176 | 80% | 20% | 2 | 1.6 | 0.3 | 9.5 |
| | 112 | 90% | 11% | 3 | 0.5 | 0.0 | 10.4 |
| | 412 | 20% | 71% | 4 | 2.2 | 0.4 | 11.2 |
| | 480 | 10% | 80% | | | | |
| | 572 | 0% | 90% | | | | |

sCr only

| Time prior AKI stage | Cutoff value | sens | spec | Quartile | OR | 95% CI of OR | |
|---|---|---|---|---|---|---|---|
| 0 hours | 209 | 75% | 28% | 1 | | | |
| | 205 | 88% | 26% | 2 | 0.5 | 0.0 | 10.1 |
| | 156 | 100% | 16% | 3 | 2.1 | 0.4 | 10.0 |
| | 401 | 25% | 70% | 4 | 0.5 | 0.0 | 10.1 |
| | 469 | 13% | 80% | | | | |
| | 534 | 0% | 90% | | | | |
| 24 hours | 209 | 75% | 28% | 1 | | | |
| | 205 | 88% | 26% | 2 | 2.0 | 0.1 | 42.2 |
| | 156 | 100% | 16% | 3 | 4.3 | 0.3 | 54.2 |
| | 401 | 13% | 70% | 4 | 1.0 | 0.0 | 54.9 |
| | 469 | 13% | 80% | | | | |
| | 534 | 0% | 90% | | | | |
| 48 hours | 209 | 80% | 28% | 1 | | | |
| | 209 | 80% | 28% | 2 | na | na | na |
| | 205 | 100% | 26% | 3 | na | na | na |
| | 401 | 20% | 70% | 4 | na | na | na |
| | 469 | 20% | 80% | | | | |
| | 534 | 0% | 90% | | | | |

UO only

| Time prior AKI stage | Cutoff value | sens | spec | Quartile | OR | 95% CI of OR | |
|---|---|---|---|---|---|---|---|
| 0 hours | 176 | 73% | 18% | 1 | | | |
| | 112 | 82% | 10% | 2 | 1.6 | 0.3 | 9.6 |
| | 67.6 | 91% | 2% | 3 | 1.0 | 0.1 | 8.4 |
| | 427 | 18% | 71% | 4 | 2.2 | 0.4 | 11.4 |
| | 491 | 0% | 81% | | | | |
| | 576 | 0% | 91% | | | | |
| 24 hours | 176 | 73% | 18% | 1 | | | |
| | 112 | 82% | 10% | 2 | 1.0 | 0.1 | 8.4 |
| | 67.6 | 91% | 2% | 3 | 1.6 | 0.3 | 9.6 |
| | 427 | 18% | 71% | 4 | 2.2 | 0.4 | 11.4 |
| | 491 | 0% | 81% | | | | |
| | 576 | 0% | 91% | | | | |
| 48 hours | 176 | 71% | 18% | 1 | | | |
| | 112 | 86% | 10% | 2 | 0.5 | 0.0 | 10.8 |
| | 0 | 100% | 0% | 3 | 0.5 | 0.0 | 10.8 |
| | 427 | 29% | 71% | 4 | 1.6 | 0.3 | 9.7 |
| | 491 | 0% | 81% | | | | |
| | 576 | 0% | 91% | | | | |

**Thrombospondin-1**

sCr or UO

| 0 hr prior to AKI stage | 24 hr prior to AKI stage | 48 hr prior to AKI stage |
|---|---|---|

Fig. 8 - 19

| | Cohort 1 | Cohort 2 | Cohort 1 | Cohort 2 | Cohort 1 | Cohort 2 |
|---|---|---|---|---|---|---|
| median | 2346.491 | 1056.437 | 2346.491 | 816.231 | 2346.491 | 674.270 |
| average | 3228.079 | 1204.766 | 3228.079 | 780.207 | 3228.079 | 743.543 |
| stdev | 2512.665 | 876.922 | 2512.665 | 381.033 | 2512.665 | 411.486 |
| p (t-test) | | 0.017 | | 0.015 | | 0.021 |
| min | 177.557 | 338.785 | 177.557 | 205.966 | 177.557 | 205.966 |
| max | 9514.803 | 3314.145 | 9514.803 | 1335.139 | 9514.803 | 1335.139 |
| n (Samp) | 37 | 10 | 37 | 7 | 37 | 6 |
| n (Pat) | 37 | 10 | 37 | 7 | 37 | 6 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 2300.310 | 816.231 | 2300.310 | 816.231 | 2300.310 | 1075.685 |
| average | 2961.527 | 784.483 | 2961.527 | 906.919 | 2961.527 | 1075.685 |
| stdev | 2469.791 | 372.589 | 2469.791 | 390.849 | 2469.791 | 366.923 |
| p (t-test) | | 0.055 | | 0.158 | | 0.288 |
| min | 177.557 | 338.785 | 177.557 | 569.386 | 177.557 | 816.231 |
| max | 9514.803 | 1335.139 | 9514.803 | 1335.139 | 9514.803 | 1335.139 |
| n (Samp) | 60 | 5 | 60 | 3 | 60 | 2 |
| n (Pat) | 60 | 5 | 60 | 3 | 60 | 2 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 2346.491 | 1486.842 | 2346.491 | 735.646 | 2346.491 | 520.243 |
| average | 3534.326 | 1625.048 | 3534.326 | 685.173 | 3534.326 | 577.471 |
| stdev | 2670.174 | 1072.295 | 2670.174 | 400.541 | 2670.174 | 356.378 |
| p (t-test) | | 0.130 | | 0.044 | | 0.037 |
| min | 606.462 | 368.114 | 606.462 | 205.966 | 606.462 | 205.966 |
| max | 9514.803 | 3314.145 | 9514.803 | 1063.433 | 9514.803 | 1063.433 |
| n (Samp) | 27 | 5 | 27 | 4 | 27 | 4 |
| n (Pat) | 27 | 5 | 27 | 4 | 27 | 4 |

sCr or UO

| Time prior AKI stage | AUC | SE | nCohort 1 | nCohort 2 | p |
|---|---|---|---|---|---|
| 0 hours | 0.21 | 0.072 | 37 | 10 | 0.000 |
| 24 hours | 0.13 | 0.058 | 37 | 7 | 0.000 |
| 48 hours | 0.13 | 0.060 | 37 | 6 | 0.000 |

sCr only

| Time prior AKI stage | AUC | SE | nCohort 1 | nCohort 2 | p |
|---|---|---|---|---|---|
| 0 hours | 0.17 | 0.073 | 60 | 5 | 0.000 |
| 24 hours | 0.21 | 0.106 | 60 | 3 | 0.006 |
| 48 hours | 0.26 | 0.147 | 60 | 2 | 0.100 |

UO only

| Time prior AKI stage | AUC | SE | nCohort 1 | nCohort 2 | p |
|---|---|---|---|---|---|
| 0 hours | 0.27 | 0.110 | 27 | 5 | 0.041 |
| 24 hours | 0.06 | 0.043 | 27 | 4 | 0.000 |
| 48 hours | 0.04 | 0.034 | 27 | 4 | 0.000 |

# Fig. 8 - 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

### Non-patent literature cited in the description

- Harrison's Principles of Internal Medicine. McGraw Hill, 1741-1830
- Current Medical Diagnosis & Treatment. McGraw Hill, 2008, 785-815
- **PRAUGHT ; SHLIPAK.** *Curr Opin Nephrol Hypertens,* 2005, vol. 14, 265-270
- **CHERTOW et al.** *JAm Soc Nephrol,* 2005, vol. 16, 3365-3370
- **LASSNIGG.** *J Am Soc Nephrol,* 2004, vol. 15, 1597-1605
- **BELLOMO et al.** *Crit Care.,* 2004, vol. 8 (4), R204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 (4), 177-197
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed.,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant,* 2008, vol. 23, 1203-1210
- Merck Manual of Diagnosis and Therapy. Merck Research Laboratories, 1999
- **WIJEYSUNDERA et al.** *JAMA,* 2007, vol. 297, 1801-9
- **HANLEY, J. A. ; MCNEIL, B.J.** The meaning and use of the area under a receiver operating characteristic (ROC) curve. *Radiology,* 1982, vol. 143, 29-36

SEQUENCE LISTING

<110> ASTUTE MEDICAL, INC.

<120> METHODS AND COMPOSITIONS FOR DIAGNOSIS AND PROGNOSIS OF RENAL INJURY AND RENAL FAILURE

<130> AST-18300-PC

<140> PCT/US2009/061562
<141> 2009-10-21

<150> 61/117,136
<151> 2008-11-22

<150> 61/117,138
<151> 2008-11-22

<150> 61/117,143
<151> 2008-11-22

<150> 61/115,043
<151> 2008-11-15

<150> 61/115,046
<151> 2008-11-15

<150> 61/115,050
<151> 2008-11-15

<150> 61/115,026
<151> 2008-11-14

<150> 61/115,028
<151> 2008-11-14

<150> 61/113,039
<151> 2008-11-10

<150> 61/113,059
<151> 2008-11-10

<150> 61/113,069
<151> 2008-11-10

<150> 61/113,078
<151> 2008-11-10

<150> 61/113,034
<151> 2008-11-10

<150> 61/113,093
<151> 2008-11-10

<150> 61/107,287
<151> 2008-10-21

<150> 61/107,293
<151> 2008-10-21

<150> 61/107,307
<151> 2008-10-21

<160> 17

```
<170> PatentIn version 3.5

<210> 1
<211> 742
<212> PRT
<213> Homo sapiens

<400> 1
Met Asp Lys Phe Trp Trp His Ala Ala Trp Gly Leu Cys Leu Val Pro
1               5                   10                  15


Leu Ser Leu Ala Gln Ile Asp Leu Asn Ile Thr Cys Arg Phe Ala Gly
            20                  25                  30


Val Phe His Val Glu Lys Asn Gly Arg Tyr Ser Ile Ser Arg Thr Glu
            35                  40                  45


Ala Ala Asp Leu Cys Lys Ala Phe Asn Ser Thr Leu Pro Thr Met Ala
            50                  55                  60


Gln Met Glu Lys Ala Leu Ser Ile Gly Phe Glu Thr Cys Arg Tyr Gly
65                  70                  75                  80


Phe Ile Glu Gly His Val Val Ile Pro Arg Ile His Pro Asn Ser Ile
                85                  90                  95


Cys Ala Ala Asn Asn Thr Gly Val Tyr Ile Leu Thr Ser Asn Thr Ser
                100                 105                 110


Gln Tyr Asp Thr Tyr Cys Phe Asn Ala Ser Ala Pro Pro Glu Glu Asp
            115                 120                 125


Cys Thr Ser Val Thr Asp Leu Pro Asn Ala Phe Asp Gly Pro Ile Thr
            130                 135                 140


Ile Thr Ile Val Asn Arg Asp Gly Thr Arg Tyr Val Gln Lys Gly Glu
145                 150                 155                 160


Tyr Arg Thr Asn Pro Glu Asp Ile Tyr Pro Ser Asn Pro Thr Asp Asp
                165                 170                 175


Asp Val Ser Ser Gly Ser Ser Ser Glu Arg Ser Ser Thr Ser Gly Gly
            180                 185                 190


Tyr Ile Phe Tyr Thr Phe Ser Thr Val His Pro Ile Pro Asp Glu Asp
            195                 200                 205


Ser Pro Trp Ile Thr Asp Ser Thr Asp Arg Ile Pro Ala Thr Thr Leu
            210                 215                 220


Met Ser Thr Ser Ala Thr Ala Thr Glu Thr Ala Thr Lys Arg Gln Glu
```

225 230 235 240

Thr Trp Asp Trp Phe Ser Trp Leu Phe Leu Pro Ser Glu Ser Lys Asn
245 250 255

His Leu His Thr Thr Thr Gln Met Ala Gly Thr Ser Ser Asn Thr Ile
260 265 270

Ser Ala Gly Trp Glu Pro Asn Glu Glu Asn Glu Asp Glu Arg Asp Arg
275 280 285

His Leu Ser Phe Ser Gly Ser Gly Ile Asp Asp Asp Glu Asp Phe Ile
290 295 300

Ser Ser Thr Ile Ser Thr Thr Pro Arg Ala Phe Asp His Thr Lys Gln
305 310 315 320

Asn Gln Asp Trp Thr Gln Trp Asn Pro Ser His Ser Asn Pro Glu Val
325 330 335

Leu Leu Gln Thr Thr Thr Arg Met Thr Asp Val Asp Arg Asn Gly Thr
340 345 350

Thr Ala Tyr Glu Gly Asn Trp Asn Pro Glu Ala His Pro Pro Leu Ile
355 360 365

His His Glu His His Glu Glu Glu Glu Thr Pro His Ser Thr Ser Thr
370 375 380

Ile Gln Ala Thr Pro Ser Ser Thr Thr Glu Glu Thr Ala Thr Gln Lys
385 390 395 400

Glu Gln Trp Phe Gly Asn Arg Trp His Glu Gly Tyr Arg Gln Thr Pro
405 410 415

Arg Glu Asp Ser His Ser Thr Thr Gly Thr Ala Ala Ala Ser Ala His
420 425 430

Thr Ser His Pro Met Gln Gly Arg Thr Thr Pro Ser Pro Glu Asp Ser
435 440 445

Ser Trp Thr Asp Phe Phe Asn Pro Ile Ser His Pro Met Gly Arg Gly
450 455 460

His Gln Ala Gly Arg Arg Met Asp Met Asp Ser Ser His Ser Thr Thr
465 470 475 480

Leu Gln Pro Thr Ala Asn Pro Asn Thr Gly Leu Val Glu Asp Leu Asp
485 490 495

```
Arg Thr Gly Pro Leu Ser Met Thr Thr Gln Gln Ser Asn Ser Gln Ser
        500             505                 510

Phe Ser Thr Ser His Glu Gly Leu Glu Glu Asp Lys Asp His Pro Thr
        515             520                 525

Thr Ser Thr Leu Thr Ser Ser Asn Arg Asn Asp Val Thr Gly Gly Arg
        530             535                 540

Arg Asp Pro Asn His Ser Glu Gly Ser Thr Thr Leu Leu Glu Gly Tyr
545             550                 555                 560

Thr Ser His Tyr Pro His Thr Lys Glu Ser Arg Thr Phe Ile Pro Val
            565                 570                 575

Thr Ser Ala Lys Thr Gly Ser Phe Gly Val Thr Ala Val Thr Val Gly
        580             585                 590

Asp Ser Asn Ser Asn Val Asn Arg Ser Leu Ser Gly Asp Gln Asp Thr
        595             600                 605

Phe His Pro Ser Gly Gly Ser His Thr Thr His Gly Ser Glu Ser Asp
        610             615                 620

Gly His Ser His Gly Ser Gln Glu Gly Gly Ala Asn Thr Thr Ser Gly
625             630                 635                 640

Pro Ile Arg Thr Pro Gln Ile Pro Glu Trp Leu Ile Ile Leu Ala Ser
            645                 650                 655

Leu Leu Ala Leu Ala Leu Ile Leu Ala Val Cys Ile Ala Val Asn Ser
        660             665                 670

Arg Arg Arg Cys Gly Gln Lys Lys Lys Leu Val Ile Asn Ser Gly Asn
        675             680                 685

Gly Ala Val Glu Asp Arg Lys Pro Ser Gly Leu Asn Gly Glu Ala Ser
        690             695                 700

Lys Ser Gln Glu Met Val His Leu Val Asn Lys Glu Ser Ser Glu Thr
705             710                 715                 720

Pro Asp Gln Phe Met Thr Ala Asp Glu Thr Arg Asn Leu Gln Asn Val
            725                 730                 735

Asp Met Lys Ile Gly Val
            740
```

```
<210> 2
<211> 498
<212> PRT
<213> Homo sapiens

<400> 2
Met Thr Val Phe Leu Ser Phe Ala Phe Leu Ala Ala Ile Leu Thr His
1               5                   10                  15


Ile Gly Cys Ser Asn Gln Arg Arg Ser Pro Glu Asn Ser Gly Arg Arg
            20                  25                  30


Tyr Asn Arg Ile Gln His Gly Gln Cys Ala Tyr Thr Phe Ile Leu Pro
        35                  40                  45


Glu His Asp Gly Asn Cys Arg Glu Ser Thr Thr Asp Gln Tyr Asn Thr
    50                  55                  60


Asn Ala Leu Gln Arg Asp Ala Pro His Val Glu Pro Asp Phe Ser Ser
65                  70                  75                  80


Gln Lys Leu Gln His Leu Glu His Val Met Glu Asn Tyr Thr Gln Trp
                85                  90                  95


Leu Gln Lys Leu Glu Asn Tyr Ile Val Glu Asn Met Lys Ser Glu Met
            100                 105                 110


Ala Gln Ile Gln Gln Asn Ala Val Gln Asn His Thr Ala Thr Met Leu
            115                 120                 125


Glu Ile Gly Thr Ser Leu Leu Ser Gln Thr Ala Glu Gln Thr Arg Lys
    130                 135                 140


Leu Thr Asp Val Glu Thr Gln Val Leu Asn Gln Thr Ser Arg Leu Glu
145                 150                 155                 160


Ile Gln Leu Leu Glu Asn Ser Leu Ser Thr Tyr Lys Leu Glu Lys Gln
                165                 170                 175


Leu Leu Gln Gln Thr Asn Glu Ile Leu Lys Ile His Glu Lys Asn Ser
            180                 185                 190


Leu Leu Glu His Lys Ile Leu Glu Met Glu Gly Lys His Lys Glu Glu
            195                 200                 205


Leu Asp Thr Leu Lys Glu Glu Lys Glu Asn Leu Gln Gly Leu Val Thr
    210                 215                 220


Arg Gln Thr Tyr Ile Ile Gln Glu Leu Glu Lys Gln Leu Asn Arg Ala
225                 230                 235                 240
```

Thr Thr Asn Asn Ser Val Leu Gln Lys Gln Gln Leu Glu Leu Met Asp
                245              250              255

Thr Val His Asn Leu Val Asn Leu Cys Thr Lys Glu Gly Val Leu Leu
                260              265              270

Lys Gly Gly Lys Arg Glu Glu Glu Lys Pro Phe Arg Asp Cys Ala Asp
                275              280              285

Val Tyr Gln Ala Gly Phe Asn Lys Ser Gly Ile Tyr Thr Ile Tyr Ile
    290              295              300

Asn Asn Met Pro Glu Pro Lys Lys Val Phe Cys Asn Met Asp Val Asn
305              310              315              320

Gly Gly Gly Trp Thr Val Ile Gln His Arg Glu Asp Gly Ser Leu Asp
                325              330              335

Phe Gln Arg Gly Trp Lys Glu Tyr Lys Met Gly Phe Gly Asn Pro Ser
                340              345              350

Gly Glu Tyr Trp Leu Gly Asn Glu Phe Ile Phe Ala Ile Thr Ser Gln
    355              360              365

Arg Gln Tyr Met Leu Arg Ile Glu Leu Met Asp Trp Glu Gly Asn Arg
    370              375              380

Ala Tyr Ser Gln Tyr Asp Arg Phe His Ile Gly Asn Glu Lys Gln Asn
385              390              395              400

Tyr Arg Leu Tyr Leu Lys Gly His Thr Gly Thr Ala Gly Lys Gln Ser
                405              410              415

Ser Leu Ile Leu His Gly Ala Asp Phe Ser Thr Lys Asp Ala Asp Asn
                420              425              430

Asp Asn Cys Met Cys Lys Cys Ala Leu Met Leu Thr Gly Gly Trp Trp
                435              440              445

Phe Asp Ala Cys Gly Pro Ser Asn Leu Asn Gly Met Phe Tyr Thr Ala
    450              455              460

Gly Gln Asn His Gly Lys Leu Asn Gly Ile Lys Trp His Tyr Phe Lys
465              470              475              480

Gly Pro Ser Tyr Ser Leu Arg Ser Thr Thr Met Met Ile Arg Pro Leu
                485              490              495

Asp Phe

<210> 3
<211> 1124
<212> PRT
<213> Homo sapiens

<400> 3
Met Asp Ser Leu Ala Ser Leu Val Leu Cys Gly Val Ser Leu Leu Leu
1               5               10              15

Ser Gly Thr Val Glu Gly Ala Met Asp Leu Ile Leu Ile Asn Ser Leu
            20              25              30

Pro Leu Val Ser Asp Ala Glu Thr Ser Leu Thr Cys Ile Ala Ser Gly
            35              40              45

Trp Arg Pro His Glu Pro Ile Thr Ile Gly Arg Asp Phe Glu Ala Leu
    50              55              60

Met Asn Gln His Gln Asp Pro Leu Glu Val Thr Gln Asp Val Thr Arg
65              70              75              80

Glu Trp Ala Lys Lys Val Val Trp Lys Arg Glu Lys Ala Ser Lys Ile
                85              90              95

Asn Gly Ala Tyr Phe Cys Glu Gly Arg Val Arg Gly Glu Ala Ile Arg
            100             105             110

Ile Arg Thr Met Lys Met Arg Gln Gln Ala Ser Phe Leu Pro Ala Thr
        115             120             125

Leu Thr Met Thr Val Asp Lys Gly Asp Asn Val Asn Ile Ser Phe Lys
    130             135             140

Lys Val Leu Ile Lys Glu Glu Asp Ala Val Ile Tyr Lys Asn Gly Ser
145             150             155             160

Phe Ile His Ser Val Pro Arg His Glu Val Pro Asp Ile Leu Glu Val
                165             170             175

His Leu Pro His Ala Gln Pro Gln Asp Ala Gly Val Tyr Ser Ala Arg
            180             185             190

Tyr Ile Gly Gly Asn Leu Phe Thr Ser Ala Phe Thr Arg Leu Ile Val
            195             200             205

Arg Arg Cys Glu Ala Gln Lys Trp Gly Pro Glu Cys Asn His Leu Cys
    210             215             220

```
Thr Ala Cys Met Asn Asn Gly Val Cys His Glu Asp Thr Gly Glu Cys
225             230             235             240

Ile Cys Pro Pro Gly Phe Met Gly Arg Thr Cys Glu Lys Ala Cys Glu
            245             250             255

Leu His Thr Phe Gly Arg Thr Cys Lys Glu Arg Cys Ser Gly Gln Glu
            260             265             270

Gly Cys Lys Ser Tyr Val Phe Cys Leu Pro Asp Pro Tyr Gly Cys Ser
            275             280             285

Cys Ala Thr Gly Trp Lys Gly Leu Gln Cys Asn Glu Ala Cys His Pro
            290             295             300

Gly Phe Tyr Gly Pro Asp Cys Lys Leu Arg Cys Ser Cys Asn Asn Gly
305             310             315             320

Glu Met Cys Asp Arg Phe Gln Gly Cys Leu Cys Ser Pro Gly Trp Gln
            325             330             335

Gly Leu Gln Cys Glu Arg Glu Gly Ile Pro Arg Met Thr Pro Lys Ile
            340             345             350

Val Asp Leu Pro Asp His Ile Glu Val Asn Ser Gly Lys Phe Asn Pro
            355             360             365

Ile Cys Lys Ala Ser Gly Trp Pro Leu Pro Thr Asn Glu Glu Met Thr
            370             375             380

Leu Val Lys Pro Asp Gly Thr Val Leu His Pro Lys Asp Phe Asn His
385             390             395             400

Thr Asp His Phe Ser Val Ala Ile Phe Thr Ile His Arg Ile Leu Pro
            405             410             415

Pro Asp Ser Gly Val Trp Val Cys Ser Val Asn Thr Val Ala Gly Met
            420             425             430

Val Glu Lys Pro Phe Asn Ile Ser Val Lys Val Leu Pro Lys Pro Leu
            435             440             445

Asn Ala Pro Asn Val Ile Asp Thr Gly His Asn Phe Ala Val Ile Asn
            450             455             460

Ile Ser Ser Glu Pro Tyr Phe Gly Asp Gly Pro Ile Lys Ser Lys Lys
465             470             475             480
```

52

```
Leu Leu Tyr Lys Pro Val Asn His Tyr Glu Ala Trp Gln His Ile Gln
            485             490                 495

Val Thr Asn Glu Ile Val Thr Leu Asn Tyr Leu Glu Pro Arg Thr Glu
            500             505                 510

Tyr Glu Leu Cys Val Gln Leu Val Arg Arg Gly Glu Gly Gly Glu Gly
            515             520                 525

His Pro Gly Pro Val Arg Arg Phe Thr Thr Ala Ser Ile Gly Leu Pro
            530             535                 540

Pro Pro Arg Gly Leu Asn Leu Leu Pro Lys Ser Gln Thr Thr Leu Asn
545             550             555                 560

Leu Thr Trp Gln Pro Ile Phe Pro Ser Ser Glu Asp Asp Phe Tyr Val
            565             570                 575

Glu Val Glu Arg Arg Ser Val Gln Lys Ser Asp Gln Gln Asn Ile Lys
            580             585                 590

Val Pro Gly Asn Leu Thr Ser Val Leu Leu Asn Asn Leu His Pro Arg
            595             600                 605

Glu Gln Tyr Val Val Arg Ala Arg Val Asn Thr Lys Ala Gln Gly Glu
            610             615                 620

Trp Ser Glu Asp Leu Thr Ala Trp Thr Leu Ser Asp Ile Leu Pro Pro
625             630             635                 640

Gln Pro Glu Asn Ile Lys Ile Ser Asn Ile Thr His Ser Ser Ala Val
            645             650                 655

Ile Ser Trp Thr Ile Leu Asp Gly Tyr Ser Ile Ser Ser Ile Thr Ile
            660             665                 670

Arg Tyr Lys Val Gln Gly Lys Asn Glu Asp Gln His Val Asp Val Lys
            675             680                 685

Ile Lys Asn Ala Thr Ile Ile Gln Tyr Gln Leu Lys Gly Leu Glu Pro
            690             695                 700

Glu Thr Ala Tyr Gln Val Asp Ile Phe Ala Glu Asn Asn Ile Gly Ser
705             710             715                 720

Ser Asn Pro Ala Phe Ser His Glu Leu Val Thr Leu Pro Glu Ser Gln
            725             730                 735
```

```
Ala Pro Ala Asp Leu Gly Gly Gly Lys Met Leu Leu Ile Ala Ile Leu
            740               745               750

Gly Ser Ala Gly Met Thr Cys Leu Thr Val Leu Leu Ala Phe Leu Ile
            755               760               765

Ile Leu Gln Leu Lys Arg Ala Asn Val Gln Arg Arg Met Ala Gln Ala
    770               775               780

Phe Gln Asn Val Arg Glu Glu Pro Ala Val Gln Phe Asn Ser Gly Thr
785               790               795               800

Leu Ala Leu Asn Arg Lys Val Lys Asn Asn Pro Asp Pro Thr Ile Tyr
            805               810               815

Pro Val Leu Asp Trp Asn Asp Ile Lys Phe Gln Asp Val Ile Gly Glu
            820               825               830

Gly Asn Phe Gly Gln Val Leu Lys Ala Arg Ile Lys Lys Asp Gly Leu
            835               840               845

Arg Met Asp Ala Ala Ile Lys Arg Met Lys Glu Tyr Ala Ser Lys Asp
    850               855               860

Asp His Arg Asp Phe Ala Gly Glu Leu Glu Val Leu Cys Lys Leu Gly
865               870               875               880

His His Pro Asn Ile Ile Asn Leu Leu Gly Ala Cys Glu His Arg Gly
            885               890               895

Tyr Leu Tyr Leu Ala Ile Glu Tyr Ala Pro His Gly Asn Leu Leu Asp
            900               905               910

Phe Leu Arg Lys Ser Arg Val Leu Glu Thr Asp Pro Ala Phe Ala Ile
            915               920               925

Ala Asn Ser Thr Ala Ser Thr Leu Ser Ser Gln Gln Leu Leu His Phe
    930               935               940

Ala Ala Asp Val Ala Arg Gly Met Asp Tyr Leu Ser Gln Lys Gln Phe
945               950               955               960

Ile His Arg Asp Leu Ala Ala Arg Asn Ile Leu Val Gly Glu Asn Tyr
            965               970               975

Val Ala Lys Ile Ala Asp Phe Gly Leu Ser Arg Gly Gln Glu Val Tyr
            980               985               990

Val Lys Lys Thr Met Gly Arg Leu  Pro Val Arg Trp Met  Ala Ile Glu
```

54

995                     1000                    1005

Ser Leu Asn Tyr Ser Val Tyr  Thr Thr Asn Ser Asp  Val Trp Ser
    1010                1015                1020

Tyr Gly Val Leu Leu Trp Glu  Ile Val Ser Leu Gly  Gly Thr Pro
    1025                1030                1035

Tyr Cys Gly Met Thr Cys Ala  Glu Leu Tyr Glu Lys  Leu Pro Gln
    1040                1045                1050

Gly Tyr Arg Leu Glu Lys Pro  Leu Asn Cys Asp Asp  Glu Val Tyr
    1055                1060                1065

Asp Leu Met Arg Gln Cys Trp  Arg Glu Lys Pro Tyr  Glu Arg Pro
    1070                1075                1080

Ser Phe Ala Gln Ile Leu Val  Ser Leu Asn Arg Met  Leu Glu Glu
    1085                1090                1095

Arg Lys Thr Tyr Val Asn Thr  Thr Leu Tyr Glu Lys  Phe Thr Tyr
    1100                1105                1110

Ala Gly Ile Asp Cys Ser Ala  Glu Glu Ala Ala
    1115                1120

<210> 4
<211> 114
<212> PRT
<213> Homo sapiens

<400> 4
Met Ser Leu Leu Ser Ser Arg Ala Ala Arg Val Pro Gly Pro Ser Ser
1           5              10               15

Ser Leu Cys Ala Leu Leu Val Leu Leu Leu Leu Thr Gln Pro Gly
        20            25              30

Pro Ile Ala Ser Ala Gly Pro Ala Ala Ala Val Leu Arg Glu Leu Arg
      35              40              45

Cys Val Cys Leu Gln Thr Thr Gln Gly Val His Pro Lys Met Ile Ser
    50            55              60

Asn Leu Gln Val Phe Ala Ile Gly Pro Gln Cys Ser Lys Val Glu Val
65              70              75              80

Val Ala Ser Leu Lys Asn Gly Lys Glu Ile Cys Leu Asp Pro Glu Ala
              85              90              95

```
Pro Phe Leu Lys Lys Val Ile Gln Lys Ile Leu Asp Gly Gly Asn Lys
            100                 105                 110

Glu Asn


<210> 5
<211> 658
<212> PRT
<213> Homo sapiens

<400> 5
Met Asp Arg Gly Thr Leu Pro Leu Ala Val Ala Leu Leu Leu Ala Ser
1               5               10                  15

Cys Ser Leu Ser Pro Thr Ser Leu Ala Glu Thr Val His Cys Asp Leu
            20                  25                  30

Gln Pro Val Gly Pro Glu Arg Gly Glu Val Thr Tyr Thr Thr Ser Gln
            35                  40                  45

Val Ser Lys Gly Cys Val Ala Gln Ala Pro Asn Ala Ile Leu Glu Val
            50                  55                  60

His Val Leu Phe Leu Glu Phe Pro Thr Gly Pro Ser Gln Leu Glu Leu
65                  70                  75                  80

Thr Leu Gln Ala Ser Lys Gln Asn Gly Thr Trp Pro Arg Glu Val Leu
                85                  90                  95

Leu Val Leu Ser Val Asn Ser Ser Val Phe Leu His Leu Gln Ala Leu
                100                 105                 110

Gly Ile Pro Leu His Leu Ala Tyr Asn Ser Ser Leu Val Thr Phe Gln
            115                 120                 125

Glu Pro Pro Gly Val Asn Thr Thr Glu Leu Pro Ser Phe Pro Lys Thr
            130                 135                 140

Gln Ile Leu Glu Trp Ala Ala Glu Arg Gly Pro Ile Thr Ser Ala Ala
145                 150                 155                 160

Glu Leu Asn Asp Pro Gln Ser Ile Leu Leu Arg Leu Gly Gln Ala Gln
                165                 170                 175

Gly Ser Leu Ser Phe Cys Met Leu Glu Ala Ser Gln Asp Met Gly Arg
                180                 185                 190

Thr Leu Glu Trp Arg Pro Arg Thr Pro Ala Leu Val Arg Gly Cys His
                195                 200                 205
```

Leu Glu Gly Val Ala Gly His Lys Glu Ala His Ile Leu Arg Val Leu
210             215                 220

Pro Gly His Ser Ala Gly Pro Arg Thr Val Thr Val Lys Val Glu Leu
225                 230                 235                 240

Ser Cys Ala Pro Gly Asp Leu Asp Ala Val Leu Ile Leu Gln Gly Pro
                245                 250                 255

Pro Tyr Val Ser Trp Leu Ile Asp Ala Asn His Asn Met Gln Ile Trp
                260                 265                 270

Thr Thr Gly Glu Tyr Ser Phe Lys Ile Phe Pro Glu Lys Asn Ile Arg
                275                 280                 285

Gly Phe Lys Leu Pro Asp Thr Pro Gln Gly Leu Leu Gly Glu Ala Arg
                290                 295                 300

Met Leu Asn Ala Ser Ile Val Ala Ser Phe Val Glu Leu Pro Leu Ala
305                 310                 315                 320

Ser Ile Val Ser Leu His Ala Ser Ser Cys Gly Gly Arg Leu Gln Thr
                325                 330                 335

Ser Pro Ala Pro Ile Gln Thr Thr Pro Pro Lys Asp Thr Cys Ser Pro
                340                 345                 350

Glu Leu Leu Met Ser Leu Ile Gln Thr Lys Cys Ala Asp Asp Ala Met
                355                 360                 365

Thr Leu Val Leu Lys Lys Glu Leu Val Ala His Leu Lys Cys Thr Ile
370                 375                 380

Thr Gly Leu Thr Phe Trp Asp Pro Ser Cys Glu Ala Glu Asp Arg Gly
385                 390                 395                 400

Asp Lys Phe Val Leu Arg Ser Ala Tyr Ser Ser Cys Gly Met Gln Val
                405                 410                 415

Ser Ala Ser Met Ile Ser Asn Glu Ala Val Val Asn Ile Leu Ser Ser
                420                 425                 430

Ser Ser Pro Gln Arg Lys Lys Val His Cys Leu Asn Met Asp Ser Leu
                435                 440                 445

Ser Phe Gln Leu Gly Leu Tyr Leu Ser Pro His Phe Leu Gln Ala Ser
                450                 455                 460

```
Asn Thr Ile Glu Pro Gly Gln Gln Ser Phe Val Gln Val Arg Val Ser
465             470             475             480


Pro Ser Val Ser Glu Phe Leu Leu Gln Leu Asp Ser Cys His Leu Asp
                485             490             495


Leu Gly Pro Glu Gly Gly Thr Val Glu Leu Ile Gln Gly Arg Ala Ala
            500             505             510


Lys Gly Asn Cys Val Ser Leu Leu Ser Pro Ser Pro Glu Gly Asp Pro
        515             520             525


Arg Phe Ser Phe Leu Leu His Phe Tyr Thr Val Pro Ile Pro Lys Thr
    530             535             540


Gly Thr Leu Ser Cys Thr Val Ala Leu Arg Pro Lys Thr Gly Ser Gln
545             550             555             560


Asp Gln Glu Val His Arg Thr Val Phe Met Arg Leu Asn Ile Ile Ser
            565             570             575


Pro Asp Leu Ser Gly Cys Thr Ser Lys Gly Leu Val Leu Pro Ala Val
        580             585             590


Leu Gly Ile Thr Phe Gly Ala Phe Leu Ile Gly Ala Leu Leu Thr Ala
        595             600             605


Ala Leu Trp Tyr Ile Tyr Ser His Thr Arg Ser Pro Ser Lys Arg Glu
    610             615             620


Pro Val Val Ala Val Ala Ala Pro Ala Ser Ser Glu Ser Ser Ser Thr
625             630             635             640


Asn His Ser Ile Gly Ser Thr Gln Ser Thr Pro Cys Ser Thr Ser Ser
            645             650             655


Met Ala


<210> 6
<211> 314
<212> PRT
<213> Homo sapiens

<400> 6
Met Ala Pro Pro Gln Val Leu Ala Phe Gly Leu Leu Leu Ala Ala Ala
1               5               10              15


Thr Ala Thr Phe Ala Ala Ala Gln Glu Glu Cys Val Cys Glu Asn Tyr
            20              25              30
```

```
Lys Leu Ala Val Asn Cys Phe Val Asn Asn Asn Arg Gln Cys Gln Cys
        35                  40                  45

Thr Ser Val Gly Ala Gln Asn Thr Val Ile Cys Ser Lys Leu Ala Ala
        50                  55                  60

Lys Cys Leu Val Met Lys Ala Glu Met Asn Gly Ser Lys Leu Gly Arg
65                  70                  75                  80

Arg Ala Lys Pro Glu Gly Ala Leu Gln Asn Asn Asp Gly Leu Tyr Asp
                85                  90                  95

Pro Asp Cys Asp Glu Ser Gly Leu Phe Lys Ala Lys Gln Cys Asn Gly
            100                 105                 110

Thr Ser Met Cys Trp Cys Val Asn Thr Ala Gly Val Arg Arg Thr Asp
            115                 120                 125

Lys Asp Thr Glu Ile Thr Cys Ser Glu Arg Val Arg Thr Tyr Trp Ile
    130                 135                 140

Ile Ile Glu Leu Lys His Lys Ala Arg Glu Lys Pro Tyr Asp Ser Lys
145                 150                 155                 160

Ser Leu Arg Thr Ala Leu Gln Lys Glu Ile Thr Thr Arg Tyr Gln Leu
                165                 170                 175

Asp Pro Lys Phe Ile Thr Ser Ile Leu Tyr Glu Asn Asn Val Ile Thr
            180                 185                 190

Ile Asp Leu Val Gln Asn Ser Ser Gln Lys Thr Gln Asn Asp Val Asp
            195                 200                 205

Ile Ala Asp Val Ala Tyr Tyr Phe Glu Lys Asp Val Lys Gly Glu Ser
    210                 215                 220

Leu Phe His Ser Lys Lys Met Asp Leu Thr Val Asn Gly Glu Gln Leu
225                 230                 235                 240

Asp Leu Asp Pro Gly Gln Thr Leu Ile Tyr Tyr Val Asp Glu Lys Ala
                245                 250                 255

Pro Glu Phe Ser Met Gln Gly Leu Lys Ala Gly Val Ile Ala Val Ile
            260                 265                 270

Val Val Val Val Ile Ala Val Val Ala Gly Ile Val Val Leu Val Ile
    275                 280                 285
```

Ser Arg Lys Lys Arg Met Ala Lys Tyr Glu Lys Ala Glu Ile Lys Glu
    290                 295                 300

Met Gly Glu Met His Arg Glu Leu Asn Ala
305                 310

<210> 7
<211> 193
<212> PRT
<213> Homo sapiens

<400> 7
Met Gly Val His Glu Cys Pro Ala Trp Leu Trp Leu Leu Leu Ser Leu
1               5               10              15

Leu Ser Leu Pro Leu Gly Leu Pro Val Leu Gly Ala Pro Pro Arg Leu
            20              25              30

Ile Cys Asp Ser Arg Val Leu Glu Arg Tyr Leu Leu Glu Ala Lys Glu
            35              40              45

Ala Glu Asn Ile Thr Thr Gly Cys Ala Glu His Cys Ser Leu Asn Glu
            50              55              60

Asn Ile Thr Val Pro Asp Thr Lys Val Asn Phe Tyr Ala Trp Lys Arg
65              70              75              80

Met Glu Val Gly Gln Gln Ala Val Glu Val Trp Gln Gly Leu Ala Leu
                85              90              95

Leu Ser Glu Ala Val Leu Arg Gly Gln Ala Leu Leu Val Asn Ser Ser
            100             105             110

Gln Pro Trp Glu Pro Leu Gln Leu His Val Asp Lys Ala Val Ser Gly
            115             120             125

Leu Arg Ser Leu Thr Thr Leu Leu Arg Ala Leu Gly Ala Gln Lys Glu
            130             135             140

Ala Ile Ser Pro Pro Asp Ala Ala Ser Ala Ala Pro Leu Arg Thr Ile
145             150             155             160

Thr Ala Asp Thr Phe Arg Lys Leu Phe Arg Val Tyr Ser Asn Phe Leu
            165             170             175

Arg Gly Lys Leu Lys Leu Tyr Thr Gly Glu Ala Cys Arg Thr Gly Asp
            180             185             190

Arg

<210> 8
<211> 397
<212> PRT
<213> Homo sapiens

<400> 8
Met Ala Pro Ile Ser Leu Ser Trp Leu Leu Arg Leu Ala Thr Phe Cys
1               5                   10                  15


His Leu Thr Val Leu Leu Ala Gly Gln His His Gly Val Thr Lys Cys
            20                  25                  30


Asn Ile Thr Cys Ser Lys Met Thr Ser Lys Ile Pro Val Ala Leu Leu
            35                  40                  45


Ile His Tyr Gln Gln Asn Gln Ala Ser Cys Gly Lys Arg Ala Ile Ile
    50                  55                  60


Leu Glu Thr Arg Gln His Arg Leu Phe Cys Ala Asp Pro Lys Glu Gln
65                  70                  75                  80


Trp Val Lys Asp Ala Met Gln His Leu Asp Arg Gln Ala Ala Ala Leu
                85                  90                  95


Thr Arg Asn Gly Gly Thr Phe Glu Lys Gln Ile Gly Glu Val Lys Pro
                100                 105                 110


Arg Thr Thr Pro Ala Ala Gly Gly Met Asp Glu Ser Val Val Leu Glu
            115                 120                 125


Pro Glu Ala Thr Gly Glu Ser Ser Ser Leu Glu Pro Thr Pro Ser Ser
    130                 135                 140


Gln Glu Ala Gln Arg Ala Leu Gly Thr Ser Pro Glu Leu Pro Thr Gly
145                 150                 155                 160


Val Thr Gly Ser Ser Gly Thr Arg Leu Pro Pro Thr Pro Lys Ala Gln
                165                 170                 175


Asp Gly Gly Pro Val Gly Thr Glu Leu Phe Arg Val Pro Pro Val Ser
                180                 185                 190


Thr Ala Ala Thr Trp Gln Ser Ser Ala Pro His Gln Pro Gly Pro Ser
            195                 200                 205


Leu Trp Ala Glu Ala Lys Thr Ser Glu Ala Pro Ser Thr Gln Asp Pro
    210                 215                 220


Ser Thr Gln Ala Ser Thr Ala Ser Ser Pro Ala Pro Glu Glu Asn Ala
225                 230                 235                 240

```
        Pro Ser Glu Gly Gln Arg Val Trp Gly Gln Gly Gln Ser Pro Arg Pro
                        245             250                 255


        Glu Asn Ser Leu Glu Arg Glu Glu Met Gly Pro Val Pro Ala His Thr
                        260             265                 270


        Asp Ala Phe Gln Asp Trp Gly Pro Gly Ser Met Ala His Val Ser Val
                        275             280                 285


        Val Pro Val Ser Ser Glu Gly Thr Pro Ser Arg Glu Pro Val Ala Ser
                        290             295                 300


        Gly Ser Trp Thr Pro Lys Ala Glu Glu Pro Ile His Ala Thr Met Asp
        305             310                 315                 320


        Pro Gln Arg Leu Gly Val Leu Ile Thr Pro Val Pro Asp Ala Gln Ala
                        325             330                 335


        Ala Thr Arg Arg Gln Ala Val Gly Leu Leu Ala Phe Leu Gly Leu Leu
                        340             345                 350


        Phe Cys Leu Gly Val Ala Met Phe Thr Tyr Gln Ser Leu Gln Gly Cys
                        355             360                 365


        Pro Arg Lys Met Ala Gly Glu Met Ala Glu Gly Leu Arg Tyr Ile Pro
                        370             375                 380


        Arg Ser Cys Gly Ser Asn Ser Tyr Val Leu Val Pro Val
        385             390                 395


        <210> 9
        <211> 288
        <212> PRT
        <213> Homo sapiens

        <400> 9
        Met Glu Arg Pro Gln Pro Asp Ser Met Pro Gln Asp Leu Ser Glu Ala
        1               5                   10                  15


        Leu Lys Glu Ala Thr Lys Glu Val His Thr Gln Ala Glu Asn Ala Glu
                        20                  25                  30


        Phe Met Arg Asn Phe Gln Lys Gly Gln Val Thr Arg Asp Gly Phe Lys
                        35                  40                  45


        Leu Val Met Ala Ser Leu Tyr His Ile Tyr Val Ala Leu Glu Glu Glu
                50                  55                  60


        Ile Glu Arg Asn Lys Glu Ser Pro Val Phe Ala Pro Val Tyr Phe Pro
```

|  |  |  |  |  |  |  |  |  |  |  |  |  |  |  |
|---|---|---|---|---|---|---|---|---|---|---|---|---|---|---|
| 65 | | | | 70 | | | | 75 | | | | 80 | | |

Glu Glu Leu His Arg Lys Ala Ala Leu Glu Gln Asp Leu Ala Phe Trp
            85              90              95

Tyr Gly Pro Arg Trp Gln Glu Val Ile Pro Tyr Thr Pro Ala Met Gln
            100             105             110

Arg Tyr Val Lys Arg Leu His Glu Val Gly Arg Thr Glu Pro Glu Leu
            115             120             125

Leu Val Ala His Ala Tyr Thr Arg Tyr Leu Gly Asp Leu Ser Gly Gly
            130             135             140

Gln Val Leu Lys Lys Ile Ala Gln Lys Ala Leu Asp Leu Pro Ser Ser
145             150             155             160

Gly Glu Gly Leu Ala Phe Phe Thr Phe Pro Asn Ile Ala Ser Ala Thr
            165             170             175

Lys Phe Lys Gln Leu Tyr Arg Ser Arg Met Asn Ser Leu Glu Met Thr
            180             185             190

Pro Ala Val Arg Gln Arg Val Ile Glu Glu Ala Lys Thr Ala Phe Leu
            195             200             205

Leu Asn Ile Gln Leu Phe Glu Glu Leu Gln Glu Leu Leu Thr His Asp
            210             215             220

Thr Lys Asp Gln Ser Pro Ser Arg Ala Pro Gly Leu Arg Gln Arg Ala
225             230             235             240

Ser Asn Lys Val Gln Asp Ser Ala Pro Val Glu Thr Pro Arg Gly Lys
            245             250             255

Pro Pro Leu Asn Thr Arg Ser Gln Ala Pro Leu Leu Arg Trp Val Leu
            260             265             270

Thr Leu Ser Phe Leu Val Ala Thr Val Ala Val Gly Leu Tyr Ala Met
            275             280             285

<210> 10
<211> 398
<212> PRT
<213> Homo sapiens

<400> 10
Met Leu Arg Leu Tyr Val Leu Val Met Gly Val Ser Ala Phe Thr Leu
1               5               10              15

```
Gln Pro Ala Ala His Thr Gly Ala Ala Arg Ser Cys Arg Phe Arg Gly
         20                  25                  30

Arg His Tyr Lys Arg Glu Phe Arg Leu Glu Gly Glu Pro Val Ala Leu
         35                  40                  45

Arg Cys Pro Gln Val Pro Tyr Trp Leu Trp Ala Ser Val Ser Pro Arg
         50                  55                  60

Ile Asn Leu Thr Trp His Lys Asn Asp Ser Ala Arg Thr Val Pro Gly
65                  70                  75                  80

Glu Glu Glu Thr Arg Met Trp Ala Gln Asp Gly Ala Leu Trp Leu Leu
                85                  90                  95

Pro Ala Leu Gln Glu Asp Ser Gly Thr Tyr Val Cys Thr Thr Arg Asn
             100                 105                 110

Ala Ser Tyr Cys Asp Lys Met Ser Ile Glu Leu Arg Val Phe Glu Asn
         115                 120                 125

Thr Asp Ala Phe Leu Pro Phe Ile Ser Tyr Pro Gln Ile Leu Thr Leu
     130                 135                 140

Ser Thr Ser Gly Val Leu Val Cys Pro Asp Leu Ser Glu Phe Thr Arg
145                 150                 155                 160

Asp Lys Thr Asp Val Lys Ile Gln Trp Tyr Lys Asp Ser Leu Leu Leu
                165                 170                 175

Asp Lys Asp Asn Glu Lys Phe Leu Ser Val Arg Gly Thr Thr His Leu
             180                 185                 190

Leu Val His Asp Val Ala Leu Glu Asp Ala Gly Tyr Tyr Arg Cys Val
         195                 200                 205

Leu Thr Phe Ala His Glu Gly Gln Gln Tyr Asn Ile Thr Arg Ser Ile
     210                 215                 220

Glu Leu Arg Ile Lys Lys Lys Lys Glu Glu Thr Ile Pro Val Ile Ile
225                 230                 235                 240

Ser Pro Leu Lys Thr Ile Ser Ala Ser Leu Gly Ser Arg Leu Thr Ile
             245                 250                 255

Pro Cys Lys Val Phe Leu Gly Thr Gly Thr Pro Leu Thr Thr Met Leu
         260                 265                 270

Trp Trp Thr Ala Asn Asp Thr His Ile Glu Ser Ala Tyr Pro Gly Gly
```

                275                    280                    285

Arg Val Thr Glu Gly Pro Arg Gln Glu Tyr Ser Glu Asn Asn Glu Asn
    290                    295                300

Tyr Ile Glu Val Pro Leu Ile Phe Asp Pro Val Thr Arg Glu Asp Leu
305                    310                    315                320

His Met Asp Phe Lys Cys Val Val His Asn Thr Leu Ser Phe Gln Thr
                325                    330                    335

Leu Arg Thr Thr Val Lys Glu Ala Ser Ser Thr Phe Ser Trp Gly Ile
                340                    345                    350

Val Leu Ala Pro Leu Ser Leu Ala Phe Leu Val Leu Gly Gly Ile Trp
                355                    360                    365

Met His Arg Arg Cys Lys His Arg Thr Gly Lys Ala Asp Gly Leu Thr
    370                    375                    380

Val Leu Trp Pro His His Gln Asp Phe Gln Ser Tyr Pro Lys
385                    390                    395

<210> 11
<211> 468
<212> PRT
<213> Homo sapiens

<400> 11
Met Leu Ala Val Gly Cys Ala Leu Leu Ala Ala Leu Leu Ala Ala Pro
1                   5                   10                  15

Gly Ala Ala Leu Ala Pro Arg Arg Cys Pro Ala Gln Glu Val Ala Arg
                20                  25                  30

Gly Val Leu Thr Ser Leu Pro Gly Asp Ser Val Thr Leu Thr Cys Pro
                35                  40                  45

Gly Val Glu Pro Glu Asp Asn Ala Thr Val His Trp Val Leu Arg Lys
                50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
                100                    105                    110

```
Pro Pro Glu Glu Pro Gln Leu Ser Cys Phe Arg Lys Ser Pro Leu Ser
        115             120             125

Asn Val Val Cys Glu Trp Gly Pro Arg Ser Thr Pro Ser Leu Thr Thr
    130             135             140

Lys Ala Val Leu Leu Val Arg Lys Phe Gln Asn Ser Pro Ala Glu Asp
145             150             155             160

Phe Gln Glu Pro Cys Gln Tyr Ser Gln Glu Ser Gln Lys Phe Ser Cys
            165             170             175

Gln Leu Ala Val Pro Glu Gly Asp Ser Ser Phe Tyr Ile Val Ser Met
            180             185             190

Cys Val Ala Ser Ser Val Gly Ser Lys Phe Ser Lys Thr Gln Thr Phe
        195             200             205

Gln Gly Cys Gly Ile Leu Gln Pro Asp Pro Pro Ala Asn Ile Thr Val
    210             215             220

Thr Ala Val Ala Arg Asn Pro Arg Trp Leu Ser Val Thr Trp Gln Asp
225             230             235             240

Pro His Ser Trp Asn Ser Ser Phe Tyr Arg Leu Arg Phe Glu Leu Arg
            245             250             255

Tyr Arg Ala Glu Arg Ser Lys Thr Phe Thr Thr Trp Met Val Lys Asp
            260             265             270

Leu Gln His His Cys Val Ile His Asp Ala Trp Ser Gly Leu Arg His
    275             280             285

Val Val Gln Leu Arg Ala Gln Glu Glu Phe Gly Gln Gly Glu Trp Ser
    290             295             300

Glu Trp Ser Pro Glu Ala Met Gly Thr Pro Trp Thr Glu Ser Arg Ser
305             310             315             320

Pro Pro Ala Glu Asn Glu Val Ser Thr Pro Met Gln Ala Leu Thr Thr
            325             330             335

Asn Lys Asp Asp Asp Asn Ile Leu Phe Arg Asp Ser Ala Asn Ala Thr
            340             345             350

Ser Leu Pro Val Gln Asp Ser Ser Ser Val Pro Leu Pro Thr Phe Leu
        355             360             365

Val Ala Gly Gly Ser Leu Ala Phe Gly Thr Leu Leu Cys Ile Ala Ile
```

370             375             380

Val Leu Arg Phe Lys Lys Thr Trp Lys Leu Arg Ala Leu Lys Glu Gly
385         390         395         400

Lys Thr Ser Met His Pro Pro Tyr Ser Leu Gly Gln Leu Val Pro Glu
            405         410         415

Arg Pro Arg Pro Thr Pro Val Leu Val Pro Leu Ile Ser Pro Pro Val
            420         425         430

Ser Pro Ser Ser Leu Gly Ser Asp Asn Thr Ser Ser His Asn Arg Pro
            435         440         445

Asp Ala Arg Asp Pro Arg Ser Pro Tyr Asp Ile Ser Asn Thr Asp Tyr
    450         455         460

Phe Phe Pro Arg
465


<210> 12
<211> 114
<212> PRT
<213> Homo sapiens

<400> 12
Met Arg Leu Leu Ile Leu Ala Leu Leu Gly Ile Cys Ser Leu Thr Ala
1           5           10          15

Tyr Ile Val Glu Gly Val Gly Ser Glu Val Ser Asp Lys Arg Thr Cys
            20          25          30

Val Ser Leu Thr Thr Gln Arg Leu Pro Val Ser Arg Ile Lys Thr Tyr
    35          40          45

Thr Ile Thr Glu Gly Ser Leu Arg Ala Val Ile Phe Ile Thr Lys Arg
    50          55          60

Gly Leu Lys Val Cys Ala Asp Pro Gln Ala Thr Trp Val Arg Asp Val
65          70          75          80

Val Arg Ser Met Asp Arg Lys Ser Asn Thr Arg Asn Asn Met Ile Gln
            85          90          95

Thr Lys Pro Thr Gly Thr Gln Gln Ser Thr Asn Thr Ala Val Thr Leu
            100         105         110

Thr Gly

<210> 13
<211> 205
<212> PRT
<213> Homo sapiens

<400> 13
Met Thr Pro Pro Glu Arg Leu Phe Leu Pro Arg Val Cys Gly Thr Thr
1               5                   10                  15

Leu His Leu Leu Leu Leu Gly Leu Leu Leu Val Leu Leu Pro Gly Ala
            20                  25                  30

Gln Gly Leu Pro Gly Val Gly Leu Thr Pro Ser Ala Ala Gln Thr Ala
            35                  40                  45

Arg Gln His Pro Lys Met His Leu Ala His Ser Thr Leu Lys Pro Ala
        50                  55                  60

Ala His Leu Ile Gly Asp Pro Ser Lys Gln Asn Ser Leu Leu Trp Arg
65                  70                  75                  80

Ala Asn Thr Asp Arg Ala Phe Leu Gln Asp Gly Phe Ser Leu Ser Asn
                85                  90                  95

Asn Ser Leu Leu Val Pro Thr Ser Gly Ile Tyr Phe Val Tyr Ser Gln
            100                 105                 110

Val Val Phe Ser Gly Lys Ala Tyr Ser Pro Lys Ala Thr Ser Ser Pro
            115                 120                 125

Leu Tyr Leu Ala His Glu Val Gln Leu Phe Ser Ser Gln Tyr Pro Phe
            130                 135                 140

His Val Pro Leu Leu Ser Ser Gln Lys Met Val Tyr Pro Gly Leu Gln
145                 150                 155                 160

Glu Pro Trp Leu His Ser Met Tyr His Gly Ala Ala Phe Gln Leu Thr
                165                 170                 175

Gln Gly Asp Gln Leu Ser Thr His Thr Asp Gly Ile Pro His Leu Val
            180                 185                 190

Leu Ser Pro Ser Thr Val Phe Phe Gly Ala Phe Ala Leu
            195                 200                 205

<210> 14
<211> 477
<212> PRT
<213> Homo sapiens

<400> 14
Met Lys Ser Leu Pro Ile Leu Leu Leu Leu Cys Val Ala Val Cys Ser

```
                1               5                       10                      15

        Ala Tyr Pro Leu Asp Gly Ala Ala Arg Gly Glu Asp Thr Ser Met Asn
                    20              25                  30

        Leu Val Gln Lys Tyr Leu Glu Asn Tyr Tyr Asp Leu Lys Lys Asp Val
                35              40                  45

        Lys Gln Phe Val Arg Arg Lys Asp Ser Gly Pro Val Val Lys Lys Ile
            50              55                  60

        Arg Glu Met Gln Lys Phe Leu Gly Leu Glu Val Thr Gly Lys Leu Asp
        65              70                  75                  80

        Ser Asp Thr Leu Glu Val Met Arg Lys Pro Arg Cys Gly Val Pro Asp
                    85                  90                  95

        Val Gly His Phe Arg Thr Phe Pro Gly Ile Pro Lys Trp Arg Lys Thr
                    100             105                 110

        His Leu Thr Tyr Arg Ile Val Asn Tyr Thr Pro Asp Leu Pro Lys Asp
                115                 120                 125

        Ala Val Asp Ser Ala Val Glu Lys Ala Leu Lys Val Trp Glu Glu Val
            130                 135                 140

        Thr Pro Leu Thr Phe Ser Arg Leu Tyr Glu Gly Glu Ala Asp Ile Met
        145                 150                 155                 160

        Ile Ser Phe Ala Val Arg Glu His Gly Asp Phe Tyr Pro Phe Asp Gly
                    165                 170                 175

        Pro Gly Asn Val Leu Ala His Ala Tyr Ala Pro Gly Pro Gly Ile Asn
                    180                 185                 190

        Gly Asp Ala His Phe Asp Asp Asp Glu Gln Trp Thr Lys Asp Thr Thr
                195                 200                 205

        Gly Thr Asn Leu Phe Leu Val Ala Ala His Glu Ile Gly His Ser Leu
            210                 215                 220

        Gly Leu Phe His Ser Ala Asn Thr Glu Ala Leu Met Tyr Pro Leu Tyr
        225                 230                 235                 240

        His Ser Leu Thr Asp Leu Thr Arg Phe Arg Leu Ser Gln Asp Asp Ile
                    245                 250                 255

        Asn Gly Ile Gln Ser Leu Tyr Gly Pro Pro Pro Asp Ser Pro Glu Thr
                    260                 265                 270
```

```
Pro Leu Val Pro Thr Glu Pro Val Pro Pro Glu Pro Gly Thr Pro Ala
        275             280             285

Asn Cys Asp Pro Ala Leu Ser Phe Asp Ala Val Ser Thr Leu Arg Gly
        290             295             300

Glu Ile Leu Ile Phe Lys Asp Arg His Phe Trp Arg Lys Ser Leu Arg
305             310             315             320

Lys Leu Glu Pro Glu Leu His Leu Ile Ser Ser Phe Trp Pro Ser Leu
            325             330             335

Pro Ser Gly Val Asp Ala Ala Tyr Glu Val Thr Ser Lys Asp Leu Val
            340             345             350

Phe Ile Phe Lys Gly Asn Gln Phe Trp Ala Ile Arg Gly Asn Glu Val
        355             360             365

Arg Ala Gly Tyr Pro Arg Gly Ile His Thr Leu Gly Phe Pro Pro Thr
    370             375             380

Val Arg Lys Ile Asp Ala Ala Ile Ser Asp Lys Glu Lys Asn Lys Thr
385             390             395             400

Tyr Phe Phe Val Glu Asp Lys Tyr Trp Arg Phe Asp Glu Lys Arg Asn
            405             410             415

Ser Met Glu Pro Gly Phe Pro Lys Gln Ile Ala Glu Asp Phe Pro Gly
            420             425             430

Ile Asp Ser Lys Ile Asp Ala Val Phe Glu Glu Phe Gly Phe Phe Tyr
        435             440             445

Phe Phe Thr Gly Ser Ser Gln Leu Glu Phe Asp Pro Asn Ala Lys Lys
    450             455             460

Val Thr His Thr Leu Lys Ser Asn Ser Trp Leu Asn Cys
465             470             475
```

<210> 15
<211> 93
<212> PRT
<213> Homo sapiens

<400> 15

```
Met Ala Arg Leu Gln Thr Ala Leu Leu Val Val Leu Val Leu Leu Ala
1               5               10              15

Val Ala Leu Gln Ala Thr Glu Ala Gly Pro Tyr Gly Ala Asn Met Glu
```

20                    25                30

```
                20                      25                      30


          Asp Ser Val Cys Cys Arg Asp Tyr Val Arg Tyr Arg Leu Pro Leu Arg
              35                  40                  45


          Val Val Lys His Phe Tyr Trp Thr Ser Asp Ser Cys Pro Arg Pro Gly
              50                  55                  60


          Val Val Leu Leu Thr Phe Arg Asp Lys Glu Ile Cys Ala Asp Pro Arg
          65                  70                  75                  80


          Val Pro Trp Val Lys Met Ile Leu Asn Lys Leu Ser Gln
                          85                  90


          <210> 16
          <211> 91
          <212> PRT
          <213> Homo sapiens

          <400> 16
          Met Lys Val Ser Ala Ala Ala Leu Ala Val Ile Leu Ile Ala Thr Ala
          1               5                   10                  15


          Leu Cys Ala Pro Ala Ser Ala Ser Pro Tyr Ser Ser Asp Thr Thr Pro
                          20                  25                  30


          Cys Cys Phe Ala Tyr Ile Ala Arg Pro Leu Pro Arg Ala His Ile Lys
                      35                  40                  45


          Glu Tyr Phe Tyr Thr Ser Gly Lys Cys Ser Asn Pro Ala Val Val Phe
              50                  55                  60


          Val Thr Arg Lys Asn Arg Gln Val Cys Ala Asn Pro Glu Lys Lys Trp
          65                  70                  75                  80


          Val Arg Glu Tyr Ile Asn Ser Leu Glu Met Ser
                          85                  90


          <210> 17
          <211> 1170
          <212> PRT
          <213> Homo sapiens

          <400> 17
          Met Gly Leu Ala Trp Gly Leu Gly Val Leu Phe Leu Met His Val Cys
          1               5                   10                  15


          Gly Thr Asn Arg Ile Pro Glu Ser Gly Gly Asp Asn Ser Val Phe Asp
                          20                  25                  30


          Ile Phe Glu Leu Thr Gly Ala Ala Arg Lys Gly Ser Gly Arg Arg Leu
                      35                  40                  45
```

```
Val Lys Gly Pro Asp Pro Ser Ser Pro Ala Phe Arg Ile Glu Asp Ala
    50              55              60

Asn Leu Ile Pro Pro Val Pro Asp Asp Lys Phe Gln Asp Leu Val Asp
65              70              75              80

Ala Val Arg Ala Glu Lys Gly Phe Leu Leu Leu Ala Ser Leu Arg Gln
            85              90              95

Met Lys Lys Thr Arg Gly Thr Leu Leu Ala Leu Glu Arg Lys Asp His
            100             105             110

Ser Gly Gln Val Phe Ser Val Val Ser Asn Gly Lys Ala Gly Thr Leu
            115             120             125

Asp Leu Ser Leu Thr Val Gln Gly Lys Gln His Val Val Ser Val Glu
    130             135             140

Glu Ala Leu Leu Ala Thr Gly Gln Trp Lys Ser Ile Thr Leu Phe Val
145             150             155             160

Gln Glu Asp Arg Ala Gln Leu Tyr Ile Asp Cys Glu Lys Met Glu Asn
            165             170             175

Ala Glu Leu Asp Val Pro Ile Gln Ser Val Phe Thr Arg Asp Leu Ala
            180             185             190

Ser Ile Ala Arg Leu Arg Ile Ala Lys Gly Gly Val Asn Asp Asn Phe
            195             200             205

Gln Gly Val Leu Gln Asn Val Arg Phe Val Phe Gly Thr Thr Pro Glu
    210             215             220

Asp Ile Leu Arg Asn Lys Gly Cys Ser Ser Ser Thr Ser Val Leu Leu
225             230             235             240

Thr Leu Asp Asn Asn Val Val Asn Gly Ser Ser Pro Ala Ile Arg Thr
            245             250             255

Asn Tyr Ile Gly His Lys Thr Lys Asp Leu Gln Ala Ile Cys Gly Ile
            260             265             270

Ser Cys Asp Glu Leu Ser Ser Met Val Leu Glu Leu Arg Gly Leu Arg
            275             280             285

Thr Ile Val Thr Thr Leu Gln Asp Ser Ile Arg Lys Val Thr Glu Glu
    290             295             300
```

72

```
Asn Lys Glu Leu Ala Asn Glu Leu Arg Arg Pro Pro Leu Cys Tyr His
305             310             315             320

Asn Gly Val Gln Tyr Arg Asn Asn Glu Glu Trp Thr Val Asp Ser Cys
            325             330             335

Thr Glu Cys His Cys Gln Asn Ser Val Thr Ile Cys Lys Lys Val Ser
            340             345             350

Cys Pro Ile Met Pro Cys Ser Asn Ala Thr Val Pro Asp Gly Glu Cys
            355             360             365

Cys Pro Arg Cys Trp Pro Ser Asp Ser Ala Asp Asp Gly Trp Ser Pro
            370             375             380

Trp Ser Glu Trp Thr Ser Cys Ser Thr Ser Cys Gly Asn Gly Ile Gln
385             390             395             400

Gln Arg Gly Arg Ser Cys Asp Ser Leu Asn Asn Arg Cys Glu Gly Ser
            405             410             415

Ser Val Gln Thr Arg Thr Cys His Ile Gln Glu Cys Asp Lys Arg Phe
            420             425             430

Lys Gln Asp Gly Gly Trp Ser His Trp Ser Pro Trp Ser Ser Cys Ser
            435             440             445

Val Thr Cys Gly Asp Gly Val Ile Thr Arg Ile Arg Leu Cys Asn Ser
            450             455             460

Pro Ser Pro Gln Met Asn Gly Lys Pro Cys Glu Gly Glu Ala Arg Glu
465             470             475             480

Thr Lys Ala Cys Lys Lys Asp Ala Cys Pro Ile Asn Gly Gly Trp Gly
            485             490             495

Pro Trp Ser Pro Trp Asp Ile Cys Ser Val Thr Cys Gly Gly Gly Val
            500             505             510

Gln Lys Arg Ser Arg Leu Cys Asn Asn Pro Thr Pro Gln Phe Gly Gly
            515             520             525

Lys Asp Cys Val Gly Asp Val Thr Glu Asn Gln Ile Cys Asn Lys Gln
            530             535             540

Asp Cys Pro Ile Asp Gly Cys Leu Ser Asn Pro Cys Phe Ala Gly Val
545             550             555             560
```

Lys Cys Thr Ser Tyr Pro Asp Gly Ser Trp Lys Cys Gly Ala Cys Pro
                565                 570                 575

Pro Gly Tyr Ser Gly Asn Gly Ile Gln Cys Thr Asp Val Asp Glu Cys
                580                 585                 590

Lys Glu Val Pro Asp Ala Cys Phe Asn His Asn Gly Glu His Arg Cys
                595                 600                 605

Glu Asn Thr Asp Pro Gly Tyr Asn Cys Leu Pro Cys Pro Pro Arg Phe
    610                 615                 620

Thr Gly Ser Gln Pro Phe Gly Gln Gly Val Glu His Ala Thr Ala Asn
625                 630                 635                 640

Lys Gln Val Cys Lys Pro Arg Asn Pro Cys Thr Asp Gly Thr His Asp
                645                 650                 655

Cys Asn Lys Asn Ala Lys Cys Asn Tyr Leu Gly His Tyr Ser Asp Pro
                660                 665                 670

Met Tyr Arg Cys Glu Cys Lys Pro Gly Tyr Ala Gly Asn Gly Ile Ile
                675                 680                 685

Cys Gly Glu Asp Thr Asp Leu Asp Gly Trp Pro Asn Glu Asn Leu Val
    690                 695                 700

Cys Val Ala Asn Ala Thr Tyr His Cys Lys Lys Asp Asn Cys Pro Asn
705                 710                 715                 720

Leu Pro Asn Ser Gly Gln Glu Asp Tyr Asp Lys Asp Gly Ile Gly Asp
                725                 730                 735

Ala Cys Asp Asp Asp Asp Asp Asn Asp Lys Ile Pro Asp Asp Arg Asp
                740                 745                 750

Asn Cys Pro Phe His Tyr Asn Pro Ala Gln Tyr Asp Tyr Asp Arg Asp
                755                 760                 765

Asp Val Gly Asp Arg Cys Asp Asn Cys Pro Tyr Asn His Asn Pro Asp
    770                 775                 780

Gln Ala Asp Thr Asp Asn Asn Gly Glu Gly Asp Ala Cys Ala Ala Asp
785                 790                 795                 800

Ile Asp Gly Asp Gly Ile Leu Asn Glu Arg Asp Asn Cys Gln Tyr Val
                805                 810                 815

Tyr Asn Val Asp Gln Arg Asp Thr Asp Met Asp Gly Val Gly Asp Gln

74

```
                 820                      825                       830

        Cys Asp Asn Cys Pro Leu Glu His Asn Pro Asp Gln Leu Asp Ser Asp
                835                      840                  845


        Ser Asp Arg Ile Gly Asp Thr Cys Asp Asn Asn Gln Asp Ile Asp Glu
        850                      855                  860


        Asp Gly His Gln Asn Asn Leu Asp Asn Cys Pro Tyr Val Pro Asn Ala
        865                      870                  875                 880


        Asn Gln Ala Asp His Asp Lys Asp Gly Lys Gly Asp Ala Cys Asp His
                            885                  890                  895


        Asp Asp Asp Asn Asp Gly Ile Pro Asp Asp Lys Asp Asn Cys Arg Leu
                    900                  905                  910


        Val Pro Asn Pro Asp Gln Lys Asp Ser Asp Gly Asp Gly Arg Gly Asp
                915                  920                  925


        Ala Cys Lys Asp Asp Phe Asp His Asp Ser Val Pro Asp Ile Asp Asp
                930                  935                  940


        Ile Cys Pro Glu Asn Val Asp Ile Ser Glu Thr Asp Phe Arg Arg Phe
        945                      950                  955                 960


        Gln Met Ile Pro Leu Asp Pro Lys Gly Thr Ser Gln Asn Asp Pro Asn
                    965                  970                  975


        Trp Val Val Arg His Gln Gly Lys Glu Leu Val Gln Thr Val Asn Cys
                    980                  985                  990


        Asp Pro Gly Leu Ala Val Gly Tyr  Asp Glu Phe Asn Ala  Val Asp Phe
                995                  1000                 1005


        Ser Gly  Thr Phe Phe Ile Asn  Thr Glu Arg Asp Asp  Asp Tyr Ala
            1010                 1015                 1020


        Gly Phe  Val Phe Gly Tyr Gln  Ser Ser Ser Arg Phe  Tyr Val Val
            1025                 1030                 1035


        Met Trp  Lys Gln Val Thr Gln  Ser Tyr Trp Asp Thr  Asn Pro Thr
            1040                 1045                 1050


        Arg Ala  Gln Gly Tyr Ser Gly  Leu Ser Val Lys Val  Val Asn Ser
            1055                 1060                 1065


        Thr Thr  Gly Pro Gly Glu His  Leu Arg Asn Ala Leu  Trp His Thr
            1070                 1075                 1080
```

```
Gly Asn  Thr Pro Gly Gln Val  Arg Thr Leu Trp His  Asp Pro Arg
    1085             1090             1095

His Ile  Gly Trp Lys Asp Phe  Thr Ala Tyr Arg Trp  Arg Leu Ser
    1100             1105             1110

His Arg  Pro Lys Thr Gly Phe  Ile Arg Val Val Met  Tyr Glu Gly
    1115             1120             1125

Lys Lys  Ile Met Ala Asp Ser  Gly Pro Ile Tyr Asp  Lys Thr Tyr
    1130             1135             1140

Ala Gly  Gly Arg Leu Gly Leu  Phe Val Phe Ser Gln  Glu Met Val
    1145             1150             1155

Phe Phe  Ser Asp Leu Lys Tyr  Glu Cys Arg Asp Pro
    1160             1165             1170
```